(19) Europäisches Patentamt
European Patent Office
Office européen des brevets

(11) EP 3 430 163 B1

(12) EUROPEAN PATENT SPECIFICATION

(45) Date of publication and mention of the grant of the patent:
**19.10.2022 Bulletin 2022/42**

(21) Application number: **17713053.1**

(22) Date of filing: **15.03.2017**

(51) International Patent Classification (IPC):
***C12Q 1/6886*** *(2018.01)*

(52) Cooperative Patent Classification (CPC):
**C12Q 1/6886;** C12Q 2600/106; C12Q 2600/118; C12Q 2600/158; Y02A 90/10

(86) International application number:
**PCT/GB2017/050712**

(87) International publication number:
**WO 2017/158358 (21.09.2017 Gazette 2017/38)**

(54) **GENE SIGNATURES FOR CANCER DETECTION AND TREATMENT**

GENSIGNATUREN FÜR DEN NACHWEIS UND DIE BEHANDLUNG VON KREBS

SIGNATURES GÉNIQUES POUR LA DÉTECTION ET LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2016 GB 201604398**
**07.06.2016 GB 201609944**
**15.12.2016 GB 201621384**

(43) Date of publication of application:
**23.01.2019 Bulletin 2019/04**

(73) Proprietor: **Almac Diagnostic Services Limited**
**Craigavon BT63 5QD (GB)**

(72) Inventors:
- **EL-HELALI, Aya**
  **Craigavon BT63 5QD (GB)**
- **MCGIVERN, Niamh**
  **Craigavon BT63 5QD (GB)**
- **MCCAVIGAN, Andrena**
  **Craigavon BT63 5QD (GB)**
- **PRICE, Bethanie**
  **Craigavon BT63 5QD (GB)**
- **KNIGHT, Laura**
  **Craigavon BT63 5QD (GB)**
- **MCCABE, Nuala**
  **Craigavon BT63 5QD (GB)**
- **KENNEDY, Richard**
  **Craigavon BT63 5QD (GB)**
- **HARKIN, Paul**
  **Craigavon BT63 5QD (GB)**
- **GOURLEY, Charlie**
  **Craigavon BT63 5QD (GB)**

(74) Representative: **Boult Wade Tennant LLP**
**Salisbury Square House**
**8 Salisbury Square**
**London EC4Y 8AP (GB)**

(56) References cited:

| | |
|---|---|
| **EP-A1- 2 715 348** | **WO-A1-2012/142330** |
| **WO-A1-2013/169858** | **WO-A1-2014/150671** |
| **WO-A1-2015/050500** | **US-A1- 2008 038 251** |
| **US-A1- 2014 073 523** | **US-A1- 2014 342 924** |

- **Tao Xu ET AL: "Upregulation of CD147 promotes cell invasion, epithelial-to-mesenchymal transition and activates MAPK/ERK signaling pathway in colorectal cancer", Int J Clin Exp Pathol, 1 January 2014 (2014-01-01), XP055373512, Retrieved from the Internet: URL:https://www.ncbi.nlm.nih.gov/pmc/articles/PMC4270601/pdf/ijcep0007-7432.pdf**
- **TANG HAITAO ET AL: "SRPX2 Enhances the Epithelial-Mesenchymal Transition and Temozolomide Resistance in Glioblastoma Cells", CELLULAR AND MOLECULAR NEUROBIOLOGY, SPRINGER NEW YORK, US, vol. 36, no. 7, 7 December 2015 (2015-12-07), pages 1067-1076, XP036017194, ISSN: 0272-4340, DOI: 10.1007/S10571-015-0300-9 [retrieved on 2015-12-07]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **NATALIE E SIMPSON ET AL: "An in vitro investigation of metabolically sensitive biomarkers in breast cancer progression", BREAST CANCER RESEARCH AND TREATMENT, KLUWER ACADEMIC PUBLISHERS, BO, vol. 133, no. 3, 20 November 2011 (2011-11-20), pages 959-968, XP035079201, ISSN: 1573-7217, DOI: 10.1007/S10549-011-1871-X**

Remarks:

The file contains technical information submitted after the application was filed and not included in this specification

## Description

### FIELD OF THE INVENTION

The present invention relates to methods for selecting a treatment for a subject with cancer, predicting responsiveness of a subject with cancer to therapeutic agents including MAPK, EMT and SRC pathway inhibitors and determining clinical prognosis based on assessing the expression level of biomarkers.

### BACKGROUND OF THE INVENTION

Mitogen-activated protein kinases (MAPKs) are components of a vital intracellular pathway which controls a vast array of physiologic processes. These enzymes are regulated by a characteristic phosphor-relay system, in which a series of three protein kinases phosphorylate and activate one another. The extracellular signal-regulated kinases (ERKs) function in the control of cell division. The c-Jun amino-terminal kinases (JNKs) are critical regulators of transcription. The p38 MAPKs are activated by inflammatory cytokines and environmental stresses. Because of its role in cell proliferation and carcinogenesis, the most characterised MAPK pathway is the RAS/RAF/MEK/ERK pathway. Mutations of each of these genes are mutually exclusive, but all lead to constitutive activation of the MAPK signal transduction pathway. However this is one of the most frequently dysregulated signal transduction pathways in human cancers, often through gain-of-function mutations of *RAS* and *RAF* family members. Mutations in *KRAS* have been found in 90% of pancreatic cancers, 20% of non-small cell lung cancers (NSCLC), and up to 50% of colorectal and thyroid cancers (Jose et al., 1984) whereas mutations of *BRAF* have been identified in more than 60% of melanoma and 40% to 60% of papillary thyroid cancers (Cohen et al., 2003; Davies et al., 2002, Xu et al., 2003). Although *MEK1/2* is rarely mutated, constitutively active MEK has been found in more than 30% of primary tumour cell lines tested (Hoshino et al., 1999). With regards to ovarian cancer, low-grade serous ovarian carcinoma which accounts for a small proportion of all ovarian serous carcinomas (<20%) are characterized by mutations of the KRAS, BRAF, ERBB2 genes (Lopez et al., 2013). In addition, it is hypothesised that alterations, other than mutation, exist in High grade serous ovarian cancer (HGSOC). It has been found that 11% of HGSOC have KRAS amplification and 12% have BRAF amplification, suggesting that the RAS/RAF/MEK/ERK pathway may have utility outside low grade serous ovarian cancer (The Cancer Genome Atlas (TCGA, Nature 2011). In addition 12% of these tumours have also alteration in the NF1 gene, a RAS GTPase and negative regulator of RAS (TCGA, Nature 2011).

The RAS/RAF/MEK/ERK pathway is activated by a wide array of growth factors and cytokines acting through receptor tyrosine kinases such as EGF, IGF, and TGF. The activated receptors recruit nucleotide exchange proteins which activate RAS through a conversion from the inactive GDP-bound form to the active GTP-bound form. Activated RAS recruits RAF kinase to the membrane, where it is activated by multiple phosphorylation events and where it activates MEK1/2 kinase. MEK1/2 are dualspecificity kinases, catalysing the phosphorylation of both tyrosine and threonine on ERK1 and ERK2. Phosphorylated ERK can translocate to the nucleus where it phosphorylates and activates various transcription factors (Marshall, 1996). Activated ERK1/2 catalyse the phosphorylation of numerous cytoplasmic and nuclear substrates, regulating diverse cellular responses such as mitosis, embryogenesis, cell differentiation, motility, metabolism, and programmed death, as well as angiogenesis (Shaul et al., 2007; Lewis et al., 1998; Johnson et al., 1994; D'Angelo et al., 1995; Na et al., 2010).

Factors associated with resistance to platinum include those that limit the formation of cytotoxic platinum-DNA adducts and those that prevent cell death occurring after platinumadduct formation (Davis et al., 2014). The former may result from reduced uptake of cisplatin into cells, increased efflux via alterations to transport proteins or by inactivation of intracellular cisplatin by conversion into cisplatin-thiol conjugates. The latter form of resistance may occur by increased DNA repair after adduct formation. Alterations in various proteins associated with these repair mechanisms have been associated with platinum resistance, for example high levels of excision repair cross-complementation 1 (ERCC1) protein, mutations or down-regulation of MLH1, MSH2 and MSH1 and secondary mutations of BRCA1 or 2, which can cause reversion to the BRCA genotype and reestablishment of BRCA function, hence increasing HR (Lord and Ashworth, 2013). These various factors may either be present at diagnosis or acquired over time.

A number of studies have tried to characterise the mechanisms of acquired resistance in ovarian cancer. Analysis of 135 spatially and temporally separated samples from 14 patients with HGSOC who received platinum-based chemotherapy found that NF1 deletion showed a progressive increase in tumour allele fraction during chemotherapy (Schwarz et al., 2015). This suggested that subclonal tumour populations are present in pre-treatment biopsies in HGSOC and can undergo expansion during chemotherapy, causing clinical relapse (Schwarz et al., 2015). Additionally alteration of the NF1 gene has been associated with innate cisplatin resistance in HGSOC, whereby 20% of primary tumours showed inactivation of the NF1 gene by mutation or gene breakage (Patch et al., 2015). Furthermore mutation of the RAS-MAPK has been associated with chemotherapy resistance in relapsed neuroblastomas (Eleveld et al., 2015). Additionally, in cell line models, the MAPK pathway has been implicated in cisplatin resistance in ovarian cancer (Benedetti et al., 2008) and in squamous cell carcinoma (Kong et al., 2015).

WO2012/142330 relates to predicting the presence of secondary site metastases by detecting a microRNA sequence. The microRNA detection reagent may be combined with a further detection reagent specific for an mRNA, for example GFPT2.

WO2015/050500 relates to method of making a prognosis as to whether a patient having renal cancer is likely to survive comprising determining the level of expression for each marker of a panel of markers, wherein the panel comprises at least one housekeeping gene selected from the group consisting of ACTB, RPL13A, RPL9, and RPS29 and at least one prognostic gene selected from the group consisting of CXCL5, EFNA5, EMCN, G6PC, GFPT2 , HIST2H3C, IGFBP1, LAMB3, MMP9, MOCOS, PLG, PRAME, RARRES1, SDPR, SLC6A19, TK1, KDELR3 and TSPAN7.

US2014/073523 relates to methods in which glucose metabolism is correlated to oncogenesis through certain specific pathways; inhibition of certain enzymes is shown to interfere with oncogenic signaling, and measurement of certain enzyme levels is correlated with patient survival. The methods comprise measuring the level of expression of at least one of the enzymes involved in glucose uptake or metabolism. Genes whose expression level is measured may include GLUT3, PFKP, GAPDH, ALDOC, LDHA and GFPT2.

EP2715348 relates to predicting a subject's responsiveness to an anti-angiogenic therapeutic agent comprising measuring the expression levels of one or more biomarkers in cancer tissue wherein the biomarkers are defined by an expression signature.

## DESCRIPTION OF THE INVENTION

A cancer with a given histopathological diagnosis may represent multiple diseases at a molecular level. The present inventors have defined a molecular subgroup of cancer characterised by misregulation of the MAPK signalling pathway and the epithelial-mesenchymal transition (EMT) pathway. Biomarker signatures devised by the present inventors can be used to identify cancers within the molecular subgroup. The signatures are also useful for identifying the treatment that is best suited for a given patient.

Thus, in a first aspect the invention provides a method for selecting a treatment for a subject having a cancer, comprising:

(i) measuring the expression level(s) of at least 1 biomarker, wherein the at least one biomarker comprises GFPT2, in a sample from the subject;

(ii) assessing from the expression level(s) of the at least 1 biomarker whether the sample from the subject is positive or negative for a biomarker signature comprising the at least 1 biomarker, wherein:

(a) if the sample is positive for the biomarker signature a MAPK pathway inhibitor is indicated and/or if the sample is negative for the biomarker signature a MAPK pathway inhibitor is not indicated; and/or

(b) if the sample is positive for the biomarker signature an EMT pathway inhibitor is indicated and/or if the sample is negative for the biomarker signature an EMT pathway inhibitor is not indicated; and/or

(c) if the sample is positive for the biomarker signature an SRC pathway inhibitor is not indicated and/or if the sample is negative for the biomarker signature an SRC pathway inhibitor is indicated; and/or

(d) if the sample is positive for the biomarker signature a taxane is indicated and/or if the sample is negative for the biomarker signature a taxane is not indicated.

The methods of the invention may include measuring one or more additional, up to all, of the biomarkers listed in Table B (optionally together with one or more biomarkers from Table A and/or one or more additional biomarkers).

The present inventors have identified that treatment of tumour cells resistant to a platinum-based chemotherapeutic agent (and positive for the biomarker signature) with a MAPK pathway inhibitor can re-sensitise the tumour cells to the platinum-based chemotherapeutic agent. Thus, in the methods described herein the MAPK pathway inhibitor may be combined with a platinum based chemotherapeutic agent. The platinum based chemotherapeutic agent may be administered before, together with, or after the MAPK pathway inhibitor. Preferably, the platinum based chemotherapeutic agent is administered together with, or after, the MAPK pathway inhibitor.

Furthermore, the present inventors have identified that the MAPK and SRC pathways act in parallel such that the inhibition of one signaling cascade leads to the activation of the other. Thus, in the methods described herein the MAPK pathway inhibitor may be combined with a SRC pathway inhibitor. The SRC pathway inhibitor may be administered before, together with, or after the MAPK pathway inhibitor. Preferably, the SRC pathway inhibitor is administered together with, or after, the MAPK pathway inhibitor.

By "indicated" is meant "indicated for treatment", i.e. that the therapeutic agent is predicted to positively treat the cancer. A therapeutic agent is thus "indicated" if the cancer's rate of growth is expected to, or will, decelerate as a result of contact with the therapeutic agent, compared to its growth in the absence of contact with the therapeutic agent. A therapeutic agent can also be considered "indicated" if the subject's overall prognosis (progression free survival and/or overall survival) is expected to, or will, improve by administration of the therapeutic agent.

By "not indicated" is meant "not indicated for treatment", i.e. that the therapeutic agent is predicted not to positively treat the cancer. A therapeutic agent is thus "not indicated" if the cancer's rate of growth is expected to, or will, not decelerate as a result of contact with the therapeutic agent, compared to its growth in the absence of contact with the therapeutic agent. A therapeutic agent can also be considered "not indicated" if the subject's overall prognosis (progression free survival and/or overall survival) is expected to, or will, not improve by administration of the therapeutic agent. A therapeutic agent can also be considered "not indicated" if it is "contraindicated". By "contraindicated" is meant that a worse outcome is expected for the subject than if the subject was treated with the therapeutic agent.

According to a related aspect of the invention there is provided a method for predicting the responsiveness of a subject with cancer to a therapeutic agent comprising:

(i) measuring the expression level(s) of at least 1 biomarker(s), wherein the at least one biomarker comprises GFPT2, in a sample from the subject;
(ii) assessing from the expression level(s) of the at least 1 biomarker(s) whether the sample from the subject is positive or negative for a biomarker signature comprising the at least 1 biomarker;
(iii) classifying the subject as:

(a) predicted to be responsive to a MAPK pathway inhibitor if the sample is positive for the biomarker signature and/or predicted to be non-responsive to the MAPK pathway inhibitor if the sample is negative for the biomarker signature; and/or
(b) predicted to be responsive to an EMT pathway inhibitor if the sample is positive for the biomarker signature and/or predicted to be non-responsive to the EMT pathway inhibitor if the sample is negative for the biomarker signature; and/or
(c) predicted to be non-responsive to a SRC pathway inhibitor if the sample is positive for the biomarker signature and/or predicted to be responsive to the SRC inhibitor if the sample is negative for the biomarker signature; and/or
(d) predicted to be non-responsive to a platinum-based chemotherapeutic agent if the sample is positive for the biomarker signature and/or predicted to be responsive to a platinum-based chemotherapeutic agent if the sample is negative for the biomarker signature; and/or
(e) predicted to be responsive to a taxane if the sample is positive for the biomarker signature and/or predicted to be non-responsive to the taxane if the sample is negative for the biomarker signature.

A cancer is "responsive" to a therapeutic agent if its rate of growth is inhibited as a result of contact with the therapeutic agent, compared to its growth in the absence of contact with the therapeutic agent. Growth of a cancer can be measured in a variety of ways. For instance, the size of a tumor or measuring the expression of tumour markers appropriate for that tumour type. A cancer can also be considered responsive to a therapeutic agent if the subject's overall prognosis (progression free survival and/or overall survival) is improved by the administration of the therapeutic agent.

A cancer is "non-responsive" to a therapeutic agent if its rate of growth is not inhibited, or inhibited to a very low degree or to a non-statistically significant degree, as a result of contact with the therapeutic agent when compared to its growth in the absence of contact with the therapeutic agent. As stated above, growth of a cancer can be measured in a variety of ways, for instance, the size of a tumour or measuring the expression of tumour markers appropriate for that tumour type. A cancer can also be considered non-responsive to a therapeutic agent if the subject's overall prognosis (progression free survival and/or overall survival) is not improved by the administration of the therapeutic agent. Still further, measures of non-responsiveness can be assessed using additional criteria beyond growth size of a tumor such as, but not limited to, patient quality of life, and degree of metastases.

In yet a further aspect, the present invention relates to a method of determining clinical prognosis of a subject with cancer comprising:

(i) measuring the expression level(s) of at least 1 biomarker(s), wherein the at least one biomarker comprises GFPT2, in a sample from the subject;
(ii) assessing from the expression level(s) of the at least 1 biomarker(s) whether the sample from the subject is positive or negative for a biomarker signature comprising the at least 1 biomarker;
(iii) classifying the subject as having a poor prognosis if the sample is positive for the biomarker signature and/or having a good prognosis if the sample is negative for the biomarker signature,

wherein the cancer is prostate cancer, colon cancer, bladder cancer, cervical cancer, glioblastoma, head and neck cancer, glioma, pancreatic cancer, melanoma, stomach cancer or lung cancer; and wherein the biomarker signature identifies the cancer as being in a molecular subgroup of cancer characterized by misregulation of the MAPK signaling pathway and the EMT pathway.

In some embodiments, the at least 1 biomarker further comprises COL5A1 and/or THBS1.

"Poor prognosis" may indicate decreased progression free survival and/or overall survival rates compared to samples that are negative for the biomarker signature and/or good prognosis may indicate increased progression free survival or overall survival rates compared to samples that are positive for the biomarker signature. Poor prognosis may indicate increased likelihood of recurrence or metastasis compared to samples that are negative for the biomarker signature and/or good prognosis may indicate decreased likelihood of recurrence or metastasis compared to samples that are positive for the biomarker signature. Metastasis, or metastatic disease, is the spread of a cancer from one organ or part to another non-adjacent organ or part. The new occurrences of disease thus generated are referred to as metastases.

In certain embodiments the cancer of the subject whose prognosis is determined is not a glioblastoma. In specific embodiments the cancer of the subject whose prognosis is determined is lung adenocarcinoma. In specific embodiments the cancer of the subject whose prognosis is determined is prostate cancer and the subject is not receiving a taxane, optionally wherein the subject with prostate cancer has been treated with radical prostatectomy and/or radical radiotherapy. The cancer may also be lower grade glioma. In certain embodiments the subject is receiving, has received and/or will receive treatment with the standard of care treatment. A skilled practitioner is aware of the standard of care treatment for the particular cancer. In some embodiments, the standard of care treatment incorporates a platinum-based chemotherapeutic agent (as defined herein).

The signatures disclosed herein provide a prognostic indication. This may apply to untreated patients. It may also apply to patients treated with standard of care treatment. However, the signatures disclosed herein also predict responsiveness to particular targeted, or indicated, therapeutic agents. Accordingly, in specific embodiments a subject with cancer whose sample is positive for the biomarker signature may have a better prognosis when treated with an indicated therapeutic agent to which they are predicted to be responsive than a subject with a cancer whose sample is negative for the biomarker signature and who is treated with the same therapeutic agent. For example, it is shown herein that subjects with (de novo) metastatic prostate cancer who are signature positive have a good prognosis (increased overall survival) relative to signature negative subjects when treated with a taxane. Thus, according to all aspects of the invention, a subject that is signature positive may be selected for therapy and this improves their prognosis. Alternatively, a subject that is signature negative has an improved prognosis and thus is not selected for therapy.

According to a further aspect the invention provides a method for selecting a treatment for a subject having a cancer, comprising:

(i) measuring the expression level(s) of at least 1 biomarker, wherein the at least one biomarker comprises GFPT2, in a sample from the subject;

(ii) assessing from the expression level(s) of the at least 1 biomarker whether the sample from the subject is positive or negative for a biomarker signature comprising the at least 1 biomarker, wherein an increased expression level and/or a decreased expression level of the at least 1 biomarker as shown in Tables C and D indicates that the sample is positive or negative for the biomarker signature, wherein:

(a) if the sample is positive for the biomarker signature a MAPK pathway inhibitor is indicated and/or if the sample is negative for the biomarker signature a MAPK pathway inhibitor is not indicated; and/or

(b) if the sample is positive for the biomarker signature an EMT pathway inhibitor is indicated and/or if the sample is negative for the biomarker signature an EMT pathway inhibitor is not indicated; and/or

(c) if the sample is positive for the biomarker signature an SRC pathway inhibitor is not indicated and/or if the sample is negative for the biomarker signature an SRC pathway inhibitor is indicated.

By "treatment" is meant any therapy or surgery that may be provided to a subject in order to improve, stabilise, or minimise deterioration of a medical condition. The condition relevant to the present invention is cancer (in particular a cancer of the type indicated herein). Means of administration of therapeutic agents include oral, rectal, sublingual, sublabial, intravenous, intraatricular, intracardiac, intracavernous, intramuscular, epidural, intracerebral, intracerebroventricular, epicutaneous, nasal, intrathecal, or via a gastral or duodenal feeding tube and are known in the art. Similarly dosage forms and dosage regimes are known for therapeutic agents and can be determined by a practising physician. Therapeutic agents are approved and marketed for administration in a given dosage form, together with detailed prescribing instructions. Thus, the invention is not limited in relation to how, or in what form, the therapeutic agent is administered since the skilled person would be in a position to determine this based on the therapeutic agent of interest.

**Table A - Weighting and bias for the 45 gene signature**

| 45 Gene Signature | | | |
|---|---|---|---|
| Rank | Gene Name | Weight | Bias |
| 1 | TMEM200A | 0.059481295 | 3.681329367 |
| 2 | GJB2 | 0.055985433 | 4.479833955 |
| 3 | MMP13 | 0.038284076 | 3.724107067 |
| 4 | GFPT2 | 0.037990641 | 4.860237265 |
| 5 | POSTN | -0.035480409 | 4.359882019 |
| 6 | BICC1 | 0.030426737 | 3.698203663 |
| 7 | CDH11 | 0.028340142 | 4.996780524 |
| 8 | MRVI1 | 0.025598535 | 5.076083782 |
| 9 | PMP22 | 0.024034610 | 5.564463361 |
| 10 | COL11A1 | -0.023672753 | 3.500170591 |
| 11 | IGFL2 | 0.022225316 | 3.310383445 |
| 12 | LUM | -0.022014619 | 8.336273473 |
| 13 | NTM | -0.021750365 | 4.230245127 |
| 14 | BGN | 0.021089508 | 10.15236225 |
| 15 | COL3A1 | -0.021023256 | 8.323635399 |
| 16 | COL10A1 | 0.019650845 | 6.353832828 |
| 17 | RAB31 | 0.018014921 | 5.317119481 |
| 18 | ANGPTL2 | 0.016630934 | 5.639562288 |
| 19 | PLAU | 0.016596202 | 5.848820224 |
| 20 | COL8A1 | 0.016373799 | 6.419330171 |
| 21 | MIR1245 | 0.015290888 | 5.455187262 |
| 22 | POLD2 | 0.014555548 | 9.38782491 |
| 23 | NKD2 | 0.014468847 | 7.371707677 |
| 24 | FZD1 | 0.014334768 | 4.151874676 |
| 25 | COPZ2 | 0.013866713 | 5.103944696 |
| 26 | ITGA5 | 0.013561913 | 8.36627973 |
| 27 | VGLL3 | 0.012488674 | 4.501866677 |
| 28 | INHBA | -0.011763261 | 4.684272993 |
| 29 | MMP14 | 0.011010832 | 10.08406264 |
| 30 | VCAN | 0.009977966 | 5.551759846 |
| 31 | THBS2 | -0.008700202 | 8.130920944 |
| 32 | RUNX2 | 0.008333275 | 4.73450528 |
| 33 | TIMP3 | 0.008141253 | 6.498316457 |
| 34 | SFRP2 | -0.007890741 | 5.601725816 |
| 35 | COL1A2 | 0.007788938 | 6.01000198 |
| 36 | COL5A2 | -0.007217722 | 3.567060064 |

(continued)

| 45 Gene Signature | | | |
|---|---|---|---|
| Rank | Gene Name | Weight | Bias |
| 37 | SERPINF1 | 0.006801251 | 10.8333948 |
| 38 | KIF26B | -0.005249312 | 4.97815094 |
| 39 | TNFAIP6 | 0.004963450 | 5.361760185 |
| 40 | MMP2 | 0.003988003 | 5.362247865 |
| 41 | FN1 | 0.003130435 | 4.984016427 |
| 42 | ALPK2 | 0.002394440 | 3.513604572 |
| 43 | CTSK | 0.001542586 | 5.732155915 |
| 44 | LOXL1 | -0.001415170 | 9.593869933 |
| 45 | FAP | -0.000007237 | 5.23E+00 |

**Table B** - **Weighting and bias for the 15 gene signature**

| 15 Gene Signature | | | | |
|---|---|---|---|---|
| Rank | Gene Name | Entrez Gene ID | Weight | Bias |
| 1 | GJB2 | 2706 | 0.089719778 | 4.478098614 |
| 2 | CDH11 | 1009 | 0.066544238 | 4.990055702 |
| 3 | GFPT2 | 9945 | 0.058421032 | 4.885349473 |
| 4 | COL10A1 | 1300 | 0.040148445 | 6.357258041 |
| 5 | ANGPTL2 | 23452 | 0.038272311 | 5.631697532 |
| 6 | THBS1 | 7057 | 0.036613387 | 6.42811488 3 |
| 7 | RAB31 | 11031 | 0.033158407 | 5.300536304 |
| 8 | THBS2 | 7058 | -0.030849235 | 8.135441538 |
| 9 | INHBA | 3624 | -0.028500708 | 4.68290899 |
| 10 | MMP14 | 4323 | 0.020727894 | 10.07844987 |
| 11 | VCAN | 1462 | 0.020706504 | 5.529961284 |
| 12 | PLAU | 5328 | 0.019342831 | 5.850016491 |
| 13 | COL5A1 | 1289 | 0.010674165 | 5.569094517 |
| 14 | FAP | 2191 | -0.006101691 | 5.226391586 |
| 15 | FN1 | 2335 | -0.005998124 | 4.982941989 |

According to all aspects of the invention an increased expression level and/or a decreased expression level of the biomarker(s) may contribute to the determination that the sample is positive or negative for the biomarker signature. As shown in Tables C and D, a threshold level of gene expression can be set and a value above or below that threshold may then indicate increased or decreased expression levels. Of course, the invention is not limited to the specific values; the skilled person would appreciate that the suitable values may be determined depending upon the data set in question.

**Table C - Up/Down Regulation in signature positive and negative groups for the 45 gene signature**

| | | R0171 | | | ICON7 | | | Tothill | | |
|---|---|---|---|---|---|---|---|---|---|---|
| 45 Gene Signature | | Up/Down Regulation | | | Up/Down Regulation | | | Up/Down Regulation | | |
| Rank | Gene Name | Median Threshold | Sig Pos | Sig Neg | Median Threshold | Sig Pos | Sig Neg | Median Threshold | Sig Pos | Sig Neg |
| 1 | TMEM200A | 3.6466 | 5.0657 | 2.968975 | 3.431955 | 5.07791 | 2.68994 | 4.75751 | 6.21209 | 4.579655 |
| 2 | GJB2 | 4.2951 | 6.2298 | 3.22823 | 4.8581 | 6.37259 | 3.81911 | 5.42481 | 8.50465 | 5.246225 |
| 3 | MMP13 | 2.5777 | 5.3747 | 2.147585 | 2.100465 | 5.42182 | 1.88248 | 2.72939 | 5.18685 | 2.66861 |
| 4 | GFPT2 | 5.0409 | 5.8341 | 4.445485 | 4.98664 | 5.52659 | 4.3683 | 4.47669 | 5.48663 | 4.34832 |
| 5 | POSTN | 3.9196 | 6.0564 | 3.111495 | 3.89304 | 5.37474 | 3.44404 | 6.0011 | 8.89395 | 5.52013 |
| 6 | BICC1 | 3.6377 | 4.3439 | 3.25068 | 3.86463 | 4.46272 | 3.52687 | 3.52687 | 4.60078 | 3.41863 |
| 7 | CDH11 | 4.959 | 5.9437 | 4.50373 | 4.97344 | 5.70289 | 4.47351 | 4.7575 | 5.75619 | 4.573065 |
| 8 | MRVI1 | 5.0188 | 5.896 | 4.67305 | 5.83413 | 6.37259 | 5.66073 | 4.9964 | 6.07559 | 4.85401 |
| 9 | PMP22 | 5.4884 | 6.0903 | 5.227205 | 5.321515 | 5.95533 | 4.9538 | 4.5429 | 5.08954 | 4.462555 |
| 10 | COL11A1 | 3.1807 | 5.2886 | 2.57622 | 2.55951 | 3.73033 | 2.26097 | 5.7018 | 9.63595 | 5.3606 |
| 11 | IGFL2 | 3.1305 | 3.9419 | 2.7999 | 3.11509 | 3.60992 | 2.93275 | 4.47336 | 6.6255 | 4.37059 |
| 12 | LUM | 8.3049 | 9.8639 | 7.72196 | 7.754525 | 9.50299 | 7.22957 | 8.44188 | 10.9589 | 8.20698 |
| 13 | NTM | 4.1848 | 5.5422 | 3.40771 | 4.744045 | 5.94384 | 4.18479 | 4.47668 | 6.64725 | 4.124785 |
| 14 | BGN | 10.123 | 11.055 | 9.72598 | 9.636855 | 10.6681 | 9.26884 | 7.03593 | 9.26754 | 6.88147 |
| 15 | COL3A1 | 8.4315 | 9.6558 | 7.62774 | 9.130145 | 10.5306 | 8.63779 | 11.3205 | 12.3325 | 11.1339 |
| 16 | COL10A1 | 6.1925 | 7.639 | 5.687805 | 6.18854 | 7.62534 | 5.67381 | 5.67381 | 8.43772 | 5.350655 |
| 17 | RAB31 | 5.2259 | 6.0563 | 4.85749 | 4.87908 | 5.26397 | 4.75062 | 7.84569 | 9.69752 | 7.72347 |
| 18 | ANGPTL2 | 5.5728 | 6.1959 | 5.35038 | 6.13942 | 6.79376 | 5.80861 | 4.50872 | 5.3606 | 4.473445 |
| 19 | PLAU | 5.7826 | 7.0685 | 5.39088 | 6.085085 | 7.02735 | 5.75619 | 6.37259 | 8.86471 | 6.14652 |
| 20 | COL8A1 | 6.2628 | 7.3746 | 5.92596 | 6.146445 | 7.3527 | 5.83413 | 2.18156 | 4.50373 | 2.04513 |
| 21 | MIR1245 | 5.4218 | 6.9783 | 4.613905 | 4.42027 | 5.74407 | 3.6255 | 6.45712 | 9.67813 | 6.20229 |
| 22 | POLD2 | 9.4897 | 10.075 | 8.97053 | 9.91729 | 10.2823 | 9.71438 | 6.58238 | 6.0885 | 6.64004 |
| 23 | NKD2 | 7.3833 | 8.3311 | 6.710345 | 6.20246 | 7.38327 | 5.74407 | 5.72925 | 6.37317 | 5.59965 |

(continued)

| | | R0171 | | | ICON7 | | | Tothill | | |
|---|---|---|---|---|---|---|---|---|---|---|
| 45 Gene Signature | | Up/Down Regulation | | | Up/Down Regulation | | | Up/Down Regulation | | |
| Rank | Gene Name | Median Threshold | Sig Pos | Sig Neg | Median Threshold | Sig Pos | Sig Neg | Median Threshold | Sig Pos | Sig Neg |
| 24 | FZD1 | 4.1376 | 4.7122 | 3.835685 | 4.18478 | 4.67879 | 3.87365 | 5.74279 | 6.48761 | 5.64527 |
| 25 | COPZ2 | 5.022 | 5.7167 | 4.793585 | 4.4387 | 4.84834 | 4.28628 | 5.14939 | 6.10955 | 5.06428 |
| 26 | ITGA5 | 8.3489 | 9.247 | 7.84255 | 7.075035 | 7.70612 | 6.74078 | 6.02345 | 7.05465 | 5.93189 |
| 27 | VGLL3 | 4.7684 | 5.8592 | 3.929875 | 4.283195 | 5.6291 | 3.0979 | 3.50074 | 5.65796 | 3.238385 |
| 28 | INHBA | 4.799 | 5.8341 | 3.95036 | 4.15386 | 5.55123 | 3.6377 | 5.75619 | 8.93272 | 5.35302 |
| 29 | MMP14 | 10.15 | 10.959 | 9.74279 | 10.1416 | 10.9561 | 9.75752 | 5.5194 | 6.22976 | 5.463645 |
| 30 | VCAN | 5.6749 | 6.9728 | 5.02986 | 6.0345 | 7.40045 | 5.30103 | 7.48408 | 10.3997 | 6.896115 |
| 31 | THBS2 | 8.2106 | 9.91 | 7.244625 | 8.12437 | 9.68603 | 7.14035 | 7.98033 | 11.2575 | 7.53488 |
| 32 | RUNX2 | 4.7684 | 5.5194 | 4.45994 | 4.9964 | 5.52013 | 4.78477 | 3.71929 | 4.71445 | 3.616915 |
| 33 | TIMP3 | 6.6328 | 7.618 | 6.148145 | 6.65443 | 7.52754 | 6.07098 | 6.33734 | 8.3709 | 6.20779 |
| 34 | SFRP2 | 5.1867 | 6.4853 | 4.87875 | 4.96453 | 6.77789 | 4.57074 | 6.1626 | 10.7345 | 5.687825 |
| 35 | COL1 A2 | 6.0235 | 6.8854 | 5.5466 | 5.85296 | 6.92015 | 5.6464 | 11.7545 | 12.4163 | 11.5546 |
| 36 | COL5A2 | 3.3203 | 4.7575 | 2.7999 | 2.109115 | 3.45142 | 1.93264 | 7.58833 | 10.9619 | 7.378935 |
| 37 | SERPINF1 | 10.852 | 11.276 | 10.542 | 10.5045 | 10.85 | 10.3568 | 8.23385 | 10.7163 | 7.971665 |
| 38 | KIF26B | 5.0465 | 5.8838 | 4.646265 | 4.487515 | 5.6464 | 4.18041 | 4.02287 | 5.18685 | 3.94881 |
| 39 | TNFAIP6 | 5.6232 | 7.0236 | 4.696 | 5.39467 | 6.28051 | 4.50373 | 5.02198 | 6.66868 | 4.73379 |
| 40 | MMP2 | 5.0657 | 6.1321 | 4.7595 | 5.56188 | 6.41938 | 5.08709 | 6.89652 | 9.34124 | 6.692765 |
| 41 | FN1 | 5.0465 | 6.2104 | 4.43652 | 4.83753 | 6.29804 | 4.58857 | 10.6461 | 11.8498 | 10.36865 |
| 42 | ALPK2 | 3.1273 | 4.4405 | 2.636575 | 3.005285 | 3.87365 | 2.84256 | 3.50074 | 5.45585 | 3.41863 |
| 43 | CTSK | 5.6453 | 7.2439 | 5.14669 | 6.0665 | 7.53176 | 5.54197 | 7.46379 | 10.6461 | 7.21515 |
| 44 | LOXL1 | 9.6781 | 10.522 | 9.18543 | 9.40259 | 10.269 | 9.07973 | 6.83225 | 8.48727 | 6.658005 |
| 45 | FAP | 5.4623 | 7.0793 | 4.05782 | 5.05518 | 6.94192 | 3.94881 | 6.06714 | 8.72705 | 5.65024 |

**Table D - Up/Down Regulation in signature positive and negative groups for the 15 gene signature**

| | | R0171 | | | ICON7 | | | Tothill | | |
|---|---|---|---|---|---|---|---|---|---|---|
| 15 Gene Signature | | Up/Down Regulation | | | Up/Down Regulation | | | Up/Down Regulation | | |
| Rank | Gene Name | Median Threshold | Sig Pos | Sig Neg | Median Threshold | Sig Pos | Sig Neg | Median Threshold | Sig Pos | Sig Neg |
| 1 | GJB2 | 4.33026 | 6.48402 | 3.25515 | 4.85745 | 6.19138 | 3.21176 | 5.35967 | 7.58391 | 4.50547 |
| 2 | CDH11 | 4.95514 | 5.957695 | 4.57074 | 4.97344 | 5.44587 | 4.45419 | 5.86851 | 6.98271 | 5.38762 |
| 3 | GFPT2 | 5.0409 | 5.908965 | 4.51315 | 5.009845 | 5.4989 | 4.02287 | 4.90305 | 5.82236 | 4.46339 |
| 4 | COL10A1 | 6.17841 | 7.691215 | 5.73055 | 6.179605 | 7.29219 | 5.54159 | 5.59965 | 7.79347 | 4.70377 |
| 5 | ANGPTL2 | 5.57261 | 6.272925 | 5.35491 | 6.152555 | 6.79748 | 5.75619 | 4.58191 | 5.32149 | 4.27131 |
| 6 | THBS1 | 6.42853 | 7.53905 | 6.05232 | 7.087685 | 7.96919 | 6.64004 | 3.88865 | 4.79113 | 3.47753 |
| 7 | RAB31 | 5.20634 | 6.124385 | 4.86097 | 4.854735 | 5.24978 | 4.64093 | 7.89046 | 9.1462 | 7.38142 |
| 8 | THBS2 | 8.20969 | 9.916045 | 7.25212 | 8.197455 | 9.54184 | 6.99702 | 7.85242 | 10.0919 | 6.65443 |
| 9 | INHBA | 4.7808 | 5.934 | 3.92521 | 4.1656 | 5.44265 | 3.39663 | 5.64527 | 7.70293 | 4.75757 |
| 10 | MMP14 | 10.1356 | 11.0802 | 9.73076 | 10.12305 | 10.687 | 9.58809 | 5.462 | 5.7299 | 5.32419 |
| 11 | VCAN | 5.68904 | 7.053055 | 5.0409 | 6.0165 | 7.09525 | 5.04218 | 7.37274 | 9.14764 | 6.30121 |
| 12 | PLAU | 5.78374 | 7.113495 | 5.36554 | 6.085085 | 6.90371 | 5.54197 | 6.27759 | 7.92671 | 5.71559 |
| 13 | COL5A1 | 5.34396 | 6.458105 | 5.06873 | 5.4289 | 6.1982 | 5.14939 | 6.9052 | 8.47557 | 6.24942 |
| 14 | FAP | 5.462 | 7.256835 | 4.06058 | 5.0628 | 6.82319 | 3.69681 | 5.98698 | 8.11692 | 4.83618 |
| 15 | FN1 | 5.04218 | 6.220655 | 4.43401 | 4.83223 | 6.02411 | 4.4343 | 10.9984 | 12.3804 | 10.276 |

The biomarker signature may be defined by the probesets listed in Tables E and F and by the expression levels of the corresponding genes, which may be measured using the probesets. The biomarker signature may include the expression levels of one or more additional biomarkers, which may be measured in any suitable way, for example using one or more additional probesets.

**Table E - 45 gene signature probeset information**

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OCAD NP. 123 61_s_a t | Expre ssion probe set | Sense (includ es Introni c) | 11 | ENS G000 0019 8796 | AL PK 2 | 115701 | alpha-kinase 2 [Source:HGNC Symbol ;Acc: 20565] | Chr 18 | Rev erse Stra nd | 1 |
| OC3SN G. 596-9a_s_a t | Expre ssion probe set | Sense (Fully Exonic ) | 11 | ENS G000 0019 8796 | AL PK 2 | 115701 | alpha-kinase 2 [Source:HGNC Symbol ;Acc: 20565] | Chr 18 | Rev erse Stra nd | 2 |
| OC3P. 2679.C 1_s_ at | Expre ssion probe set | Sense (Fully Exonic ) | 11 | ENS G000 0013 6859 | AN GP TL2 | 23452 | angiopoietin-like 2 [Source:HGNC Symbol ; Acc:490] | Chr 9 | Rev erse Stra nd | 3 |
| ADXStr ongB12 _at | Almac pixella tion control | Sense (Fully Exonic ) | 1 | ENS G000 0013 8061 | CY P1 B1 | 1545 | cytochrome P450, family 1, subfamily B, polypeptide 1 [Source:HGNC Symbol ;Acc: 2597] | Chr 2 | Rev erse Stra nd | N/A |
| ADXStr ongB12 _at | Almac pixella tion contro l | Sense (Fully Exonic ) | 1 | ENS G000 0008 3168 | KA T6 A | 7994 | K(lysine) acetyltransferase 6A [Source: HGNC Symbol ;Acc:13013] | Chr 8 | Rev erse Stra nd | N/A |
| ADXStr ongB12 _at | Almac pixella tion contro l | Sense (Fully Exonic ) | 1 | ENS GOOO 0007 1051 | NC K2 | 8440 | NCK adaptor protein 2 [Source:HGNC Symbol ;Acc:7665] | Chr 2 | For ward Stra nd | N/A |
| ADXStr ongB12 _at | Almac pixella tion contro l | Sense (Fully Exonic ) | 1 | ENS GOOO 0013 6859 | AN GP TL2 | 23452 | angiopoietin-like 2 [Source:HGNC Symbol ; Acc:490] | Chr 9 | Rev erse Stra nd | N/A |
| ADXStr ongB12 _at | Almac pixella tion contro l | Sense (Fully Exonic ) | 1 | ENS GOOO 0015 4146 | NR GN | 4900 | neurogranin (protein kinase C substrate, RC3) [Source:HGNC Symbol ;Acc:8000] | Chr 11 | For ward Stra nd | N/A |
| ADXStr ongB12 _at | Almac pixella tion contro l | Sense (Fully Exonic ) | 1 | ENS GOOO 0007 4370 | AT P2 A3 | 489 | ATPase, Ca++ transporting, ubiquitous [Source:HGNC Symbol;Acc:813] | Chr 17 | Rev erse Stra nd | N/A |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| ADXStr ongB12 _at | Almac pixella tion contro l | Sense (Fully Exonic ) | 1 | ENS GOOO 0007 6513 | AN KR D1 3A | 88455 | ankyrin repeat domain 13A [Source:HGNC Symbol ;Acc:21268] | Chr 12 | For ward Stra nd | N/A |
| ADXStr ongB12 _at | Almac pixella tion contro l | Sense (Fully Exonic ) | 1 | ENS GOOO 0018 2208 | MO B2 | 81532 | MOB kinase activator 2 [Source:HGNC Symbol ;Acc:24904] | Chr 11 | Rev erse Stra nd | N/A |
| ADXStr ongB12 _at | Almac pixella tion contro l | Sense (Fully Exonic ) | 1 | ENS GOOO 0014 8334 | PT GE S2 | 80142 | prostaglandin E synthase 2 [Source:HGNC Symbol ;Acc:17822] | Chr 9 | Rev erse Stra nd | N/A |
| ADXStr ongB12 _at | Almac pixella tion contro l | Sense (Fully Exonic ) | 1 | ENS GOOO 0018 5650 | ZF P36 L1 | 677 | ZFP36 ring finger protein-like 1 [Source: HGNC Symboi;Acc:1107] | Chr 14 | Rev erse Stra nd | N/A |
| OC3P. 9834.C1_s_ at | Expre ssion probe set | Sense (Fully Exonic ) | 11 | ENS GOOO 0013 6859 | AN GP TL2 | 23452 | angiopoietin-like 2 [Source:HGNC Symbol ; Acc:490] | Chr 9 | Rev erse Stra nd | 4 |
| OCMX. 9546.C 1_x_ at | Expre ssion probe set | Sense (Fully Exonic ) | 1 | ENS G000 0013 6859 | AN GP TL2 | 23452 | angiopoietin-like 2 [Source:HGNC Symbol ; Acc:490] | Chr 9 | Rev erse Stra nd | 5 |
| OCAD A. 8226 _s_at | Expre ssion probe set | Sense (includ es Introni c) | 11 | ENS G000 0013 6859 | AN GP TL2 | 23452 | angiopoietin-like 2 [Source:HGNC Symbol ; Acc:490] | Chr 9 | Rev erse Stra nd | 6 |
| OCAD NP. 881 1_s_at | Expre ssion probe set | Sense (Fully Exonic ) | 8 | ENS G000 0013 6859 | AN GP TL2 | 23452 | angiopoietin-like 2 [Source:HGNC Symbol ; Acc:490] | Chr 9 | Rev erse Stra nd | 7 |
| OCHP. 937_s_ at | Expre ssion probe set | Sense (Fully Exonic ) | 11 | ENS G000 0018 2492 | BG N | 633 | biglycan [Source:HGNC Symbol ;Acc:1044] | Chr X | For ward Stra nd | 8 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OCAD NP. 988 3_s_at | Expre ssion probe set | Sense (Fully Exonic ) | 11 | ENS G000 0018 2492 | BG N | 633 | biglycan [Source:HGNC Symbol ;Acc:1044] | Chr X | For ward Stra nd | 9 |
| ADXStr ong61_ at | Almac pixella tion contro l | Sense (Fully Exonic ) | 1 | ENS G000 0021 3625 | LE PR OT | 54741 | leptin receptor overlapping transcript [Source:HGNC Symbol ;Acc:29477] | Chr 1 | For ward Stra nd | N/A |
| ADXStr ong61_ at | Almac pixella tion contro l | Sense (Fully Exonic ) | 1 | ENS G000 0010 4886 | PL EK HJ1 | 55111 /// 102466 736 | pleckstrin homology domain containing, family J member 1 [Source:HGNC Symbol ; Acc:18211] | Chr 19 | Rev erse Stra nd | N/A |
| ADXStr ong61_ at | Almac pixella tion contro l | Sense (Fully Exonic ) | 1 | ENS G000 0023 9672 | NM E1 | 4830 | NME/NM23 nucleoside diphosphate kinase 1 [Source:HGNC Symbol ;Acc:7849] | Chr 17 | For ward Stra nd | N/A |
| ADXStr ong61_ at | Almac pixella tion contro l | Sense (Fully Exonic ) | 1 | ENS G000 0010 3275 | UB E2I | 7329 | ubiquitin-conjugating enzyme E2I [Source: HGNC Symbol ;Acc:12485] | Chr 16 | For ward Stra nd | N/A |
| ADXStr ong61_ at | Almac pixella tion contro l | Sense (Fully Exonic ) | 1 | ENS G000 0010 1639 | CE P19 2 | 55125 | centrosomal protein 192kDa [Source:HGNC Symbol;Acc:25515] | Chr 18 | For ward Stra nd | N/A |
| ADXStr ong61_ at | Almac pixella tion contro l | Sense (Fully Exonic ) | 1 | ENS G000 0018 2492 | BG N | 633 | biglycan [Source:HGNC Symbol ;Acc:1044] | Chr X | For ward Stra nd | N/A |
| ADXStr ong61_ at | Almac pixella tion contro l | Sense (Fully Exonic ) | 1 | ENS G000 0013 6021 | SC YL2 | 55681 | SCY1-like 2 (S. cerevisiae) [Source:HGNC Symbol ;Acc:19286] | Chr 12 | For ward Stra nd | N/A |
| ADXStr ong61_ at | Almac pixella tion contro l | Sense (Fully Exonic ) | 1 | ENS G000 0013 0175 | PR KC SH | 5589 | protein kinase C substrate 80K-H [Source: HGNC Symbol;Acc:9411] | Chr 19 | For ward Stra nd | N/A |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| ADXStr ong61_ at | Almac pixella tion contro l | Sense (Fully Exonic ) | 1 | ENS G000 0022 1411 | MI R1 227 | 100302 283 | microRNA 1227 [Source:HGNC Symbol ; Acc:33932] | Chr 19 | Rev erse Stra nd | N/A |
| ADXStr ong61_ at | Almac pixella tion contro l | Sense (Fully Exonic ) | 1 | ENS G000 0012 9007 | CA LM L4 | 91860 | calmodulin-like 4 [Source:HGNC Symbol ; Acc:18445] | Chr 15 | Rev erse Stra nd | N/A |
| ADXStr ong61_ at | Almac pixella tion contro l | Sense (Fully Exonic ) | 1 | ENS G000 0026 0007 | N/A | N/A | Uncharacterized protein [Source:UniProtKB/ TrEMBL;Ac c:H3BRN7] | Chr 15 | Rev erse Stra nd | N/A |
| OCAD NP. 195 0_s_at | Expre ssion probe set | Sense (includ es Introni c) | 11 | ENS G000 0012 2870 | BIC C1 | 80114 | bicaudal C homolog 1 (Drosophila) [Source: HGNC Symbol ;Acc:19351] | Chr 10 | For ward Stra nd | 10 |
| OCAD A. 1038 8_s_at | Expre ssion probe set | Sense (Fully Exonic ) | 11 | ENS G000 0012 2870 | BIC C1 | 80114 | bicaudal C homolog 1 (Drosophila) [Source: HGNC Symbol ;Acc:19351] | Chr 10 | For ward Stra nd | 11 |
| OCMXSNG.4 199_x_ at | Expre ssion probe set | Sense (includ es Introni c) | 6 | ENS G000 0012 2870 | BIC C1 | 80114 | bicaudal C homolog 1 (Drosophila) [Source: HGNC Symbol ;Acc:19351] | Chr 10 | For ward Stra nd | 12 |
| OC3SN Gnh. 70 31_s_a t | Expre ssion probe set | Sense (Fully Exonic ) | 11 | ENS G000 0012 2870 | BIC C1 | 80114 | bicaudal C homolog 1 (Drosophila) [Source: HGNC Symbol ;Acc:19351] | Chr 10 | For ward Stra nd | 13 |
| OCRS2 . 4990_ s_at | Expre ssion probe set | Sense (Fully Exonic ) | 11 | ENS G000 0012 2870 | BIC C1 | 80114 | bicaudal C homolog 1 (Drosophila) [Source: HGNC Symbol ;Acc:19351] | Chr 10 | For ward Stra nd | 14 |
| OC3SN Gnh. 67 78_s_a t | Expre ssion probe set | Sense (includ es Introni c) | 11 | ENS G000 0012 2870 | BIC C1 | 80114 | bicaudal C homolog 1 (Drosophila) [Source: HGNC Symbol ;Acc:19351] | Chr 10 | For ward Stra nd | 15 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OC3SN Gnh. 11 887_x_ at | Expre ssion probe set | Sense (includ es Introni c) | 11 | ENS GOOO 0012 2870 | BIC C1 | 80114 | bicaudal C homolog 1 (Drosophila) [Source: HGNC Symbol ;Acc:19351] | Chr 10 | For ward Stra nd | 16 |
| OC3P. 14147. C1_s_ a t | Expre ssion probe set | Sense (Fully Exonic ) | 11 | ENS G000 0014 0937 | CD H1 1 | 1009 | cadherin 11, type 2, OBcadherin (osteoblast) [Source:HGNC Symbol ;Acc:1750] | Chr 16 | Rev erse Stra nd | 17 |
| OCAD NP. 100 24_s_a t | Expre ssion probe set | Sense (Fully Exonic ) | 11 | ENS G000 0014 0937 | CD H1 1 | 1009 | cadherin 11, type 2, OBcadherin (osteoblast) [Source:HGNC Symbol ;Acc:1750] | Chr 16 | Rev erse Stra nd | 18 |
| OCHP. 148_s_ at | Expre ssion probe set | Sense (Fully Exonic ) | 11 | ENS G000 0014 0937 | CD H1 1 | 1009 | cadherin 11, type 2, OBcadherin (osteoblast) [Source:HGNC Symbol ;Acc:1750] | Chr 16 | Rev erse Stra nd | 19 |
| OCAD A. 6210 _s_at | Expre ssion probe set | Sense (includ es Introni c) | 9 | ENS G000 0014 0937 | CD H1 1 | 1009 | cadherin 11, type 2, OBcadherin (osteoblast) [Source:HGNC Symbol ;Acc:1750] | Chr 16 | Rev erse Stra nd | 20 |
| OC3SN Gnh. 50 56_x_a t | Expre ssion probe set | Sense (includ es Introni c) | 6 | ENS G000 0014 0937 | CD H1 1 | 1009 | cadherin 11, type 2, OBcadherin (osteoblast) [Source:HGNC Symbol ;Acc:1750] | Chr 16 | Rev erse Stra nd | 21 |
| OC3SN Gnh. 40 32_s_a t | Expre ssion probe set | Sense (includ es Introni c) | 11 | ENS G000 0014 0937 | CD H1 1 | 1009 | cadherin 11, type 2, OBcadherin (osteoblast) [Source:HGNC Symbol ;Acc:1750] | Chr 16 | Rev erse Stra nd | 22 |
| OCHP RC. 58_ s_at | Expre ssion probe set | Sense (Fully Exonic ) | 11 | ENS G000 0014 0937 | CD H1 1 | 1009 | cadherin 11, type 2, OBcadherin (osteoblast) [Source:HGNC Symbol ;Acc:1750] | Chr 16 | Rev erse Stra nd | 23 |
| OCMX. 1718.C 1_s_ at | Expre ssion probe set | Sense (Fully Exonic) | 11 | ENS G000 00140937 | CD H1 1 | 1009 | cadherin 11, type 2, OBcadherin (osteoblast) [Source:HGNC Symbol ;Acc:1750] | Chr 16 | Rev erse Strand | 24 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OCAD A. 8067 _x_at | Expre ssion probe set | Sense (includ es Introni c) | 11 | ENS G000 0014 0937 | CD H1 1 | 1009 | cadherin 11, type 2, OBcadherin (osteoblast) [Source:HGNC Symbol ;Acc:1750] | Chr 16 | Rev erse Stra nd | 25 |
| OCRS. 383_s_ at | Expre ssion probe set | Sense (Fully Exonic ) | 11 | ENS G000 0012 3500 | CO L10 A1 | 1300 | collagen, type X, alpha 1 [Source:HGNC Symbol ;Acc:2185] | Chr 6 | Rev erse Stra nd | 26 |
| OC3SN G. 1834 947a_s _at | Expre ssion probe set | Sense (Fully Exonic ) | 11 | ENS G000 0012 3500 | CO L10 A1 | 1300 | collagen, type X, alpha 1 [Source:HGNC Symbol ;Acc:2185] | Chr 6 | Rev erse Stra nd | 27 |
| OC3P. 1561.C 1_s_ at | Expre ssion probe set | Sense (Fully Exonic ) | 11 | ENS G000 0006 0718 | CO L11 A1 | 1301 | collagen, type XI, alpha 1 [Source:HGNC Symbol ;Acc:2186] | Chr 1 | Rev erse Stra nd | 28 |
| OC3P. 6907.C 1_s_ at | Expre ssion probe set | Sense (Fully Exonic ) | 11 | ENS G000 0006 0718 | CO L11 A1 | 1301 | collagen, type XI, alpha 1 [Source:HGNC Symbol ;Acc:2186] | Chr 1 | Rev erse Stra nd | 29 |
| OC3P. 1561.C 1_x_ at | Expre ssion probe set | Sense (Fully Exonic ) | 3 | ENS G000 0006 0718 | CO L11 A1 | 1301 | collagen, type XI, alpha 1 [Source:HGNC Symbol ;Acc:2186] | Chr 1 | Rev erse Stra nd | 30 |
| OCAD A. 4133 _s_at | Expre ssion probe set | Sense (includ es Introni c) | 11 | ENS G000 0006 0718 | CO L11 A1 | 1301 | collagen, type XI, alpha 1 [Source:HGNC Symbol ;Acc:2186] | Chr 1 | Rev erse Stra nd | 31 |
| OC3SN Gnh. 16 343_x_ at | Expre ssion probe set | Sense (includ es Introni c) | 11 | ENS G000 0006 0718 | CO L11 A1 | 1301 | collagen, type XI, alpha 1 [Source:HGNC Symbol ;Acc:2186] | Chr 1 | Rev erse Stra nd | 32 |
| OC3SN Gn. 847 4-50a_x_ at | Expre ssion probe set | Sense (Fully Exonic ) | 9 | ENS G000 0016 4692 | CO L1A 2 | 1278 | collagen, type I, alpha 2 [Source:HGNC Symbol ;Acc:2198] | Chr 7 | For ward Stra nd | 33 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OCMX. 184.C1 1_s_ at | Expre ssion probe set | Sense (Fully Exonic ) | 11 | ENS G000 0016 4692 | CO L1A 2 | 1278 | collagen, type I, alpha 2 [Source:HGNC Symbol ;Acc:2198] | Chr 7 | For ward Stra nd | 34 |
| OC3SN G. 115-2502a-at | Expression probe set | Sense (includ es Introni c) | 9 | ENS G000 0016 4692 | CO L1A 2 | 1278 | collagen, type I, alpha 2 [Source:HGNC Symbol ;Acc:2198] | Chr 7 | Forward Stra nd | 35 |
| OC3SN G. 116-9169a_ s_at | Expre ssion probe set | Sense (Fully Exonic ) | 11 | ENS G000 0016 4692 | CO L1A 2 | 1278 | collagen, type I, alpha 2 [Source:HGNC Symbol ;Acc:2198] | Chr 7 | For ward Stra nd | 36 |
| OC3P. 60.CB2 _x_at | Expre ssion probe set | Sense (Fully Exonic ) | 1 | ENS G000 0016 4692 | CO L1A 2 | 1278 | collagen, type I, alpha 2 [Source:HGNC Symbol ;Acc:2198] | Chr 7 | For ward Stra nd | 37 |
| OC3P. 6454.C 1_s_ at | Expre ssion probe set | Sense (Fully Exonic ) | 11 | ENS G000 0016 4692 | CO L1A 2 | 1278 | collagen, type I, alpha 2 [Source:HGNC Symbol ;Acc:2198] | Chr 7 | For ward Stra nd | 38 |
| OC3SN G. 115-2502ax_ at | Expre ssion probe set | Sense (includ es Introni c) | 10 | ENS G000 0016 4692 | CO L1A 2 | 1278 | collagen, type I, alpha 2 [Source:HGNC Symbol ;Acc:2198] | Chr 7 | For ward Stra nd | 39 |
| OCMX. 184.C1 6_x_ at | Expre ssion probe set | Sense (Fully Exonic ) | 2 | ENS G000 0016 4692 | CO L1A 2 | 1278 | collagen, type I, alpha 2 [Source:HGNC Symbol ;Acc:2198] | Chr 7 | For ward Stra nd | 40 |
| OCHP. 173_x_ at | Expre ssion probe set | Sense (Fully Exonic ) | 11 | ENS G000 0016 4692 | CO L1A 2 | 1278 | collagen, type I, alpha 2 [Source:HGNC Symbol ;Acc:2198] | Chr 7 | For ward Stra nd | 41 |
| OC3P. 60.CB1 _x_at | Expre ssion probe set | Sense (Fully Exonic ) | 1 | ENS G000 0016 4692 | CO L1A 2 | 1278 | collagen, type I, alpha 2 [Source:HGNC Symbol ;Acc:2198] | Chr 7 | For ward Stra nd | 42 |

(continued)

| Probe Set ID | Type | Orientation | No. probes aligned | Ensembl Gene | Gene Symbol | Entrez Gene ID | Description | Chromosome | Strand | SEQ ID NO. |
|---|---|---|---|---|---|---|---|---|---|---|
| OC3SN Gn. 253 8-539a_x _at | Expre ssion probe set | Sense (Fully Exonic ) | 11 | ENS G000 0016 4692 | CO L1A 2 | 1278 | collagen, type I, alpha 2 [Source:HGNC Symbol ;Acc:2198] | Chr 7 | For ward Stra nd | 43 |
| OCMX. 184.C1 6_s_ at | Expre ssion probe set | Sense (Fully Exonic ) | 11 | ENS G000 0016 4692 | CO L1A 2 | 1278 | collagen, type alpha 2 [Source:HGNC Symbol ;Acc:2198] | Chr 7 | For ward Stra nd | 44 |
| OCAD NP. 404 8_s_at | Expre ssion probe set | Sense (includ es Introni c) | 11 | ENS G000 0016 8542 | CO L3A 1 | 1281 | collagen, type III, alpha 1 [Source:HGNC Symbol ;Acc:2201] | Chr 2 | For ward Stra nd | 45 |
| OC3P. 81.CB2 _s_at | Expre ssion probe set | Sense (Fully Exonic ) | 11 | ENS G000 0016 8542 | CO L3A 1 | 1281 | collagen, type III, alpha 1 [Source:HGNC Symbol ;Acc:2201] | Chr 2 | For ward Stra nd | 46 |
| OC3SN Gnh. 19 127_s_ at | Expre ssion probe set | Sense (Fully Exonic ) | 11 | ENS G000 0016 8542 | CO L3A 1 | 1281 | collagen, type III, alpha 1 [Source:HGNC Symbol ;Acc:2201] | Chr 2 | For ward Stra nd | 47 |
| OC3SN Gn. 121 1-6a_s_a t | Expre ssion probe set | Sense (Fully Exonic ) | 11 | ENS G000 0016 8542 | CO L3A 1 | 1281 | collagen, type III, alpha 1 [Source:HGNC Symbol ;Acc:2201] | Chr 2 | For ward Stra nd | 48 |
| OCMX. 338.C1 _at | Expre ssion probe set | Sense (includ es Introni c) | 11 | ENS G000 0020 4262 | CO L5A 2 | 1290 | collagen, type V, alpha 2 [Source:HGNC Symbol ;Acc:221 0] | Chr 2 | Rev erse Stra nd | 49 |
| OC3P. 6029. C 1_s_at | Expre ssion probe set | Sense (Fully Exonic ) | 11 | ENS G000 0020 4262 | CO L5A 2 | 1290 | collagen, type V, alpha 2 [Source:HGNC Symbol ;Acc:221 0] | Chr 2 | Rev erse Stra nd | 50 |
| OCRS2 . 8960_ s_at | Expre ssion probe set | Sense (Fully Exonic ) | 11 | ENS G000 0020 4262 | CO L5A 2 | 1290 | collagen, type V, alpha 2 [Source:HGNC Symbol ;Acc:221 0] | Chr 2 | Rev erse Stra nd | 51 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OCMX. 338.C1 _x_at | Expre ssion probe set | Sense (includ es Introni c) | 11 | ENS G000 0020 4262 | CO L5A 2 | 1290 | collagen, type V, alpha 2 [Source:HGNC Symbol ;Acc:221 0] | Chr 2 | Rev erse Stra nd | 52 |
| OC3P. 2713.C 1_s_ at | Expre ssion probe set | Sense (Fully Exonic ) | 11 | ENS G000 0020 4262 | CO L5A 2 | 1290 | collagen, type V, alpha 2 [Source:HGNC Symbol ;Acc:221 0] | Chr 2 | Rev erse Stra nd | 53 |
| OC3P. 12307. C1_x_ a t | Expre ssion probe set | Sense (includ es Introni c) | 11 | ENS G000 0020 4262 | CO L5A 2 | 1290 | collagen, type V, alpha 2 [Source:HGNC Symbol ;Acc:221 0] | Chr 2 | Rev erse Stra nd | 54 |
| OC3SN Gnh. 18 844_at | Expre ssion probe set | Sense (includ es Introni c) | 8 | ENS G000 0014 4810 | CO L8A 1 | 1295 | collagen, type VIII, alpha 1 [Source:HGNC Symbol ;Acc:2215] | Chr 3 | For ward Stra nd | 55 |
| OC3P. 1087.C 1_s_ at | Expre ssion probe set | Sense (Fully Exonic) | 11 | ENS G000 00144810 | CO L8A 1 | 1295 | collagen, type VIII, alpha 1 [Source:HGNC Symbol ;Acc:2215] | Chr 3 | For ward Strand | 56 |
| OC3P. 13652. C1_s_ a t | Expre ssion probe set | Sense (Fully Exonic ) | 11 | ENS G000 0014 4810 | CO L8A 1 | 1295 | collagen, type VIII, alpha 1 [Source:HGNC Symbol ;Acc:2215] | Chr 3 | For ward Stra nd | 57 |
| OCAD NP. 149 32_s_a t | Expre ssion probe set | Sense (includ es Introni c) | 11 | ENS G000 0014 4810 | CO L8A 1 | 1295 | collagen, type VIII, alpha 1 [Source:HGNC Symbol ;Acc:2215] | Chr 3 | For ward Stra nd | 58 |
| OC3P. 10562. C1_s_ a t | Expre ssion probe set | Sense (Fully Exonic ) | 11 | ENS G000 0014 4810 | CO L8A 1 | 1295 | collagen, type VIII, alpha 1 [Source:HGNC Symbol ;Acc:2215] | Chr 3 | For ward Stra nd | 59 |
| OC3SN Gnh. 20 566_s_ at | Expre ssion probe set | Sense (Fully Exonic ) | 11 | ENS G000 0000 5243 | CO PZ 2 | 51226 | coatomer protein complex, subunit zeta 2 [Source:HGNC Symbol ;Acc:19356] | Chr 17 | Rev erse Stra nd | 60 |

(continued)

| Probe Set ID | Type | Orientation | No. probes aligned | Ensembl Gene | Gene Symbol | Entrez Gene ID | Description | Chromosome | Strand | SEQ ID NO. |
|---|---|---|---|---|---|---|---|---|---|---|
| OCADA. 4902_s_at | Expression probe set | Sense (Fully Exonic) | 11 | ENSG00000005243 | COPZ2 | 51226 | coatomer protein complex, subunit zeta 2 [Source:HGNC Symbol ;Acc:19356] | Chr 17 | Reverse Strand | 61 |
| OC3P. 4572.C1_s_at | Expression probe set | Sense (Fully Exonic) | 11 | ENSG00000143387 | CTSK | 1513 | cathepsin K [Source:HGNC Symbol ;Acc: 2536] | Chr 1 | Reverse Strand | 62 |
| OC3SNGn. 3016-7a_s_at | Expression probe set | Sense (Fully Exonic) | 8 | ENSG00000078098 | FAP | 2191 | fibroblast activation protein, alpha [Source: HGNC Symbol ;Acc:3590] | Chr 2 | Reverse Strand | 63 |
| OCADA. 9856_x_at | Expression probe set | Sense (includes Intronic) | 7 | ENSG00000078098 | FAP | 2191 | fibroblast activation protein, alpha [Source: HGNC Symbol ;Acc:3590] | Chr 2 | Reverse Strand | 64 |
| OC3P. 8736.C1_s_at | Expression probe set | Sense (Fully Exonic) | 9 | ENSG00000078098 | FAP | 2191 | fibroblast activation protein, alpha [Source: HGNC Symbol ;Acc:3590] | Chr 2 | Reverse Strand | 65 |
| OC3SNGn. 6397-360a_at | Expression probe set | Sense (Fully Exonic) | 11 | ENSG00000115414 | FN1 | 2335 | fibronectin 1 [Source:HGNC Symbol ;Acc: 3778] | Chr 2 | Reverse Strand | 66 |
| OCMX. 493. C1_s_at | Expression probe set | Sense (Fully Exonic) | 11 | ENS G00000115414 | FN1 | 2335 | fibronectin 1 [Source:HGNC Symbol ;Acc: 3778] | Chr 2 | Reverse Strand | 67 |
| OC3SNGnh. 14004_at | Expression probe set | Sense (includes Intronic) | 7 | ENSG00000115414 | FN1 | 2335 | fibronectin 1 [Source:HGNC Symbol ;Acc: 3778] | Chr 2 | Reverse Strand | 68 |
| OCEM. 2081_at | Expression probe set | Sense (Fully Exonic) | 11 | ENSG00000115414 | FN1 | 2335 | fibronectin 1 [Source:HGNC Symbol ;Acc: 3778] | Chr 2 | Reverse Strand | 69 |

(continued)

| Probe Set ID | Type | Orientation | No. probes aligned | Ensembl Gene | Gene Symbol | Entrez Gene ID | Description | Chromosome | Strand | SEQ ID NO. |
|---|---|---|---|---|---|---|---|---|---|---|
| OCEM. 958_x_ at | Expression probe set | Sense (includes Intronic) | 11 | ENS G000 0011 5414 | FN 1 | 2335 | fibronectin 1 [Source:HGNC Symbol ;Acc: 3778] | Chr 2 | Reverse Strand | 70 |
| OCAD A. 7873 _s_at | Expression probe set | Sense (includes Intronic) | 11 | ENS G000 0011 5414 | FN 1 | 2335 | fibronectin 1 [Source:HGNC Symbol ;Acc: 3778] | Chr 2 | Reverse Strand | 71 |
| OCHP. 451_s_ at | Expression probe set | Sense (Fully Exonic ) | 11 | ENS G000 0011 5414 | FN 1 | 2335 | fibronectin 1 [Source:HGNC Symbol ;Acc: 3778] | Chr 2 | Reverse Strand | 72 |
| OCEM. 958_at | Expression probe set | Sense (includes Intronic) | 11 | ENS G000 0011 5414 | FN 1 | 2335 | fibronectin 1 [Source:HGNC Symbol ;Acc: 3778] | Chr 2 | Reverse Strand | 73 |
| OC3SN Gn. 465 0-857a_x _at | Expression probe set | Sense (includes Intronic) | 11 | ENS G000 0011 5414 | FN 1 | 2335 | fibronectin 1 [Source:HGNC Symbol ;Acc: 3778] | Chr 2 | Reverse Strand | 74 |
| OC3SN Gnh. 59 67_at | Expression probe set | Sense (includes Intronic) | 9 | ENS G000 0011 5414 | FN 1 | 2335 | fibronectin 1 [Source:HGNC Symbol ;Acc: 3778] | Chr 2 | Reverse Strand | 75 |
| OCEM. 2082_s _at | Expression probe set | Sense (Fully Exonic ) | 11 | ENS G000 0011 5414 | FN 1 | 2335 | fibronectin 1 [Source:HGNC Symbol ;Acc: 3778] | Chr 2 | Reverse Strand | 76 |
| OCAD A. 1039 _s_at | Expression probe set | Sense (Fully Exonic ) | 11 | ENS G000 0011 5414 | FN 1 | 2335 | fibronectin 1 [Source:HGNC Symbol ;Acc: 3778] | Chr 2 | Reverse Strand | 77 |
| OCEM. 2082_a t | Expression probe set | Sense (Fully Exonic ) | 11 | ENS G000 0011 5414 | FN 1 | 2335 | fibronectin 1 [Source:HGNC Symbol ;Acc: 3778] | Chr 2 | Reverse Strand | 78 |

(continued)

| Probe Set ID | Type | Orientation | No. probes aligned | Ensembl Gene | Gene Symbol | Entrez Gene ID | Description | Chromosome | Strand | SEQ ID NO. |
|---|---|---|---|---|---|---|---|---|---|---|
| OC3SNGnh.4044_x_at | Expression probe set | Sense (includes Intronic) | 8 | ENSG00000115414 | FN1 | 2335 | fibronectin 1 [Source:HGNC Symbol;Acc:3778] | Chr 2 | Reverse Strand | 79 |
| OC3P.43.CB1-415a_s_at | Expression probe set | Sense (Fully Exonic) | 11 | ENSG00000115414 | FN1 | 2335 | fibronectin 1 [Source:HGNC Symbol;Acc:3778] | Chr 2 | Reverse Strand | 80 |
| OCHP.470_s_at | Expression probe set | Sense (Fully Exonic) | 11 | ENSG00000115414 | FN1 | 2335 | fibronectin 1 [Source:HGNC Symbol;Acc:3778] | Chr 2 | Reverse Strand | 81 |
| OC3SNGnh.14004_x_at | Expression probe set | Sense (includes Intronic) | 8 | ENSG00000115414 | FN1 | 2335 | fibronectin 1 [Source:HGNC Symbol;Acc:3778] | Chr 2 | Reverse Strand | 82 |
| OCEM.2081_x_at | Expression probe set | Sense (Fully Exonic) | 11 | ENSG00000115414 | FN1 | 2335 | fibronectin 1 [Source:HGNC Symbol;Acc:3778] | Chr 2 | Reverse Strand | 83 |
| OC3SNGnh.9261_at | Expression probe set | Sense (includes Intronic) | 11 | ENSG00000115414 | FN1 | 2335 | fibronectin 1 [Source:HGNC Symbol;Acc:3778] | Chr 2 | Reverse Strand | 84 |
| OC3P.4921.C1_at | Expression probe set | Sense (Fully Exonic) | 8 | ENSG00000157240 | FZD1 | 8321 | frizzled family receptor 1 [Source:HGNC Symbol;Acc:4038] | Chr 7 | Forward Strand | 85 |
| OC3P.4921.C1-347a_s_at | Expression probe set | Sense (Fully Exonic) | 11 | ENSG00000157240 | FZD1 | 8321 | frizzled family receptor 1 [Source:HGNC Symbol;Acc:4038] | Chr 7 | Forward Strand | 86 |
| OCADNP.7579_s_at | Expression probe set | Sense (Fully Exonic) | 11 | ENSG00000157240 | FZD1 | 8321 | frizzled family receptor 1 [Source:HGNC Symbol;Acc:4038] | Chr 7 | Forward Strand | 87 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OC3P. 4921.C 1_x_ at | Expre ssion probe set | Sense (Fully Exonic) | 8 | ENS 00157240 | FZ D1 | 8321 | frizzled family receptor 1 [Source:HGNC Symbol ;Acc:4038] | Chr 7 | For ward Strand | 88 |
| OCAD A. 1231 9_s_at | Expre ssion probe set | Sense (includ es Introni c) | 11 | ENS G000 0013 1459 | GF PT 2 | 9945 | glutamine-fructose-6-phosphate transaminase 2 [Source:HGNC Symbol ;Acc: 4242] | Chr 5 | Rev erse Stra nd | 89 |
| OC3SN Gnh. 16 386_at | Expre ssion probe set | Sense (includ es Introni c) | 10 | ENS G000 0013 1459 | GF PT 2 | 9945 | glutamine-fructose-6-phosphate transaminase 2 [Source:HGNC Symbol ;Acc: 4242] | Chr 5 | Rev erse Stra nd | 90 |
| OCHP. 202_s_ at | Expre ssion probe set | Sense (Fully Exonic ) | 11 | ENS G000 0013 1459 | GF PT 2 | 9945 | glutamine-fructose-6-phosphate transaminase 2 [Source:HGNC Symbol ;Acc: 4242] | Chr 5 | Rev erse Stra nd | 91 |
| OCHP. 838_s_ at | Expre ssion probe set | Sense (Fully Exonic ) | 11 | ENS G000 0016 5474 | GJ B2 | 2706 | gap junction protein, beta 2, 26kDa [Source: HGNC Symbol ;Acc:4284] | Chr 13 | Rev erse Stra nd | 92 |
| OC3P. 7485.C 1-335a_s _at | Expre ssion probe set | Sense (Fully Exonic ) | 11 | ENS G000 0016 5474 | GJ B2 | 2706 | gap junction protein, beta 2, 26kDa [Source: HGNC Symbol ;Acc:4284] | Chr 13 | Rev erse Stra nd | 93 |
| OC3SN Gnh. 14 657_s_ at | Expre ssion probe set | Sense (Fully Exonic ) | 9 | ENS G000 0020 4866 | IGF L2 | 147920 | IGF-like family member 2 [Source:HGNC Symbol ;Acc:32929] | Chr 19 | For ward Stra nd | 94 |
| ADXBa d28_at | Almac pixella tion contro l | Sense (Fully Exonic ) | 1 | ENS G000 0020 4866 | IGF L2 | 147920 | IGF-like family member 2 [Source:HGNC Symbol ;Acc:32929] | Chr 19 | For ward Stra nd | N/A |
| ADXBa d28_at | Almac pixella tion contro l | Sense (Fully Exonic ) | 1 | ENS G000 0024 6339 | EX TL3 - AS 1 | 101929 402 | EXTL3 antisense RNA 1 [Source:HGNC Symbol ;Acc:27985] | Chr 8 | Rev erse Stra nd | N/A |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| ADXBa d28_at | Almac pixella tion contro l | Sense (Fully Exonic ) | 1 | ENS G000 0027 2994 | N/A | N/A | NOVEL lincRNA (Clone_based_vega_gene) | Chr 2 | Rev erse Stra nd | N/A |
| ADXBa d28_at | Almac pixella tion contro l | Sense (Fully Exonic ) | 1 | ENS G000 0026 1829 | N/A | N/A | NOVEL sense_overlapping (Clone_based_ vega_gene) | Chr 2 | For ward Stra nd | N/A |
| ADXBa d28_at | Almac pixella tion contro l | Sense (Fully Exonic ) | 1 | ENS G000 0015 4175 | ABI 3B P | 25890 | ABI family, member 3 (NESH) binding protein [Source:HGNC Symbol ;Acc:17265] | Chr 3 | Rev erse Stra nd | N/A |
| ADXBa d38_at | Almac pixella tion contro l | Sense (Fully Exonic ) | 1 | ENS G000 0020 4866 | IGF L2 | 147920 | IGF-like family member 2 [Source:HGNC Symbol ;Acc:32929] | Chr 19 | For ward Stra nd | N/A |
| ADXBa d38_at | Almac pixella tion contro l | Sense (Fully Exonic ) | 1 | ENS G000 0012 3700 | KC NJ2 | 3759 | potassium inwardly-rectifying channel, subfamily J, member 2 [Source:HGNC Symbol ;Acc:6263] | Chr 17 | For ward Stra nd | N/A |
| ADXBa d38_at | Almac pixella tion contro l | Sense (Fully Exonic ) | 1 | ENS G000 0012 4391 | IL1 7C | 27189 | interleukin 17C [Source:HGNC Symbol ;Acc: 5983] | Chr 16 | For ward Stra nd | N/A |
| ADXBa d38_at | Almac pixella tion contro l | Sense (Fully Exonic ) | 1 | ENS G000 0011 0723 | EX PH 5 | 23086 | exophilin 5 [Source:HGNC Symbol ;Acc: 30578] | Chr 11 | Rev erse Stra nd | N/A |
| ADXBa d38_at | Almac pixella tion contro l | Sense (Fully Exonic ) | 1 | ENS G000 0016 6856 | GP R1 82 | 11318 | G protein-coupled receptor 182 [Source: HGNC Symbol ;Acc:13708] | Chr 12 | For ward Stra nd | N/A |
| ADXBa d38_at | Almac pixella tion contro l | Sense (Fully Exonic ) | 1 | ENS G000 0013 4201 | GS TM 5 | 2949 | glutathione S-transferase mu 5 [Source: HGNC Symbol ;Acc:4637] | Chr 1 | For ward Stra nd | N/A |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| ADXBa d38_at | Almac pixella tion contro l | Sense (Fully Exonic ) | 1 | ENS G000 0002 1488 | SL C7 A9 | 11136 | solute carrier family 7 (amino acid transporter light chain, bo,+ system), member 9 [Source: HGNC Symboi;Acc:11067] | Chr 19 | Rev erse Stra nd | N/A |
| ADXBa d38_at | Almac pixella tion contro l | Sense (Fully Exonic ) | 1 | ENS G000 0016 6923 | GR EM 1 | 26585 | gremlin 1, DAN family BMP antagonist [Source:HGNC Symbol ;Acc:2001] | Chr 15 | For ward Stra nd | N/A |
| OC3SN Gnh. 36 06_s_a t | Expre ssion probe set | Sense (Fully Exonic) | 11 | ENS G000 00122641 | INH BA | 3624 | inhibin, beta A [Source:HGNC Symbol ;Acc: 6066] | Chr 7 | Rev erse Strand | 95 |
| OCEM. 2109_s _at | Expre ssion probe set | Sense (Fully Exonic ) | 11 | ENS G000 0012 2641 | INH BA | 3624 | inhibin, beta A [Source:HGNC Symbol ;Acc: 6066] | Chr 7 | Rev erse Stra nd | 96 |
| OCEM. 2108_a t | Expre ssion probe set | Sense (Fully Exonic ) | 11 | ENS G000 0012 2641 | INH BA | 3624 | inhibin, beta A [Source:HGNC Symbol ;Acc: 6066] | Chr 7 | Rev erse Stra nd | 97 |
| OCRS. 977_s_ at | Expre ssion probe set | Sense (Fully Exonic ) | 11 | ENS G000 0012 2641 | INH BA | 3624 | inhibin, beta A [Source:HGNC Symbol ;Acc: 6066] | Chr 7 | Rev erse Stra nd | 98 |
| OCEM. 2109_a t | Expre ssion probe set | Sense (Fully Exonic ) | 8 | ENS G000 0012 2641 | INH BA | 3624 | inhibin, beta A [Source:HGNC Symbol ;Acc: 6066] | Chr 7 | Rev erse Stra nd | 99 |
| OC3P. 10944. C1_s_ a t | Expre ssion probe set | Sense (Fully Exonic ) | 11 | ENS G000 0012 2641 | INH BA | 3624 | inhibin, beta A [Source:HGNC Symbol ;Acc: 6066] | Chr 7 | Rev erse Stra nd | 100 |
| OCAD NP. 761 8_s_at | Expre ssion probe set | Sense (Fully Exonic ) | 11 | ENS G000 0012 2641 | INH BA | 3624 | inhibin, beta A [Source:HGNC Symbol ;Acc: 6066] | Chr 7 | Rev erse Stra nd | 101 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OCEM. 2108_x _at | Expre ssion probe set | Sense (Fully Exonic ) | 11 | ENS G000 0012 2641 | INH BA | 3624 | inhibin, beta A [Source:HGNC Symbol ;Acc: 6066] | Chr 7 | Rev erse Stra nd | 102 |
| OC3P. 2699. C 1_s_at | Expre ssion probe set | Sense (Fully Exonic ) | 11 | ENS G000 0016 1638 | ITG A5 | 3678 | integrin, alpha 5 (fibronectin receptor, alpha polypeptide) [Source:HGNC Symbol ;Acc: 6141] | Chr 12 | Rev erse Stra nd | 103 |
| OC3SN Gnh. 12 739_x_ at | Expre ssion probe set | Sense (includ es Introni c) | 11 | ENS G000 0016 2849 | KIF 26B | 55083 | kinesin family member 26B [Source:HGNC Symbol ;Acc:25484] | Chr 1 | For ward Stra nd | 104 |
| OC3SN Gnh. 12 739_at | Expre ssion probe set | Sense (includ es Introni c) | 11 | ENS G000 0016 2849 | KIF 26B | 55083 | kinesin family member 26B [Source:HGNC Symbol ;Acc:25484] | Chr 1 | For ward Stra nd | 105 |
| OC3SN Gnh. 31 11_x_a t | Expre ssion probe set | Sense (includ es Intronic) | 9 | ENS 000 0016 2849 | KIF 26B | 55083 | kinesin family member 26B [Source:HGNC Symbol ;Acc:25484] | Chr 1 | For ward Stra nd | 106 |
| OCAD NP. 177 12_s_a t | Expre ssion probe set | Sense (Fully Exonic ) | 10 | ENS G000 0016 2849 | KIF 26B | 55083 | kinesin family member 26B [Source:HGNC Symbol ;Acc:25484] | Chr 1 | For ward Stra nd | 107 |
| OCAD A. 1652 _x_at | Expre ssion probe set | Sense (includ es Introni c) | 11 | ENS G000 0016 2849 | KIF 26B | 55083 | kinesin family member 26B [Source:HGNC Symbol ;Acc:25484] | Chr 1 | For ward Stra nd | 108 |
| OC3SN Gnh. 26 7_s_at | Expre ssion probe set | Sense (includ es Introni c) | 11 | ENS G000 0016 2849 | KIF 26B | 55083 | kinesin family member 26B [Source:HGNC Symbol ;Acc:25484] | Chr 1 | For ward Stra nd | 109 |
| OC3SN Gn. 747 1-125a_s _at | Expre ssion probe set | Sense (Fully Exonic ) | 11 | ENS G000 0016 2849 | KIF 26B | 55083 | kinesin family member 26B [Source:HGNC Symbol ;Acc:25484] | Chr 1 | For ward Stra nd | 110 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OC3SN Gnh. 96 22_x_a t | Expre ssion probe set | Sense (includ es Introni c) | 11 | ENS G000 0016 2849 | KIF 26B | 55083 | kinesin family member 26B [Source:HGNC Symbol ;Acc:25484] | Chr 1 | For ward Stra nd | 111 |
| OC3SN Gnh. 16 827_s_ at | Expre ssion probe set | Sense (includ es Introni c) | 11 | ENS G000 0016 2849 | KIF 26B | 55083 | kinesin family member 26B [Source:HGNC Symbol ;Acc:25484] | Chr 1 | For ward Stra nd | 112 |
| OCHP. 1306_s _at | Expre ssion probe set | Sense (Fully Exonic ) | 11 | ENS G000 0012 9038 | LO XL1 | 4016 | lysyl oxidase-like 1 [Source:HGNC Symbol ; Acc:6665] | Chr 15 | For ward Stra nd | 113 |
| OCHP. 1534_x _at | Expre ssion probe set | Sense (Fully Exonic ) | 11 | ENS G G000 0013 9329 | LU M | 4060 | lumican [Source:HGNC Symbol ;Acc:6724] | Chr 12 | Rev erse Stra nd | 114 |
| OCHP. 1534_s _at | Expre ssion probe set | Sense (Fully Exonic ) | 11 | ENS G000 0013 9329 | LU M | 4060 | lumican [Source:HGNC Symbol ;Acc:6724] | Chr 12 | Rev erse Stra nd | 115 |
| OC3SN Gnh. 34 22_s_a t | Expre ssion probe set | Sense (Fully Exonic ) | 11 | ENS G000 0022 1502 | MI R1 245 A | 100302 219 /// 100616 324 | microRNA 1245a [Source:HGNC Symbol ; Acc:35311] | Chr 2 | For ward Stra nd | 116 |
| OCHP. 983_s_ at | Expression probe set | Sense (Fully Exonic ) | 11 | ENS 000 0013 7745 | MMP13 | 4322 | matrix metallopeptidase 13 (collagenase 3) [Source:HGNC Symbol ;Acc:7159] | Chr 11 | Reverse Stra nd | 117 |
| OCHP. 228_s_ at | Expre ssion probe set | Sense (Fully Exonic ) | 11 | ENS G000 0015 7227 | MM P14 | 4323 | matrix metallopeptidase 14 (membrane-inserted) [Source:HGNC Symbol ;Acc:7160] | Chr 14 | For ward Stra nd | 118 |
| OC3P. 4123.C 1_x_ at | Expre ssion probe set | Sense (Fully Exonic ) | 9 | ENS G000 0015 7227 | MM P14 | 4323 | matrix metallopeptidase 14 (membrane-inserted) [Source:HGNC Symbol ;Acc:7160] | Chr 14 | For ward Stra nd | 119 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OC3P. 4123.C 1_s_ at | Expre ssion probe set | Sense (Fully Exonic ) | 11 | ENS G000 0015 7227 | MM P14 | 4323 | matrix metallopeptidase 14 (membrane-inserted) [Source:HGNC Symbol ;Acc:7160] | Chr 14 | For ward Stra nd | 120 |
| OC3P. 1163.C 3_s_ at | Expre ssion probe set | Sense (Fully Exonic ) | 11 | ENS G000 0008 7245 | MM P2 | 4313 | matrix metallopeptidase 2 (gelatinase A, 72kDa gelatinase, 72kDa type IV collagenase) [Source:HGNC Symbol ;Acc: 7166] | Chr 16 | For ward Stra nd | 121 |
| OCHP. 374_s_ at | Expre ssion probe set | Sense (Fully Exonic ) | 11 | ENS G000 0008 7245 | MM P2 | 4313 | matrix metallopeptidase 2 (gelatinase A, 72kDa gelatinase, 72kDa type IV collagenase) [Source:HGNC Symbol ;Acc: 7166] | Chr 16 | For ward Stra nd | 122 |
| OCAD NP. 725 1_s_at | Expre ssion probe set | Sense (Fully Exonic ) | 11 | ENS 000 0008 7245 | MM P2 | 4313 | matrix metallopeptidase 2 (gelatinase A, 72kDa gelatinase, 72kDa type IV collagenase) [Source:HGNC Symbol ;Acc: 7166] | Chr 16 | For ward Stra nd | 123 |
| OCAD A. 2310 _s_at | Expre ssion probe set | Sense (Fully Exonic ) | 11 | ENS G000 0008 7245 | MM P2 | 4313 | matrix metallopeptidase 2 (gelatinase A, 72kDa gelatinase, 72kDa type IV collagenase) [Source:HGNC Symbol ;Acc: 7166] | Chr 16 | For ward Stra nd | 124 |
| OCAD A. 3580 _s_at | Expre ssion probe set | Sense (Fully Exonic ) | 11 | ENS G000 0007 2952 | MR VI1 | 10335 | murine retrovirus integration site 1 homolog [Source:HGNC Symbol ;Acc:7237] | Chr 11 | Rev erse Stra nd | 125 |
| OC3P. 1058.C 1_s_ at | Expre ssion probe set | Sense (Fully Exonic ) | 11 | ENS G000 0007 2952 | MR VI1 | 10335 | murine retrovirus integration site 1 homolog [Source:HGNC Symbol ;Acc:7237] | Chr 11 | Rev erse Stra nd | 126 |
| OC3P. 13126. C1_s_ a t | Expre ssion probe set | Sense (Fully Exonic ) | 11 | ENS G000 0007 2952 | MR VI1 | 10335 | murine retrovirus integration site 1 homolog [Source:HGNC Symbol ;Acc:7237] | Chr 11 | Rev erse Stra nd | 127 |

(continued)

| Probe Set ID | Type | Orientation | No. probes aligned | Ensembl Gene | Gene Symbol | Entrez Gene ID | Description | Chromosome | Strand | SEQ ID NO. |
|---|---|---|---|---|---|---|---|---|---|---|
| OCAD NP. 102 37_s_a t | Expre ssion probe set | Sense (Fully Exonic ) | 11 | ENS 000 0007 2952 | MR VI1 | 10335 | murine retrovirus integration site 1 homolog [Source:HGNC Symbol ;Acc:7237] | Chr 11 | Rev erse Stra nd | 128 |
| OCAD NP. 584 9_s_at | Expre ssion probe set | Sense (Fully Exonic ) | 9 | ENS 000 0014 5506 | NK D2 | 85409 | naked cuticle homolog 2 (Drosophila) [Source:HGNC Symbol ;Acc:17046] | Chr 5 | For ward Stra nd | 129 |
| OCAD NP. 655 5_s_at | Expre ssion probe set | Sense (Fully Exonic ) | 11 | ENS 000 0018 2667 | NT M | 50863 | neurotrimin [Source:HGNC Symbol ;Acc: 17941] | Chr 11 | For ward Stra nd | 130 |
| OCAD A. 3177 _s_at | Expre ssion probe set | Sense (Fully Exonic ) | 11 | ENS 000 0018 2667 | NT M | 50863 | neurotrimin [Source:HGNC Symbol ;Acc: 17941] | Chr 11 | For ward Stra nd | 131 |
| OC3SN G. 2346 440a_s _at | Expre ssion probe set | Sense (Fully Exonic ) | 11 | ENS 000 0018 2667 | NT M | 50863 | neurotrimin [Source:HGNC Symbol ;Acc: 17941] | Chr 11 | For ward Stra nd | 132 |
| OC3SN Gnh. 75 67_x_a t | Expre ssion probe set | Sense (includ es Introni c) | 1 | ENS 000 0018 2667 | NT M | 50863 | neurotrimin [Source:HGNC Symbol ;Acc: 17941] | Chr 11 | For ward Stra nd | 133 |
| OC3SN Gnh. 75 67_x_a t | Expre ssion probe set | Sense (includ es Introni c) | 1 | ENS 000 0015 1065 | DC P1 B | 196513 | decapping mRNA 1 B [Source:HGNC Symbol ;Acc:24451] | Chr 12 | Rev erse Stra nd | 133 |
| OCHP. 739_s_ at | Expre ssion probe set | Sense (Fully Exonic ) | 11 | ENS 000 0012 2861 | PL AU | 5328 | plasminogen activator, urokinase [Source: HGNC Symbol ;Acc:9052] | Chr 10 | For ward Stra nd | 134 |
| OCAD NP. 865 3_s_at | Expre ssion probe set | Sense (Fully Exonic ) | 10 | ENS 000 0012 2861 | PL AU | 5328 | plasminogen activator, urokinase [Source: HGNC Symbol ;Acc:9052] | Chr 10 | For ward Stra nd | 135 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| ADXGo od72_a t | Almac pixella tion contro l | Sense (Fully Exonic ) | 1 | ENS G000 0015 8109 | TP RG 1L | 127262 | tumor protein p63 regulated 1-like [Source: HGNC Symbol ;Acc:27007] | Chr 1 | For ward Stra nd | N/A |
| ADXGo od72_a t | Almac pixella tion control | Sense (Fully Exonic ) | 1 | ENS 000 0015 2700 | SA R1 B | 51128 | SAR1 homolog B (S. cerevisiae) [Source: HGNC Symbol ;Acc:10535] | Chr 5 | Rev erse Stra nd | N/A |
| ADXGo od72_a t | Almac pixella tion contro l | Sense (Fully Exonic ) | 1 | ENS 000 0015 8286 | RN F20 7 | 388591 | ring finger protein 207 [Source:HGNC Symbol ;Acc:32947] | Chr 1 | For ward Stra nd | N/A |
| ADXGo od72_a t | Almac pixella tion contro l | Sense (Fully Exonic ) | 1 | ENS 000 0010 1019 | UQ CC 1 | 55245 | ubiquinol-cytochrome c reductase complex assembly factor 1 [Source:HGNC Symbol ; Acc:15891] | Chr 20 | Rev erse Stra nd | N/A |
| ADXGo od72_a t | Almac pixella tion contro l | Sense (Fully Exonic ) | 1 | ENS 000 0020 5336 | GP R5 6 | 9289 | G protein-coupled receptor 56 [Source: HGNC Symbol ;Acc:4512] | Chr 16 | For ward Stra nd | N/A |
| ADXGo od72_a t | Almac pixella tion contro l | Sense (Fully Exonic ) | 1 | ENS 000 0010 9099 | PM P22 | 5376 | peripheral myelin protein 22 [Source:HGNC Symbol ;Acc:9118] | Chr 17 | Rev erse Stra nd | N/A |
| ADXGo od72_a t | Almac pixella tion contro l | Sense (Fully Exonic ) | 1 | ENS 000 0014 3228 | NU F2 | 83540 | NUF2, NDC80 kinetochore complex component [Source:HGNC Symbol ;Acc: 14621] | Chr 1 | For ward Stra nd | N/A |
| ADXGo od72_a t | Almac pixella tion contro l | Sense (Fully Exonic ) | 1 | ENS 000 0017 5279 | API TD 1 | 378708/// 100526 739 | apoptosis-inducing, TAF9-like domain 1 [Source:HGNC Symbol ;Acc:23163] | Chr 1 | For ward Stra nd | N/A |
| ADXGo od72_a t | Almac pixella tion contro l | Sense (Fully Exonic ) | 1 | ENS G000 0021 3024 | NU P62 | 23636 | nucleoporin 62kDa [Source:HGNC Symbol ; Acc:8066] | Chr 19 | Rev erse Stra nd | N/A |

(continued)

| Probe Set ID | Type | Orientation | No. probes aligned | Ensembl Gene | Gene Symbol | Entrez Gene ID | Description | Chromosome | Strand | SEQ ID NO. |
|---|---|---|---|---|---|---|---|---|---|---|
| ADXGood72_at | Almac pixellation control | Sense (Fully Exonic) | 1 | ENSG00000180008 | SOCS4 | 122809 | suppressor of cytokine signaling 4 [Source: HGNC Symbol ;Acc:19392] | Chr 14 | Forward Strand | N/A |
| ADXGood72_at | Almac pixellation control | Sense (Fully Exonic) | 1 | ENSG00000163703 | CRELD1 | 78987 | cysteine-rich with EGF-like domains 1 [Source:HGNC Symbol ;Acc:14630] | Chr 3 | Forward Strand | N/A |
| OCADA.9170_s_at | Expression probe set | Sense (includes Intronic) | 11 | ENSG00000109099 | PMP22 | 5376 | peripheral myelin protein 22 [Source:HGNC Symbol ;Acc:9118] | Chr 17 | Reverse Strand | 136 |
| OC3P.10622.C1_s_at | Expression probe set | Sense (Fully Exonic) | 11 | ENSG00000109099 | PMP22 | 5376 | peripheral myelin protein 22 [Source:HGNC Symbol ;Acc:9118] | Chr 17 | Reverse Strand | 137 |
| OC3SNGnh.8944_s_at | Expression probe set | Sense (includes Intronic) | 11 | ENSG00000109099 | PMP22 | 5376 | peripheral myelin protein 22 [Source:HGNC Symbol ;Acc:9118] | Chr 17 | Reverse Strand | 138 |
| OC3SNG.457122a_x_at | Expression probe set | Sense (Fully Exonic) | 11 | ENSG00000106628 | POLD2 | 5425 | polymerase (DNA directed), delta 2, accessory subunit [Source:HGNC Symbol ; Acc:9176] | Chr 7 | Reverse Strand | 139 |
| OCEM.1126_s_at | Expression probe set | Sense (Fully Exonic) | 11 | ENSG00000106628 | POLD2 | 5425 | polymerase (DNA directed), delta 2, accessory subunit [Source:HGNC Symbol ; Acc:9176] | Chr 7 | Reverse Strand | 140 |
| ADXGood4_at | Almac pixellation control | Sense (Fully Exonic) | 1 | ENSG00000150907 | FOXO1 | 2308 | forkhead box O1 [Source:HGNC Symbol ; Acc:3819] | Chr 13 | Reverse Strand | N/A |
| ADXGood4_at | Almac pixellation control | Sense (Fully Exonic) | 1 | ENSH00000122126 | OCRL | 4952 | oculocerebrorenal syndrome of Lowe [Source:HGNC Symbol;Acc:8108] | Chr X | Forward Strand | N/A |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| ADXGo od4_at | Almac pixella tion contro l | Sense (Fully Exonic ) | 1 | ENS 000 0010 6628 | PO LD 2 | 5425 | polymerase (DNA directed), delta 2, accessory subunit [Source:HGNC Symbol ; Acc:9176] | Chr 7 | Rev erse Stra nd | N/A |
| ADXGo od4_at | Almac pixella tion contro l | Sense (Fully Exonic ) | 1 | ENS G000 0011 7461 | PIK 3R 3 | 8503 | phosphoinositide-3-kinase, regulatory subunit 3 (gamma) [Source:HGNC Symboi; Acc:8981] | Chr 1 | Rev erse Stra nd | N/A |
| ADXGo od4_at | Almac pixella tion contro l | Sense (Fully Exonic ) | 1 | ENS 000 0019 7747 | S10 0A1 0 | 6281 | S100 calcium binding protein A10 [Source: HGNC Symbol ;Acc:10487] | Chr 1 | Rev erse Stra nd | N/A |
| ADXGo od4_at | Almac pixellation contro l | Sense (Fully Exonic ) | 1 | ENS G0000023 1925 | TA PBP | 6892 | TAP binding protein (tapasin) [Source:HGNC Symbol ;Acc:11566] | Chr 6 | Rev erse Stra nd | N/A |
| ADXGo od4_at | Almac pixella tion contro l | Sense (Fully Exonic ) | 1 | ENS G000 0017 6783 | RU FY 1 | 80230 | RUN and FYVE domain containing 1 [Source: HGNC Symbol ;Acc:19760] | Chr 5 | For ward Stra nd | N/A |
| ADXGo od4_at | Almac pixella tion contro l | Sense (Fully Exonic ) | 1 | ENS G000 0014 4136 | SL C2 0A1 | 6574 | solute carrier family 20 (phosphate transporter), member 1 [Source:HGNC Symbol ;Acc:10946] | Chr 2 | For ward Stra nd | N/A |
| ADXGo od4_at | Almac pixella tion contro l | Sense (Fully Exonic ) | 1 | ENS G000 0016 2607 | US P1 | 7398 | ubiquitin specific peptidase 1 [Source:HGNC Symbol ;Acc:12607] | Chr 1 | For ward Stra nd | N/A |
| ADXGo od4_at | Almac pixella tion contro l | Sense (Fully Exonic ) | 1 | ENS G000 0018 7837 | HIS T1 H1 C | 3006 | histone cluster 1, H1c [Source:HGNC Symbol;Acc:4716] | Chr 6 | Rev erse Stra nd | N/A |
| OC3SN Gn. 890 5a_s_a t | Expre ssion probe set | Sense (Fully Exonic ) | 11 | ENS G000 0010 6628 | PO LD 2 | 5425 | polymerase (DNA directed), delta 2, accessory subunit [Source:HGNC Symbol ; Acc:9176] | Chr 7 | Rev erse Stra nd | 141 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OCAD A. 7987 _s_at | Expre ssion probe set | Sense (Fully Exonic ) | 11 | ENS G000 0013 3110 | PO ST N | 10631 | periostin, osteoblast specific factor [Source: HGNC Symbol ;Acc:16953] | Chr 13 | Rev erse Stra nd | 142 |
| OC3SN Gnh. 57 24_at | Expre ssion probe set | Sense (Fully Exonic ) | 11 | ENS G000 0013 3110 | PO ST N | 10631 | periostin, osteoblast specific factor [Source: HGNC Symbol ;Acc:16953] | Chr 13 | Rev erse Stra nd | 143 |
| OCHP. 402_s_ at | Expre ssion probe set | Sense (Fully Exonic ) | 11 | ENS G000 0013 3110 | PO ST N | 10631 | periostin, osteoblast specific factor [Source: HGNC Symbol ;Acc:16953] | Chr 13 | Rev erse Stra nd | 144 |
| OC3P. 1013.C 1_x_ at | Expre ssion probe set | Sense (Fully Exonic ) | 11 | ENS G000 0013 3110 | PO ST N | 10631 | periostin, osteoblast specific factor [Source: HGNC Symbol ;Acc:16953] | Chr 13 | Rev erse Stra nd | 145 |
| OC3P. 1013.C 2_x_ at | Expre ssion probe set | Sense (Fully Exonic ) | 6 | ENS G000 0013 3110 | PO ST N | 10631 | periostin, osteoblast specific factor [Source: HGNC Symbol ;Acc:16953] | Chr 13 | Rev erse Stra nd | 146 |
| OCAD NP. 115 85_s_a t | Expre ssion probe set | Sense (includ es Introni c) | 11 | ENS G000 0013 3110 | PO ST N | 10631 | periostin, osteoblast specific factor [Source: HGNC Symbol ;Acc:16953] | Chr 13 | Rev erse Stra nd | 147 |
| OC3P. 1013.C 1_s_ at | Expre ssion probe set | Sense (Fully Exonic ) | 11 | ENS G000 0013 3110 | PO ST N | 10631 | periostin, osteoblast specific factor [Source: HGNC Symbol ;Acc:16953] | Chr 13 | Rev erse Stra nd | 148 |
| OC3SN Gnh. 57 24_x_a t | Expre ssion probe set | Sense (Fully Exonic ) | 11 | ENS G000 0013 3110 | PO ST N | 10631 | periostin, osteoblast specific factor [Source: HGNC Symbol ;Acc:16953] | Chr 13 | Rev erse Stra nd | 149 |
| OC3P. 8262.C 1_s_ at | Expre ssion probe set | Sense (Fully Exonic ) | 11 | ENS G000 0016 8461 | RA B31 | 11031 | RAB31, member RAS oncogene family [Source:HGNC Symbol;Acc:9771] | Chr 18 | For ward Stra nd | 150 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OC3SN Gnh. 17 870_s_ at | Expre ssion probe set | Sense (includ es Introni c) | 11 | ENS 000 0016 8461 | RA B31 | 11031 | RAB31, member RAS oncogene family [Source:HGNC Symbol;Acc:9771] | Chr 18 | For ward Stra nd | 151 |
| OC3P. 11285. C1_s_ a t | Expre ssion probe set | Sense (Fully Exonic ) | 11 | ENS G000 0016 8461 | RA B31 | 11031 | RAB31, member RAS oncogene family [Source:HGNC Symbol;Acc:9771] | Chr 18 | For ward Stra nd | 152 |
| OCHP. 1160_s _at | Expre ssion probe set | Sense (Fully Exonic ) | 11 | ENS G000 0016 8461 | RA B31 | 11031 | RAB31, member RAS oncogene family [Source:HGNC Symbol;Acc:9771] | Chr 18 | For ward Stra nd | 153 |
| OCMX. 11222. C1_at | Expre ssion probe set | Sense (includ es Introni c) | 10 | ENS G000 0016 8461 | RA B31 | 11031 | RAB31, member RAS oncogene family [Source:HGNC Symbol;Acc:9771] | Chr 18 | For ward Stra nd | 154 |
| OC3SN Gnh. 14 334_x_ at | Expre ssion probe set | Sense (includ es Introni c) | 11 | ENS G000 0012 4813 | RU NX 2 | 860 | runt-related transcription factor 2 [Source: HGNC SymboLi;Acc:10472] | Chr 6 | For ward Stra nd | 155 |
| OCAD A. 8000 _s_at | Expre ssion probe set | Sense (Fully Exonic ) | 11 | ENS G000 0012 4813 | RU NX 2 | 860 | runt-related transcription factor 2 [Source: HGNC SymboLi;Acc:10472] | Chr 6 | For ward Stra nd | 156 |
| OCAD NP. 631 5_s_at | Expre ssion probe set | Sense (Fully Exonic ) | 11 | ENS G000 0012 4813 | RU NX 2 | 860 | runt-related transcription factor 2 [Source: HGNC SymboLi;Acc:10472] | Chr 6 | For ward Stra nd | 157 |
| OCMX. 1543.C 1_s_ at | Expre ssion probe set | Sense (Fully Exonic ) | 9 | ENS 000 0012 4813 | RU NX 2 | 860 | runt-related transcription factor 2 [Source: HGNC SymboLi;Acc:10472] | Chr 6 | For ward Stra nd | 158 |
| OCHP. 1038_s _at | Expre ssion probe set | Sense (Fully Exonic ) | 11 | ENS G000 0012 4813 | RU NX 2 | 860 | runt-related transcription factor 2 [Source: HGNC SymboLi;Acc:10472] | Chr 6 | For ward Stra nd | 159 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OCHP. 781_s_ at | Expre ssion probe set | Sense (Fully Exonic ) | 11 | ENS G000 0013 2386 | SE RPI NF 1 | 5176 | serpin peptidase inhibitor, clade F (alpha-2 antiplasmin, pigment epithelium derived factor), member 1 [Source:HGNC Symbol ; Acc:8824] | Chr 17 | For ward Stra nd | 160 |
| ADXStr ong15_ at | Almac pixella tion contro l | Sense (Fully Exonic ) | 1 | ENS G000 0010 0299 | AR SA | 410 | arylsulfatase A [Source:HGNC Symbol;Acc: 713] | Chr 22 | Rev erse Stra nd | N/A |
| ADXStr ong15_ at | Almac pixella tion contro l | Sense (Fully Exonic ) | 1 | ENS G000 0014 9480 | MT A2 | 9219 | metastasis associated 1 family, member 2 [Source:HGNC SymboL;Acc:7411] | Chr 11 | Rev erse Stra nd | N/A |
| ADXStr ong15_ at | Almac pixella tion contro l | Sense (Fully Exonic ) | 1 | ENS G000 0023 3664 | ND UF S5 P3 | N/A | NADH dehydrogenase (ubiquinone) Fe-S protein 5, 15kDa (NADH-coenzyme Q reductase) pseudogene 3 [Source:HGNC Symbol ;Acc:44041] | Chr 1 | For ward Stra nd | N/A |
| ADXStr ong15_ at | Almac pixella tion contro l | Sense (Fully Exonic ) | 1 | ENS G000 0016 8653 | ND UF S5 | 4725 | NADH dehydrogenase (ubiquinone) Fe-S protein 5, 15kDa (NADH-coenzyme Q reductase) [Source:HGNC Symbol ;Acc: 7712] | Chr 1 | For ward Stra nd | N/A |
| ADXStr ong15_ at | Almac pixella tion contro l | Sense (Fully Exonic ) | 1 | ENS G000 0006 0688 | SN RN P40 | 9410 | small nuclear ribonucleoprotein 40kDa (U5) [Source:HGNC Symbol ;Acc:30857] | Chr 1 | Rev erse Stra nd | N/A |
| ADXStr ong15_ at | Almac pixella tion contro l | Sense (Fully Exonic ) | 1 | ENS G000 0007 9805 | DN M2 | 1785 | dynamin 2 [Source:HGNC Symbol ;Acc: 2974] | Chr 19 | For ward Stra nd | N/A |
| ADXStr ong15_ at | Almac pixella tion control | Sense (Fully Exonic ) | 1 | ENS G000 0013 3639 | BT G1 | 694 | B-cell translocation gene 1, anti-proliferative [Source:HGNC Symbol;Acc:1130] | Chr 12 | Rev erse Stra nd | N/A |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| ADXStr ong15_ at | Almac pixella tion contro l | Sense (Fully Exonic ) | 1 | ENS 000 0019 6605 | ZN F84 6 | 162993/// 100505 555 | zinc finger protein 846 [Source:HGNC Symbol ;Acc:27260] | Chr 19 | Rev erse Stra nd | N/A |
| ADXStr ong15_ at | Almac pixella tion contro l | Sense (Fully Exonic ) | 1 | ENS G000 0013 2386 | SE RPI NF 1 | 5176 | serpin peptidase inhibitor, clade F (alpha-2 antiplasmin, pigment epithelium derived factor), member 1 [Source:HGNC Symbol ; Acc:8824] | Chr 17 | For ward Stra nd | N/A |
| ADXStr ong15_ at | Almac pixella tion contro l | Sense (Fully Exonic ) | 1 | ENS G000 0016 2896 | PIG R | 5284 | polymeric immunoglobulin receptor [Source: HGNC Symbol ;Acc:8968] | Chr 1 | Rev erse Stra nd | N/A |
| ADXStr ong15_ at | Almac pixella tion contro l | Sense (Fully Exonic ) | 1 | ENS G000 0023 0671 | ND UF S5 P5 | N/A | NADH dehydrogenase (ubiquinone) Fe-S protein 5, 15kDa (NADH-coenzyme Q reductase) pseudogene 5 [Source:HGNC Symbol ;Acc:44043] | Chr 4 | Rev erse Stra nd | N/A |
| ADXStr ong15_ at | Almac pixella tion contro l | Sense (Fully Exonic ) | 1 | ENS G000 0009 2820 | EZ R | 7430 | ezrin [Source:HGNC Symbol ;Acc:12691] | Chr 6 | Rev erse Stra nd | N/A |
| OCEM. 1960_a t | Expre ssion probe set | Sense (Fully Exonic ) | 11 | ENS G000 0013 2386 | SE RPI NF 1 | 5176 | serpin peptidase inhibitor, clade F (alpha-2 antiplasmin, pigment epithelium derived factor), member 1 [Source:HGNC Symbol ; Acc:8824] | Chr 17 | For ward Stra nd | 161 |
| ADXStr ong8_a t | Almac pixella tion contro l | Sense (Fully Exonic ) | 1 | ENS G000 0025 4612 | N/A | N/A | KNOWN pseudogene (Clone_based_vega_ gene) | Chr 11 | Rev erse Stra nd | N/A |
| ADXStr ong8_a t | Almac pixella tion contro l | Sense (Fully Exonic ) | 1 | ENS G000 0010 5993 | DN AJ B6 | 10049 | DnaJ (Hsp40) homolog, subfamily B, member 6 [Source:HGNC Symbol ;Acc:14888] | Chr 7 | For ward Stra nd | N/A |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| ADXStr ong8_a t | Almac pixella tion contro l | Sense (Fully Exonic ) | 1 | ENS G000 0000 5889 | ZF X | 7543 | zinc finger protein, X-linked [Source:HGNC Symbol ;Acc:12869] | Chr X | For ward Stra nd | N/A |
| ADXStr | Almac | Sense | 1 | ENS | DN | 3300 | DnaJ (Hsp40) homolog, | Chr 2 | For | N/A |
| ong8_a t | pixella tion contro l | (Fully Exonic ) | | GOOO 0013 5924 | AJ B2 | | subfamily B, member 2 [Source:HGNC Symbol ;Acc:5228] | | ward Stra nd | |
| ADXStr ong8_a t | Almac pixella tion contro l | Sense (Fully Exonic ) | 1 | ENS GOOO 0010 6105 | GA RS | 2617 | glycyl-tRNA synthetase [Source:HGNC Symbol ;Acc:4162] | Chr 7 | For ward Stra nd | N/A |
| ADXStr ong8_a t | Almac pixella tion contro l | Sense (Fully Exonic ) | 1 | ENS GOOO 0014 3207 | RF WD 2 | 64326 | ring finger and WD repeat domain 2, E3 ubiquitin protein ligase [Source:HGNC Symbol;Acc:17440] | Chr 1 | Rev erse Stra nd | N/A |
| ADXStr ong8_a t | Almac pixella tion contro l | Sense (Fully Exonic ) | 1 | ENS GOOO 0015 7601 | MX 1 | 4599 | myxovirus (influenza virus) resistance 1, interferoninducible protein p78 (mouse) [Source:HGNC Symbol ;Acc:7532] | Chr 21 | For ward Stra nd | N/A |
| ADXStr ong8_a t | Almac pixella tion contro l | Sense (Fully Exonic ) | 1 | ENS GOOO 0013 2386 | SE RPI NF 1 | 5176 | serpin peptidase inhibitor, clade F (alpha-2 antiplasmin, pigment epithelium derived factor), member 1 [Source:HGNC Symbol ; Acc:8824] | Chr 17 | For ward Stra nd | N/A |
| ADXStr ong8_a t | Almac pixella tion contro l | Sense (Fully Exonic ) | 1 | ENS GOOO 0026 9242 | N/A | N/A | NOVEL protein_coding (Clone_based_ vega_gene) | Chr 19 | Rev erse Stra nd | N/A |
| ADXStr ong8_a t | Almac pixella tion contro l | Sense (Fully Exonic ) | 1 | ENS GOOO 0012 4155 | PIG T | 51604 | phosphatidylinositol glycan anchor biosynthesis, class T [Source:HGNC Symbol ;Acc:14938] | Chr 20 | For ward Stra nd | N/A |
| ADXStr ong8_a t | Almac pixella tion contro l | Sense (Fully Exonic ) | 1 | ENS GOOO 0017 2236 | TP SA B1 | 7177 | tryptase alpha/beta 1 [Source:HGNC Symbol ;Acc:12019] | Chr 16 | For ward Stra nd | N/A |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| ADXStr ong8_a t | Almac pixella tion contro l | Sense (Fully Exonic ) | 1 | ENS GOOO 0014 5494 | ND UF S6 | 4726 | NADH dehydrogenase (ubiquinone) Fe-S protein 6, 13kDa (NADH-coenzyme Q reductase) [Source:HGNC Symbol ;Acc: 7713] | Chr 5 | For ward Stra nd | N/A |
| ADXStr ong8_a t | Almac pixella tion control | Sense (Fully Exonic ) | 1 | ENS GOOO 0019 7253 | TP SB 2 | 64499 | tryptase beta 2 (gene/pseudogene) [Source: HGNC Symbol ;Acc:14120] | Chr 16 | Rev erse Stra nd | N/A |
| ADXStr ong8_a t | Almac pixella tion contro l | Sense (Fully Exonic ) | 1 | ENS 000 0010 4774 | MA N2 B1 | 4125 | mannosidase, alpha, class 2B, member 1 [Source:HGNC Symbol ;Acc:6826] | Chr 19 | Rev erse Stra nd | N/A |
| OC3SN Gn. 251 21a_s_ at | Expre ssion probe set | Sense (Fully Exonic ) | 11 | ENS G000 0014 5423 | SF RP 2 | 6423 | secreted frizzled-related protein 2 [Source: HGNC Symbol ;Acc:10777] | Chr 4 | Rev erse Stra nd | 162 |
| OC3P. 13621. C1_s_ a t | Expre ssion probe set | Sense (Fully Exonic ) | 11 | ENS G000 0014 5423 | SF RP 2 | 6423 | secreted frizzled-related protein 2 [Source: HGNC Symbol ;Acc:10777] | Chr 4 | Rev erse Stra nd | 163 |
| OCHP. 677_s_ at | Expre ssion probe set | Sense (Fully Exonic ) | 11 | ENS G000 0018 6340 | TH BS 2 | 7058 | thrombospondin 2 [Source:HGNC Symbol; Acc:11786] | Chr 6 | Rev erse Stra nd | 164 |
| OC3P. 4296.C 2_s_ at | Expre ssion probe set | Sense (Fully Exonic ) | 11 | ENS G000 0018 6340 | TH BS 2 | 7058 | thrombospondin 2 [Source:HGNC Symbol ; Acc:11786] | Chr 6 | Rev erse Stra nd | 165 |
| OC3SN Gnh. 14 530_s_ at | Expre ssion probe set | Sense (includ es Introni c) | 11 | ENS G000 0018 6340 | TH BS 2 | 7058 | thrombospondin 2 [Source:HGNC Symbol ; Acc:11786] | Chr 6 | Rev erse Stra nd | 166 |
| OC3SN Gnh. 14 530_at | Expre ssion probe set | Sense (includ es Introni c) | 11 | ENS G000 0018 6340 | TH BS 2 | 7058 | thrombospondin 2 [Source:HGNC Symbol ; Acc:11786] | Chr 6 | Rev erse Stra nd | 167 |

(continued)

| Probe Set ID | Type | Orientation | No. probes aligned | Ensembl Gene | Gene Symbol | Entrez Gene ID | Description | Chromosome | Strand | SEQ ID NO. |
|---|---|---|---|---|---|---|---|---|---|---|
| OC3P. 4296.C 1-409a_s _at | Expre ssion probe set | Sense (Fully Exonic ) | 11 | ENS G000 0018 6340 | TH BS 2 | 7058 | thrombospondin 2 [Source:HGNC Symbol ; Acc:11786] | Chr 6 | Rev erse Stra nd | 168 |
| OC3SN Gn. 187 3-1656a_ s_at | Expre ssion probe set | Sense (Fully Exonic ) | 11 | ENS G000 0018 6340 | TH BS 2 | 7058 | thrombospondin 2 [Source:HGNC Symbol ; Acc:11786] | Chr 6 | Rev erse Stra nd | 169 |
| OCAD NP. 130 17_s_a t | Expre ssion probe set | Sense (includ es Introni c) | 11 | ENS G000 0010 0234 | TIM P3 | 7078 | TIMP metallopeptidase inhibitor 3 [Source: HGNC Symbol;Acc:11822] | Chr 22 | For ward Stra nd | 170 |
| OCAD A. 9324 _s_at | Expre ssion probe set | Sense (Fully Exonic ) | 11 | ENS 000 0010 0234 | TIM P3 | 7078 | TIMP metallopeptidase inhibitor 3 [Source: HGNC Symbol;Acc:11822] | Chr 22 | For ward Stra nd | 171 |
| OCHP. 1200_s _at | Expre ssion probe set | Sense (Fully Exonic ) | 11 | ENS G000 0010 0234 | TIM P3 | 7078 | TIMP metallopeptidase inhibitor 3 [Source: HGNC Symbol;Acc:11822] | Chr 22 | For ward Stra nd | 172 |
| ADXGo od73_a t | Almac pixella tion contro l | Sense (Fully Exonic ) | 1 | ENS G000 0014 4118 | RA LB | 5899 | v-ral simian leukemia viral oncogene homolog B [Source:HGNC Symbol ;Acc: 9840] | Chr 2 | For ward Stra nd | N/A |
| ADXGo od73_a t | Almac pixella tion contro l | Sense (Fully Exonic ) | 1 | ENS G000 0018 4500 | PR OS 1 | 5627 | protein S (alpha) [Source:HGNC Symbol ; Acc:9456] | Chr 3 | Rev erse Stra nd | N/A |
| ADXGo od73_a t | Almac pixella tion contro l | Sense (Fully Exonic ) | 1 | ENS G000 0013 8031 | AD CY 3 | 109 | adenylate cyclase 3 [Source:HGNC Symbol ; Acc:234] | Chr 2 | Rev erse Stra nd | N/A |
| ADXGo od73_a t | Almac pixella tion contro l | Sense (Fully Exonic ) | 1 | ENS G000 0010 0234 | TIM P3 | 7078 | TIMP metallopeptidase inhibitor 3 [Source: HGNC Symbol;Acc:11822] | Chr 22 | For ward Stra nd | N/A |

(continued)

| Probe Set ID | Type | Orientation | No. probes aligned | Ensembl Gene | Gene Symbol | Entrez Gene ID | Description | Chromosome | Strand | SEQ ID NO. |
|---|---|---|---|---|---|---|---|---|---|---|
| ADXGood73_at | Almac pixellation control | Sense (Fully Exonic) | 1 | ENSG00000130826 | DKC1 | 1736 /// 677835/// 100847052 | dyskeratosis congenita 1, dyskerin [Source: HGNC Symbol ;Acc:2890] | Chr X | Forward Strand | N/A |
| ADXGood73_at | Almac pixellation control | Sense (Fully Exonic) | 1 | ENSG00000107223 | EDF1 | 8721 | endothelial differentiation-related factor 1 [Source:HGNC Symbol;Acc:3164] | Chr 9 | Reverse Strand | N/A |
| ADXGood73_at | Almac pixellation control | Sense (Fully Exonic) | 1 | ENSG00000172037 | LAMB2 | 3913 | laminin, beta 2 (laminin S) [Source:HGNC Symbol ;Acc:6487] | Chr 3 | Reverse Strand | N/A |
| ADXGood73_at | Almac pixellation control | Sense (Fully Exonic) | 1 | ENSG00000104969 | SGTA | 6449 | small glutamine-rich tetratricopeptide repeat (TPR)-containing, alpha [Source:HGNC Symbol ;Acc:10819] | Chr 19 | Reverse Strand | N/A |
| ADXGood73_at | Almac pixellation control | Sense (Fully Exonic) | 1 | ENSG00000134684 | YARS | 8565 | tyrosyl-tRNA synthetase [Source:HGNC Symbol ;Acc:12840] | Chr 1 | Reverse Strand | N/A |
| ADXGood73_at | Almac pixellation control | Sense (Fully Exonic) | 1 | ENSG00000110711 | AIP | 9049 | aryl hydrocarbon receptor interacting protein [Source:HGNC Symbol ;Acc:358] | Chr 11 | Forward Strand | N/A |
| ADXGood73_at | Almac pixellation control | Sense (Fully Exonic) | 1 | ENSG00000188522 | FAM83G | 644815 | family with sequence similarity 83, member G [Source:HGNC Symbol ;Acc:32554] | Chr 17 | Reverse Strand | N/A |
| OC3P.10470.C1_s_at | Expression probe set | Sense (Fully Exonic) | 11 | ENSG00000100234 | TIMP3 | 7078 | TIMP metallopeptidase inhibitor 3 [Source: HGNC Symbol;Acc:11822] | Chr 22 | Forward Strand | 173 |
| OC3P.15327.C1_at | Expression probe set | Sense (includes Intronic) | 8 | ENSG00000100234 | TIMP3 | 7078 | TIMP metallopeptidase inhibitor 3 [Source: HGNC Symbol;Acc:11822] | Chr 22 | Forward Strand | 174 |

(continued)

| Probe Set ID | Type | Orient ation | No. prob es align ed | Ense mbl Gen e | Ge ne Sy mb ol | Entrez Gene ID | Description | Chro moso me | Stra nd | SEQ ID NO. |
|---|---|---|---|---|---|---|---|---|---|---|
| OCHP. 112_s_ at | Expre ssion probe set | Sense (Fully Exonic ) | 11 | ENS G000 0010 0234 | TIM P3 | 7078 | TIMP metallopeptidase inhibitor 3 [Source: HGNC Symbol;Acc:11822] | Chr 22 | For ward Stra nd | 175 |
| OC3P. 6478.C 1_s_ at | Expre ssion probe set | Sense (Fully Exonic ) | 11 | ENS G000 0016 4484 | TM EM 200 A | 114801 | transmembrane protein 200A [Source:HGNC Symbol;Acc:21075] | Chr 6 | For ward Stra nd | 176 |
| OC3P. 6478.C 1-363a_s _at | Expre ssion probe set | Sense (Fully Exonic ) | 11 | ENS G000 0016 4484 | TM EM 200 A | 114801 | transmembrane protein 200A [Source:HGNC Symbol;Acc:21075] | Chr 6 | For ward Stra nd | 177 |
| OCRS2 . 10857 _x_at | Expre ssion probe set | Sense (Fully Exonic ) | 10 | ENS G000 0012 3610 | TN FAI P6 | 7130 | tumor necrosis factor, alphainduced protein 6 [Source:HGNC Symbol;Acc:11898] | Chr 2 | For ward Stra nd | 178 |
| OC3P. 15028. C1_s_ a t | Expre ssion probe set | Sense (Fully Exonic ) | 11 | ENS G000 0003 8427 | VC AN | 1462 | versican [Source:HGNC Symbol ;Acc:2464] | Chr 5 | For ward Stra nd | 179 |
| OCAD NP. 965 7_s_at | Expre ssion probe set | Sense (Fully Exonic) | 8 | ENS G000 00038427 | VC AN | 1462 | versican [Source:HGNC Symbol ;Acc:2464] | Chr 5 | For ward Strand | 180 |
| OCMX. 15173. C1_s_ a t | Expre ssion probe set | Sense (Fully Exonic ) | 11 | ENS G000 0003 8427 | VC AN | 1462 | versican [Source:HGNC Symbol ;Acc:2464] | Chr 5 | For ward Stra nd | 181 |
| OCAD NP. 619 7_s_at | Expre ssion probe set | Sense (Fully Exonic ) | 11 | ENS G000 0003 8427 | VC AN | 1462 | versican [Source:HGNC Symbol ;Acc:2464] | Chr 5 | For ward Stra nd | 182 |
| OCRS2 . 1143_ s_at | Expre ssion probe set | Sense (Fully Exonic ) | 11 | ENS G000 0003 8427 | VC AN | 1462 | versican [Source:HGNC Symbol ;Acc:2464] | Chr 5 | For ward Stra nd | 183 |

(continued)

| Probe Set ID | Type | Orientation | No. probes aligned | Ensembl Gene | Gene Symbol | Entrez Gene ID | Description | Chromosome | Strand | SEQ ID NO. |
|---|---|---|---|---|---|---|---|---|---|---|
| OC3SN Gnh. 16 280_x_ at | Expression probe set | Sense (includes Intronic) | 7 | ENS G000 0003 8427 | VC AN | 1462 | versican [Source:HGNC Symbol ;Acc:2464] | Chr 5 | Forward Strand | 184 |
| OC3P. 1200.C 1_s_ at | Expression probe set | Sense (Fully Exonic ) | 11 | ENS G000 0003 8427 | VC AN | 1462 | versican [Source:HGNC Symbol ;Acc:2464] | Chr 5 | Forward Strand | 185 |
| OCAD A. 3158 _s_at | Expression probe set | Sense (Fully Exonic ) | 11 | ENS G000 0020 6538 | VG LL3 | 389136 | vestigial like 3 (Drosophila) [Source:HGNC Symbol ;Acc:24327] | Chr 3 | Reverse Strand | 186 |
| OCHP. 176_s_ at | Expression probe set | Sense (Fully Exonic ) | 11 | ENS G000 0020 6538 | VG LL3 | 389136 | vestigial like 3 (Drosophila) [Source:HGNC Symbol ;Acc:24327] | Chr 3 | Reverse Strand | 187 |

**Table F - 15 gene signature probeset information**

| Probe Set ID | Type | Orient ation | No. probe s aligne d | Ens embl Gen e | Gene Symb ol | Entr ez Gen e ID | Description | Chro moso me | Stra nd | SEQ ID NO. |
|---|---|---|---|---|---|---|---|---|---|---|
| OC3P.1 4147.C 1_s_at | Expre ssion probes et | Sense (Fully Exonic ) | 11 | ENS G00 000 140 937 | CDH1 1 | 100 9 | cadherin 11, type 2, OBcadherin (osteoblast) [Source:HGNC Symbol; Acc:1750] | Chr 16 | Rev erse Stra nd | 188 |
| OC3SN Gnh.40 32_s_at | Expre ssion probes et | Sense (includ es Introni c) | 11 | ENS G00 000 140 937 | CDH1 1 | 100 9 | cadherin 11, type 2, OBcadherin (osteoblast) [Source:HGNC Symbol; Acc:1750] | Chr 16 | Rev erse Stra nd | 189 |
| OC3SN Gnh.50 56_x_at | Expre ssion probes et | Sense (includes Introni c) | 6 | ENS G00000 140 937 | CDH1 1 | 100 9 | cadherin 11, type 2, OBcadherin (osteoblast) [Source:HGNC Symbol; Acc:1750] | Chr 16 | Rev erse Stra nd | 190 |
| OCADA .6210_s _at | Expre ssion probes et | Sense (includ es Introni c) | 9 | ENS G00 000 140 937 | CDH1 1 | 100 9 | cadherin 11, type 2, OBcadherin (osteoblast) [Source:HGNC Symbol; Acc:1750] | Chr 16 | Rev erse Stra nd | 191 |
| OCADA ,8067_x _at | Expre ssion probes et | Sense (includ es Introni c) | 11 | ENS G00 000 140 937 | CDH1 1 | 100 9 | cadherin 11, type 2, OBcadherin (osteoblast) [Source:HGNC Symbol; Acc:1750] | Chr 16 | Rev erse Stra nd | 192 |
| OCADN P.10024 _s_at | Expre ssion probes et | Sense (Fully Exonic ) | 11 | ENS G00 000 140 937 | CDH1 1 | 100 9 | cadherin 11, type 2, OBcadherin (osteoblast) [Source:HGNC Symbol; Acc:1750] | Chr 16 | Rev erse Stra nd | 193 |
| OCHP.1 48_s_at | Expre ssion probes et | Sense (Fully Exonic ) | 11 | ENS G00 000 140 937 | CDH1 1 | 100 9 | cadherin 11, type 2, OBcadherin (osteoblast) [Source:HGNC Symbol; Acc:1750] | Chr 16 | Rev erse Stra nd | 194 |
| OCHPR C.58_s_ at | Expre ssion probes et | Sense (Fully Exonic ) | 11 | ENS G00 000 140 937 | CDH1 1 | 100 9 | cadherin 11, type 2, OBcadherin (osteoblast) [Source:HGNC Symbol; Acc:1750] | Chr 16 | Rev erse Stra nd | 195 |
| OCMX. 1718.C 1_s_at | Expre ssion probes et | Sense (Fully Exonic ) | 11 | ENS G00 000 140 937 | CDH1 1 | 100 9 | cadherin 11, type 2, OBcadherin (osteoblast) [Source:HGNC Symbol; Acc:1750] | Chr 16 | Rev erse Stra nd | 196 |
| OC3P.1 1285.C 1_s_at | Expre ssion probes et | Sense (Fully Exonic ) | 11 | ENS G00 000 168 461 | RAB3 1 | 110 31 | RAB31, member RAS oncogene family [Source:HGNC Symbol;Acc: 9771] | Chr 18 | For ward Stra nd | 197 |

(continued)

| Probe Set ID | Type | Orient ation | No. probe s aligne d | Ensembl Gen e | Gene Symb ol | Entr ez Gen e ID | Description | Chro moso me | Stra nd | SEQ ID NO. |
|---|---|---|---|---|---|---|---|---|---|---|
| OC3P.8 262.C1 _s_at | Expre ssion probes et | Sense (Fully Exonic ) | 11 | ENS G00 000 168 461 | RAB3 1 | 110 31 | RAB31, member RAS oncogene family [Source:HGNC Symbol;Acc: 9771] | Chr 18 | For ward Stra nd | 198 |
| OC3SN Gnh.17 870_s_ at | Expre ssion probes et | Sense (includ es Introni c) | 11 | ENS G00 000 168 461 | RAB3 1 | 110 31 | RAB31, member RAS oncogene family [Source:HGNC Symbol;Acc: 9771] | Chr 18 | For ward Stra nd | 199 |
| OCHP.1 160_s_ at | Expre ssion probes et | Sense (Fully Exonic ) | 11 | ENS G00 000 168 461 | RAB3 1 | 110 31 | RAB31, member RAS oncogene family [Source:HGNC Symbol;Acc: 9771] | Chr 18 | For ward Stra nd | 200 |
| OCMX. 11222. C1_at | Expre ssion probes et | Sense (includ es Introni c) | 10 | ENS G00 000 168 461 | RAB3 1 | 110 31 | RAB31, member RAS oncogene family [Source:HGNC Symbol;Acc: 9771] | Chr 18 | For ward Stra nd | 201 |
| OC3P. 2342.C1-300a_s _at | Expression probes et | Sense (Fully Exonic ) | 11 | ENSG00 000 130 635 | COL5A1 | 1289 | collagen, type V, alpha 1 [Source: HGNC Symbol;Acc:2209] | Chr 9 | Forward Stra nd | 202 |
| OC3P.4 984.C1-787a_s _at | Expre ssion probes et | Sense (Fully Exonic ) | 11 | ENS G00 000 130 635 | COL5 A1 | 128 9 | collagen, type V, alpha 1 [Source: HGNC Symbol;Acc:2209] | Chr 9 | For ward Stra nd | 203 |
| OC3P.4 984.C1 _s_at | Expre ssion probes et | Sense (Fully Exonic ) | 11 | ENS G00 000 130 635 | COL5 A1 | 128 9 | collagen, type V, alpha 1 [Source: HGNC Symbol;Acc:2209] | Chr 9 | For ward Stra nd | 204 |
| OC3SN Gnh.10 085_x_ at | Expre ssion probes et | Sense (includ es Introni c) | 7 | ENS G00 000 130 635 | COL5 A1 | 128 9 | collagen, type V, alpha 1 [Source: HGNC Symbol;Acc:2209] | Chr 9 | For ward Stra nd | 205 |
| OC3SN Gnh.11 037_at | Expre ssion probes et | Sense (includ es Introni c) | 10 | ENS G00 000 130 635 | COL5 A1 | 128 9 | collagen, type V, alpha 1 [Source: HGNC Symbol;Acc:2209] | Chr 9 | For ward Stra nd | 206 |
| OC3SN Gnh.11 037_x_ at | Expre ssion probes et | Sense (includ es Introni c) | 10 | ENS G00 000 130 635 | COL5 A1 | 128 9 | collagen, type V, alpha 1 [Source: HGNC Symbol;Acc:2209] | Chr 9 | For ward Stra nd | 207 |

(continued)

| Probe Set ID | Type | Orient ation | No. probe s aligne d | Ensembl Gen e | Gene Symb ol | Entr ez Gen e ID | Description | Chro moso me | Stra nd | SEQ ID NO. |
|---|---|---|---|---|---|---|---|---|---|---|
| OC3SN Gnh.17 281_at | Expre ssion probes et | Sense (includ es Introni c) | 9 | ENS G00 000 130 635 | COL5 A1 | 128 9 | collagen, type V, alpha 1 [Source: HGNC Symbol;Acc:2209] | Chr 9 | For ward Stra nd | 208 |
| OCADA .582_s at | Expre ssion probes et | Sense (includ es Introni c) | 11 | ENS G00 000 130 635 | COL5 A1 | 128 9 | collagen, type V, alpha 1 [Source: HGNC Symbol;Acc:2209] | Chr 9 | For ward Stra nd | 209 |
| OCHP.1 005_s_ at | Expre ssion probes et | Sense (Fully Exonic ) | 11 | ENS G00 000 130 635 | COL5 A1 | 128 9 | collagen, type V, alpha 1 [Source: HGNC Symbol;Acc:2209] | Chr 9 | For ward Stra nd | 210 |
| OCMX. 8587.C 1_s_at | Expre ssion probes et | Sense (Fully Exonic ) | 9 | ENS G00 000 130 635 | COL5 A1 | 128 9 | collagen, type V, alpha 1 [Source: HGNC Symbol;Acc:2209] | Chr 9 | For ward Stra nd | 211 |
| OC3SN G. 1834-947a_s _at | Expre ssion probes et | Sense (Fully Exonic ) | 11 | ENS G00 000 123 500 | COL1 0A1 | 130 0 | collagen, type X, alpha 1 [Source: HGNC Symbol;Acc:2185] | Chr 6 | Rev erse Stra nd | 212 |
| OCRS.3 83_s_at | Expre ssion probes et | Sense (Fully Exonic ) | 11 | ENS G00 000 123 500 | COL1 0A1 | 130 0 | collagen, type X, alpha 1 [Source: HGNC Symbol;Acc:2185] | Chr 6 | Rev erse Stra nd | 213 |
| OC3P.1 200.C1 _s_at | Expre ssion probes et | Sense (Fully Exonic ) | 11 | ENS G00 000 038 427 | VCAN | 146 2 | versican [Source:HGNC Symbol; Acc:2464] | Chr 5 | For ward Stra nd | 214 |
| OC3P.1 5028.C 1_s_at | Expre ssion probes et | Sense (Fully Exonic ) | 11 | ENS G00 000 038 427 | VCAN | 146 2 | versican [Source:HGNC Symbol; Acc:2464] | Chr 5 | For ward Stra nd | 215 |
| OC3SN Gnh.16 280_x_ at | Expre ssion probes et | Sense (includ es Introni c) | 7 | ENS G00 000 038 427 | VCAN | 146 2 | versican [Source:HGNC Symbol; Acc:2464] | Chr 5 | For ward Stra nd | 216 |
| OCADN P.6197_ s_at | Expre ssion probes et | Sense (Fully Exonic ) | 11 | ENS G00 000 038 427 | VCAN | 146 2 | versican [Source:HGNC Symbol; Acc:2464] | Chr 5 | For ward Stra nd | 217 |

(continued)

| Probe Set ID | Type | Orient ation | No. probe s aligne d | Ensembl Gen e | Gene Symb ol | Entr ez Gen e ID | Description | Chro moso me | Stra nd | SEQ ID NO. |
|---|---|---|---|---|---|---|---|---|---|---|
| OCADN P.9657_ s_at | Expre ssion probes et | Sense (Fully Exonic ) | 8 | ENS G00 000 038 427 | VCAN | 146 2 | versican [Source:HGNC Symbol; Acc:2464] | Chr 5 | For ward Stra nd | 218 |
| OCMX. 15173. C1_s_at | Expre ssion probes et | Sense (Fully Exonic ) | 11 | ENS G00 000 038 427 | VCAN | 146 2 | versican [Source:HGNC Symbol; Acc:2464] | Chr 5 | For ward Stra nd | 219 |
| OCRS2. 1143_s _at | Expre ssion probes et | Sense (Fully Exonic ) | 11 | ENS G00 000 038 427 | VCAN | 146 2 | versican [Source:HGNC Symbol; Acc:2464] | Chr 5 | For ward Stra nd | 220 |
| OC3P.8 736.C1 _s_at | Expre ssion probes et | Sense (Fully Exonic ) | 9 | ENS G00 000 078 098 | FAP | 219 1 | fibroblast activation protein, alpha [Source:HGNC Symbol;Acc:3590] | Chr 2 | Rev erse Stra nd | 221 |
| OC3SN Gn.301 6-7a_s_at | Expre ssion probes et | Sense (Fully Exonic ) | 8 | ENS G00 000 078 098 | FAP | 219 1 | fibroblast activation protein, alpha [Source:HGNC Symbol;Acc:3590] | Chr 2 | Rev erse Stra nd | 222 |
| OCADA .9856_x _at | Expre ssion probes et | Sense (includ es Introni c) | 7 | ENS G00 000 078 098 | FAP | 219 1 | fibroblast activation protein, alpha [Source:HGNC Symbol;Acc:3590] | Chr 2 | Rev erse Stra nd | 223 |
| OC3P.8 43.CB1-415a_s _at | Expre ssion probes et | Sense (Fully Exonic ) | 11 | ENS G00 000 115 414 | FN1 | 233 5 | fibronectin 1 [Source:HGNC Symbol;Acc:3778] | Chr 2 | Rev erse Stra nd | 224 |
| OC3SN Gn.465 0-857a_x_at | Expre ssion probes et | Sense (includ es Intronic) | 11 | ENS G00 000 115414 | FN1 | 233 5 | fibronectin 1 [Source:HGNC Symbol;Acc:3778] | Chr 2 | Rev erse Stra nd | 225 |
| OC3SN Gn.639 7-360a_at | Expre ssion probes et | Sense (Fully Exonic ) | 11 | ENS G00 000 115 414 | FN1 | 233 5 | fibronectin 1 [Source:HGNC Symbol;Acc:3778] | Chr 2 | Rev erse Stra nd | 226 |
| OC3SN Gnh.14 004_at | Expre ssion probes et | Sense (includ es Introni c) | 7 | ENS G00 000 115 414 | FN1 | 233 5 | fibronectin 1 [Source:HGNC Symbol;Acc:3778] | Chr 2 | Rev erse Stra nd | 227 |

(continued)

| Probe Set ID | Type | Orient ation | No. probe s aligne d | Ensembl Gen e | Gene Symb ol | Entr ez Gen e ID | Description | Chro moso me | Stra nd | SEQ ID NO. |
|---|---|---|---|---|---|---|---|---|---|---|
| OC3SN Gnh.14 004_x_ at | Expre ssion probes et | Sense (includ es Introni c) | 8 | ENS G00 000 115 414 | FN1 | 233 5 | fibronectin 1 [Source:HGNC Symbol;Acc:3778] | Chr 2 | Rev erse Stra nd | 228 |
| OC3SN Gnh.40 44_x_at | Expre ssion probes et | Sense (includ es Introni c) | 8 | ENS G00 000 115 414 | FN1 | 233 5 | fibronectin 1 [Source:HGNC Symbol;Acc:3778] | Chr 2 | Rev erse Stra nd | 229 |
| OC3SN Gnh.59 67_at | Expre ssion probes et | Sense (includ es Introni c) | 9 | ENS G00 000 115 414 | FN1 | 233 5 | fibronectin 1 [Source:HGNC Symbol;Acc:3778] | Chr 2 | Rev erse Stra nd | 230 |
| OC3SN Gnh.92 61_at | Expre ssion probes et | Sense (includ es Introni c) | 11 | ENS G00 000 115 414 | FN1 | 233 5 | fibronectin 1 [Source:HGNC Symbol;Acc:3778] | Chr 2 | Rev erse Stra nd | 231 |
| OCADA .1039_s _at | Expre ssion probes et | Sense (Fully Exonic ) | 11 | ENS G00 000 115 414 | FN1 | 233 5 | fibronectin 1 [Source:HGNC Symbol;Acc:3778] | Chr 2 | Rev erse Stra nd | 232 |
| OCADA .7873_s _at | Expre ssion probes et | Sense (includ es Introni c) | 11 | ENS G00 000 115 414 | FN1 | 233 5 | fibronectin 1 [Source:HGNC Symbol;Acc:3778] | Chr 2 | Rev erse Stra nd | 233 |
| OCEM. 2081_at | Expre ssion probes et | Sense (Fully Exonic ) | 11 | ENS G00 000 115 414 | FN1 | 233 5 | fibronectin 1 [Source:HGNC Symbol;Acc:3778] | Chr 2 | Rev erse Stra nd | 234 |
| OCEM. 2081_x _at | Expre ssion probes et | Sense (Fully Exonic ) | 11 | ENS G00 000 115 414 | FN1 | 233 5 | fibronectin 1 [Source:HGNC Symbol;Acc:3778] | Chr 2 | Rev erse Stra nd | 235 |
| OCEM. 2082_at | Expre ssion probes et | Sense (Fully Exonic ) | 11 | ENS G00 000 115 414 | FN1 | 233 5 | fibronectin 1 [Source:HGNC Symbol;Acc:3778] | Chr 2 | Rev erse Stra nd | 236 |
| OCEM. 2082_s _at | Expre ssion probes et | Sense (Fully Exonic) | 11 | ENS G00 000115 414 | FN1 | 233 5 | fibronectin 1 [Source:HGNC Symbol;Acc:3778] | Chr 2 | Rev erse Strand | 237 |

(continued)

| Probe Set ID | Type | Orient ation | No. probe s aligne d | Ensembl Gen e | Gene Symb ol | Entr ez Gen e ID | Description | Chro moso me | Stra nd | SEQ ID NO. |
|---|---|---|---|---|---|---|---|---|---|---|
| OCEM. 958_at | Expre ssion probes et | Sense (includ es Introni c) | 11 | ENS G00 000 115 414 | FN1 | 233 5 | fibronectin 1 [Source:HGNC Symbol;Acc:3778] | Chr 2 | Rev erse Stra nd | 238 |
| OCEM. 958_x_ at | Expre ssion probes et | Sense (includ es Introni c) | 11 | ENS G00 000 115 414 | FN1 | 233 5 | fibronectin 1 [Source:HGNC Symbol;Acc:3778] | Chr 2 | Rev erse Stra nd | 239 |
| OCHP.4 51_s_at | Expre ssion probes et | Sense (Fully Exonic ) | 11 | ENS G00 000 115 414 | FN1 | 233 5 | fibronectin 1 [Source:HGNC Symbol;Acc:3778] | Chr 2 | Rev erse Stra nd | 240 |
| OCHP.4 70_s_at | Expre ssion probes et | Sense (Fully Exonic ) | 11 | ENS G00 000 115 414 | FN1 | 233 5 | fibronectin 1 [Source:HGNC Symbol;Acc:3778] | Chr 2 | Rev erse Stra nd | 241 |
| OCMX. 493.C1 _s_at | Expre ssion probes et | Sense (Fully Exonic ) | 11 | ENS G00 000 115 414 | FN1 | 233 5 | fibronectin 1 [Source:HGNC Symbol;Acc:3778] | Chr 2 | Rev erse Stra nd | 242 |
| OC3P.2 679.C1 _s_at | Expre ssion probes et | Sense (Fully Exonic ) | 11 | ENS G00 000 136 859 | ANGP TL2 | 234 52 | angiopoietin-like 2 [Source:HGNC Symbol;Acc:490] | Chr 9 | Rev erse Stra nd | 243 |
| OC3P.9 834.C1 _s_at | Expre ssion probes et | Sense (Fully Exonic ) | 11 | ENS G00 000 136 859 | ANGP TL2 | 234 52 | angiopoietin-like 2 [Source:HGNC Symbol;Acc:490] | Chr 9 | Rev erse Stra nd | 244 |
| OCADA .8226_s _at | Expre ssion probes et | Sense (includ es Introni c) | 11 | ENS G00 000 136 859 | ANGP TL2 | 234 52 | angiopoietin-like 2 [Source:HGNC Symbol;Acc:490] | Chr 9 | Rev erse Stra nd | 245 |
| OCADN P.8811_ s_at | Expre ssion probes et | Sense (Fully Exonic ) | 8 | ENS G00 000 136 859 | ANGP TL2 | 234 52 | angiopoietin-like 2 [Source:HGNC Symbol;Acc:490] | Chr 9 | Rev erse Stra nd | 246 |
| OC3P.7 485.C1-335a_s _at | Expre ssion probes et | Sense (Fully Exonic ) | 11 | ENS G00 000 165 474 | GJB2 | 270 6 | gap junction protein, beta 2, 26kDa [Source:HGNC Symbol;Acc:4284] | Chr 13 | Rev erse Stra nd | 247 |

(continued)

| Probe Set ID | Type | Orient ation | No. probe s aligne d | Ensembl Gen e | Gene Symb ol | Entr ez Gen e ID | Description | Chro moso me | Stra nd | SEQ ID NO. |
|---|---|---|---|---|---|---|---|---|---|---|
| OCHP.8 38_s_at | Expre ssion probes et | Sense (Fully Exonic ) | 11 | ENS G00 000 165 474 | GJB2 | 270 6 | gap junction protein, beta 2, 26kDa [Source:HGNC Symbol;Acc:4284] | Chr 13 | Rev erse Stra nd | 248 |
| OC3P.1 0944.C1_s_at | Expre ssion probes et | Sense (Fully Exonic ) | 11 | ENS G00000 122 641 | INHBA | 362 4 | inhibin, beta A [Source:HGNC Symbol;Acc:6066] | Chr 7 | Rev erse Stra nd | 249 |
| OC3SN Gnh.36 06_s_at | Expre ssion probes et | Sense (Fully Exonic ) | 11 | ENS G00 000 122 641 | INHBA | 362 4 | inhibin, beta A [Source:HGNC Symbol;Acc:6066] | Chr 7 | Rev erse Stra nd | 250 |
| OCADN P.7618_ s_at | Expre ssion probes et | Sense (Fully Exonic ) | 11 | ENS G00 000 122 641 | INHBA | 362 4 | inhibin, beta A [Source:HGNC Symbol;Acc:6066] | Chr 7 | Rev erse Stra nd | 251 |
| OCEM. 2108_at | Expre ssion probes et | Sense (Fully Exonic ) | 11 | ENS G00 000 122 641 | INHBA | 362 4 | inhibin, beta A [Source:HGNC Symbol;Acc:6066] | Chr 7 | Rev erse Stra nd | 252 |
| OCEM. 2108_x _at | Expre ssion probes et | Sense (Fully Exonic ) | 11 | ENS G00 000 122 641 | INHBA | 362 4 | inhibin, beta A [Source:HGNC Symbol;Acc:6066] | Chr 7 | Rev erse Stra nd | 253 |
| OCEM. 2109_at | Expre ssion probes et | Sense (Fully Exonic ) | 8 | ENS G00 000 122 641 | INHBA | 362 4 | inhibin, beta A [Source:HGNC Symbol;Acc:6066] | Chr 7 | Rev erse Stra nd | 254 |
| OCEM. 2109_s _at | Expre ssion probes et | Sense (Fully Exonic ) | 11 | ENS G00 000 122 641 | INHBA | 362 4 | inhibin, beta A [Source:HGNC Symbol;Acc:6066] | Chr 7 | Rev erse Stra nd | 255 |
| OCRS.9 77_s_at | Expre ssion probes et | Sense (Fully Exonic ) | 11 | ENS G00 000 122 641 | INHBA | 362 4 | inhibin, beta A [Source:HGNC Symbol;Acc:6066] | Chr 7 | Rev erse Stra nd | 256 |
| OC3P.4 123.C1 _s_at | Expre ssion probes et | Sense (Fully Exonic ) | 11 | ENS G00 000 157 227 | MMP1 4 | 432 3 | matrix metallopeptidase 14 (membrane-inserted) [Source: HGNC Symbol;Acc:7160] | Chr 14 | For ward Stra nd | 257 |

(continued)

| Probe Set ID | Type | Orient ation | No. probe s aligne d | Ensembl Gen e | Gene Symb ol | Entr ez Gen e ID | Description | Chro moso me | Stra nd | SEQ ID NO. |
|---|---|---|---|---|---|---|---|---|---|---|
| OC3P.4 123.C1 _x_at | Expre ssion probes et | Sense (Fully Exonic ) | 9 | ENS G00 000 157 227 | MMP1 4 | 432 3 | matrix metallopeptidase 14 (membrane-inserted) [Source: HGNC Symbol;Acc:7160] | Chr 14 | For ward Stra nd | 258 |
| OCHP.2 28_s_at | Expre ssion probes et | Sense (Fully Exonic ) | 11 | ENS G00 000 157 227 | MMP1 4 | 432 3 | matrix metallopeptidase 14 (membrane-inserted) [Source: HGNC Symbol;Acc:7160] | Chr 14 | For ward Stra nd | 259 |
| OCADN P.8653_ s_at | Expre ssion probes et | Sense (Fully Exonic ) | 10 | ENS G00 000 122 861 | PLAU | 532 8 | plasminogen activator, urokinase [Source:HGNC Symbol;Acc:9052] | Chr 10 | For ward Stra nd | 260 |
| OCHP.739_s_at | Expression probes et | Sense (Fully Exonic ) | 11 | ENSG00 000 122 861 | PLAU | 5328 | plasminogen activator, urokinase [Source:HGNC Symbol;Acc:9052] | Chr 10 | Forward Stra nd | 261 |
| OC3P.1 1604.C 1_s_at | Expre ssion probes et | Sense (Fully Exonic ) | 9 | ENS G00 000 137 801 | THBS 1 | 705 7 | thrombospondin 1 [Source:HGNC Symbol;Acc:11785] | Chr 15 | For ward Stra nd | 262 |
| OC3P.9 115.C1-992a_s _at | Expre ssion probes et | Sense (Fully Exonic ) | 11 | ENS G00 000 137 801 | THBS 1 | 705 7 | thrombospondin 1 [Source:HGNC Symbol;Acc:11785] | Chr 15 | For ward Stra nd | 263 |
| OCADN P.4824_ s_at | Expre ssion probes et | Sense (Fully Exonic ) | 11 | ENS G00 000 137 801 | THBS 1 | 705 7 | thrombospondin 1 [Source:HGNC Symbol;Acc:11785] | Chr 15 | For ward Stra nd | 264 |
| OCADN P.6208_ s_at | Expre ssion probes et | Sense (Fully Exonic ) | 11 | ENS G00 000 137 801 | THBS 1 | 705 7 | thrombospondin 1 [Source:HGNC Symbol;Acc:11785] | Chr 15 | For ward Stra nd | 265 |
| OCHP.1 68_x_at | Expre ssion probes et | Sense (Fully Exonic ) | 11 | ENS G00 000 137 801 | THBS 1 | 705 7 | thrombospondin 1 [Source:HGNC Symbol;Acc:11785] | Chr 15 | For ward Stra nd | 266 |
| OCMX. 2515.C 1_s_at | Expre ssion probes et | Sense (Fully Exonic ) | 9 | ENS G00 000 137 801 | THBS 1 | 705 7 | thrombospondin 1 [Source:HGNC Symbol;Acc:11785] | Chr 15 | For ward Stra nd | 267 |

(continued)

| Probe Set ID | Type | Orient ation | No. probe s aligne d | Ens embl Gen e | Gene Symb ol | Entr ez Gen e ID | Description | Chro moso me | Stra nd | SEQ ID NO. |
|---|---|---|---|---|---|---|---|---|---|---|
| OCMX. 318.C1 _s_at | Expre ssion probes et | Sense (Fully Exonic ) | 9 | ENS G00 000 137 801 | THBS 1 | 705 7 | thrombospondin 1 [Source:HGNC Symbol;Acc:11785] | Chr 15 | For ward Stra nd | 268 |
| OC3P.4 296.C1-409a_s _at | Expre ssion probes et | Sense (Fully Exonic ) | 11 | ENS G00 000 186 340 | THBS 2 | 705 8 | thrombospondin 2 [Source:HGNC Symbol;Acc:11786] | Chr 6 | Rev erse Stra nd | 269 |
| OC3P.4 296.C2 _s_at | Expre ssion probes et | Sense (Fully Exonic ) | 11 | ENS G00 000 186 340 | THBS 2 | 705 8 | thrombospondin 2 [Source:HGNC Symbol;Acc:11786] | Chr 6 | Rev erse Stra nd | 270 |
| OC3SN Gn.187 3-1656a_ s_at | Expre ssion probes et | Sense (Fully Exonic ) | 11 | ENS G00 000 186 340 | THBS 2 | 705 8 | thrombospondin 2 [Source:HGNC Symbol;Acc:11786] | Chr 6 | Rev erse Stra nd | 271 |
| OC3SN Gnh.14 530_at | Expre ssion probes et | Sense (includ es Introni c) | 11 | ENS G00 000 186 340 | THBS 2 | 705 8 | thrombospondin 2 [Source:HGNC Symbol;Acc:11786] | Chr 6 | Rev erse Stra nd | 272 |
| OC3SN Gnh.14 530_s_ at | Expre ssion probes et | Sense (includ es Introni c) | 11 | ENS G00 000 186 340 | THBS 2 | 705 8 | thrombospondin 2 [Source:HGNC Symbol;Acc:11786] | Chr 6 | Rev erse Stra nd | 273 |
| OCHP.6 77_s_at | Expre ssion probes et | Sense (Fully Exonic ) | 11 | ENS G00 000 186 340 | THBS 2 | 705 8 | thrombospondin 2 [Source:HGNC Symbol;Acc:11786] | Chr 6 | Rev erse Stra nd | 274 |
| OC3SN Gnh.16 386_at | Expre ssion probes et | Sense (includ es Introni c) | 10 | ENS G00 000 131 459 | GFPT 2 | 994 5 | glutamine-fructose-6-phosphate transaminase 2 [Source:HGNC Symbol;Acc:4242] | Chr 5 | Rev erse Stra nd | 275 |
| OCADA .12319_ s_at | Expre ssion probes et | Sense (includ es Introni c) | 11 | ENS G00 000 131 459 | GFPT 2 | 994 5 | glutamine-fructose-6-phosphate transaminase 2 [Source:HGNC Symbol;Acc:4242] | Chr 5 | Rev erse Stra nd | 276 |
| OCHP.2 02_s_at | Expre ssion probes et | Sense (Fully Exonic ) | 11 | ENS G00 000 131 459 | GFPT 2 | 994 5 | glutamine-fructose-6-phosphate transaminase 2 [Source:HGNC Symbol;Acc:4242] | Chr 5 | Rev erse Stra nd | 277 |

By "biomarker signature" is meant an identifier comprised of one or more biomarkers (such as a DNA or RNA sequence, a protein or other biological molecule, a cell etc.). The expression level of the one or more biomarkers is measured and the measured expression levels allow the sample to be defined as signature positive or signature negative. Thus, at its simplest, an increased level of expression of one or more biomarkers defines a sample as positive for the biomarker signature. For certain biomarkers, a decreased level of expression of one or more biomarkers defines a sample as positive for the biomarker signature. However, where the expression level of a plurality of biomarkers is measured, the combination of expression levels is typically aggregated in order to determine whether the sample is positive for the biomarker signature. Thus, some biomarkers may display increased expression and some biomarkers may display decreased expression. This can be achieved in various ways, as discussed in detail herein.

In a general sense, in some embodiments, the biomarker signature may be considered as indicative of a particular biological state (such as the presence of a disease condition or developmental state or belonging to a particular biological subgroup). "Positive" for a biomarker signature thus may be interpreted to mean that the sample reflects the relevant biological state that the biomarker signature identifies. Similarly, "negative" for a biomarker signature means that the sample is not in (or reflective of) the relevant biological state. In the present invention, the biological state indicated by the biomarker signature is a molecular subgroup of cancer characterised by misregulation of the MAPK signalling pathway and the epithelial-mesenchymal transition (EMT) pathway. Thus, the cancer identified by the signature may have increased MAPK signalling. The cancer identified by the signature may have increased expression of both immune response and angiogenesis/vascular development genes. The cancer identified by the signature may display higher expression of EMT associated genes. This may include increased expression of VIMENTIN, AXL, TWIST1, SNAIL and/or SLUG. The increased signalling or expression is as compared to other cancers of the same type. So, for example, the cancer may be an ovarian cancer and the subgroup displays increased signalling or expression as compared to other ovarian cancers. Genes defining the EMT/Angio-Immune/MAPK pathway molecular subgroup of cancer are listed in Tables 9 and 10 below. The expression level of the genes may be measured using the probesets in Table 11. In Table 9 up-regulation and down-regulation are presented relative to gene expression levels in the overall sample set.

The biomarker signature is also correlated with particular end points as discussed in detail herein. The biomarker signature may permit selection of appropriate therapeutic interventions for example.

According to all aspects of the invention assessing whether the sample is positive or negative for the biomarker signature may comprise use of classification trees.

According to all aspects of the invention assessing whether the sample is positive or negative for the biomarker signature may comprise:

determining a sample expression score for the biomarker(s);
comparing the sample expression score to a threshold score; and
determining whether the sample expression score is above, equal to, or below the threshold expression score, wherein if the sample expression score is above or equal to the threshold expression score the sample is positive for the biomarker signature and/or if the sample expression score is below the threshold score the sample is negative for the biomarker signature.

The skilled person will be aware that threshold expression scores may be set in a number of ways, as discussed in greater detail herein below, for example in order to maximise sensitivity and/or specificity. Thus, the sample expression score and threshold score may also be determined such that if the sample expression score is below or equal to the threshold expression score the sample is positive for the biomarker signature and/or if the sample expression score is above the threshold score the sample is negative for the biomarker signature.

"Expression levels" of biomarkers may be numerical values or directions of expression. By "directions" is meant increased or decreased expression, which may be determined as against a control or threshold expression level as explained further herein.

In the methods the sample expression score (or "signature score") may be derived according to the formula:

$$SignatureScore = \sum_i w_i \times (ge_i - b_i) + k$$

Where $w_i$ is a weight for each gene, $b_i$ is a gene-specific bias, $ge_i$ is the gene expression after pre-processing, and k is a constant offset.

The sample expression score may be derived using the expression level(s) of any of the genes or groups of genes described herein. The sample expression score may be derived using the expression level of one or more additional genes.

According to all aspects of the invention the expression score may be calculated using a weight value and a bias value for each biomarker. For example, the weight value and the bias value may be as defined for each biomarker in Table A and/or Table B. The expression score may be calculated using a weight value for each biomarker.

As used herein, the term "weight" refers to the absolute magnitude of an item in a mathematical calculation. The weight of each biomarker in a gene expression classifier or signature may be determined on a data set of patient samples using learning methods known in the art. As used herein the term "bias" or "offset" refers to a constant term derived using the mean expression of the signatures genes in a training set and is used to mean-center each gene analyzed in the test dataset.

By "expression score" is meant a compound decision score that summarizes the expression levels of the biomarkers. This may be compared to a threshold score that is mathematically derived from a training set of patient data. The threshold score is established with the purpose of maximizing the ability to separate cancers into those that are positive for the biomarker signature and those that are negative. The patient training set data is preferably derived from cancer tissue samples having been characterized by sub-type, prognosis, likelihood of recurrence, long term survival, clinical outcome, treatment response, diagnosis, cancer classification, or personalized genomics profile. Expression profiles, and corresponding decision scores from patient samples may be correlated with the characteristics of patient samples in the training set that are on the same side of the mathematically derived score decision threshold. In certain example embodiments, the threshold of the (linear) classifier scalar output is optimized to maximize the sum of sensitivity and specificity under cross-validation as observed within the training dataset.

The overall expression data for a given sample may be normalized using methods known to those skilled in the art in order to correct for differing amounts of starting material, varying efficiencies of the extraction and amplification reactions, etc.

In one embodiment, the biomarker expression levels in a sample are evaluated by a (linear) classifier. As used herein, a (linear) classifier refers to a weighted sum of the individual biomarker intensities into a compound decision score ("decision function"). The decision score is then compared to a pre-defined cut-off score threshold, corresponding to a certain set-point in terms of sensitivity and specificity which indicates if a sample is equal to or above the score threshold (decision function positive) or below (decision function negative).

Using a (linear) classifier on the normalized data to make a call (e.g. positive or negative for a biomarker signature) effectively means to split the data space, i.e. all possible combinations of expression values for all genes in the classifier, into two disjoint segments by means of a separating hyperplane. This split is empirically derived on a (large) set of training examples. Without loss of generality, one can assume a certain fixed set of values for all but one biomarker, which would automatically define a threshold value for this remaining biomarker where the decision would change from, for example, positive or negative for the biomarker signature. The precise value of this threshold depends on the actual measured expression profile of all other biomarkers within the classifier, but the general indication of certain biomarkers remains fixed. Therefore, in the context of the overall gene expression classifier, relative expression can indicate if either up- or down-regulation of a certain biomarker is indicative of being positive for the signature or not. In certain example embodiments, a sample expression score above the threshold expression score indicates the sample is positive for the biomarker signature. In certain other example embodiments, a sample expression score above a threshold score indicates the subject has a poor clinical prognosis compared to a subject with a sample expression score below the threshold score.

In certain other example embodiments, the expression signature is derived using a decision tree (Hastie et al. The Elements of Statistical Learning, Springer, New York 2001), a random forest (Breiman, 2001 Random Forests, Machine Learning 45:5), a neural network (Bishop, Neural Networks for Pattern Recognition, Clarendon Press, Oxford 1995), discriminant analysis (Duda et al. Pattern Classification, 2nd ed., John Wiley, New York 2001), including, but not limited to linear, diagonal linear, quadratic and logistic discriminant analysis, a Prediction Analysis for Microarrays (PAM, (Tibshirani et al., 2002, Proc. Natl. Acad. Sci. USA 99:6567-6572)) or a Soft Independent Modeling of Class Analogy analysis. (SIMCA, (Wold, 1976, Pattern Recogn. 8:127-139)). Classification trees (Breiman, Leo; Friedman, J. H.; Olshen, R. A.; Stone, C. J. (1984). Classification and regression trees. Monterey, CA: Wadsworth & Brooks/Cole Advanced Books & Software. ISBN 978-0-412-04841-8) provide a means of predicting outcomes based on logic and rules. A classification tree is built through a process called binary recursive partitioning, which is an iterative procedure of splitting the data into partitions/branches. The goal is to build a tree that distinguishes among pre-defined classes. Each node in the tree corresponds to a variable. To choose the best split at a node, each variable is considered in turn, where every possible split is tried and considered, and the best split is the one which produces the largest decrease in diversity of the classification label within each partition. This is repeated for all variables, and the winner is chosen as the best splitter for that node. The process is continued at the next node and in this manner, a full tree is generated. One of the advantages of classification trees over other supervised learning approaches such as discriminant analysis, is that the variables that are used to build the tree can be either categorical, or numeric, or a mix of both. In this way it is possible to generate a classification tree for predicting outcomes based on say the directionality of gene expression. Random forest algorithms (Breiman, Leo (2001). "Random Forests". Machine Learning 45 (1): 5-32. doi:10.1023/A:1010933404324) provide a further extension to classification trees, whereby a collection of classification trees are randomly generated to form a "forest" and an average of the predicted outcomes from each tree is used to make inference with respect to the outcome.

Biomarker expression values may be defined in combination with corresponding scalar weights on the real scale with varying magnitude, which are further combined through linear or non-linear, algebraic, trigonometric or correlative means into a single scalar value via an algebraic, statistical learning, Bayesian, regression, or similar algorithms which together with a mathematically derived decision function on the scalar value provide a predictive model by which expression profiles from samples may be resolved into discrete classes of responder or non-responder, resistant or non-resistant, to a specified drug, drug class, molecular subtype, or treatment regimen. Such predictive models, including biomarker membership, are developed by learning weights and the decision threshold, optimized for sensitivity, specificity, negative and positive predictive values, hazard ratio or any combination thereof, under cross-validation, bootstrapping or similar sampling techniques, from a set of representative expression profiles from historical patient samples with known drug response and/or resistance.

In one embodiment, the biomarkers are used to form a weighted sum of their signals, where individual weights can be positive or negative. The resulting sum ("expression score") is compared with a pre-determined reference point or value. The comparison with the reference point or value may be used to diagnose, or predict a clinical condition or outcome.

As described above, one of ordinary skill in the art will appreciate that the biomarkers included in the classifier provided in Table A and/ or Table B will carry unequal weights in a classifier. Therefore, while as few as one biomarker may be used to diagnose or predict a clinical prognosis or response to a therapeutic agent, the specificity and sensitivity or diagnosis or prediction accuracy may increase using more biomarkers.

In certain example embodiments, the expression signature is defined by a decision function. A decision function is a set of weighted expression values derived using a (linear) classifier. All linear classifiers define the decision function using the following equation:

$$f(x) = w' \bullet x + b = \sum w_i \bullet x_i + b \quad (1)$$

All measurement values, such as the microarray gene expression intensities $x_i$, for a certain sample are collected in a vector x. Each intensity is then multiplied with a corresponding weight $w_i$ to obtain the value of the decision function f(x) after adding an offset term *b.* In deriving the decision function, the linear classifier will further define a threshold value that splits the gene expression data space into two disjoint sections. Example (linear) classifiers include but are not limited to partial least squares (PLS), (Nguyen et al., Bioinformatics 18 (2002) 39-50), support vector machines (SVM) (Schölkopf et al., Learning with Kernels, MIT Press, Cambridge 2002), and shrinkage discriminant analysis (SDA) (Ahdesmäki et al., Annals of applied statistics 4, 503-519 (2010)). In one example embodiment, the (linear) classifier is a PLS linear classifier.

The decision function is empirically derived on a large set of training samples, for example from patients showing a good or poor clinical prognosis. The threshold separates a patient group based on different characteristics such as, but not limited to, clinical prognosis before or after a given therapeutic treatment. The interpretation of this quantity, i.e. the cut-off threshold, is derived in the development phase ("training") from a set of patients with known outcome. The corresponding weights and the responsiveness/resistance cut-off threshold for the decision score are fixed a *priori* from training data by methods known to those skilled in the art. In one example embodiment, Partial Least Squares Discriminant Analysis (PLS-DA) is used for determining the weights. (L. Ståhle, S. Wold, J. Chemom. 1 (1987) 185-196; D. V. Nguyen, D.M. Rocke, Bioinformatics 18 (2002) 39-50).

Effectively, this means that the data space, i.e. the set of all possible combinations of biomarker expression values, is split into two mutually exclusive groups corresponding to different clinical classifications or predictions, for example, one corresponding to good clinical prognosis and poor clinical prognosis. In the context of the overall classifier, relative over-expression of a certain biomarker can either increase the decision score (positive weight) or reduce it (negative weight) and thus contribute to an overall decision of, for example, a good clinical prognosis.

In certain example embodiments of the invention, the data is transformed non-linearly before applying a weighted sum as described above. This non-linear transformation might include increasing the dimensionality of the data. The non-linear transformation and weighted summation might also be performed implicitly, for example, through the use of a kernel function. (Schölkopf et al. Learning with Kernels, MIT Press, Cambridge 2002).

In certain example embodiments, the patient training set data is derived by isolated RNA from a corresponding cancer tissue sample set and determining expression values by hybridizing the (cDNA amplified from) isolated RNA to a microarray. In certain example embodiments, the microarray used in deriving the expression signature is a transcriptome array. As used herein a "transcriptome array" refers to a microarray containing probe sets that are designed to hybridize to sequences that have been verified as expressed in the diseased tissue of interest. Given alternative splicing and variable poly-A tail processing between tissues and biological contexts, it is possible that probes designed against the same gene sequence derived from another tissue source or biological context will not effectively bind to transcripts expressed in the diseased tissue of interest, leading to a loss of potentially relevant biological information. Accordingly, it is beneficial to verify what sequences are expressed in the disease tissue of interest before deriving a microarray probe set. Verification of expressed sequences in a particular disease context may be done, for example, by isolating and sequencing total RNA from a diseased tissue sample set and cross-referencing the isolated sequences with known nucleic acid sequence databases to verify that the probe set on the transcriptome array is designed against the sequences actually expressed in the diseased tissue of interest. Methods for making transcriptome arrays are described in United States Patent Application Publication No. 2006/0134663. In certain example embodiments, the probe set of the transcriptome array is designed to bind within 300 nucleotides of the 3' end of a transcript. Methods for designing transcriptome arrays with probe sets that bind within 300 nucleotides of the 3' end of target transcripts are disclosed in United States Patent Application Publication No. 2009/0082218. In certain example embodiments, the microarray used in deriving the gene expression profiles of the present invention is the Almac Ovarian Cancer DSA™ microarray (Almac Group, Craigavon, United Kingdom).

An optimal (linear) classifier can be selected by evaluating a (linear) classifier's performance using such diagnostics as "area under the curve" (AUC). AUC refers to the area under the curve of a receiver operating characteristic (ROC) curve, both of which are well known in the art. AUC measures are useful for comparing the accuracy of a classifier across the complete data range. (Linear) classifiers with a higher AUC have a greater capacity to classify unknowns correctly between two groups of interest (e.g., ovarian cancer samples and normal or control samples). ROC curves are useful for plotting the performance of a particular feature (e.g., any of the biomarkers described herein and/or any item of additional biomedical information) in distinguishing between two populations (e.g., individuals responding and not responding to a therapeutic agent). Typically, the feature data across the entire population (e.g., the cases and controls) are sorted in ascending order based on the value of a single feature. Then, for each value for that feature, the true positive and false positive rates for the data are calculated. The true positive rate is determined by counting the number of cases above the value for that feature and then dividing by the total number of positive cases. The false positive rate is determined by counting the number of controls above the value for that feature and then dividing by the total number of controls. Although this definition refers to scenarios in which a feature is elevated in cases compared to controls, this definition also applies to scenarios in which a feature is lower in cases compared to the controls (in such a scenario, samples below the value for that feature would be counted). ROC curves can be generated for a single feature as well as for other single outputs, for example, a combination of two or more features can be mathematically combined (e.g., added, subtracted, multiplied, etc.) to provide a single sum value, and this single sum value can be plotted in a ROC curve. Additionally, any combination of multiple features, in which the combination derives a single output value, can be plotted in a ROC curve. These combinations of features may comprise a test. The ROC curve is the plot of the true positive rate (sensitivity) of a test against the false positive rate (1-specificity) of the test.

Alternatively, an optimal classifier can be selected by evaluating performance against time-to-event endpoints using methods such as Cox proportional hazards (PH) and measures of performance across all possible thresholds assessed via the concordance-index (C-index) (Harrell, Jr. 2010). The C-Index is analagous to the "area under the curve" (AUC) metric (used for dichotomised endpoints), and it is used to measure performance with respect to association with survival data. Note that the extension of AUC to time-to-event endpoints is the C-index, with threshold selection optimised to maximise the hazard ratio (HR) under cross-validation. In this instance, the partial Cox regression algorithm (Li and Gui, 2004) was chosen for the biomarker discovery analyses. It is analogous to principal components analysis in that the first few latent components explain most of the information in the data. Implementation is as described in Ahdesmaki et al 2013.

C-index values can be generated for a single feature as well as for other single outputs, for example, a combination of two or more features can be mathematically combined (e.g., added, subtracted, multiplied, etc.) to provide a single sum value, and this single sum value can be evaluated for statistical significance. Additionally, any combination of multiple features, in which the combination derives a single output value, can be evaluated as a C-index for assessing utility for time-to-event class separation. These combinations of features may comprise a test. The C-index (Harrell, Jr. 2010, see Equation 4) of the continuous cross-validation test set risk score predictions was evaluated as the main performance measure.

In one example embodiment an expression signature is directed to the biomarkers detailed in Table A and/or Table B with corresponding ranks, and weights and associated bias detailed in the tables or alternative rankings, and weightings and bias, depending, for example, on the disease setting. The methods of the invention may rely upon measuring one or more, up to all, of the biomarkers listed in Table A and/or Table B (optionally together with one or more additional biomarkers).

The invention provides for patient selection for therapy and thus may contribute to improved outcomes in response to particular classes of therapy. Accordingly, the invention also relates to a MAPK pathway inhibitor, an EMT pathway inhibitor, an SRC pathway inhibitor, a taxane and/or a platinum-based chemotherapeutic agent for use in treating cancer in a subject, wherein the subject is selected for treatment on the basis of a method as described herein.

In yet a further related aspect, the present invention provides a therapeutic agent for use in treating cancer in a subject wherein the subject is selected for treatment by:

(i) measuring the expression level(s) of at least 1 biomarker(s), wherein the at least 1 biomarker comprises GFPT2, in a sample from the subject;
(ii) assessing from the expression level(s) of the at least 1 biomarker(s) whether the sample from the subject is positive or negative for a biomarker signature comprising the at least 1 biomarker, wherein:

(a) if the sample is positive for the biomarker signature the therapeutic agent is a MAPK pathway inhibitor; and/or
(b) if the sample is positive for the biomarker signature the therapeutic agent is an EMT pathway inhibitor; and/or
(c) if the sample is negative for the biomarker signature the therapeutic agent is an SRC pathway inhibitor; and/or
(d) if the sample is negative for the biomarker signature the therapeutic agent is a platinum-based chemotherapeutic agent; and/or
(e) if the sample is positive for the biomarker signature the therapeutic agent is a taxane.

The invention also relates to a therapeutic agent for use in treating cancer in a subject, wherein

(a) if the sample is positive for a biomarker signature comprising the expression level(s) of at least 1 biomarker(s), wherein the at least 1 biomarker comprises GFPT2, the therapeutic agent is a MAPK pathway inhibitor; and/or
(b) if the sample is positive for the biomarker signature comprising the expression level(s) of at least 1 biomarker(s), wherein the at least 1 biomarker comprises GFPT2, the therapeutic agent is an EMT pathway inhibitor; and/or
(c) if the sample is negative for the biomarker signature comprising the expression level(s) of at least 1 biomarker(s), wherein the at least 1 biomarker comprises GFPT2, the therapeutic agent is an SRC pathway inhibitor; and/or
(d) if the sample is negative for the biomarker signature comprising the expression level(s) of at least 1 biomarker(s), wherein the at least 1 biomarker comprises GFPT2, the therapeutic agent is a platinum-based chemotherapeutic agent;and/or
(e) if the sample is positive for a biomarker signature comprising the expression level(s) of at least 1 biomarker(s), wherein the at least 1 biomarker comprises GFPT2, the therapeutic agent is a taxane.

Also described herein there is a therapeutic agent for use in treating cancer in a subject, wherein

(a) the subject has been identified as having an EMT cancer and the therapeutic agent is a MAPK pathway inhibitor; and/or
(b) the subject has been identified as having an EMT cancer and the therapeutic agent is an EMT pathway inhibitor; and/or
(c) the subject has been identified as having a non-EMT cancer and the therapeutic agent is an SRC pathway inhibitor; and/or
(d) the subject has been identified as having a non-EMT cancer and the therapeutic agent is a platinum-based chemotherapeutic agent; and/or
(e) the subject has been identified as having an EMT cancer and the therapeutic agent is a taxane.

By "EMT cancer" is meant a cancer falling within the molecular subgroup identified by the present inventors, which is detectable using the biomarker signatures of the invention and described herein, based on the expression levels of one or more biomarkers from Tables A and B, wherein the one of more biomarkers comprises GFPT2. The cancer may thus display epithelial-mesenchymal transition (EMT), which may contribute to angiogenic processes and disease progression. An EMT cancer can also be termed an Angio-Immune cancer or a MAPK pathway (MEK) cancer in view of the contributing pathways to the subgroup. Genes defining the EMT/Angio-Immune/MAPK pathway molecular subgroup of cancer are listed in Tables 9 and 10 below. In Table 9 up-regulation and down-regulation are presented relative to gene expression levels in the overall sample set.

According to all aspects of the invention the therapeutic agent may be a MAPK pathway inhibitor combined with a platinum-based chemotherapeutic agent and/or an SRC pathway inhibitor.

According to all relevant aspects of the invention, the platinum-based chemotherapeutic agent and the MAPK pathway inhibitor may be administered together and/or sequentially in time in either order.

According to all aspects of the invention, a therapeutic agent may be a chemically synthesized pharmaceutical, a biologic, vaccine or small molecule. Biologics include antibodies and derivatives thereof as discussed further herein, recombinant therapeutic proteins, sugars and nucleic acids.

By "MAPK pathway inhibitor" is meant a therapeutic agent, such as a pharmaceutical drug, that inhibits signalling via the MAPK pathway. The inhibitor may be specific for the MAPK pathway. Thus, in certain embodiments the MAPK pathway inhibitor is not a multi-pathway inhibitor. In further embodiments the MAPK pathway inhibitor is a RAS/RAF/MEK/ERK pathway inhibitor. In specific embodiments the MAPK pathway inhibitor is a (specific) RAS, RAF, MEK and/or MAPK inhibitor. By MEK inhibitor is meant a therapeutic agent, such as a pharmaceutical drug, that (specifically) inhibits the mitogen-activated protein kinase kinase enzymes MEK1 and/or MEK2. In certain embodiments the MAPK pathway inhibitor is selected from Table G and/or H. In certain embodiments the MAPK pathway inhibitor (specifically) inhibits one or more of the targets listed in Table H. In specific embodiments the MAPK pathway inhibitor is trametinib. In further specific embodiments the MAPK pathway inhibitor is selumetinib (synonyms: AZD6244 and ARRY-142886).

**Table G - MAPK pathway inhibitors**

| **Preclinical - Phase I** | |
|---|---|
| **DRUG NAME** | **COMPANY** |
| BAL-3833 | Basilea Pharmaceutica |
| BGB-283 | Merck KGaA |
| HM-95573 | Hanmi Pharmaceuticals |
| LY-3009120 | Eli Lilly |
| RG-7304 | Roche |
| RG-7842 | Genentech |
| Salirasib | Ono Pharmaceutical |
| AEZS-136 | Aeterna Zentaris Inc. |
| ARI-4175 | Arisaph Pharmaceuticals |
| ASN-003 | Asana BioSciences |
| CCT-196969 | Basilea Pharmaceutica |
| CCT-241161 | Basilea Pharmaceutica |
| CS-410 | Chipscreen Biosciences |
| MAP4K4 Inhibitor (small molecule) for Cancer | Genentech, Inc. |
| pan-RAF Kinase inhibitor (small molecule) for Oncology | Novartis AG |
| CT-207 | HEC Pharm Co., Ltd. |
| CT-317 | HEC Pharm Co., Ltd. |
| B-Raf Kinase inhibitor for Cancer | Ruga Corporation |
| EBI-907 | Eternity Bioscience Inc. |
| EBI-945 | Eternity Bioscience Inc. |
| KO-947 | Kura Oncology, Inc. |
| LXH-254 | Novartis AG |
| MDC-1016 | Medicon Pharmaceuticals, Inc |
| MT-477 | Medisyn Technologies, Inc. |
| NCB-0594 | Carna Biosciences, Inc. |
| NCB-0846 | Carna Biosciences, Inc. |
| NMSP-285 | Nerviano Medical Sciences S.r.l. |

(continued)

| Preclinical - Phase I | |
|---|---|
| **DRUG NAME** | **COMPANY** |
| ON-108600 | Onconova Therapeutics, Inc. |
| PV-103 | PepVax, Inc. |
| RX-8243 | Rexahn Pharmaceuticals, Inc. |
| STP-503 | Sirnaomics, Inc. |
| Raf Kinases inhibitor (small molecule) for Cancer | Amitech Therapeutic Solutions, Inc. |
| TAK-632 | Takeda Pharmaceutical Company Limited |
| TEW-0201 | MedPacto, Inc. |
| AIK-4 | Allinky Biopharma |
| AR-00457679 | Array BioPharma Inc. |
| CB-745 | AGV Discovery, SAS |
| HD-001 | AstraZeneca Plc |
| SCH-722984 | Merck & Co., Inc. |
| K-RAS inhibitor (small molecule) for Oncology | Aurigene Discovery Technologies Limited |
| B-RAF Kinase inhibitor (small molecule) for Oncology | Sareum Holdings Plc |
| ERK2 and Aurora B Kinase inhibitor (small molecule) for Cancer | Aeterna Zentaris Inc. |
| ARQ-736 | ArQule, Inc. |
| K-Ras inhibitor (small molecule) for Oncology | Boehringer Ingelheim GmbH |
| KRAS inhibitor (small molecule) for Cancer | NantBioScience |
| KRas inhibitor (small molecule) for Solid Tumor | Nimbus Therapeutics, LLC |
| Pan-Raf Kinases inhibitor (small molecule) for Colorectal Cancer | Redx Pharma Plc |
| TNIK inhibitor (small molecule) for Oncology | Astex Pharmaceuticals, Inc. |
| KRAS inhibitor (synthetic peptide) for Oncology | PeptiDream Inc. |
| K-Ras inhibitor (small molecule) for Cancer | Araxes Pharma LLC |

(continued)

| Phase II, Phase III, Marketed | | |
|---|---|---|
| **DRUG NAME** | **COMPANY NAME** | **DISEASE INDICATION** |
| vemurafenib (Zelboraf) | Roche | Marketed: mMelanoma Phase II: Bile Duct, Bladder, CLL, GI, Leukemia, Ovarian, Prostate, Sarcomas, Thyroid |
| regorafenib (Stivarga) | Bayer | Marketed: GIST, mCRC PhIll: HCC PhII: Bile Duct, Ovarian, Pancreatic, RCC, Salivary Gland, Soft Tissue Sarcoma; Bladder |
| dabrafenib (Tafinlar) | Novartis | Marketed: mMelanoma PhII: Thyroid, CRC, NSCLC, (GIST), Glioma, Leukemia, Brain, Multiple Myeloma, Germ Cell Tumors |
| RAF-265 | Novartis | PhII: mMelanoma |
| Encorafenib | Array Biopharma | PhIll: mMelanoma PhII: mCRC |
| Donafenib | Suzhou Zelgen Biopharmaceutical | PhII: Esophageal, GI, HCC, mCRC |
| NEO-100 | Neonc Technologies | PhII: Recurrent Glioblastoma Multiforme (GBM) Preclinical: Lung |
| PLX-8394 | Plexxikon | phII: Thyroid, Bile Duct, CRC, Melanoma, NSCLC phI: Leukemias |
| RXDX-105 | Ingnyta | PhII: Colon Carcinoma, Melanoma, mCRC |
| TAK-580 | Millennium Pharmaceuticals | PhII: Metastatic Melanoma; Solid Tumor PhI: Nonhematologic Malignancy |
| Ulixertinib | BioMed Valley Discoveries | PhII: AML, CRC, Melanoma, Myelodysplastic Syndrome; NSCLC Preclinical: Metastatic Adenocarcinoma of The Pancreas; Pancreatic |

## TABLE H – MAPK pathway inhibitors data

| **Mechanism Of Action:** | Ras Inhibitor;Raf Kinase Inhibitor;A-Raf Kinase Inhibitor;B-Raf Kinase Inhibitor;C-Raf Kinase Inhibitor;TRAF2 and NCK-Interacting Protein Kinase (TNIK) Inhibitor;Mitogen Activated Protein Kinase Kinase Kinase (cMos or cRaf or MAPKKK or MAP3K or MAP Kinase Kinase Kinase or EC 2.7.11.25) Inhibitor;Mitogen Activated Protein Kinase 3 (MAP Kinase 3 or MAPK 3 or Extracellular Signal Regulated Kinase 1 or ERK-1) Inhibitor;Mitogen-Activated Protein Kinase Kinase Kinase Kinase 2 (Germinal Center Kinase or MAPK/ERK Kinase Kinase Kinase 2) Inhibitor;Mitogen Activated Protein Kinase 1 (MAP Kinase 1 or MAPK 1 or Extracellular Signal Regulated Kinase 2 or ERK-2 or Protein Tyrosine Kinase ERK2 or EC 2.7.11.24) Inhibitor;Mitogen Activated Protein Kinase Kinase Kinase 5 (Apoptosis Signal Regulating Kinase 1 or MAPK/ERK Kinase Kinase 5 or MEK Kinase 5 or ASK-1 or MAP3K5 or EC 2.7.11.25) Inhibitor;Mitogen-Activated Protein Kinase Kinase Kinase Kinase 4 (HPK/GCK-Like Kinase HGK or MAPK/ERK Kinase Kinase Kinase 4 or MEK Kinase Kinase 4 or MEKKK 4 or MAP4K4 or EC 2.7.11.1) Inhibitor | | | | | | | |
|---|---|---|---|---|---|---|---|---|
| **Drug Name** | **Generic Name** | **Brand Name** | **Company** | **Therapy Area** | **Indications** | **Product Stage** | **Product Geography** | **MOA** |
| vemurafenib | vemurafenib | Zelboraf | Chugai Pharmaceutical Co., Ltd. | Oncology | Metastatic Melanoma | Marketed | Japan | B-Raf Kinase Inhibitor |
| vemurafenib | vemurafenib | Zelboraf | F. Hoffmann-La Roche Ltd. | Oncology | Metastatic Melanoma | Marketed | Brazil | B-Raf Kinase Inhibitor |
| vemurafenib | vemurafenib | Zelboraf | Hoffmann-La Roche Inc. | Oncology | Metastatic Melanoma | Marketed | Canada; United States | B-Raf Kinase Inhibitor |
| vemurafenib | vemurafenib | Zelboraf | Roche Korea Co. Ltd. | Oncology | Metastatic Melanoma | Marketed | South Korea | B-Raf Kinase Inhibitor |

| | | | | | | | | |
|---|---|---|---|---|---|---|---|---|
| vemurafenib | vemurafenib | Zelboraf | Roche Pharma AG | Oncology | Metastatic Melanoma | Marketed | Germany | B-Raf Kinase Inhibitor |
| vemurafenib | vemurafenib | Zelboraf | Roche Products (Pty) Ltd | Oncology | Metastatic Melanoma | Marketed | Australia | B-Raf Kinase Inhibitor |
| vemurafenib | vemurafenib | Zelboraf | Roche Products Limited | Oncology | Metastatic Melanoma | Marketed | United Kingdom | B-Raf Kinase Inhibitor |
| vemurafenib | vemurafenib | Zelboraf | Roche Registration Ltd | Oncology | Metastatic Melanoma | Marketed | EU; France; Spain | B-Raf Kinase Inhibitor |
| vemurafenib | vemurafenib | Zelboraf | Roche S.p.A. | Oncology | Metastatic Melanoma | Marketed | Italy | B-Raf Kinase Inhibitor |
| vemurafenib | vemurafenib | Zelboraf | F. Hoffmann-La Roche Ltd. | Oncology | Metastatic Melanoma | Phase III | EU; United States | B-Raf Kinase Inhibitor |
| vemurafenib | vemurafenib | Zelboraf | F. Hoffmann-La Roche Ltd. | Oncology | Bile Duct Cancer (Cholangiocarcinoma) ; Bladder Cancer; Chronic Lymphocytic Leukemia (CLL); Gastrointestinal Stromal Tumor (GIST); Hairy Cell Leukemia; Ovarian Cancer; Prostate Cancer; Sarcomas; Thyroid Cancer | Phase II | Global | B-Raf Kinase Inhibitor |
| vemurafenib | vemurafenib | Zelboraf | F. Hoffmann-La Roche Ltd. | Oncology | Colorectal Cancer | Phase II | EU; United States | B-Raf Kinase Inhibitor |
| vemurafenib | vemurafeni | Zelboraf | F. Hoffmann- | Oncolog | Non-Small Cell Lung | Phase II | Global | B-Raf Kinase Inhibitor |

| | | | | | | | | |
|---|---|---|---|---|---|---|---|---|
| | b | | La Roche Ltd. | y | Cancer; Refractory Multiple Myeloma; Relapsed Multiple Myeloma | | | |
| vemurafenib | vemurafenib | Zelboraf | F. Hoffmann-La Roche Ltd. | Oncology | Papillary Thyroid Cancer | Discontinued | EU; United States | B-Raf Kinase Inhibitor |
| regorafenib | regorafenib | Stivarga | Bayer (Pty) Ltd. | Oncology | Gastrointestinal Stromal Tumor (GIST); Metastatic Colorectal Cancer | Marketed | South Africa | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regorafenib | regorafenib | Stivarga | Bayer Australia Ltd. | Oncology | Gastrointestinal Stromal Tumor (GIST); Metastatic Colorectal Cancer | Marketed | Australia | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 |

| | | | | | | | | |
|---|---|---|---|---|---|---|---|---|
| | | | | | | | | (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regorafenib | regorafenib | Stivarga | Bayer Corporation | Oncology | Gastrointestinal Stromal Tumor (GIST); Metastatic Colorectal Cancer | Marketed | United States | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; |

| | | | | | | | | |
|---|---|---|---|---|---|---|---|---|
| | | | | | | | | Vascular Endothelial Growth Factor Receptor-3 (VEGFR-3) Antagonist |
| regorafenib | regorafenib | Stivarga | Bayer HealthCare AG | Oncology | Gastrointestinal Stromal Tumor (GIST); Metastatic Colorectal Cancer | Marketed | EU | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regorafenib | regorafenib | Stivarga | Bayer Hispania SL | Oncology | Gastrointestinal Stromal Tumor (GIST); Metastatic Colorectal Cancer | Marketed | Spain | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) |

| | | | | | | | | |
|---|---|---|---|---|---|---|---|---|
| | | | | | | | | Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regora fenib | regor afenib | Stivarga | Bayer Inc. | Onc olog y | Gastrointesti nal Stromal Tumor (GIST); Metastatic Colorectal Cancer | Mark eted | Canada | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regora fenib | regor afenib | Stivarga | Bayer Korea Ltd. | Onc olog y | Gastrointesti nal Stromal Tumor (GIST); Metastatic Colorectal | Mark eted | South Korea | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; |

| | | | | | | | | |
|---|---|---|---|---|---|---|---|---|
| | | | | | Cancer | | | Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regora fenib | regor afenib | Stivarga | Bayer S.p.A | Onc olog y | Gastrointesti nal Stromal Tumor (GIST); Metastatic Colorectal Cancer | Mark eted | Italy | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor |

| | | | | | | | | |
|---|---|---|---|---|---|---|---|---|
| | | | | | | | | Receptor-2 (VEGFR-2) Antagonist; Vascular Endothelial Growth Factor Receptor-3 (VEGFR-3) Antagonist |
| regorafenib | regorafenib | Stivarga | Bayer Sante SAS | Oncology | Gastrointestinal Stromal Tumor (GIST); Metastatic Colorectal Cancer | Marketed | France | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regorafenib | regorafenib | Stivarga | Bayer UK Limited | Oncology | Gastrointestinal Stromal Tumor (GIST); Metastatic Colorectal Cancer | Marketed | United Kingdom | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth |

| | | | | | | | | |
|---|---|---|---|---|---|---|---|---|
| | | | | | | | | Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regora fenib | regor afenib | Stivarga | Bayer Yakuhin, Ltd. | Onc olog y | Gastrointesti nal Stromal Tumor (GIST); Metastatic Colorectal Cancer | Mark eted | Japan | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regora fenib | regor afenib | Stivarga | Bayern Internation al GmbH | Onc olog y | Gastrointesti nal Stromal Tumor (GIST); Metastatic | Mark eted | German y | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, |

| | | | | | | | | |
|---|---|---|---|---|---|---|---|---|
| | | | | | Colorectal Cancer | | | Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regorafenib | regorafenib | | Bayer AG | Oncology | Hepatocellular Carcinoma | Phase III | Global | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; |

| | | | | | | | | |
|---|---|---|---|---|---|---|---|---|
| | | | | | | | | Vascular Endothelial Growth Factor Receptor-2 (VEGFR-2) Antagonist; Vascular Endothelial Growth Factor Receptor-3 (VEGFR-3) Antagonist |
| regorafenib | regorafenib | | Bayer AG | Oncology | Metastatic Colorectal Cancer | Phase III | Asia-Pacific; Global | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regorafenib | regorafenib | | Bayer AG | Oncology | Bile Duct Cancer (Cholangiocarcinoma) ; Epithelial Ovarian Cancer; Fallopian Tube Cancer; Metastatic Colorectal Cancer; Pancreatic Cancer; | Phase II | Global | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) |

| | | | | | | | | |
|---|---|---|---|---|---|---|---|---|
| | | | | | Peritoneal Cancer; Renal Cell Carcinoma; Salivary Gland Cancer; Soft Tissue Sarcoma; Transitional Cell Cancer (Urothelial Cell Cancer) | | | Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regora fenib | regor afenib | | Bayer AG | Onc olog y | Non-Small Cell Lung Cancer | Inact ive | Global | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regora fenib | regor afenib | | Bayer AG | Onc olog y | Solid Tumor | Inact ive | China; Japan | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or |

| | | | | | | | | |
|---|---|---|---|---|---|---|---|---|
| | | | | | | | | Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| regorafenib | regorafenib | | Bayer AG | Ophthalmology | Wet (Neovascular / Exudative) Macular Degeneration | Discontinued | Global | Abl Tyrosine Kinase Inhibitor; B-Raf Kinase Inhibitor; c-kit Receptor (SCFR, CD117) Antagonist; C-Raf Kinase Inhibitor; CD167b (Discoidin Domain-Containing Receptor 2 or Neurotrophic Tyrosine Kinase, Receptor-Related 3) Inhibitor; Fibroblast Growth Factor Receptor 1 (FGFR1) Antagonist; Fibroblast Growth Factor Receptor 2 (Keratinocyte Growth Factor Receptor or CD332 or FGFR2 or KGFR or K-sam or EC 2.7.10.1) Antagonist; Platelet Derived Growth Factor Receptor Alpha (PDGFR-Alpha) Antagonist; Platelet Derived Growth Factor Receptor Beta (PDGFR-Beta) Antagonist; Proto-Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Tie-2 Receptor Antagonist; Vascular Endothelial Growth Factor |

| | | | | | | | | |
|---|---|---|---|---|---|---|---|---|
| | | | | | | | | Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| dabrafenib mesylate | dabrafenib mesylate | Tafinlar | Novartis Corporation | Oncology | Metastatic Melanoma | Marketed | United States | B-Raf Kinase Inhibitor |
| dabrafenib mesylate | dabrafenib mesylate | Tafinlar | Novartis Europharm Ltd. | Oncology | Metastatic Melanoma | Marketed | United Kingdom | B-Raf Kinase Inhibitor |
| dabrafenib mesylate | dabrafenib mesylate | Tafinlar | Novartis Farma S.p.A | Oncology | Metastatic Melanoma | Marketed | Italy | B-Raf Kinase Inhibitor |
| dabrafenib mesylate | dabrafenib mesylate | Tafinlar | Novartis Farmaceutica, SA | Oncology | Metastatic Melanoma | Marketed | Spain | B-Raf Kinase Inhibitor |
| dabrafenib mesylate | dabrafenib mesylate | Tafinlar | Novartis Pharma GmbH | Oncology | Metastatic Melanoma | Marketed | Germany | B-Raf Kinase Inhibitor |
| dabrafenib mesylate | dabrafenib mesylate | Tafinlar | Novartis Pharma S.A.S. | Oncology | Metastatic Melanoma | Marketed | France | B-Raf Kinase Inhibitor |
| dabrafenib mesylate | dabrafenib mesylate | Tafinlar | Novartis Pharmaceuticals Australia Pty Limited | Oncology | Metastatic Melanoma | Marketed | Australia | B-Raf Kinase Inhibitor |
| dabrafenib mesylate | dabrafenib mesylate | Tafinlar | Novartis Pharmaceuticals Canada Inc. | Oncology | Metastatic Melanoma | Marketed | Canada | B-Raf Kinase Inhibitor |
| dabrafenib mesylate | dabrafenib mesylate | Tafinlar | Novartis Pharmaceuticals UK Limited | Oncology | Metastatic Melanoma | Marketed | EU | B-Raf Kinase Inhibitor |
| dabrafenib mesylate | dabrafenib mesylate | | Novartis AG | Oncology | Metastatic Melanoma | Phase III | Global | B-Raf Kinase Inhibitor |

| | | | | | | | | |
|---|---|---|---|---|---|---|---|---|
| dabrafenib mesylate | dabrafenib mesylate | | Novartis AG | Oncology | Follicular Thyroid Cancer; Papillary Thyroid Cancer | Phase II | Global | B-Raf Kinase Inhibitor |
| dabrafenib mesylate | dabrafenib mesylate | Tafinlar | Novartis AG | Oncology | Metastatic Colorectal Cancer; Non-Small Cell Lung Cancer | Phase II | Global | B-Raf Kinase Inhibitor |
| dabrafenib mesylate + trametinib dimethyl sulfoxide | dabrafenib mesylate + trametinib dimethyl sulfoxide | Tafinlar + Mekinist | Novartis Corporation | Oncology | Metastatic Melanoma | Marketed | United States | B-Raf Kinase Inhibitor; Mitogen Activated Protein Kinase Kinase 1 (MEK-1 or MAP2K1) Inhibitor; Mitogen Activated Protein Kinase Kinase 2 (MEK-2 or MAP2K2) Inhibitor |
| dabrafenib mesylate + trametinib dimethyl sulfoxide | dabrafenib mesylate + trametinib dimethyl sulfoxide | Tafinlar + Mekinist | Novartis Europharm Ltd. | Oncology | Metastatic Melanoma | Marketed | EU | B-Raf Kinase Inhibitor; Mitogen Activated Protein Kinase Kinase 1 (MEK-1 or MAP2K1) Inhibitor; Mitogen Activated Protein Kinase Kinase 2 (MEK-2 or MAP2K2) Inhibitor |
| dabrafenib mesylate + trametinib dimethyl sulfoxide | dabrafenib mesylate + trametinib dimethyl sulfoxide | Sulfoxide | Novartis AG | Oncology | Metastatic Melanoma | Pre-Registration | Japan | B-Raf Kinase Inhibitor; Mitogen Activated Protein Kinase Kinase 1 (MEK-1 or MAP2K1) Inhibitor; Mitogen Activated Protein Kinase Kinase 2 (MEK-2 or MAP2K2) Inhibitor |
| dabrafenib mesylate + trametinib | dabrafenib mesylate + trametinib | Sulfoxide | Novartis AG | Oncology | Melanoma; Metastatic Melanoma | Phase III | Global | B-Raf Kinase Inhibitor; Mitogen Activated Protein Kinase Kinase 1 (MEK-1 or MAP2K1) Inhibitor; Mitogen Activated Protein Kinase Kinase 2 (MEK-2 or MAP2K2) Inhibitor |

| | | | | | | | | |
|---|---|---|---|---|---|---|---|---|
| dimethyl sulfoxide | dimethyl sulfoxide | | | | | | | |
| dabrafenib mesylate + trametinib dimethyl sulfoxide | dabrafenib mesylate + trametinib dimethyl sulfoxide | Sulfoxide | Novartis AG | Oncology | Acral Lentiginous Melanoma; Adenocarcinoma; Anaplastic Thyroid Cancer; Biliary Tumor; Colorectal Cancer; Gastrointestinal Stromal Tumor (GIST); Glioma; Hairy Cell Leukemia; High-Grade Glioma; Metastatic Brain Tumor; Multiple Myeloma (Kahler Disease); Non-Small Cell Lung Cancer; Nongerminomatous (Nonseminomatous) Germ Cell Tumors | Phase II | Global | B-Raf Kinase Inhibitor; Mitogen Activated Protein Kinase Kinase 1 (MEK-1 or MAP2K1) Inhibitor; Mitogen Activated Protein Kinase Kinase 2 (MEK-2 or MAP2K2) Inhibitor |
| dabrafenib mesylate + trametinib dimeth | dabrafenib mesylate + trametinib dimet | Sulfoxide | Novartis AG | Oncology | Solid Tumor | Phase II | Japan | B-Raf Kinase Inhibitor; Mitogen Activated Protein Kinase Kinase 1 (MEK-1 or MAP2K1) Inhibitor; Mitogen Activated Protein Kinase Kinase 2 (MEK-2 or MAP2K2) Inhibitor |

| | | | | | | | | |
|---|---|---|---|---|---|---|---|---|
| yl sulfoxide | hyl sulfoxide | | | | | | | |
| sorafenib tosylate | sorafenib tosylate | Sorafenib Tosylate | Cipla Ltd. | Oncology | Renal Cell Carcinoma | Marketed | India | B-Raf Kinase Inhibitor; c-kit Receptor (SCFR, CD117) Antagonist; C-Raf Kinase Inhibitor; Platelet Derived Growth Factor Receptor Beta (PDGFR-Beta) Antagonist; Receptor-Type Tyrosine-Protein Kinase FLT3 (FMS-Like Tyrosine Kinase 3 or FL Cytokine Receptor or Stem Cell Tyrosine Kinase 1 or CD135 or Fetal Liver Kinase-2 or EC 2.7.10.1) Antagonist; Vascular Endothelial Growth Factor Receptor-2 (VEGFR-2) Antagonist; Vascular Endothelial Growth Factor Receptor-3 (VEGFR-3) Antagonist |
| binimetinib + encorafenib | binimetinib + encorafenib | | Array BioPharma Inc. | Oncology | Metastatic Melanoma | Phase III | Global | B-Raf Kinase Inhibitor; Mitogen Activated Protein Kinase Kinase 1 (MEK-1 or MAP2K1) Inhibitor; Mitogen Activated Protein Kinase Kinase 2 (MEK-2 or MAP2K2) Inhibitor |
| binimetinib + encorafenib | binimetinib + encorafenib | | Array BioPharma Inc. | Oncology | Metastatic Colorectal Cancer; Solid Tumor | Phase II | Global | B-Raf Kinase Inhibitor; Mitogen Activated Protein Kinase Kinase 1 (MEK-1 or MAP2K1) Inhibitor; Mitogen Activated Protein Kinase Kinase 2 (MEK-2 or MAP2K2) Inhibitor |
| encorafenib | encorafenib | | Array BioPharma Inc. | Oncology | Metastatic Melanoma | Phase III | Global | B-Raf Kinase Inhibitor |
| encorafenib | encorafenib | | Array BioPharma Inc. | Oncology | Hematological Tumor; Metastatic Colorectal Cancer; Solid Tumor | Phase II | Global | B-Raf Kinase Inhibitor |
| encorafenib | encorafenib | | Array BioPharma Inc. | Oncology | Thyroid Cancer | Unknown | Global | B-Raf Kinase Inhibitor |
| encorafenib | encorafenib | | Array BioPharma Inc. | Oncology | Non-Small Cell Lung Cancer | Inactive | Global | B-Raf Kinase Inhibitor |
| dabrafenib | dabrafenib | | GlaxoSmithKline Plc | Oncolog | Colorectal Cancer | Phase II | Global | B-Raf Kinase Inhibitor; Epidermal Growth Factor Receptor (EGFR, |

| | | | | | | | | |
|---|---|---|---|---|---|---|---|---|
| mesylate + panitumumab + trametinib dimethyl sulfoxide | mesylate + panitumumab + trametinib dimethyl sulfoxide | | | y | | | | HER-1 or ErbB-1) Antagonist; Mitogen Activated Protein Kinase Kinase 1 (MEK-1 or MAP2K1) Inhibitor; Mitogen Activated Protein Kinase Kinase 2 (MEK-2 or MAP2K2) Inhibitor |
| donafenib | | | Suzhou Zelgen Biopharmaceutical Co., Ltd. | Oncology | Esophageal Cancer; Gastric Cancer; Hepatocellular Carcinoma; Metastatic Colorectal Cancer | Phase II | Global | Raf Kinase Inhibitor; Receptor Tyrosine Kinase Inhibitor |
| NEO-100 | | | Neonc Technologies, Inc. | Oncology | Recurrent Glioblastoma Multiforme (GBM) | Phase II | Global | G Protein-Coupled Receptor 78 (GPR78) Agonist; Ras Inhibitor |
| NEO-100 | | | Neonc Technologies, Inc. | Oncology | Lung Cancer | Preclinical | Global | G Protein-Coupled Receptor 78 (GPR78) Agonist; Ras Inhibitor |
| PLX-8394 | | | Plexxikon Inc. | Oncology | Anaplastic Thyroid Cancer; Bile Duct Cancer (Cholangiocarcinoma) ; Colorectal Cancer; Melanoma; Non-Small Cell Lung Cancer; Papillary Thyroid Cancer; Solid Tumor | Phase II | United States | B-Raf Kinase Inhibitor |
| PLX-8394 | | | Plexxikon Inc. | Oncology | Leukemias | Phase I | United States | B-Raf Kinase Inhibitor |
| PLX- | | | Plexxikon | Onc | Hairy Cell | Inact | United | B-Raf Kinase Inhibitor |

| | | | | | | | | |
|---|---|---|---|---|---|---|---|---|
| 8394 | | | Inc. | ology | Leukemia | ive | States | |
| RAF-265 | | | Novartis AG | Oncology | Metastatic Melanoma | Phase II | Global | B-Raf Kinase Inhibitor; C-Raf Kinase Inhibitor; Vascular Endothelial Growth Factor Receptor-2 (VEGFR-2) Antagonist |
| RAF-265 | | | Novartis AG | Oncology | Solid Tumor | Inactive | Global | B-Raf Kinase Inhibitor; C-Raf Kinase Inhibitor; Vascular Endothelial Growth Factor Receptor-2 (VEGFR-2) Antagonist |
| RXDX-105 | | | Ignyta, Inc. | Oncology | Colon Carcinoma; Melanoma; Metastatic Colorectal Cancer; Solid Tumor | Phase II | Global | B-Raf Kinase Inhibitor; Cytotoxic To Cells Expressing Epidermal Growth Factor Receptor (EGFR, HER-1 or ErbB-1 or Proto-Oncogene c-ErbB-1 or Receptor Tyrosine-Protein Kinase erbB-1); Proto-Oncogene Tyrosine-Protein Kinase ROS (Proto-Oncogene c-Ros-1 or Receptor Tyrosine Kinase c-Ros Oncogene 1 or c-Ros Receptor Tyrosine Kinase or EC 2.7.10.1) Inhibitor |
| TAK-580 | | | Millennium Pharmaceuticals, Inc. | Oncology | Metastatic Melanoma; Solid Tumor | Phase II | Global | A-Raf Kinase Inhibitor; B-Raf Kinase Inhibitor; C-Raf Kinase Inhibitor |
| TAK-580 | | | Millennium Pharmaceuticals, Inc. | Oncology | Nonhematologic Malignancy | Phase I | Global | A-Raf Kinase Inhibitor; B-Raf Kinase Inhibitor; C-Raf Kinase Inhibitor |
| ulixertinib | ulixertinib [INN] | | BioMed Valley Discoveries, Inc | Oncology | Acute Myelocytic Leukemia (AML, Acute Myeloblastic Leukemia); Advanced Malignancy; Colorectal Cancer; Melanoma; Metastatic Cancer; Myelodysplastic Syndrome; Non-Small Cell Lung Cancer | Phase II | Global | Mitogen Activated Protein Kinase 1 (MAP Kinase 1 or MAPK 1 or Extracellular Signal Regulated Kinase 2 or ERK-2 or Protein Tyrosine Kinase ERK2 or EC 2.7.11.24) Inhibitor; Mitogen Activated Protein Kinase 3 (MAP Kinase 3 or MAPK 3 or Extracellular Signal Regulated Kinase 1 or ERK-1) Inhibitor |
| ulixerti | ulixert | | BioMed | Onc | Metastatic | Prec | Global | Mitogen Activated Protein Kinase 1 |

| | | | | | | | | |
|---|---|---|---|---|---|---|---|---|
| nib | inib [INN] | | Valley Discoveries, Inc | ology | Adenocarcinoma of The Pancreas; Metastatic Pancreatic Cancer; Pancreatic Cancer | linical | | (MAP Kinase 1 or MAPK 1 or Extracellular Signal Regulated Kinase 2 or ERK-2 or Protein Tyrosine Kinase ERK2 or EC 2.7.11.24) Inhibitor; Mitogen Activated Protein Kinase 3 (MAP Kinase 3 or MAPK 3 or Extracellular Signal Regulated Kinase 1 or ERK-1) Inhibitor |
| BAL-3833 | | | Basilea Pharmaceutica AG | Oncology | Metastatic Melanoma | Phase I | Global | A-Raf Kinase Inhibitor; B-Raf Kinase Inhibitor; C-Raf Kinase Inhibitor |
| BGB-283 | | | BeiGene(Beijing) Co.,Ltd | Oncology | Colorectal Cancer; Endometrial Cancer; Melanoma; Non-Small Cell Lung Cancer; Solid Tumor; Thyroid Cancer | Phase I | China | B-Raf Kinase Inhibitor; Epidermal Growth Factor Receptor (EGFR, HER-1 or ErbB-1) Antagonist |
| BGB-283 | | | Merck KGaA | Oncology | Solid Tumor | Phase I | Global | B-Raf Kinase Inhibitor; Epidermal Growth Factor Receptor (EGFR, HER-1 or ErbB-1) Antagonist |
| dabrafenib mesylate + durvalumab + trametinib dimethyl sulfoxide | dabrafenib mesylate + durvalumab + trametinib dimethyl sulfoxide | | AstraZeneca Plc | Oncology | Metastatic Melanoma | Phase I | Global | B-Raf Kinase Inhibitor; CD274 (Programmed Cell Death 1-Ligand 1 or PDL-1) Inhibitor; Mitogen Activated Protein Kinase Kinase 1 (MEK-1 or MAP2K1) Inhibitor; Mitogen Activated Protein Kinase Kinase 2 (MEK-2 or MAP2K2) Inhibitor |
| HM-95573 | | | Hanmi Pharmaceuticals, Co. Ltd. | Oncology | Melanoma; Solid Tumor | Phase I | South Korea | Raf Kinase Inhibitor |
| hydroxychloroquine + sorafe | hydroxychloroquine [INN] | | VG Life Sciences, Inc. | Oncology | Breast Cancer; Lung Adenocarcinoma; | Phase I | Global | B-Raf Kinase Inhibitor; c-kit Receptor (SCFR, CD117) Antagonist; C-Raf Kinase Inhibitor; Platelet Derived Growth Factor Receptor Beta (PDGFR-Beta) Antagonist; Proto- |

| | | | | | | | | |
|---|---|---|---|---|---|---|---|---|
| nib tosylate | + sorafenib tosylate | | | | Metastatic Ovarian Cancer; Solid Tumor | | | Oncogene Tyrosine-Protein Kinase Receptor Ret (RET Receptor Tyrosine Kinase or Cadherin Family Member 12 or Proto-Oncogene c-Ret or EC 2.7.10.1) Inhibitor; Receptor-Type Tyrosine-Protein Kinase FLT3 (FMS-Like Tyrosine Kinase 3 or FL Cytokine Receptor or Stem Cell Tyrosine Kinase 1 or CD135 or Fetal Liver Kinase-2 or EC 2.7.10.1) Antagonist; Vascular Endothelial Growth Factor Receptor-1 (VEGFR-1) Antagonist; Vascular Endothelial Growth Factor Receptor-2 (VEGFR-2) Antagonist; Vascular Endothelial Growth Factor Receptor-3 (VEGFR-3) Antagonist |
| LY-3009120 | | | Eli Lilly and Company | Oncology | Colorectal Cancer; Malignant Neoplasms; Metastatic Melanoma; Non-Small Cell Lung Cancer | Phase I | Global | A-Raf Kinase Inhibitor; B-Raf Kinase Inhibitor; C-Raf Kinase Inhibitor |
| RG-7304 | | | Chugai Pharmaceutical Co., Ltd. | Oncology | Multiple Myeloma (Kahler Disease); Solid Tumor | Phase I | Japan | B-Raf Kinase Inhibitor; C-Raf Kinase Inhibitor; Mitogen Activated Protein Kinase Kinase 1 (MEK-1 or MAP2K1) Inhibitor |
| RG-7304 | | | F. Hoffmann-La Roche Ltd. | Oncology | Melanoma; Non-Small Cell Lung Cancer; Pancreatic Cancer | Phase I | Global | B-Raf Kinase Inhibitor; C-Raf Kinase Inhibitor; Mitogen Activated Protein Kinase Kinase 1 (MEK-1 or MAP2K1) Inhibitor |
| RG-7842 | | | Genentech, Inc. | Oncology | Solid Tumor | Phase I | Global | Mitogen Activated Protein Kinase 1 (MAP Kinase 1 or MAPK 1 or Extracellular Signal Regulated Kinase 2 or ERK-2 or Protein Tyrosine Kinase ERK2 or EC 2.7.11.24) Inhibitor; Mitogen Activated Protein Kinase 3 (MAP Kinase 3 or MAPK 3 or Extracellular Signal Regulated Kinase 1 or ERK-1) Inhibitor |
| salirasib | salirasib | | Ono Pharmaceutical Co., | Oncology | Pancreatic Cancer; Solid Tumor | Phase I | Japan | Ras Inhibitor |

| | | | | | | | | |
|---|---|---|---|---|---|---|---|---|
| | | | Ltd. | | | | | |
| salirasib | salirasib | | Kadmon Corporation, LLC | Oncology | Colorectal Cancer; Non-Small Cell Lung Cancer; Pancreatic Cancer | Inactive | Global | Ras Inhibitor |
| AEZS-136 | | | Aeterna Zentaris Inc. | Oncology | Solid Tumor | Preclinical | Global | Mitogen Activated Protein Kinase 1 (MAP Kinase 1 or MAPK 1 or Extracellular Signal Regulated Kinase 2 or ERK-2 or Protein Tyrosine Kinase ERK2 or EC 2.7.11.24) Inhibitor; Mitogen Activated Protein Kinase 3 (MAP Kinase 3 or MAPK 3 or Extracellular Signal Regulated Kinase 1 or ERK-1) Inhibitor; Phosphatidylinositol 3-Kinase (PI3K) Inhibitor |
| ARI-4175 | | | Arisaph Pharmaceuticals, Inc. | Oncology | Colorectal Cancer; Metastatic Melanoma; Sarcomas | Preclinical | Global | B-Raf Kinase Inhibitor; Dipeptidyl Peptidase-4 (DPP-4) Inhibitor |
| ASN-003 | | | Asana BioSciences, LLC | Oncology | Melanoma | Preclinical | Global | B-Raf Kinase Inhibitor |
| CCT-196969 | | | Basilea Pharmaceutica AG | Oncology | Melanoma | Preclinical | Global | B-Raf Kinase Inhibitor; c-Src Tyrosine Kinase Inhibitor; Lck Tyrosine Kinase Inhibitor; Mitogen Activated Protein Kinase Kinase (MEK or MAP2K) Inhibitor; Mitogen Activated Protein Kinase Kinase Kinase (cMos or cRaf or MAPKKK or MAP3K or MAP Kinase Kinase Kinase or EC 2.7.11.25) Inhibitor; p38 MAP Kinase Inhibitor; Tyrosine-Protein Kinase Mer (Proto-Oncogene c-Mer) Antagonist |
| CCT-241161 | | | Basilea Pharmaceutica AG | Oncology | Melanoma | Preclinical | Global | B-Raf Kinase Inhibitor; c-Src Tyrosine Kinase Inhibitor; Lck Tyrosine Kinase Inhibitor; Mitogen Activated Protein Kinase Kinase (MEK or MAP2K) Inhibitor; Mitogen Activated Protein Kinase Kinase Kinase (cMos or cRaf or MAPKKK or MAP3K or MAP Kinase Kinase Kinase or EC 2.7.11.25) Inhibitor; p38 |

| | | | | | | | | |
|---|---|---|---|---|---|---|---|---|
| | | | | | | | | MAP Kinase Inhibitor; Tyrosine-Protein Kinase Mer (Proto-Oncogene c-Mer) Antagonist |
| CS-410 | | | Chipscreen Biosciences Ltd | Cardiovascular | Cardiovascular Disease | Preclinical | Global | Mitogen Activated Protein Kinase Kinase Kinase 5 (Apoptosis Signal Regulating Kinase 1 or MAPK/ERK Kinase Kinase 5 or MEK Kinase 5 or ASK-1 or MAP3K5 or EC 2.7.11.25) Inhibitor |
| CS-410 | | | Chipscreen Biosciences Ltd | Central Nervous System | Neurodegenerative Diseases | Preclinical | Global | Mitogen Activated Protein Kinase Kinase Kinase 5 (Apoptosis Signal Regulating Kinase 1 or MAPK/ERK Kinase Kinase 5 or MEK Kinase 5 or ASK-1 or MAP3K5 or EC 2.7.11.25) Inhibitor |
| CS-410 | | | Chipscreen Biosciences Ltd | Oncology | Gastric Cancer | Preclinical | Global | Mitogen Activated Protein Kinase Kinase Kinase 5 (Apoptosis Signal Regulating Kinase 1 or MAPK/ERK Kinase Kinase 5 or MEK Kinase 5 or ASK-1 or MAP3K5 or EC 2.7.11.25) Inhibitor |
| CT-207 | | | HEC Pharm Co., Ltd. | Oncology | Melanoma | Preclinical | Global | B-Raf Kinase Inhibitor |
| CT-317 | | | HEC Pharm Co., Ltd. | Oncology | Melanoma | Preclinical | Global | B-Raf Kinase Inhibitor |
| Drugs to Inhibit B-Raf Kinase for Cancer | | | Ruga Corporation | Oncology | | Preclinical | Global | B-Raf Kinase Inhibitor |
| EBI-907 | | | Eternity Bioscience Inc. | Oncology | Melanoma; Metastatic Colorectal Cancer | Preclinical | Global | B-Raf Kinase Inhibitor |
| EBI-945 | | | Eternity Bioscience Inc. | Oncology | | Preclinical | Global | B-Raf Kinase Inhibitor |
| KO-947 | | | Kura Oncology, Inc. | Oncology | Colorectal Cancer; Melanoma; Non-Small Cell Lung | Preclinical | Global | Mitogen Activated Protein Kinase 1 (MAP Kinase 1 or MAPK 1 or Extracellular Signal Regulated Kinase 2 or ERK-2 or Protein Tyrosine Kinase ERK2 or EC 2.7.11.24) |

| | | | | | | | | |
|---|---|---|---|---|---|---|---|---|
| | | | | | Cancer; Pancreatic Cancer | | | Inhibitor; Mitogen Activated Protein Kinase 3 (MAP Kinase 3 or MAPK 3 or Extracellular Signal Regulated Kinase 1 or ERK-1) Inhibitor |
| LXH-254 | | | Novartis AG | Oncology | Solid Tumor | Preclinical | Global | A-Raf Kinase Inhibitor; B-Raf Kinase Inhibitor; C-Raf Kinase Inhibitor |
| MDC-1016 | | | Medicon Pharmaceuticals, Inc | Oncology | Pancreatic Tumor | Preclinical | Global | Ras Inhibitor |
| MT-477 | | | Medisyn Technologies, Inc. | Oncology | Lung Adenocarcinoma; Pancreatic Ductal Adenocarcinoma | Preclinical | Global | Protein Kinase C Alpha (PKC-Alpha) Inhibitor; Ras Inhibitor |
| NCB-0594 | | | Carna Biosciences, Inc. | Oncology | | Preclinical | Global | TRAF2 and NCK-Interacting Protein Kinase (TNIK) Inhibitor |
| NCB-0846 | | | Carna Biosciences, Inc. | Oncology | Colon Cancer | Preclinical | Global | TRAF2 and NCK-Interacting Protein Kinase (TNIK) Inhibitor |
| NMSP-285 | | | Nerviano Medical Sciences S.r.l. | Oncology | | Preclinical | Global | B-Raf Kinase Inhibitor |
| ON-108600 | | | Onconova Therapeutics, Inc. | Oncology | Breast Cancer; Solid Tumor | Preclinical | Global | Casein Kinase 2 Inhibitor; TRAF2 and NCK-Interacting Protein Kinase (TNIK) Inhibitor |
| PV-103 | | | PepVax, Inc. | Oncology | Colorectal Cancer; Melanoma | Preclinical | Global | B-Raf Kinase Inhibitor |
| RX-8243 | | | Rexahn Pharmaceuticals, Inc. | Oncology | Colorectal Cancer; Ovarian Cancer | Preclinical | Global | Aurora A Kinase Inhibitor; Mitogen-Activated Protein (MAP) Kinase Inhibitor; p38 MAP Kinase Inhibitor; Protein Kinase B (PKB or Akt) Inhibitor; Ras Inhibitor |
| Small Molecule to Inhibit MAP4K4 for Cancer | | | Genentech, Inc. | Oncology | | Preclinical | Global | Mitogen-Activated Protein Kinase Kinase Kinase Kinase 4 (HPK/GCK-Like Kinase HGK or MAPK/ERK Kinase Kinase Kinase 4 or MEK Kinase Kinase 4 or MEKKK 4 or MAP4K4 or EC 2.7.11.1) Inhibitor |
| Small | | | Novartis | Onc | | Prec | Global | A-Raf Kinase Inhibitor; B-Raf Kinase |

| | | | | | | | | |
|---|---|---|---|---|---|---|---|---|
| Molecules to Inhibit pan-RAF Kinase for Oncology | | | AG | ology | | linical | | Inhibitor; C-Raf Kinase Inhibitor |
| Small Molecules to Inhibit Raf Kinases for Cancer | | | Amitech Therapeutic Solutions, Inc. | Oncology | Pancreatic Cancer; Solid Tumor | Preclinical | Global | B-Raf Kinase Inhibitor; C-Raf Kinase Inhibitor |
| STP-503 | | Trisilensa | Sirnaomics, Inc. | Oncology | Breast Cancer; Lung Cancer | Preclinical | Global | C-Raf Kinase Inhibitor; Epidermal Growth Factor Receptor (EGFR, HER-1 or ErbB-1) Antagonist; Serine/Threonine-Protein Kinase mTOR (Mammalian Target Of Rapamycin or Mechanistic Target of Rapamycin or EC 2.7.11.1) Inhibitor |
| TAK-632 | | | Takeda Pharmaceutical Company Limited | Oncology | Melanoma | Preclinical | Global | B-Raf Kinase Inhibitor |
| TEW-0201 | | | MedPacto, Inc. | Oncology | Colorectal Cancer; Melanoma | Preclinical | Global | B-Raf Kinase Inhibitor; C-Raf Kinase Inhibitor; Raf Kinase Inhibitor |
| AIK-4 | | | Allinky Biopharma | Oncology | Colorectal Cancer; Lung Cancer; Non-Hodgkin Lymphoma; Pancreatic Cancer | Discovery | Global | Ras Inhibitor |
| AR-00457679 | | | Array BioPharma Inc. | Oncology | | Discovery | Global | B-Raf Kinase Inhibitor |
| CB-745 | | | AGV Discovery, SAS | Oncology | Colon Cancer; Metastatic | Discovery | Global | Mitogen Activated Protein Kinase 1 (MAP Kinase 1 or MAPK 1 or Extracellular Signal Regulated Kinase |

| | | | | | | | | |
|---|---|---|---|---|---|---|---|---|
| | | | | | Melanoma; Pancreatic Cancer | | | 2 or ERK-2 or Protein Tyrosine Kinase ERK2 or EC 2.7.11.24) Inhibitor; Mitogen Activated Protein Kinase 3 (MAP Kinase 3 or MAPK 3 or Extracellular Signal Regulated Kinase 1 or ERK-1) Inhibitor |
| HD-001 | | | AstraZeneca Plc | Oncology | | Discovery | Global | GTPase KRas (K-Ras 2 or Ki-Ras or c-K-Ras or c-Ki-Ras or EC 3.6.5.2) Inhibitor |
| SCH-722984 | | | Merck & Co., Inc. | Oncology | Melanoma | Discovery | Global | Mitogen Activated Protein Kinase 3 (MAP Kinase 3 or MAPK 3 or Extracellular Signal Regulated Kinase 1 or ERK-1) Inhibitor |
| Small Molecule to Inhibit K-RAS for Oncology | | | Aurigene Discovery Technologies Limited | Oncology | | Discovery | Global | GTPase KRas (K-Ras 2 or Ki-Ras or c-K-Ras or c-Ki-Ras or EC 3.6.5.2) Inhibitor |
| Small Molecules to Inhibit B-RAF Kinase for Oncology | | | Sareum Holdings Plc | Oncology | | Discovery | Global | B-Raf Kinase Inhibitor |
| Small Molecules to Inhibit ERK2 and Aurora B Kinase for Cancer | | | Aeterna Zentaris Inc. | Oncology | | Discovery | Global | Aurora B Kinase Inhibitor; Mitogen Activated Protein Kinase 1 (MAP Kinase 1 or MAPK 1 or Extracellular Signal Regulated Kinase 2 or ERK-2 or Protein Tyrosine Kinase ERK2 or EC 2.7.11.24) Inhibitor |
| Small Molecules to Inhibit K-Ras for | | | Araxes Pharma LLC | Oncology | | Discovery | Global | GTPase KRas (K-Ras 2 or Ki-Ras or c-K-Ras or c-Ki-Ras or EC 3.6.5.2) Inhibitor |

| | | | | | | | | |
|---|---|---|---|---|---|---|---|---|
| Cancer | | | | | | | | |
| Small Molecules to Inhibit K-Ras for Oncology | | | Boehringer Ingelheim GmbH | Oncology | | Discovery | Global | GTPase KRas (K-Ras 2 or Ki-Ras or c-K-Ras or c-Ki-Ras or EC 3.6.5.2) Inhibitor |
| Small Molecules to Inhibit KRAS for Cancer | | | NantBioScience | Oncology | | Discovery | Global | GTPase KRas (K-Ras 2 or Ki-Ras or c-K-Ras or c-Ki-Ras or EC 3.6.5.2) Inhibitor |
| Small Molecules to Inhibit KRas for Solid Tumor | | | Nimbus Therapeutics, LLC | Oncology | Solid Tumor | Discovery | Global | GTPase KRas (K-Ras 2 or Ki-Ras or c-K-Ras or c-Ki-Ras or EC 3.6.5.2) Inhibitor |
| Small Molecules to Inhibit Pan-Raf Kinases for Colorectal Cancer | | | Redx Pharma Plc | Oncology | Colorectal Cancer | Discovery | Global | A-Raf Kinase Inhibitor; B-Raf Kinase Inhibitor; C-Raf Kinase Inhibitor |
| Small Molecules to Inhibit TNIK for Oncology | | | Astex Pharmaceuticals, Inc. | Oncology | | Discovery | Global | TRAF2 and NCK-Interacting Protein Kinase (TNIK) Inhibitor |
| Synthetic | | | PeptiDream Inc. | Oncolog | | Discover | Global | GTPase KRas (K-Ras 2 or Ki-Ras or c-K-Ras or c-Ki-Ras or EC 3.6.5.2) |

| | | | | | | | | |
|---|---|---|---|---|---|---|---|---|
| Peptides to Inhibit KRAS for Oncology | | | | y | | y | | Inhibitor |
| ARQ-736 | | | ArQule, Inc. | Oncology | Solid Tumor | Inactive | Global | A-Raf Kinase Inhibitor; B-Raf Kinase Inhibitor; C-Raf Kinase Inhibitor; Raf Kinase Inhibitor |
| CKBP-002 | | | CK Life Sciences Int'l., (Holdings) Inc. | Oncology | Liver Cancer; Pancreatic Cancer; Solid Tumor | Inactive | Global | C-Raf Kinase Inhibitor; Mitogen Activated Protein Kinase Kinase (MEK or MAP2K) Inhibitor; Mitogen-Activated Protein (MAP) Kinase Inhibitor; Signal Transducer And Activator Of Transcription 3 (STAT3) Inhibitor |
| DP-2514 | | | Eli Lilly and Company | Oncology | Melanoma | Inactive | Global | B-Raf Kinase Inhibitor |
| DP-3346 | | | Eli Lilly and Company | Oncology | Melanoma | Inactive | Global | B-Raf Kinase Inhibitor |
| Fluorapacin | | | ACEA Biosciences, Inc. | Oncology | Advanced Malignancy | Inactive | Global | Mitogen Activated Protein Kinase 3 (MAP Kinase 3 or MAPK 3 or Extracellular Signal Regulated Kinase 1 or ERK-1) Inhibitor; Mitogen Activated Protein Kinase Kinase 1 (MEK-1 or MAP2K1) Inhibitor |
| GDC-0879 | | | Genentech, Inc. | Oncology | Melanoma | Inactive | Global | B-Raf Kinase Inhibitor |
| LE-rafAON | | | Insys Therapeutics, Inc. | Oncology | Solid Tumor | Inactive | Global | C-Raf Kinase Inhibitor |
| MK-8353 | | | Merck & Co., Inc. | Oncology | Metastatic Colorectal Cancer; Metastatic Melanoma; Solid Tumor | Inactive | Global | Mitogen Activated Protein Kinase 3 (MAP Kinase 3 or MAPK 3 or Extracellular Signal Regulated Kinase 1 or ERK-1) Inhibitor |
| NMSP-383 | | | Nerviano Medical Sciences S.r.l. | Oncology | Melanoma | Inactive | Global | B-Raf Kinase Inhibitor |
| NMSP-730 | | | Nerviano Medical Sciences | Oncology | Melanoma | Inactive | Global | B-Raf Kinase Inhibitor |

| | | | | | | | | |
|---|---|---|---|---|---|---|---|---|
| | | | S.r.l. | | | | | |
| PLX-4720 | | | Plexxikon Inc. | Musculoskeletal Disorders | Cachexia | Inactive | Global | B-Raf Kinase Inhibitor |
| PLX-4720 | | | Plexxikon Inc. | Oncology | Anaplastic Thyroid Cancer | Inactive | Global | B-Raf Kinase Inhibitor |
| PNT-300 | | | ProNAi Therapeutics, Inc. | Oncology | | Inactive | Global | GTPase KRas (K-Ras 2 or Ki-Ras or c-K-Ras or c-Ki-Ras or EC 3.6.5.2) Inhibitor |
| SCH-772984 | | | Merck & Co., Inc. | Oncology | Solid Tumor | Inactive | Global | Mitogen Activated Protein Kinase 3 (MAP Kinase 3 or MAPK 3 or Extracellular Signal Regulated Kinase 1 or ERK-1) Inhibitor |
| Small Molecule to Inhibit C-Raf for Oncology | | | Locus Pharmaceuticals, Inc. (Inactive) | Oncology | | Inactive | Global | C-Raf Kinase Inhibitor |
| Small Molecule to Inhibit K-Ras for Multiple Myeloma | | | TetraGene, LLC | Oncology | Multiple Myeloma (Kahler Disease) | Inactive | Global | GTPase KRas (K-Ras 2 or Ki-Ras or c-K-Ras or c-Ki-Ras or EC 3.6.5.2) Inhibitor |
| Small Molecules to Inhibit B-raf/C-Raf for Cancer | | | Redx Pharma Plc | Oncology | Colorectal Cancer; Melanoma | Inactive | Global | B-Raf Kinase Inhibitor; C-Raf Kinase Inhibitor |
| Small Molecules to | | | Astex Pharmaceuticals, Inc. | Oncology | | Inactive | Global | B-Raf Kinase Inhibitor |

| | | | | | | | | |
|---|---|---|---|---|---|---|---|---|
| Inhibit BRAF for Oncology | | | | | | | | |
| Small Molecules to Inhibit ERK-1 and ERK-2 for Solid Tumors | | | Kura Oncology, Inc. | Oncology | Solid Tumor | Inactive | Global | Mitogen Activated Protein Kinase 1 (MAP Kinase 1 or MAPK 1 or Extracellular Signal Regulated Kinase 2 or ERK-2 or Protein Tyrosine Kinase ERK2 or EC 2.7.11.24) Inhibitor; Mitogen Activated Protein Kinase 3 (MAP Kinase 3 or MAPK 3 or Extracellular Signal Regulated Kinase 1 or ERK-1) Inhibitor |
| Small Molecules to Inhibit K-Ras for Oncology | | | Navigen Pharmaceuticals, Inc. | Oncology | | Inactive | Global | GTPase KRas (K-Ras 2 or Ki-Ras or c-K-Ras or c-Ki-Ras or EC 3.6.5.2) Inhibitor |
| Small Molecules to Inhibit TNIK for Cancer | | | CrystalGenomics, Inc. | Oncology | | Inactive | Global | TRAF2 and NCK-Interacting Protein Kinase (TNIK) Inhibitor |
| Tumor Adaptive Responses Program | | | Ruga Corporation | Oncology | Metastatic Ovarian Cancer | Inactive | Global | B-Raf Kinase Inhibitor |
| AEZS-131 | | | AEterna Zentaris Inc. | Oncology | Breast Cancer; Colon Cancer | Discontinued | Global | Mitogen Activated Protein Kinase 3 (MAP Kinase 3 or MAPK 3 or Extracellular Signal Regulated Kinase 1 or ERK-1) Inhibitor; Mitogen Activated Protein Kinase Kinase 1 (MEK-1 or MAP2K1) Inhibitor |
| ISIS-5132 | | | Ionis Pharmaceuticals, Inc. | Oncology | Epithelial Ovarian Cancer; | Discontinued | Global | C-Raf Kinase Inhibitor |

| | | | | | | | | |
|---|---|---|---|---|---|---|---|---|
| | | | | | Hormone Refractory (Castration Resistant, Androgen-Independent) Prostate Cancer; Metastatic Breast Cancer; Metastatic Colorectal Cancer | | | |
| PLX-3603 | | | Plexxikon Inc. | Oncology | Solid Tumor | Discontinued | Global | B-Raf Kinase Inhibitor |
| XL-281 | | | Exelixis, Inc. | Oncology | Colorectal Cancer; Melanoma; Solid Tumor | Discontinued | Global | B-Raf Kinase Inhibitor; C-Raf Kinase Inhibitor |
| MM-41 | | | University College London | Oncology | Pancreatic Cancer | Preclinical | Global | Bcl-2 Inhibitor; GTPase KRas (K-Ras 2 or Ki-Ras or c-K-Ras or c-Ki-Ras or EC 3.6.5.2) Inhibitor |
| Oligonucleotide to Inhibit KRAS for Cancer | | | UNC Lineberger Comprehensive Cancer Center | Oncology | Colon Cancer; Lung Cancer | Preclinical | Global | GTPase KRas (K-Ras 2 or Ki-Ras or c-K-Ras or c-Ki-Ras or EC 3.6.5.2) Inhibitor |
| Antisense RNAi Oligonucleotides to Inhibit KRAS for Cancer | | | Brigham and Women's Hospital | Oncology | | Discovery | Global | GTPase KRas (K-Ras 2 or Ki-Ras or c-K-Ras or c-Ki-Ras or EC 3.6.5.2) Inhibitor |
| Monoclonal Antibody to Target | | | Vanderbilt University | Oncology | | Discovery | Global | B-Raf Kinase Inhibitor |

| | | | | | | | | |
|---|---|---|---|---|---|---|---|---|
| B-Raf Kinase for Oncology | | | | | | | | |
| Small Molecule to Inhibit GCK for Oncology | | | Institut Curie | Oncology | | Discovery | Global | Mitogen-Activated Protein Kinase Kinase Kinase Kinase 2 (Germinal Center Kinase or MAPK/ERK Kinase Kinase Kinase 2) Inhibitor |
| Small Molecules to Inhibit K-Ras for Oncology | | | Vanderbilt University | Oncology | | Discovery | Global | GTPase KRas (K-Ras 2 or Ki-Ras or c-K-Ras or c-Ki-Ras or EC 3.6.5.2) Inhibitor |
| Small Molecules to Inhibit Ras for Oncology | | | University of Texas Health Science Center at Houston | Oncology | | Discovery | Global | Ras Inhibitor |
| SML-8731 | | | Dana-Farber Cancer Institute, Inc. | Oncology | | Discovery | Global | GTPase KRas (K-Ras 2 or Ki-Ras or c-K-Ras or c-Ki-Ras or EC 3.6.5.2) Inhibitor |

By "EMT pathway inhibitor" is meant a therapeutic agent, such as a pharmaceutical drug, that acts to inhibit the epithelial-mesenchymal transition (EMT). The inhibitor may be specific for the EMT pathway. Thus, in certain embodiments the EMT pathway inhibitor is not a multi-pathway inhibitor. In certain embodiments, the EMT pathway inhibitor is selected from Table I.

In further embodiments, the EMT pathway inhibitor is an FKBP-L polypeptide or a biologically active peptide fragment thereof. In preferred embodiments, the biologically active peptide fragment of FKBP-L comprises the amino acid sequence IRQQPRDPPTETLELEVSPDPAS (SEQ ID NO:791; referred to herein also as ALM201), or a sequence at least 90% identical thereto. In further embodiments, the FKBP-L polypeptide comprises the amino acid sequence shown as SEQ ID NO:789 or SEQ ID NO:790, or a sequence at least 90% identical thereto. In further embodiments, the biologically active peptide fragment of FKBP-L comprises the amino acid sequence shown as any one of SEQ ID Nos 792 to 811, or a sequence at least 90% identical thereto.

As used herein, the term "biologically active FKBP-L peptide" (e.g., fragment and/or modified polypeptides) is used to refer to a peptide or polypeptide that displays the same or similar amount and type of activity as the full-length FKBP-L polypeptide. In this context "biological activity" of an FKBP-L polypeptide, fragment or derivative refers to the ability to inhibit and/or reverse the EMT pathway (and/or the ability to down-regulate the MAPK pathway). MAPK is known to induce EMT via phosphorylation of the SNAIL/SLUG transcription factors, (Virtakoivu *et al.,* 2015).

Biological activity of FKBP-L fragments or derivatives may be tested in comparison to full length FKBP-L using any of the *in vitro* or *in vivo* assays described in the accompanying examples, including cell-based assays of the mesenchymal phenotype, such as for example the colony formation assay, migration assay or invasion assay. In other embodiments, "biological activity" of an FKBP-L polypeptide, fragment or derivative may be demonstrated by assaying expression of one or more biomarkers of the EMT pathway (e.g. mesenchymal markers), or one or more biomarkers of the MAPK pathway.

The term "FKBP-L" refers to the protein FK506 binding protein-like, (McKeen et al. Endocrinology, 2008, Vol 149(11), 5724-34; Gene ID:63943). FKBP-L and peptide fragments thereof have previously been demonstrated to possess potent anti-angiogenic activity (WO 2007/141533). The anti-angiogenic activity of FKBP-L peptide fragments appears to be dependent on an amino acid sequence located between amino acids 34-57, in the N-terminal region of the full-length protein. This anti-angiogenic activity suggested a clinical utility of the peptide in the treatment of cancers, particularly solid tumours.

The expression "FKBP-L polypeptide" is used in the specification according to its broadest meaning. It designates the naturally occurring full-length protein as shown in SEQ ID NO:789, together with homologues due to polymorphisms, other variants, mutants and portions of said polypeptide which retain their biological activities. For example, in certain embodiments, the FKBP-L polypeptide comprises SEQ ID NO:789 (GENBank Accession No. NP_071393; NM_022110; [gi:34304364]), or SEQ ID NO:790 with a Threonine at position 181 and a Glycine at position 186 of the wild-type sequence. Example constructs of other FKBP-L polypeptides (e.g., fragments and other modifications) and polynucleotide constructs encoding for FKBP-L polypeptides are described in WO 2007/141533.

In SEQ ID NO: 790, the FKBP-L insert (originally cloned into PUC18 by Cambridge Bioscience and now cloned into pcDNA3.1); had two inserted point mutations compared to the sequence that is deposited on the PUBMED database (SEQ ID NO: 789). There is a point mutation at 540 bp (from start codon): TCT to ACT which therefore converts a serine (S) to a Threonine (T) (amino acid: 181). There is also a point mutation at 555 bp (from start codon): AGG to GGG which therefore converts an Arginine (R) to a Glycine (G) (amino acid: 186). Both FKBP-L polypeptides (SEQ ID NO: 789 and SEQ ID NO: 790) display biological activity.

An FKBP-L polypeptide or peptide may include natural and/or chemically synthesized or artificial FKBP-L peptides, peptide mimetics, modified peptides (e.g., phosphopeptides, cyclic peptides, peptides containing D- and unnatural amino-acids, stapled peptides, peptides containing radiolabels), or peptides linked to antibodies, carbohydrates, monosaccharides, oligosaccharides, polysaccharides, glycolipids, heterocyclic compounds, nucleosides or nucleotides or parts thereof, and/or small organic or inorganic molecules (e.g., peptides modified with PEG or other stabilizing groups). Thus, the FKBP-L (poly)peptides utilized in the methods and therapeutic agents for use according to the present invention also include chemically modified peptides or isomers and racemic forms.

As described herein, the methods and therapeutic agents for use according to the present invention may utilize a full-length FKBP-L polypeptide, or biologically active fragments of the polypeptide. Thus, in certain embodiments of the present invention the EMT pathway inhibitor is a FKBP-L derivative which comprises or consists of a biologically active portion of the N-terminal amino acid sequence of naturally occurring FKBP-L. This sequence may comprise, consist essentially of, or consist of an active N-terminal portion of the FKBP-L polypeptide. In alternate embodiments, the polypeptide may comprise, consist essentially of, or consist of amino acids 1 to 57 of SEQ ID NO: 790 (i.e., SEQ ID NO: 796), or amino acids 34-57 of SEQ ID NO:790 (i.e., SEQ ID NO: 792), or amino acids 35-57 of SEQ ID NO:790 (i.e. SEQ ID NO:791). Or, the peptide may comprise, consist essentially of, or consist of a sequence that comprises at least 18 contiguous amino acids of SEQ ID NO: 792 (e.g., SEQ ID NOs: 798, 800, or 807). In alternate embodiments, the polypeptide used in the methods and compositions of the present invention may comprise, consist essential of, or consist of one of the amino acid sequences shown in any one of SEQ ID NOs: 789-811. In certain embodiments of the present invention the EMT pathway inhibitor is a biologically active fragment of FKBP-L, wherein said polypeptide includes no more than 200 consecutive amino acids of the amino acid sequence shown in SEQ ID NO:789, or SEQ ID NO:790, with the proviso that said polypeptide includes the amino acid sequence shown as SEQ ID NO:791.

As described herein, the peptides may be modified (e.g., to contain PEG and/or His tags, albumin conjugates or other modifications). Or, the present invention may utilize isolated polypeptides having a sequence at least 70%, or 75%, or 80%, or 85%, or 90%, or 95%, or 96%, or 97%, or 98%, or 99% identical to the amino acid sequences as set forth in any one of SEQ ID NOS: 789-811, including in particular sequences at least 70%, or 75%, or 80%, or 85%, or 90%, or 95%, or 96%, or 97%, or 98%, or 99% identical to the amino acid sequence shown as SEQ ID NO:791. In this regard, deliberate amino acid substitutions may be made in the peptide on the basis of similarity in polarity, charge, solubility, hydrophobicity, or hydrophilicity of the residues, as long as the specific biological activity (i.e. function) of the peptide is retained. The FKBP-L peptide may be of variable length as long as it retains its biological activity and can be used according to the various aspects of the invention described above.

Certain regions of the N-terminus of the FKBP-L protein may display biological activity, therefore the invention encompasses biologically active fragments of FKBP-L, in particular any fragment which exhibits biological activity substantially equivalent to that of the 23-mer peptide (SEQ ID NO:791). In certain embodiments, the biological activity of the FKBP-L 23mer peptide (SEQ ID NO:791; referred to herein also as ALM201) is exhibited as a reduction in expression of mesenchymal markers in Kuramochi cells or OVCAR3 cisplatin resistant cells. In further embodiments, the biological activity of the FKBP-L 23mer peptide (SEQ ID NO:791; referred to herein also as ALM201) is exhibited as a reversal of the mesenchymal phenotype in OVCAR3 or OVCAR4 cisplatin resistant cells.

A "fragment" of a FKBP-L polypeptide means an isolated peptide comprising a contiguous sequence of at least 6 amino acids, preferably at least 10 amino acids, or at least 15 amino acids, or at least 20 amino acids, or at least 23 amino acids of FKBP-L. The "fragment" preferably contains no more than 50, or no more than 45, or no more than 40, or no more than 35, or no more than 30, or no more than 25, or no more than 23 contiguous amino acids of FKBP-L. Preferred fragments for use according to the invention are those having the amino acid sequences shown in any one of SEQ ID Nos: 792-811, or minor sequence variants thereof (e.g. variants containing one or more conservative amino acid substitutions).

In certain embodiments the EMT pathway inhibitor (specifically) inhibits Vimentin, AXL, TWIST1 SNAIL and/or SLUG.

The EMT pathway inhibitor may be used together with a platinum-based chemotherapeutic agent as a first line treatment. Alternatively, the EMT pathway inhibitor may be used as a second line treatment after treatment with a platinum-based chemotherapeutic agent.

**TABLE I - EMT pathway inhibitors**

| DRUG NAME | COMPANY | INDICATION | PRODUCT STAGE |
|---|---|---|---|
| gilteritinib fumarate | Astellas | NSCLC Leukemia | Ph III Ph II |
| MGCD-265 | Mirati Therapeutics | Head & Neck, NSCLC | Ph II |
| MGCD-516 | Mirati Therapeutics | Lung | Ph I |
| S-49076 | Servier | NSCLC, Brain Colon, HCC | Ph II Pre-clinical |
| BPI-9016 | Zhejiang BetaPharma | Gastic, Liver, Lung | Ph I |
| CT-053 | EC Pharm Co., Ltd. | Brain Bladder, Breast, HCC, RCC, Lung, Ovarian | Ph I Pre-clinical |
| BGB-324 | BerGenBio | Leukemia, Lung Breast, Pancreatic | Ph I Pre-clinical |
| BGB-10C9 | BerGenBio | Pancreatic | Pre-clinical |
| Misc AXLi | BerGenBio | Oncology | Pre-clinical |
| HuMax-AXL-ADC | GenMab | Solid Tumor | Pre-clinical |
| LDC-2636 | Lead Discovery Center | Leukemia | Pre-clinical |
| Misc AXLi | Protelica Incorporated | Oncology | Pre-clinical |
| NPS-1034 | NeoPharm | NSCLC | Pre-clinical |
| Q-701 | Qurient Co., Ltd. | Oncology | Pre-clinical |
| RXDX-106 | Ignyta, Inc. | Leukemia, Breast, GI, Melanoma; mCRC, NSCLC, Ovarian, Pancreatic | Pre-clinical |
| SGI-7079 | Tolero Pharmaceuticals, Inc. | NSCLC | Pre-clinical |
| TP-0903 | Tolero Pharmaceuticals, Inc. | Leukemia; Head And Neck, Lung, Pancreatic | Pre-clinical |
| Misc AXLi | SignalChem Lifesciences | Oncology | Pre-clinical |
| Misc AXLi | University of Colorado | NSCLC | Pre-clinical |
| Misc AXLi | Kolltan Pharmaceuticals | Oncology | Discovery |

By "SRC pathway inhibitor" is meant a therapeutic agent, such as a pharmaceutical drug, that acts to inhibit signalling by the SRC pathway. The inhibitor may be specific for the SRC pathway. Thus, in certain embodiments the SRC pathway inhibitor is not a multi-pathway inhibitor. In further embodiments the SRC pathway inhibitor is a (specific) inhibitor of an SRC family kinase.

In certain embodiments the SRC pathway inhibitor is selected from Table J. In further embodiments the SRC pathway inhibitor is not Dasatinib and/or pazopanib (hydrochloride), which are multi-targeted pathway inhibitors.

**TABLE J - SRC pathway inhibitors**

| DRUG NAME | COMPANY | INDICATION | PRODUCT STAGE |
|---|---|---|---|
| Ilorasertib | AbbVie | Solid Tumor Ovarian | Ph II Pre-clinical |
| KX-01 | Athenex | Leukemia | PhI |
| pazopanib hydrochloride + pembrolizumab | GSK | mRCC | PhII |
| tesevatinib tosylate | Kadmon Corporation | mBrain, mBreast, NSCLC | PhII |
| VAL-201 | ValiRx Plc | Breast, mProstate, Ovarian | PhII |
| AZD-0424 | AZ | Oncology | PhI |
| BGB-102 | BeiGene(Beijing) | Oncology | PhI |
| KX-02 | Athenex, Inc. | Brain, Lymphoma | PhI |
| rebastinib tosylate | Deciphera Pharmaceuticals | Leukemia mBreast | PhI Pre-clinical |
| ASN-006 | Asana BioSciences | Oncology | Pre-clinical |
| CCT-196969 CCT-241161 | Basilea Pharmaceutica AG | Melanoma | Pre-clinical |
| ORB-0001 | OriBase Pharma | Leukemia | Pre-clinical |
| Misc | MI.TO. Technology S.r.L. | Oncology | Pre-clinical |
| Misc | University of Toledo | Prostate | Pre-clinical |
| RK-20449 | Riken Advanced Science Institute | Leukemia | Pre-clinical |
| Various others in Discovery stage by Academic institutions | | | |

According to all aspects of the invention the platinum-based chemotherapeutic agent may comprise one or more of, or be selected from carboplatin, cisplatin, oxaliplatin, satraplatin, picoplatin, Nedaplatin, Triplatin and/or Lipoplatin.

According to all aspects of the invention the taxane may comprise Paclitaxel and/or Docetaxel. In specific embodiments the therapeutic agent is a taxane and the cancer is prostate cancer. The prostate cancer may be metastatic prostate cancer, in particular de novo metastatic prostate cancer.

The inhibitors described herein may act by inhibiting the expression (reducing the levels) and/or the function of one (or more) targets. Inhibition of function can include inhibiting interactions with one (or more) binding partners.

By "anti-angiogenic therapeutic agent" is meant a therapeutic agent, such as a pharmaceutical drug, that acts to inhibit angiogenesis. Examples of anti-angiogenic therapeutic agents include VEGF pathway-targeted therapeutic agents, including multi-targeted pathway inhibitors (VEGF/PDGF/FGF/EGFT/FLT-3/c-KIT), Angiopoietin-TIE2 pathway inhibitors, endogenous angiogenic inhibitors, and immunomodulatory Agents. VEGF specific inhibitors include, but are not limited to, Bevacizumab (Avastin), Afibercept (VEGF Trap), IMC-1121B (Ramucirumab). Multi-targeted pathway inhibitors include, but are not limited to, Imatinib (Gleevec), Sorafenib (Nexavar), Gefitinib (Iressa), Sunitinib (Sutent), Erlotinib, Tivozinib, Cediranib (Recentin), Pazopanib (Votrient), BIBF 1120 (Vargatef), Dovitinib, Semaxanib (Sugen), Axitinib (AG013736), Vandetanib (Zactima), Nilotinib (Tasigna), Dasatinib (Sprycel), Vatalanib, Motesanib, ABT-869, TKI-258. Angiopoietin-TIE2 pathway inhibitors include, but are not limited to, AMG-386 (Trebananib), PF-4856884 CVX-060, CEP-11981, CE-245677, MEDI-3617, CVX-241, Trastuzumab (Herceptin). Endogenous angiogenic inhibitors include, but are not limited to, Thombospondin, Endostatin, Tumstatin, Canstatin, Arrestin, Angiostatin, Vasostatin, Interferon alpha. Immunomodulatory Agents include, but are not limited to, Thalidomide and Lenalidomide. The inhibitor may be specific for angiogenesis processes or pathways. Optionally the anti-angiogenic therapeutic agent is not a multi-pathway inhibitor.

The anti-angiogenic therapeutic agent may be selected from Table K.

**Table K - Anti-angiogenic therapeutic agents**

| Trade Name | Generic Name | Companies |
|---|---|---|
| Avastin | Bevacizumab | Chugai Pharmaceutical Co., Ltd; Genentech, Inc.; PDL BioPharma, Inc. ; Roche |
| Nexavar | Sorafenib | Bayer AG; Onyx Pharmaceuticals, Inc. |
| Nintedanib | Nintedanib | Boehringer Ingelheim GmbH |
| VEGFR-2 siRNA Formulated With Staramine-mPEG | | EGEN, Inc. |
| Small Molecule Vegf Inhibitor | | Interprotein Corporation |
| TRC105 | | Santen Pharmaceutical Co., Ltd; Tracon Pharmaceuticals |
| Zaltrap | Ziv-Aflibercept | Regeneron Pharmaceuticals, Inc.; Sanofi |
| CS3158 | | Shenzhen Chipscreen Biosciences |
| Fruquintinib | Fruquintinib | Eli Lilly & Co.; Hutchison Medipharma Limited |
| Zactima | Vandetanib | AstraZeneca PLC |
| Ramucirumab | Ramucirumab | Dyax Corporation; Eli Lilly & Co.; ImClone Systems |
| Dovitinib | Dovitinib | Novartis AG |
| hVEGF-trunc Vaccine | | Immunovo BV; Pepscan |
| Votrient | Pazopanib | GlaxoSmithKline plc |
| Inlyta | Axitinib | Pfizer, Inc.; Sfj Pharmaceuticals, Inc. |
| Stivarga | Regorafenib | Bayer AG; Onyx Pharmaceuticals, Inc. |
| Iclusig | Ponatinib | Ariad Pharmaceuticals, Inc.; Specialised Therapeutics Australia, Pty, Ltd. |
| Lucitanib | Lucitanib | Clovis Oncology Inc; Ethical Oncology Science; Les Laboratoires Servier; Shanghai Institute of Materia Medica |
| E7080 | Lenvatinib | Eisai Co., Ltd.; Sfj Pharmaceuticals, Inc. |
| Recentin | Cediranib | AstraZeneca PLC |
| Brivanib Alaninate | Brivanib Alaninate | Bristol-Myers Squibb |
| Cometriq | Cabozantinib | Bristol-Myers Squibb; Exelixis, Inc.; Swedish Orphan Biovitrum |
| OTS102 | | Oncotherapy Science |
| Tivozanib | Tivozanib | AVEO Oncology; Astellas Pharma, Inc.; Kirin Pharmaceutical; Kyowa Hakko Kirin Pharma, Inc |
| Sutent | Sunitinib Malate | Pfizer, Inc. |
| APX003 | | Apexigen, Inc.; Simcere Pharmaceutical Group |

(continued)

| Trade Name | Generic Name | Companies |
|---|---|---|
| Sareum VEGFR-3 Program | | Sareum Ltd. |
| PRS-050 | | Pieris AG |
| X-82 | | Xcovery, Inc. |
| CM-082 | | AnewPharma; Xcovery, Inc. |
| Pieris/Syngenta Anticalin Program | | Pieris AG; Syngenta Corporation |
| CTx-0357927 | | Bionomics Ltd.; Cancer Therapeutics Crc |
| ABT-869 | Linifanib | Abbott Laboratories; Abbvie; Genentech, Inc.; Roche |
| MGCD265 | | Methylgene, Inc. |
| Dalantercept | | Acceleron Pharma |
| Norcantharidin | Norcantharidin | Shandong Hualu Pharmaceutical Co., Ltd. |
| NOX-S93 | | Noxxon Pharma AG |
| VEGF Inhibitor Program | | Legochembiosciences, Inc. |
| R3 Antibody | | Affitech A/S; Peregrine Pharmaceuticals, Inc. |
| AT001/r84 | | Affitech A/S; Peregrine Pharmaceuticals, Inc. |
| Muparfostat | Muparfostat Sodium | Medigen Biotechnology Corp; Progen Pharmaceuticals Limited |
| Foretinib | Foretinib | Exelixis, Inc.; GlaxoSmithKline plc |
| Telatinib | Telatinib | Act Biotech, Inc.; Bayer AG; Eddingpharm (Cayman), Inc. |
| YN968D1 | Apatinib | Advenchen Laboratories, LLC |
| AL3818 | | Advenchen Laboratories, LLC |
| AL3810 | | Advenchen Laboratories, LLC |
| AL8326 | | Advenchen Laboratories, LLC |
| Icrucumab | Icrucumab | Eli Lilly & Co.; ImClone Systems |
| PTC299 | | PTC Therapeutics, Inc. |
| Aplidin | Plitidepsin | PharmaMar |
| Veglin | Vascular Endothelial Growth Factor Antisense Oligonucleotide | Vasgene Therapeutics |
| BMS-817378 | | Bristol-Myers Squibb; Simcere Pharmaceutical Group |
| MG516 | | Methylgene, Inc. |
| FP-1039 | | Five Prime Therapeutics, Inc.; GlaxoSmithKline plc |
| IMC-3C5 | Vegfr 3 Monoclonal Antibody | Circadian Technologies Limited; Eli Lilly & Co.; ImClone Systems |
| TAS-115 | | Otsuka Pharmaceutical Co., Ltd; Taiho Pharmaceutical Co., Ltd |

(continued)

| Trade Name | Generic Name | Companies |
|---|---|---|
| TRAP Based VEGFR2 Inhibitor | | Telik, Inc. |
| TLK60404 | | Telik, Inc. |
| Trap Based Ar + Vegf Dual Inhibitor | | Telik, Inc. |
| RG7221 | | Roche |
| DCC-2701 | | Deciphera Pharmaceuticals |
| DP-2473 | | Deciphera Pharmaceuticals |
| DP-2514 | | Deciphera Pharmaceuticals; Eli Lilly & Co. |
| VEGF R-2 Inhibitor | | Bristol-Myers Squibb |
| Erbb + Vegf Receptor Inhibitor | | Bristol-Myers Squibb |
| Motesanib | Motesanib Diphosphate | Amgen, Inc.; Millennium Pharmaceuticals, Inc.; Takeda Pharmaceutical Company Limited |
| Semaxanib | Semaxanib | Pfizer, Inc. |
| VGX-200 | | Circadian Technologies Limited; Teva Pharmaceutical Industries Ltd |
| E7050 | Golvatinib | Eisai Co., Ltd. |
| GSK089 | | GlaxoSmithKline plc |
| KW-2401 | Irinotecan | Kyowa Hakko Kirin Pharma, Inc |
| GNR-011 | Apagin | International Biotechnology Center Generium |
| CYC116 | | Cyclacel Pharmaceuticals, Inc. |
| FAK-FLT3-VEGFR3 Program | | Cancer Therapeutics Crc |
| DMI-6867 | | Ampio Pharmaceuticals, Inc. |
| VGX-100 | | Circadian Technologies Limited |
| A6 | | Angstrom Pharmaceuticals, Inc. |
| 1181 | | Egenix Inc |
| 4EGI-1 | | Egenix Inc |
| Egenix Cancer Therapeutics Program | | Egenix Inc |
| CFAK-C4 | | Curefaktor Pharmaceuticals |
| PMX 2058 | | Pharminox Limited |
| GFB-204 | | H. Lee Moffitt Cancer Center & Research Institute; Kirax Corporation; Yale University Office of Cooperative Research |

According to all aspects of the invention the method may further comprise obtaining a test sample from the subject. In certain embodiments the methods involving determining gene expression are *in vitro* methods performed on an isolated sample.

According to all aspects of the invention samples may be of any suitable form including any material, biological fluid, tissue, or cell obtained or otherwise derived from an individual. Typically, the sample includes cancer cells or genetic material (DNA or RNA) derived from the cancer cells, to include cell-free genetic material (e.g. found in the peripheral blood). In specific embodiments the sample comprises, consists essentially of or consists of a formalin-fixed paraffin-embedded biopsy sample. In further embodiments the sample comprises, consists essentially of or consists of a fresh/frozen (FF) sample. The sample may comprise, consist essentially of or consist of tumour (cancer) tissue, optionally ovarian tumour (cancer) tissue. The sample may comprise, consist essentially of or consist of tumour (cancer) cells, optionally ovarian tumour (cancer) cells. The tissue sample may be obtained by any suitable technique. Examples include a biopsy procedure, optionally a fine needle aspirate biopsy procedure. Body fluid samples may also be utilised. Suitable sample types include blood (including whole blood, leukocytes, peripheral blood mononuclear cells, buffy coat, plasma, and serum), sputum, tears, mucus, nasal washes, nasal aspirate, breath, urine, semen, saliva, meningeal fluid, amniotic fluid, glandular fluid, lymph fluid, nipple aspirate, bronchial aspirate, synovial fluid, joint aspirate, ascites, cells, a cellular extract, and cerebrospinal fluid. This also includes experimentally separated fractions of all of the preceding. For example, a blood sample can be fractionated into serum or into fractions containing particular types of blood cells, such as red blood cells or white blood cells (leukocytes). If desired, a sample can be a combination of samples from an individual, such as a combination of a tissue and fluid sample. The term "sample" also includes materials containing homogenized solid material, such as from a stool sample, a tissue sample, or a tissue biopsy, for example. The term "sample" also includes materials derived from a tissue culture or a cell culture, including tissue resection and biopsy samples. Example methods for obtaining a sample include, e.g., phlebotomy, swab (e.g., buccal swab). Samples can also be collected, e.g., by micro dissection (e.g., laser capture micro dissection (LCM) or laser micro dissection (LMD)), bladder wash, smear (e.g., a PAP smear), or ductal lavage. A "sample" obtained or derived from an individual includes any such sample that has been processed in any suitable manner after being obtained from the individual. The methods of the invention as defined herein may begin with an obtained sample and thus do not necessarily incorporate the step of obtaining the sample from the patient. As used herein, the term "patient" includes human and non-human animals. The preferred patient for treatment is a human. "Patient," "individual" and "subject" are used interchangeably herein.

According to all aspects of the invention, unless otherwise defined herein, the cancer may be ovarian, prostate, colon or lung cancer or melanoma. In certain embodiments the ovarian cancer is serous ovarian cancer. In specific embodiments the ovarian cancer is high grade serous ovarian cancer. In certain embodiments the lung cancer is non-small cell lung cancer and/or lung adenocarcinoma. The cancer may also be leukaemia, brain cancer, glioblastoma, head and neck cancer, liver cancer, stomach cancer, colorectal cancer, thyroid cancer, neuroendocrine cancer, gastrointestinal stromal tumors (GIST), gastric cancer, lymphoma, throat cancer, breast cancer, skin cancer, melanoma, multiple myeloma, sarcoma, cervical cancer, testicular cancer, bladder cancer, endocrine cancer, endometrial cancer, esophageal cancer, glioma (optionally lower grade glioma), lymphoma, neuroblastoma, osteosarcoma, pancreatic cancer, pituitary cancer, renal cancer (optionally renal clear cell cancer and/or renal papillary cancer), and the like. As used herein, colorectal cancer encompasses cancers that may involve cancer in tissues of both the rectum and other portions of the colon as well as cancers that may be individually classified as either colon cancer or rectal cancer. In certain embodiments the cancer is not prostate cancer and/or glioblastoma.

Optionally, according to all aspects, the method may comprise measuring the expression levels of at least around 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46 or each of the biomarkers listed in Table A and/or Table B. Combinations from Tables A and B are also envisaged. "Around" may mean plus or minus five. By "corresponding" may mean that the probe hybridizes to the gene/biomarker or can be used to detect expression of the gene/biomarker. Smaller gene signatures may be based around those markers having greater weight values in Tables A and B and thus, in some embodiments, sub-signatures are generated by taking a selection of the larger signatures in numerical order. Thus, for example, a 5 gene signature may be composed of the first 5 genes listed in Table A and/or Table B. It could also be composed of the 5 genes with the highest weight values from Tables A and B combined. In other embodiments, the gene signatures may comprise one of the markers with the highest weight values (e.g. selected from the top 2, 3, 4, 5, 6, 7, 8, 9, or 10 markers), either alone or combined with other markers. In other embodiments the methods may further comprise measuring the expression levels of one or more up to all of the following biomarkers: GJB2, CDH11, COL10A1, ANGPTL2, THBS1, RAB31, THBS2, INHBA, MMP14, VCAN, PLAU, FAP, FN1.

Optionally, according to all aspects, the methods may further comprise measuring the expression levels of one or more up to all of the following biomarkers: TMEM200A, GJB2, MMP13, POSTN, BICC1, MRVI1, COL11A1, IGFL2, NTM, BGN, COL10A1, RAB31, ANGPTL2, PLAU, COL8A1, MIR1245, POLD2, NKD2, FZD1, COPZ2, ITGA5, VGLL3, INHBA, MMP14, THBS2, RUNX2, TIMP3, SFRP2, COL1A2, COL5A2, SERPINF1, KIF26B, ALPK2, CTSK, LOXL1 and FAP (optionally together with one or more up to all of the following biomarkers: CDH11, PMP22, LUM, COL3A1, VCAN, TNFAIP6, MMP2 and FN1); and/or one or more up to all of the following biomarkers: GJB2, COL10A1, ANGPTL2, THBS1, RAB31, THBS2, INHBA, MMP14, PLAU and FAP (optionally together with one or more up to all of the following biomarkers: CDH11,VCAN, COL5A1 and FN1).

Optionally, according to all aspects, the methods may further comprise measuring the expression levels of one or more up to all of the following biomarkers: TMEM200A, GJB2, MMP13, POSTN, BICC1, CDH11, MRVI1, PMP22, COL11A1, IGFL2, LUM, NTM, BGN, COL3A1, COL10A1, RAB31, ANGPTL2, PLAU, COL8A1, MIR1245, POLD2, NKD2, FZD1, COPZ2, ITGA5, VGLL3, INHBA, MMP14, VCAN, THBS2, RUNX2, TIMP3, SFRP2, COL1A2, COL5A2, SERPINF1, KIF26B, TNFAIP6, ALPK2, CTSK, LOXL1 and FAP (optionally together with one or more up to all of the following biomarkers: MMP2 and FN1); and/or one or more up to all of the following biomarkers: GJB2, CDH11, COL10A1, ANGPTL2, THBS1, RAB31, THBS2, INHBA, MMP14, VCAN, PLAU, COL5A1 and FAP (optionally together with FN1).

In further embodiments the methods may further comprise measuring the expression levels of one or more, up to all of the biomarkers in Table 13 with an LCI C-index of more than 0.5. In yet further embodiments the methods may comprise measuring the expression levels of one or more, up to all of the top 10 ranked biomarkers in Table 14 and/or Table 15. In specific embodiments the methods may comprise measuring the expression levels of the sets of 17, 13, 11, 9, 8, 7, and 6 biomarkers listed below. Each of these signatures has been shown to give high levels of performance in identifying the relevant molecular subgroup of cancer:

| **22 gene** | | **19 gene** | | **17 gene** | | **13 gene** | | **11 gene** | |
|---|---|---|---|---|---|---|---|---|---|
| Entrez Gene ID | Gene Name | Entrez Gene ID | Gene Name | Entrez Gene ID | Gene Name | Entrez Gene ID | Gene Name | Entrez Gene ID | Gene Name |
| 1009 | CDH11 | 1009 | CDH11 | 1009 | CDH11 | 1009 | CDH11 | 1009 | CDH11 |
| 11031 | RAB31 | 11031 | RAB31 | 11031 | RAB31 | 11031 | RAB31 | 1289 | COL5A1 |
| 1278 | COL1A2 | 1289 | COL5A1 | 1289 | COL5A1 | 1289 | COL5A1 | 1300 | COL10A1 |
| 1281 | COL3A1 | 1300 | COL10A1 | 1300 | COL10A1 | 1300 | COL10A1 | 1462 | VCAN |
| 1289 | COL5A1 | 1462 | VCAN | 1462 | VCAN | 1462 | VCAN | 2191 | FAP |
| 1300 | COL10A1 | 2191 | FAP | 2191 | FAP | 2191 | FAP | 2706 | GJB2 |
| 1462 | VCAN | 2200 | FBN1 | 2200 | FBN1 | 2706 | GJB2 | 4323 | MMP14 |
| 2191 | FAP | 2335 | FN1 | 2335 | FN1 | 3624 | INHBA | 5328 | PLAU |
| 2200 | FBN1 | 23452 | ANGPTL2 | 23452 | ANGPTL2 | 4323 | MMP14 | 7057 | THBS1 |
| 2335 | FN1 | 2706 | GJB2 | 2706 | GJB2 | 5328 | PLAU | 7058 | THBS2 |
| 23452 | ANGPTL2 | 3624 | INHBA | 3624 | INHBA | 7057 | THBS1 | 9945 | GFPT2 |
| 2706 | GJB2 | 4060 | LUM | 4060 | LUM | 7058 | THBS2 | | |
| 3624 | INHBA | 4323 | MMP14 | 4323 | MMP14 | 9945 | GFPT2 | | |
| 3678 | ITGA5 | 5328 | PLAU | 5328 | PLAU | | | | |
| 4060 | LUM | 633 | BGN | 7057 | THBS1 | | | | |
| 4323 | MMP14 | 7057 | THBS1 | 7058 | THBS2 | | | | |
| 5328 | PLAU | 7058 | THBS2 | 9945 | GFPT2 | | | | |
| 633 | BGN | 9509 | ADAMTS2 | | | | | | |

| | | | | |
|---|---|---|---|---|
| 7057 THBS1<br>7058 THBS2<br>9509 ADAMTS2<br>9945 GFPT2 | 9945 GFPT2 | | | |

| 9 gene | | 8 gene | | 7 gene | | 6 gene | | 5 gene | |
|---|---|---|---|---|---|---|---|---|---|
| Entrez Gene ID | Gene Name | Entrez Gene ID | Gene Name | Entrez Gene ID | Gene Name | Entrez Gene ID | Gene Name | Entrez Gene ID | Gene Name |
| 1009 | CDH11 | 1289 | COL5A1 | 1462 | VCAN | 1462 | VCAN | 1462 | VCAN |
| 1289 | COL5A1 | 1462 | VCAN | 2191 | FAP | 2191 | FAP | 2191 | FAP |
| 1462 | VCAN | 2191 | FAP | 2706 | GJB2 | 2706 | GJB2 | 2706 | GJB2 |
| 2191 | FAP | 2706 | GJB2 | 5328 | PLAU | 7057 | THBS1 | 7057 | THBS1 |
| 2706 | GJB2 | 5328 | PLAU | 7057 | THBS1 | 7058 | THBS2 | 7058 | THBS2 |
| 5328 | PLAU | 7057 | THBS1 | 7058 | THBS2 | 9945 | GFPT2 | | |
| 7057 | THBS1 | 7058 | THBS2 | 9945 | GFPT2 | | | | |
| 7058 | THBS2 | 9945 | GFPT2 | | | | | | |
| 9945 | GFPT2 | | | | | | | | |

Combinations of these signatures are also envisaged, for example to generate suitable 2, 3, 4, 10, 12, 14, 16, 18, 20 and 21 gene signatures. Thus, for example, a 10 gene signature may be formed by adding a single gene to the 9 gene signature. This gene could be selected from the additional genes included in another signature, for example in the 11 gene signature. Alternatively it could be derived from elsewhere and tested according to the methods known in the art and described herein.

The expression levels of the biomarkers in these sets may be measured using the probesets listed in Tables E, F and L as appropriate for each biomarker.

**Table L - Probeset information for FBN1 and ADAMTS2**

| Probe Set ID | Type | Orientation | No. probes aligned | Ensembl Gene | Gene Symbol | Entrez Gene ID | Description | Chromosome | Strand | SEQ ID NO. |
|---|---|---|---|---|---|---|---|---|---|---|
| OC3P. 7887.C1_s_at | Expression probeset | Sense (Fully Exonic) | 11 | ENSG00000166147 | FBN1 | 2200 | fibrillin 1 [Source:HGNC Symbol;Acc:3603] | Chr 15 | Reverse Strand | 292 |
| OC3SNG. 6052-16a_s_at | Expression probeset | Sense (Fully Exonic) | 11 | ENSG00000166147 | FBN1 | 2200 | fibrillin 1 [Source:HGNC Symbol;Acc:3603] | Chr 15 | Reverse Strand | 293 |
| OC3SNGn. 8707-2674a_s_at | Expression probeset | Sense (Fully Exonic) | 11 | ENSG00000166147 | FBN1 | 2200 | fibrillin 1 [Source:HGNC Symbol;Acc:3603] | Chr 15 | Reverse Strand | 294 |
| OC3SNGnh. 5433_at | Expression probeset | Sense (includes Intronic) | 9 | ENSG00000166147 | FBN1 | 2200 | fibrillin 1 [Source:HGNC Symbol;Acc:3603] | Chr 15 | Reverse Strand | 295 |
| OCADA. 4751_s_at | Expression probeset | Sense (Fully Exonic) | 11 | ENSG00000166147 | FBN1 | 2200 | fibrillin 1 [Source:HGNC Symbol;Acc:3603] | Chr 15 | Reverse Strand | 296 |
| OCADNP. 2122_s_at | Expression probeset | Sense (Fully Exonic) | 9 | ENSG00000166147 | FBN1 | 2200 | fibrillin 1 [Source:HGNC Symbol;Acc:3603] | Chr 15 | Reverse Strand | 297 |
| OCMX. 14880.C1_s_at | Expression probeset | Sense (Fully Exonic) | 10 | ENSG00000166147 | FBN1 | 2200 | fibrillin 1 [Source:HGNC Symbol;Acc:3603] | Chr 15 | Reverse Strand | 298 |
| OC3SNGn. 1835-5a_s_at | Expression probeset | Sense (Fully Exonic) | 11 | ENSG00000087116 | ADAMTS2 | 9509 | ADAM metallopeptidase with thrombospondin type 1 motif, 2 [Source:HGNC Symbol;Acc:218] | Chr 5 | Reverse Strand | 299 |
| OCADA. 5272_s_at | Expression probeset | Sense (Fully Exonic) | 7 | ENSG00000087116 | ADAMTS2 | 9509 | ADAM metallopeptidase with thrombospondin type 1 motif, 2 [Source:HGNC Symbol;Acc:218] | Chr 5 | Reverse Strand | 300 |

(continued)

| Probe Set ID | Type | Orientation | No. probes aligned | Ensembl Gene | Gene Symbol | Entrez Gene ID | Description | Chromosome | Strand | SEQ ID NO. |
|---|---|---|---|---|---|---|---|---|---|---|
| OCADNP. 3907_s_at | Expressi on probeset | Sense (Fully Exonic) | 11 | ENSG00000087116 | ADAMTS2 | 9509 | ADAM metallopeptidase with thrombospondin type 1 motif, 2 [Source:HGNC Symbol;Acc:218] | Chr 5 | Reverse Strand | 301 |
| OCHPRC. 106_s_at | Expressi on probeset | Sense (Fully Exonic) | 11 | ENSG00000087116 | ADAMTS2 | 9509 | ADAM metallopeptidase with thrombospondin type 1 motif, 2 [Source:HGNC Symbol;Acc:218] | Chr 5 | Reverse Strand | 302 |

In particular embodiments the at least 1 biomarker selected from Table B is not COL5A1. In certain embodiments the at least 1 biomarker selected from Table A or Table B is not one or more up to all of ANGPTL2, CDH11, COL1A2, COL8A1, LOXL1, MMP14, POLD2 and/or TIMP3. Additionally or alternatively, in certain embodiments the at least 1 biomarker selected from Table A or Table B is not one or more up to all of CDH11, PMP22, LUM, COL3A1, VCAN, TNFAIP6, MMP2, FN1 and/or COL5A1. In further embodiments the at least 1 biomarker selected from Table A or Table B is not MMP2 and/or FN1. In specific embodiments the at least 1 biomarker selected from Table A or Table B does not consist of from 1 to 63 of the biomarkers shown in Table M. In further specific embodiments the EMT pathway inhibitor is ALM201 and the at least 1 biomarker selected from Table A or Table B does not consist of from 1 to 63 of the biomarkers shown in Table M.

**Table M**

| **GeneSymbol** |
|---|
| IGF2 |
| SOX11 |
| INS |
| CXCL17 |
| SLC5A1 |
| TMEM45A |
| CXCR2P1 |
| MFAP2 |
| MATN3 |
| RTP4 |
| COL3A1 |
| CDR1 |
| RARRES3 |
| TNFSF10 |
| NUAK1 |
| SNORD114-14 |
| SRPX |
| SPARC |
| GJB1 |
| TIMP3 |
| ISLR |
| TUBA1A |
| DEXI |
| BASP1 |
| PXDN |
| GBP4 |
| SLC28A3 |
| HLA-DRA |
| TAP2 |
| ACSL5 |
| CDH11 |

(continued)

| GeneSymbol |
|---|
| PSMB9 |
| MMP14 |
| CD74 |
| LOXL1 |
| CIITA |
| ZNF697 |
| SH3RF2 |
| MIR198 |
| COL1A2 |
| TNFRSF14 |
| COL8A1 |
| C21orf63 |
| TAP1 |
| PDPN |
| RHOBTB3 |
| BCL11A |
| HLA-DOB |
| XAF1 |
| ARHGAP26 |
| POLD2 |
| DPYSL2 |
| COL4A1 |
| ID3 |
| CFB |
| NID1 |
| FKBP7 |
| TIMP2 |
| RCBTB1 |
| ANGPTL2 |
| ENTPD7 |
| SHISA4 |
| HINT1 |

On the basis of the information provided herein other biomarker signatures may be derived by the skilled person for use according to the invention. By using one or more of the biomarker signatures described herein (such as the 15 or 45 gene signature) the skilled person could classify a sample set into those positive and negative for the biomarker signature. The skilled person could then derive further signatures using methods described herein or known in the art (such as partial least squares paired with forward feature selection) that reproduce the classification ability of the biomarker signatures described herein. Alternatively, the skilled person could carry out the gene expression profiling and hierarchical clustering described herein and in WO2012/167278 to identify samples that fall within the EMT/AngioImmune/MAPK pathway molecular subgroup of cancer identified by the present inventors. The skilled person could then use methods such as partial least squares paired with forward feature selection to derive further signatures that are able to detect the EMT/Angio-Immune/MAPK pathway molecular subgroup of cancer. The further signatures could be generated on an initial training dataset and then tested in a subsequent dataset for their ability to identify the EMT/Angio-Immune/MAPK pathway molecular subgroup of cancer or their classification ability.

Methods for determining the expression levels of the biomarkers are described in greater detail herein. Typically, the methods may involve contacting a sample obtained from a subject with a detection agent, such as primers/probes/antibodies (as discussed in detail herein) specific for the biomarker and detecting biomarker expression products.

According to all aspects of the invention the expression level of the gene or genes may be measured by any suitable method. Genes may also be referred to, interchangeably, as biomarkers. In certain embodiments the expression level is determined at the level of protein, RNA or epigenetic modification. The epigenetic modification may be DNA methylation.

The expression level may be determined by immunohistochemistry. By "Immunohistochemistry" is meant the detection of proteins in cells of a tissue sample by using a binding reagent such as an antibody or aptamer that binds specifically to the proteins. Accordingly, described herein is an antibody or aptamer that binds specifically to a protein product of at least one of the biomarkers described herein.

Antibodies useful for therapeutic and detection purposes as required herein may be of monoclonal or polyclonal origin. Fragments and derivative antibodies may also be utilised, to include without limitation Fab fragments, ScFv, single domain antibodies, nanoantibodies, heavy chain antibodies, aptamers, highly constrained bicyclic peptides ("bicycles") etc. which retain specific binding function and these are included in the definition of "antibody". Such antibodies are useful in the methods of the invention. Therapeutic antibodies may be conjugated to a drug to form an antibody drug conjugate. Many such ADC systems are known in the art. They may be used to measure the level of a particular protein, or in some instances one or more specific isoforms of a protein. The skilled person is well able to identify epitopes that permit specific isoforms to be discriminated from one another.

Methods for generating specific antibodies are known to those skilled in the art. Antibodies may be of human or non-human origin (e.g. rodent, such as rat or mouse) and be humanized etc. according to known techniques (Jones et al., Nature (1986) May 29-Jun. 4;321 (6069):522-5; Roguska et al., Protein Engineering, 1996, 9(10):895-904; and Studnicka et al., Humanizing Mouse Antibody Frameworks While Preserving 3-D Structure. Protein Engineering, 1994, Vol.7, pg 805).

In certain embodiments the expression level is determined using an antibody or aptamer conjugated to a label. By label is meant a component that permits detection, directly or indirectly. For example, the label may be an enzyme, optionally a peroxidase, or a fluorophore.

Where the antibody is conjugated to an enzyme a chemical composition may be used such that the enzyme catalyses a chemical reaction to produce a detectable product. The products of reactions catalyzed by appropriate enzymes can be, without limitation, fluorescent, luminescent, or radioactive or they may absorb visible or ultraviolet light. Examples of detectors suitable for detecting such detectable labels include, without limitation, x-ray film, radioactivity counters, scintillation counters, spectrophotometers, colorimeters, fluorometers, luminometers, and densitometers. In certain embodiments a secondary antibody is used and the expression level is then determined using an unlabeled primary antibody that binds to the target protein and a secondary antibody conjugated to a label, wherein the secondary antibody binds to the primary antibody.

Additional techniques for determining expression level at the level of protein include, for example, Western blot, immunoprecipitation, immunocytochemistry, mass spectrometry, ELISA and others (see ImmunoAssay: A Practical Guide, edited by Brian Law, published by Taylor & Francis, Ltd., 2005 edition). To improve specificity and sensitivity of an assay method based on immunoreactivity, monoclonal antibodies are often used because of their specific epitope recognition. Polyclonal antibodies have also been successfully used in various immunoassays because of their increased affinity for the target as compared to monoclonal antibodies.

Measuring mRNA in a biological sample may be used as a surrogate for detection of the level of the corresponding protein in the biological sample. Thus, the expression level of any of the genes described herein can also be detected by detecting the appropriate RNA. RNA from the sample may be converted into cDNA and the amount of the appropriate cDNA measured using any suitable method, for example via hybridization of (fluorescently labelled) probes. The amount of cDNA from the sample may then be compared with a reference amount of the relevant cDNA. cDNA based measurements may employ second generation sequencing technologies such as Illumina and Ion Torrent sequencing. Direct RNA measurements are also possible, for example using third generation sequencing technologies such as SMRT sequencing (Pacific Biosciences), nanopore sequencing and SeqLL (Helicos) sequencing.

Accordingly, in specific embodiments the expression level is determined by microarray, northern blotting, RNA-seq (RNA sequencing), in situ RNA detection or nucleic acid amplification. Nucleic acid amplification includes PCR and all variants thereof such as real-time and end point methods and qPCR. Other nucleic acid amplification techniques are well known in the art, and include methods such as NASBA, 3SR and Transcription Mediated Amplification (TMA). Other suitable amplification methods include the ligase chain reaction (LCR), selective amplification of target polynucleotide sequences (US Patent No. 6,410,276), consensus sequence primed polymerase chain reaction (US Patent No 4,437,975), arbitrarily primed polymerase chain reaction (WO 90/06995), invader technology, strand displacement technology, and nick displacement amplification (WO 2004/067726). This list is not intended to be exhaustive; any nucleic acid amplification technique may be used provided the appropriate nucleic acid product is specifically amplified. Design of suitable primers and/or probes is within the capability of one skilled in the art. Various primer design tools are freely available to assist in this process such as the NCBI Primer-BLAST tool. Primers and/or probes may be at least 15, 16, 17, 18, 19, 20, 21, 22, 23, 24 or 25 (or more) nucleotides in length. mRNA expression levels may be measured by reverse transcription quantitative polymerase chain reaction (RT-PCR followed with qPCR). RT-PCR is used to create a cDNA from the mRNA. The cDNA may be used in a qPCR assay to produce fluorescence as the DNA amplification process progresses. By comparison to a standard curve, qPCR can produce an absolute measurement such as number of copies of mRNA per cell. Northern blots, microarrays, Invader assays, and RT-PCR combined with capillary electrophoresis have all been used to measure expression levels of mRNA in a sample. See Gene Expression Profiling: Methods and Protocols, Richard A. Shimkets, editor, Humana Press, 2004.

RNA-seq uses next-generation sequencing to measure changes in gene expression. RNA may be converted into cDNA or directly sequenced. Next generation sequencing techniques include pyrosequencing, SOLiD sequencing, Ion Torrent semiconductor sequencing, Illumina dye sequencing, single-molecule real-time sequencing or DNA nanoball sequencing.

In situ RNA detection involves detecting RNA without extraction from tissues and cells. In situ RNA detection includes In situ hybridization (ISH) which uses a labeled (e.g. radio labelled, antigen labelled or fluorescence labelled) probe (complementary DNA or RNA strand) to localize a specific RNA sequence in a portion or section of tissue, or in the entire tissue (whole mount ISH), or in cells. A branched DNA assay can also be used for RNA in situ hybridization assays with single molecule sensitivity. This approach includes ViewRNA assays.

RNA expression may be determined by hybridization of RNA to a set of probes. The probes may be arranged in an array. Microarray platforms include those manufactured by companies such as Affymetrix, Illumina and Agilent. Examples of microarray platforms manufactured by Affymetrix include the U133 Plus2 array, the Almac proprietary Xcel™ array and the Almac proprietary Cancer DSAs®. In specific embodiments a sample of target nucleic acids is first prepared from the initial nucleic acid sample being assayed, where preparation may include labeling of the target nucleic acids with a label, e.g., a member of a signal producing system. Following target nucleic acid sample preparation, the sample is contacted with the array under hybridization conditions, whereby complexes are formed between target nucleic acids that are complementary to probe sequences attached to the array surface. The presence of hybridized complexes is then detected, either qualitatively or quantitatively. Specific hybridization technology which may be practiced to generate the expression profiles employed in the subject methods includes the technology described in U.S. Pat. Nos. 5,143,854; 5,288,644; 5,324,633; 5,432,049; 5,470,710; 5,492,806; 5,503,980; 5,510,270; 5,525,464; 5,547,839; 5,580,732; 5,661,028; 5,800,992; as well as WO 95/21265; WO 96/31622; WO 97/10365; WO 97/27317; EP 373 203; and EP 785 280. In these methods, an array of "probe" nucleic acids that includes a probe for each of the biomarkers whose expression is being assayed is contacted with target nucleic acids as described above. Contact is carried out under hybridization conditions, e.g., stringent hybridization conditions as described above, and unbound nucleic acid is then removed. The resultant pattern of hybridized nucleic acids provides information regarding expression for each of the biomarkers that have been probed, where the expression information is in terms of whether or not the gene is expressed and, typically, at what level, where the expression data, i.e., expression profile, may be both qualitative and quantitative.

In certain embodiments, measuring the expression levels of the at least 1 biomarker selected from Table A or Table B comprises contacting the sample with a set of nucleic acid probes or primers that bind to the at least 1 biomarker and detecting binding of the set of nucleic acid probes or primers to the at least 1 biomarker(s) by microarray, northern blotting, or nucleic acid amplification.

The methods described herein may further comprise extracting total nucleic acid or RNA from the sample. Suitable methods are known in the art and include use of commercially available kits such as RNeasy and GeneJET RNA purification kit.

In specific embodiments, expression of the at least one gene may be determined using one or more probes described herein.

These probes may also be incorporated into the kits of the invention. The probe sequences may also be used in order to design primers for detection of expression, for example by RT-PCR. Such primers may also be included in the kits of the invention.

The invention also relates to a system or device for performing a method as described herein.

Thus, the present invention relates to a system or test kit for selecting a treatment for a subject having a cancer, comprising:

(a) one or more testing devices for determining the expression level of at least 1 biomarker, wherein the at least 1 biomarker comprises GFPT2, in a sample from the subject
(b) a processor; and
(c) storage medium comprising a computer application that, when executed by the processor, is configured to:

(i) access and/or calculate the determined expression levels of the at least 1 biomarker in the sample on the one or more testing devices
(ii) calculate from the expression level(s) of the at least 1 biomarker whether the sample from the subject is positive or negative for a biomarker signature comprising the at least 1 biomarker; and
(iii) output from the processor the selected treatment.

In certain embodiments:

(a) if the sample is positive for the biomarker signature a MAPK pathway inhibitor is selected and/or if the sample is negative for the biomarker signature a MAPK pathway inhibitor is not selected; and/or
(b) if the sample is positive for the biomarker signature an EMT pathway inhibitor is selected and/or if the sample is negative for the biomarker signature an EMT pathway inhibitor is not selected; and/or
(c) if the sample is positive for the biomarker signature an SRC pathway inhibitor is not selected and/or if the sample is negative for the biomarker signature an SRC pathway inhibitor is selected; and/or
(d) if the sample is positive for the biomarker signature a taxane is selected and/or if the sample is negative for the biomarker signature a taxane is not selected.

In yet a further aspect, the present invention relates to system or test kit for predicting the responsiveness of a subject with cancer to a therapeutic agent comprising:

(a) one or more testing devices for determining the expression level of at least 1 biomarker, wherein the at least 1 biomarker comprises GFPT2, in a sample from the subject
(b) a processor; and
(c) storage medium comprising a computer application that, when executed by the processor, is configured to:

(i) access and/or calculate the determined expression levels of the at least 1 biomarker in the sample on the one or more testing devices
(ii) calculate from the expression level(s) of the at least 1 biomarker whether the sample from the subject is positive or negative for a biomarker signature comprising the at least 1 biomarker; and
(iii) output from the processor the predicted responsiveness.

In certain embodiments the subject is classified as

(a) predicted to be responsive to a MAPK pathway inhibitor if the sample is positive for the biomarker signature and/or predicted to be non-responsive to the MAPK pathway inhibitor if the sample is negative for the biomarker signature; and/or
(b) predicted to be responsive to an EMT pathway inhibitor if the sample is positive for the biomarker signature and/or predicted to be non-responsive to the EMT pathway inhibitor if the sample is negative for the biomarker signature; and/or
(c) predicted to be non-responsive to a SRC pathway inhibitor if the sample is positive for the biomarker signature and/or predicted to be responsive to the SRC inhibitor if the sample is negative for the biomarker signature; and/or
(d) predicted to be non-responsive to a platinum-based chemotherapeutic agent if the sample is positive for the biomarker signature and/or predicted to be responsive to a platinum-based chemotherapeutic agent if the sample is negative for the biomarker signature; and/or
(e) predicted to be responsive to a taxane if the sample is positive for the biomarker signature and/or predicted to be non-responsive to the taxane if the sample is negative for the biomarker signature.

The invention also relates to a system or test kit for determining the clinical prognosis of a subject with cancer comprising:

a) one or more testing devices for determining the expression level of at least 1 biomarker, wherein the at least 1 biomarker comprises GFPT2, in a sample from the subject
b) a processor; and
c) storage medium comprising a computer application that, when executed by the processor, is configured to:

(i) access and/or calculate the determined expression levels of the at least 1 biomarker in the sample on the one or more testing devices

(ii) calculate from the expression level(s) of the at least 1 biomarker whether the sample from the subject is positive or negative for a biomarker signature comprising the at least 1 biomarker; and

(iii) output from the processor the prognosis for the subject,

wherein the cancer is prostate cancer, colon cancer, bladder cancer, cervical cancer, glioblastoma, head and neck cancer, glioma, pancreatic cancer, melanoma, stomach cancer or lung cancer; and wherein the biomarker signature identifies the cancer as being in a molecular subgroup of cancer characterized by misregulation of the MAPK signaling pathway and the EMT pathway.

In certain embodiments the subject is classified as having a poor prognosis if the sample is positive for the biomarker signature and/or having a good prognosis if the sample is negative for the biomarker signature.

The system or test kit may further comprise a display for the output from the processor.

By testing device is meant a combination of components that allows the expression level of a gene to be determined. The components may include any of those described above with respect to the methods for determining expression level at the level of protein, RNA or epigenetic modification. For example the components may be antibodies, primers, detection agents and so on. Components may also include one or more of the following: microscopes, microscope slides, x-ray film, radioactivity counters, scintillation counters, spectrophotometers, colorimeters, fluorometers, luminometers, and densitometers.

The invention also relates to a computer application or storage medium comprising a computer application as defined above.

In certain example embodiments, provided is a computer-implemented method, system, and a computer program product for selecting a treatment for a subject having a cancer and/or prediction of the responsiveness of a subject with cancer to a therapeutic agent and/or determining the clinical prognosis of a subject with cancer, in accordance with the methods described herein. The computer program product comprises a non-transitory computer-readable storage device having computer-readable program instructions embodied thereon that cause the computer to:

(i) access and/or calculate the determined expression levels of the at least 1 biomarker comprising GFPT2 in a sample on one or more testing devices;

(ii) calculate from the expression level(s) of the at least 1 biomarker whether the sample from the subject is positive or negative for a biomarker signature comprising the at least 1 biomarker in the sample; and,

(iii) provide an output, wherein the output is selection of a treatment as defined herein, prediction of responsiveness to a therapeutic agent as defined herein or determination of clinical prognosis as defined herein.

In certain example embodiments, the computer-implemented method, system, and computer program product may be embodied in a computer application, for example, that operates and executes on a computing machine and a module. When executed, the application may select whether to administer a treatment to a subject having a cancer and/or predict the responsiveness of a subject with cancer to a therapeutic agent and/or determine the clinical prognosis of a subject with cancer, in accordance with the example embodiments described herein.

As used herein, the computing machine may correspond to any computers, servers, embedded systems, or computing systems. The module may comprise one or more hardware or software elements configured to facilitate the computing machine in performing the various methods and processing functions presented herein. The computing machine may include various internal or attached components such as a processor, system bus, system memory, storage media, input/output interface, and a network interface for communicating with a network, for example.

The computing machine may be implemented as a conventional computer system, an embedded controller, a laptop, a server, a customized machine, any other hardware platform, such as a laboratory computer or device, for example, or any combination thereof. The computing machine may be a distributed system configured to function using multiple computing machines interconnected via a data network or bus system, for example.

The processor may be configured to execute code or instructions to perform the operations and functionality described herein, manage request flow and address mappings, and to perform calculations and generate commands. The processor may be configured to monitor and control the operation of the components in the computing machine. The processor may be a general purpose processor, a processor core, a multiprocessor, a reconfigurable processor, a microcontroller, a digital signal processor ("DSP"), an application specific integrated circuit ("ASIC"), a graphics processing unit ("GPU"), a field programmable gate array ("FPGA"), a programmable logic device ("PLD"), a controller, a state machine, gated logic, discrete hardware components, any other processing unit, or any combination or multiplicity thereof. The processor may be a single processing unit, multiple processing units, a single processing core, multiple processing cores, special purpose processing cores, co-processors, or any combination thereof. According to certain example embodiments, the processor, along with other components of the computing machine, may be a virtualized computing machine executing within one or more other computing machines.

The system memory may include non-volatile memories such as read-only memory ("ROM"), programmable read-only memory ("PROM"), erasable programmable read-only memory ("EPROM"), flash memory, or any other device capable of storing program instructions or data with or without applied power. The system memory may also include volatile memories such as random access memory ("RAM"), static random access memory ("SRAM"), dynamic random access memory ("DRAM"), and synchronous dynamic random access memory ("SDRAM"). Other types of RAM also may be used to implement the system memory. The system memory may be implemented using a single memory module or multiple memory modules. While the system memory may be part of the computing machine, one skilled in the art will recognize that the system memory may be separate from the computing machine without departing from the scope of the subject technology. It should also be appreciated that the system memory may include, or operate in conjunction with, a non-volatile storage device such as the storage media.

The storage media may include a hard disk, a floppy disk, a compact disc read only memory ("CD-ROM"), a digital versatile disc ("DVD"), a Blu-ray disc, a magnetic tape, a flash memory, other non-volatile memory device, a solid state drive ("SSD"), any magnetic storage device, any optical storage device, any electrical storage device, any semiconductor storage device, any physical-based storage device, any other data storage device, or any combination or multiplicity thereof. The storage media may store one or more operating systems, application programs and program modules such as module, data, or any other information. The storage media may be part of, or connected to, the computing machine. The storage media may also be part of one or more other computing machines that are in communication with the computing machine, such as servers, database servers, cloud storage, network attached storage, and so forth.

The module may comprise one or more hardware or software elements configured to facilitate the computing machine with performing the various methods and processing functions presented herein. The module may include one or more sequences of instructions stored as software or firmware in association with the system memory, the storage media, or both. The storage media may therefore represent examples of machine or computer readable media on which instructions or code may be stored for execution by the processor. Machine or computer readable media may generally refer to any medium or media used to provide instructions to the processor. Such machine or computer readable media associated with the module may comprise a computer software product. It should be appreciated that a computer software product comprising the module may also be associated with one or more processes or methods for delivering the module to the computing machine via a network, any signal-bearing medium, or any other communication or delivery technology. The module may also comprise hardware circuits or information for configuring hardware circuits such as microcode or configuration information for an FPGA or other PLD.

The input/output ("I/O") interface may be configured to couple to one or more external devices, to receive data from the one or more external devices, and to send data to the one or more external devices. Such external devices along with the various internal devices may also be known as peripheral devices. The I/O interface may include both electrical and physical connections for operably coupling the various peripheral devices to the computing machine or the processor. The I/O interface may be configured to communicate data, addresses, and control signals between the peripheral devices, the computing machine, or the processor. The I/O interface may be configured to implement any standard interface, such as small computer system interface ("SCSI"), serial-attached SCSI ("SAS"), fiber channel, peripheral component interconnect ("PCI"), PCI express (PCIe), serial bus, parallel bus, advanced technology attached ("ATA"), serial ATA ("SATA"), universal serial bus ("USB"), Thunderbolt, FireWire, various video buses, and the like. The I/O interface may be configured to implement only one interface or bus technology.

Alternatively, the I/O interface may be configured to implement multiple interfaces or bus technologies. The I/O interface may be configured as part of, all of, or to operate in conjunction with, the system bus. The I/O interface may include one or more buffers for buffering transmissions between one or more external devices, internal devices, the computing machine, or the processor.

The I/O interface may couple the computing machine to various input devices including mice, touch-screens, scanners, electronic digitizers, sensors, receivers, touchpads, trackballs, cameras, microphones, keyboards, any other pointing devices, or any combinations thereof. The I/O interface may couple the computing machine to various output devices including video displays, speakers, printers, projectors, tactile feedback devices, automation control, robotic components, actuators, motors, fans, solenoids, valves, pumps, transmitters, signal emitters, lights, and so forth.

The computing machine may operate in a networked environment using logical connections through the network interface to one or more other systems or computing machines across the network. The network may include wide area networks (WAN), local area networks (LAN), intranets, the Internet, wireless access networks, wired networks, mobile networks, telephone networks, optical networks, or combinations thereof. The network may be packet switched, circuit switched, of any topology, and may use any communication protocol. Communication links within the network may involve various digital or an analog communication media such as fiber optic cables, free-space optics, waveguides, electrical conductors, wireless links, antennas, radio-frequency communications, and so forth.

The processor may be connected to the other elements of the computing machine or the various peripherals discussed herein through the system bus. It should be appreciated that the system bus may be within the processor, outside the processor, or both. According to some embodiments, any of the processor, the other elements of the computing machine, or the various peripherals discussed herein may be integrated into a single device such as a system on chip ("SOC"), system on package ("SOP"), or ASIC device.

Embodiments may comprise a computer program that embodies the functions described and illustrated herein, wherein the computer program is implemented in a computer system that comprises instructions stored in a machine-readable medium and a processor that executes the instructions. However, it should be apparent that there could be many different ways of implementing embodiments in computer programming, and the embodiments should not be construed as limited to any one set of computer program instructions. Further, a skilled programmer would be able to write such a computer program to implement one or more of the disclosed embodiments described herein. Therefore, disclosure of a particular set of program code instructions is not considered necessary for an adequate understanding of how to make and use embodiments. Further, those skilled in the art will appreciate that one or more aspects of embodiments described herein may be performed by hardware, software, or a combination thereof, as may be embodied in one or more computing systems. Moreover, any reference to an act being performed by a computer should not be construed as being performed by a single computer as more than one computer may perform the act.

The example embodiments described herein can be used with computer hardware and software that perform the methods and processing functions described previously. The systems, methods, and procedures described herein can be embodied in a programmable computer, computer-executable software, or digital circuitry. The software can be stored on computer-readable media. For example, computer-readable media can include a floppy disk, RAM, ROM, hard disk, removable media, flash memory, memory stick, optical media, magnetooptical media, CD-ROM, etc. Digital circuitry can include integrated circuits, gate arrays, building block logic, field programmable gate arrays (FPGA), etc.

Reagents, tools, and/or instructions for performing the methods described herein can be provided in a kit. There is provided a kit for use in a method for selecting a treatment for a subject having a cancer as described herein and/or for use in a method for predicting the responsiveness of a subject with cancer to a therapeutic agent as described herein and/or for use in a method of determining a clinical prognosis for a subject with cancer as described herein.

The kit may include reagents for collecting a tissue sample from a patient, such as by biopsy, and reagents for processing the tissue. Thus, the kit may include suitable fixatives, such as formalin and embedding reagents, such as paraffin. The kit can also include one or more reagents for performing an expression level analysis, such as reagents for performing nucleic acid amplification, including RT-PCR and qPCR, NGS (RNA-seq), northern blot, proteomic analysis, or immunohistochemistry to determine expression levels of biomarkers in a sample of a patient. For example, primers for performing RT-PCR, probes for performing northern blot analyses or bDNA assays, and/or antibodies or aptamers, as discussed herein, for performing proteomic analysis such as Western blot, immunohistochemistry and ELISA analyses can be included in such kits. Appropriate buffers for the assays can also be included. Detection reagents required for any of these assays can also be included. The kits may be array or PCR based kits for example and may include additional reagents, such as a polymerase and/or dNTPs for example. The kits featured herein can also include an instruction sheet describing how to perform the assays for measuring expression levels.

The kit may include one or more primer pairs and/or probes complementary to at least one gene selected from Table A or Table B. The kits may include one or more probes or primers (primer pairs) designed to hybridize with the target sequences or full sequences listed in Table A or Table B and thus permit expression levels to be determined. The probes and probesets identified in Table A and Table B may be employed according to all aspects of the invention.

The kits may include primers/primer pairs/probes/probesets to form any of the gene signatures specified herein.

The kits may also include one or more primer pairs complementary to a reference gene.

Such a kit can also include primer pairs complementary to at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, or 45 of the genes listed in Table A and/or primer pairs complementary to at least 2, 3, 4, 5, 6, 7, 8, 9, 10, 11, 12, 13, 14, or 15 of the genes listed in Table B.

There is provided a kit for use in a method for selecting a treatment for a subject having a cancer as described herein and/or for use in a method for predicting the responsiveness of a subject with cancer to a therapeutic agent as described herein and/or for use in a method of determining a clinical prognosis for a subject with cancer as described herein comprising one or more primers and/or primer pairs for amplifying and/or which specifically hybridize with at least one gene, full sequence or target sequence selected from Table A or Table B. There is also provided a kit for use in a method for selecting a treatment for a subject having a cancer as described herein and/or for use in a method for predicting the responsiveness of a subject with cancer to a therapeutic agent as described herein and/or for use in a method of determining a clinical prognosis for a subject with cancer as described herein comprising one or more probes that specifically hybridize with at least one gene, full sequence or target sequence selected from Table A or Table B.

By "probeset" is meant the collection of probes designed to target (by hybridization) a single gene.

Informational material included in the kits can be descriptive, instructional, marketing or other material that relates to the methods described herein and/or the use of the reagents for the methods described herein. For example, the informational material of the kit can contain contact information, e.g., a physical address, email address, website, or telephone number, where a user of the kit can obtain substantive information about performing a gene expression analysis and interpreting the results.

The kit may further comprise a computer application or storage medium as described above.

## DESCRIPTION OF THE FIGURES

Figure 1: **Identification of molecular subgroups of HGSOC**

**A.** Heat map showing unsupervised hierarchical clustering of gene expression data using the 1040 most variable genes in the Edinburgh 265 high grade serous ovarian carcinomas. Gene expression across all samples is represented horizontally. Functional processes corresponding to each gene cluster are labelled along the right of the figure. Angio (blue), Immune (green), and Angio_Immune (red) subgroups are labelled for each of the sample clusters, and colour coded along the top as described in the legend box. A gene expression signature to detect each of the subgroups was generated. **B.** Kaplan-Meier Progression-Free Survival analysis of subgroups as defined by unsupervised clustering analysis of Edinburgh 265 HGSOC Samples. Additionally Kaplan-Meier overall survival analysis of subgroups as defined by unsupervised clustering analysis of Edinburgh 265 HGSOC Samples. C. Kaplan-Meier to show the prognostic utility of the Angio_Immune subgroup in HGSOC (PFS HR 1.4 (1.092 to 1.880) p=0.0256 and OS HR 1.4 (1.05-1.87) p= 0.0224). **D.** Molecular subgroups are dynamic in the context of chemotherapy. The effect of chemotherapy treatment on 48 matched pre-chemotherapy and post-chemotherapy samples and analysis of subgroup switching based on assessment of the 3 gene signature scores (22 Angio signature; 63 Immune signature and 45 Angio_Immune signature) generated from the treatment niave Discover dataset.

Figure **2:Cisplatin resistant cell line models have elevated 45 gene signature score**

**A.** Generation of Cisplatin resistant OVCAR3 cell lines. 10-day colony formation assay assessing sensitivity of OVCAR3-WT and OVCAR3-CP cells to increasing concentrations of cisplatin. **B.** Cisplatin sensitive and resistant A2780 cell line models were scored with each of the 3 gene signatures and scores plotted in a bar graph, Angio_Immune (p=0.0057), Angio (p=0.3959) and Immune (p=0.0124). **C.** Cisplatin sensitive and resistant OVCAR3 cell line models were scored with each of the 3 gene signatures and scores plotted, Angio_Immune (p=0.0244), Angio (p=0.2478) and Immune (p=0.028). **D.** Western blot analysis showing increased MAPK signalling in the A2780 and OVCAR3 cisplatin resistant cells compared to cisplatin sensitive counterparts. **E.** Colony formation assay with cisplatin in 15 ovarian cell lines, plotting 45-gene signature scores based on median centred IC50 doses (AUC 0.7917 (0.6350-0.9483), p=0.0008) and plotting IC50 doses based on median centred signature scores (AUC 0.6838 (0.5184-0.8491), p=0.0377).

Figure **3:The Angio_Immune group is driven by the MAPK pathway**

**A.** Semi-supervised clustering analysis was performed on the Discovery dataset using the 3 public gene lists. Genes separating the ovarian samples were selected for further analysis. These were combined and a compilation gene list compiled and semi-supervised analysis of the Discovery dataset performed again. **B.** Venn diagrams illustrating the overlap of the 'MEK ON' population with the 3 gene signatures. This demonstrated 77% overlap with the Angio_Immune subgroup. **C.** TCGA ovarian samples were scored with the 3 ovarian gene signatures. Correlation with the gene signatures and the pMAPK RRPA data was investigated using ROC analysis. Each of the 3 gene signature scores of TCGA samples were median centered and defined as being High and Low scores. A ROC curve was generated using the binary signature scores and the continuous pMAPK expression (TCGA) in 237 samples. A statistically significant result was found with the 45- gene signature (p=0.04786) but not with the 63 or 22 gene signatures (p=0.4337 and p=0.4109 respectively). **D.** (i) Colony formation assay with Trametinib in 16 ovarian cell lines, plotting 45-gene signature scores based on median centred IC50 doses (AUC 0.7234 (0.5778-0.8690), p=0.0090) and plotting IC50 doses based on median centred signature scores (AUC 0.7147 (0.5674-0.8620), p=0.0117). **ii.** 881 cell lines from the Sanger center were scored with the 45 gene AngioImmune signature and correlated to IC50 response to Trametinib. AngioImmune gene signature scores were plotted based on median centred IC50 doses and IC50 doses were plotted based on median centred signature scores. **iii.** 760 cell lines from the Sanger center were scored with the 45 gene AngioImmune signature and correlated to IC50 response to Selumetinib. AngioImmune gene signature scores were plotted based on median centred IC50 doses and IC50 doses were plotted based on median centred signature scores.

Figure **4:The MEK signature is altered by KRAS status and MEK inhibitor**

**A.** The 45-gene, 22-gene and 63 gene signature scores from the E-GEOD-55624 data whereby SW480 cells (KRAS G12D) were treated with a MEK inhibitor for 4 and 16 hours. The Angio_Immune signature scores was significantly reduced post MEK inhibitor treatment at both 4 and 16 hours (p=0.0055 and p=0.0143 respectively). **B.** Differences in the 3 gene signatures between HCT116 (KRAS MT) and HKH2 cells (KRAS WT) using the E-MEXP-3557 dataset. The 45-gene signature scores were elevated in KRAS mutant cells. C. E-GEOD 12764: MCF10 breast cells transfected with empty vector (EV) or HRAS or MEK1 confirmed elevated 45-gene signature scores in the HRAS and MEK1 mutants (p=0.0004 and p<0.0001 respectively). **D.** Inhibition of MEK with Trametinib decreases the 45-gene Angio_Immune signature score in OVCAR3 cells (p=0.0011).

Figure **5:MEK inhibition in cisplatin resistant OVCAR3 cells, re-sensitises to cisplatin**
**A.** 10 day colony formation assay assessing sensitivity of OVCAR3-WT and OVCAR3-CP cells to increasing concentrations of Cisplatin and MEK inhibitor as single agents. **B.** 10 day colony formation assay assessing sensitivity of OVCAR3-WT and OVCAR3-CP cells to increasing concentrations of Trametinib (GSK1120212). Table shows IC50 values for OVCAR3-WT and OVCAR3-CP cells for Cisplatin and Cisplatin in combination with Trametinib.

Figure **6:The Angioimmune subgroup** is **associated with increased EMT signalling**
**A.** Box and whisker plots depicting the expression of EMT related genes across the 3 HGSOC molecular subgroups. Expression of VIM, AXL, TWIST1, SNAIL and SLUG is enhanced in the Angio_Immune subgroup (p<0.0001). **B.** Box and whisker plot of 45-gene signature scores in MCF7 control and SNAIL overexpressing cells (E-GEOD-58252). The 45-gene signature is enhanced by SNAIL overexpression (p=0.0004).

Figure 7: **Activation of the EMT phenotype is enhanced in Cisplatin resistant ovarian cell lines**
**A.** 10-day colony formation assay assessing sensitivity of OVCAR3-WT and OVCAR3-CP cells to increasing concentrations of cisplatin (left panel). Western blot analysis showing increased MAPK signalling in the OVCAR4 cisplatin resistant cells compared to cisplatin sensitive counterparts (right panel). **B.** Western blot analysis showing activation of EMT signalling in OVCAR3 CP and OVCAR4 CP (cisplatin resistant) with increased protein expression of Vimentin, N-cadherin and SLUG whilst decreasing protein expression of E-cadherin. B-actin was used as a loading control. C. Quantitative real-time PCR (qRT-PCR) expression of EMT markers (N-cadherin, SLUG, SNAIL, Vimentin, TWIST and TGF-$\beta$3) in cisplatin resistant OVCAR3 cells. Fold change plotted relative to wildtype counterparts. **D.** Quantitative real-time PCR (qRT-PCR) expression of EMT markers (N-cadherin, SLUG, SNAIL, Vimentin, TWIST and TGF-$\beta$3) in cisplatin resistant OVCAR4 cells. Fold change plotted relative to wildtype counterparts. **E.** Bar charts to show the fold change increase in migration of OVCAR3 and OVCAR4 cisplatin resistant cells compared to the wildtype ovarian cell lines.

Figure 8: **The EMT signature predicts resistance to inhibitors of the SRC pathway**
**A.** Representative western blot showing levels of phosphorylated ERK, and SRC following treatment of TOV112D cells with 1 $\mu$M SRC inhibitor, Saracatinib for 3,6,12 and 24 hours. Total ERK and total SRC expression are also shown. Beta actin was used as a loading control. Representative western blot showing levels of phosphorylated SRC and ERK following treatment of TOV112D cells with 1 $\mu$M MEK inhibitor, Trametinib for 3,6,12 and 24 hours. Total SRC and total ERK expression are also shown. Beta-actin was used as a loading control. **B.** Box and whisker plots showing differences in the 45-gene signature scores between SRC inhibitor resistant and sensitive cells.

Figure 9: **The MEK subgroup is present in colon cancer and the EMT signature is prognostic**
**A.** Heatmap representation of semi-supervised analysis of the MARISA dataset (GSE40967) using the Angio_Immune genes. Five individual clusters were identified, with Sample Cluster 3 (highlighted by the red box) defining the MEK driven subgroup. **B.** Kaplan-Meier to show the relapse-free survival of the five sample cluster groups. The MEK driven group represents poor prognosis in comparison to the other subgroups (p=0.037). **C.** Kaplan-Meier to show the relapse-free survival using the 45-gene signature scores from Marisa. The MEK ON group represents poor prognosis in comparison to the MEK OFF group (AUC 1.5949 (1.0951-2.3228), p=0.0063). **D.** Kaplan-Meier to show the disease-free survival using the 45-gene signature scores in the Jorissen dataset (GSE14333). The MEK ON group represents poor prognosis in comparison to the MEK OFF group (AUC 2.4543 (1.2049-4.999), p=0.0014).

Figure 10: **The MEK subgroup is present in NSCLC cancer and the EMT signature is prognostic**
**A.** Heatmap representation of semi-supervised analysis of the Okayama dataset (GSE31210) using the Angio_Immune genes. Five individual clusters were identified, with Sample Cluster 4 (highlighted by the red box) defining the MEK driven subgroup. **B.** Kaplan-Meier to show the relapse-free survival of the five sample cluster groups. The MEK driven group represents poor prognosis in comparison to the other subgroups (p=0.0004). C.

Kaplan-Meier to show the progression-free survival using the 45-gene signature scores from Okayama. The SIG POS group represents poor prognosis in comparison to the SIG NEG group (AUC 3.045 (1.631-5.686), p=0.0005). **D.** Kaplan-Meier to show the overall survival using the 45-gene signature scores in the Okayama dataset. The SIG POS group represents poor prognosis in comparison to the SIG NEG group (AUC 2.872 (1.271-6.489), p=0.0312).

Figure 11: **The 15 gene signature predicts cisplatin response and is elevated in cisplatin resistant cells**
**A.** Kaplan-Meier to show the prognostic utility of the 15-gene Angio_Immune subgroup in HGSOC (PFS HR = 1.3564 [1.0156-1.8117]; p = 0.0279 and OS HR = 1.3464 [0.9901-1.8308]; p = 0.0441). **B.** Colony formation assay with cisplatin in 15 ovarian cell lines, plotting 15-gene signature scores based on median centred IC50 doses (AUC 0.6905 (0.5254-8556), p=0.0290) and plotting IC50 doses based on median centred signature scores (AUC 0.6897 (0.5326-0.8468), p=0.02932). **C.** Cisplatin sensitive and resistant OVCAR3 cell line models were scored with the 15-gene signature and scores plotted in a box and whisker plot, (p=0.046).

Figure 12: **Association of the 15 gene signature with the MAPK pathway**
**A.** Differences in the 15-gene signature between HCT116 (KRAS MT) and HKH2 cells (KRAS WT) using the E-MEXP-3557 dataset. The 15-gene signature scores were elevated in KRAS mutant cells (p=0.0443). **B.** E-GEOD 12764: MCF10 breast cells transfected with empty vector (EV) or HRAS or MEK1 confirmed elevated 15-gene signature scores in the HRAS and MEK1 mutants (p<0.0001). **C.** Inhibition of MEK with Trametinib decreases the 15-gene Angio_Immune signature score in OVCAR3 cells (p=0.0023). **D.** Colony formation assay with Trametinib in 15 ovarian cell lines, plotting 15-gene signature scores based on median centred IC50 doses (AUC 0.850 (0.7366-0.9636), p<0.0001) and plotting IC50 doses based on median centred signature scores (AUC 0.737 (0.5820-0.8974, p=0.006495).

Figure 13: **The 15 gene signature is elevated by EMT**
Box and whisker plot of 15-gene signature scores in MCF7 control and SNAIL overexpressing cells (E-GEOD-58252). The 15-gene signature is enhanced by SNAIL overexpression (p=0.0015).

Figure 14: **The 15-gene EMT signature predicts resistance to inhibitors of the SRC pathway**
**A and B.** Box and whisker plots showing differences in the 15-gene signature scores between SRC inhibitor resistant and sensitive cells following treatment with Saracatinib. Median centered on signature score (AUC 0.7289 (0.5544-0.9035), p=0.01454) or median centered on IC50 of Saracatinib (AUC 0.7698 (0.6054-0.9343), p=0.004076).

Figure 15: **The 15 gene signature is prognostic in colon cancer**
**A.** Kaplan-Meier to show the disease-free survival using the 15-gene signature scores in the Jorissen dataset (GSE14333). The MEK ON group represents poor prognosis in comparison to the MEK OFF group (p=0.0328). **B.** Kaplan-Meier to show the relapse-free survival using the 15-gene signature scores from Marisa. The MEK ON group represents poor prognosis in comparison to the MEK OFF group (p=0.0161).

Figure 16: **The 15 gene signature** is **prognostic in NSCLC cancer**
**A.** Kaplan-Meier to show the progression-free survival using the 15-gene signature scores from Okayama. The SIG POS group represents poor prognosis in comparison to the SIG NEG group (p=0.0024). B. Kaplan-Meier to show the overall survival using the 15-gene signature scores in the Okayama dataset. The SIG POS group represents poor prognosis in comparison to the SIG NEG group (p=0.0396).

Figure 17: **Scatterplots of combined variance-intensity rank of the 19920 Entrez gene IDs in Ovarian Cancer, Colon Cancer, Lung Cancer and Melanoma**

Figure 18: **Intersection of top ranked genes within different disease indications**

Figure 19: **C-index figures within cross validation in the training dataset**

Figure 20: **Curve of sensitivity and specificity to determine threshold for classification of MEK signature (X: 0.5899, Y: 1.567)**

Figure 21: **Functional analysis of gene ontology (GO) biological processes (BP) for probeset clusters identified in hierarchical clustering.**

Figure 22: **Core set analysis: Tothill_HR_Final_Core Set Analysis_15 Gene**

Figure 23: **Core set analysis: ICON7_HR_Final_Core Set Analysis_15 Gene**

Figure 24: **Minimum gene set analysis: Tothill_Validation _Min Gene Analysis_15 Gene**

Figure 25: **Minimum gene set analysis: ICON7_Validation_Min Gene Analysis_15 Gene**

Figure 26: **Core set analysis: Tothill_HR_Final_Core Set Analysis_45 Gene**

Figure 27: **Core set analysis: ICON7_HR_Final_Core Set Analysis_45 Gene**

Figure 28: **Minimum gene set analysis: Tothill_Validation_Min Gene Analysis _45 Gene**

Figure 29: **Minimum gene set analysis: ICON7_Validation_Min Gene Analysis_45 Gene**

Figure 30: **ALM201 reverses mesenchymal markers in the Kuramochi and OVCAR3 cisplatin-resistant cells**

**A.** Western Blot analysis showing reversal of the EMT pathway and downregulation of the MAPK pathway by ALM201 in ovarian Kuramochi cells.
**B.** Western blot demonstrating activity of ALM201 in reversing EMT markers, downregulation of the MAPK pathway following addition of 1nM and 10nM ALM201 at 24 hour treatment time point in the OVCAR3 cisplatin resistant cell line.
**C.** 10-day colony formation assay assessing sensitivity of OVCAR3-WT, OVCAR3-CP, OVCAR4-WT and OVCAR4-CP cells to increasing concentrations of ALM201. Table shows IC50 values for OVCAR3-WT and OVCAR3-CP cells for Cisplatin and Cisplatin in combination with ALM201.
**D.** xCELLigence migration and invasion assay illustrating that 0.1nM, 1nM and 10nM ALM201 inhibits migration (p=0.08544, p=0.015522 and p=0.036739, respectively) and invasion (p=0.0211, p=0.0026 and p=0.3373, respectively) in the OVCAR3 platinum resistant cell line.

Figure 31: **The 45 gene and 15 gene signatures are predictive of response to MEK inhibitors**
**A.** 739 cell lines from 'The Genomics of Drug Sensitivity in Cancer Project' (http://www.cancerrxgene.org/) were scored with the 45-gene AngioImmune signature and correlated to IC50 response to Trametinib. AngioImmune gene signature scores were plotted based on median centred IC50 doses and IC50 doses were plotted based on median centred signature scores. **B.** 759 cell lines from the 'The Genomics of Drug Sensitivity in Cancer Project' were scored with the 45-gene AngioImmune signature and correlated to IC50 response to Selumetinib. AngioImmune gene signature scores were plotted based on median centred IC50 doses and IC50 doses were plotted based on median centred signature scores. **C.** 739 cell lines from 'The Genomics of Drug Sensitivity in Cancer Project' (http://www.cancerrxgene.org/) were scored with the 15-gene AngioImmune signature and correlated to IC50 response to Trametinib. AngioImmune gene signature scores were plotted based on median centred IC50 doses and IC50 doses were plotted based on median centred signature scores. **D.** 760 cell lines from the 'The Genomics of Drug Sensitivity in Cancer Project' were scored with the 15-gene AngioImmune signature and correlated to IC50 response to Selumetinib. AngioImmune gene signature scores were plotted based on median centred IC50 doses and IC50 doses were plotted based on median centred signature scores.

Figure 32: **The 45 gene signature is predictive of response to taxanes**
A.Scatter plot of 45-gene signature scores across two clinical groups, PSA responders and PSA non-responders. **B.** Kaplan-Meier to show patient survival using the 45-gene signature scores in response to taxane based chemotherapy in prostate cancer. The EMT positive group (blue) represents the good prognosis group who respond well to taxane in comparison to the EMT negative group (red) **C.** Table representing the breakdown of PSA responders and non-responders who are EMT positive and negative within the pilot cohort.

Figure 33: **The 15 gene signature is predictive of response to taxanes**
**A.Scatter** plot of 15-gene signature scores across two clinical groups, PSA responders and PSA non-responders. **B.** Kaplan-Meier to show patient survival using the 15-gene signature scores in response to taxane based chemotherapy in prostate cancer. The EMT positive group (blue) represents the good prognosis group who respond well to taxane in comparison to the EMT negative group (red) **C.** Table representing the breakdown of PSA responders and non-responders who are EMT positive and negative within the pilot cohort.

Figure 34: **EMT signature is prognostic in Prostate Cancer, predicting disease recurrence and poor prognosis**

**post radical surgery**
Kaplan-Meier to show prognostic relevance of the 15-gene signature scores in predicting biochemical recurrence in prostate cancer. The EMT positive group (15-gene signature high) (blue) represents the poor prognosis group who have poorer survival and greater chance of biochemical recurrence in comparison to the EMT negative group (15-gene signature low) (green).

Figure 35: **EMT signature is prognostic in Prostate Cancer, predicting disease recurrence, metastasis and poor prognosis post radical radiotherapy**
A.Kaplan-Meier to show prognostic relevance of the 15-gene signature scores in predicting biochemical recurrence in prostate cancer. The EMT positive group (15-gene signature high) (green) represents the poor prognosis group who have poorer survival and greater chance of biochemical recurrence in comparison to the EMT negative group (15-gene signature low) (blue). **B.** Kaplan-Meier to show prognostic relevance of the 15-gene signature scores in predicting biochemical recurrence in prostate cancer. The EMT positive group (15-gene signature high) (green) represents the poor prognosis group who have poorer survival and greater chance of metastatic progression in comparison to the EMT negative group (15-gene signature low) (blue).

**Figure 36: The 15 gene signature is prognostic in multiple diseases**
**A.** Kaplan-Meier to show the disease-free survival using the 15-gene signature scores in the TCGA RNA-seq dataset across multiple diseases (shown in B) The MEK/EMT ON group represents poor prognosis in comparison to the MEK/EMT OFF group). **B.** Table showing hazard ratios and statistical significance of EMT biomarker across individual diseases.

**Figure 37: Platinum therapy demonstrates a selection pressure for an angiogenesis enriched tumor microenvironment A.** CD31 Immunohistochemistry (IHC) quantification of micro vessel density (MVD) of 12 matched pairs of patient samples pre- and post- platinum-based chemotherapy. Specifically, samples were obtained at diagnosis and then from debulking surgery following relapse after completion of chemotherapy. This data shows that chemotherapy creates a selection pressure for an angiogenesis-dependent tumour microenvironment. Tumours that have relapsed following platinum therapy have acquired higher micro-vessel density (MVD) compared to their treatment naïve pair (p-value= <0.0001). B. The post-platinum treatment patient samples have higher 15 gene signature score than is paired platinum-naïve tissue (p-value: 0.0094)

**Figure 38: Angiogenesis assay using Matrigel plugs in Athymic nude mice illustrating that the OVCAR3 cisplatin resistant cell have hallmarks of vascular mimicry**
OVCAR3 and OVCAR4 HGSOC cell lines were continuously exposed to increasing concentrations cisplatin over 6 months to generate cisplatin resistant OVCAR3CP and OVCAR4CP cells respectively. In-vivo matrigel plug assay to demonstrate the MVD in the OVCAR3 isogenic cell lines. H&E quantification of MVD of the OVCAR3 isogenic cell lines shows that co-culturing the OVCAR3 CP cell lines with ECFCs have a higher MVD (p-value: 0.0041) compared to the parental cell lines (p-value: 0.8712). The OVCAR3 CP cell lines has a higher 15-gene signature score relative to the OVCAR3 WT cell line (p-value: 0.046)

**Figure 39: Platinum resistant cell lines are associated with expression of ligands and their reciprocal RTK associated with the angiogenesis process.**
Cytokine array shows that the platinum resistant OVCAR3 (A) and OVCAR4 (B) have higher expression of cytokines that a key regulators of angiogenesis (C) Western blot showing that VEGFa protein expression levels are higher in OVCAR3 and OVCAR4 cisplatin-resistant cells in comparison to the OVCAR3 and OVCAR4 cisplatin-naive cells.

**Figure 40: Table depicting the expression of Receptor tyrosine kinases (RTKs) and associated ligands and genes involved in the EMT in the AngioImmune molecular subgroup and the pre/post chemotherapy samples.**

A. AngioImmune subgroup is characterised by expression of RTKs that are key regulators of the mesenchymal and proliferative phenotype in ovarian cancer compared to the other 2 subgroups.
B. Pre chemotherapy samples verses post-chemotherapy samples. RTKs shown represent those which were statistically associated either by ROC analysis (AUC) or student t-test where indicated.

**Figure 41: Platinum resistant cell lines are associated with expression of RTK associated with the angiogenesis process**

A. pRTK array shows that the OVCAR3 cisplatin-resistant cell line has higher basal expression of pRTK relative to the platinum-naïve OVCAR3 pair.

B. Further validation of the pRTK array by western blot shows basal upregulation of phospho-VEGFR2, VEGFR3, PDGFRα and phospho-AXL in the OVCAR3 cisplatin-resistant relative to the OVCAR3 cisplatin-naïve pair

**Figure 42: TKIs have specificity for platinum resistant OVCAR3 isogenic cell line**

A. 10-day colony formation assay of Cediranib in the OVCAR3 isogenic cell lines demonstrates sensitivity for the OVCAR3 cisplatin-resistant (IC50 1.194) relative to the OVCAR3 cisplatin-naïve cell line (IC50 4.994).

B. 10-day colony formation assay of Nintedanib in the OVCAR3 isogenic cell lines demonstrates sensitivity for the OVCAR3 cisplatin-resistant (IC50 3.777) relative to the OVCAR3 cisplatin-naïve cell line (IC50 >10).

**Figure 43: TKIs that target downstream RTKs lead to inhibition of tumor VEGFa expression.**

A CellTiter Glo assay was carried out to determine the IC50 for Cediranib (IC50 5.569μM at 48 hour time point) and Nintedanib (IC50 9.097μM at 48 hour time point).

B. Western blot showing that VEGFa protein expression levels are down-regulated in OVCAR3 and OVCAR4 cisplatin-resistant cells treated with an IC50 concentration of Cediranib and Nintedanib.

**Figure 44: Angiogenesis assay using Matrigel plugs in Athymic nude mice: Effect of Bevacizumab on MVD**
In-vivo matrigel plug assay to demonstrate the effect of bevacizumab on MVD in the OVCAR3 isogenic cell lines. IF quantification of MVD of the OVCAR3 isogenic cell lines shows that co-culturing the OVCAR3 CP cell lines with ECFCs have a higher MVD (p-value: 0.0024) compared to the parental cell lines (p-value: 0.84525).

**EXAMPLES**

The present invention will be further understood by reference to the following experimental examples.

**EXAMPLE 1**

***MEK activation is associated with a molecular subgroup in high grade serous ovarian cancer***

Epithelial ovarian cancer (EOC) ranks among the top ten diagnosed and top five deadliest cancers in most countries (Ferlay et al., 2010). Continental rates are highest in Europe (10.1 per 100,000) with 41,448 deaths from ovarian cancer in 2008, representing 5.5% of all female cancer deaths in Europe. The high death rate is because most patients (>60%) are diagnosed at an advanced stage of disease (Stage III and IV) (Vaugh et al., 2012). The most common type of EOC is high-grade serous ovarian cancer (HGSOC) which accounts for at least 70% of cases, the majority of which are stage III and IV disease (Bowtell, 2010). Currently, the standard treatment used in initial management is cytoreductive surgery and adjuvant chemotherapy with a platinum-based regimen. However, despite an initial complete clinicalresponse rate of 65%-80%, most stage III and IV ovarian carcinomas relapse with an overall 5-year survival rate of only 10%-30% and a median survival of 2 to 3 years (www.cancerresearchuk.org). Classic clinicopathological factors, such as age, stage, residual tumour after surgery, differentiation grade and histopathological features, are currently the most important prognostic markers, but it is not possible to select optimal chemotherapy on an individual patient basis using these factors. Over the past 20 years there has been very little progress in the treatment of HGSOC, with five-year survival figures remaining unchanged for stage III and IV disease (www.cancerresearchuk.org).

A number of studies have tried to characterise the mechanisms of acquired resistance in ovarian cancer. Analysis of 135 spatially and temporally separated samples from 14 patients with HGSOC who received platinum-based chemotherapy found that NF1 deletion showed a progressive increase in tumour allele fraction during chemotherapy (Schwarz et al., 2015). This suggested that subclonal tumour populations are present in pre-treatment biopsies in HGSOC and can undergo expansion during chemotherapy, causing clinical relapse (Schwarz et al., 2015). Additionally alteration of the NF1 gene has been associated with innate cisplatin resistance in HGSOC, whereby 20% of primary tumours showed inactivation of the NF1 gene by mutation or gene breakage (Patch et al., 2015). Furthermore mutation of the RAS-MAPK has been associated with chemotherapy resistance in relapsed neuroblastomas (Eleveld et al., 2015). Additionally, in cell line models, the MAPK pathway has been implicated in cisplatin resistance in ovarian cancer (Benedetti et al., 2008) and in squamous cell carcinoma (Kong et al., 2015).

**Key Messages**

- MAPK is a pathway of innate and acquired resistance in High Grade Serous Ovarian Cancer (HGSOC).
- After cisplatin treatment, samples switch molecular groups, move into a MAPK/EMT molecular group and become more angiogenic.
- We have developed a 45 and 15 gene expression signature which can detect the MAPK molecular subgroup.
- 45 and 15 gene signatures predict sensitivity to drugs targeting the MAPK pathway: MEK inhibitors.
- 45 and 15 gene signatures predict resistance to SRC inhibitors.
- 45 and 15 gene signatures detect a cisplatin resistant group and predicts resistance to cisplatin.
- 45 and 15 gene signatures predict a bad prognosis molecular subgroup in colon cancer (CRC) and non-small cell lung cancer (NSCLC).

**Materials & Methods**

***High Grade Serous Ovarian Cancer (HGSOC) Tumour Material***

This study performed gene expression analysis of a cohort of 265 macrodissected ovarian cancer FFPE tissue samples sourced from the Edinburgh Ovarian Cancer Database. Ethical approval for Edinburgh dataset analysis was obtained from Lothian Local Research Ethics Committee 2 (Ref: 07/S1102/33).

This cohort of samples can be further described using the following inclusion criteria:

- Primary ovarian, peritoneal or fallopian tube cancer
- High grade serous histology
- Treatment-naïve FFPE tissue samples
- Matched pre chemotherapy and post-chemotherapy samples

***Prostate Tumour Material***

Three separate prostate cancer cohorts were sourced and used to assess the association of EMT with Prostate Cancer prognosis.

1) Pilot Cohort - 56 prostate biopsy samples with de novo metastatic disease, collected in collaboration with NI Biobank
2) Resection Cohort - multicentre retrospective cohort of 322 prostatectomy specimens collected from Wales Cancer Bank, University College Dublin, University of Surrey and Oslo University Hospital.
3) Biopsy Cohort - retrospective radiation cohort of 248 prostate biopsy specimens collected in collaboration with FASTMAN- Movember Centre of Excellence.

***Gene Expression Profiling of HGSOC and Prostate samples***

Total RNA was extracted from the macrodissected FFPE tumour samples using the Roche High Pure RNA Paraffin Kit (Roche Diagnostics GmbH, Mannheim, Germany) as described previously (Kennedy et al, 2011). Total RNA was amplified using the NuGEN WT-Ovation™ FFPE System (NuGEN Technologies Inc., San Carlos, CA, USA). It was then hybridised to the Almac Ovarian Cancer DSA™ as described previously (Kennedy et al, 2011) or Prostate DSA™ for prostate samples (Tanney et al, 2008). Arrays were scanned using the Affymetrix Genechip® Scanner 7G (Affymetrix Inc., Santa Clara, CA).

***Data Preparation & Hierarchical Clustering***

Quality Control (QC) of profiled samples was carried out using MAS5 pre-processing algorithm to assess technical aspects of the samples i.e. average noise and background homogeneity, percentage of present call (array quality), signal quality, RNA quality and hybridization quality. Distributions and Median Absolute Deviation of corresponding parameters were analyzed and used to identify possible outliers. Sample pre-processing was carried out using RMA (Irizarry *et al,* 2003). The pre-processed data matrix was sorted by decreasing variance, decreasing intensity and increasing correlation to cDNA yield. Following filtering of probe sets (PS) correlated with cDNA yield (to remove any technical bias in the expression data), hierarchical clustering analysis was performed (Pearson correlation distance and Ward's linkage methods (Ward *et al,* 1963). Subsets of the data matrix were tested for cluster stability using the GAP statistic (Tibshirani *et al,* 2001), which gives an indication of the within-cluster tightness and between-cluster separateness. The GAP statistic was applied to calculate the optimal number of sample clusters in each sub-matrix, while the stability of cluster composition was assessed using a partition comparison tool (Carriço *et al,* 2006; Pinto *et al,* 2008). The smallest number of PS generating the optimal sample cluster number was selected as the list of most variable PS to take forward for hierarchical cluster analysis.

*Functional Analysis of 3 Molecular Gene Clusters*

To establish the functional significance of the gene clusters an enrichment analysis, based on the hypergeometric function (False Discovery Rate applied (Benjamini and Hochberg, 1995, J. R. Stat. Soc. 57:289:300)), was performed. Over-representation of biological processes and pathways were analysed for each gene group generated by the hierarchical clustering using Gene Ontology biological processes. Hypergeometric p-values were assessed for each enriched functional entity class. Functional entity classes with the highest p-values were selected as representative of the group and a general functional category representing these functional entities was assigned to the gene clusters based on significance of representation (i.e. p-value).

*Gene Selection for Signature Generation*

Genes that are variable and highly expressed across multiple disease indications were determined prior to model development. The disease indications that were included in this evaluation were: ovarian cancer; colon cancer; lung cancer and melanoma. Two data sets per disease indication were assessed with the exception of prostate cancer where only one dataset was evaluated. Data sets were pre-processed using RMA and summarised to Entrez Gene ID level using the median of probe sets for each Entrez Gene ID on the Ovarian Cancer DSA™. Within each data set, Entrez Gene IDs were ranked based on the average rank by variance and mean intensity across samples (high rank = high variance, high mean intensity). A single combined rank value per gene was calculated based on the average variance-intensity rank within each disease indication. Genes with no expression level were removed from further analysis. Scatterplots were generated to show the combined variance-intensity rank of the 19920 Entrez gene IDs in the disease indications evaluated with two datasets (Figure 17: (a) ovarian cancer; (b) colon cancer; (c) lung cancer; and (d) Melanoma) where the x and y axis represent the rank for the two data sets evaluated within each indication. A final classification of expressed genes as high/low rank was defined within each disease indication. Finally the overlap in high ranking genes across disease indications was determined and the top 75% ranked genes were identified. This list was then used as the starting list for signature generation (Figure 18 & Table 1).

*Signature Generation of the 45-gene and 15-gene signatures*

The genes that had common high expression and variance in ovarian, colon, lung, melanoma and prostate were used as a starting set for model development. The Edinburgh ovarian cancer sample cohort was used to train the signature under 5 fold cross validation (CV) with 10 repeats. Partial least squares (PLS) (de Jong, 1993) was paired with Forward Feature Selection (FFS) to generate signatures for the top 75% ranked list. Table 4 indicates the weightings and bias for each probeset incorporated within the 45-gene signature (A) and the 15-gene signature (B).

*Model Selection and Signature Validation for the 15-gene signature*

The C-index performance was calculated using the progression free survival (PFS) time endpoint and signature scores generated within cross validation for each evaluated signature length. This data was then used to determine the signature length at which optimal performance is reached with respect to association between signature scores and PFS. The highest C-index values were compared for signatures of length less than 100 and greater than 10 features. The signature with the shortest length and highest C-index within this subset was selected as the final model for identifying the subgroup.

Figure 19 shows the C-index performance calculated under cross validation for the training set across all feature lengths, from a maximum of 5000 genes, removing 10% at each feature selection iteration until a minimum of 5 features. The C-index performance metric was the primary metric analysed for model selection. The C-index was significant across the majority of feature lengths in the training set (Figure 19) and the C-index performance was highest at a feature length of 15 (56.62 [57.86-55.55]). Table 2 lists the Entrez Gene ID and corresponding Gene Symbol for the 15 gene signature.

A threshold was generated for classification of signature scores by using the value where the sum of sensitivity and specificity with respect to predicting the subtype in the training data is highest. This threshold was set at 0.5899 using the curve of sensitivity and specificity (Figure 20). Samples with scores above the selected threshold would be classified as MEK ON whereas samples with scores below or equal to the threshold would be labelled as MEK OFF.

*Functional Analysis*

Functional enrichment analysis of the selected model was performed using the Gene Ontology biological processes classification to gain an understanding of the underlying biology behind the selected signature. Table 3 presents the top 20 GO biological processes and GO terms from functional enrichment analysis of the signature, where the top 20 biological processes include:

- Angiogenesis (p = 2.09e-05)
- Blood vessel development (p = 5.55e-06)
- Cell-cell junction organization (p = 2.55e-05)

**Cell culture and reagents:**

Human epithelial ovarian cancer cell lines OVCAR3 and OVCAR4 were obtained from the American Type Culture Collection. Tumour cells were cultured in RPMI (Gibco™ Life technologies) supplemented with 20% foetal calf serum (FCS) nd maintained in 5% CO2 at 37C. Pharmaceutical grade cisplatin and bevacizumab were kindly provided by the Belfast City Hospital pharmacy department. Cediranib and Nintedanib were purchased through Selleckchem and re-suspended in DMSO to a stock concentration of 10mM.

***Colony Formation Assays***

Cells were seeded at predetermined densities, 24 hours later treated with drug, which was replenished every 3-4 days. After 10 days, cells were washed with PBS, fixed in methanol, stained with crystal violet and colonies counted. The surviving fraction (SF) for a given dose was calculated and dose-response curves plotted and IC50 generated using GraphPad Prism™ 5. Receiver operator curves (ROC) were plotted by dicotomising the IC50 values based on the median of the IC50 and defining the higher IC50 values as resistant and the lower IC50 values as sensitive. The gene signatures associated with the cell lines were plotted based on sensitive and resistant cells. Additionally ROCs were plotted by dicotomising the signature scores based on the median of the scores and defining the higher signature score as signature positive and the lower signature scores as signature negative. The IC50s associated with the cell lines were plotted based on signature positive and signature negative cells.

***Migration Assay***

The migration assay was performed using the xCELLigence RTCA DP system and carried out with CIM-plate 16 (ACEA Bioscience). Endothelial progenitor cell conditioned media, fresh endothelial media with growth factors (VEGF, IGF-1, bFGF, EGF) with 10% foetal bovine serum (FBS) and endothelial media with 10% foetal bovine serum (FBS) only, were the three chemoattractant conditions used in the bottom chamber. 160 μl of the chemo-attractant was added to each bottom chamber of a CIM-plate 16. The CIM-Plate 16 is assembled by placing the top chamber onto the bottom chamber and snapping the two together. 30 μl pf serum-free medium is placed in the top chamber to hydrate and pre-incubate the membrane for 2 hours in the CO2 incubator at 37 °C before obtaining a background measurement. The protocol is optimised for the two paired cancer cell lines: OVCAR3, OVCAR4 parental and OVCAR3, OVCAR4 platinum resistant cell lines. Platinum resistant cell lines were washed x3 with PBS, to remove cisplatin, and fresh platinum free media was added to the cells for 24 hours prior to carrying out the experiment. Cells were then grown in serum free medium for 2 hours prior to seeding. Cells are lightly trypsinised, pelleted and re-suspended at 100 μl, containing 50,000 cells, in serum-free medium. Once the CIM-Plate 16 has been equilibrated, it is placed in the RTCA DP station and the background cell-index values are measured. The CIM-Plate 16 is then removed from the RTCA DP station and the cells are added to the top chamber. The CIM-Plate 16 is placed in the RTCA DP station and migration is monitored every 5 minutes for several hours. Each experimental condition was performed in triplicate. For quantification of the migration rate, the slope of the curve was used to determine the rate if change in cell index. The average and standard deviation slope values were then quantified relative to the controls.

***Invasion Assay***

The invasion assay was performed using the xCELLigence RTCA DP system and carried out with CIM-plate 16 (ACEA bioscience).

Normal cell media growth conditions (RPMI 1640, 1% L-Glut and 20% FCS) was the chemoattractant condition used in the bottom chamber. 160μl of the chemoattractant was added to each bottom chamber of a CIM-plate 16. The CIM-Plate 16 is assembled by placing the top chamber onto the bottom chamber and snapping the two together. 20μl Matrigel growth factor reduced (GFR) (phenol-red free) basement membrane matrix (Cornig, ref: 356231) was diluted in 400μl optimem (serum free) giving a final working concentration of GFR Matrigel of 5%. 20μl of the Matrigel-optimem master mix is placed in the top chamber to hydrate and pre-incubate the membrane for 2 hours in the CO2 incubator at 37 °C before obtaining a background measurement.

The protocol is optimized for the two paired cancer cell lines: OVCAR3, OVCAR4 parental and OVCAR3, OVCAR4 platinum resistant cell lines. Platinum resistant cell lines were grown for 24 hours in media containing 0.1nM, 1nM and 10nM ALM201. On the experimental day, cells are washed x1 with PBS. Cells are lightly trypsinized, pelleted and re-suspended at 100μl, containing 50,000 cells, in optimem (serum-free) medium in the presence of 0.1nM, 1nM and 10nM ALM201. Once the CIM-Plate 16 has been equilibrated, it is placed in the RTCA DP station and the background cell-index values are measured. The CIM-Plate 16 is then removed from the RTCA DP station and the cells are added to the top chamber. The CIM-Plate 16 is placed in the RTCA DP station and migration is monitored every 5 minutes for several hours. Each experimental condition was performed in triplicate. For quantification of the migration rate, the slope of the curve was used to determine the rate if change in cell index. The average and standard deviation slope values were then quantified relative to that at the control condition.

***Proliferation (3-day) assay***

The proliferation assay was performed using 6-well plates. The experiment was set-up with two controls mechanisms to ensure accuracy of results. Each cell line was seeded in duplicates and the experiment was performed in triplicate. For quantification of proliferation, cell numbers were counted manually using a coulter counter on day 1, day 2 and day 3 in three different concentrations of ALM201 (0.1nM, 1nM and 10nM). Media is changed on day 2 and day 3 with fresh media containing the 3 concentrations of ALM201.

On day 0, each cell line was lightly trypsinized, counted and seeded at a concentration of $5\times10^4$ per well in the presence of ALM201 (0.1nM, 1nM and 10nM concentration). 2mls of cells was added to each well in three 6 well plates (representing day 1, 2 and 3) and left to incubate for 24 hours in the CO2 incubator at 37 °C prior to counting cells for day 1, 48 hour incubation prior to count day 2 and 72 hour incubation prior to counting day 3.

At each time point, media was aspirated from the wells and wells were washed with PBS x1. 500μl 5% trypsin was added to each well and incubated 3-5 mins. 1.5mls media was added to each well to neutralise the trypsin. Cells were counted using the coulter counter. To estimate significance, the unpaired, two-tailed student T-test was calculated using the T-test calculator available on GraphPad Prism 5.0 software.

***Therapeutic Agents***

Dasatinib (BMS354825), Saracatinib (AZD0530) and Trametinib (GSK1120212) were purchased from Selleck Chemicals, dissolved in DMSO to constitute a 10 mM stock solution, and stored at -20°C. Cisplatin was acquired from Belfast City Hospital Pharmacy department and diluted in PBS to produce a 10 μM stock solution. Cisplatin was stored at room temperature and protected from light.

***Generation of OVCAR3 and OVCAR4 Cisplatin Resistant Cell Lines***

OVCAR3 and OVCAR4 cells were trypsinised and relevant cell numbers were seeded into P90 plates. Cells were allowed to adhere overnight. The following day media was removed and replaced with media containing 25 nM cisplatin. The concentration of cisplatin was increased every 2 weeks, doubling the concentration at every increment. Batches of cells were frozen every two weeks upon increasing the concentration of cisplatin. Once cells were stably growing at 200 nM cisplatin, sensitivity to cisplatin was tested by clonogenic assay. Cells were continuously grown in 200 nM cisplatin.

**Cell viability assay:**

Cells were trysanised and counted using the Countess™ Automated Cell Counter. 5,000 cells were seeded into each well of a 96 well plate. Cells were allowed to adhere overnight and were then treated with titrated concentrations of cisplatin, cedirianib and nintedanib (10μM to 0.005μM concentration). Under sterile conditions in tissue culture, at the 24 hour time point, the drug-conditioned media was removed and replaced with 100μl of fresh media. The 96 well plate was allowed to stand at room temperature for 20-30 mins. In the meantime the CellTiter-Glo Luminescent was allowed to thaw from the -20 freezer. Following the 20-30 minute incubation, 75μl of the CellTiter-Glo Luminescent was added per well, this was then shaken for 2 minutes and then allowed to stand for 10 minutes. The analysis was performed using the Bioscience BioTek plate reader.

**In-vivo matrigel plug angiogenesis assay:**

All animal experiments were performed in conformity to UK Home Office Regulations (PPL2729) and with authorization from Queen's University Belfast Animal Welfare and Ethical Review Body (AWERB). Eight week-old male Athymic nude mice (Harlan Laboratories) were used. ECFC were inoculated at a high density of 2.45 x 106 and co-cultured with GFP-labelled OVCAR3 WT or GFP-labelled OVCAR3-CP at a density of 1.4 x 106. The GFP-labelled OVCAR3 WT and GFP-labelled OVCAR3-CP were seeded alone at a density of 1.4 x 106. Each condition was diluted in 10 μL of phenol red-free DMEM and re-suspended in 90 μL of growth factor-reduced Matrigel (Corning) and injected subcutaneously. After 8 days, mice were sacrificed under isoflurane anaesthesia using 31 gauge needless intraperitoneal (IP) administration of sodium pentobarbital at 200 mg/kg, and implants were removed and fixed in 4% formaldehyde overnight. Fixed Matrigel implants were then embedded in paraffin and 10 μm sections were prepared for staining. For 5mg/Kg bevaciziumab was administered IP once weekly for 14 days. Treatment was commenced on day 3. Mice were sacrificed after a 14-day treatment period as previously described.

**Human Angiogenesis Antibody Array**

Protein lysate was obtained from OVCAR3 isogenic cell lines and analysed using the proteome profiler human angiogenesis array (R&D Systems, Abingdon, UK) in accordance with the manufacturer guidelines. Briefly, samples were adjusted to a final volume of 1.5ml with array buffer and mixed with a detection antibody cocktail for 1 hour. After a membrane blocking step, samples containing antibody cocktail were added to membranes and left to incubate on a rocking platform overnight at 4°C. After several washes, membranes were incubated with strepavidin-horseradish peroxidase secondary antibody and spots were detected using a UVP bioimaging system (Millipore). Densitometry was quantified using Image J software.

**Human Cytokine antibody Array:**

Conditioned media was collected from OVCAR3 and OVCAR4 isogenic cell lines and added to the cytokine array (Abcam) membranes. The experiment was carried out in accordance with the manufacturer guidelines. Comparison between samples was performed using Image J densitometry software for a semi-quantitative comparison.

**Xenograft immunostaining - Histologic and immunofluorescence analysis**

IHC and immunofluorescence studies were conducted as previously describe [22]. Conventional H&E staining was done and examined by light microscopy. Immunofluorescence was done using anti-mouse CD31 (1:00; Baca), anti-rabbit α-smooth muscle actin (a-SMA; 1:100; Baca). For micro vessel counts, paraffin embedded tissues were sectioned and stained with anti-CD31 and anti-aSMA antibody. CD31 and αSMA stained vessels were then counted per high power field (200x) in three separate fields of three independent tumors from each group. Blood vessels associated with α-SMA-positive cells were considered mature. Sections were stained with α-SMA and with anti-CD31, which stains both mature and immature vessels.

***Quantitative Real-Time Polymerase Chain Reaction (qRT-PCR)***

Reverse transcription was performed using the First Strand cDNA synthesis kit (Roche). 500 ng of RNA was reverse transcribed according to manufacturer's instructions. Exon-spanning qPCR primers were designed using Roche Universal Probe Library Assay Design Centre and were used at a concentration of 0.5 μM. The following primer sequences were used:

| | | |
|---|---|---|
| **N-cadherin** | | |
| Forward | CTC-CAT-GTG-CCG-GAT-AGC (SEQ ID NO. 278) | |
| Reverse | | CGA-TTT-CAC-CAG-AAG-CCT-CTA-C (SEQ ID NO. 279) |
| **SLUG** | | |
| Forward | TGT-TGC-AGT-GAG-GGC-AAG-AA (SEQ ID NO. 280) | |
| Reverse | | GAC-CCT-GGT-TGC-TTC-AAG-GA (SEQ ID NO. 281) |
| **SNAIL** | | |
| Forward | ACC-ACT-ATG-CCG-CGC-TCT-T (SEQ ID NO. 282) | |
| Reverse | | GGT-CGT-AGG-GCT-GCT-GGA-A (SEQ ID NO. 283) |
| **Vimentin** | | |
| Forward | TGG-TCT-AAC-GGT-TTC-CCC-TA (SEQ ID NO. 284) | |
| Reverse | | GAC-CTC-GGA-GCG-AGA-GTG (SEQ ID NO. 285) |

(continued)

| | | |
|---|---|---|
| **TWIST** | | |
| Forward | AGC-TAC-GCC-TTC-TCG-GTC-T (SEQ ID NO. 286) | |
| Reverse | | CCT-TCT-CTG-GAA-ACA-ATG-ACA-TC (SEQ ID NO. 287) |
| **TGF-β3** | | |
| Forward | AAG-TGG-GTC-CAT-GAA-CCT-AA (SEQ ID NO. 288) | |
| Reverse | | AAA-TTC-ACT-CTG-CCC-AGG-ACG (SEQ ID NO. 289) |
| **PUM1(Housekeepinq qene)** | | |
| Forward | 5' CCA | GAA AGC TCT TGA GTT TAT TCC 3' (SEQ ID NO. 290) |
| Reverse | 5' CAT | CTA GTT CCC GAA CCA TCT C 3' (SEQ ID NO. 291) |

To preform absolute quantification from qPCR, we used a standard curve method. The efficiency of each primer set was derived from the standard curve using the following equation:

$$E= 10^{(-1/slope)}$$

The product of Reverse Transcription was diluted 1:10 in Nuclease Free Water (NFW). Each 10 μl PCR reaction, consisted of 0.5 μl of 10 μM Forward primer, 0.5μl of 10 μM Reverse primer, 5 μl of 2X LightCycler® 480 SYBR Green I Master mix (Roche), 1.5 μl NFW and 2.5 μl diluted Reverse Transcription product. These 10 μl reactions were pipetted into wells of a LightCycler® 480 multiwell 96 plate (Roche), the plate was then sealed using clear adhesive film (Roche). The plate was placed into the LightCycler® 480 (Roche) and run with the following protocol. (95°C for 10 mins, 45 cycles of; 95°C for 15 secs, 55°C for 30 secs and 72°C for 30 secs, finishing with a melt curve for confirmation of primer specificity. All qPCR data was analysed using the LightCycler® 480 software provided by Roche. For analysis, the Cp value from a technical duplicate was calculated and the relative amount of a gene was calculated Cp value to an in-run standard curve. Each mean value was then normalised to the mean concentration of the housekeeping gene PUM1 within the corresponding sample, by dividing the concentration of the target gene by the concentration of the house keeping gene. Relative expression refers to the gene expression levels which have been normalised to the housekeeping gene and made relative to the associated control samples. From these normalized values, the fold changes for each gene were calculated and the average of three individual fold changes were derived from three independent experimental triplicates. The unpaired, two-tailed students T-test available on GraphPad Prism 5.0 software was used to detect statistical significance.

### *Western Blotting*

30 μg of protein lysates were mixed with LDS loading dye (Invitrogen) and Reducing Agent (Invitrogen) and denatured for 10 minutes at 70 ºC. Samples were briefly centrifuged, and loaded onto a Bolt 4-12% Bis-Tris gel and electrophoresed at 165 V for 1 hour 30 minutes using MOPS running buffer. SeeBlue Pre Stained protein ladder (Invitrogen) was used as a reference for protein size. After electrophoresis proteins were transferred onto immobilon-P PVDF membrane (Millipore) at 30 V for 2 hours using the XCell surelock mini-cell transfer system (Invitrogen). To ensure proper transferring of proteins onto membrane, the membrane was stained with Ponceau S solution (Sigma). Membranes were incubated in blocking solution (5% bovine serum albumin) for 1 hour at room temperature on a rocking platform in order to prevent non-specific binding of antibody to membrane. Membranes were then incubated in primary antibody overnight (see appendix 3 for diltuions) at 4 oC. The following day, the membranes were washed 3 times in TBS-T for 10 minutes and incubated in secondary antibody at a 1:5000 dilution 1 hour 30 minutes at room temperature. Membranes were then washed 6 times for 5 minutes in TBS-T, and incubated for 5 minutes in Luminata Cresendo or Forte (Millipore) detection reagent. Analysis was performed using Alpha Innotech Imager FlourChem Software.

### *Antibodies*

**ERK (Cell Signalling 2496)** - monoclonal mouse antibody for total p44/42 MAP Kinase (ERK1/2), used at a 1:1000 dilution in 5% milk.

**pERK (Cell Signalling 4370)** - polyclonal rabbit antibody for p44 and p42 MAP Kinase (ERK1/2), used at a 1:1000 dilution in 5% BSA.

**MEK (Cell Signalling 4694)** - mouse monoclonal antibody for total MEK1/2, used at a dilution of 1:1000 in 5% milk.
**pMEK (Cell Signalling 9121)** - rabbit polyclonal antibody for phosph0-MEK1/2 at Ser217/221, used at a dilution of 1:1000 in 5% BSA.
**SRC (Cell Signalling 2123)** - rabbit polyclonal antibody for SRC, used at a dilution of 1:000 in 5% BSA.
**pSRC (Cell Signalling 2101)** - polyclonal rabbit antibody for phosphor-SRC at tyrosine 416, used at a dilution of 1:1000 in 5% BSA.
**N-cadherin (Cell Signalling)** - rabbit monoclonal, used at a dilution of 1:1000 in 5% milk.
**E-cadherin (Cell Signalling 24E10)** - rabbit monoclonal antibody used at a dilution of 1:1000 in 5% milk.
**Vimentin (Cell Signalling R28)** - rabbit monoclonal antibody to detect Vimentin, used at a dilution of 1:1000 in 5% milk.
**SLUG (Cell Signalling C10G7)** - rabbit monoclonal antibody to detect SLUG EMT marker, used at a dilution of 1:1000 in 5% milk.
**VEGFa (Abcam)** - rabbit polyclonal antibody to detect VEGFa, used at a dilution of 1:1000 in 5% BSA.
**VEGFR1 (Abcam)** - rabbit polyclonal antibody, a dilution of 1:500 in 5% BSA. Phospho-VEGFR2 (Cell Signalling) - rabbit polyclonal antibody, a dilution of 1:500 in 5% BSA.
**VEGFR2 (Cell Signalling)** - rabbit polyclonal antibody, a dilution of 1:500 in 5% BSA.
**PDGFRα (Cell Signalling)** - rabbit polyclonal antibody, a dilution of 1:500 in 5% BSA.
**Phospho-PDGFRβ (Cell Signalling)** - rabbit polyclonal antibody, a dilution of 1:500 in 5% BSA.
**Phospho-AXL (Cell Signalling)** - rabbit polyclonal antibody, a dilution of 1:500 in 5% BSA.
**AXL (Cell Signalling)** - rabbit polyclonal antibody, a dilution of 1:500 in 5% BSA.
**B-actin (Sigma A2228)** - mouse monoclonal antibody detecting the N-terminus of β-actin, used at a dilution o 1:5000 in 5% milk.
**Secondary antibodies** - anti-rabbit and anti-mouse (Cell Signalling) were used at a dilution of 1:5000 in 5% milk.

## Results

### *Identification of molecular subgroups of High Grade Serous Ovarian Cancer (HGSOC)*

We have defined 3 molecular subgroups of High grade serous ovarian cancer (HGSOC), an Angiogenesis subgroup (HGS1), an Immune subgroup (HGS2) and an Angio_Immune subgroup (HGS3) (Figure 1a) using gene expression data from 265 FFPE HGSOC samples obtained from treatment naive patients but who were treated with carboplatin + paclitaxel or carboplatin only Standard of Care (SoC) chemotherapy (Gourley, Kennedy et al., manuscript in preparation). Functional analysis of the gene clusters revealed one subgroup was characterised by up-regulation of immune response genes (Immune subgroup), a second by up-regulation of angiogenesis/vascular development genes (Angio subgroup) and a third by up-regulation of both immune response and angiogenesis/vascular development genes (AngioImmune subgroup). Functional analysis of the gene clusters revealed that two of the 4 gene clusters had no significantly enriched processes (PS clusters 1 & 3), the third was characterised by Immune processes (PS cluster 2) and the fourth by Angiogenesis processes (PS cluster 4) (Figure 21). Patients within these 3 molecular subgroups respond differently to SoC surgery and chemotherapy. The Immune group (green) has the best prognosis, represented by both increased progression-free survival (PFS) (HR=0.60 (0.44-0.82) compared to Angio_Immune subgroup and HR=0.64 (0.49-0.92) compared to Angio subgroup) and overall survival (OS) (HR=0.58 (0.41-0.82) compared to Angio_Immune subgroup and (HR=0.55 (0.37-0.80) compared to Angio subgroup), while the Angio (blue) and Angio_Immune (red) subgroups respond similarly (Figure 1b and 1c). We have defined this as the Discovery dataset.

### *The 45 gene signature is prognostic in HGSOC*

The Angio_Immune subgroup is defined by the 45-gene signature. We hypothesised that the Angio_Immune group would be prognostic in the context of SoC treatment in ovarian cancer. We therefore investigated this in the treatment naive Discovery dataset. The 45-gene signature was associated with worse prognosis (PFS HR 1.6403 (1.2252-2.1960) p=0.0002 and OS HR 1.6562 (1.2169-2.2540) p= 0.0004) and therefore predicted response to cisplatin based therapy (Figure 1c).

### *Effect of cisplatin treatment on molecular subgroups*

We wanted to investigate the effect of platinum treatment on the previously identified molecular subgroups. To do this, we analysed 48 matched (from the same patient) pre-chemotherapy and post-chemotherapy samples by gene expression analysis on the ovarian DSA™. Each of the samples were then scored with each of the 3 gene signatures, the 22 Angio signature, the 63 gene Immune signature and the 45 Angio_Immune signature. This analysis allowed us to define which of the 3 molecular subgroups, the samples fell into. This analysis demonstrated that treatment with cisplatin based chemotherapy caused samples to move between subgroups, specifically more of the post-chemotherapy samples were aligned with the Angio_Immune subgroup, rather than the immune and Angio subgroups. 40% of the pre-treatment patient samples were aligned with the Angio_Immune subgroup which increased to 54% post-chemotherapy (Figure 1d). This was especially evident in the Immune group where 52% of samples previously associated with the good prognosis Immune group pre chemotherapy treatment, were found to be associated with the bad prognosis subgroup, the Angio-Immune group post-chemotherapy. This suggests that samples move into the Angio_Immune group and out of the Immune groups after cisplatin treatment. Hence the Angio_Immune subgroup represents a subgroup of tumours which are cisplatin insensitive upfront but which also provide a mechanism of acquired resistance post-chemotherapy treatment. 12 matched pre and post-platinum chemotherapy patients sample were stained with CD31 to assess micro-vessel density (MVD) and determine whether platinum therapy promotes an increased tumour vascular infiltration. The post-platinum chemotherapy patient samples have a higher 15-gene signature score relative to their paired treatment-naïve pair (paired t-test; p-value: 0.0094) (Figure 37). Quantification of MVD in the paired samples demonstrates that the post-platinum patient treatment samples have a higher MVD relative to their paired treatment-naïve pair (paired t-test; p-value: 0.0001) (Figure 37).

***Cisplatin resistant cell line models have elevated 45 and 15-gene signature scores***

We used a number of cisplatin-sensitive and -resistant cell lines to model the Angio_immune group *in vitro* including the A2780 and A2780CP and a further cisplatin-sensitive and -resistant cell line generated in-house using the HGSOC OVCAR3 cell line. As high-grade serous ovarian cancer accounts for 70% of ovarian cancers (Seidman et al. 2004) we used the OVCAR3 cell line as they have been confirmed as high-grade serous (Domcke et al. 2013). Although this cell line was generated from a patient following treatment with platinum based chemotherapy, our research has demonstrated that this cell line remains sensitive to cisplatin treatment. The cisplatin resistant OVCAR3 cells had an IC50 of 0.29 $\mu$M compared to the cisplatin sensitive cells which had an IC50 of 0.066 $\mu$M representing a 4.4-fold difference in IC50 doses (Figure 2a).

Furthermore, both cisplatin resistant cells (A2780CP and OVCAR3PT) had significantly increased 45-gene signature scores (Angio_Immune) compared to their sensitive counterparts (p=0057 and p=0.0244 respectively) (Figure 2b and 2c). Additionally the OVCA3PT cells had elevated 15 gene signature score compared to OVCAR3 sensitive counterparts (p=0.045) (Figure 11c). This was specifically related to the Angio_Immune subgroup as the 22-gene Angio signature scores remained unchanged, and the 63-gene Immune group signature was decreased between resistant and sensitive cells (Figure 2b and 2c). It has been previously shown that the MAPK pathway is a mechanism of cisplatin resistance in multiple solid tumours including ovarian cancer (Schwarz et al., 2015, Patch et al., 2015, Benedetti et al., 2008, Eleveld et al., 2015). We therefore hypothesised that MAPK signalling may have been driving the Angio_Immune subgroup. Interestingly, both cisplatin resistant A2780 and OVCAR3 (A2780-CP and OVCAR3-CP) cells had increased MAPK signalling compared to the cisplatin sensitive counterparts, as measured by phospho-MEK and phospho-ERK protein expression (Figure 2d, top panels).

Since analysis of the clinical samples of the Discovery dataset demonstrated that the 45-gene signature could predict response to cisplatin based SoC treatment, we used a panel of 15 ovarian cell lines to investigate this further. These cells lines were analysed by DNA microarray analysis using the Ovarian Cancer DSA™ and signature scores were generated as previously described. The cell lines were also used to perform colony formation assays with Pharmaceutical grade cisplatin. A ROC curve was generated. Cisplatin resistance (res) or sensitivity (sens) was defined based on the median of the IC50 values and correlation with signature scores and AUC scores determined. This demonstrated that in cell line models the 45-gene signature could predict resistance to cisplatin upfront as shown by the increased 45-gene signature scores in resistant ovarian cell line panels (res) (AUC 0.7917 (0.6350-0.9483), p=0.0008) (Figure 2e). Additionally ROC generated using the median of the signature scores demonstrated that signature positive (sign pos) were resistant to cisplatin compared to signature negative (sign neg) cells (AUC 0.6838, (0.5184-0.8492) P= 0.03477). In contrast the Angio or the Immune signatures did not predict resistance to cisplatin.

***The Angio-_Immune subgroup is driven by MAPK signalling***

To further investigate whether the Angio_Immune group was driven by this MAPK signalling pathway, we performed *in silico* analysis of the gene expression data from the Discovery dataset. To do this we firstly identified 3 different gene lists representing MAPK activation from the literature (Dry et al., 2010, Loboda et al., 2010, Creighton et al., 2006). We used these gene lists separately to perform semi-supervised hierarchical clustering of the Discovery dataset which were then compiled to generate a refined gene list representing a MAPK driven patient population ('MEK ON', represented by the red box) (Figure 3a). In total, this analysis identified 101 samples of the 285 Discovery dataset as having increased MAPK signalling. The overlap of these MAPK driven patients with the 3 identified molecular subgroups is represented by the Venn diagrams in Figure 3b. In total, 77% (56/73 patients) of the 'MEK ON' patients fall within the Angio_Immune group, whilst only 6% (4/63 patients) and 17% (10/60 patients) of the 'MEK ON' patients fall into the Angio and Immune subgroups respectively (Figure 3b). This suggests that the Angio_Immune subgroup represents the 'MEK ON' population and this molecular subgroup is driven by MAPK signalling.

Reverse Phase Proteomic Array (RPPA) data was utilised from The Cancer Genome Atlas (TCGA) dataset. The continuous Phospho-MAPK (pMAPK) scores (serine 217/221) and total MAPK scores were downloaded from TCGA (http://bioinformatics.mdanderson.org/main/TCPA). Phospho-MAPK scores were calculated as a ratio of total MAPK. Gene signature scores were then correlated with the RPPA data and the Angio_Immune gene signature was specifically found to correlate with pMAPK serine 217/221 expression using ROC analysis (Figure 3c). Both the Angio and Immune gene signatures did not significantly correlate with pMEK expression. Significant differences in pMAPK expression were observed between the Angio_Immune subgroup and the Angio and Immune subgroups respectively (p=0.0057 and p=0.0250) (Figure 3c). This indicates that the Angio_Immune subgroup is being driven by the MAPK pathway and is associated with MEK expression.

Furthermore the 45-gene signature could predict sensitivity to the MEK inhibitor Trametinib (Mekinist, GSK) as demonstrated by the increased 45-gene signature score in sensitive ovarian cell line panels (sens) (AUC 0.72 (0.5778-0.8690) p= 0.009066) (Figure 3d). Additionally ROC generated using the median of the signature scores demonstrated that signature positive (sign pos) were sensitive to Trametinib compared to signature negative (sign neg) cells (AUC 0.7147 (0.5674-0.8620) P= 0.01170). This phenotype was specifically related to the 45-gene signature as the Angio and Immune signatures did not predict sensitivity. The Angio_Immune subgroup may now be defined by the 45-gene signature or 'MEK signature'.

*Assessment of 45 gene expression signature with drug response in Sanger cell lines*

Gene expression data (Affymetrix U219 chip) downloaded from https://www.ebi.ac.uk/arravexpress/experiments/E-MTAB-3610/ and cell lines sensitivity data with regards to the MEK inhibitors Trametinib and Selumetinib downloaded from http://www.cancerrxgene.org/. The cancer cell lines were scored with the 45 gene AngioImmune signature and correlations of signature score and IC50 response were determined as before: based on median centred IC50 doses against signature scores and vice versa plotting median centered signature scores against IC50 doses. ROC analysis demonstrated the 45 gene signature could predict response to both MEK inhibitors in these cell lines. For Trametinib there was 881 solid tumour cell lines which had both gene expression data and drug IC50 data and for Selumetinib there was 760 solid tumour cell lines which had both gene expression data and drug IC50 data.

*The 45-gene signature is altered by KRAS status and MEK inhibitors*

As mentioned previously, it has been found that 11% of HGSOC have KRAS amplification and 12% have BRAF amplification, highlighting the potential that drugs targeting the RAS/RAF/MEK/ERK pathway may have utility in HGSOC. The link between KRAS status and MEK was assessed using three publically available datasets; E-GEOD-55624 which profiles KRAS mutant cancer cells treated with a MEK inhibitor, E-MEXP-3557 which transcriptionally profiles human KRAS mutant and wildtype cells and E-GEOD-12764 which transcriptionally profiled MCF10 cells with overexpressed HRAS or MEK1. Using the E-GEOD-55624 dataset, following treatment of SW480 cells which harbour a KRAS mutation (G12D), with a MEK inhibitor exhibited a reduction in the 45-gene Angio_Immune signature scores at both 4 and 16 hours (p=0.0055 and p=0.0143), in comparison to DMSO control (Figure 4a). Of note, the reduction is enhanced post MEK inhibitor treatment at 16 hours. This effect was specific to the Angio_Immune subgroup, as both the Angio and Immune signatures remained unchanged. Furthermore, data from the E-MEXP-3557 dataset showed that KRAS mutant (KRAS MT) HCT116 cells had an association with the Angio_Immune subgroup with higher 45-gene signature scores compared to the wildtype HKH2 cells (KRAS WT) (p=0.0072) (Figure 4b). No association was observed between KRAS status and either the Angio or Immune gene signatures. Moreover, MCF10 epithelial breast cells transfected with either HRAS or MEK1 also show an association with the 45-gene signature (p=0.0004 and p=<0.0001 respectively) compared to the empty vector (EV) (Figure 4c). As expected, no significant changes or association were observed between HRAS or MEK1 and the Angio or Immune subgroups. Moreover, inhibition of MEK with Trametinib significantly decreases the 45-gene Angio_Immune signature score in OVCAR3 cells (p=0.0011) (Figure 4d). This data suggests that the MEK signature is altered by KRAS status and MEK inhibitors.

***a) MEK inhibition in cisplatin resistant OVCAR3 cells, re-sensitises cells to cisplatin***

*b)* To further investigate the role of MEK signalling in driving resistance to cisplatin, OVCAR3-WT and OVCAR3-CP cells were treated with either cisplatin alone or a combination of cisplatin and the MEK inhibitor Trametinib. Following treatment of OVCAR3-WT cells with increasing concentrations of cisplatin, OVCAR3-CP cells formed more colonies than OVCAR3-WT cells. However, the addition of 0.5 uM Trametinib to increasing concentrations of cisplatin resulted in a decrease in colony formation of both OVCAR3-WT and OVCAR3-CP cells (Figure 5a, red line). This resulted in a 2 fold decrease in the IC50 of OVCAR3-WT cells from 0.066uM to 0.033uM following the addition of Trametinib to cisplatin. The addition of Trametinib to increasing concentrations of cisplatin resulted in a 4.8 fold decrease in the IC50 of OVCAR3-CP cells from 0.029 uM to 0.06 uM. Importantly, there was a greater fold change in the IC50 cells following the addition of Trametinib to cisplatin for OVCAR3-CP cells (Figure 5b, orange line).

***The Angio_Immune subgroup has increased EMT signalling***

MAPK is known to phosphorylate SLUG and other key players of the SNAIL/SLUG transcription factors, to induce epithelial-mesenchymal transition (EMT) which is known to be a contributing mechanism to angiogenesis and of progressive disease (Virtakoivu et al., 2015). Gene expression profiling showed that a range of EMT associated genes had higher expression levels in the Angio-Immune subgroup as opposed to the Angio and Immune subgroups. This includes significantly increased expression of VIMENTIN, AXL, TWIST1, SNAIL and SLUG in the Angio_Immune subgroup (p=<0.001) compared to the Angio and Immune subgroups (Figure 6a). Likewise, in the E-GEOD-58582 dataset, MCF7 breast cells transfected with SNAIL show a positive association with the 45-gene signature (p=0004) (Figure 6b). In sum, the Angio_Immune subgroup has increased EMT signalling. As a result, the Angio_Immune subgroup may now be defined forthwith as the 'EMT signature'.

***Activation of the EMT phenotype is enhanced in Cisplatin resistant ovarian cell lines***

Above we showed that MAPK signalling may be driving the Angio_Immune subgroup. Interestingly, cisplatin resistant OVCAR3 and OVCAR 4 cells had increased MAPK signalling compared to the cisplatin sensitive counterparts, as measured by phospho-MEK and phospho-ERK protein expression (Figure 7a and Figure 2d). Moreover, we already demonstrated that the 45- and -15-gene signature not only predicts cisplatin resistance but is also elevated by EMT. Therefore we decided to investigate the relationship between cisplatin resistance *in-vitro* and an associated EMT phenotype utilising both cisplatin-sensitive and -resistant cell lines generated in-house; HGSOC OVCAR3 and OVCAR4 cell lines. It appears that cisplatin resistant ovarian cells (OVCAR3 CP and OVCAR4 CP) exhibit activation of EMT signalling as demonstrated by the upregulated protein expression of Vimentin, N-cadherin and SLUG in conjunction with decreased levels of E-cadherin, compared to their wildtype counterparts (Figure 7b). These observed changes in EMT markers are all indicative of EMT activation. In addition, cisplatin resistant OVCAR3 and OVCAR4 cells also exhibit significantly increased mRNA expression levels of some EMT markers, including N-cadherin, SLUG, SNAIL, Vimentin, TWIST and TGF-$\beta$3. This is shown by the greater fold change difference in OVCAR3 CP and OVCAR4 CP relative to the wildtype cells (Figure 7c and d). Moreover, cisplatin resistance in OVCAR3 and OVCAR4 shows greater cell migration and an enhanced migratory phenotype (Figure 7e), hence suggesting the activation of EMT and Angiogenic phenotypes.

***ALM201 reverses the EMT phenotype in OVCAR3 and 4 cisplatin resistant cells***

We used the EMT on cell line Kuramochi and the OVCAR3 and 4 cisplatin resistant cells to examine the effects of ALM201 on EMT markers and associated phenotypes. ALM201 treatment caused reduced MAPK signalling and EMT signalling in the Kuramochi cell line (Figure 30a). The same effect was seen in the OVCAR3 cisplatin resistant cell line (Figure 30b). Additionally in the OVCAR3 and OVCAR4 cells treatment with ALM201 inhibited the proliferation capacity (Figure 30c), and in the OVCAR3 cisplatin resistant cells, ALM201 inhibited the migration and invasion potential of the cells (Figure 30d).

***The EMT signature predicts resistance to inhibitors of the SRC pathway***

Further investigation suggests that both the MAPK and SRC signalling pathways signal as parallel pathways and may contribute to drug resistance. Treatment with 1 $\mu$M Saracatinib (SRCi) over 24 hours, reduces the protein expression of pSRC (left, top panel) whilst increasing the protein levels of pERK (left, third panel). In contrast to this, treatment with 1 $\mu$M Trametinib (MEKi) over 24 hours exhibits the opposite effect, enhancing pSRC protein levels (right, third panel) and decreasing pERK protein expression (right, top panel) (Figure 8a). Resistance (res) or sensitivity (sens) to SRC inhibitors was defined based on the median of the IC50 values and correlation with 45-gene signature scores and AUC scores determined. This demonstrated that in cell line models the EMT signature could predict resistance to SRC inhibitor, Saracatinib (AUC 0.8867 (0.7757-0.9978), p=0.00019) as shown by the increased 45-gene signature score in resistant cell lines (res) (Figure 8b). This suggests that the SRC and MAPK pathways act in parallel whereby the inhibition of one signalling cascade leads to the activation of the other. To conclude, the EMT signature predicts response to MEK inhibitors (sensitivity) and SRC inhibitors (resistance). Theoretically, targeting both pathway would be a plausible therapeutic combination.

***The EMT signature detects a poor prognosis subgroup in Colon Cancer (CRC) and Non-Small Cell Lung Cancer (NSCLC)***

As the Angio_Immune group is driven by MAPK signalling, we hypothesised that the EMT signature may also have prognostic utility in alternative disease indications, namely colon cancer (CRC) and non-small cell lung cancer (NSCLC) which have high incidence of alterations in the MAPK pathway. We therefore investigated this in two publically available colon datasets both in the context of treatment (Marisa GSE40967 and Jorissen GSE14333) and one NSCLC dataset which incorporates an untreated population (Okayama GSE31210). The Marisa dataset consisting of 566 Stage I-IV colon cancers, had the MEK defined subgroup present in sample cluster 3 (C3) following hierarchical clustering (Figure 9a, red box). The MEK subgroup (C3) was associated with worse prognosis (RFS, p=0.037) (Figure 9b, red line). Additionally, the 45-gene EMT signature described as 'MEK ON' was associated with poor prognosis, (RFS HR 1.594 (1.095-2.323), p=0.0063) (Figure 9c, red line). The Jorissen dataset consisting of 260 Stage I-IV colon cancers also showed a poor prognostic subgroup detected by the 45-gene EMT signature, (DFS HR 2.454 (1.205-4.999), p=0.0014) (Figure 9d, red line).

In relation to NSCLC, the Okayama dataset consisting of Stage I and II untreated NSCLC samples also had the MEK defined subgroup present in sample cluster 4 (C4) following hierarchical clustering (Figure 10a, red box). The MEK subgroup (C4) was associated with worse prognosis (p=0.0004) (Figure 10b, red line). Additionally, the 45-gene EMT signature described as 'SIGN POS' was associated with poor prognosis, (PFS HR 3.045 (1.631-5.686), p=0.0005) (Figure 10c, red line). The Okayama dataset confirmed a poor prognostic subgroup detected by the 45-gene EMT signature, (OS HR 2.872 (1.271-6.489), p=0.0312) (Figure 10d, red line). In sum, the 45-gene EMT signature detects a poor prognosis subgroup of patients driven by the MAPK pathway activation and is therefore independent of treatment.

***The 15-gene signature predicts response to Cisplatin and is elevated in cisplatin resistant cells***

The Angio_Immune subgroup may now be defined forthwith as the 15-gene signature. AS with the 45-gene signature, we hypothesised that the Angio_Immune group would be prognostic in the context of SoC treatment in ovarian cancer. We therefore investigated this in the treatment naive Discovery dataset. The 15-gene signature was also associated with worse prognosis (PFS, HR = 1.3564 [1.0156-1.8117]; p = 0.0279 and OS, HR = 1.3464 [0.9901-1.8308]; p = 0.0441) and could predict response to cisplatin based therapy (Figure 11a).

Since analysis of the clinical samples of the Discovery dataset demonstrated that the 15-gene signature could similarly predict response to cisplatin based standard of care treatment, we used a panel of 15 ovarian cell lines to investigate this further. These cells lines were analysed by DNA microarray analysis using the Ovarian Cancer DSA™ and signature scores were generated as previously described. The cell lines were also used to perform colony formation assays with Pharmaceutical grade cisplatin. A ROC curve was generated. Cisplatin resistance (res) or sensitivity (sens) was defined based on the median of the IC50 values and correlation with signature scores and AUC scores determined. This demonstrated that in cell line models the 15-gene signature could predict resistance to cisplatin upfront as shown by the increased 15-gene signature scores in resistant ovarian cell line panels (res) (AUC 0.6905 (0.5254-0.8556), p=0.2900) (Figure 11b). Additionally ROC generated using the median of the signature scores demonstrated that signature positive (sign pos) were resistant to cisplatin compared to signature negative (sign neg) cells (AUC .0.6897 (0.5326-0.8468) P= 0.02932). Furthermore, both OVCAR3 cisplatin resistant cells (OVCAR3PT) had significantly increased 15-gene signature scores (Angio_Immune) compared to their sensitive counterparts (0.046) (Figure 11c).

***The 15-gene signature is also associated with the MAPK pathway***

The potential link between the MAPK pathway and the 15-gene signature was assessed using two previously mentioned publically available datasets; E-MEXP-3557 and E-GEOD-12764. Data from the E-MEXP-3557 dataset showed that KRAS mutant (KRAS MT) HCT116 cells had an association with the Angio_Immune subgroup with higher 15-gene signature scores compared to the wildtype HKH2 cells (KRAS WT) (p=0.0443) (Figure 12a). Moreover, MCF10 epithelial breast cells transfected with either HRAS or MEK1 also show an association with the 15-gene signature (p<0.0001) compared to the empty vector (EV) (Figure 12b). Moreover, inhibition of MEK with Trametinib significantly decreases the 15-gene Angio_Immune signature score in OVCAR3 cells (p=0.0023) (Figure 12c). This data suggests that the 15-gene signature is elevated by KRAS status or MEK1 overexpression and decreased by MEK inhibitors.

Utilising the Reverse Phase Proteomic Array (RPPA) data from The Cancer Genome Atlas (TCGA) dataset, phospho-MAPK scores were calculated as a ratio of total MAPK. Gene signature scores were then correlated with the RPPA data and AS previously mentioned Angio_Immune subgroup was specifically found to correlate with pMAPK serine 217/221 expression using ROC analysis (Figure 3c). This confirms that the Angio_Immune subgroup is being driven by the MAPK pathway and the 15-gene signature is associated with MEK expression.

Furthermore the 15-gene signature could predict sensitivity to the MEK inhibitor Trametinib (Mekinist, GSK) as demonstrated by the increased 15-gene signature scores in ovarian cell line panels (sens) (AUC 0.7234 (0.5778-0.8690) p= 0.0090) (Figure 12d). Additionally ROC generated using the median of the signature scores demonstrated that signature positive (sign pos) cells were sensitive to Trametinib compared to signature negative (sign neg) cells (AUC .0.6897 (0.5326-0.8468) P= 0.02932). The Angio_Immune subgroup may now be defined forthwith as the 15-gene signature or 'MEK signature'.

***The 15-gene signature exhibits elevated EMT signalling***

As mentioned previously, MAPK is known to phosphorylate SLUG and other key players of the SNAIL/SLUG transcription factors, to induce epithelial-mesenchymal transition (EMT) which is known to be a contributing mechanism of progressive disease. Using the E-GEOD-58582 dataset, MCF7 breast cells overexpressing SNAIL show a positive association with the 15-gene signature (p=0.0015) (Figure 13). In sum, the 15-gene signature is also associated with enhanced EMT signalling. As a result, the 15-gene signature may now be defined forthwith as the 'EMT signature'.

***The 15-gene EMT signature also predicts resistance to inhibitors of the SRC pathway***

Further investigation suggests that both the MAPK and SRC signalling pathways signal as parallel pathways and may contribute to drug resistance. Resistance (res) or sensitivity (sens) to SRC inhibitor, Saracatinib was defined based on the median of the IC50 values and correlation with 15-gene signature scores (Sign Pos vs Sign Neg) and AUC scores determined (AUC 0.7289, (0.5544-0.9035), p=0.01454) (Figure 14a). This demonstrated that in cell line models the 15-gene EMT signature could predict resistance to SRC inhibitor, Saracatinib (AUC 0.7698 (0.6054-0.9343), p=0.004), as shown by the increased 15-gene signature score in resistant cell lines (res) (Figure 14b). This suggests that the SRC and MAPK pathways act in parallel whereby the inhibition of one signalling cascade leads to the activation of the other.

***The 15-gene EMT signature detects a poor prognosis subgroup in Colon Cancer (CRC) and Non-Small Cell Lung Cancer (NSCLC)***

As the Angio_Immune group is driven by MAPK signalling, we hypothesised that the 15-gene EMT signature may also exhibit prognostic utility in alternative disease indications; colon cancer and non-small cell lung cancer (NSCLC). We therefore investigated this in the two publically available colon datasets in relation to treatment (Marisa GSE40967 and Jorissen GSE14333) and one untreated NSCLC dataset (Okayama GSE31210). The Jorissen dataset consisting of 260 Stage I-IV colon cancers showed a poor prognostic subgroup detected by the 15-gene EMT signature, (DFS, p=0.0328) (Figure 15a, red line). The Marisa dataset confirmed that the 15-gene signature was associated with worse prognosis (RFS, p=0.0161) (Figure 15b, red line).

With regards to NSCLC, the Okayama dataset consisting of Stage I and II untreated NSCLC samples also associated with 15-gene signature with worse prognosis. The 15-gene EMT signature described as 'SIG POS' was associated with poor prognosis, (PFS, p=0.0024) (Figure 16a, red line). The Okayama dataset confirmed a poor prognostic subgroup detected by the 15-gene EMT signature with reduced overall survival in the 'SIG POS' cohort, (OS, p=0.0396) (Figure 16b, red line). Additionally the 15 gene signature is prognostic across a number of tissues p<0.0001 (Figure 36a and b). In sum, the 15-gene EMT signature detects a poor prognosis subgroup of patients across multiple diseases and is driven by the MAPK pathway activation and EMT signalling.

***The 45 gene and 15 gene signatures are predictive of response to MEK inhibitors***

Furthermore the both the 45 and 15-gene signature could predict sensitivity to the MEK inhibitors, Trametinib (Mekinist, GSK) and Selumetinib from The Genomics of Drug Sensitivity in Cancer Project' cell line data. This is demonstrated by the increased 45-gene signature scores in a panel of cell lines treated with either Trametinib or Selumetinib (sens) (AUC 0.7277 (0.6945-0.7609) p<0.0001 and AUC 0.6598 [0.6213-0.6983]; p<0.0001 respectively) (Figure 31a and b). Additionally ROC generated using the median of the 45-gene signature scores demonstrated that signature positive (sign pos) cells were sensitive to Trametinib and Selumetinib compared to signature negative (sign neg) cells (AUC 0.7042 [0.6695-0.7389]; p<0.0001 and AUC 0.6476 [0.6083-0.6869]; p<0.0001 respectively).

Additionally, this is also demonstrated by the increased 15-gene signature scores in a panel of cell lines treated with either Trametinib or Selumetinib (sens) (AUC 0.629 (0.593-0.664) p<0.0001 and AUC 0.619 [0.584-0.654]; p<0.0001 respectively) (Figure 31c and 17d). Additionally ROC generated using the median of the 15-gene signature scores demonstrated that signature positive (sign pos) cells were sensitive to Trametinib and Selumetinib compared to signature negative (sign neg) cells (AUC 0.627 [0.591-0.662]; p<0.0001 and AUC 0.609 [0.573-0.644]; p<0.0001 respectively).

**Platinum resistance creates a selection pressure for an angiogenesis enriched microenvironment**

Microvessel density (MVD) is a measure of the angiogenic capacity of a tumor and as the angiogenic potential increases so too does its MVD. An in-vivo matrigel-plug assay was used to determine the micro-vessel density (MVD) in the OVCAR3 isogenic cell lines in co-culture with Human endothelial colony forming cells (EFCF). The OVCAR3 platinum-resistant cell lines have a higher MVD than the OVCAR3 platinum-naïve pair (p-value: 0.0041) (Figure 38) suggesting that the OVCAR3-PT cells were more associated with an angiogenic phenotype than the OVCAR3 platinum sensitive/naïve cells.

To investigate if there was any specific chemokines driving the angiogenesis like phenotype in the OVCAR3-PT and OVCAR4-PT cells, we performed a cytokine array. This demonstrated increased expression of a number of cytokines that are key regulators of tumour angiogenesis (HGF, VEGF, TIMP1&2, PIGF and Angiogenin) in the PT resistant cells relative to the treatment-naïve control (Figure 39). Since angiogenesis is largely driven by the expression of receptor tyrosine kinases, we analysed the gene expression profiling data from the HGSOC dataset and discovered a number of receptor tyrosine kinases (RTKs) which had higher expression levels in the AngioImmune subgroup (compared to the angio and immune groups) including PDGFR$\alpha$ (P= < 0.0001), PDGFR$\beta$ (P= < 0.0001), FGFR2 (P= 0.008683), FGFR4 (P= 0.008085), AXL (P= < 0.0001), VEGFR1 (P= < 0.0001), VEGFR2 (P= < 0.0001), EGFR (P= 0.04859), MET (P= 0.02659), EPHA4 (P= 0.00019), FZD1 (P= < 0.0001). Interestingly PDGFR$\alpha$ (P=0.007074) and PDGFR$\beta$ (P=0.004868) were also associated with the post-platinum treated patient samples (Figure 40). Additionally pRTK array was performed and demonstrated that the OVCAR3 platinum-resistant cells have a higher expression of key RTKs that are key regulators of downstream pathways that regulate tumour angiogenesis (FGFR2, FGFR3, AXL, VEGFR3, Insulin receptor, ERBb2), this was further validated by western blot (Figure 41).

**Tyrosine kinase inhibitors (TKi) selectively target platinum resistant cells**

As many of the RTK were associated with the AngioImmune group and post-chemotherapy samples, we wanted to examine whether the OVCAR3-PT resistant cells would be sensitive to inhibitors of RTKs. We used 2 RTK inhibitors, Cediranib which targets VEGFR1-3 and PDGFR$\alpha/\beta$ and Nintedanib which targets VEGFR1-3, PDGFR$\alpha/\beta$ FGFR1-3 and performed 10-day colony formation assays. This demonstrated that Cediranib (VEGFR1-3 and PDGFR$\alpha/\beta$ inhibitor) and Nintedanib (VEGFR1-3, PDGFR$\alpha/\beta$ FGFR1-3 inhibitor) have specificity for the cisplatin-resistant OVCAR3 cells relative to the OVCAR3 cisplatin-naïve cell line (fold change 0.2390869 and 0.377, respectively) (Figure 42). Additionally Cediranib and Nintedanib downregulated the expression of the VEGFR ligand, VEGfa, in the OVCAR3 and OVCAR4 cisplatin resistant cell lines. (Figure 43) suggesting that the mechanism of sensitivity if through VEGFa. To examine the dependency of the angiogenesis phenotype on VEGFA, we performed an in-vivo matrigel plug assay where OVCAR3 cells in co-cultured with ECFCs and the mice were treated with 5mg/kg of the VEGFRA inhibitor Bevacizumab. Quantification of MVD showed that the OVCAR3 cisplatin-resistant cells following treatment with bevacizumab (5mg/kg) have less MVD in comparison to the OVCAR3 cisplatin-naïve cells (p-value: 0.0024) (Figure 44). This demonstrates that the OVCAR3-PT cells (which have elevated 45 and 15 gene signature scores) are more sensitive to the VEGFA inhibitor Bevacizumab.

***The 45 and 15-gene signatures are predictive of response to taxanes in prostate cancer***

Within a pilot of 56 biopsy samples with de novo metastatic disease, 50 samples passed all QC metrics and were utilised for data analysis, 24 of which were PSA-responders and 26 non PSA-responders. It was observed that within the proportion of PSA responders, there was a significant increase in the 45-gene signature scores (p=0.0083) (Figure 32a). Of these PSA-responders, 16 were 45-gene signature positive and 8 were 45-gene signature negative. Conversely to this, within the non PSA-responders 10 were 45-gene positive and 16 were 45-gene negative (Figure 32c). EMT positive (45-gene signature positive) patients show a significant benefit to receiving taxane based chemotherapy with significantly increased overall survival in comparison to the EMT negative patients (HR 0.5094 [0.2694-0.9004]; p=0.0238 (Figure 32b).

It was also observed that within the proportion of PSA responders, there was a significant increase in the 15-gene signature scores (p=0.04) (Figure 33a). Of these PSA-responders, 16 were 15-gene signature positive and 8 were 15-gene signature negative. Conversely to this, within the non PSA-responders 10 were 15-gene positive and 16 were 15-gene negative (Figure 33c). Additionally, EMT positive (15-gene signature positive) patients show a significant benefit to receiving taxane based chemotherapy with increased overall survival in comparison to the EMT negative patients (HR 0.66 [0.36-1.20]; p=0.160 (Figure 33b).

***EMT signature is prognostic in Prostate Cancer, predicting disease recurrence and poor prognosis post radical surgery***

In a retrospective cohort of 322 radical prostatectomy specimens, the EMT signature exhibits a prognostic relevance in Prostate Cancer. The EMT assay predicts biochemical disease recurrence whereby high EMT patients demonstrate a significantly poor prognostic group in comparison to low EMT patients (HR = 1.8095 [1.1499-2.8474]; p=0.0658) (Figure 34). Likewise, in a retrospective cohort of 248 radical radiotherapy specimens, the EMT signature also exhibits a prognostic relationship in Prostate Cancer. The EMT assay predicts both biochemical disease recurrence and metastatic disease progression whereby high EMT patients demonstrate a significantly poor prognostic group in comparison to low EMT patients (Biochemical: 2.4924 [1.4293-4.3463]; p=0.004 and Metastatic: HR = 2.6351 [1.0998-6.3137]; p=0.020) (Figure 35a and b).

**Discussion**

We have demonstrated that the MAPK pathway is a mechanism of innate and acquired resistance to SoC cisplatin-based therapy in HGSOC. The Angio_Immune group represents 44% of treatment naive HGSOCs and also represents a subgroup which samples migrate to after platinum-based chemotherapy treatment. At present all HGSOCs are treated the same however even though there are differences in prognosis on SoC platinum based therapy. The Angio_Immune group is a poor prognosis subgroup on SoC therapy and therefore represents an opportunity for treatment with more targeted and effective therapeutic agents. Importantly, the 45 and 15 EMT signatures which detect the Angio_Immune group can act as predictive assays for these targeted therapies and hence should predict response to therapeutics in any cancer disease setting. This is significant as these patients cannot be identified by a pathologist. The Angio_Immune group and the 45 and 15 gene signatures which detect the subgroup have a number of potential utilities.

***Predicting cisplatin resistant HGSOC***

The Angio_Immune group represents a poor prognosis subgroup (Figure 1a & 1b) and the gene signatures which detect the subgroup are significant in SoC platinum based therapy in HGSOC for PFS and OS (Figure 1c and 11a). In line with this, this has been demonstrated across multiple cell line models also (Figure 2e and 11b). The Angio_Immune group represents a group which is selected for by SoC platinum based chemotherapy (Figure 1d). This has also been demonstrated in ovarian cell line models which have been made cisplatin resistant in vitro and which have higher 45 and 15 signature scores (Figure 2 and 11c).

***Predicting response to inhibitors of the MAPK pathway***

We have demonstrated that the Angio_Immune group is driven by MAPK signalling using publically available and internal datasets and methods (Figure 3 and 4). The MAPK pathway is currently being targeted by numerous drugs from major Pharmaceutical companies (Table 5). We would predict that the 45 and 15 gene signatures which detect the Angio_Immune group would predicts response to agents targeting the MAPK pathway in human cancer. We have demonstrated that both the 45 and 15 gene signatures predict response to the MEK inhibitor Trametinib across multiple cell line models (Figure 3d and 12d). Additionally the combination of MAPK inhibitor with cisplatin may be a viable option for first line therapy or as a therapeutic strategy for cisplatin resistant disease. We have demonstrated this utility by generating novel HGSOC cell line models and shown increased sensitivity to cisplatin after MEK inhibition in the cisplatin resistant cells compared to cisplatin sensitive counterparts (Figure 5b).

***Predicting response to inhibitors of the EMT pathway***

Since the Angio_Immune group is associated with increased expression of EMT pathway related genes (Figure 7), we would predict that the 45 and 15 gene expression signatures detecting this group would predict response to agents targeting the EMT pathway. Additionally the cisplatin resistant cells generated in this study have elevated EMT signalling as demonstrated by increased expression of vimentin, N-cadherin and slug and decreased E-cadherin expression (Figure 7). This suggests that the EMT phenotype is related to cisplatin resistance. This has been previously been documented in the literature (Ahmed et al., 2010; Haslehurst et al., 2012; Marchini et al., 2013). Table 7 highlights the major compounds in development which target the EMT pathway, predominantly AXL inhibitors.

***Predicting response to inhibitors of the SRC pathway***

We have demonstrated that the MAPK and SRC pathways act in parallel and that activation of the MAPK pathway may predict resistance to inhibitors of the SRC pathway. Figures 8 and 14 demonstrate that the 45 and 15 EMT signatures can predict resistance to SRC inhibitors in cell line models. This would be worthy of further investigation in clinical samples where patients have been treated with a SRC inhibitor. We would hypothesise that the 45 and 15 gene expression signatures would predict resistant to agents targeting the SRC pathway. Table 6 outlines the key Pharma players with SRC inhibitors currently in development for a range of relevant disease indications.

***Predicting response to Anti-angiogenic agents***

Our data suggest that after chemotherapy treatment, patient tumours move into the Angio_Immune group and therefore become more angiogenic like. The Angio_Immune subgroup is largely driven by angiogenic-like processed (Figure 1). Therefore the use of anti-angiogenic agents should have better responses in the second line treatment of ovarian cancer following SoC cisplatin-based chemotherapy in the first line setting. Additionally we would hypothesise that since the Angio_Immune group is driven by Receptor Tyrsoine Kinases, the MAPK and EMT pathways and is increased angiogenesis signalling, the 45 and 15 gene signatures should predict response to any agent targeting the angiogenesis process. A number of anti-angiogenics agents are in clinical development, including bevacizumab, pazopanib, cedirinib, nintedanib, aflibercept, trebananib, sunitinib, sorafenib, and (PDGFR) imatinib.

Following a critical review of the various phase III anti-angiogenic clinical trials, there is a clear change in the biology of ovarian cancer following relapse and particularly on platinum resistance. There is a trend to a higher number of positive anti-angiogenic trials in platinum resistant ovarian cancer (see below). This confirms our hypothesis and suggests that the use of anti-angiogenic targeted agents may work better in second line trials after primary cisplatin treatment. The 45 and 15 EMT signatures should therefore predict response to these agents.

***Predicting response to Taxanes***

We have demonstrated that the EMT subgroup can predict response to taxane based chemotherapy. Figures 32 and 33 demonstrate that the 45 and 15 EMT signatures can predict sensitivity to taxanes in prostate cancer patients with de novo metastatic disease. This would be worthy of further investigation in clinical samples which incorporate a larger clinical validation cohort. We would hypothesise that the 45 and 15 gene expression signatures would predict response to taxane based chemotherapy whereby MEK positivity will be associated with increased overall survival.

***First-line treatment***

There has been a number of phase II/III clinical trials focusing on target treatment with an aim to improve the overall survival of patients with high risk stage Ic and II-IV ovarian cancer. GOG 218 and ICON7 explored the role of bevacizumab (Avastin) in combination with upfront chemotherapy. Bevacizumab is a recombinant humanised monoclonal antibody that binds to vascular endothelial growth factor A (VEGF-A). ICON7 showed superior progression free survival (PFS) in the group of patients who received bevacizumab in combination with standard chemotherapy (20.3 months and 21.8 months, standard therapy versus standard therapy plus bevacizumab respectively (hazard ratio (HR) 0.81; 95% confidence interval (CI), 0.70 to 0.94; P = 0.004)). Benefit was observed in patients with high risk disease (defined as FIGO III, ≥1.0 cm disease following debulking or FIGO stage IV).

GOG-218 similarly compared three arms; standard chemotherapy, bevacizumab with standard chemotherapy from cycle 2 to cycle 6 and bevacizumab plus standard chemotherapy cycle 2 through to cycle 22. There was again superior PFS when comparing standard chemotherapy and standard treatment with the addition of bevacizumab (10.3, 11.2, and 14.1 months, control group, bevacizumab (cycle 2-6), and bevacizumab (cylce2-22), respectively). The HR for PFS was 0.908; 95% CI, 0.795 to 1.040; P = 0.16 for bevacizumab cycle2-6 and 0.717; 95% CI, 0.625 to 0.824 (P<0.001) for bevacizumab plus chemotherapy from cycle 2-22.

***Recurrent/Relapsed disease***

As previously discussed the prognosis of ovarian cancer is directly correlated with platinum sensitivity and timing or recurrence or relapse following completion of platinum-based chemotherapy. Therefore, anti-angiogenics were explored in recurrent/relapsed ovarian cancer. Two phase III trials OCEANS and AURELIA explored the impact of bevacizumab in relation to the timing of disease relapse following platinum-based chemotherapy.

OCEANS is a phase III trial exploring the efficacy of bevacizumab in combination with gemcitabine and carboplatin (GC) in platinum sensitive recurrent ovarian cancer. Patients were assigned to bevacizumab + GC or placebo + GC, total number of six to ten cycles. Median progression free survival was 12.4 months and 8.4 months respectively (HR 0.484; 95% CI:0.388-0.605; p-value< 0.0001). The role of bevacizumab in platinum resistant epithelial ovarian cancer was explored in the AURELIA trial. In this phase III clinical trials patients were randomly assigned to single agent chemotherapy (investigators choice: peglated doxorubicin, paclitaxel or topotecan) with or without bevacizumab. Progression free survival as a primary endpoint was reached, 6.7 months in the chemotherapy and bevacizumab treated patients and 3.4 months in the chemotherapy alone arm (H^ 0.42; 95% CI: 0.32-0.53; p-value< 0.001). The objective response rate (ORR) in patients treated with bevacizumab and chemotherapy was 30.9%. Most importantly the benefit in PFS was reflected in the overall survival; 16.6 months in the chemotherapy and bevacizumab arm compared to 13.3 months in the chemotherapy alone arm (HR 0.89; 95% CI: 0.69-1.14; p-value< 0.174). However this was not statistically significant. This trial led to the Food Drugs Advisory (FDA) approval of bevacizumab in platinum resistant epithelial ovarian cancer in November 2014, with an added benefit in relation to progression free survival in patients treated with bevacizumab and paclitaxel (PFS 9.6 months).

ICON6 was an international phase III clinical trial, testing the efficacy of cediranib, an oral potent inhibitor of VEGFR 1, 2 and 3 that as a direct effect in stopping the VEGF signal, in relapsed platinum sensitive epithelial ovarian cancer. Patients were randomised into three arms; chemotherapy plus placebo maintenance (reference arm), concurrent platinum chemotherapy and cediranib followed by maintenance cediranib or cediranib plus maintenance cediranib. There was statistically significant progression free and overall survival benefit (PFS 9.4 months and 11.4 months respectively; HR 0.68; p-value = 0.0022, Overall survival 17.6 months and 20.3 months respectively; HR 0.70; p-value = 0.0419).

**Predicting poor prognosis group in multiple solid tumours**

Many cancers are driven by alterations in the MAPK pathway. For example mutations in the *RAS* genes (*KRAS, HRAS,* and *NRAS*), are present in approximately 50% of all patients with colorectal cancer. This results in hyper-activation of RAS proteins and their corresponding downstream pathways such as the MAPK pathway, thereby stimulating the development and progression of malignancy. We have demonstrated that both the 45 and 15 gene signatures predict poor prognosis subgroups in colon, lung and prostate cancer (Figures 9-10, 15-16, 32-35). Additionally the 15 gene signature was prognostic across a number of tissues (Figure 36), suggesting that the EMT subgroup and the signatures which detect it (15 and 45 gene signatures) define a poor prognosis subgroup of patients across multiple diseases and is driven by the MAPK pathway activation and EMT signalling. We would hypothesise that this would be the situation for most solid tumours which have alterations in the MAPK or EMT pathways by mutation or mechanisms other than mutation which can activate the pathway.

**EXAMPLE 2**

**15 gene signature minimum and core gene analysis**

**Samples:**

- Internal training samples : This sample set comprised of 265 macro-dissected ovarian cancer FFPE tissue samples sourced from the Edinburgh Ovarian Cancer Database
- Tothill samples: This is a publically available dataset, from which 278 Ovarian samples were used for analysis
- ICON7 samples: This sample set comprised 140 SOC (Standard of Care) samples from a phase III randomized trial of carboplatin and paclitaxel with or without bevacizumab first line cancer treatment which were accessed through the MRC (Medical Research Council). The 140 samples used were patients who did not receive the addition of bevacizumab.

**Methods:**

**Subtype analysis**

This analysis evaluates if the gene expression data for each of the signature genes has the ability to significantly detect the MEK subtype independent of the other genes. For each gene an area under the receiver operating curve (AUC) and ANOVA p-values were calculated in the internal training samples. Signature genes with an ANOVA p-value less than 0.05 are statistically significant at detecting the subtype, independent of the other signature genes.

C-index values were also generated to determine if the gene expression for each of the signature genes has the ability to significantly detect PFS (Progression Free Survival) independent of the other signature genes. The upper and lower confidence intervals (CI) were derived using bootstrapping with 1000 samplings. If the C-Index lower CI is greater than 0.5 or the upper CI is less than 0.5 then the C-index indicates that the gene expression is significantly associated to the observed survival.

**Core gene analysis**

The purpose of evaluating the core gene set of the signature is to determine a ranking for the genes based upon their impact on performance when removed from the signature.

This analysis involved 10,000 random samplings of 10 signature genes from the original 15 signature gene set. At each iteration, 10 randomly selected signature genes were removed and the performance of the remaining 5 genes was evaluated using the endpoint to determine the impact on HR (Hazard Ratio) performance when these 10 genes were removed in the following 2 datasets:

- Tothill - 278 samples
- ICON7 SOC (Standard of Care) - 140 samples

ICON7 was evaluated using the PFS endpoint and Tothill was evaluated using the OS (Overall Survival) endpoint. Within each of the 2 datasets, the signature genes were weighted based upon the change in HR performance (Delta HR) based upon their inclusion or exclusion. Genes ranked '1' have the most negative impact on performance when removed and those ranked '15' have the least impact on performance when removed.

**Minimum gene analysis**

The purpose of evaluating the minimum number of genes is to determine if significant performance can be achieved within smaller subsets of the original signature.

This analysis involved 10,000 random samplings of the 15 signature genes starting at 1 gene/feature, up to a maximum of 10 genes/features. For each randomly selected feature length, the signature was redeveloped using the PLS machine learning method under CV and model parameters derived. At each feature length, all randomly selected signatures were applied to calculate signature scores for the following 2 datasets:

- Tothill - 278 samples
- ICON7 SOC (Standard of Care) - 140 samples

Continuous signature scores were evaluated with outcome to determine the HR effect; ICON7 was evaluated with PFS and Tothill was evaluated with OS. The HR for all random signatures at each feature length was summarized and figures generated to visualize the performance over CV.

**Results**

**Subtype analysis**

The results for the subset analysis of the 15 gene signature in the internal training dataset is provided in this section.

- AUC and ANOVA: All genes have a p value significantly less than the 0.05 threshold for ANOVA (See Table 12). This suggests that every individual gene in the 15 gene signature has the ability to detect the subtype independent of the other 14 genes.
- C-Index: All genes have expression values that significantly discriminated the survival probability of the patients (See Table 12). The C-Index values for each of these genes is higher than 0.5 therefore indicating that lower expression correlates to higher survival probability for all 15 genes.

**Core gene analysis**

The results for the core gene analysis of the 15 gene signature in the 2 datasets is provided in this section.

- Tothill: Delta HR performance measured in this dataset for the 15 signature genes is shown in Figure 22. This figure highlights the top 10 ranked genes in the signature which are the most important in retaining a good HR performance within this dataset.

- ICON7: Delta HR performance measured in this dataset for the 15 signature genes is shown in Figure 23. This figure highlights the top 10 ranked genes in the signature which are the most important in retaining a good HR performance within this dataset.
- Delta HR across these 2 datasets was evaluated to obtain a combined gene ranking for each of the signature genes. The ranks assigned to the signature genes based on the core set analysis has been outlined in Table B.

**Minimum gene analysis**

The results for the minimum gene analysis of the 15 gene signature in 2 datasets is provided in this section.

- Tothill: The average HR performance measured in this dataset using the random sampling of the signature genes from a feature length of 1 to 10 is shown in Figure 24. This figure shows that to retain a significant HR performance (i.e. HR < 1) a minimum of 1 of the signature genes must be selected.
- ICON7 SOC: The average HR performance measured in this dataset using the random sampling of the signature genes from a feature length of 1 to 10 is shown in Figure 25. This figure shows that to retain a significant HR performance (i.e. HR < 1) a minimum of 1 of the signature genes must be selected.
- In summary, it is recommended that a minimum of at least 1 gene can be used and significant performance will be retained.

**EXAMPLE 3**

**45 gene signature minimum and core gene analysis**

**Samples:**

- Internal training samples : This sample set comprised of 265 macro-dissected ovarian cancer FFPE tissue samples sourced from the Edinburgh Ovarian Cancer Database
- Tothill samples: This is a publically available dataset, from which 278 Ovarian samples were used for analysis
- ICON7 samples: This sample set comprised 140 SOC (Standard of Care) samples from a phase III randomized trial of carboplatin and paclitaxel with or without bevacizumab first line cancer treatment which were accessed through the MRC (Medical Research Council). The 140 samples used were patients who did not receive the addition of bevacizumab.

**Methods:**

**Subtype analysis**

This analysis evaluates if the gene expression data for each of the signature genes has the ability to significantly detect the MEK subtype independent of the other genes. For each gene an area under the receiver operating curve (AUC) and ANOVA p-values were calculated in the internal training samples. Signature genes with an ANOVA p-value less than 0.05 are statistically significant at detecting the subtype, independent of the other signature genes.

C-index values were also generated to determine if the gene expression for each of the signature genes has the ability to significantly detect PFS (Progression Free Survival) independent of the other signature genes. The upper and lower confidence intervals (CI) were derived using bootstrapping with 1000 samplings. If the C-Index lower CI is greater than 0.5 or the upper CI is less than 0.5 then the C-index indicates that the gene expression is significantly associated to the observed survival.

**Core gene analysis**

The purpose of evaluating the core gene set of the signature is to determine a ranking for the genes based upon their impact on performance when removed from the signature.

This analysis involved 10,000 random samplings of 10 signature genes from the original 45 signature gene set. At each iteration, 10 randomly selected signature genes were removed and the performance of the remaining 35 genes was evaluated using the endpoint to determine the impact on HR (Hazard Ratio) performance when these 10 genes were removed in the following 2 datasets:

- Tothill - 278 samples
- ICON7 SOC (Standard of Care) - 140 samples

ICON7 was evaluated using the PFS endpoint and Tothill was evaluated using the OS (Overall Survival) endpoint. Within each of the 2 datasets, the signature genes were weighted based upon the change in HR performance (Delta HR) based upon their inclusion or exclusion. Genes ranked '1' have the most negative impact on performance when removed and those ranked '45' have the least impact on performance when removed.

**Minimum gene analysis**

The purpose of evaluating the minimum number of genes is to determine if significant performance can be achieved within smaller subsets of the original signature.

This analysis involved 10,000 random samplings of the 45 signature genes starting at 1 gene/feature, up to a maximum of 20 genes/features. For each randomly selected feature length, the signature was redeveloped using the PLS machine learning method under CV and model parameters derived. At each feature length, all randomly selected signatures were applied to calculate signature scores for the following 2 datasets:

- Tothill - 278 samples
- ICON7 SOC (Standard of Care) - 140 samples

Continuous signature scores were evaluated with outcome to determine the HR effect; ICON7 was evaluated with PFS and Tothill was evaluated with OS. The HR for all random signatures at each feature length was summarized and figures generated to visualize the performance over CV.

**Results**

**Subtype analysis**

The results for the subset analysis of the 45 gene signature in the internal training dataset is provided in this section.

- AUC and ANOVA: All genes have a p value significantly less than the 0.05 threshold for ANOVA (See Table 13). This suggests that every individual gene in the 45 gene signature has the ability to detect the subtype independent of the other 44 genes.
- C-Index: 38 of the 45 genes have expression values that significantly discriminate the survival probability of the patients (See Table 13). The C-Index values for each of these genes is higher than 0.5 therefore indicating that lower expression correlates to higher survival probability for the 38 genes.

**Core gene analysis**

The results for the core gene analysis of the 45 gene signature in the 2 datasets is provided in this section.

- Tothill: Delta HR performance measured in this dataset for the 45 signature genes is shown in Figure 26 (which is also represented as Table 14). This figure highlights the top 10 ranked genes in the signature which are the most important in retaining a good HR performance within this dataset.
- ICON7: Delta HR performance measured in this dataset for the 45 signature genes is shown in Figure 27 (which is also represented as Table 15). This figure highlights the top 10 ranked genes in the signature which are the most important in retaining a good HR performance within this dataset.
- Delta HR across these 2 datasets was evaluated to obtain a combined gene ranking for each of the signature genes. The ranks assigned to the signature genes based on the core set analysis has been outlined in Table A.

**Minimum gene analysis**

The results for the minimum gene analysis of the 45 gene signature in 2 datasets is provided in this section.

- Tothill: The average HR performance measured in this dataset using the random sampling of the signature genes from a feature length of 1 to 20 is shown in Figure 28. This figure shows that to retain a significant HR performance (i.e. HR < 1) a minimum of 2 of the signature genes must be selected.

- ICON7 SOC: The average HR performance measured in this dataset using the random sampling of the signature genes from a feature length of 1 to 20 is shown in Figure 29. This figure shows that to retain a significant HR performance (i.e. HR < 1) a minimum of 1 of the signature genes must be selected.

## TABLES

***Table 1*: Number of dataset overlapping genes defined as high ranking by combined average variance-intensit in each disease indication**

| Disease Indication | # High Ranked Genes |
|---|---|
| **Ovarian** | 14507 |
| **Colon** | 13151 |
| **Lung** | 13422 |
| **Melanoma** | 10820 |

***Table 2:*** List of Entrez gene IDs, gene symbols and description for the 15 gene signature which was selected to predict the MEK subtype

| Entrez Gene ID | Gene Symbol | Description |
|---|---|---|
| 1009 | CDH11 | cadherin 11, type 2, OB-cadherin (osteoblast) |
| 11031 | RAB31 | RAB31, member RAS oncogene family |
| 1289 | COL5A1 | collagen, type V, alpha 1 |
| 1300 | COL10A1 | collagen, type X, alpha 1 |
| 1462 | VCAN | versican |
| 2191 | FAP | fibroblast activation protein, alpha |
| 2335 | FN1 | fibronectin 1 |
| 23452 | ANGPTL2 | angiopoietin-like 2 |
| 2706 | GJB2 | gap junction protein, beta 2, 26kDa |
| 3624 | INHBA | inhibin, beta A |
| 4323 | MMP14 | matrix metallopeptidase 14 |
| 5328 | PLAU | plasminogen activator, urokinase |
| 7057 | THBS1 | thrombospondin 1 |
| 7058 | THBS2 | thrombospondin 2 |
| 9945 | GFPT2 | glutamine-fructose-6-phosphate transaminase 2 |

***Table 3:*** The top 20 GO biological processes and GO terms from functional enrichment analysis of the 15-gene signature

| # | ID | Gene Ontology - biological process | P-value (FDR) |
|---|---|---|---|
| 1 | GO:0044243 | multicellular organismal catabolic process | 8.68E-07 |
| 2 | GO:0022610 | biological adhesion | 9.26E-07 |
| 3 | GO:0007155 | cell adhesion | 9.11E-07 |

(continued)

| # | ID | Gene Ontology - biological process | P-value (FDR) |
|---|---|---|---|
| 4 | GO:0034329 | cell junction assembly | 8.21E-07 |
| 5 | GO:0043062 | extracellular structure organization | 6.51E-07 |
| 6 | GO:0030198 | extracellular matrix organization | 6.51E-07 |
| 7 | GO:0034330 | cell junction organization | 1.46E-06 |
| 8 | GO:0072359 | circulatory system development | 4.65E-06 |
| 9 | GO:0072358 | cardiovascular system development | 4.65E-06 |
| 10 | GO:0022617 | extracellular matrix disassembly | 4.71E-06 |
| 11 | GO:0001568 | blood vessel development | 5.55E-06 |
| 12 | GO:0044236 | multicellular organismal metabolic process | 6.06E-06 |
| 13 | GO:0001944 | vasculature development | 7.34E-06 |
| 14 | GO:0007160 | cell-matrix adhesion | 1.41E-05 |
| 15 | GO:0001525 | angiogenesis | 2.09E-05 |
| 16 | GO:0045216 | cell-cell junction organization | 2.55E-05 |
| 17 | GO:0009653 | anatomical structure morphogenesis | 3.04E-05 |
| 18 | GO:0007044 | cell-substrate junction assembly | 3.41E-05 |
| 19 | GO:0051895 | negative regulation of focal adhesion assembly | 3.94E-05 |
| 20 | GO:0001952 | regulation of cell-matrix adhesion | 3.56E-05 |

***Table 4:*** **A)** Weighting and bias breakdown for each probeset within the 45-gene signature. B) Weighting and bias breakdown for each probeset within the 15-gene signature.

| a. | | | |
|---|---|---|---|
| 45 Gene Signature | | | |
| Rank | Gene Name | Weight | Bias |
| 1 | TMEM200A | 0.059481295 | 3.681329367 |
| 2 | GJB2 | 0.055985433 | 4.479833955 |
| 3 | MMP13 | 0.038284076 | 3.724107067 |
| 4 | GFPT2 | 0.037990641 | 4.860237265 |
| 5 | POSTN | -0.035480409 | 4.359882019 |
| 6 | BICC1 | 0.030426737 | 3.698203663 |
| 7 | CDH11 | 0.028340142 | 4.996780524 |
| 8 | MRVI1 | 0.025598535 | 5.076083782 |
| 9 | PMP22 | 0.024034610 | 5.564463361 |
| 10 | COL11A1 | -0.023672753 | 3.500170591 |
| 11 | IGFL2 | 0.022225316 | 3.310383445 |
| 12 | LUM | -0.022014619 | 8.336273473 |
| 13 | NTM | -0.021750365 | 4.230245127 |
| 14 | BGN | 0.021089508 | 10.15236225 |

(continued)

| a. | | | |
|---|---|---|---|
| 45 Gene Signature | | | |
| Rank | Gene Name | Weight | Bias |
| 15 | COL3A1 | -0.021023256 | 8.323635399 |
| 16 | COL10A1 | 0.019650845 | 6.353832828 |
| 17 | RAB31 | 0.018014921 | 5.317119481 |
| 18 | ANGPTL2 | 0.016630934 | 5.639562288 |
| 19 | PLAU | 0.016596202 | 5.848820224 |
| 20 | COL8A1 | 0.016373799 | 6.419330171 |
| 21 | MIR1245 | 0.015290888 | 5.455187262 |
| 22 | POLD2 | 0.014555548 | 9.38782491 |
| 23 | NKD2 | 0.014468847 | 7.371707677 |
| 24 | FZD1 | 0.014334768 | 4.151874676 |
| 25 | COPZ2 | 0.013866713 | 5.103944696 |
| 26 | ITGA5 | 0.013561913 | 8.36627973 |
| 27 | VGLL3 | 0.012488674 | 4.501866677 |
| 28 | INHBA | -0.011763261 | 4.684272993 |
| 29 | MMP14 | 0.011010832 | 10.08406264 |
| 30 | VCAN | 0.009977966 | 5.551759846 |
| 31 | THBS2 | -0.008700202 | 8.130920944 |
| 32 | RUNX2 | 0.008333275 | 4.73450528 |
| 33 | TIMP3 | 0.008141253 | 6.498316457 |
| 34 | SFRP2 | -0.007890741 | 5.601725816 |
| 35 | COL1A2 | 0.007788938 | 6.01000198 |
| 36 | COL5A2 | -0.007217722 | 3.567060064 |
| 37 | SERPINF1 | 0.006801251 | 10.8333948 |
| 38 | KIF26B | -0.005249312 | 4.97815094 |
| 39 | TNFAIP6 | 0.004963450 | 5.361760185 |
| 40 | MMP2 | 0.003988003 | 5.362247865 |
| 41 | FN1 | 0.003130435 | 4.984016427 |
| 42 | ALPK2 | 0.002394440 | 3.513604572 |
| 43 | CTSK | 0.001542586 | 5.732155915 |
| 44 | LOXL1 | -0.001415170 | 9.593869933 |
| 45 | FAP | -0.000007237 | 5.23E+00 |

| b. | | | | |
|---|---|---|---|---|
| 15 Gene Signature | | | | |
| Rank | Gene Name | Entrez Gene ID | Weight | Bias |
| 1 | GJB2 | 2706 | 0.089719778 | 4.478098614 |
| 2 | CDH11 | 1009 | 0.066544238 | 4.990055702 |

(continued)

| b. | | | | |
|---|---|---|---|---|
| 15 Gene Signature | | | | |
| Rank | Gene Name | Entrez Gene ID | Weight | Bias |
| 3 | GFPT2 | 9945 | 0.058421032 | 4.885349473 |
| 4 | COL10A1 | 1300 | 0.040148445 | 6.357258041 |
| 5 | ANGPTL2 | 23452 | 0.038272311 | 5.631697532 |
| 6 | THBS1 | 7057 | 0.036613387 | 6.42811488 3 |
| 7 | RAB31 | 11031 | 0.033158407 | 5.300536304 |
| 8 | THBS2 | 7058 | -0.030849235 | 8.135441538 |
| 9 | INHBA | 3624 | -0.028500708 | 4.68290899 |
| 10 | MMP14 | 4323 | 0.020727894 | 10.07844987 |
| 11 | VCAN | 1462 | 0.020706504 | 5.529961284 |
| 12 | PLAU | 5328 | 0.019342831 | 5.850016491 |
| 13 | COL5A1 | 1289 | 0.010674165 | 5.569094517 |
| 14 | FAP | 2191 | -0.006101691 | 5.226391586 |
| 15 | FN1 | 2335 | -0.005998124 | 4.982941989 |

***Table 5:*** Key Pharma players with major MEK inhibitor compounds which the 45-gene and 15-gene signatures could predict response for.

| **A) RAS/RAF/MEK/ERK Inhibitors - Phase II/III Marketed** | | |
|---|---|---|
| **DRUG NAME** | **COMPANY NAME** | **DISEASE INDICATION** |
| vemurafenib (Zelboraf) | Roche | Marketed: mMelanoma Phase II: Bile Duct, Bladder, CLL, GI, Leukemia, Ovarian, Prostate, Sarcomas, Thyroid |
| regorafenib (Stivarga) | Bayer | Marketed: GIST, mCRC PhIII: HCC PhII: Bile Duct, Ovarian, Pancreatic, RCC, Salivary Gland, Soft Tissue Sarcoma; Bladder |
| dabrafenib (Tafinlar) | Novartis | Marketed: mMelanoma PhII: Thyroid, CRC, NSCLC, (GIST), Glioma, Leukemia, Brain, Multiple Myeloma, Germ Cell Tumors |
| RAF-265 | Novartis | PhII: mMelanoma |
| encorafenib | Array Biopharma | PhIII: mMelanoma PhII: mCRC |
| donafenib | Suzhou Zelgen Biopharmaceutical | PhII: Esophageal, GI, HCC, mCRC |
| NEO-100 | Neonc Technologies | PhII: Recurrent Glioblastoma Multiforme (GBM) Preclinical: Lung |
| PLX-8394 | Plexxikon | phII: Thyroid, Bile Duct, CRC, Melanoma, NSCLC phI: Leukemias |
| RXDX-105 | Ingnyta | PhII: Colon Carcinoma, Melanoma, mCRC |
| TAK-580 | Millennium Pharmaceuticals | PhII: Metastatic Melanoma; Solid Tumor PhI: Nonhematologic Malignancy |

(continued)

| A) RAS/RAF/MEK/ERK Inhibitors - Phase II/III Marketed | | |
|---|---|---|
| **DRUG NAME** | **COMPANY NAME** | **DISEASE INDICATION** |
| ulixertinib | BioMed Valley Discoveries | PhII: AML, CRC, Melanoma, Myelodysplastic Syndrome; NSCLC Preclinical: Metastatic Adenocarcinoma of The Pancreas; Pancreatic |

| B)RAS/RAF/MEK/ERK Inhibitors - Phase I, Pre-Clinical | |
|---|---|
| **DRUG NAME** | **COMPANY** |
| BAL-3833 | Basilea Pharmaceutica |
| BGB-283 | Merck KGaA |
| HM-95573 | Hanmi Pharmaceuticals |
| LY-3009120 | Eli Lilly |
| RG-7304 | Roche |
| RG-7842 | Genentech |
| salirasib | Ono Pharmaceutical |
| AEZS-136 | Aeterna Zentaris Inc. |
| ARI-4175 | Arisaph Pharmaceuticals |
| ASN-003 | Asana BioSciences |
| CCT-196969 | Basilea Pharmaceutica |
| CCT-241161 | Basilea Pharmaceutica |
| CS-410 | Chipscreen Biosciences |
| Small Molecule to Inhibit MAP4K4 for Cancer | Genentech, Inc. |
| Small Molecules to Inhibit pan-RAF Kinase for Oncology | Novartis AG |
| | |
| **DRUG NAME** | **COMPANY** |
| CT-207 | HEC Pharm Co., Ltd. |
| CT-317 | HEC Pharm Co., Ltd. |
| Drugs to Inhibit B-Raf Kinase for Cancer | Ruga Corporation |
| EBI-907 | Eternity Bioscience Inc. |
| EBI-945 | Eternity Bioscience Inc. |
| KO-947 | Kura Oncology, Inc. |
| LXH-254 | Novartis AG |
| MDC-1016 | Medicon Pharmaceuticals, Inc |
| MT-477 | Medisyn Technologies, Inc. |
| NCB-0594 | Carna Biosciences, Inc. |
| NCB-0846 | Carna Biosciences, Inc. |

(continued)

| DRUG NAME | COMPANY |
|---|---|
| NMSP-285 | Nerviano Medical Sciences S.r.l. |
| ON-108600 | Onconova Therapeutics, Inc. |
| PV-103 | PepVax, Inc. |
| RX-8243 | Rexahn Pharmaceuticals, Inc. |
| STP-503 | Sirnaomics, Inc. |
| BAL-3833 | Basilea Pharmaceutica |
| BGB-283 | Merck KGaA |
| HM-95573 | Hanmi Pharmaceuticals |
| LY-3009120 | Eli Lilly |
| RG-7304 | Roche |
| RG-7842 | Genentech |
| salirasib | Ono Pharmaceutical |
| AEZS-136 | Aeterna Zentaris Inc. |
| ARI-4175 | Arisaph Pharmaceuticals |
| ASN-003 | Asana BioSciences |
| CCT-196969 | Basilea Pharmaceutica |
| CCT-241161 | Basilea Pharmaceutica |
| CS-410 | Chipscreen Biosciences |
| Small Molecule to Inhibit MAP4K4 for Cancer | Genentech, Inc. |
| Small Molecules to Inhibit pan-RAF Kinase for Oncology | Novartis AG |
| | |
| **DRUG NAME** | **COMPANY** |
| CT-207 | HEC Pharm Co., Ltd. |
| CT-317 | HEC Pharm Co., Ltd. |
| Drugs to Inhibit B-Raf Kinase for Cancer | Ruga Corporation |
| EBI-907 | Eternity Bioscience Inc. |
| EBI-945 | Eternity Bioscience Inc. |
| KO-947 | Kura Oncology, Inc. |
| LXH-254 | Novartis AG |
| MDC-1016 | Medicon Pharmaceuticals, Inc |
| MT-477 | Medisyn Technologies, Inc. |

(continued)

| **DRUG NAME** | **COMPANY** |
|---|---|
| NCB-0594 | Carna Biosciences, Inc. |
| NCB-0846 | Carna Biosciences, Inc. |
| NMSP-285 | Nerviano Medical Sciences S.r.l. |
| ON-108600 | Onconova Therapeutics, Inc. |
| PV-103 | PepVax, Inc. |
| RX-8243 | Rexahn Pharmaceuticals, Inc. |
| STP-503 | Sirnaomics, Inc. |

***Table 6:*** Key Pharma players with major SRC inhibitor compounds which the 45-gene and 15-gene signatures could predict response for.

| DRUG NAME | COMPANY | INDICATION | PRODUCT STAGE |
|---|---|---|---|
| ilorasertib | AbbVie | Solid Tumor Ovarian | Ph II Pre-clinical |
| KX-01 | Athenex | Leukemia | PhI |
| pazopanib hydrochloride + pembrolizumab | GSK | mRCC | PhII |
| tesevatinib tosylate | Kadmon Corporation | mBrain, mBreast, NSCLC | PhII |
| VAL-201 | ValiRx Plc | Breast, mProstate, Ovarian | PhII |
| AZD-0424 | AZ | Oncology | PhI |
| BGB-102 | BeiGene(Beijing) | Oncology | PhI |
| KX-02 | Athenex, Inc. | Brain, Lymphoma | PhI |
| rebastinib tosylate | Deciphera Pharmaceuticals | Leukemia mBreast | PhI Pre-clinical |
| ASN-006 | Asana BioSciences | Oncology | Pre-clinical |
| CCT-196969 CCT-241161 | Basilea Pharmaceutica AG | Melanoma | Pre-clinical |
| ORB-0001 | OriBase Pharma | Leukemia | Pre-clinical |
| Misc | MI.TO. Technology S.r.L. | Oncology | Pre-clinical |
| Misc | University of Toledo | Prostate | Pre-clinical |
| RK-20449 | Riken Advanced Science Institute | Leukemia | Pre-clinical |
| Various others in Discovery stage by Academic institutions | | | |

***Table 7:*** Key Pharma players with major EMT inhibitor compounds, mainly AXL inhibitors, which the 45-gene and 15-gene signatures could predict response for.

| DRUG NAME | COMPANY | INDICATION | PRODUCT STAGE |
|---|---|---|---|
| gilteritinib fumarate | Astellas | NSCLC Leukemia | Ph III Ph II |
| MGCD-265 | Mirati Therapeutics | Head & Neck, NSCLC | Ph II |
| MGCD-516 | Mirati Therapeutics | Lung | Ph I |
| S-49076 | Servier | NSCLC, Brain Colon, HCC | Ph II Pre-clinical |
| BPI-9016 | Zhejiang BetaPharma | Gastic, Liver, Lung | Ph I |
| CT-053 | EC Pharm Co., Ltd. | Brain Bladder, Breast, HCC, RCC, Lung, Ovarian | Ph I Pre-clinical |
| BGB-324 | BerGenBio | Leukemia, Lung Breast, Pancreatic | Ph I Pre-clinical |
| BGB-10C9 | BerGenBio | Pancreatic | Pre-clinical |
| Misc AXLi | BerGenBio | Oncology | Pre-clinical |
| HuMax-AXL-ADC | GenMab | Solid Tumor | Pre-clinical |
| LDC-2636 | Lead Discovery Center | Leukemia | Pre-clinical |
| Misc AXLi | Protelica Incorporated | Oncology | Pre-clinical |
| NPS-1034 | NeoPharm | NSCLC | Pre-clinical |
| Q-701 | Qurient Co., Ltd. | Oncology | Pre-clinical |
| RXDX-106 | Ignyta, Inc. | Leukemia, Breast, GI, Melanoma; mCRC, NSCLC, Ovarian, Pancreatic | Pre-clinical |
| SGI-7079 | Tolero Pharmaceuticals, Inc. | NSCLC | Pre-clinical |
| TP-0903 | Tolero Pharmaceuticals, Inc. | Leukemia; Head And Neck, Lung, Pancreatic | Pre-clinical |
| Misc AXLi | SignalChem Lifesciences | Oncology | Pre-clinical |
| Misc AXLi | University of Colorado | NSCLC | Pre-clinical |
| Misc AXLi | Kolltan Pharmaceuticals | Oncology | Discovery |

**Table 8 – EMT gene analysis: Angio_immune/MEK/EMT group vs Rest**

| EMT related gene | AUC (CI) | P VALUE |
|---|---|---|
| VIMENTIN | 0.6706 (0.6053 to 0.7359) | P= < 0.0001 |
| AXL RTK | 0.6637 0.5971 to 0.7304 | P= < 0.0001 |
| TWIST1 | 0.7674 (0.7096 to 0.8252) | P= < 0.0001 |
| SNAIL | 0.5748 (0.5144 to 0.6452) | P= 0.03922 |
| SLUG | 0.7998 (0.7471 to 0.8525) | P= < 0.0001 |

**Table 9 - Up-regulated and Down-regulated genes in the Angio-Immune subgroup**

| AngioImmune | |
|---|---|
| **Up-regulated** | **Downregulated** |
| RASGRF2 | RBFOX1 |
| COL1A2 | FNBP1 |
| GDF6 | GABRG2 |
| CNTN1 | AZGP1 |
| BCHE | SLCO1B1 |
| HAND2-AS1 | RBM24 |
| FABP4 | GPR98 |
| GDF6 | SMCO3 |
| EFEMP1 | PTPRT |
| ADIPOQ | IGSF1 |
| SPATS2L | LINC00240 |
| GRP | RMST |
| EFEMP1 | MYB |
| PDLIM5 | CYP19A1 |
| MEGF10 | CYP19A1 |
| ZFHX4 | GNAO1 |
| PTPRC | NPY6R |
| THSD7A | ENDOD1 |
| UCA1 | RMST |
| ADH1B | GJB7 |

(continued)

| AngioImmune | |
|---|---|
| **Up-regulated** | **Downregulated** |
| PRKG1 | TPTE2P5 |
| CRISPLD2 | FAM124A |
| GLRX | FCGBP |
| LRRN4CL | SH3GL2 |
| DEPDC7 | DBF4 |
| PIEZO2 | MYH6 |
| HOXA5 | SLC6A15 |
| GFRA1 | PKHD1L1 |
| FYB | SYBU |
| HS3ST3B1 | C11orf70 |
| FMO2 | TPRG1 |
| BEND6 | ZNF98 |
| PPP1R3B | RNF39 |
| GALNT15 | C6orf183 |
| CBLN4 | GLDN |
| ADH1B | OR52L1 |
| TBX5 | CYP4A11 |
| PAPPA | CACHD1 |
| TFEC | CHRM2 |
| C4orf32 | SMARCE1P2 |
| BNC1 | EPHA6 |
| MFAP5 | HIBCH |
| CALB1 | RFX3 |
| MCTP1 | |
| CDH13 | |
| HOXA5 | |
| FEM1B | |
| TDO2 | |
| UCA1 | |
| RUNX1T1 | |
| IGHG3 | |
| CHSY3 | |
| ELN | |
| RAB30 | |
| C1QTNF7 | |
| CBLC | |
| CD36 | |

(continued)

| AngioImmune | |
|---|---|
| **Up-regulated** | **Downregulated** |
| MDFIC | |
| TENM3 | |
| LSAMP | |
| TAGAP | |
| LMOD1 | |
| HRH1 | |
| FPR3 | |
| GAPDHP59 | |
| GALNT5 | |
| PDE1A | |
| EMCN | |
| STEAP4 | |
| IL18R1 | |
| CXCL9 | |
| DOCK8 | |
| ASIC2 | |
| HMCN1 | |
| CAMK2D | |
| SPATA9 | |
| CD2 | |
| MDFIC | |
| CXCL5 | |
| FBXO32 | |
| ARHGAP42 | |
| IFNG-AS1 | |
| LCP2 | |
| SLC7A5 | |
| PLIN1 | |
| COL12A1 | |
| P2RY8 | |
| CCR1 | |
| DLX5 | |
| ADAMTS5 | |
| SNORD114-7 | |
| CHRNA1 | |
| TLR1 | |
| NR4A1 | |

(continued)

| AngioImmune | |
|---|---|
| **Up-regulated** | **Downregulated** |
| GLRX | |
| DCLK1 | |
| PDE1A | |
| NR4A3 | |
| MMP1 | |
| LRRK2 | |
| CELF2 | |
| GUCY1A3 | |
| SLAM F7 | |
| BIRC3 | |
| DPP4 | |
| ADH1B | |
| SIGLEC10 | |
| LRRK2 | |
| CRLS1 | |
| TFEC | |
| CLEC1A | |
| ADRA2A | |
| NFATC2 | |
| SATB2 | |
| RGCC | |
| IL6 | |
| WNT4 | |
| HIVEP3 | |
| SDC2 | |
| JAKMIP1 | |
| GPR34 | |
| PRELP | |
| HAND2-AS1 | |
| SHOX2 | |
| GPC6 | |
| TSPAN5 | |
| TNFRSF11A | |
| ALOX5AP | |
| SNORD114-31 | |
| RUNX1T1 | |
| HGF | |

(continued)

| AngioImmune | |
|---|---|
| **Up-regulated** | **Downregulated** |
| TFPI2 | |
| CHRNA1 | |
| AOX1 | |
| PDE1A | |
| ANGPTL2 | |
| FBXO40 | |
| FCRL6 | |
| NAMPT | |
| MS4A6A | |
| MPP4 | |
| PNMA2 | |
| DCLK1 | |
| CAMK2D | |
| ITGB3 | |
| FAM129A | |
| GCNT4 | |
| NPAS2 | |
| STAP1 | |
| MEGF10 | |
| FOSL2 | |
| BNIP3L | |
| SLC22A3 | |
| TNNI2 | |
| SNORD114-7 | |
| SLC24A3 | |
| PSTPIP2 | |
| PLA2R1 | |
| PRDM6 | |
| HGF | |
| AOX1 | |
| HRH1 | |
| BNC2 | |
| LINC00842 | |
| IGJ | |
| ERCC1 | |
| RFX2 | |
| RARRES1 | |

(continued)

| AngioImmune | |
|---|---|
| **Up-regulated** | **Downregulated** |
| CPXM2 | |
| ATP11A | |
| BAG2 | |
| IRF4 | |
| SKAP2 | |
| UBAC2 | |
| ADAMTS9-AS2 | |
| SEMA5A | |
| BMP2 | |
| FOXO1 | |
| LINC00626 | |
| NKD1 | |
| ZMYM5 | |
| NPTX1 | |
| NR4A3 | |

## Table 10: Angio_Immune molecular subtype gene expression and function data

| Directionality | Group | Description | Query.size | Population | Target.size | Intersection | Pvalue | Pvalue.fdr | Genes |
|---|---|---|---|---|---|---|---|---|---|
| upregulated | GO:0048870 | cell motility | 139 | 15462 | 978 | 30 | 2.24E-09 | 2.25E-06 | GPC6, DCLK1, SOS1, SEMA5A, ADRA2A, CD2, HOXA5, NFATC2, MMP1, |

| | | | | | | | | | |
|---|---|---|---|---|---|---|---|---|---|
| | | | | | | | | | SATB2, LRRK2, NKD1, NR4A1, PRKG1, CDH13, DPP4, CCR1, COL1A2, PTPRC, BMP2, RGCC, TNFRSF11A, ITGB3, WNT4, CXCL5, IL6, TBX5, SLC7A5, CXCL9, ADIPOQ |
| upreg ulated | GO:00 51674 | localization of cell | 139 | 154 62 | 978 | 30 | 2.24 E-09 | 2.25 E-06 | GPC6, DCLK1, SOS1, SEMA5A, ADRA2A, CD2, HOXA5, NFATC2, MMP1, SATB2, LRRK2, NKD1, NR4A1, PRKG1, CDH13, DPP4, CCR1, COL1A2, PTPRC, BMP2, RGCC, TNFRSF11A, ITGB3, WNT4, CXCL5, IL6, TBX5, SLC7A5, CXCL9, ADIPOQ |
| upreg ulated | GO:00 40011 | locomotion | 139 | 154 62 | 129 6 | 35 | 2.25 E-09 | 2.25 E-06 | GPC6, DCLK1, ADRA2A, CD2, HOXA5, NFATC2, NKD1, GFRA1, DPP4, COL1A2, ITGB3, RGCC, CXCL9, CNTN1, NR4A3, FPR3, SEMA5A, SOS1, SATB2, MMP1, LRRK2, NR4A1, PRKG1, DLX5, CDH13, PTPRC, BMP2, CCR1, TNFRSF11A, CXCL5, WNT4, IL6, TBX5, SLC7A5, ADIPOQ |
| upreg ulated | GO:00 16477 | cell migration | 139 | 154 62 | 934 | 29 | 3.33 E-09 | 2.50 E-06 | GPC6, DCLK1, SOS1, SEMA5A, ADRA2A, CD2, HOXA5, NFATC2, MMP1, SATB2, LRRK2, NR4A1, PRKG1, CDH13, DPP4, CCR1, COL1A2, PTPRC, BMP2, RGCC, TNFRSF11A, ITGB3, WNT4, CXCL5, IL6, TBX5, SLC7A5, CXCL9, ADIPOQ |
| upreg ulated | GO:00 06928 | cellular component movement | 139 | 154 62 | 141 3 | 35 | 2.10 E-08 | 1.26 E-05 | GPC6, DCLK1, ADRA2A, CD2, HOXA5, NFATC2, NKD1, GFRA1, DPP4, COL1A2, ITGB3, RGCC, CXCL9, CNTN1, NR4A3, FPR3, SEMA5A, SOS1, SATB2, MMP1, LRRK2, NR4A1, PRKG1, DLX5, CDH13, PTPRC, BMP2, CCR1, TNFRSF11A, CXCL5, WNT4, IL6, TBX5, SLC7A5, ADIPOQ |
| upreg ulated | GO:00 48584 | positive regulation of response to stimulus | 139 | 154 62 | 135 9 | 34 | 2.76 E-08 | 1.38 E-05 | IRF4, SHOX2, FABP4, ADRA2A, NFATC2, NPAS2, NKD1, GDF6, FYB, ITGB3, RGCC, LCP2, CXCL9, TSPAN5, TLR1, SEMA5A, SOS1, PLA2R1, MDFIC, ALOX5AP, CD36, LRRK2, STAP1, BIRC3, DLX5, CDH13, PTPRC, BMP2, CCR1, TNFRSF11A, WNT4, IL6, SKAP2, ADIPOQ |
| upreg ulated | GO:00 32501 | multicellular organismal process | 139 | 154 62 | 607 1 | 84 | 3.32 E-07 | 0.00 013 827 7 | SHOX2, IRF4, SPATA9, FABP4, TNNI2, NPAS2, NPTX1, COL1A2, LCP2, ASIC2, BCHE, FOXO1, CNTN1, PAPPA, TLR1, PRELP, TENM3, SOS1, CHRNA1, PLA2R1, FEM1B, ALOX5AP, FOSL2, PIEZO2, CD36, MMP1, SATB2, PRKG1, PDE1A, GUCY1A3, TNFRSF11A, IL6, TBX5, SLC7A5, DCLK1, ADRA2A, CD2, COL12A1, THSD7A, HOXA5, IGJ, NFATC2, PDLIM5, LSAMP, NKD1, GDF6, CELF2, GFRA1, IL18R1, JAKMIP1, DPP4, HIVEP3, CAMK2D, ITGB3, RGCC, PRDM6, BNC2, ERCC1, TFPI2, NR4A3, HMCN1, SEMA5A, EFEMP1, LMOD1, CALB1, DOCK8, CRISPLD2, FBXO32, ELN, LRRK2, BIRC3, NR4A1, DLX5, MEGF10, SDC2, GCNT4, CDH13, ANGPTL4, PTPRC, CCR1, BMP2, WNT4, NAMPT, ADIPOQ |
| upreg ulated | GO:00 44707 | single-multicellular organism process | 139 | 154 62 | 587 0 | 82 | 3.69 E-07 | 0.00 013 827 7 | SHOX2, IRF4, SPATA9, FABP4, TNNI2, NPAS2, NPTX1, COL1A2, LCP2, ASIC2, BCHE, FOXO1, CNTN1, TLR1, PRELP, TENM3, SOS1, CHRNA1, PLA2R1, FEM1B, ALOX5AP, PIEZO2, CD36, MMP1, SATB2, PRKG1, PDE1A, GUCY1A3, TNFRSF11A, IL6, TBX5, SLC7A5, DCLK1, ADRA2A, CD2, COL12A1, THSD7A, HOXA5, IGJ, NFATC2, PDLIM5, LSAMP, NKD1, GDF6, |

| | | | | | | | | | |
|---|---|---|---|---|---|---|---|---|---|
| | | | | | | | | | CELF2, GFRA1, IL18R1, JAKMIP1, DPP4, CAMK2D, HIVEP3, ITGB3, RGCC, PRDM6, BNC2, ERCC1, TFPI2, NR4A3, HMCN1, SEMA5A, EFEMP1, LMOD1, CALB1, DOCK8, CRISPLD2, FBXO32, ELN, LRRK2, BIRC3, NR4A1, DLX5, MEGF10, SDC2, GCNT4, CDH13, ANGPTL4, PTPRC, CCR1, BMP2, WNT4, NAMPT, ADIPOQ |
| upreg ulated | GO:00 09611 | response to wounding | 139 | 154 62 | 113 8 | 28 | 8.51 E-07 | 0.00 028 384 6 | TLR1, SOS1, FABP4, ADRA2A, CD2, ALOX5AP, CD36, MMP1, DOCK8, BIRC3, PRKG1, PDE1A, SDC2, AOX1, CCR1, BMP2, COL1A2, GUCY1A3, TNFRSF11A, ITGB3, WNT4, LCP2, IL6, ASIC2, SLC7A5, TFPI2, CXCL9, ADIPOQ |
| upreg ulated | GO:00 50896 | response to stimulus | 139 | 154 62 | 723 0 | 93 | 1.19 E-06 | 0.00 035 769 9 | TAGAP, FAM129A, P2RY8, SHOX2, IRF4, FABP4, RAB30, BNIP3L, TFEC, NPAS2, GRP, NPTX1, AOX1, COL1A2, LCP2, ASIC2, BCHE, FOXO1, ARHGAP42, CNTN1, PAPPA, TSPAN5, TLR1, TENM3, SOS1, CHRNA1, PLA2R1, MDFIC, FEM1B, ALOX5AP, FOSL2, CD36, PIEZO2, MMP1, SATB2, GPR34, PRKG1, PDE1A, GUCY1A3, TNFRSF11A, IL6, SLC7A5, GPC6, CBLC, DCLK1, ADRA2A, CD2, NFATC2, IGJ, NKD1, GDF6, GFRA1, RASGRF2, IL18R1, DEPDC7, DPP4, FYB, CAMK2D, ITGB3, RGCC, ERCC1, TFPI2, ADH1B, CXCL9, SLC22A3, NR4A3, SLAMF7, HMCN1, FPR3, SEMA5A, CLEC1A, EFEMP1, MCTP1, CALB1, DOCK8, LRRK2, FBXO32, STAP1, BIRC3, NR4A1, DLX5, SDC2, GCNT4, CDH13, ANGPTL4, CCR1, BMP2, PTPRC, WNT4, CXCL5, NAMPT, SKAP2, ADIPOQ |
| upreg ulated | GO:00 48731 | system development | 139 | 154 62 | 361 5 | 57 | 2.69 E-06 | 0.00 068 549 2 | IRF4, DCLK1, SHOX2, CD2, COL12A1, THSD7A, HOXA5, PDLIM5, LSAMP, NPAS2, NKD1, GDF6, NPTX1, GFRA1, IL18R1, COL1A2, HIVEP3, ITGB3, RGCC, PRDM6, BNC2, ERCC1, ASIC2, BCHE, CNTN1, FOXO1, NR4A3, PRELP, CHRNA1, TENM3, SEMA5A, SOS1, EFEMP1, FEM1B, CALB1, SATB2, CRISPLD2, ELN, LRRK2, NR4A1, PRKG1, DLX5, MEGF10, SDC2, GCNT4, CDH13, ANGPTL4, PTPRC, BMP2, CCR1, TNFRSF11A, WNT4, IL6, TBX5, NAMPT, SLC7A5, ADIPOQ |
| upreg ulated | GO:00 06950 | response to stress | 139 | 154 62 | 307 2 | 51 | 2.80 E-06 | 0.00 068 549 2 | FAM129A, IRF4, FABP4, ADRA2A, CD2, IGJ, NFATC2, BNIP3L, TFEC, NPAS2, NKD1, DPP4, AOX1, COL1A2, CAMK2D, ITGB3, RGCC, LCP2, ERCC1, ASIC2, TFPI2, CXCL9, FOXO1, NR4A3, SLAMF7, TLR1, SOS1, CLEC1A, MDFIC, PLA2R1, FEM1B, ALOX5AP, CD36, MMP1, DOCK8, LRRK2, BIRC3, NR4A1, PRKG1, SDC2, PDE1A, ANGPTL4, PTPRC, GUCY1A3, BMP2, CCR1, TNFRSF11A, WNT4, IL6, SLC7A5, ADIPOQ |
| upreg ulated | GO:00 30154 | cell differentiation | 139 | 154 62 | 298 9 | 50 | 2.97 E-06 | 0.00 068 549 2 | IRF4, DCLK1, SHOX2, FABP4, SPATA9, CD2, THSD7A, HOXA5, PDLIM5, NKD1, GDF6, NPTX1, GFRA1, IL18R1, HIVEP3, ITGB3, RGCC, PRDM6, ERCC1, BCHE, FOXO1, CNTN1, PAPPA, NR4A3, STEAP4, TENM3, SOS1, SEMA5A, RUNX1T1, EFEMP1, FEM1B, CD36, SATB2, LRRK2, NR4A1, PRKG1, DLX5, MEGF10, SDC2, ANGPTL4, PTPRC, BMP2, CCR1, TNFRSF11A, FBXO40, WNT4, IL6, TBX5, SLC7A5, ADIPOQ |
| upreg | GO:00 | positive | 139 | 154 | 946 | 24 | 3.50 | 0.00 | TSPAN5, SOS1, SEMA5A, SHOX2, |

| | | | | | | | | | |
|---|---|---|---|---|---|---|---|---|---|
| ulated | 09967 | regulation of signal transduction | | 62 | | | E-06 | 075 003 8 | ADRA2A, MDFIC, PLA2R1, CD36, LRRK2, NKD1, STAP1, GDF6, DLX5, CDH13, CCR1, PTPRC, BMP2, TNFRSF11A, ITGB3, WNT4, IL6, CXCL9, SKAP2, ADIPOQ |
| upreg ulated | GO:00 09605 | response to external stimulus | 139 | 154 62 | 177 5 | 35 | 4.95 E-06 | 0.00 094 339 6 | GPC6, DCLK1, FABP4, IGJ, BNIP3L, GFRA1, ITGB3, ERCC1, ASIC2, CXCL9, SLC22A3, CNTN1, FOXO1, NR4A3, FPR3, TLR1, SEMA5A, SOS1, ALOX5AP, FOSL2, PIEZO2, CD36, BIRC3, NR4A1, DLX5, SDC2, CDH13, CCR1, GUCY1A3, PTPRC, TNFRSF11A, CXCL5, WNT4, IL6, ADIPOQ |
| upreg ulated | GO:00 48518 | positive regulation of biological process | 139 | 154 62 | 387 3 | 59 | 5.03 E-06 | 0.00 094 339 6 | FAM129A, IRF4, SHOX2, FABP4, ADRA2A, CD2, TNNI2, HOXA5, IGJ, NFATC2, BNIP3L, NPAS2, NKD1, GDF6, IL18R1, RASGRF2, DPP4, PNMA2, HIVEP3, FYB, CAMK2D, ITGB3, RGCC, LCP2, ASIC2, CXCL9, CNTN1, FOXO1, NR4A3, TSPAN5, TLR1, TENM3, SEMA5A, SOS1, PLA2R1, MDFIC, ALOX5AP, FOSL2, CD36, SATB2, LRRK2, STAP1, BIRC3, NR4A1, DLX5, CDH13, ANGPTL4, PTPRC, GUCY1A3, BMP2, CCR1, TNFRSF11A, CXCL5, WNT4, IL6, TBX5, NAMPT, SKAP2, ADIPOQ |
| upreg ulated | GO:00 44699 | single-organism process | 139 | 154 62 | 122 24 | 12 9 | 7.63 E-06 | 0.00 132 686 | TAGAP, P2RY8, SHOX2, IRF4, SPATA9, FABP4, HS3ST3B1, TNNI2, RAB30, BNIP3L, NPAS2, MFAP5, GRP, NPTX1, PNMA2, AOX1, COL1A2, LCP2, ASIC2, BCHE, FOXO1, ARHGAP42, CNTN1, PAPPA, TSPAN5, CRLS1, TLR1, PRELP, STEAP4, TENM3, CHRNA1, SOS1, MDFIC, PLA2R1, FEM1B, ALOX5AP, FOSL2, PIEZO2, CD36, MMP1, SATB2, GPR34, PRKG1, PDE1A, GUCY1A3, TNFRSF11A, GALNT14, CHSY3, IL6, CBLN4, TBX5, SLC7A5, GPC6, CBLC, DCLK1, CD2, ADRA2A, COL12A1, THSD7A, HOXA5, IGJ, NFATC2, PDLIM5, LSAMP, NKD1, GDF6, CELF2, GLRX, GFRA1, RASGRF2, GALNT5, IL18R1, DPP4, DEPDC7, JAKMIP1, FYB, CAMK2D, HIVEP3, RGCC, ITGB3, PRDM6, FMO4, BNC2, ERCC1, TFPI2, CXCL9, ADH1B, SLC22A3, NR4A3, HMCN1, FPR3, SLAMF7, SEMA5A, CLEC1A, ADAMTS5, RUNX1T1, EFEMP1, PPP1R3B, LMOD1, MCTP1, DOCK8, CALB1, ATP11A, CRISPLD2, ELN, FBXO32, LRRK2, BIRC3, STAP1, NR4A1, DLX5, CDH13, SDC2, GCNT4, MEGF10, ANGPTL4, PLIN1, BMP2, PTPRC, CCR1, FBXO40, WNT4, CXCL5, SLC24A3, RARRES1, NAMPT, SKAP2, TDO2, ADIPOQ |
| upreg ulated | GO:00 10647 | positive regulation of cell communication | 139 | 154 62 | 993 | 24 | 7.99 E-06 | 0.00 132 686 | TSPAN5, SOS1, SEMA5A, SHOX2, ADRA2A, MDFIC, PLA2R1, CD36, LRRK2, NKD1, STAP1, GDF6, DLX5, CDH13, CCR1, PTPRC, BMP2, TNFRSF11A, ITGB3, WNT4, IL6, CXCL9, SKAP2, ADIPOQ |
| upreg ulated | GO:00 23056 | positive regulation of signaling | 139 | 154 62 | 996 | 24 | 8.40 E-06 | 0.00 132 686 | TSPAN5, SOS1, SEMA5A, SHOX2, ADRA2A, MDFIC, PLA2R1, CD36, LRRK2, NKD1, STAP1, GDF6, DLX5, CDH13, CCR1, PTPRC, BMP2, TNFRSF11A, ITGB3, WNT4, IL6, CXCL9, SKAP2, ADIPOQ |
| upreg ulated | GO:00 51716 | cellular response to stimulus | 139 | 154 62 | 586 6 | 78 | 9.63 E-06 | 0.00 144 454 | TAGAP, FAM129A, P2RY8, SHOX2, IRF4, FABP4, RAB30, BNIP3L, TFEC, NPAS2, GRP, NPTX1, COL1A2, LCP2, |

| | | | | | | | | | |
|---|---|---|---|---|---|---|---|---|---|
| | | | | | | | | 2 | ASIC2, FOXO1, ARHGAP42, CNTN1, TSPAN5, TLR1, TENM3, SOS1, CHRNA1, PLA2R1, MDFIC, FEM1B, ALOX5AP, FOSL2, CD36, SATB2, GPR34, PRKG1, PDE1A, GUCY1A3, TNFRSF11A, IL6, GPC6, CBLC, DCLK1, ADRA2A, CD2, NFATC2, NKD1, GDF6, GFRA1, IL18R1, RASGRF2, DEPDC7, FYB, CAMK2D, ITGB3, RGCC, ERCC1, ADH1B, CXCL9, NR4A3, FPR3, SEMA5A, CLEC1A, EFEMP1, MCTP1, CALB1, DOCK8, LRRK2, STAP1, BIRC3, NR4A1, DLX5, SDC2, CDH13, CCR1, BMP2, PTPRC, CXCL5, WNT4, NAMPT, SKAP2, ADIPOQ |
| upreg ulated | GO:00 48869 | cellular developmental process | 139 | 154 62 | 311 7 | 50 | 1.01 E-05 | 0.00 145 023 6 | IRF4, DCLK1, SHOX2, FABP4, SPATA9, CD2, THSD7A, HOXA5, PDLIM5, NKD1, GDF6, NPTX1, GFRA1, IL18R1, HIVEP3, ITGB3, RGCC, PRDM6, ERCC1, BCHE, FOXO1, CNTN1, PAPPA, NR4A3, STEAP4, TENM3, SOS1, SEMA5A, RUNX1T1, EFEMP1, FEM1B, CD36, SATB2, LRRK2, NR4A1, PRKG1, DLX5, MEGF10, SDC2, ANGPTL4, PTPRC, BMP2, CCR1, TNFRSF11A, FBXO40, WNT4, IL6, TBX5, SLC7A5, ADIPOQ |
| upreg ulated | GO:00 07596 | blood coagulation | 139 | 154 62 | 500 | 16 | 1.09 E-05 | 0.00 146 810 8 | SOS1, ADRA2A, CD2, CD36, MMP1, DOCK8, PRKG1, PDE1A, COL1A2, GUCY1A3, ITGB3, LCP2, IL6, ASIC2, TFPI2, SLC7A5 |
| upreg ulated | GO:00 42060 | wound healing | 139 | 154 62 | 620 | 18 | 1.13 E-05 | 0.00 146 810 8 | SOS1, ADRA2A, CD2, CD36, MMP1, DOCK8, PRKG1, PDE1A, SDC2, COL1A2, GUCY1A3, ITGB3, LCP2, WNT4, IL6, ASIC2, SLC7A5, TFPI2 |
| upreg ulated | GO:00 50817 | coagulation | 139 | 154 62 | 503 | 16 | 1.18 E-05 | 0.00 147 042 7 | SOS1, ADRA2A, CD2, CD36, MMP1, DOCK8, PRKG1, PDE1A, COL1A2, GUCY1A3, ITGB3, LCP2, IL6, ASIC2, TFPI2, SLC7A5 |
| upreg ulated | GO:00 07599 | hemostasis | 139 | 154 62 | 505 | 16 | 1.24 E-05 | 0.00 148 280 4 | SOS1, ADRA2A, CD2, CD36, MMP1, DOCK8, PRKG1, PDE1A, COL1A2, GUCY1A3, ITGB3, LCP2, IL6, ASIC2, TFPI2, SLC7A5 |
| upreg ulated | GO:00 51239 | regulation of multicellular organismal process | 139 | 154 62 | 194 3 | 36 | 1.42 E-05 | 0.00 164 182 2 | IRF4, SHOX2, ADRA2A, CD2, HOXA5, PDLIM5, NKD1, CELF2, GDF6, IL18R1, CAMK2D, ITGB3, RGCC, ASIC2, CNTN1, FOXO1, TLR1, SEMA5A, TENM3, EFEMP1, CD36, LRRK2, FBXO32, BIRC3, DLX5, SDC2, MEGF10, ANGPTL4, GUCY1A3, BMP2, CCR1, TNFRSF11A, WNT4, IL6, TBX5, ADIPOQ |
| upreg ulated | GO:00 01775 | cell activation | 139 | 154 62 | 763 | 20 | 1.58 E-05 | 0.00 175 334 8 | SLAMF7, TLR1, SOS1, IRF4, ADRA2A, CD2, NFATC2, CD36, DOCK8, IL18R1, DPP4, COL1A2, PTPRC, RGCC, ITGB3, WNT4, LCP2, IL6, ERCC1, SKAP2 |
| upreg ulated | GO:00 43068 | positive regulation of programmed cell death | 139 | 154 62 | 352 | 13 | 1.74 E-05 | 0.00 186 546 2 | NR4A3, LRRK2, NR4A1, RASGRF2, PNMA2, SOS1, BMP2, RGCC, IL6, HOXA5, BNIP3L, ADIPOQ, FOXO1 |
| upreg ulated | GO:00 42127 | regulation of cell proliferation | 139 | 154 62 | 126 3 | 27 | 1.88 E-05 | 0.00 186 959 1 | SHOX2, SEMA5A, ADRA2A, HOXA5, NFATC2, FOSL2, LRRK2, NR4A1, DLX5, PDE1A, CDH13, DPP4, BMP2, PTPRC, TNFRSF11A, ITGB3, RGCC, CXCL5, IL6, RARRES1, TBX5, NAMPT, CXCL9, BCHE, SKAP2, FOXO1, ADIPOQ |
| upreg ulated | GO:00 44700 | single organism signaling | 139 | 154 62 | 543 8 | 73 | 1.93 E-05 | 0.00 186 959 1 | TAGAP, P2RY8, SHOX2, IRF4, RAB30, BNIP3L, NPAS2, GRP, NPTX1, COL1A2, LCP2, ASIC2, BCHE, FOXO1, ARHGAP42, CNTN1, TSPAN5, TLR1, TENM3, SOS1, CHRNA1, PLA2R1, MDFIC, FEM1B, CD36, GPR34, PRKG1, PDE1A, GUCY1A3, TNFRSF11A, IL6, TBX5, GPC6, CBLC, DCLK1, ADRA2A, CD2, HOXA5, NFATC2, PDLIM5, NKD1, |

| | | | | | | | | | |
|---|---|---|---|---|---|---|---|---|---|
| | | | | | | | | | GDF6, GFRA1, IL18R1, RASGRF2, DEPDC7, FYB, CAMK2D, ITGB3, CXCL9, NR4A3, FPR3, SEMA5A, CLEC1A, EFEMP1, MCTP1, CALB1, DOCK8, LRRK2, STAP1, BIRC3, NR4A1, DLX5, SDC2, CDH13, CCR1, BMP2, PTPRC, CXCL5, WNT4, NAMPT, SKAP2, ADIPOQ |
| upreg ulated | GO:00 23052 | signaling | 139 | 154 62 | 543 8 | 73 | 1.93 E-05 | 0.00 186 959 1 | TAGAP, P2RY8, SHOX2, IRF4, RAB30, BNIP3L, NPAS2, GRP, NPTX1, COL1A2, LCP2, ASIC2, BCHE, FOXO1, ARHGAP42, CNTN1, TSPAN5, TLR1, TENM3, SOS1, CHRNA1, PLA2R1, MDFIC, FEM1B, CD36, GPR34, PRKG1, PDE1A, GUCY1A3, TNFRSF11A, IL6, TBX5, GPC6, CBLC, DCLK1, ADRA2A, CD2, HOXA5, NFATC2, PDLIM5, NKD1, GDF6, GFRA1, IL18R1, RASGRF2, DEPDC7, FYB, CAMK2D, ITGB3, CXCL9, NR4A3, FPR3, SEMA5A, CLEC1A, EFEMP1, MCTP1, CALB1, DOCK8, LRRK2, STAP1, BIRC3, NR4A1, DLX5, SDC2, CDH13, CCR1, BMP2, PTPRC, CXCL5, WNT4, NAMPT, SKAP2, ADIPOQ |
| upreg ulated | GO:00 30198 | extracellular matrix organization | 139 | 154 62 | 361 | 13 | 2.27 E-05 | 0.00 206 338 5 | CRISPLD2, MFAP5, ELN, SDC2, DPP4, BMP2, ADAMTS5, COL1A2, RGCC, EFEMP1, ITGB3, COL12A1, MMP1 |
| upreg ulated | GO:00 43062 | extracellular structure organization | 139 | 154 62 | 361 | 13 | 2.27 E-05 | 0.00 206 338 5 | CRISPLD2, MFAP5, ELN, SDC2, DPP4, BMP2, ADAMTS5, COL1A2, RGCC, EFEMP1, ITGB3, COL12A1, MMP1 |
| upreg ulated | GO:00 07154 | cell communication | 139 | 154 62 | 549 3 | 73 | 2.85 E-05 | 0.00 251 443 8 | TAGAP, P2RY8, SHOX2, IRF4, RAB30, BNIP3L, NPAS2, GRP, NPTX1, COL1A2, LCP2, ASIC2, BCHE, FOXO1, ARHGAP42, CNTN1, TSPAN5, TLR1, TENM3, SOS1, CHRNA1, PLA2R1, MDFIC, FEM1B, CD36, GPR34, PRKG1, PDE1A, GUCY1A3, TNFRSF11A, IL6, TBX5, GPC6, CBLC, DCLK1, ADRA2A, CD2, HOXA5, NFATC2, PDLIM5, NKD1, GDF6, GFRA1, IL18R1, RASGRF2, DEPDC7, FYB, CAMK2D, ITGB3, CXCL9, NR4A3, FPR3, SEMA5A, CLEC1A, EFEMP1, MCTP1, CALB1, DOCK8, LRRK2, STAP1, BIRC3, NR4A1, DLX5, SDC2, CDH13, CCR1, BMP2, PTPRC, CXCL5, WNT4, NAMPT, SKAP2, ADIPOQ |
| upreg ulated | GO:00 10942 | positive regulation of cell death | 139 | 154 62 | 372 | 13 | 3.10 E-05 | 0.00 265 981 1 | NR4A3, LRRK2, NR4A1, RASGRF2, PNMA2, SOS1, BMP2, RGCC, IL6, HOXA5, BNIP3L, ADIPOQ, FOXO1 |
| upreg ulated | GO:00 45595 | regulation of cell differentiation | 139 | 154 62 | 115 3 | 25 | 3.20 E-05 | 0.00 266 887 9 | IRF4, SHOX2, TENM3, SEMA5A, EFEMP1, CD2, HOXA5, PDLIM5, CD36, LRRK2, GDF6, DLX5, MEGF10, SDC2, CCR1, BMP2, ITGB3, RGCC, WNT4, PRDM6, IL6, TBX5, FOXO1, ADIPOQ, CNTN1 |
| upreg ulated | GO:00 48583 | regulation of response to stimulus | 139 | 154 62 | 272 4 | 44 | 3.85 E-05 | 0.00 312 279 1 | TAGAP, CBLC, IRF4, SHOX2, FABP4, ADRA2A, NFATC2, NPAS2, NKD1, GDF6, IL18R1, RASGRF2, DEPDC7, FYB, CAMK2D, ITGB3, RGCC, LCP2, ASIC2, CXCL9, FOXO1, TSPAN5, TLR1, SOS1, SEMA5A, MDFIC, PLA2R1, FEM1B, ALOX5AP, CD36, FBXO32, LRRK2, STAP1, BIRC3, DLX5, CDH13, PTPRC, BMP2, CCR1, TNFRSF11A, WNT4, IL6, SKAP2, ADIPOQ |
| upreg ulated | GO:00 07165 | signal transduction | 139 | 154 62 | 493 5 | 67 | 4.33 E-05 | 0.00 341 574 6 | TAGAP, P2RY8, SHOX2, IRF4, RAB30, BNIP3L, NPAS2, GRP, COL1A2, LCP2, ASIC2, FOXO1, ARHGAP42, CNTN1, TSPAN5, TLR1, TENM3, SOS1, CHRNA1, PLA2R1, MDFIC, FEM1B, CD36, GPR34, |

| | | | | | | | | | |
|---|---|---|---|---|---|---|---|---|---|
| | | | | | | | | | PRKG1, PDE1A, GUCY1A3, TNFRSF11A, IL6, GPC6, CBLC, DCLK1, CD2, ADRA2A, NFATC2, NKD1, GDF6, GFRA1, IL18R1, RASGRF2, DEPDC7, FYB, CAMK2D, ITGB3, CXCL9, NR4A3, FPR3, SEMA5A, CLEC1A, EFEMP1, MCTP1, DOCK8, LRRK2, STAP1, BIRC3, NR4A1, DLX5, SDC2, CDH13, PTPRC, CCR1, BMP2, CXCL5, WNT4, NAMPT, SKAP2, ADIPOQ |
| upreg ulated | GO:00 44767 | single-organism developmental process | 139 | 154 62 | 474 1 | 65 | 4.59 E-05 | 0.00 352 903 8 | SHOX2, IRF4, SPATA9, FABP4, NPAS2, NPTX1, COL1A2, ASIC2, BCHE, FOXO1, CNTN1, PAPPA, STEAP4, PRELP, TENM3, SOS1, CHRNA1, PLA2R1, FEM1B, CD36, SATB2, PRKG1, TNFRSF11A, IL6, TBX5, SLC7A5, DCLK1, CD2, COL12A1, THSD7A, HOXA5, PDLIM5, LSAMP, NKD1, GDF6, GFRA1, IL18R1, HIVEP3, RGCC, ITGB3, PRDM6, BNC2, ERCC1, NR4A3, SEMA5A, RUNX1T1, EFEMP1, CALB1, ELN, LRRK2, CRISPLD2, NR4A1, DLX5, SDC2, GCNT4, CDH13, MEGF10, ANGPTL4, PTPRC, BMP2, CCR1, FBXO40, WNT4, NAMPT, ADIPOQ |
| upreg ulated | GO:00 65008 | regulation of biological quality | 139 | 154 62 | 275 5 | 44 | 5.09 E-05 | 0.00 382 217 6 | FABP4, ADRA2A, CD2, HOXA5, IGJ, BNIP3L, PDLIM5, GLRX, NPTX1, COL1A2, CAMK2D, ITGB3, LCP2, ERCC1, ASIC2, TFPI2, CXCL9, SLC22A3, FOXO1, STEAP4, CHRNA1, SOS1, SEMA5A, CD36, PIEZO2, MMP1, DOCK8, CALB1, ATP11A, ELN, LRRK2, PRKG1, GCNT4, PDE1A, ANGPTL4, PTPRC, BMP2, CCR1, GUCY1A3, TNFRSF11A, WNT4, IL6, SLC7A5, ADIPOQ |
| upreg ulated | GO:00 08283 | cell proliferation | 139 | 154 62 | 166 8 | 31 | 6.25 E-05 | 0.00 454 168 2 | SHOX2, SEMA5A, ADRA2A, HOXA5, NFATC2, FOSL2, DOCK8, LRRK2, ELN, NR4A1, DLX5, CDH13, PDE1A, DPP4, BMP2, PTPRC, TNFRSF11A, ITGB3, RGCC, WNT4, CXCL5, IL6, RARRES1, ERCC1, TBX5, NAMPT, CXCL9, BCHE, SKAP2, FOXO1, ADIPOQ |
| upreg ulated | GO:00 50878 | regulation of body fluid levels | 139 | 154 62 | 580 | 16 | 6.59 E-05 | 0.00 454 168 2 | SOS1, CD2, ADRA2A, CD36, MMP1, DOCK8, PRKG1, PDE1A, COL1A2, GUCY1A3, ITGB3, LCP2, IL6, ASIC2, TFPI2, SLC7A5 |
| upreg ulated | GO:00 32502 | developmental process | 139 | 154 62 | 479 4 | 65 | 6.67 E-05 | 0.00 454 168 2 | SHOX2, IRF4, SPATA9, FABP4, NPAS2, NPTX1, COL1A2, ASIC2, BCHE, FOXO1, CNTN1, PAPPA, STEAP4, PRELP, TENM3, SOS1, CHRNA1, PLA2R1, FEM1B, CD36, SATB2, PRKG1, TNFRSF11A, IL6, TBX5, SLC7A5, DCLK1, CD2, COL12A1, THSD7A, HOXA5, PDLIM5, LSAMP, NKD1, GDF6, GFRA1, IL18R1, HIVEP3, RGCC, ITGB3, PRDM6, BNC2, ERCC1, NR4A3, SEMA5A, RUNX1T1, EFEMP1, CALB1, ELN, LRRK2, CRISPLD2, NR4A1, DLX5, SDC2, GCNT4, CDH13, MEGF10, ANGPTL4, PTPRC, BMP2, CCR1, FBXO40, WNT4, NAMPT, ADIPOQ |
| upreg ulated | GO:00 65007 | biological regulation | 139 | 154 62 | 974 9 | 10 9 | 6.70 E-05 | 0.00 454 168 2 | TAGAP, FAM129A, P2RY8, SHOX2, IRF4, FABP4, TNNI2, RAB30, BNIP3L, TFEC, NPAS2, GRP, NPTX1, PNMA2, COL1A2, LCP2, ASIC2, BCHE, FOXO1, ARHGAP42, CNTN1, TSPAN5, TLR1, STEAP4, TENM3, CHRNA1, SOS1, MDFIC, PLA2R1, FEM1B, ALOX5AP, FOSL2, PIEZO2, CD36, MMP1, SATB2, ZFHX4, GPR34, PRKG1, PDE1A, GUCY1A3, TNFRSF11A, IL6, TBX5, SLC7A5, GPC6, CBLC, DCLK1, CD2, |

| | | | | | | | | | |
|---|---|---|---|---|---|---|---|---|---|
| | | | | | | | | | ADRA2A, HOXA5, IGJ, NFATC2, PDLIM5, NKD1, GDF6, CELF2, GLRX, GFRA1, RASGRF2, IL18R1, DPP4, DEPDC7, DHX34, CAMK2D, HIVEP3, FYB, RGCC, ITGB3, PRDM6, BNC2, ERCC1, TFPI2, CXCL9, SLC22A3, NR4A3, FPR3, SLAMF7, SEMA5A, CLEC1A, RFX2, RUNX1T1, EFEMP1, PPP1R3B, MCTP1, DOCK8, CALB1, ATP11A, ELN, FBXO32, LRRK2, BIRC3, STAP1, NR4A1, DLX5, CDH13, SDC2, GCNT4, MEGF10, ANGPTL4, BMP2, PTPRC, CCR1, WNT4, CXCL5, RARRES1, NAMPT, SKAP2, ADIPOQ |
| upreg ulated | GO:00 43065 | positive regulation of apoptotic process | 139 | 154 62 | 346 | 12 | 6.81 E-05 | 0.00 454 168 2 | NR4A3, NR4A1, RASGRF2, PNMA2, SOS1, BMP2, RGCC, IL6, HOXA5, BNIP3L, FOXO1, ADIPOQ |
| upreg ulated | GO:00 48522 | positive regulation of cellular process | 139 | 154 62 | 344 3 | 51 | 7.47 E-05 | 0.00 487 186 8 | FAM129A, IRF4, SHOX2, ADRA2A, CD2, TNNI2, HOXA5, NFATC2, BNIP3L, NPAS2, NKD1, GDF6, RASGRF2, DPP4, PNMA2, HIVEP3, ITGB3, RGCC, ASIC2, CXCL9, CNTN1, FOXO1, NR4A3, TSPAN5, TLR1, TENM3, SEMA5A, SOS1, PLA2R1, MDFIC, FOSL2, CD36, SATB2, LRRK2, STAP1, BIRC3, NR4A1, DLX5, CDH13, PTPRC, GUCY1A3, BMP2, CCR1, TNFRSF11A, CXCL5, WNT4, IL6, TBX5, NAMPT, SKAP2, ADIPOQ |
| upreg ulated | GO:00 55095 | lipoprotein particle mediated signaling | 139 | 154 62 | 2 | 2 | 8.02 E-05 | 0.00 501 666 8 | CDH13, CD36 |
| upreg ulated | GO:00 55096 | low-density lipoprotein particle mediated signaling | 139 | 154 62 | 2 | 2 | 8.02 E-05 | 0.00 501 666 8 | CDH13, CD36 |
| upreg ulated | GO:00 07169 | transmembrane receptor protein tyrosine kinase signaling pathway | 139 | 154 62 | 663 | 17 | 9.47 E-05 | 0.00 576 639 | CBLC, SOS1, ADRA2A, EFEMP1, STAP1, NR4A1, GFRA1, CDH13, PDE1A, RASGRF2, BMP2, ITGB3, LCP2, WNT4, NAMPT, FOXO1, ADIPOQ |
| upreg ulated | GO:00 02376 | immune system process | 139 | 154 62 | 204 3 | 35 | 9.99 E-05 | 0.00 576 639 | IRF4, CD2, HOXA5, IGJ, NFATC2, BNIP3L, IL18R1, DPP4, COL1A2, FYB, CAMK2D, ITGB3, RGCC, LCP2, ERCC1, CXCL9, FOXO1, SLAMF7, TLR1, SOS1, CD36, DOCK8, MMP1, BIRC3, NR4A1, PDE1A, PTPRC, CCR1, TNFRSF11A, CXCL5, WNT4, IL6, SLC7A5, SKAP2, ADIPOQ |
| upreg ulated | GO:00 06935 | chemotaxis | 139 | 154 62 | 601 | 16 | 9.99 E-05 | 0.00 576 639 | NR4A3, FPR3, NR4A1, DLX5, GFRA1, CDH13, SOS1, SEMA5A, CCR1, PTPRC, TNFRSF11A, ITGB3, CXCL5, IL6, CXCL9, CNTN1 |
| upreg ulated | GO:00 42330 | taxis | 139 | 154 62 | 601 | 16 | 9.99 E-05 | 0.00 576 639 | NR4A3, FPR3, NR4A1, DLX5, GFRA1, CDH13, SOS1, SEMA5A, CCR1, PTPRC, TNFRSF11A, ITGB3, CXCL5, IL6, CXCL9, CNTN1 |
| upreg ulated | GO:00 70887 | cellular response to chemical stimulus | 139 | 154 62 | 205 0 | 35 | 0.00 010 704 4 | 0.00 606 114 1 | IRF4, FABP4, ADRA2A, NPTX1, IL18R1, RASGRF2, CAMK2D, COL1A2, ITGB3, RGCC, ADH1B, CXCL9, FOXO1, SOS1, SEMA5A, PLA2R1, FOSL2, ALOX5AP, CD36, SATB2, CALB1, LRRK2, NR4A1, DLX5, CDH13, PDE1A, CCR1, BMP2, PTPRC, TNFRSF11A, WNT4, CXCL5, IL6, NAMPT, ADIPOQ |
| upreg ulated | GO:00 00904 | cell morphogenesis involved in differentiation | 139 | 154 62 | 741 | 18 | 0.00 011 544 2 | 0.00 637 929 1 | NR4A3, DCLK1, SOS1, SEMA5A, SHOX2, PDLIM5, LRRK2, GFRA1, DLX5, NPTX1, SDC2, PTPRC, BMP2, RGCC, ITGB3, WNT4, TBX5, CNTN1 |
| upreg | GO:00 | anatomical | 139 | 154 | 205 | 35 | 0.00 | 0.00 | DCLK1, SHOX2, THSD7A, HOXA5, |

| | | | | | | | | | |
|---|---|---|---|---|---|---|---|---|---|
| ulated | 09653 | structure morphogenesis | | 62 | 9 | | 011 691 5 | 637 929 1 | PDLIM5, NKD1, NPTX1, GFRA1, COL1A2, ITGB3, RGCC, ERCC1, CNTN1, NR4A3, SOS1, SEMA5A, TENM3, FEM1B, SATB2, CALB1, ELN, LRRK2, CRISPLD2, NR4A1, DLX5, CDH13, GCNT4, SDC2, ANGPTL4, BMP2, PTPRC, WNT4, IL6, TBX5, ADIPOQ |
| upreg ulated | GO:00 07275 | multicellular organismal development | 139 | 154 62 | 418 0 | 58 | 0.00 011 992 4 | 0.00 642 664 6 | DCLK1, SHOX2, IRF4, SPATA9, CD2, COL12A1, HOXA5, THSD7A, PDLIM5, LSAMP, NPAS2, NKD1, GDF6, GFRA1, NPTX1, IL18R1, COL1A2, HIVEP3, ITGB3, RGCC, PRDM6, BNC2, ERCC1, ASIC2, BCHE, FOXO1, CNTN1, NR4A3, PRELP, TENM3, SEMA5A, SOS1, CHRNA1, EFEMP1, FEM1B, CALB1, SATB2, CRISPLD2, LRRK2, ELN, NR4A1, PRKG1, DLX5, MEGF10, CDH13, GCNT4, SDC2, ANGPTL4, CCR1, BMP2, PTPRC, TNFRSF11A, WNT4, IL6, TBX5, NAMPT, SLC7A5, ADIPOQ |
| upreg ulated | GO:00 01936 | regulation of endothelial cell proliferation | 139 | 154 62 | 86 | 6 | 0.00 012 345 9 | 0.00 649 998 4 | RGCC, ITGB3, NR4A1, CDH13, SEMA5A, BMP2 |
| upreg ulated | GO:00 48856 | anatomical structure development | 139 | 154 62 | 419 3 | 58 | 0.00 013 143 | 0.00 680 036 9 | DCLK1, SHOX2, IRF4, CD2, COL12A1, HOXA5, THSD7A, PDLIM5, LSAMP, NPAS2, NKD1, GDF6, GFRA1, NPTX1, IL18R1, COL1A2, HIVEP3, ITGB3, RGCC, PRDM6, BNC2, ERCC1, ASIC2, BCHE, FOXO1, CNTN1, NR4A3, PRELP, TENM3, SEMA5A, SOS1, CHRNA1, EFEMP1, FEM1B, CALB1, SATB2, CRISPLD2, LRRK2, ELN, NR4A1, PRKG1, DLX5, MEGF10, CDH13, GCNT4, SDC2, ANGPTL4, CCR1, BMP2, PTPRC, TNFRSF11A, FBXO40, WNT4, IL6, TBX5, NAMPT, SLC7A5, ADIPOQ |
| upreg ulated | GO:00 06952 | defense response | 139 | 154 62 | 134 2 | 26 | 0.00 014 213 1 | 0.00 722 940 9 | SLAMF7, TLR1, IRF4, SOS1, FABP4, CLEC1A, ADRA2A, IGJ, NFATC2, BNIP3L, ALOX5AP, CD36, BIRC3, NR4A1, PDE1A, AOX1, CCR1, CAMK2D, PTPRC, BMP2, TNFRSF11A, LCP2, IL6, CXCL9, ADIPOQ, FOXO1 |
| upreg ulated | GO:00 61061 | muscle structure development | 139 | 154 62 | 442 | 13 | 0.00 017 687 4 | 0.00 875 784 5 | ELN, NKD1, NR4A1, MEGF10, CHRNA1, SHOX2, BMP2, HIVEP3, FBXO40, WNT4, PRDM6, IL6, TBX5 |
| upreg ulated | GO:00 50793 | regulation of developmental process | 139 | 154 62 | 152 0 | 28 | 0.00 017 801 7 | 0.00 875 784 5 | IRF4, SHOX2, TENM3, SEMA5A, EFEMP1, CD2, HOXA5, PDLIM5, CD36, LRRK2, NKD1, GDF6, DLX5, MEGF10, SDC2, ANGPTL4, CCR1, BMP2, ITGB3, RGCC, WNT4, PRDM6, IL6, TBX5, ASIC2, CNTN1, FOXO1, ADIPOQ |
| upreg ulated | GO:00 09893 | positive regulation of metabolic process | 139 | 154 62 | 229 1 | 37 | 0.00 020 341 3 | 0.00 975 042 6 | FAM129A, IRF4, SHOX2, ADRA2A, TNNI2, HOXA5, IGJ, NFATC2, NPAS2, NKD1, GDF6, HIVEP3, ITGB3, RGCC, CXCL9, FOXO1, CNTN1, NR4A3, TLR1, MDFIC, CD36, SATB2, LRRK2, BIRC3, NR4A1, DLX5, CDH13, BMP2, CCR1, GUCY1A3, PTPRC, TNFRSF11A, WNT4, IL6, TBX5, NAMPT, ADIPOQ |
| upreg ulated | GO:00 07167 | enzyme linked receptor protein signaling pathway | 139 | 154 62 | 918 | 20 | 0.00 020 469 1 | 0.00 975 042 6 | CBLC, SOS1, ADRA2A, EFEMP1, STAP1, NR4A1, GDF6, GFRA1, DLX5, CDH13, PDE1A, RASGRF2, COL1A2, BMP2, ITGB3, WNT4, LCP2, NAMPT, FOXO1, ADIPOQ |
| upreg ulated | GO:00 07166 | cell surface receptor signaling pathway | 139 | 154 62 | 301 8 | 45 | 0.00 021 445 2 | 0.01 005 578 3 | P2RY8, CBLC, IRF4, SHOX2, ADRA2A, CD2, NFATC2, NKD1, GDF6, GRP, GFRA1, IL18R1, RASGRF2, COL1A2, FYB, CAMK2D, ITGB3, LCP2, CXCL9, CNTN1, FOXO1, FPR3, TSPAN5, SOS1, CLEC1A, EFEMP1, MDFIC, FEM1B, |

| | | | | | | | | | |
|---|---|---|---|---|---|---|---|---|---|
| | | | | | | | | | CD36, LRRK2, STAP1, BIRC3, NR4A1, GPR34, DLX5, CDH13, PDE1A, PTPRC, BMP2, CCR1, TNFRSF11A, WNT4, IL6, NAMPT, ADIPOQ |
| upreg ulated | GO:00 32844 | regulation of homeostatic process | 139 | 154 62 | 281 | 10 | 0.00 022 734 4 | 0.01 049 630 3 | LRRK2, PTPRC, CAMK2D, ADRA2A, ITGB3, TNFRSF11A, HOXA5, ERCC1, CXCL9, FOXO1 |
| upreg ulated | GO:00 50679 | positive regulation of epithelial cell proliferation | 139 | 154 62 | 137 | 7 | 0.00 023 743 9 | 0.01 056 077 2 | ITGB3, IL6, NR4A1, DLX5, CDH13, SEMA5A, BMP2 |
| upreg ulated | GO:00 45715 | negative regulation of low-density lipoprotein particle receptor biosynthetic process | 139 | 154 62 | 3 | 2 | 0.00 023 929 8 | 0.01 056 077 2 | ITGB3, ADIPOQ |
| upreg ulated | GO:00 71404 | cellular response to low-density lipoprotein particle stimulus | 139 | 154 62 | 3 | 2 | 0.00 023 929 8 | 0.01 056 077 2 | CDH13, CD36 |
| upreg ulated | GO:00 01938 | positive regulation of endothelial cell proliferation | 139 | 154 62 | 63 | 5 | 0.00 025 265 1 | 0.01 098 848 8 | ITGB3, NR4A1, CDH13, SEMA5A, BMP2 |
| upreg ulated | GO:00 01816 | cytokine production | 139 | 154 62 | 522 | 14 | 0.00 025 743 5 | 0.01 103 659 3 | BIRC3, TLR1, IL18R1, IRF4, FABP4, RGCC, ADRA2A, CD2, LCP2, IL6, PLA2R1, NFATC2, CD36, ADIPOQ |
| upreg ulated | GO:00 50678 | regulation of epithelial cell proliferation | 139 | 154 62 | 234 | 9 | 0.00 026 609 3 | 0.01 124 712 8 | NR4A1, DLX5, CDH13, SEMA5A, BMP2, ITGB3, RGCC, IL6, HOXA5 |
| upreg ulated | GO:00 01935 | endothelial cell proliferation | 139 | 154 62 | 100 | 6 | 0.00 028 248 3 | 0.01 167 291 6 | RGCC, ITGB3, NR4A1, CDH13, SEMA5A, BMP2 |
| upreg ulated | GO:00 22008 | neurogenesis | 139 | 154 62 | 124 4 | 24 | 0.00 028 394 6 | 0.01 167 291 6 | NR4A3, SHOX2, TENM3, DCLK1, SOS1, SEMA5A, PDLIM5, SATB2, LRRK2, NKD1, GDF6, PRKG1, NPTX1, DLX5, GFRA1, SDC2, PTPRC, BMP2, ITGB3, WNT4, PRDM6, IL6, BCHE, CNTN1 |
| upreg ulated | GO:00 48699 | generation of neurons | 139 | 154 62 | 118 0 | 23 | 0.00 033 421 8 | 0.01 355 388 6 | NR4A3, SHOX2, TENM3, DCLK1, SOS1, SEMA5A, PDLIM5, SATB2, LRRK2, NKD1, GDF6, PRKG1, NPTX1, DLX5, GFRA1, SDC2, PTPRC, BMP2, ITGB3, WNT4, IL6, BCHE, CNTN1 |
| upreg ulated | GO:00 06024 | glycosaminoglyc an biosynthetic process | 139 | 154 62 | 104 | 6 | 0.00 034 919 5 | 0.01 397 243 8 | GPC6, CHSY3, PRELP, SDC2, GALNT5, HS3ST3B1 |
| upreg ulated | GO:00 06023 | aminoglycan biosynthetic process | 139 | 154 62 | 105 | 6 | 0.00 036 765 6 | 0.01 451 757 3 | GPC6, CHSY3, PRELP, SDC2, GALNT5, HS3ST3B1 |
| upreg ulated | GO:00 40012 | regulation of locomotion | 139 | 154 62 | 542 | 14 | 0.00 037 647 4 | 0.01 467 272 | LRRK2, NKD1, CDH13, SEMA5A, CCR1, BMP2, PTPRC, RGCC, ITGB3, ADRA2A, WNT4, IL6, TBX5, ADIPOQ |
| upreg ulated | GO:00 45444 | fat cell differentiation | 139 | 154 62 | 149 | 7 | 0.00 039 595 9 | 0.01 523 426 8 | IL6, STEAP4, RUNX1T1, ADIPOQ, BMP2, FOXO1, FABP4 |
| upreg ulated | GO:00 31175 | neuron projection development | 139 | 154 62 | 754 | 17 | 0.00 043 125 4 | 0.01 638 219 1 | NR4A3, TENM3, DCLK1, SOS1, SEMA5A, SHOX2, PDLIM5, LRRK2, PRKG1, GFRA1, DLX5, NPTX1, SDC2, PTPRC, ITGB3, IL6, CNTN1 |
| upreg ulated | GO:20 00026 | regulation of multicellular | 139 | 154 62 | 120 4 | 23 | 0.00 044 | 0.01 667 | IRF4, SHOX2, TENM3, SEMA5A, EFEMP1, CD2, HOXA5, PDLIM5, LRRK2, |

| | | | | | | | | | |
|---|---|---|---|---|---|---|---|---|---|
| | | organismal development | | | | | 444 6 | 226 9 | NKD1, GDF6, MEGF10, SDC2, ANGPTL4, CCR1, BMP2, RGCC, WNT4, IL6, TBX5, ASIC2, ADIPOQ, CNTN1 |
| upreg ulated | GO:00 71402 | cellular response to lipoprotein particle stimulus | 139 | 154 62 | 4 | 2 | 0.00 047 577 | 0.01 762 698 6 | CDH13, CD36 |
| upreg ulated | GO:00 32963 | collagen metabolic process | 139 | 154 62 | 111 | 6 | 0.00 049 504 5 | 0.01 811 743 | RGCC, WNT4, COL12A1, IL6, COL1A2, MMP1 |
| upreg ulated | GO:20 00145 | regulation of cell motility | 139 | 154 62 | 493 | 13 | 0.00 050 483 9 | 0.01 825 326 4 | NKD1, CDH13, SEMA5A, CCR1, BMP2, PTPRC, RGCC, ADRA2A, ITGB3, WNT4, IL6, TBX5, ADIPOQ |
| upreg ulated | GO:00 10869 | regulation of receptor biosynthetic process | 139 | 154 62 | 18 | 3 | 0.00 052 551 6 | 0.01 845 726 3 | ITGB3, HOXA5, ADIPOQ |
| upreg ulated | GO:00 48468 | cell development | 139 | 154 62 | 162 4 | 28 | 0.00 052 737 9 | 0.01 845 726 3 | NR4A3, SHOX2, TENM3, DCLK1, SOS1, SEMA5A, HOXA5, FEM1B, PDLIM5, SATB2, LRRK2, GDF6, PRKG1, NPTX1, DLX5, GFRA1, MEGF10, SDC2, BMP2, PTPRC, ITGB3, RGCC, WNT4, IL6, TBX5, ERCC1, CNTN1, ADIPOQ |
| upreg ulated | GO:00 44763 | single-organism cellular process | 139 | 154 62 | 108 71 | 11 5 | 0.00 053 435 2 | 0.01 845 726 3 | TAGAP, P2RY8, SHOX2, IRF4, SPATA9, FABP4, HS3ST3B1, RAB30, BNIP3L, NPAS2, MFAP5, GRP, NPTX1, PNMA2, AOX1, COL1A2, LCP2, ASIC2, BCHE, FOXO1, ARHGAP42, CNTN1, PAPPA, TSPAN5, CRLS1, TLR1, PRELP, STEAP4, TENM3, CHRNA1, SOS1, MDFIC, PLA2R1, FEM1B, ALOX5AP, FOSL2, PIEZO2, CD36, MMP1, SATB2, GPR34, PRKG1, PDE1A, GUCY1A3, TNFRSF11A, GALNT14, CHSY3, IL6, CBLN4, TBX5, SLC7A5, GPC6, CBLC, DCLK1, CD2, ADRA2A, COL12A1, THSD7A, HOXA5, NFATC2, PDLIM5, NKD1, GDF6, GLRX, GFRA1, RASGRF2, GALNT5, IL18R1, DPP4, DEPDC7, CAMK2D, HIVEP3, FYB, RGCC, ITGB3, PRDM6, ERCC1, CXCL9, SLC22A3, NR4A3, FPR3, SLAMF7, SEMA5A, CLEC1A, ADAMTS5, RUNX1T1, EFEMP1, PPP1R3B, MCTP1, DOCK8, CALB1, CRISPLD2, ELN, LRRK2, BIRC3, STAP1, NR4A1, DLX5, CDH13, GCNT4, SDC2, MEGF10, PLIN1, ANGPTL4, BMP2, PTPRC, CCR1, FBXO40, WNT4, CXCL5, SLC24A3, NAMPT, SKAP2, TDO2, ADIPOQ |
| upreg ulated | GO:00 22407 | regulation of cell-cell adhesion | 139 | 154 62 | 74 | 5 | 0.00 053 508 2 | 0.01 845 726 3 | RGCC, WNT4, DPP4, ADIPOQ, BMP2 |
| upreg ulated | GO:00 80134 | regulation of response to stress | 139 | 154 62 | 842 | 18 | 0.00 054 599 8 | 0.01 861 976 1 | TLR1, IRF4, FABP4, ADRA2A, PLA2R1, MDFIC, FEM1B, ALOX5AP, CD36, NPAS2, BIRC3, BMP2, TNFRSF11A, WNT4, IL6, ASIC2, FOXO1, ADIPOQ |
| upreg ulated | GO:00 48513 | organ development | 139 | 154 62 | 259 7 | 39 | 0.00 058 612 6 | 0.01 976 364 | IRF4, DCLK1, SHOX2, CD2, HOXA5, NKD1, IL18R1, COL1A2, HIVEP3, RGCC, BNC2, ERCC1, FOXO1, CNTN1, NR4A3, CHRNA1, SEMA5A, TENM3, EFEMP1, FEM1B, SATB2, CALB1, ELN, LRRK2, CRISPLD2, NR4A1, PRKG1, DLX5, MEGF10, GCNT4, CCR1, BMP2, PTPRC, TNFRSF11A, WNT4, IL6, TBX5, NAMPT, ADIPOQ |
| upreg ulated | GO:00 09888 | tissue development | 139 | 154 62 | 147 4 | 26 | 0.00 061 424 | 0.02 048 149 | NR4A3, CHRNA1, SHOX2, SEMA5A, EFEMP1, HOXA5, FEM1B, CALB1, SATB2, ELN, NKD1, NR4A1, DLX5, |

| | | | | | | | | | |
|---|---|---|---|---|---|---|---|---|---|
| | | | | | | | | | MEGF10, GCNT4, CCR1, BMP2, HIVEP3, PTPRC, RGCC, WNT4, IL6, BNC2, TBX5, NAMPT, ADIPOQ |
| upreg ulated | GO:00 48667 | cell morphogenesis involved in neuron differentiation | 139 | 154 62 | 572 | 14 | 0.00 064 211 4 | 0.02 054 854 6 | NR4A3, LRRK2, NPTX1, DLX5, GFRA1, SDC2, DCLK1, SOS1, SEMA5A, SHOX2, PTPRC, ITGB3, PDLIM5, CNTN1 |
| upreg ulated | GO:00 22603 | regulation of anatomical structure morphogenesis | 139 | 154 62 | 572 | 14 | 0.00 064 211 4 | 0.02 054 854 6 | LRRK2, NKD1, SDC2, ANGPTL4, SEMA5A, SHOX2, BMP2, RGCC, WNT4, IL6, HOXA5, TBX5, PDLIM5, ADIPOQ |
| upreg ulated | GO:19 03034 | regulation of response to wounding | 139 | 154 62 | 321 | 10 | 0.00 064 846 5 | 0.02 054 854 6 | BIRC3, FABP4, TNFRSF11A, ADRA2A, WNT4, IL6, ASIC2, ALOX5AP, CD36, ADIPOQ |
| upreg ulated | GO:00 44259 | multicellular organismal macromolecule metabolic process | 139 | 154 62 | 117 | 6 | 0.00 065 444 8 | 0.02 054 854 6 | RGCC, WNT4, COL12A1, IL6, COL1A2, MMP1 |
| upreg ulated | GO:00 31325 | positive regulation of cellular metabolic process | 139 | 154 62 | 216 3 | 34 | 0.00 065 649 3 | 0.02 054 854 6 | FAM129A, IRF4, SHOX2, ADRA2A, TNNI2, HOXA5, NFATC2, NPAS2, GDF6, HIVEP3, ITGB3, RGCC, CXCL9, FOXO1, NR4A3, TLR1, MDFIC, CD36, SATB2, LRRK2, BIRC3, NR4A1, DLX5, CDH13, CCR1, BMP2, GUCY1A3, PTPRC, TNFRSF11A, WNT4, IL6, TBX5, NAMPT, ADIPOQ |
| upreg ulated | GO:00 07517 | muscle organ development | 139 | 154 62 | 265 | 9 | 0.00 065 733 4 | 0.02 054 854 6 | ELN, NR4A1, MEGF10, CHRNA1, SHOX2, BMP2, HIVEP3, IL6, TBX5 |
| upreg ulated | GO:00 30182 | neuron differentiation | 139 | 154 62 | 108 3 | 21 | 0.00 066 732 5 | 0.02 064 579 2 | NR4A3, SHOX2, TENM3, DCLK1, SOS1, SEMA5A, PDLIM5, LRRK2, NKD1, GDF6, PRKG1, NPTX1, GFRA1, DLX5, SDC2, PTPRC, BMP2, ITGB3, WNT4, IL6, CNTN1 |
| upreg ulated | GO:00 48634 | regulation of muscle organ development | 139 | 154 62 | 78 | 5 | 0.00 068 142 9 | 0.02 086 703 6 | IL6, TBX5, MEGF10, SHOX2, BMP2 |
| upreg ulated | GO:00 60993 | kidney morphogenesis | 139 | 154 62 | 45 | 4 | 0.00 070 017 4 | 0.02 122 445 6 | WNT4, LRRK2, GCNT4, CALB1 |
| upreg ulated | GO:00 45321 | leukocyte activation | 139 | 154 62 | 578 | 14 | 0.00 071 100 7 | 0.02 128 291 1 | SLAMF7, TLR1, IL18R1, IRF4, DPP4, PTPRC, CD2, WNT4, LCP2, IL6, ERCC1, NFATC2, SKAP2, DOCK8 |
| upreg ulated | GO:00 08284 | positive regulation of cell proliferation | 139 | 154 62 | 717 | 16 | 0.00 071 628 6 | 0.02 128 291 1 | NR4A1, DLX5, CDH13, DPP4, SEMA5A, SHOX2, BMP2, PTPRC, TNFRSF11A, ITGB3, ADRA2A, CXCL5, IL6, NAMPT, NFATC2, FOSL2 |
| upreg ulated | GO:00 10604 | positive regulation of macromolecule metabolic process | 139 | 154 62 | 208 7 | 33 | 0.00 072 599 4 | 0.02 135 989 6 | FAM129A, IRF4, SHOX2, ADRA2A, TNNI2, HOXA5, NFATC2, NPAS2, NKD1, GDF6, HIVEP3, ITGB3, RGCC, FOXO1, CNTN1, NR4A3, TLR1, MDFIC, CD36, SATB2, LRRK2, BIRC3, NR4A1, DLX5, CDH13, BMP2, PTPRC, TNFRSF11A, WNT4, IL6, TBX5, NAMPT, ADIPOQ |
| upreg ulated | GO:00 32103 | positive regulation of response to external stimulus | 139 | 154 62 | 167 | 7 | 0.00 078 196 7 | 0.02 170 946 9 | TNFRSF11A, IL6, CDH13, ALOX5AP, SEMA5A, CCR1, FABP4 |
| upreg ulated | GO:00 48812 | neuron projection morphogenesis | 139 | 154 62 | 584 | 14 | 0.00 078 607 8 | 0.02 170 946 9 | NR4A3, LRRK2, NPTX1, DLX5, GFRA1, SDC2, DCLK1, SOS1, SEMA5A, SHOX2, PTPRC, ITGB3, PDLIM5, CNTN1 |
| upreg ulated | GO:20 00064 | regulation of cortisol | 139 | 154 62 | 5 | 2 | 0.00 078 | 0.02 170 | WNT4, BMP2 |

| | | | | | | | | | |
|---|---|---|---|---|---|---|---|---|---|
| | | biosynthetic process | | | | | 827 2 | 946 9 | |
| upreg ulated | GO:00 71221 | cellular response to bacterial lipopeptide | 139 | 154 62 | 5 | 2 | 0.00 078 827 2 | 0.02 170 946 9 | TLR1, CD36 |
| upreg ulated | GO:00 71220 | cellular response to bacterial lipoprotein | 139 | 154 62 | 5 | 2 | 0.00 078 827 2 | 0.02 170 946 9 | TLR1, CD36 |
| upreg ulated | GO:00 70339 | response to bacterial lipopeptide | 139 | 154 62 | 5 | 2 | 0.00 078 827 2 | 0.02 170 946 9 | TLR1, CD36 |
| upreg ulated | GO:00 50900 | leukocyte migration | 139 | 154 62 | 272 | 9 | 0.00 079 130 5 | 0.02 170 946 9 | SOS1, CCR1, COL1A2, CD2, ITGB3, TNFRSF11A, IL6, SLC7A5, MMP1 |
| upreg ulated | GO:00 51270 | regulation of cellular component movement | 139 | 154 62 | 518 | 13 | 0.00 079 970 3 | 0.02 170 946 9 | NKD1, CDH13, SEMA5A, CCR1, BMP2, PTPRC, RGCC, ADRA2A, ITGB3, WNT4, IL6, TBX5, ADIPOQ |
| upreg ulated | GO:00 09966 | regulation of signal transduction | 139 | 154 62 | 209 9 | 33 | 0.00 080 298 3 | 0.02 170 946 9 | TAGAP, CBLC, IRF4, SHOX2, ADRA2A, NKD1, GDF6, IL18R1, RASGRF2, DEPDC7, ITGB3, CXCL9, FOXO1, TSPAN5, SOS1, SEMA5A, MDFIC, PLA2R1, FEM1B, CD36, LRRK2, STAP1, BIRC3, DLX5, CDH13, BMP2, CCR1, PTPRC, TNFRSF11A, WNT4, IL6, SKAP2, ADIPOQ |
| upreg ulated | GO:00 22617 | extracellular matrix disassembly | 139 | 154 62 | 122 | 6 | 0.00 081 546 4 | 0.02 185 005 4 | ELN, COL12A1, DPP4, COL1A2, ADAMTS5, MMP1 |
| upreg ulated | GO:00 42592 | homeostatic process | 139 | 154 62 | 118 0 | 22 | 0.00 082 963 1 | 0.02 203 294 2 | STEAP4, CHRNA1, FABP4, ADRA2A, HOXA5, IGJ, CALB1, LRRK2, GLRX, NPTX1, GCNT4, ANGPTL4, CCR1, CAMK2D, PTPRC, TNFRSF11A, ITGB3, IL6, ERCC1, CXCL9, ADIPOQ, FOXO1 |
| upreg ulated | GO:00 02675 | positive regulation of acute inflammatory response | 139 | 154 62 | 21 | 3 | 0.00 083 980 3 | 0.02 210 745 2 | TNFRSF11A, IL6, ALOX5AP |
| upreg ulated | GO:00 43542 | endothelial cell migration | 139 | 154 62 | 123 | 6 | 0.00 085 104 2 | 0.02 220 33 | RGCC, ITGB3, NR4A1, CDH13, DPP4, SEMA5A |
| upreg ulated | GO:00 01568 | blood vessel development | 139 | 154 62 | 522 | 13 | 0.00 085 824 2 | 0.02 220 33 | NR4A1, CDH13, ANGPTL4, SEMA5A, COL1A2, RGCC, ITGB3, WNT4, IL6, HOXA5, TBX5, THSD7A, FOXO1 |
| upreg ulated | GO:00 07155 | cell adhesion | 139 | 154 62 | 957 | 19 | 0.00 093 019 4 | 0.02 385 907 1 | SLAMF7, TENM3, SEMA5A, CD2, COL12A1, LSAMP, CD36, CDH13, MEGF10, DPP4, CCR1, BMP2, RGCC, ITGB3, WNT4, CPXM2, SIGLEC10, CNTN1, ADIPOQ |
| upreg ulated | GO:00 22610 | biological adhesion | 139 | 154 62 | 959 | 19 | 0.00 095 350 5 | 0.02 424 974 5 | SLAMF7, TENM3, SEMA5A, CD2, COL12A1, LSAMP, CD36, CDH13, MEGF10, DPP4, CCR1, BMP2, RGCC, ITGB3, WNT4, CPXM2, SIGLEC10, CNTN1, ADIPOQ |
| upreg ulated | GO:00 32800 | receptor biosynthetic process | 139 | 154 62 | 22 | 3 | 0.00 096 603 4 | 0.02 436 190 8 | ITGB3, HOXA5, ADIPOQ |
| upreg ulated | GO:00 50673 | epithelial cell proliferation | 139 | 154 62 | 281 | 9 | 0.00 099 547 1 | 0.02 489 506 5 | NR4A1, DLX5, CDH13, SEMA5A, BMP2, ITGB3, RGCC, IL6, HOXA5 |
| upreg | GO:00 | negative | 139 | 154 | 600 | 14 | 0.00 | 0.02 | NKD1, SEMA5A, CCR1, BMP2, RGCC, |

| | | | | | | | | | |
|---|---|---|---|---|---|---|---|---|---|
| ulated | 51093 | regulation of developmental process | | 62 | | | 101 985 5 | 529 410 1 | ITGB3, EFEMP1, WNT4, PRDM6, IL6, HOXA5, TBX5, ADIPOQ, FOXO1 |
| upreg ulated | GO:00 07173 | epidermal growth factor receptor signaling pathway | 139 | 154 62 | 227 | 8 | 0.00 103 415 | 0.02 543 840 3 | NR4A1, CBLC, CDH13, PDE1A, SOS1, EFEMP1, ADRA2A, FOXO1 |
| upreg ulated | GO:00 07267 | cell-cell signaling | 139 | 154 62 | 112 2 | 21 | 0.00 104 877 1 | 0.02 558 830 1 | CHRNA1, SEMA5A, ADRA2A, HOXA5, PDLIM5, CALB1, LRRK2, NPTX1, RASGRF2, CAMK2D, BMP2, CCR1, TNFRSF11A, CXCL5, IL6, TBX5, NAMPT, ASIC2, CXCL9, BCHE, ADIPOQ |
| upreg ulated | GO:00 30334 | regulation of cell migration | 139 | 154 62 | 469 | 12 | 0.00 107 965 6 | 0.02 612 941 6 | CDH13, SEMA5A, CCR1, BMP2, PTPRC, RGCC, ADRA2A, ITGB3, WNT4, IL6, TBX5, ADIPOQ |
| upreg ulated | GO:19 02533 | positive regulation of intracellular signal transduction | 139 | 154 62 | 605 | 14 | 0.00 110 396 9 | 0.02 650 408 7 | LRRK2, CDH13, SOS1, SEMA5A, CCR1, BMP2, TNFRSF11A, ADRA2A, IL6, PLA2R1, MDFIC, CXCL9, CD36, ADIPOQ |
| upreg ulated | GO:00 44236 | multicellular organismal metabolic process | 139 | 154 62 | 130 | 6 | 0.00 113 480 7 | 0.02 676 064 6 | RGCC, WNT4, COL12A1, IL6, MMP1, COL1A2 |
| upreg ulated | GO:00 06955 | immune response | 139 | 154 62 | 129 2 | 23 | 0.00 116 892 4 | 0.02 676 064 6 | SLAMF7, TLR1, IRF4, SOS1, IGJ, NFATC2, CD36, BIRC3, NR4A1, PDE1A, IL18R1, CCR1, FYB, CAMK2D, PTPRC, TNFRSF11A, RGCC, CXCL5, LCP2, IL6, ERCC1, CXCL9, FOXO1 |
| upreg ulated | GO:00 55098 | response to low-density lipoprotein particle | 139 | 154 62 | 6 | 2 | 0.00 117 543 7 | 0.02 676 064 6 | CDH13, CD36 |
| upreg ulated | GO:00 32493 | response to bacterial lipoprotein | 139 | 154 62 | 6 | 2 | 0.00 117 543 7 | 0.02 676 064 6 | TLR1, CD36 |
| upreg ulated | GO:00 32347 | regulation of aldosterone biosynthetic process | 139 | 154 62 | 6 | 2 | 0.00 117 543 7 | 0.02 676 064 6 | WNT4, BMP2 |
| upreg ulated | GO:00 10871 | negative regulation of receptor biosynthetic process | 139 | 154 62 | 6 | 2 | 0.00 117 543 7 | 0.02 676 064 6 | ITGB3, ADIPOQ |
| upreg ulated | GO:00 10628 | positive regulation of gene expression | 139 | 154 62 | 121 2 | 22 | 0.00 117 707 6 | 0.02 676 064 6 | NR4A3, IRF4, SHOX2, TNNI2, MDFIC, HOXA5, NFATC2, SATB2, NPAS2, NR4A1, GDF6, DLX5, CDH13, HIVEP3, BMP2, RGCC, WNT4, IL6, TBX5, NAMPT, FOXO1, CNTN1 |
| upreg ulated | GO:00 38127 | ERBB signaling pathway | 139 | 154 62 | 232 | 8 | 0.00 118 914 1 | 0.02 683 168 | NR4A1, CBLC, CDH13, PDE1A, SOS1, EFEMP1, ADRA2A, FOXO1 |
| upreg ulated | GO:00 50789 | regulation of biological process | 139 | 154 62 | 928 3 | 10 1 | 0.00 123 166 4 | 0.02 742 562 1 | TAGAP, FAM129A, P2RY8, SHOX2, IRF4, FABP4, TNNI2, RAB30, BNIP3L, TFEC, NPAS2, GRP, PNMA2, COL1A2, LCP2, ASIC2, BCHE, FOXO1, ARHGAP42, CNTN1, TSPAN5, TLR1, TENM3, CHRNA1, SOS1, MDFIC, PLA2R1, FEM1B, ALOX5AP, FOSL2, CD36, SATB2, ZFHX4, GPR34, PRKG1, PDE1A, GUCY1A3, TNFRSF11A, IL6, TBX5, GPC6, CBLC, DCLK1, CD2, ADRA2A, HOXA5, IGJ, NFATC2, PDLIM5, NKD1, GDF6, CELF2, GLRX, GFRA1, RASGRF2, IL18R1, DPP4, DEPDC7, DHX34, CAMK2D, HIVEP3, FYB, RGCC, ITGB3, PRDM6, BNC2, ERCC1, TFPI2, |

| | | | | | | | | | |
|---|---|---|---|---|---|---|---|---|---|
| | | | | | | | | | CXCL9, NR4A3, FPR3, SLAMF7, SEMA5A, RUNX1T1, RFX2, CLEC1A, EFEMP1, PPP1R3B, MCTP1, DOCK8, CALB1, ELN, FBXO32, LRRK2, BIRC3, STAP1, NR4A1, DLX5, CDH13, SDC2, MEGF10, ANGPTL4, PTPRC, CCR1, BMP2, WNT4, CXCL5, RARRES1, NAMPT, SKAP2, ADIPOQ |
| upreg ulated | GO:00 10646 | regulation of cell communication | 139 | 154 62 | 233 3 | 35 | 0.00 125 157 6 | 0.02 742 562 1 | TAGAP, CBLC, IRF4, SHOX2, ADRA2A, NKD1, GDF6, IL18R1, RASGRF2, DEPDC7, ITGB3, CXCL9, BCHE, FOXO1, TSPAN5, SOS1, SEMA5A, MDFIC, PLA2R1, FEM1B, CD36, CALB1, LRRK2, STAP1, BIRC3, DLX5, CDH13, BMP2, CCR1, PTPRC, TNFRSF11A, WNT4, IL6, SKAP2, ADIPOQ |
| upreg ulated | GO:00 03170 | heart valve development | 139 | 154 62 | 24 | 3 | 0.00 125 307 4 | 0.02 742 562 1 | TBX5, SHOX2, BMP2 |
| upreg ulated | GO:00 10769 | regulation of cell morphogenesis involved in differentiation | 139 | 154 62 | 234 | 8 | 0.00 125 610 2 | 0.02 742 562 1 | LRRK2, SDC2, SHOX2, SEMA5A, BMP2, RGCC, TBX5, PDLIM5 |
| upreg ulated | GO:00 23051 | regulation of signaling | 139 | 154 62 | 233 4 | 35 | 0.00 126 115 8 | 0.02 742 562 1 | TAGAP, CBLC, IRF4, SHOX2, ADRA2A, NKD1, GDF6, IL18R1, RASGRF2, DEPDC7, ITGB3, CXCL9, BCHE, FOXO1, TSPAN5, SOS1, SEMA5A, MDFIC, PLA2R1, FEM1B, CD36, CALB1, LRRK2, STAP1, BIRC3, DLX5, CDH13, BMP2, CCR1, PTPRC, TNFRSF11A, WNT4, IL6, SKAP2, ADIPOQ |
| upreg ulated | GO:00 71310 | cellular response to organic substance | 139 | 154 62 | 163 4 | 27 | 0.00 128 323 | 0.02 770 483 4 | IRF4, SOS1, ADRA2A, FOSL2, CD36, CALB1, SATB2, LRRK2, NR4A1, NPTX1, DLX5, RASGRF2, PDE1A, CDH13, IL18R1, CCR1, COL1A2, CAMK2D, PTPRC, BMP2, TNFRSF11A, ITGB3, WNT4, IL6, NAMPT, ADIPOQ, FOXO1 |
| upreg ulated | GO:00 06029 | proteoglycan metabolic process | 139 | 154 62 | 90 | 5 | 0.00 130 188 4 | 0.02 790 680 5 | GPC6, CHSY3, SDC2, BMP2, HS3ST3B1 |
| upreg ulated | GO:00 01944 | vasculature development | 139 | 154 62 | 548 | 13 | 0.00 133 353 8 | 0.02 838 260 6 | NR4A1, CDH13, ANGPTL4, SEMA5A, COL1A2, RGCC, ITGB3, WNT4, IL6, HOXA5, TBX5, THSD7A, FOXO1 |
| upreg ulated | GO:00 10557 | positive regulation of macromolecule biosynthetic process | 139 | 154 62 | 130 6 | 23 | 0.00 134 898 3 | 0.02 850 913 6 | FAM129A, NR4A3, TLR1, IRF4, SHOX2, TNNI2, MDFIC, HOXA5, NFATC2, SATB2, NPAS2, NR4A1, GDF6, DLX5, CDH13, HIVEP3, BMP2, RGCC, WNT4, IL6, TBX5, NAMPT, FOXO1 |
| upreg ulated | GO:00 32101 | regulation of response to external stimulus | 139 | 154 62 | 482 | 12 | 0.00 136 288 8 | 0.02 860 159 1 | BIRC3, CDH13, SEMA5A, CCR1, FABP4, TNFRSF11A, WNT4, IL6, ASIC2, ALOX5AP, CD36, ADIPOQ |
| upreg ulated | GO:00 60326 | cell chemotaxis | 139 | 154 62 | 184 | 7 | 0.00 137 539 4 | 0.02 866 359 2 | TNFRSF11A, CXCL5, IL6, NR4A1, CXCL9, SEMA5A, CCR1 |
| upreg ulated | GO:19 03036 | positive regulation of response to wounding | 139 | 154 62 | 92 | 5 | 0.00 143 607 4 | 0.02 972 178 | ADRA2A, TNFRSF11A, IL6, ALOX5AP, FABP4 |
| upreg ulated | GO:00 32965 | regulation of collagen biosynthetic process | 139 | 154 62 | 26 | 3 | 0.00 158 869 | 0.03 167 347 3 | RGCC, WNT4, IL6 |
| upreg ulated | GO:00 31328 | positive regulation of cellular biosynthetic | 139 | 154 62 | 140 7 | 24 | 0.00 162 082 8 | 0.03 167 347 3 | FAM129A, NR4A3, TLR1, IRF4, SHOX2, TNNI2, MDFIC, HOXA5, NFATC2, SATB2, NPAS2, NR4A1, GDF6, DLX5, CDH13, HIVEP3, GUCY1A3, BMP2, RGCC, |

| | | | | | | | | | |
|---|---|---|---|---|---|---|---|---|---|
| | | process | | | | | | | WNT4, IL6, TBX5, NAMPT, FOXO1 |
| upreg ulated | GO:00 10566 | regulation of ketone biosynthetic process | 139 | 154 62 | 7 | 2 | 0.00 163 591 7 | 0.03 167 347 3 | WNT4, BMP2 |
| upreg ulated | GO:00 72540 | T-helper 17 cell lineage commitment | 139 | 154 62 | 7 | 2 | 0.00 163 591 7 | 0.03 167 347 3 | IL6, IRF4 |
| upreg ulated | GO:00 31946 | regulation of glucocorticoid biosynthetic process | 139 | 154 62 | 7 | 2 | 0.00 163 591 7 | 0.03 167 347 3 | WNT4, BMP2 |
| upreg ulated | GO:00 45714 | regulation of low-density lipoprotein particle receptor biosynthetic process | 139 | 154 62 | 7 | 2 | 0.00 163 591 7 | 0.03 167 347 3 | ITGB3, ADIPOQ |
| upreg ulated | GO:19 01522 | positive regulation of transcription from RNA polymerase II promoter involved in cellular response to chemical stimulus | 139 | 154 62 | 7 | 2 | 0.00 163 591 7 | 0.03 167 347 3 | DLX5, BMP2 |
| upreg ulated | GO:00 32353 | negative regulation of hormone biosynthetic process | 139 | 154 62 | 7 | 2 | 0.00 163 591 7 | 0.03 167 347 3 | WNT4, BMP2 |
| upreg ulated | GO:00 38030 | non-canonical Wnt signaling pathway via MAPK cascade | 139 | 154 62 | 7 | 2 | 0.00 163 591 7 | 0.03 167 347 3 | WNT4, NKD1 |
| upreg ulated | GO:00 32344 | regulation of aldosterone metabolic process | 139 | 154 62 | 7 | 2 | 0.00 163 591 7 | 0.03 167 347 3 | WNT4, BMP2 |
| upreg ulated | GO:00 07162 | negative regulation of cell adhesion | 139 | 154 62 | 96 | 5 | 0.00 173 467 9 | 0.03 337 033 8 | RGCC, CDH13, SEMA5A, ADIPOQ, BMP2 |
| upreg ulated | GO:00 22408 | negative regulation of cell-cell adhesion | 139 | 154 62 | 27 | 3 | 0.00 177 559 2 | 0.03 393 981 5 | RGCC, ADIPOQ, BMP2 |
| upreg ulated | GO:00 33002 | muscle cell proliferation | 139 | 154 62 | 97 | 5 | 0.00 181 594 6 | 0.03 438 989 3 | IL6, TBX5, CDH13, PDE1A, ADIPOQ |
| upreg ulated | GO:00 45669 | positive regulation of osteoblast differentiation | 139 | 154 62 | 58 | 4 | 0.00 182 205 7 | 0.03 438 989 3 | WNT4, IL6, DLX5, BMP2 |
| upreg ulated | GO:00 45935 | positive regulation of nucleobase-containing compound metabolic process | 139 | 154 62 | 133 9 | 23 | 0.00 187 077 2 | 0.03 508 867 2 | NR4A3, IRF4, SHOX2, TNNI2, MDFIC, HOXA5, NFATC2, SATB2, NPAS2, NR4A1, GDF6, DLX5, CDH13, HIVEP3, GUCY1A3, BMP2, RGCC, WNT4, IL6, TBX5, NAMPT, CXCL9, FOXO1 |
| upreg ulated | GO:00 50794 | regulation of cellular process | 139 | 154 62 | 878 2 | 96 | 0.00 193 801 7 | 0.03 592 328 9 | TAGAP, FAM129A, P2RY8, SHOX2, IRF4, FABP4, TNNI2, RAB30, BNIP3L, TFEC, NPAS2, GRP, PNMA2, COL1A2, LCP2, ASIC2, BCHE, FOXO1, ARHGAP42, CNTN1, TSPAN5, TLR1, TENM3, CHRNA1, SOS1, MDFIC, |

| | | | | | | | | | |
|---|---|---|---|---|---|---|---|---|---|
| | | | | | | | | | PLA2R1, FEM1B, FOSL2, CD36, SATB2, ZFHX4, GPR34, PRKG1, PDE1A, GUCY1A3, TNFRSF11A, IL6, TBX5, GPC6, CBLC, DCLK1, CD2, ADRA2A, HOXA5, NFATC2, PDLIM5, NKD1, GDF6, GLRX, GFRA1, RASGRF2, IL18R1, DPP4, DEPDC7, DHX34, CAMK2D, HIVEP3, FYB, RGCC, ITGB3, PRDM6, BNC2, ERCC1, CXCL9, NR4A3, FPR3, SLAMF7, SEMA5A, RUNX1T1, RFX2, CLEC1A, EFEMP1, PPP1R3B, MCTP1, DOCK8, CALB1, ELN, LRRK2, BIRC3, STAP1, NR4A1, DLX5, CDH13, SDC2, MEGF10, ANGPTL4, PTPRC, CCR1, BMP2, WNT4, CXCL5, RARRES1, NAMPT, SKAP2, ADIPOQ |
| upreg ulated | GO:00 48666 | neuron development | 139 | 154 62 | 866 | 17 | 0.00 197 406 3 | 0.03 592 328 9 | NR4A3, TENM3, DCLK1, SOS1, SEMA5A, SHOX2, PDLIM5, LRRK2, PRKG1, GFRA1, DLX5, NPTX1, SDC2, PTPRC, ITGB3, IL6, CNTN1 |
| upreg ulated | GO:00 10743 | regulation of macrophage derived foam cell differentiation | 139 | 154 62 | 28 | 3 | 0.00 197 566 7 | 0.03 592 328 9 | ITGB3, CD36, ADIPOQ |
| upreg ulated | GO:00 46649 | lymphocyte activation | 139 | 154 62 | 504 | 12 | 0.00 198 139 9 | 0.03 592 328 9 | SLAMF7, IL18R1, IRF4, DPP4, PTPRC, CD2, WNT4, IL6, ERCC1, NFATC2, SKAP2, DOCK8 |
| upreg ulated | GO:00 07519 | skeletal muscle tissue development | 139 | 154 62 | 145 | 6 | 0.00 198 291 8 | 0.03 592 328 9 | ELN, NR4A1, MEGF10, SHOX2, CHRNA1, HIVEP3 |
| upreg ulated | GO:00 09891 | positive regulation of biosynthetic process | 139 | 154 62 | 142 9 | 24 | 0.00 199 495 1 | 0.03 592 328 9 | FAM129A, NR4A3, TLR1, IRF4, SHOX2, TNNI2, MDFIC, HOXA5, NFATC2, SATB2, NPAS2, NR4A1, GDF6, DLX5, CDH13, HIVEP3, GUCY1A3, BMP2, RGCC, WNT4, IL6, TBX5, NAMPT, FOXO1 |
| upreg ulated | GO:00 00902 | cell morphogenesis | 139 | 154 62 | 944 | 18 | 0.00 199 906 3 | 0.03 592 328 9 | NR4A3, DCLK1, SOS1, SEMA5A, SHOX2, PDLIM5, LRRK2, GFRA1, DLX5, NPTX1, SDC2, PTPRC, BMP2, RGCC, ITGB3, WNT4, TBX5, CNTN1 |
| upreg ulated | GO:00 45664 | regulation of neuron differentiation | 139 | 154 62 | 374 | 10 | 0.00 204 281 5 | 0.03 605 107 3 | LRRK2, GDF6, SDC2, TENM3, SEMA5A, SHOX2, BMP2, IL6, PDLIM5, CNTN1 |
| upreg ulated | GO:00 09887 | organ morphogenesis | 139 | 154 62 | 719 | 15 | 0.00 204 811 8 | 0.03 605 107 3 | ELN, LRRK2, NKD1, DLX5, GCNT4, TENM3, SHOX2, BMP2, COL1A2, WNT4, HOXA5, FEM1B, TBX5, CALB1, SATB2 |
| upreg ulated | GO:00 60538 | skeletal muscle organ development | 139 | 154 62 | 146 | 6 | 0.00 205 289 6 | 0.03 605 107 3 | ELN, NR4A1, MEGF10, SHOX2, CHRNA1, HIVEP3 |
| upreg ulated | GO:00 22604 | regulation of cell morphogenesis | 139 | 154 62 | 253 | 8 | 0.00 205 422 6 | 0.03 605 107 3 | LRRK2, SDC2, SHOX2, SEMA5A, BMP2, RGCC, TBX5, PDLIM5 |
| upreg ulated | GO:19 02531 | regulation of intracellular signal transduction | 139 | 154 62 | 126 8 | 22 | 0.00 209 096 7 | 0.03 648 250 8 | TAGAP, CBLC, SOS1, SEMA5A, ADRA2A, MDFIC, PLA2R1, CD36, LRRK2, BIRC3, CDH13, IL18R1, RASGRF2, DEPDC7, CCR1, BMP2, TNFRSF11A, WNT4, IL6, CXCL9, ADIPOQ, FOXO1 |
| upreg ulated | GO:00 34650 | cortisol metabolic process | 139 | 154 62 | 8 | 2 | 0.00 216 838 2 | 0.03 655 794 8 | WNT4, BMP2 |
| upreg ulated | GO:00 45713 | low-density lipoprotein particle receptor biosynthetic | 139 | 154 62 | 8 | 2 | 0.00 216 838 2 | 0.03 655 794 8 | ITGB3, ADIPOQ |

| | | | | | | | | | |
|---|---|---|---|---|---|---|---|---|---|
| | | process | | | | | | | |
| upreg ulated | GO:00 55094 | response to lipoprotein particle | 139 | 154 62 | 8 | 2 | 0.00 216 838 2 | 0.03 655 794 8 | CDH13, CD36 |
| upreg ulated | GO:00 34651 | cortisol biosynthetic process | 139 | 154 62 | 8 | 2 | 0.00 216 838 2 | 0.03 655 794 8 | WNT4, BMP2 |
| upreg ulated | GO:00 32351 | negative regulation of hormone metabolic process | 139 | 154 62 | 8 | 2 | 0.00 216 838 2 | 0.03 655 794 8 | WNT4, BMP2 |
| upreg ulated | GO:00 06910 | phagocytosis, recognition | 139 | 154 62 | 8 | 2 | 0.00 216 838 2 | 0.03 655 794 8 | MEGF10, CD36 |
| upreg ulated | GO:00 10712 | regulation of collagen metabolic process | 139 | 154 62 | 29 | 3 | 0.00 218 923 5 | 0.03 670 331 6 | RGCC, WNT4, IL6 |
| upreg ulated | GO:00 45893 | positive regulation of transcription, DNA-templated | 139 | 154 62 | 111 2 | 20 | 0.00 223 257 2 | 0.03 722 193 4 | NR4A3, IRF4, SHOX2, TNNI2, MDFIC, HOXA5, NFATC2, SATB2, NPAS2, NR4A1, GDF6, DLX5, CDH13, HIVEP3, BMP2, WNT4, IL6, TBX5, NAMPT, FOXO1 |
| upreg ulated | GO:00 10941 | regulation of cell death | 139 | 154 62 | 127 6 | 22 | 0.00 226 145 3 | 0.03 733 793 6 | NR4A3, SOS1, SEMA5A, HOXA5, FEM1B, BNIP3L, LRRK2, NPAS2, BIRC3, NR4A1, RASGRF2, PDE1A, ANGPTL4, PNMA2, BMP2, RGCC, WNT4, IL6, TBX5, ASIC2, FOXO1, ADIPOQ |
| upreg ulated | GO:00 14706 | striated muscle tissue development | 139 | 154 62 | 257 | 8 | 0.00 226 441 3 | 0.03 733 793 6 | ELN, NR4A1, MEGF10, CHRNA1, SHOX2, BMP2, HIVEP3, TBX5 |
| upreg ulated | GO:00 00165 | MAPK cascade | 139 | 154 62 | 583 | 13 | 0.00 230 341 9 | 0.03 777 355 4 | LRRK2, NKD1, CBLC, CCR1, BMP2, TNFRSF11A, ADRA2A, WNT4, IL6, MDFIC, CD36, ADIPOQ, FOXO1 |
| upreg ulated | GO:00 51173 | positive regulation of nitrogen compound metabolic process | 139 | 154 62 | 136 2 | 23 | 0.00 232 957 1 | 0.03 799 479 6 | NR4A3, IRF4, SHOX2, TNNI2, MDFIC, HOXA5, NFATC2, SATB2, NPAS2, NR4A1, GDF6, DLX5, CDH13, HIVEP3, GUCY1A3, BMP2, RGCC, WNT4, IL6, TBX5, NAMPT, CXCL9, FOXO1 |
| upreg ulated | GO:00 19220 | regulation of phosphate metabolic process | 139 | 154 62 | 170 5 | 27 | 0.00 238 105 7 | 0.03 862 460 7 | TAGAP, FAM129A, CBLC, SOS1, FABP4, ADRA2A, MDFIC, CD36, DOCK8, LRRK2, BIRC3, GDF6, PRKG1, RASGRF2, CCR1, BMP2, GUCY1A3, PTPRC, TNFRSF11A, ITGB3, RGCC, WNT4, IL6, CXCL9, ADIPOQ, FOXO1, ARHGAP42 |
| upreg ulated | GO:00 02548 | monocyte chemotaxis | 139 | 154 62 | 30 | 3 | 0.00 241 660 1 | 0.03 878 191 5 | TNFRSF11A, IL6, CCR1 |
| upreg ulated | GO:20 00351 | regulation of endothelial cell apoptotic process | 139 | 154 62 | 30 | 3 | 0.00 241 660 1 | 0.03 878 191 5 | RGCC, SEMA5A, ANGPTL4 |
| upreg ulated | GO:00 32386 | regulation of intracellular transport | 139 | 154 62 | 320 | 9 | 0.00 242 979 3 | 0.03 878 621 7 | LRRK2, IL18R1, SEMA5A, CAMK2D, IL6, MDFIC, CXCL9, CD36, ADIPOQ |
| upreg ulated | GO:00 51223 | regulation of protein transport | 139 | 154 62 | 322 | 9 | 0.00 253 342 3 | 0.04 022 646 5 | TLR1, IL18R1, SEMA5A, CD2, RGCC, IL6, MDFIC, CD36, ADIPOQ |
| upreg ulated | GO:00 60284 | regulation of cell development | 139 | 154 62 | 590 | 13 | 0.00 255 432 | 0.04 034 486 | LRRK2, GDF6, SDC2, TENM3, SEMA5A, SHOX2, BMP2, RGCC, IL6, TBX5, PDLIM5, ADIPOQ, CNTN1 |

| | | | | | | | | | |
|---|---|---|---|---|---|---|---|---|---|
| | | | | | | | 3 | | |
| upregulated | GO:0051051 | negative regulation of transport | 139 | 15462 | 323 | 9 | 0.002586539 | 0.040639812 | LRRK2, RGCC, ITGB3, ADRA2A, IL6, PLA2R1, MDFIC, CD36, ADIPOQ |
| upregulated | GO:0048729 | tissue morphogenesis | 139 | 15462 | 453 | 11 | 0.002615755 | 0.04073483 | NR4A3, NKD1, GCNT4, SEMA5A, SHOX2, BMP2, WNT4, IL6, HOXA5, TBX5, FEM1B |
| upregulated | GO:0051174 | regulation of phosphorus metabolic process | 139 | 15462 | 1718 | 27 | 0.002651998 | 0.04073483 | TAGAP, FAM129A, CBLC, SOS1, FABP4, ADRA2A, MDFIC, CD36, DOCK8, LRRK2, BIRC3, GDF6, PRKG1, RASGRF2, CCR1, BMP2, GUCY1A3, PTPRC, TNFRSF11A, ITGB3, RGCC, WNT4, IL6, CXCL9, ADIPOQ, FOXO1, ARHGAP42 |
| upregulated | GO:0090077 | foam cell differentiation | 139 | 15462 | 31 | 3 | 0.00265806 | 0.04073483 | ITGB3, CD36, ADIPOQ |
| upregulated | GO:0010742 | macrophage derived foam cell differentiation | 139 | 15462 | 31 | 3 | 0.00265806 | 0.04073483 | ITGB3, CD36, ADIPOQ |
| upregulated | GO:0048514 | blood vessel morphogenesis | 139 | 15462 | 454 | 11 | 0.002660455 | 0.04073483 | NR4A1, CDH13, SEMA5A, ANGPTL4, RGCC, ITGB3, WNT4, IL6, HOXA5, THSD7A, TBX5 |
| upregulated | GO:0031349 | positive regulation of defense response | 139 | 15462 | 265 | 8 | 0.002735354 | 0.041586635 | TNFRSF11A, BIRC3, IL6, TLR1, ALOX5AP, IRF4, CD36, FABP4 |
| upregulated | GO:0031943 | regulation of glucocorticoid metabolic process | 139 | 15462 | 9 | 2 | 0.002771519 | 0.041586635 | WNT4, BMP2 |
| upregulated | GO:0045721 | negative regulation of gluconeogenesis | 139 | 15462 | 9 | 2 | 0.002771519 | 0.041586635 | IL6, ADIPOQ |
| upregulated | GO:0032342 | aldosterone biosynthetic process | 139 | 15462 | 9 | 2 | 0.002771519 | 0.041586635 | WNT4, BMP2 |
| upregulated | GO:0030203 | glycosaminoglycan metabolic process | 139 | 15462 | 156 | 6 | 0.002859745 | 0.042656604 | GPC6, CHSY3, PRELP, SDC2, GALNT5, HS3ST3B1 |
| upregulated | GO:0042221 | response to chemical | 139 | 15462 | 3399 | 45 | 0.002903186 | 0.042656604 | IRF4, FABP4, ADRA2A, NFATC2, NPTX1, GFRA1, IL18R1, RASGRF2, COL1A2, CAMK2D, ITGB3, RGCC, ERCC1, ASIC2, ADH1B, CXCL9, BCHE, FOXO1, CNTN1, PAPPA, NR4A3, FPR3, TLR1, SOS1, SEMA5A, PLA2R1, ALOX5AP, FOSL2, CD36, CALB1, SATB2, LRRK2, NR4A1, DLX5, CDH13, PDE1A, PTPRC, BMP2, CCR1, TNFRSF11A, CXCL5, WNT4, IL6, NAMPT, ADIPOQ |
| upregulated | GO:0050663 | cytokine secretion | 139 | 15462 | 108 | 5 | 0.002904014 | 0.042656604 | RGCC, CD2, LCP2, IL6, TLR1 |
| upregulated | GO:0032964 | collagen biosynthetic process | 139 | 15462 | 32 | 3 | 0.002913897 | 0.042656604 | RGCC, WNT4, IL6 |
| upregulated | GO:0001954 | positive regulation of cell-matrix adhesion | 139 | 15462 | 32 | 3 | 0.002913897 | 0.042656604 | WNT4, CDH13, CD36 |
| upregulated | GO:0006022 | aminoglycan metabolic process | 139 | 15462 | 158 | 6 | 0.003046131 | 0.044031472 | GPC6, CHSY3, PRELP, SDC2, GALNT5, HS3ST3B1 |

| | | | | | | | | | |
|---|---|---|---|---|---|---|---|---|---|
| upreg ulated | GO:00 30168 | platelet activation | 139 | 154 62 | 212 | 7 | 0.00 306 161 8 | 0.04 403 147 2 | ADRA2A, ITGB3, LCP2, IL6, SOS1, CD36, COL1A2 |
| upreg ulated | GO:00 30335 | positive regulation of cell migration | 139 | 154 62 | 270 | 8 | 0.00 306 650 4 | 0.04 403 147 2 | CDH13, SEMA5A, CCR1, BMP2, PTPRC, ITGB3, ADRA2A, IL6 |
| upreg ulated | GO:00 60537 | muscle tissue development | 139 | 154 62 | 270 | 8 | 0.00 306 650 4 | 0.04 403 147 2 | ELN, NR4A1, MEGF10, CHRNA1, SHOX2, BMP2, HIVEP3, TBX5 |
| upreg ulated | GO:00 43067 | regulation of programmed cell death | 139 | 154 62 | 122 7 | 21 | 0.00 312 595 2 | 0.04 467 134 3 | NR4A3, SOS1, SEMA5A, HOXA5, FEM1B, BNIP3L, LRRK2, BIRC3, NR4A1, RASGRF2, PDE1A, ANGPTL4, PNMA2, BMP2, RGCC, WNT4, IL6, TBX5, ASIC2, FOXO1, ADIPOQ |
| upreg ulated | GO:00 72577 | endothelial cell apoptotic process | 139 | 154 62 | 33 | 3 | 0.00 318 438 4 | 0.04 526 542 1 | RGCC, SEMA5A, ANGPTL4 |
| upreg ulated | GO:00 01501 | skeletal system development | 139 | 154 62 | 398 | 10 | 0.00 319 769 1 | 0.04 526 542 1 | PRELP, DLX5, SHOX2, BMP2, PTPRC, COL1A2, EFEMP1, COL12A1, HOXA5, SATB2 |
| upreg ulated | GO:00 42058 | regulation of epidermal growth factor receptor signaling pathway | 139 | 154 62 | 68 | 4 | 0.00 326 403 8 | 0.04 598 768 7 | ADRA2A, CBLC, CDH13, SOS1 |
| upreg ulated | GO:00 06954 | inflammatory response | 139 | 154 62 | 537 | 12 | 0.00 332 677 5 | 0.04 614 079 6 | BIRC3, TLR1, AOX1, CCR1, BMP2, FABP4, TNFRSF11A, ADRA2A, IL6, CXCL9, ALOX5AP, ADIPOQ |
| upreg ulated | GO:00 51247 | positive regulation of protein metabolic process | 139 | 154 62 | 990 | 18 | 0.00 333 504 3 | 0.04 614 079 6 | FAM129A, TLR1, IRF4, ADRA2A, MDFIC, CD36, LRRK2, BIRC3, NKD1, GDF6, PTPRC, BMP2, RGCC, TNFRSF11A, ITGB3, IL6, FOXO1, ADIPOQ |
| upreg ulated | GO:19 02680 | positive regulation of RNA biosynthetic process | 139 | 154 62 | 115 2 | 20 | 0.00 335 262 7 | 0.04 614 079 6 | NR4A3, IRF4, SHOX2, TNNI2, MDFIC, HOXA5, NFATC2, SATB2, NPAS2, NR4A1, GDF6, DLX5, CDH13, HIVEP3, BMP2, WNT4, IL6, TBX5, NAMPT, FOXO1 |
| upreg ulated | GO:20 00147 | positive regulation of cell motility | 139 | 154 62 | 274 | 8 | 0.00 335 327 5 | 0.04 614 079 6 | CDH13, SEMA5A, CCR1, BMP2, PTPRC, ITGB3, ADRA2A, IL6 |
| upreg ulated | GO:00 35556 | intracellular signal transduction | 139 | 154 62 | 219 5 | 32 | 0.00 336 529 5 | 0.04 614 079 6 | TAGAP, CBLC, DCLK1, SEMA5A, SOS1, ADRA2A, PLA2R1, MDFIC, RAB30, CD36, MCTP1, DOCK8, LRRK2, BIRC3, NKD1, NR4A1, IL18R1, CDH13, RASGRF2, DEPDC7, GUCY1A3, COL1A2, CCR1, FYB, CAMK2D, BMP2, TNFRSF11A, WNT4, IL6, CXCL9, ADIPOQ, FOXO1 |
| upreg ulated | GO:00 45597 | positive regulation of cell differentiation | 139 | 154 62 | 539 | 12 | 0.00 342 762 8 | 0.04 614 079 6 | GDF6, DLX5, SEMA5A, SHOX2, CCR1, BMP2, RGCC, WNT4, IL6, HOXA5, CD36, ADIPOQ |
| upreg ulated | GO:00 32341 | aldosterone metabolic process | 139 | 154 62 | 10 | 2 | 0.00 344 403 1 | 0.04 614 079 6 | WNT4, BMP2 |
| upreg ulated | GO:00 03181 | atrioventricular valve morphogenesis | 139 | 154 62 | 10 | 2 | 0.00 344 403 1 | 0.04 614 079 6 | TBX5, BMP2 |
| upreg ulated | GO:00 72539 | T-helper 17 cell differentiation | 139 | 154 62 | 10 | 2 | 0.00 344 403 1 | 0.04 614 079 6 | IL6, IRF4 |

| | | | | | | | | | |
|---|---|---|---|---|---|---|---|---|---|
| upregulated | GO:0002295 | T-helper cell lineage commitment | 139 | 15462 | 10 | 2 | 0.003444031 | 0.046140796 | IL6, IRF4 |
| upregulated | GO:0043373 | CD4-positive, alpha-beta T cell lineage commitment | 139 | 15462 | 10 | 2 | 0.003444031 | 0.046140796 | IL6, IRF4 |
| upregulated | GO:0051050 | positive regulation of transport | 139 | 15462 | 541 | 12 | 0.003530947 | 0.047094981 | IL18R1, SEMA5A, CCR1, RGCC, TNFRSF11A, CD2, PLA2R1, IL6, CXCL9, CD36, CNTN1, ADIPOQ |
| upregulated | GO:0045937 | positive regulation of phosphate metabolic process | 139 | 15462 | 840 | 16 | 0.003608223 | 0.047701662 | FAM129A, ADRA2A, MDFIC, CD36, LRRK2, GDF6, PTPRC, BMP2, GUCY1A3, CCR1, ITGB3, TNFRSF11A, RGCC, IL6, CXCL9, ADIPOQ |
| upregulated | GO:0010562 | positive regulation of phosphorus metabolic process | 139 | 15462 | 840 | 16 | 0.003608223 | 0.047701662 | FAM129A, ADRA2A, MDFIC, CD36, LRRK2, GDF6, PTPRC, BMP2, GUCY1A3, CCR1, ITGB3, TNFRSF11A, RGCC, IL6, CXCL9, ADIPOQ |
| upregulated | GO:0007399 | nervous system development | 139 | 15462 | 1848 | 28 | 0.003701949 | 0.048726093 | NR4A3, SHOX2, TENM3, DCLK1, SOS1, SEMA5A, PDLIM5, LSAMP, SATB2, LRRK2, NPAS2, NKD1, GDF6, PRKG1, NPTX1, DLX5, GFRA1, SDC2, BMP2, PTPRC, ITGB3, WNT4, PRDM6, IL6, ASIC2, SLC7A5, BCHE, CNTN1 |
| upregulated | GO:0002294 | CD4-positive, alpha-beta T cell differentiation involved in immune response | 139 | 15462 | 35 | 3 | 0.003770349 | 0.048774172 | IL6, IL18R1, IRF4 |
| upregulated | GO:0042093 | T-helper cell differentiation | 139 | 15462 | 35 | 3 | 0.003770349 | 0.048774172 | IL6, IL18R1, IRF4 |
| upregulated | GO:0090132 | epithelium migration | 139 | 15462 | 165 | 6 | 0.003770612 | 0.048774172 | RGCC, ITGB3, NR4A1, CDH13, DPP4, SEMA5A |
| upregulated | GO:0010631 | epithelial cell migration | 139 | 15462 | 165 | 6 | 0.003770612 | 0.048774172 | RGCC, ITGB3, NR4A1, CDH13, DPP4, SEMA5A |
| upregulated | GO:1901184 | regulation of ERBB signaling pathway | 139 | 15462 | 71 | 4 | 0.003814578 | 0.048808552 | ADRA2A, CBLC, CDH13, SOS1 |
| upregulated | GO:0006979 | response to oxidative stress | 139 | 15462 | 280 | 8 | 0.003822063 | 0.048808552 | NR4A3, LRRK2, IL6, PLA2R1, ERCC1, CD36, FOXO1, ADIPOQ |
| upregulated | GO:0051272 | positive regulation of cellular component movement | 139 | 15462 | 280 | 8 | 0.003822063 | 0.048808552 | CDH13, SEMA5A, CCR1, BMP2, PTPRC, ITGB3, ADRA2A, IL6 |
| upregulated | GO:0023014 | signal transduction by phosphorylation | 139 | 15462 | 619 | 13 | 0.003845017 | 0.048893623 | LRRK2, NKD1, CBLC, CCR1, BMP2, TNFRSF11A, ADRA2A, WNT4, IL6, MDFIC, CD36, ADIPOQ, FOXO1 |
| upregulated | GO:0022411 | cellular component disassembly | 139 | 15462 | 410 | 10 | 0.003944418 | 0.049945981 | ELN, DPP4, SEMA5A, ADAMTS5, COL1A2, COL12A1, TBX5, CD36, MMP1, ADIPOQ |
| upregulated | GO:0070206 | protein trimerization | 139 | 15462 | 36 | 3 | 0.004086319 | 0.051167232 | ALOX5AP, COL1A2, ADIPOQ |

| | | | | | | | | | |
|---|---|---|---|---|---|---|---|---|---|
| upregulated | GO:0045599 | negative regulation of fat cell differentiation | 139 | 15462 | 36 | 3 | 0.004086319 | 0.051167232 | IL6, ADIPOQ, FOXO1 |
| upregulated | GO:0051224 | negative regulation of protein transport | 139 | 15462 | 117 | 5 | 0.004094233 | 0.051167232 | RGCC, IL6, MDFIC, CD36, ADIPOQ |
| upregulated | GO:0051254 | positive regulation of RNA metabolic process | 139 | 15462 | 1173 | 20 | 0.004109065 | 0.051167232 | NR4A3, IRF4, SHOX2, TNNI2, MDFIC, HOXA5, NFATC2, SATB2, NPAS2, NR4A1, GDF6, DLX5, CDH13, HIVEP3, BMP2, WNT4, IL6, TBX5, NAMPT, FOXO1 |
| upregulated | GO:0070208 | protein heterotrimerization | 139 | 15462 | 11 | 2 | 0.004184643 | 0.051440901 | ADIPOQ, COL1A2 |
| upregulated | GO:0002363 | alpha-beta T cell lineage commitment | 139 | 15462 | 11 | 2 | 0.004184643 | 0.051440901 | IL6, IRF4 |
| upregulated | GO:0045596 | negative regulation of cell differentiation | 139 | 15462 | 482 | 11 | 0.004186018 | 0.051440901 | SEMA5A, BMP2, ITGB3, EFEMP1, WNT4, PRDM6, IL6, HOXA5, TBX5, ADIPOQ, FOXO1 |
| upregulated | GO:0032989 | cellular component morphogenesis | 139 | 15462 | 1012 | 18 | 0.004199607 | 0.051440901 | NR4A3, DCLK1, SOS1, SEMA5A, SHOX2, PDLIM5, LRRK2, GFRA1, DLX5, NPTX1, SDC2, PTPRC, BMP2, RGCC, ITGB3, WNT4, TBX5, CNTN1 |
| upregulated | GO:0043270 | positive regulation of ion transport | 139 | 15462 | 118 | 5 | 0.004245107 | 0.051786854 | TNFRSF11A, PLA2R1, CXCL9, CCR1, CNTN1 |
| upregulated | GO:0048646 | anatomical structure formation involved in morphogenesis | 139 | 15462 | 855 | 16 | 0.004280852 | 0.05201148 | NR4A3, SEMA5A, HOXA5, THSD7A, NKD1, NR4A1, DLX5, CDH13, ANGPTL4, BMP2, ITGB3, RGCC, WNT4, IL6, TBX5, ERCC1 |
| upregulated | GO:0090130 | tissue migration | 139 | 15462 | 170 | 6 | 0.0043614 | 0.052152179 | RGCC, ITGB3, NR4A1, CDH13, DPP4, SEMA5A |
| upregulated | GO:0002287 | alpha-beta T cell activation involved in immune response | 139 | 15462 | 37 | 3 | 0.004417926 | 0.052152179 | IL6, IL18R1, IRF4 |
| upregulated | GO:0002293 | alpha-beta T cell differentiation involved in immune response | 139 | 15462 | 37 | 3 | 0.004417926 | 0.052152179 | IL6, IL18R1, IRF4 |
| upregulated | GO:0044246 | regulation of multicellular organismal metabolic process | 139 | 15462 | 37 | 3 | 0.004417926 | 0.052152179 | RGCC, WNT4, IL6 |
| upregulated | GO:0048660 | regulation of smooth muscle cell proliferation | 139 | 15462 | 74 | 4 | 0.004424895 | 0.052152179 | IL6, CDH13, PDE1A, ADIPOQ |
| upregulated | GO:0032368 | regulation of lipid transport | 139 | 15462 | 74 | 4 | 0.004424895 | 0.052152179 | TNFRSF11A, ITGB3, PLA2R1, ADIPOQ |
| upregulated | GO:0050729 | positive regulation of inflammatory response | 139 | 15462 | 74 | 4 | 0.004424895 | 0.052152179 | TNFRSF11A, IL6, ALOX5AP, FABP4 |
| upregulated | GO:0042326 | negative regulation of | 139 | 15462 | 287 | 8 | 0.00443 | 0.05215 | FAM129A, BIRC3, CBLC, FABP4, PTPRC, IL6, FOXO1, ADIPOQ |

| | | | | | | | | | |
|---|---|---|---|---|---|---|---|---|---|
| | | phosphorylation | | | | | 145 8 | 217 9 | |
| upreg ulated | GO:00 30217 | T cell differentiation | 139 | 154 62 | 171 | 6 | 0.00 448 731 8 | 0.05 260 328 5 | CD2, WNT4, IL6, IL18R1, IRF4, PTPRC |
| upreg ulated | GO:00 40017 | positive regulation of locomotion | 139 | 154 62 | 289 | 8 | 0.00 461 861 9 | 0.05 393 181 5 | CDH13, SEMA5A, CCR1, BMP2, PTPRC, ITGB3, ADRA2A, IL6 |
| upreg ulated | GO:00 01934 | positive regulation of protein phosphorylation | 139 | 154 62 | 635 | 13 | 0.00 475 804 6 | 0.05 521 598 2 | FAM129A, LRRK2, GDF6, BMP2, PTPRC, RGCC, TNFRSF11A, ITGB3, ADRA2A, IL6, MDFIC, CD36, ADIPOQ |
| upreg ulated | GO:00 10883 | regulation of lipid storage | 139 | 154 62 | 38 | 3 | 0.00 476 539 1 | 0.05 521 598 2 | ITGB3, IL6, CD36 |
| upreg ulated | GO:00 30030 | cell projection organization | 139 | 154 62 | 102 6 | 18 | 0.00 484 104 9 | 0.05 568 394 1 | NR4A3, SHOX2, TENM3, DCLK1, SOS1, SEMA5A, PDLIM5, LRRK2, PRKG1, NPTX1, GFRA1, DLX5, SDC2, CDH13, PTPRC, ITGB3, IL6, CNTN1 |
| upreg ulated | GO:00 51240 | positive regulation of multicellular organismal process | 139 | 154 62 | 563 | 12 | 0.00 484 288 9 | 0.05 568 394 1 | BIRC3, IL18R1, CAMK2D, BMP2, RGCC, TNFRSF11A, CD2, ADRA2A, WNT4, IL6, CD36, ADIPOQ |
| upreg ulated | GO:00 16202 | regulation of striated muscle tissue development | 139 | 154 62 | 76 | 4 | 0.00 486 640 5 | 0.05 569 244 5 | TBX5, MEGF10, SHOX2, BMP2 |
| upreg ulated | GO:00 02684 | positive regulation of immune system process | 139 | 154 62 | 638 | 13 | 0.00 494 729 3 | 0.05 569 244 5 | BIRC3, TLR1, IRF4, DPP4, CCR1, FYB, PTPRC, RGCC, CD2, LCP2, IL6, NFATC2, CD36 |
| upreg ulated | GO:00 32305 | positive regulation of icosanoid secretion | 139 | 154 62 | 12 | 2 | 0.00 499 209 2 | 0.05 569 244 5 | TNFRSF11A, PLA2R1 |
| upreg ulated | GO:00 06705 | mineralocorticoid biosynthetic process | 139 | 154 62 | 12 | 2 | 0.00 499 209 2 | 0.05 569 244 5 | WNT4, BMP2 |
| upreg ulated | GO:00 43369 | CD4-positive or CD8-positive, alpha-beta T cell lineage commitment | 139 | 154 62 | 12 | 2 | 0.00 499 209 2 | 0.05 569 244 5 | IL6, IRF4 |
| upreg ulated | GO:19 02931 | negative regulation of alcohol biosynthetic process | 139 | 154 62 | 12 | 2 | 0.00 499 209 2 | 0.05 569 244 5 | WNT4, BMP2 |
| upreg ulated | GO:00 32799 | low-density lipoprotein receptor particle metabolic process | 139 | 154 62 | 12 | 2 | 0.00 499 209 2 | 0.05 569 244 5 | ITGB3, ADIPOQ |
| upreg ulated | GO:00 72538 | T-helper 17 type immune response | 139 | 154 62 | 12 | 2 | 0.00 499 209 2 | 0.05 569 244 5 | IL6, IRF4 |
| upreg ulated | GO:19 01861 | regulation of muscle tissue development | 139 | 154 62 | 77 | 4 | 0.00 509 786 1 | 0.05 597 057 4 | TBX5, MEGF10, SHOX2, BMP2 |
| upreg ulated | GO:00 32680 | regulation of tumor necrosis factor production | 139 | 154 62 | 77 | 4 | 0.00 509 786 1 | 0.05 597 057 4 | CD2, TLR1, CD36, ADIPOQ |
| upreg | GO:00 | smooth muscle | 139 | 154 | 77 | 4 | 0.00 | 0.05 | IL6, CDH13, PDE1A, ADIPOQ |

| | | | | | | | | | |
|---|---|---|---|---|---|---|---|---|---|
| ulated | 48659 | cell proliferation | | 62 | | | 509 786 1 | 597 057 4 | |
| upreg ulated | GO:00 32640 | tumor necrosis factor production | 139 | 154 62 | 77 | 4 | 0.00 509 786 1 | 0.05 597 057 4 | CD2, TLR1, CD36, ADIPOQ |
| upreg ulated | GO:00 48858 | cell projection morphogenesis | 139 | 154 62 | 716 | 14 | 0.00 511 976 1 | 0.05 597 057 4 | NR4A3, LRRK2, NPTX1, DLX5, GFRA1, SDC2, DCLK1, SOS1, SEMA5A, SHOX2, PTPRC, ITGB3, PDLIM5, CNTN1 |
| upreg ulated | GO:00 02292 | T cell differentiation involved in immune response | 139 | 154 62 | 39 | 3 | 0.00 512 892 6 | 0.05 597 057 4 | IL6, IL18R1, IRF4 |
| upreg ulated | GO:00 61138 | morphogenesis of a branching epithelium | 139 | 154 62 | 176 | 6 | 0.00 515 754 3 | 0.05 607 893 2 | WNT4, IL6, HOXA5, FEM1B, SEMA5A, BMP2 |
| upreg ulated | GO:00 02521 | leukocyte differentiation | 139 | 154 62 | 361 | 9 | 0.00 535 625 7 | 0.05 794 722 1 | IL18R1, IRF4, CCR1, PTPRC, TNFRSF11A, CD2, WNT4, IL6, ADIPOQ |
| upreg ulated | GO:00 16337 | single organismal cell-cell adhesion | 139 | 154 62 | 235 | 7 | 0.00 536 798 6 | 0.05 794 722 1 | RGCC, CD2, WNT4, MEGF10, DPP4, ADIPOQ, BMP2 |
| upreg ulated | GO:00 30155 | regulation of cell adhesion | 139 | 154 62 | 298 | 8 | 0.00 553 725 3 | 0.05 947 035 | RGCC, WNT4, CDH13, DPP4, SEMA5A, CD36, ADIPOQ, BMP2 |
| upreg ulated | GO:00 70201 | regulation of establishment of protein localization | 139 | 154 62 | 363 | 9 | 0.00 554 871 6 | 0.05 947 035 | TLR1, IL18R1, SEMA5A, CD2, RGCC, IL6, MDFIC, CD36, ADIPOQ |
| upreg ulated | GO:00 45598 | regulation of fat cell differentiation | 139 | 154 62 | 79 | 4 | 0.00 558 268 3 | 0.05 962 146 5 | IL6, ADIPOQ, BMP2, FOXO1 |
| upreg ulated | GO:00 33157 | regulation of intracellular protein transport | 139 | 154 62 | 180 | 6 | 0.00 574 448 4 | 0.06 089 662 | IL6, MDFIC, IL18R1, SEMA5A, CD36, ADIPOQ |
| upreg ulated | GO:00 61448 | connective tissue development | 139 | 154 62 | 180 | 6 | 0.00 574 448 4 | 0.06 089 662 | EFEMP1, HOXA5, NAMPT, SHOX2, SATB2, BMP2 |
| upreg ulated | GO:00 90030 | regulation of steroid hormone biosynthetic process | 139 | 154 62 | 13 | 2 | 0.00 586 513 5 | 0.06 089 662 | WNT4, BMP2 |
| upreg ulated | GO:00 45986 | negative regulation of smooth muscle contraction | 139 | 154 62 | 13 | 2 | 0.00 586 513 5 | 0.06 089 662 | ADRA2A, GUCY1A3 |
| upreg ulated | GO:00 06700 | C21-steroid hormone biosynthetic process | 139 | 154 62 | 13 | 2 | 0.00 586 513 5 | 0.06 089 662 | WNT4, BMP2 |
| upreg ulated | GO:00 10745 | negative regulation of macrophage derived foam cell differentiation | 139 | 154 62 | 13 | 2 | 0.00 586 513 5 | 0.06 089 662 | ITGB3, ADIPOQ |
| upreg ulated | GO:00 08212 | mineralocorticoid metabolic process | 139 | 154 62 | 13 | 2 | 0.00 586 513 5 | 0.06 089 662 | WNT4, BMP2 |
| upreg ulated | GO:00 50730 | regulation of peptidyl-tyrosine | 139 | 154 62 | 181 | 6 | 0.00 589 | 0.06 089 | ADRA2A, ITGB3, IL6, CBLC, CD36, ADIPOQ |

| | | | | | | | | | |
|---|---|---|---|---|---|---|---|---|---|
| | | phosphorylation | | | | | 854 7 | 662 | |
| upreg ulated | GO:00 01763 | morphogenesis of a branching structure | 139 | 154 62 | 181 | 6 | 0.00 589 854 7 | 0.06 089 662 | WNT4, IL6, HOXA5, FEM1B, SEMA5A, BMP2 |
| upreg ulated | GO:00 32370 | positive regulation of lipid transport | 139 | 154 62 | 41 | 3 | 0.00 590 500 4 | 0.06 089 662 | TNFRSF11A, PLA2R1, ADIPOQ |
| upreg ulated | GO:00 10033 | response to organic substance | 139 | 154 62 | 218 7 | 31 | 0.00 598 933 6 | 0.06 155 478 1 | PAPPA, NR4A3, TLR1, IRF4, SOS1, ADRA2A, FOSL2, CD36, CALB1, SATB2, LRRK2, NR4A1, NPTX1, DLX5, IL18R1, PDE1A, CDH13, RASGRF2, PTPRC, CAMK2D, BMP2, CCR1, COL1A2, TNFRSF11A, ITGB3, WNT4, IL6, NAMPT, BCHE, ADIPOQ, FOXO1 |
| upreg ulated | GO:00 10563 | negative regulation of phosphorus metabolic process | 139 | 154 62 | 368 | 9 | 0.00 605 335 9 | 0.06 178 955 4 | FAM129A, BIRC3, CBLC, FABP4, PTPRC, ADRA2A, IL6, FOXO1, ADIPOQ |
| upreg ulated | GO:00 45936 | negative regulation of phosphate metabolic process | 139 | 154 62 | 368 | 9 | 0.00 605 335 9 | 0.06 178 955 4 | FAM129A, BIRC3, CBLC, FABP4, PTPRC, ADRA2A, IL6, FOXO1, ADIPOQ |
| upreg ulated | GO:00 71706 | tumor necrosis factor superfamily cytokine production | 139 | 154 62 | 81 | 4 | 0.00 609 738 2 | 0.06 180 197 8 | CD2, TLR1, CD36, ADIPOQ |
| upreg ulated | GO:00 32990 | cell part morphogenesis | 139 | 154 62 | 731 | 14 | 0.00 611 982 7 | 0.06 180 197 8 | NR4A3, LRRK2, NPTX1, DLX5, GFRA1, SDC2, DCLK1, SOS1, SEMA5A, SHOX2, PTPRC, ITGB3, PDLIM5, CNTN1 |
| upreg ulated | GO:00 72359 | circulatory system development | 139 | 154 62 | 809 | 15 | 0.00 613 695 1 | 0.06 180 197 8 | NR4A1, CDH13, ANGPTL4, SEMA5A, SHOX2, BMP2, COL1A2, RGCC, ITGB3, WNT4, IL6, HOXA5, THSD7A, TBX5, FOXO1 |
| upreg ulated | GO:00 72358 | cardiovascular system development | 139 | 154 62 | 809 | 15 | 0.00 613 695 1 | 0.06 180 197 8 | NR4A1, CDH13, ANGPTL4, SEMA5A, SHOX2, BMP2, COL1A2, RGCC, ITGB3, WNT4, IL6, HOXA5, THSD7A, TBX5, FOXO1 |
| upreg ulated | GO:00 42981 | regulation of apoptotic process | 139 | 154 62 | 121 7 | 20 | 0.00 616 232 2 | 0.06 184 993 2 | NR4A3, SOS1, SEMA5A, HOXA5, FEM1B, BNIP3L, BIRC3, NR4A1, PDE1A, RASGRF2, ANGPTL4, PNMA2, BMP2, RGCC, WNT4, IL6, TBX5, ASIC2, FOXO1, ADIPOQ |
| upreg ulated | GO:00 43367 | CD4-positive, alpha-beta T cell differentiation | 139 | 154 62 | 42 | 3 | 0.00 631 792 2 | 0.06 320 028 2 | IL6, IL18R1, IRF4 |
| upreg ulated | GO:00 07409 | axonogenesis | 139 | 154 62 | 511 | 11 | 0.00 643 308 4 | 0.06 413 849 | NR4A3, NPTX1, DLX5, GFRA1, DCLK1, SEMA5A, SHOX2, SOS1, PTPRC, ITGB3, CNTN1 |
| upreg ulated | GO:00 51960 | regulation of nervous system development | 139 | 154 62 | 512 | 11 | 0.00 652 477 7 | 0.06 483 727 2 | LRRK2, GDF6, SDC2, TENM3, SEMA5A, SHOX2, BMP2, IL6, ASIC2, PDLIM5, CNTN1 |
| upreg ulated | GO:00 71902 | positive regulation of protein serine/threonine kinase activity | 139 | 154 62 | 244 | 7 | 0.00 655 831 2 | 0.06 495 542 3 | RGCC, ADRA2A, TNFRSF11A, LRRK2, MDFIC, ADIPOQ, BMP2 |
| upreg ulated | GO:00 43408 | regulation of MAPK cascade | 139 | 154 62 | 513 | 11 | 0.00 661 749 6 | 0.06 514 653 6 | LRRK2, CBLC, CCR1, BMP2, TNFRSF11A, ADRA2A, MDFIC, IL6, CD36, ADIPOQ, FOXO1 |
| upreg | GO:00 | positive | 139 | 154 | 816 | 15 | 0.00 | 0.06 | NR4A3, NPAS2, NR4A1, DLX5, CDH13, |

| | | | | | | | | | |
|---|---|---|---|---|---|---|---|---|---|
| ulated | 45944 | regulation of transcription from RNA polymerase II promoter | | 62 | | | 662 721 8 | 514 653 6 | IRF4, SHOX2, BMP2, IL6, HOXA5, TBX5, NAMPT, NFATC2, SATB2, FOXO1 |
| upreg ulated | GO:00 30177 | positive regulation of Wnt signaling pathway | 139 | 154 62 | 83 | 4 | 0.00 664 273 2 | 0.06 514 653 6 | WNT4, NKD1, DLX5, BMP2 |
| upreg ulated | GO:00 50790 | regulation of catalytic activity | 139 | 154 62 | 202 0 | 29 | 0.00 669 466 1 | 0.06 521 099 9 | TAGAP, CBLC, SOS1, FABP4, ADRA2A, MDFIC, PLA2R1, PPP1R3B, FEM1B, ALOX5AP, DOCK8, LRRK2, BIRC3, NR4A1, PRKG1, RASGRF2, PDE1A, ANGPTL4, BMP2, PTPRC, CAMK2D, TNFRSF11A, ITGB3, RGCC, WNT4, IL6, TFPI2, ADIPOQ, ARHGAP42 |
| upreg ulated | GO:00 02252 | immune effector process | 139 | 154 62 | 514 | 11 | 0.00 671 124 8 | 0.06 521 099 9 | SLAMF7, BIRC3, IL18R1, IRF4, PTPRC, RGCC, IL6, ERCC1, BNIP3L, CXCL9, CD36 |
| upreg ulated | GO:00 02250 | adaptive immune response | 139 | 154 62 | 186 | 6 | 0.00 671 449 5 | 0.06 521 099 9 | TNFRSF11A, IL6, ERCC1, IGJ, IL18R1, IRF4 |
| upreg ulated | GO:20 00193 | positive regulation of fatty acid transport | 139 | 154 62 | 14 | 2 | 0.00 680 254 4 | 0.06 564 126 5 | TNFRSF11A, PLA2R1 |
| upreg ulated | GO:00 60192 | negative regulation of lipase activity | 139 | 154 62 | 14 | 2 | 0.00 680 254 4 | 0.06 564 126 5 | PLA2R1, ANGPTL4 |
| upreg ulated | GO:00 42327 | positive regulation of phosphorylation | 139 | 154 62 | 741 | 14 | 0.00 686 989 7 | 0.06 607 872 2 | FAM129A, LRRK2, GDF6, CCR1, BMP2, PTPRC, RGCC, TNFRSF11A, ITGB3, ADRA2A, IL6, MDFIC, CD36, ADIPOQ |
| upreg ulated | GO:00 71241 | cellular response to inorganic substance | 139 | 154 62 | 84 | 4 | 0.00 692 713 9 | 0.06 641 643 7 | ALOX5AP, FOXO1, FABP4, CAMK2D |
| upreg ulated | GO:00 50731 | positive regulation of peptidyl-tyrosine phosphorylation | 139 | 154 62 | 133 | 5 | 0.00 701 055 6 | 0.06 700 216 3 | ADRA2A, ITGB3, IL6, CD36, ADIPOQ |
| upreg ulated | GO:00 51094 | positive regulation of developmental process | 139 | 154 62 | 744 | 14 | 0.00 710 875 3 | 0.06 772 497 9 | GDF6, DLX5, ANGPTL4, SEMA5A, SHOX2, CCR1, BMP2, RGCC, WNT4, IL6, HOXA5, ASIC2, CD36, ADIPOQ |
| upreg ulated | GO:00 01525 | angiogenesis | 139 | 154 62 | 378 | 9 | 0.00 716 849 8 | 0.06 807 804 6 | NR4A1, CDH13, SEMA5A, ANGPTL4, ITGB3, RGCC, IL6, HOXA5, THSD7A |
| upreg ulated | GO:00 32879 | regulation of localization | 139 | 154 62 | 167 0 | 25 | 0.00 722 091 9 | 0.06 834 128 1 | TLR1, SEMA5A, ADRA2A, CD2, MDFIC, PLA2R1, CD36, LRRK2, NKD1, CDH13, IL18R1, CCR1, CAMK2D, PTPRC, BMP2, TNFRSF11A, RGCC, ITGB3, WNT4, IL6, TBX5, ASIC2, CXCL9, CNTN1, ADIPOQ |
| upreg ulated | GO:00 01649 | osteoblast differentiation | 139 | 154 62 | 189 | 6 | 0.00 724 176 2 | 0.06 834 128 1 | WNT4, IL6, DLX5, SHOX2, SATB2, BMP2 |
| upreg ulated | GO:00 50921 | positive regulation of chemotaxis | 139 | 154 62 | 86 | 4 | 0.00 751 987 7 | 0.07 074 341 7 | IL6, CDH13, SEMA5A, CCR1 |
| upreg ulated | GO:19 01342 | regulation of vasculature development | 139 | 154 62 | 191 | 6 | 0.00 760 953 1 | 0.07 116 655 5 | RGCC, WNT4, IL6, HOXA5, ANGPTL4, SEMA5A |
| upreg ulated | GO:00 70482 | response to oxygen levels | 139 | 154 62 | 251 | 7 | 0.00 761 | 0.07 116 | RGCC, SDC2, ANGPTL4, DPP4, ADIPOQ, BMP2, FOXO1 |

| | | | | | | | | | |
|---|---|---|---|---|---|---|---|---|---|
| | | | | | | | 2284 | 6555 | |
| upregulated | GO:0002253 | activation of immune response | 139 | 15462 | 382 | 9 | 0.007656253 | 0.071182939 | RGCC, LCP2, BIRC3, TLR1, NFATC2, IRF4, CD36, PTPRC, FYB |
| upregulated | GO:0035767 | endothelial cell chemotaxis | 139 | 15462 | 15 | 2 | 0.007803108 | 0.071182939 | NR4A1, SEMA5A |
| upregulated | GO:0072202 | cell differentiation involved in metanephros development | 139 | 15462 | 15 | 2 | 0.007803108 | 0.071182939 | WNT4, ADIPOQ |
| upregulated | GO:1903053 | regulation of extracellular matrix organization | 139 | 15462 | 15 | 2 | 0.007803108 | 0.071182939 | RGCC, DPP4 |
| upregulated | GO:0032303 | regulation of icosanoid secretion | 139 | 15462 | 15 | 2 | 0.007803108 | 0.071182939 | TNFRSF11A, PLA2R1 |
| upregulated | GO:0002070 | epithelial cell maturation | 139 | 15462 | 15 | 2 | 0.007803108 | 0.071182939 | FEM1B, HOXA5 |
| upregulated | GO:0061564 | axon development | 139 | 15462 | 525 | 11 | 0.007812989 | 0.071182939 | NR4A3, NPTX1, DLX5, GFRA1, DCLK1, SOS1, SEMA5A, SHOX2, PTPRC, ITGB3, CNTN1 |
| upregulated | GO:0051216 | cartilage development | 139 | 15462 | 137 | 5 | 0.00792075 | 0.071182939 | EFEMP1, HOXA5, SHOX2, SATB2, BMP2 |
| upregulated | GO:0051049 | regulation of transport | 139 | 15462 | 1248 | 20 | 0.008067085 | 0.071182939 | TLR1, SEMA5A, ADRA2A, CD2, MDFIC, PLA2R1, CD36, LRRK2, CDH13, IL18R1, CCR1, CAMK2D, TNFRSF11A, RGCC, ITGB3, IL6, ASIC2, CXCL9, ADIPOQ, CNTN1 |
| upregulated | GO:0035710 | CD4-positive, alpha-beta T cell activation | 139 | 15462 | 46 | 3 | 0.008139429 | 0.071182939 | IL6, IL18R1, IRF4 |
| upregulated | GO:0048813 | dendrite morphogenesis | 139 | 15462 | 88 | 4 | 0.008145111 | 0.071182939 | LRRK2, PDLIM5, SDC2, DCLK1 |
| upregulated | GO:0001837 | epithelial to mesenchymal transition | 139 | 15462 | 88 | 4 | 0.008145111 | 0.071182939 | RGCC, WNT4, TBX5, BMP2 |
| upregulated | GO:0009306 | protein secretion | 139 | 15462 | 194 | 6 | 0.008186227 | 0.071182939 | RGCC, CD2, LCP2, IL6, TLR1, CBLN4 |
| upregulated | GO:0051048 | negative regulation of secretion | 139 | 15462 | 139 | 5 | 0.008405328 | 0.071182939 | RGCC, ADRA2A, IL6, PLA2R1, ADIPOQ |
| upregulated | GO:0010975 | regulation of neuron projection development | 139 | 15462 | 257 | 7 | 0.008611379 | 0.071182939 | LRRK2, SDC2, PDLIM5, TENM3, SEMA5A, SHOX2, CNTN1 |
| upregulated | GO:0050871 | positive regulation of B cell activation | 139 | 15462 | 47 | 3 | 0.008638028 | 0.071182939 | IL6, NFATC2, PTPRC |
| upregulated | GO:0050767 | regulation of neurogenesis | 139 | 15462 | 460 | 10 | 0.00865780 | 0.07118293 | LRRK2, GDF6, SDC2, TENM3, SEMA5A, SHOX2, BMP2, IL6, PDLIM5, CNTN1 |

| | | | | | | | | | |
|---|---|---|---|---|---|---|---|---|---|
| | | | | | | | 2 | 9 | |
| upregulated | GO:0051179 | localization | 139 | 15462 | 4718 | 56 | 0.008795002 | 0.071182939 | GPC6, DCLK1, FABP4, ADRA2A, CD2, HOXA5, RAB30, NFATC2, BNIP3L, NKD1, NPTX1, IL18R1, DPP4, JAKMIP1, COL1A2, CAMK2D, FYB, ITGB3, RGCC, LCP2, ASIC2, CXCL9, SLC22A3, CNTN1, TSPAN5, TLR1, STEAP4, SEMA5A, CHRNA1, SOS1, PLA2R1, MDFIC, PIEZO2, CD36, MMP1, SATB2, ATP11A, UBAC2, LRRK2, STAP1, NR4A1, PRKG1, MEGF10, CDH13, CCR1, BMP2, PTPRC, TNFRSF11A, WNT4, CXCL5, IL6, TBX5, CBLN4, SLC24A3, SLC7A5, ADIPOQ |
| upregulated | GO:0032387 | negative regulation of intracellular transport | 139 | 15462 | 90 | 4 | 0.008803541 | 0.071182939 | LRRK2, MDFIC, CD36, ADIPOQ |
| upregulated | GO:0050707 | regulation of cytokine secretion | 139 | 15462 | 90 | 4 | 0.008803541 | 0.071182939 | RGCC, CD2, IL6, TLR1 |
| upregulated | GO:0046716 | muscle cell cellular homeostasis | 139 | 15462 | 16 | 2 | 0.008865634 | 0.071182939 | IL6, CHRNA1 |
| upregulated | GO:0010888 | negative regulation of lipid storage | 139 | 15462 | 16 | 2 | 0.008865634 | 0.071182939 | ITGB3, IL6 |
| upregulated | GO:0008207 | C21-steroid hormone metabolic process | 139 | 15462 | 16 | 2 | 0.008865634 | 0.071182939 | WNT4, BMP2 |
| upregulated | GO:0002360 | T cell lineage commitment | 139 | 15462 | 16 | 2 | 0.008865634 | 0.071182939 | IL6, IRF4 |
| upregulated | GO:2000066 | positive regulation of cortisol biosynthetic process | 139 | 15462 | 1 | 1 | 0.008989781 | 0.071182939 | WNT4 |
| upregulated | GO:1900139 | negative regulation of arachidonic acid secretion | 139 | 15462 | 1 | 1 | 0.008989781 | 0.071182939 | PLA2R1 |
| upregulated | GO:0051042 | negative regulation of calcium-independent cell-cell adhesion | 139 | 15462 | 1 | 1 | 0.008989781 | 0.071182939 | BMP2 |
| upregulated | GO:1901231 | positive regulation of non-canonical Wnt signaling pathway via JNK cascade | 139 | 15462 | 1 | 1 | 0.008989781 | 0.071182939 | NKD1 |
| upregulated | GO:2000590 | negative regulation of metanephric mesenchymal cell migration | 139 | 15462 | 1 | 1 | 0.008989781 | 0.071182939 | ADIPOQ |
| upregulated | GO:0060980 | cell migration involved in coronary vasculogenesis | 139 | 15462 | 1 | 1 | 0.008989781 | 0.071182939 | TBX5 |
| upregulated | GO:0045368 | positive regulation of interleukin-13 biosynthetic process | 139 | 15462 | 1 | 1 | 0.008989781 | 0.071182939 | IRF4 |

| | | | | | | | | | |
|---|---|---|---|---|---|---|---|---|---|
| upreg ulated | GO:00 03026 | regulation of systemic arterial blood pressure by aortic arch baroreceptor feedback | 139 | 154 62 | 1 | 1 | 0.00 898 978 1 | 0.07 118 293 9 | ASIC2 |
| upreg ulated | GO:20 00534 | positive regulation of renal albumin absorption | 139 | 154 62 | 1 | 1 | 0.00 898 978 1 | 0.07 118 293 9 | ADIPOQ |
| upreg ulated | GO:20 00181 | negative regulation of blood vessel morphogenesis | 139 | 154 62 | 1 | 1 | 0.00 898 978 1 | 0.07 118 293 9 | WNT4 |
| upreg ulated | GO:20 00584 | negative regulation of platelet-derived growth factor receptor-alpha signaling pathway | 139 | 154 62 | 1 | 1 | 0.00 898 978 1 | 0.07 118 293 9 | ADIPOQ |
| upreg ulated | GO:20 00583 | regulation of platelet-derived growth factor receptor-alpha signaling pathway | 139 | 154 62 | 1 | 1 | 0.00 898 978 1 | 0.07 118 293 9 | ADIPOQ |
| upreg ulated | GO:00 70994 | detection of oxidative stress | 139 | 154 62 | 1 | 1 | 0.00 898 978 1 | 0.07 118 293 9 | ADIPOQ |
| upreg ulated | GO:20 00180 | negative regulation of androgen biosynthetic process | 139 | 154 62 | 1 | 1 | 0.00 898 978 1 | 0.07 118 293 9 | WNT4 |
| upreg ulated | GO:00 45366 | regulation of interleukin-13 biosynthetic process | 139 | 154 62 | 1 | 1 | 0.00 898 978 1 | 0.07 118 293 9 | IRF4 |
| upreg ulated | GO:00 02121 | inter-male aggressive behavior | 139 | 154 62 | 1 | 1 | 0.00 898 978 1 | 0.07 118 293 9 | GCNT4 |
| upreg ulated | GO:19 02823 | negative regulation of late endosome to lysosome transport | 139 | 154 62 | 1 | 1 | 0.00 898 978 1 | 0.07 118 293 9 | LRRK2 |
| upreg ulated | GO:20 00477 | regulation of metanephric glomerular visceral epithelial cell development | 139 | 154 62 | 1 | 1 | 0.00 898 978 1 | 0.07 118 293 9 | ADIPOQ |
| upreg ulated | GO:19 02715 | positive regulation of interferon-gamma secretion | 139 | 154 62 | 1 | 1 | 0.00 898 978 1 | 0.07 118 293 9 | CD2 |
| upreg ulated | GO:19 02713 | regulation of interferon-gamma secretion | 139 | 154 62 | 1 | 1 | 0.00 898 978 1 | 0.07 118 293 9 | CD2 |
| upreg ulated | GO:00 60804 | positive regulation of Wnt signaling pathway by BMP signaling pathway | 139 | 154 62 | 1 | 1 | 0.00 898 978 1 | 0.07 118 293 9 | BMP2 |
| upreg ulated | GO:00 61485 | memory T cell proliferation | 139 | 154 62 | 1 | 1 | 0.00 898 978 | 0.07 118 293 | DOCK8 |

| | | | | | | | | | |
|---|---|---|---|---|---|---|---|---|---|
| | | | | | | | 1 | 9 | |
| upreg ulated | GO:19 01232 | regulation of convergent extension involved in axis elongation | 139 | 154 62 | 1 | 1 | 0.00 898 978 1 | 0.07 118 293 9 | NKD1 |
| upreg ulated | GO:00 30887 | positive regulation of myeloid dendritic cell activation | 139 | 154 62 | 1 | 1 | 0.00 898 978 1 | 0.07 118 293 9 | CD2 |
| upreg ulated | GO:00 31948 | positive regulation of glucocorticoid biosynthetic process | 139 | 154 62 | 1 | 1 | 0.00 898 978 1 | 0.07 118 293 9 | WNT4 |
| upreg ulated | GO:19 01233 | negative regulation of convergent extension involved in axis elongation | 139 | 154 62 | 1 | 1 | 0.00 898 978 1 | 0.07 118 293 9 | NKD1 |
| upreg ulated | GO:19 03125 | negative regulation of thioredoxin peroxidase activity by peptidyl-threonine phosphorylation | 139 | 154 62 | 1 | 1 | 0.00 898 978 1 | 0.07 118 293 9 | LRRK2 |
| upreg ulated | GO:19 01229 | regulation of non-canonical Wnt signaling pathway via JNK cascade | 139 | 154 62 | 1 | 1 | 0.00 898 978 1 | 0.07 118 293 9 | NKD1 |
| upreg ulated | GO:00 61369 | negative regulation of testicular blood vessel morphogenesis | 139 | 154 62 | 1 | 1 | 0.00 898 978 1 | 0.07 118 293 9 | WNT4 |
| upreg ulated | GO:00 71882 | phospholipase C-activating adrenergic receptor signaling pathway | 139 | 154 62 | 1 | 1 | 0.00 898 978 1 | 0.07 118 293 9 | ADRA2A |
| upreg ulated | GO:20 00225 | negative regulation of testosterone biosynthetic process | 139 | 154 62 | 1 | 1 | 0.00 898 978 1 | 0.07 118 293 9 | WNT4 |
| upreg ulated | GO:19 90256 | signal clustering | 139 | 154 62 | 1 | 1 | 0.00 898 978 1 | 0.07 118 293 9 | SEMA5A |
| upreg ulated | GO:00 31945 | positive regulation of glucocorticoid metabolic process | 139 | 154 62 | 1 | 1 | 0.00 898 978 1 | 0.07 118 293 9 | WNT4 |
| upreg ulated | GO:20 00478 | positive regulation of metanephric glomerular visceral epithelial cell development | 139 | 154 62 | 1 | 1 | 0.00 898 978 1 | 0.07 118 293 9 | ADIPOQ |
| upreg ulated | GO:00 01817 | regulation of cytokine production | 139 | 154 62 | 464 | 10 | 0.00 916 884 1 | 0.07 240 971 9 | BIRC3, TLR1, IL18R1, IRF4, RGCC, CD2, ADRA2A, IL6, CD36, ADIPOQ |
| upreg ulated | GO:00 61041 | regulation of wound healing | 139 | 154 62 | 92 | 4 | 0.00 949 | 0.07 479 | ADRA2A, WNT4, ASIC2, CD36 |

| | | | | | | | | | |
|---|---|---|---|---|---|---|---|---|---|
| | | | | | | | 584 1 | 532 1 | |
| upreg ulated | GO:00 09100 | glycoprotein metabolic process | 139 | 154 62 | 328 | 8 | 0.00 963 074 | 0.07 565 929 6 | GPC6, SDC2, GCNT4, GALNT5, BMP2, HS3ST3B1, GALNT14, CHSY3 |
| upreg ulated | GO:00 42306 | regulation of protein import into nucleus | 139 | 154 62 | 144 | 5 | 0.00 970 599 6 | 0.07 605 142 | IL6, MDFIC, IL18R1, SEMA5A, CD36 |
| upreg ulated | GO:00 42181 | ketone biosynthetic process | 139 | 154 62 | 17 | 2 | 0.00 998 894 2 | 0.07 666 704 6 | WNT4, BMP2 |
| upreg ulated | GO:00 34383 | low-density lipoprotein particle clearance | 139 | 154 62 | 17 | 2 | 0.00 998 894 2 | 0.07 666 704 6 | CD36, ADIPOQ |
| upreg ulated | GO:00 06704 | glucocorticoid biosynthetic process | 139 | 154 62 | 17 | 2 | 0.00 998 894 2 | 0.07 666 704 6 | WNT4, BMP2 |
| upreg ulated | GO:00 60039 | pericardium development | 139 | 154 62 | 17 | 2 | 0.00 998 894 2 | 0.07 666 704 6 | TBX5, BMP2 |
| upreg ulated | GO:00 61377 | mammary gland lobule development | 139 | 154 62 | 17 | 2 | 0.00 998 894 2 | 0.07 666 704 6 | TNFRSF11A, HOXA5 |
| upreg ulated | GO:00 60749 | mammary gland alveolus development | 139 | 154 62 | 17 | 2 | 0.00 998 894 2 | 0.07 666 704 6 | TNFRSF11A, HOXA5 |
| upreg ulated | GO:00 72207 | metanephric epithelium development | 139 | 154 62 | 17 | 2 | 0.00 998 894 2 | 0.07 666 704 6 | ADIPOQ, CALB1 |
| upreg ulated | GO:00 90495 | low-density lipoprotein particle disassembly | 139 | 154 62 | 17 | 2 | 0.00 998 894 2 | 0.07 666 704 6 | CD36, ADIPOQ |
| upreg ulated | GO:00 01819 | positive regulation of cytokine production | 139 | 154 62 | 265 | 7 | 0.01 008 97 | 0.07 724 282 9 | ADRA2A, CD2, BIRC3, IL6, IL18R1, CD36, ADIPOQ |
| upreg ulated | GO:00 42325 | regulation of phosphorylation | 139 | 154 62 | 110 5 | 18 | 0.01 015 281 4 | 0.07 752 823 1 | FAM129A, CBLC, FABP4, ADRA2A, MDFIC, CD36, LRRK2, BIRC3, GDF6, CCR1, PTPRC, BMP2, TNFRSF11A, RGCC, ITGB3, IL6, ADIPOQ, FOXO1 |
| upreg ulated | GO:00 61035 | regulation of cartilage development | 139 | 154 62 | 50 | 3 | 0.01 023 970 8 | 0.07 799 330 9 | EFEMP1, SHOX2, BMP2 |
| upreg ulated | GO:00 43405 | regulation of MAP kinase activity | 139 | 154 62 | 266 | 7 | 0.01 028 673 9 | 0.07 815 317 7 | ADRA2A, TNFRSF11A, LRRK2, MDFIC, CBLC, ADIPOQ, BMP2 |
| upreg ulated | GO:00 51336 | regulation of hydrolase activity | 139 | 154 62 | 110 8 | 18 | 0.01 042 223 | 0.07 898 260 9 | TAGAP, SOS1, ADRA2A, PLA2R1, DOCK8, LRRK2, BIRC3, NR4A1, PRKG1, PDE1A, RASGRF2, ANGPTL4, CAMK2D, BMP2, WNT4, IL6, TFPI2, ARHGAP42 |
| upreg ulated | GO:00 44057 | regulation of system process | 139 | 154 62 | 333 | 8 | 0.01 048 817 4 | 0.07 928 213 9 | ADRA2A, FBXO32, IL6, CELF2, ASIC2, GUCY1A3, ADIPOQ, CAMK2D |
| upreg ulated | GO:00 16358 | dendrite development | 139 | 154 62 | 147 | 5 | 0.01 054 954 6 | 0.07 954 569 9 | LRRK2, PRKG1, PDLIM5, SDC2, DCLK1 |
| upreg | GO:00 | lipid localization | 139 | 154 | 268 | 7 | 0.01 | 0.08 | TNFRSF11A, ITGB3, ATP11A, PLA2R1, |

| | | | | | | | | | |
|---|---|---|---|---|---|---|---|---|---|
| ulated | 10876 | | | 62 | | | 068 922 9 | 019 593 8 | IL6, CD36, ADIPOQ |
| upreg ulated | GO:00 42692 | muscle cell differentiation | 139 | 154 62 | 268 | 7 | 0.01 068 922 9 | 0.08 019 593 8 | FBXO40, WNT4, PRDM6, TBX5, MEGF10, SHOX2, BMP2 |
| upreg ulated | GO:00 10717 | regulation of epithelial to mesenchymal transition | 139 | 154 62 | 51 | 3 | 0.01 080 928 4 | 0.08 069 318 4 | RGCC, TBX5, BMP2 |
| upreg ulated | GO:00 72009 | nephron epithelium development | 139 | 154 62 | 51 | 3 | 0.01 080 928 4 | 0.08 069 318 4 | WNT4, ADIPOQ, CALB1 |
| upreg ulated | GO:00 01503 | ossification | 139 | 154 62 | 335 | 8 | 0.01 084 657 8 | 0.08 077 066 8 | TNFRSF11A, WNT4, IL6, DLX5, SHOX2, CCR1, BMP2, SATB2 |
| upreg ulated | GO:19 00180 | regulation of protein localization to nucleus | 139 | 154 62 | 149 | 5 | 0.01 113 902 5 | 0.08 257 828 2 | IL6, MDFIC, IL18R1, SEMA5A, CD36 |
| upreg ulated | GO:00 03171 | atrioventricular valve development | 139 | 154 62 | 18 | 2 | 0.01 117 187 | 0.08 257 828 2 | TBX5, BMP2 |
| upreg ulated | GO:00 10894 | negative regulation of steroid biosynthetic process | 139 | 154 62 | 18 | 2 | 0.01 117 187 | 0.08 257 828 2 | WNT4, BMP2 |
| upreg ulated | GO:00 02757 | immune response-activating signal transduction | 139 | 154 62 | 337 | 8 | 0.01 121 399 5 | 0.08 268 599 2 | LCP2, BIRC3, TLR1, NFATC2, IRF4, CD36, PTPRC, FYB |
| upreg ulated | GO:00 16310 | phosphorylation | 139 | 154 62 | 173 3 | 25 | 0.01 132 333 4 | 0.08 281 361 6 | FAM129A, CBLC, DCLK1, FABP4, ADRA2A, EFEMP1, MDFIC, CD36, LRRK2, NKD1, BIRC3, GDF6, PRKG1, CCR1, BMP2, FYB, CAMK2D, PTPRC, TNFRSF11A, ITGB3, RGCC, WNT4, IL6, FOXO1, ADIPOQ |
| upreg ulated | GO:00 51384 | response to glucocorticoid | 139 | 154 62 | 97 | 4 | 0.01 137 897 8 | 0.08 281 361 6 | PAPPA, IL6, BCHE, ADIPOQ |
| upreg ulated | GO:00 48814 | regulation of dendrite morphogenesis | 139 | 154 62 | 52 | 3 | 0.01 139 687 6 | 0.08 281 361 6 | LRRK2, PDLIM5, SDC2 |
| upreg ulated | GO:00 06027 | glycosaminoglyc an catabolic process | 139 | 154 62 | 52 | 3 | 0.01 139 687 6 | 0.08 281 361 6 | GPC6, PRELP, SDC2 |
| upreg ulated | GO:00 30101 | natural killer cell activation | 139 | 154 62 | 52 | 3 | 0.01 139 687 6 | 0.08 281 361 6 | CD2, SLAMF7, IL18R1 |
| upreg ulated | GO:00 06026 | aminoglycan catabolic process | 139 | 154 62 | 52 | 3 | 0.01 139 687 6 | 0.08 281 361 6 | GPC6, PRELP, SDC2 |
| upreg ulated | GO:00 70372 | regulation of ERK1 and ERK2 cascade | 139 | 154 62 | 151 | 5 | 0.01 175 062 8 | 0.08 517 786 3 | TNFRSF11A, IL6, CCR1, ADIPOQ, BMP2 |
| upreg ulated | GO:00 12501 | programmed cell death | 139 | 154 62 | 165 1 | 24 | 0.01 199 569 1 | 0.08 674 474 3 | NR4A3, SOS1, SEMA5A, CD2, HOXA5, FEM1B, BNIP3L, LRRK2, BIRC3, NR4A1, GDF6, NPTX1, RASGRF2, PDE1A, ANGPTL4, PNMA2, BMP2, RGCC, WNT4, IL6, TBX5, ASIC2, FOXO1, ADIPOQ |

| | | | | | | | | | |
|---|---|---|---|---|---|---|---|---|---|
| upregulated | GO:0080135 | regulation of cellular response to stress | 139 | 15462 | 342 | 8 | 0.012172828 | 0.08781408 | TNFRSF11A, NPAS2, MDFIC, PLA2R1, FEM1B, CD36, BMP2, FOXO1 |
| upregulated | GO:0019932 | second-messenger-mediated signaling | 139 | 15462 | 343 | 8 | 0.012371631 | 0.088284405 | ADRA2A, CXCL9, CDH13, MCTP1, CD36, CCR1, GUCY1A3, CAMK2D |
| upregulated | GO:0045742 | positive regulation of epidermal growth factor receptor signaling pathway | 139 | 15462 | 19 | 2 | 0.012413272 | 0.088284405 | ADRA2A, SOS1 |
| upregulated | GO:2000352 | negative regulation of endothelial cell apoptotic process | 139 | 15462 | 19 | 2 | 0.012413272 | 0.088284405 | SEMA5A, ANGPTL4 |
| upregulated | GO:0045932 | negative regulation of muscle contraction | 139 | 15462 | 19 | 2 | 0.012413272 | 0.088284405 | ADRA2A, GUCY1A3 |
| upregulated | GO:0007263 | nitric oxide mediated signal transduction | 139 | 15462 | 19 | 2 | 0.012413272 | 0.088284405 | CD36, GUCY1A3 |
| upregulated | GO:0098602 | single organism cell adhesion | 139 | 15462 | 276 | 7 | 0.012414535 | 0.088284405 | RGCC, CD2, WNT4, MEGF10, DPP4, ADIPOQ, BMP2 |
| upregulated | GO:0031401 | positive regulation of protein modification process | 139 | 15462 | 797 | 14 | 0.012538238 | 0.088953318 | FAM129A, LRRK2, BIRC3, GDF6, BMP2, PTPRC, RGCC, TNFRSF11A, ADRA2A, ITGB3, IL6, MDFIC, CD36, ADIPOQ |
| upregulated | GO:0048520 | positive regulation of behavior | 139 | 15462 | 100 | 4 | 0.012616379 | 0.089157789 | IL6, CDH13, SEMA5A, CCR1 |
| upregulated | GO:0002040 | sprouting angiogenesis | 139 | 15462 | 54 | 3 | 0.012626478 | 0.089157789 | NR4A1, CDH13, SEMA5A |
| upregulated | GO:0009628 | response to abiotic stimulus | 139 | 15462 | 880 | 15 | 0.012734503 | 0.08970949 | GPC6, SDC2, ANGPTL4, DPP4, BMP2, RGCC, TNFRSF11A, IL6, ERCC1, ASIC2, TFEC, FOSL2, PIEZO2, ADIPOQ, FOXO1 |
| upregulated | GO:0016266 | O-glycan processing | 139 | 15462 | 55 | 3 | 0.013268655 | 0.093035595 | GALNT14, GCNT4, GALNT5 |
| upregulated | GO:0046888 | negative regulation of hormone secretion | 139 | 15462 | 55 | 3 | 0.013268655 | 0.093035595 | ADRA2A, IL6, ADIPOQ |
| upregulated | GO:0002764 | immune response-regulating signaling pathway | 139 | 15462 | 348 | 8 | 0.013401719 | 0.093046824 | LCP2, BIRC3, TLR1, NFATC2, IRF4, CD36, PTPRC, FYB |
| upregulated | GO:0007411 | axon guidance | 139 | 15462 | 348 | 8 | 0.013401719 | 0.093046824 | NR4A3, DLX5, GFRA1, SEMA5A, SOS1, PTPRC, ITGB3, CNTN1 |
| upregulated | GO:0097485 | neuron projection guidance | 139 | 15462 | 348 | 8 | 0.013401719 | 0.093046824 | NR4A3, DLX5, GFRA1, SEMA5A, SOS1, PTPRC, ITGB3, CNTN1 |
| upregulated | GO:0002682 | regulation of immune system | 139 | 15462 | 969 | 16 | 0.01348 | 0.09304 | SLAMF7, BIRC3, TLR1, IRF4, DPP4, CCR1, FYB, PTPRC, RGCC, CD2, LCP2, |

| | | | | | | | | | |
|---|---|---|---|---|---|---|---|---|---|
| | | process | | | | | 063 8 | 682 4 | IL6, HOXA5, NFATC2, CD36, ADIPOQ |
| upreg ulated | GO:00 45667 | regulation of osteoblast differentiation | 139 | 154 62 | 102 | 4 | 0.01 348 729 4 | 0.09 304 682 4 | WNT4, IL6, DLX5, BMP2 |
| upreg ulated | GO:00 51147 | regulation of muscle cell differentiation | 139 | 154 62 | 102 | 4 | 0.01 348 729 4 | 0.09 304 682 4 | PRDM6, MEGF10, SHOX2, BMP2 |
| upreg ulated | GO:00 45732 | positive regulation of protein catabolic process | 139 | 154 62 | 102 | 4 | 0.01 348 729 4 | 0.09 304 682 4 | ADRA2A, LRRK2, NKD1, FOXO1 |
| upreg ulated | GO:00 01655 | urogenital system development | 139 | 154 62 | 281 | 7 | 0.01 359 009 7 | 0.09 309 899 9 | WNT4, LRRK2, FEM1B, GCNT4, CALB1, ADIPOQ, BMP2 |
| upreg ulated | GO:00 43410 | positive regulation of MAPK cascade | 139 | 154 62 | 349 | 8 | 0.01 361 506 4 | 0.09 309 899 9 | LRRK2, CCR1, BMP2, TNFRSF11A, ADRA2A, IL6, MDFIC, CD36 |
| upreg ulated | GO:00 06111 | regulation of gluconeogenesis | 139 | 154 62 | 20 | 2 | 0.01 371 201 5 | 0.09 309 899 9 | IL6, ADIPOQ |
| upreg ulated | GO:00 46885 | regulation of hormone biosynthetic process | 139 | 154 62 | 20 | 2 | 0.01 371 201 5 | 0.09 309 899 9 | WNT4, BMP2 |
| upreg ulated | GO:19 01186 | positive regulation of ERBB signaling pathway | 139 | 154 62 | 20 | 2 | 0.01 371 201 5 | 0.09 309 899 9 | ADRA2A, SOS1 |
| upreg ulated | GO:00 45939 | negative regulation of steroid metabolic process | 139 | 154 62 | 20 | 2 | 0.01 371 201 5 | 0.09 309 899 9 | WNT4, BMP2 |
| upreg ulated | GO:00 61001 | regulation of dendritic spine morphogenesis | 139 | 154 62 | 20 | 2 | 0.01 371 201 5 | 0.09 309 899 9 | LRRK2, PDLIM5 |
| upreg ulated | GO:00 19915 | lipid storage | 139 | 154 62 | 56 | 3 | 0.01 392 918 3 | 0.09 435 999 8 | ITGB3, IL6, CD36 |
| upreg ulated | GO:00 31960 | response to corticosteroid | 139 | 154 62 | 104 | 4 | 0.01 439 561 1 | 0.09 717 995 5 | PAPPA, IL6, BCHE, ADIPOQ |
| upreg ulated | GO:00 32880 | regulation of protein localization | 139 | 154 62 | 424 | 9 | 0.01 444 223 | 0.09 717 995 5 | TLR1, IL18R1, SEMA5A, CD2, RGCC, IL6, MDFIC, CD36, ADIPOQ |
| upreg ulated | GO:00 65009 | regulation of molecular function | 139 | 154 62 | 242 4 | 32 | 0.01 444 260 6 | 0.09 717 995 5 | TAGAP, CBLC, IRF4, FABP4, ADRA2A, IL18R1, RASGRF2, CAMK2D, ITGB3, RGCC, TFPI2, ARHGAP42, SOS1, RUNX1T1, MDFIC, PLA2R1, PPP1R3B, FEM1B, ALOX5AP, DOCK8, LRRK2, BIRC3, PRKG1, NR4A1, PDE1A, ANGPTL4, BMP2, PTPRC, TNFRSF11A, WNT4, IL6, ADIPOQ |
| upreg ulated | GO:00 45833 | negative regulation of lipid metabolic process | 139 | 154 62 | 57 | 3 | 0.01 460 812 8 | 0.09 807 381 1 | ADRA2A, WNT4, BMP2 |
| upreg ulated | GO:00 02285 | lymphocyte activation involved in immune response | 139 | 154 62 | 105 | 4 | 0.01 486 395 4 | 0.09 937 587 2 | IL6, ERCC1, IL18R1, IRF4 |

| | | | | | | | | | |
|---|---|---|---|---|---|---|---|---|---|
| upreg ulated | GO:00 31399 | regulation of protein modification process | 139 | 154 62 | 115 1 | 18 | 0.01 495 358 | 0.09 937 587 2 | FAM129A, CBLC, FABP4, ADRA2A, MDFIC, FEM1B, CD36, LRRK2, BIRC3, GDF6, CAMK2D, PTPRC, BMP2, TNFRSF11A, ITGB3, RGCC, IL6, ADIPOQ |
| upreg ulated | GO:00 19222 | regulation of metabolic process | 139 | 154 62 | 579 0 | 65 | 0.01 501 209 1 | 0.09 937 587 2 | TAGAP, FAM129A, SHOX2, IRF4, FABP4, TNNI2, TFEC, NPAS2, ASIC2, FOXO1, ARHGAP42, CNTN1, TLR1, SOS1, PLA2R1, MDFIC, FEM1B, ALOX5AP, FOSL2, CD36, SATB2, ZFHX4, PRKG1, PDE1A, GUCY1A3, TNFRSF11A, IL6, TBX5, CBLC, ADRA2A, HOXA5, IGJ, NFATC2, NKD1, GDF6, IL18R1, RASGRF2, DHX34, CAMK2D, HIVEP3, RGCC, ITGB3, PRDM6, BNC2, ERCC1, CXCL9, TFPI2, NR4A3, RFX2, RUNX1T1, EFEMP1, PPP1R3B, DOCK8, LRRK2, BIRC3, NR4A1, DLX5, CDH13, ANGPTL4, PTPRC, CCR1, BMP2, WNT4, NAMPT, ADIPOQ |
| upreg ulated | GO:00 71772 | response to BMP | 139 | 154 62 | 21 | 2 | 0.01 506 698 5 | 0.09 937 587 2 | DLX5, BMP2 |
| upreg ulated | GO:00 90075 | relaxation of muscle | 139 | 154 62 | 21 | 2 | 0.01 506 698 5 | 0.09 937 587 2 | GUCY1A3, CAMK2D |
| upreg ulated | GO:00 50805 | negative regulation of synaptic transmission | 139 | 154 62 | 21 | 2 | 0.01 506 698 5 | 0.09 937 587 2 | BCHE, ADIPOQ |
| upreg ulated | GO:00 42745 | circadian sleep/wake cycle | 139 | 154 62 | 21 | 2 | 0.01 506 698 5 | 0.09 937 587 2 | NPAS2, IL6 |
| upreg ulated | GO:00 71773 | cellular response to BMP stimulus | 139 | 154 62 | 21 | 2 | 0.01 506 698 5 | 0.09 937 587 2 | DLX5, BMP2 |
| upreg ulated | GO:00 06606 | protein import into nucleus | 139 | 154 62 | 222 | 6 | 0.01 516 990 8 | 0.09 939 933 2 | IL6, MDFIC, IL18R1, SEMA5A, CD36, FYB |
| upreg ulated | GO:00 44744 | protein targeting to nucleus | 139 | 154 62 | 222 | 6 | 0.01 516 990 8 | 0.09 939 933 2 | IL6, MDFIC, IL18R1, SEMA5A, CD36, FYB |
| upreg ulated | GO:19 02593 | single-organism nuclear import | 139 | 154 62 | 222 | 6 | 0.01 516 990 8 | 0.09 939 933 2 | IL6, MDFIC, IL18R1, SEMA5A, CD36, FYB |
| upreg ulated | GO:00 30204 | chondroitin sulfate metabolic process | 139 | 154 62 | 58 | 3 | 0.01 530 555 1 | 0.10 006 962 3 | GPC6, CHSY3, SDC2 |
| upreg ulated | GO:00 46822 | regulation of nucleocytoplasmi c transport | 139 | 154 62 | 163 | 5 | 0.01 590 481 7 | 0.10 161 153 8 | IL6, MDFIC, IL18R1, SEMA5A, CD36 |
| upreg ulated | GO:00 43149 | stress fiber assembly | 139 | 154 62 | 59 | 3 | 0.01 602 150 3 | 0.10 161 153 8 | RGCC, ELN, WNT4 |
| upreg ulated | GO:00 51170 | nuclear import | 139 | 154 62 | 225 | 6 | 0.01 610 584 | 0.10 161 153 8 | IL6, MDFIC, IL18R1, SEMA5A, CD36, FYB |
| upreg ulated | GO:00 08219 | cell death | 139 | 154 62 | 188 0 | 26 | 0.01 627 906 | 0.10 161 153 | NR4A3, SOS1, SEMA5A, CD2, HOXA5, FEM1B, BNIP3L, FOSL2, LRRK2, NPAS2, BIRC3, NR4A1, GDF6, NPTX1, |

| | | | | | | | | | |
|---|---|---|---|---|---|---|---|---|---|
| | | | | | | | 9 | 8 | RASGRF2, PDE1A, ANGPTL4, PNMA2, BMP2, RGCC, WNT4, IL6, TBX5, ASIC2, FOXO1, ADIPOQ |
| upreg ulated | GO:00 70371 | ERK1 and ERK2 cascade | 139 | 154 62 | 164 | 5 | 0.01 628 987 5 | 0.10 161 153 8 | TNFRSF11A, IL6, CCR1, ADIPOQ, BMP2 |
| upreg ulated | GO:00 02440 | production of molecular mediator of immune response | 139 | 154 62 | 108 | 4 | 0.01 632 643 4 | 0.10 161 153 8 | IL6, ERCC1, CD36, PTPRC |
| upreg ulated | GO:20 00191 | regulation of fatty acid transport | 139 | 154 62 | 22 | 2 | 0.01 647 708 | 0.10 161 153 8 | TNFRSF11A, PLA2R1 |
| upreg ulated | GO:00 55024 | regulation of cardiac muscle tissue development | 139 | 154 62 | 22 | 2 | 0.01 647 708 | 0.10 161 153 8 | TBX5, BMP2 |
| upreg ulated | GO:00 03179 | heart valve morphogenesis | 139 | 154 62 | 22 | 2 | 0.01 647 708 | 0.10 161 153 8 | TBX5, BMP2 |
| upreg ulated | GO:00 32892 | positive regulation of organic acid transport | 139 | 154 62 | 22 | 2 | 0.01 647 708 | 0.10 161 153 8 | TNFRSF11A, PLA2R1 |
| upreg ulated | GO:00 45622 | regulation of T-helper cell differentiation | 139 | 154 62 | 22 | 2 | 0.01 647 708 | 0.10 161 153 8 | IL6, IRF4 |
| upreg ulated | GO:00 32967 | positive regulation of collagen biosynthetic process | 139 | 154 62 | 22 | 2 | 0.01 647 708 | 0.10 161 153 8 | RGCC, WNT4 |
| upreg ulated | GO:00 16265 | death | 139 | 154 62 | 188 3 | 26 | 0.01 658 673 9 | 0.10 161 153 8 | NR4A3, SOS1, SEMA5A, CD2, HOXA5, FEM1B, BNIP3L, FOSL2, LRRK2, NPAS2, BIRC3, NR4A1, GDF6, NPTX1, RASGRF2, PDE1A, ANGPTL4, PNMA2, BMP2, RGCC, WNT4, IL6, TBX5, ASIC2, FOXO1, ADIPOQ |
| upreg ulated | GO:00 30278 | regulation of ossification | 139 | 154 62 | 165 | 5 | 0.01 668 110 2 | 0.10 161 153 8 | WNT4, IL6, DLX5, CCR1, BMP2 |
| upreg ulated | GO:00 07266 | Rho protein signal transduction | 139 | 154 62 | 227 | 6 | 0.01 675 157 4 | 0.10 161 153 8 | ADRA2A, WNT4, CDH13, RASGRF2, SOS1, COL1A2 |
| upreg ulated | GO:00 50654 | chondroitin sulfate proteoglycan metabolic process | 139 | 154 62 | 60 | 3 | 0.01 675 603 4 | 0.10 161 153 8 | GPC6, CHSY3, SDC2 |
| upreg ulated | GO:00 43255 | regulation of carbohydrate biosynthetic process | 139 | 154 62 | 60 | 3 | 0.01 675 603 4 | 0.10 161 153 8 | IL6, PPP1R3B, ADIPOQ |
| upreg ulated | GO:00 02286 | T cell activation involved in immune response | 139 | 154 62 | 60 | 3 | 0.01 675 603 4 | 0.10 161 153 8 | IL6, IL18R1, IRF4 |
| upreg ulated | GO:00 03012 | muscle system process | 139 | 154 62 | 294 | 7 | 0.01 701 812 3 | 0.10 161 153 8 | ADRA2A, TNNI2, FBXO32, CHRNA1, LMOD1, GUCY1A3, CAMK2D |
| upreg ulated | GO:00 51607 | defense response to virus | 139 | 154 62 | 166 | 5 | 0.01 707 853 | 0.10 161 153 | IL6, BIRC3, BNIP3L, CXCL9, PTPRC |

| | | | | | | | | | |
|---|---|---|---|---|---|---|---|---|---|
| | | | | | | | 6 | 8 | |
| upregulated | GO:0001932 | regulation of protein phosphorylation | 139 | 15462 | 912 | 15 | 0.0171161 | 0.101611538 | FAM129A, LRRK2, CBLC, GDF6, BMP2, FABP4, PTPRC, RGCC, TNFRSF11A, ITGB3, ADRA2A, IL6, MDFIC, CD36, ADIPOQ |
| upregulated | GO:0002673 | regulation of acute inflammatory response | 139 | 15462 | 61 | 3 | 0.017509183 | 0.101611538 | TNFRSF11A, IL6, ALOX5AP |
| upregulated | GO:0050727 | regulation of inflammatory response | 139 | 15462 | 230 | 6 | 0.017753388 | 0.101611538 | TNFRSF11A, IL6, BIRC3, ALOX5AP, ADIPOQ, FABP4 |
| upregulated | GO:0006468 | protein phosphorylation | 139 | 15462 | 1349 | 20 | 0.017831568 | 0.101611538 | FAM129A, CBLC, DCLK1, FABP4, ADRA2A, EFEMP1, MDFIC, CD36, LRRK2, GDF6, PRKG1, FYB, CAMK2D, PTPRC, BMP2, TNFRSF11A, ITGB3, RGCC, IL6, ADIPOQ |
| upregulated | GO:0045082 | positive regulation of interleukin-10 biosynthetic process | 139 | 15462 | 2 | 1 | 0.017899323 | 0.101611538 | IRF4 |
| upregulated | GO:0003218 | cardiac left ventricle formation | 139 | 15462 | 2 | 1 | 0.017899323 | 0.101611538 | TBX5 |
| upregulated | GO:2000532 | regulation of renal albumin absorption | 139 | 15462 | 2 | 1 | 0.017899323 | 0.101611538 | ADIPOQ |
| upregulated | GO:2000019 | negative regulation of male gonad development | 139 | 15462 | 2 | 1 | 0.017899323 | 0.101611538 | WNT4 |
| upregulated | GO:0030237 | female sex determination | 139 | 15462 | 2 | 1 | 0.017899323 | 0.101611538 | WNT4 |
| upregulated | GO:0071848 | positive regulation of ERK1 and ERK2 cascade via TNFSF11-mediated signaling | 139 | 15462 | 2 | 1 | 0.017899323 | 0.101611538 | TNFRSF11A |
| upregulated | GO:0070473 | negative regulation of uterine smooth muscle contraction | 139 | 15462 | 2 | 1 | 0.017899323 | 0.101611538 | ADRA2A |
| upregulated | GO:0060086 | circadian temperature homeostasis | 139 | 15462 | 2 | 1 | 0.017899323 | 0.101611538 | TNFRSF11A |
| upregulated | GO:0060748 | tertiary branching involved in mammary gland duct morphogenesis | 139 | 15462 | 2 | 1 | 0.017899323 | 0.101611538 | WNT4 |
| upregulated | GO:0042495 | detection of triacyl bacterial lipopeptide | 139 | 15462 | 2 | 1 | 0.017899323 | 0.101611538 | TLR1 |
| upregulated | GO:0060979 | vasculogenesis involved in coronary vascular morphogenesis | 139 | 15462 | 2 | 1 | 0.017899323 | 0.101611538 | TBX5 |
| upreg | GO:20 | positive | 139 | 154 | 2 | 1 | 0.01 | 0.10 | IL6 |

| | | | | | | | | | |
|---|---|---|---|---|---|---|---|---|---|
| ulated | 00676 | regulation of type B pancreatic cell apoptotic process | | 62 | | | 789 932 3 | 161 153 8 | |
| upreg ulated | GO:00 52551 | response to defense-related nitric oxide production by other organism involved in symbiotic interaction | 139 | 154 62 | 2 | 1 | 0.01 789 932 3 | 0.10 161 153 8 | GUCY1A3 |
| upreg ulated | GO:20 00224 | regulation of testosterone biosynthetic process | 139 | 154 62 | 2 | 1 | 0.01 789 932 3 | 0.10 161 153 8 | WNT4 |
| upreg ulated | GO:00 14016 | neuroblast differentiation | 139 | 154 62 | 2 | 1 | 0.01 789 932 3 | 0.10 161 153 8 | BCHE |
| upreg ulated | GO:00 51040 | regulation of calcium-independent cell-cell adhesion | 139 | 154 62 | 2 | 1 | 0.01 789 932 3 | 0.10 161 153 8 | BMP2 |
| upreg ulated | GO:00 90272 | negative regulation of fibroblast growth factor production | 139 | 154 62 | 2 | 1 | 0.01 789 932 3 | 0.10 161 153 8 | RGCC |
| upreg ulated | GO:00 02384 | hepatic immune response | 139 | 154 62 | 2 | 1 | 0.01 789 932 3 | 0.10 161 153 8 | IL6 |
| upreg ulated | GO:19 02774 | late endosome to lysosome transport | 139 | 154 62 | 2 | 1 | 0.01 789 932 3 | 0.10 161 153 8 | LRRK2 |
| upreg ulated | GO:00 42231 | interleukin-13 biosynthetic process | 139 | 154 62 | 2 | 1 | 0.01 789 932 3 | 0.10 161 153 8 | IRF4 |
| upreg ulated | GO:00 97017 | renal protein absorption | 139 | 154 62 | 2 | 1 | 0.01 789 932 3 | 0.10 161 153 8 | ADIPOQ |
| upreg ulated | GO:00 71810 | regulation of fever generation by regulation of prostaglandin secretion | 139 | 154 62 | 2 | 1 | 0.01 789 932 3 | 0.10 161 153 8 | TNFRSF11A |
| upreg ulated | GO:00 32349 | positive regulation of aldosterone biosynthetic process | 139 | 154 62 | 2 | 1 | 0.01 789 932 3 | 0.10 161 153 8 | WNT4 |
| upreg ulated | GO:00 71847 | TNFSF11-mediated signaling pathway | 139 | 154 62 | 2 | 1 | 0.01 789 932 3 | 0.10 161 153 8 | TNFRSF11A |
| upreg ulated | GO:19 00138 | negative regulation of phospholipase A2 activity | 139 | 154 62 | 2 | 1 | 0.01 789 932 3 | 0.10 161 153 8 | PLA2R1 |
| upreg ulated | GO:20 00473 | positive regulation of hematopoietic stem cell migration | 139 | 154 62 | 2 | 1 | 0.01 789 932 3 | 0.10 161 153 8 | PTPRC |
| upreg ulated | GO:00 52565 | response to defense-related host nitric oxide production | 139 | 154 62 | 2 | 1 | 0.01 789 932 3 | 0.10 161 153 8 | GUCY1A3 |

| | | | | | | | | | |
|---|---|---|---|---|---|---|---|---|---|
| upregulated | GO:1902822 | regulation of late endosome to lysosome transport | 139 | 15462 | 2 | 1 | 0.017899323 | 0.101611538 | LRRK2 |
| upregulated | GO:0071725 | response to triacyl bacterial lipopeptide | 139 | 15462 | 2 | 1 | 0.017899323 | 0.101611538 | TLR1 |
| upregulated | GO:0090249 | regulation of cell motility involved in somitogenic axis elongation | 139 | 15462 | 2 | 1 | 0.017899323 | 0.101611538 | NKD1 |
| upregulated | GO:2000471 | regulation of hematopoietic stem cell migration | 139 | 15462 | 2 | 1 | 0.017899323 | 0.101611538 | PTPRC |
| upregulated | GO:0019442 | tryptophan catabolic process to acetyl-CoA | 139 | 15462 | 2 | 1 | 0.017899323 | 0.101611538 | TDO2 |
| upregulated | GO:0071727 | cellular response to triacyl bacterial lipopeptide | 139 | 15462 | 2 | 1 | 0.017899323 | 0.101611538 | TLR1 |
| upregulated | GO:0097018 | renal albumin absorption | 139 | 15462 | 2 | 1 | 0.017899323 | 0.101611538 | ADIPOQ |
| upregulated | GO:0032346 | positive regulation of aldosterone metabolic process | 139 | 15462 | 2 | 1 | 0.017899323 | 0.101611538 | WNT4 |
| upregulated | GO:0003331 | positive regulation of extracellular matrix constituent secretion | 139 | 15462 | 2 | 1 | 0.017899323 | 0.101611538 | RGCC |
| upregulated | GO:0090247 | cell motility involved in somitogenic axis elongation | 139 | 15462 | 2 | 1 | 0.017899323 | 0.101611538 | NKD1 |
| upregulated | GO:0003330 | regulation of extracellular matrix constituent secretion | 139 | 15462 | 2 | 1 | 0.017899323 | 0.101611538 | RGCC |
| upregulated | GO:0035655 | interleukin-18-mediated signaling pathway | 139 | 15462 | 2 | 1 | 0.017899323 | 0.101611538 | IL18R1 |
| upregulated | GO:0060764 | cell-cell signaling involved in mammary gland development | 139 | 15462 | 2 | 1 | 0.017899323 | 0.101611538 | HOXA5 |
| upregulated | GO:0071812 | positive regulation of fever generation by positive regulation of prostaglandin secretion | 139 | 15462 | 2 | 1 | 0.017899323 | 0.101611538 | TNFRSF11A |
| upregulated | GO:0035790 | platelet-derived growth factor receptor-alpha signaling pathway | 139 | 15462 | 2 | 1 | 0.017899323 | 0.101611538 | ADIPOQ |
| upregulated | GO:0010714 | positive regulation of collagen | 139 | 15462 | 23 | 2 | 0.01794121 | 0.10161153 | RGCC, WNT4 |

| | | | | | | | | | |
|---|---|---|---|---|---|---|---|---|---|
| | | metabolic process | | | | | 5 | 8 | |
| upregulated | GO:0043368 | positive T cell selection | 139 | 15462 | 23 | 2 | 0.017941215 | 0.101611538 | IL6, IRF4 |
| upregulated | GO:0001960 | negative regulation of cytokine-mediated signaling pathway | 139 | 15462 | 23 | 2 | 0.017941215 | 0.101611538 | ADIPOQ, PTPRC |
| upregulated | GO:0008211 | glucocorticoid metabolic process | 139 | 15462 | 23 | 2 | 0.017941215 | 0.101611538 | WNT4, BMP2 |
| upregulated | GO:0046903 | secretion | 139 | 15462 | 834 | 14 | 0.01794539 | 0.101611538 | LRRK2, TLR1, CCR1, RGCC, TNFRSF11A, CD2, ITGB3, ADRA2A, LCP2, IL6, PLA2R1, CBLN4, CD36, ADIPOQ |
| upregulated | GO:0001822 | kidney development | 139 | 15462 | 231 | 6 | 0.01809629 | 0.102273006 | WNT4, LRRK2, GCNT4, CALB1, ADIPOQ, BMP2 |
| upregulated | GO:0070374 | positive regulation of ERK1 and ERK2 cascade | 139 | 15462 | 112 | 4 | 0.018412549 | 0.103691634 | TNFRSF11A, IL6, CCR1, BMP2 |
| upregulated | GO:0071495 | cellular response to endogenous stimulus | 139 | 15462 | 837 | 14 | 0.01845167 | 0.103691634 | NR4A1, DLX5, PDE1A, CDH13, SOS1, BMP2, COL1A2, ADRA2A, WNT4, NAMPT, FOSL2, CD36, ADIPOQ, FOXO1 |
| upregulated | GO:0007264 | small GTPase mediated signal transduction | 139 | 15462 | 595 | 11 | 0.018479493 | 0.103691634 | TAGAP, LRRK2, RASGRF2, CDH13, SOS1, DEPDC7, COL1A2, ADRA2A, WNT4, RAB30, DOCK8 |
| upregulated | GO:0048878 | chemical homeostasis | 139 | 15462 | 755 | 13 | 0.018485513 | 0.103691634 | LRRK2, NPTX1, STEAP4, ANGPTL4, CCR1, CAMK2D, FABP4, PTPRC, ADRA2A, CXCL9, CALB1, ADIPOQ, FOXO1 |
| upregulated | GO:0042110 | T cell activation | 139 | 15462 | 370 | 8 | 0.018692286 | 0.104655877 | IL18R1, IRF4, DPP4, PTPRC, CD2, WNT4, IL6, DOCK8 |
| upregulated | GO:0018108 | peptidyl-tyrosine phosphorylation | 139 | 15462 | 300 | 7 | 0.01879065 | 0.104843656 | ADRA2A, EFEMP1, ITGB3, IL6, CBLC, CD36, ADIPOQ |
| upregulated | GO:0001667 | ameboidal cell migration | 139 | 15462 | 233 | 6 | 0.018795697 | 0.104843656 | RGCC, ITGB3, NR4A1, CDH13, DPP4, SEMA5A |
| upregulated | GO:0030500 | regulation of bone mineralization | 139 | 15462 | 63 | 3 | 0.019071475 | 0.106184593 | WNT4, CCR1, BMP2 |
| upregulated | GO:1902105 | regulation of leukocyte differentiation | 139 | 15462 | 171 | 5 | 0.01916013 | 0.106283825 | CD2, IL6, IRF4, CCR1, ADIPOQ |
| upregulated | GO:0042180 | cellular ketone metabolic process | 139 | 15462 | 171 | 5 | 0.01916013 | 0.106283825 | WNT4, IL6, TDO2, ADIPOQ, BMP2 |
| upregulated | GO:0048519 | negative regulation of biological process | 139 | 15462 | 3460 | 42 | 0.019211119 | 0.106323359 | FAM129A, CBLC, IRF4, SHOX2, FABP4, ADRA2A, HOXA5, BNIP3L, NPAS2, NKD1, DPP4, DHX34, CAMK2D, ITGB3, RGCC, PRDM6, ERCC1, ASIC2, CXCL9, BCHE, FOXO1, NR4A3, SEMA5A, EFEMP1, MDFIC, PLA2R1, CD36, SATB2, LRRK2, BIRC3, CDH13, |

| | | | | | | | | | |
|---|---|---|---|---|---|---|---|---|---|
| | | | | | | | | | ANGPTL4, PTPRC, GUCY1A3, BMP2, CCR1, WNT4, IL6, TBX5, RARRES1, SKAP2, ADIPOQ |
| upreg ulated | GO:00 19221 | cytokine-mediated signaling pathway | 139 | 154 62 | 372 | 8 | 0.01 923 811 5 | 0.10 632 335 9 | TNFRSF11A, IL6, IL18R1, IRF4, CCR1, ADIPOQ, PTPRC, CAMK2D |
| upreg ulated | GO:00 18212 | peptidyl-tyrosine modification | 139 | 154 62 | 302 | 7 | 0.01 940 931 9 | 0.10 686 892 9 | ADRA2A, EFEMP1, ITGB3, IL6, CBLC, CD36, ADIPOQ |
| upreg ulated | GO:00 07176 | regulation of epidermal growth factor-activated receptor activity | 139 | 154 62 | 24 | 2 | 0.01 945 831 9 | 0.10 686 892 9 | ADRA2A, CBLC |
| upreg ulated | GO:00 50974 | detection of mechanical stimulus involved in sensory perception | 139 | 154 62 | 24 | 2 | 0.01 945 831 9 | 0.10 686 892 9 | ASIC2, PIEZO2 |
| upreg ulated | GO:00 30098 | lymphocyte differentiation | 139 | 154 62 | 235 | 6 | 0.01 951 341 4 | 0.10 686 892 9 | CD2, WNT4, IL6, IL18R1, IRF4, PTPRC |
| upreg ulated | GO:00 50920 | regulation of chemotaxis | 139 | 154 62 | 114 | 4 | 0.01 951 488 6 | 0.10 686 892 9 | IL6, CDH13, SEMA5A, CCR1 |
| upreg ulated | GO:00 08015 | blood circulation | 139 | 154 62 | 374 | 8 | 0.01 979 528 | 0.10 820 698 7 | ADRA2A, ELN, CELF2, ASIC2, GUCY1A3, ADIPOQ, CAMK2D, COL1A2 |
| upreg ulated | GO:00 06869 | lipid transport | 139 | 154 62 | 236 | 6 | 0.01 987 920 2 | 0.10 827 929 1 | ATP11A, TNFRSF11A, ITGB3, PLA2R1, CD36, ADIPOQ |
| upreg ulated | GO:00 70167 | regulation of biomineral tissue development | 139 | 154 62 | 64 | 3 | 0.01 988 067 | 0.10 827 929 1 | WNT4, CCR1, BMP2 |
| upreg ulated | GO:00 45765 | regulation of angiogenesis | 139 | 154 62 | 173 | 5 | 0.02 003 750 3 | 0.10 893 577 | RGCC, IL6, HOXA5, ANGPTL4, SEMA5A |
| upreg ulated | GO:00 06915 | apoptotic process | 139 | 154 62 | 163 7 | 23 | 0.02 009 406 2 | 0.10 904 571 6 | NR4A3, SOS1, SEMA5A, CD2, HOXA5, FEM1B, BNIP3L, BIRC3, NR4A1, GDF6, NPTX1, RASGRF2, PDE1A, ANGPTL4, PNMA2, BMP2, RGCC, WNT4, IL6, TBX5, ASIC2, FOXO1, ADIPOQ |
| upreg ulated | GO:00 02520 | immune system development | 139 | 154 62 | 603 | 11 | 0.02 017 713 7 | 0.10 929 889 4 | IL18R1, IRF4, CCR1, PTPRC, TNFRSF11A, CD2, WNT4, IL6, HOXA5, ERCC1, ADIPOQ |
| upreg ulated | GO:00 01666 | response to hypoxia | 139 | 154 62 | 237 | 6 | 0.02 024 964 3 | 0.10 949 401 5 | RGCC, SDC2, ANGPTL4, DPP4, ADIPOQ, BMP2 |
| upreg ulated | GO:00 60341 | regulation of cellular localization | 139 | 154 62 | 765 | 13 | 0.02 035 833 5 | 0.10 951 981 6 | LRRK2, TLR1, IL18R1, SEMA5A, CAMK2D, RGCC, CD2, ADRA2A, MDFIC, IL6, CXCL9, CD36, ADIPOQ |
| upreg ulated | GO:00 09894 | regulation of catabolic process | 139 | 154 62 | 765 | 13 | 0.02 035 833 5 | 0.10 951 981 6 | TAGAP, LRRK2, NKD1, PRKG1, RASGRF2, SOS1, DHX34, ADRA2A, WNT4, PPP1R3B, ARHGAP42, DOCK8, FOXO1 |
| upreg ulated | GO:00 03013 | circulatory system process | 139 | 154 62 | 376 | 8 | 0.02 036 389 8 | 0.10 951 981 6 | ADRA2A, ELN, CELF2, ASIC2, GUCY1A3, ADIPOQ, CAMK2D, COL1A2 |
| upreg ulated | GO:00 42113 | B cell activation | 139 | 154 62 | 174 | 5 | 0.02 048 | 0.10 997 | IL6, ERCC1, NFATC2, SKAP2, PTPRC |

| | | | | | | | | | |
|---|---|---|---|---|---|---|---|---|---|
| | | | | | | | 591 4 | 894 1 | |
| upreg ulated | GO:00 01933 | negative regulation of protein phosphorylation | 139 | 154 62 | 238 | 6 | 0.02 062 476 2 | 0.11 031 185 8 | FAM129A, IL6, CBLC, PTPRC, ADIPOQ, FABP4 |
| upreg ulated | GO:00 02009 | morphogenesis of an epithelium | 139 | 154 62 | 377 | 8 | 0.02 065 253 8 | 0.11 031 185 8 | NKD1, SEMA5A, BMP2, WNT4, IL6, TBX5, FEM1B, HOXA5 |
| upreg ulated | GO:00 19219 | regulation of nucleobase-containing compound metabolic process | 139 | 154 62 | 408 5 | 48 | 0.02 065 820 2 | 0.11 031 185 8 | TAGAP, IRF4, SHOX2, FABP4, ADRA2A, TNNI2, HOXA5, NFATC2, TFEC, NPAS2, GDF6, IL18R1, RASGRF2, HIVEP3, DHX34, CAMK2D, RGCC, PRDM6, BNC2, ERCC1, CXCL9, ARHGAP42, FOXO1, NR4A3, SOS1, RFX2, RUNX1T1, EFEMP1, MDFIC, FOSL2, CD36, SATB2, DOCK8, LRRK2, ZFHX4, BIRC3, NR4A1, PRKG1, DLX5, CDH13, GUCY1A3, BMP2, TNFRSF11A, WNT4, IL6, TBX5, NAMPT, ADIPOQ |
| upreg ulated | GO:00 09719 | response to endogenous stimulus | 139 | 154 62 | 119 4 | 18 | 0.02 091 506 2 | 0.11 070 707 4 | PAPPA, NR4A3, NR4A1, DLX5, CDH13, PDE1A, SOS1, BMP2, COL1A2, ADRA2A, WNT4, IL6, NAMPT, FOSL2, BCHE, CD36, FOXO1, ADIPOQ |
| upreg ulated | GO:00 36293 | response to decreased oxygen levels | 139 | 154 62 | 239 | 6 | 0.02 100 458 3 | 0.11 070 707 4 | RGCC, SDC2, ANGPTL4, DPP4, ADIPOQ, BMP2 |
| upreg ulated | GO:00 51249 | regulation of lymphocyte activation | 139 | 154 62 | 307 | 7 | 0.02 101 831 2 | 0.11 070 707 4 | CD2, SLAMF7, IL6, NFATC2, IRF4, DPP4, PTPRC |
| upreg ulated | GO:00 32722 | positive regulation of chemokine production | 139 | 154 62 | 25 | 2 | 0.02 102 733 5 | 0.11 070 707 4 | IL6, ADIPOQ |
| upreg ulated | GO:00 19433 | triglyceride catabolic process | 139 | 154 62 | 25 | 2 | 0.02 102 733 5 | 0.11 070 707 4 | PLIN1, FABP4 |
| upreg ulated | GO:00 34113 | heterotypic cell-cell adhesion | 139 | 154 62 | 25 | 2 | 0.02 102 733 5 | 0.11 070 707 4 | CD2, ADIPOQ |
| upreg ulated | GO:00 32369 | negative regulation of lipid transport | 139 | 154 62 | 25 | 2 | 0.02 102 733 5 | 0.11 070 707 4 | ITGB3, PLA2R1 |
| upreg ulated | GO:00 60415 | muscle tissue morphogenesis | 139 | 154 62 | 25 | 2 | 0.02 102 733 5 | 0.11 070 707 4 | SHOX2, BMP2 |
| upreg ulated | GO:00 19725 | cellular homeostasis | 139 | 154 62 | 607 | 11 | 0.02 106 816 1 | 0.11 072 776 | GLRX, NPTX1, CHRNA1, CCR1, CAMK2D, PTPRC, ADRA2A, IL6, CXCL9, FOXO1, CALB1 |
| upreg ulated | GO:00 02526 | acute inflammatory response | 139 | 154 62 | 117 | 4 | 0.02 124 355 1 | 0.11 145 436 5 | ADRA2A, TNFRSF11A, IL6, ALOX5AP |
| upreg ulated | GO:00 40013 | negative regulation of locomotion | 139 | 154 62 | 176 | 5 | 0.02 140 235 4 | 0.11 209 156 1 | RGCC, WNT4, TBX5, SEMA5A, ADIPOQ |
| upreg ulated | GO:00 98542 | defense response to other organism | 139 | 154 62 | 309 | 7 | 0.02 168 722 7 | 0.11 338 566 1 | BIRC3, IL6, IGJ, BNIP3L, CXCL9, CD36, PTPRC |
| upreg ulated | GO:00 02377 | immunoglobulin production | 139 | 154 62 | 67 | 3 | 0.02 242 | 0.11 578 | IL6, ERCC1, PTPRC |

| | | | | | | | | | |
|---|---|---|---|---|---|---|---|---|---|
| | | | | | | | 065 2 | 247 5 | |
| upreg ulated | GO:00 46632 | alpha-beta T cell differentiation | 139 | 154 62 | 67 | 3 | 0.02 242 065 2 | 0.11 578 247 5 | IL6, IL18R1, IRF4 |
| upreg ulated | GO:00 15908 | fatty acid transport | 139 | 154 62 | 67 | 3 | 0.02 242 065 2 | 0.11 578 247 5 | TNFRSF11A, PLA2R1, CD36 |
| upreg ulated | GO:00 51707 | response to other organism | 139 | 154 62 | 613 | 11 | 0.02 245 888 9 | 0.11 578 247 5 | BIRC3, TLR1, DCLK1, GUCY1A3, PTPRC, TNFRSF11A, IL6, BNIP3L, IGJ, CXCL9, CD36 |
| upreg ulated | GO:00 43207 | response to external biotic stimulus | 139 | 154 62 | 613 | 11 | 0.02 245 888 9 | 0.11 578 247 5 | BIRC3, TLR1, DCLK1, GUCY1A3, PTPRC, TNFRSF11A, IL6, BNIP3L, IGJ, CXCL9, CD36 |
| upreg ulated | GO:00 72001 | renal system development | 139 | 154 62 | 243 | 6 | 0.02 257 137 2 | 0.11 578 247 5 | WNT4, LRRK2, GCNT4, CALB1, ADIPOQ, BMP2 |
| upreg ulated | GO:00 40007 | growth | 139 | 154 62 | 776 | 13 | 0.02 258 014 5 | 0.11 578 247 5 | NKD1, GDF6, CDH13, CHRNA1, DCLK1, SEMA5A, CAMK2D, BMP2, IL6, HOXA5, ERCC1, TBX5, CD36 |
| upreg ulated | GO:00 60997 | dendritic spine morphogenesis | 139 | 154 62 | 26 | 2 | 0.02 264 722 2 | 0.11 578 247 5 | LRRK2, PDLIM5 |
| upreg ulated | GO:00 10862 | positive regulation of pathway-restricted SMAD protein phosphorylation | 139 | 154 62 | 26 | 2 | 0.02 264 722 2 | 0.11 578 247 5 | GDF6, BMP2 |
| upreg ulated | GO:00 03338 | metanephros morphogenesis | 139 | 154 62 | 26 | 2 | 0.02 264 722 2 | 0.11 578 247 5 | WNT4, CALB1 |
| upreg ulated | GO:00 43370 | regulation of CD4-positive, alpha-beta T cell differentiation | 139 | 154 62 | 26 | 2 | 0.02 264 722 2 | 0.11 578 247 5 | IL6, IRF4 |
| upreg ulated | GO:00 03209 | cardiac atrium morphogenesis | 139 | 154 62 | 26 | 2 | 0.02 264 722 2 | 0.11 578 247 5 | TBX5, SHOX2 |
| upreg ulated | GO:00 48512 | circadian behavior | 139 | 154 62 | 26 | 2 | 0.02 264 722 2 | 0.11 578 247 5 | NPAS2, IL6 |
| upreg ulated | GO:00 50778 | positive regulation of immune response | 139 | 154 62 | 460 | 9 | 0.02 315 782 | 0.11 819 152 9 | RGCC, LCP2, BIRC3, TLR1, NFATC2, IRF4, CD36, PTPRC, FYB |
| upreg ulated | GO:00 01952 | regulation of cell-matrix adhesion | 139 | 154 62 | 68 | 3 | 0.02 330 480 4 | 0.11 858 831 2 | WNT4, CDH13, CD36 |
| upreg ulated | GO:00 08543 | fibroblast growth factor receptor signaling pathway | 139 | 154 62 | 180 | 5 | 0.02 331 459 7 | 0.11 858 831 2 | WNT4, NR4A1, PDE1A, SOS1, FOXO1 |
| upreg ulated | GO:00 60485 | mesenchyme development | 139 | 154 62 | 181 | 5 | 0.02 380 937 1 | 0.12 069 581 3 | RGCC, WNT4, BNC2, TBX5, BMP2 |
| upreg ulated | GO:00 51222 | positive regulation of protein transport | 139 | 154 62 | 181 | 5 | 0.02 380 937 | 0.12 069 581 | RGCC, CD2, IL6, IL18R1, SEMA5A |

| | | | | | | | | | |
|---|---|---|---|---|---|---|---|---|---|
| | | | | | | | 1 | 3 | |
| upregulated | GO:0030097 | hemopoiesis | 139 | 15462 | 541 | 10 | 0.024227305 | 0.121679816 | IL18R1, IRF4, CCR1, PTPRC, TNFRSF11A, CD2, WNT4, IL6, HOXA5, ADIPOQ |
| upregulated | GO:0046461 | neutral lipid catabolic process | 139 | 15462 | 27 | 2 | 0.024316952 | 0.121679816 | PLIN1, FABP4 |
| upregulated | GO:0003009 | skeletal muscle contraction | 139 | 15462 | 27 | 2 | 0.024316952 | 0.121679816 | TNNI2, CHRNA1 |
| upregulated | GO:0046464 | acylglycerol catabolic process | 139 | 15462 | 27 | 2 | 0.024316952 | 0.121679816 | PLIN1, FABP4 |
| upregulated | GO:0003197 | endocardial cushion development | 139 | 15462 | 27 | 2 | 0.024316952 | 0.121679816 | TBX5, BMP2 |
| upregulated | GO:0060761 | negative regulation of response to cytokine stimulus | 139 | 15462 | 27 | 2 | 0.024316952 | 0.121679816 | ADIPOQ, PTPRC |
| upregulated | GO:0010718 | positive regulation of epithelial to mesenchymal transition | 139 | 15462 | 27 | 2 | 0.024316952 | 0.121679816 | RGCC, BMP2 |
| upregulated | GO:0050851 | antigen receptor-mediated signaling pathway | 139 | 15462 | 122 | 4 | 0.024327854 | 0.121679816 | LCP2, NFATC2, PTPRC, FYB |
| upregulated | GO:0031344 | regulation of cell projection organization | 139 | 15462 | 317 | 7 | 0.024511172 | 0.122392725 | LRRK2, SDC2, PDLIM5, TENM3, SEMA5A, SHOX2, CNTN1 |
| upregulated | GO:0002062 | chondrocyte differentiation | 139 | 15462 | 70 | 3 | 0.025129333 | 0.122652501 | EFEMP1, SHOX2, BMP2 |
| upregulated | GO:0072073 | kidney epithelium development | 139 | 15462 | 124 | 4 | 0.025633552 | 0.122652501 | WNT4, ADIPOQ, BMP2, CALB1 |
| upregulated | GO:0007622 | rhythmic behavior | 139 | 15462 | 28 | 2 | 0.026035513 | 0.122652501 | NPAS2, IL6 |
| upregulated | GO:0032350 | regulation of hormone metabolic process | 139 | 15462 | 28 | 2 | 0.026035513 | 0.122652501 | WNT4, BMP2 |
| upregulated | GO:0033028 | myeloid cell apoptotic process | 139 | 15462 | 28 | 2 | 0.026035513 | 0.122652501 | IL6, ADIPOQ |
| upregulated | GO:0010677 | negative regulation of cellular carbohydrate metabolic process | 139 | 15462 | 28 | 2 | 0.026035513 | 0.122652501 | IL6, ADIPOQ |
| upregulated | GO:0010906 | regulation of glucose metabolic process | 139 | 15462 | 71 | 3 | 0.026069689 | 0.122652501 | IL6, PPP1R3B, ADIPOQ |
| upregulated | GO:0035270 | endocrine system development | 139 | 15462 | 125 | 4 | 0.02630195 | 0.12265250 | WNT4, IL6, HOXA5, FOXO1 |

| | | | | | | | | | |
|---|---|---|---|---|---|---|---|---|---|
| | | | | | | | 5 | 1 | |
| upreg ulated | GO:00 45859 | regulation of protein kinase activity | 139 | 154 62 | 710 | 12 | 0.02 636 191 8 | 0.12 265 250 1 | LRRK2, CBLC, BMP2, FABP4, PTPRC, RGCC, TNFRSF11A, ITGB3, ADRA2A, MDFIC, IL6, ADIPOQ |
| upreg ulated | GO:00 06469 | negative regulation of protein kinase activity | 139 | 154 62 | 186 | 5 | 0.02 638 497 2 | 0.12 265 250 1 | IL6, CBLC, PTPRC, ADIPOQ, FABP4 |
| upreg ulated | GO:00 42091 | interleukin-10 biosynthetic process | 139 | 154 62 | 3 | 1 | 0.02 672 933 5 | 0.12 265 250 1 | IRF4 |
| upreg ulated | GO:00 48312 | intracellular distribution of mitochondria | 139 | 154 62 | 3 | 1 | 0.02 672 933 5 | 0.12 265 250 1 | LRRK2 |
| upreg ulated | GO:00 60166 | olfactory pit development | 139 | 154 62 | 3 | 1 | 0.02 672 933 5 | 0.12 265 250 1 | DLX5 |
| upreg ulated | GO:00 03130 | BMP signaling pathway involved in heart induction | 139 | 154 62 | 3 | 1 | 0.02 672 933 5 | 0.12 265 250 1 | BMP2 |
| upreg ulated | GO:00 72313 | metanephric glomerular epithelial cell development | 139 | 154 62 | 3 | 1 | 0.02 672 933 5 | 0.12 265 250 1 | ADIPOQ |
| upreg ulated | GO:00 06295 | nucleotide-excision repair, DNA incision, 3'-to lesion | 139 | 154 62 | 3 | 1 | 0.02 672 933 5 | 0.12 265 250 1 | ERCC1 |
| upreg ulated | GO:20 00481 | positive regulation of cAMP-dependent protein kinase activity | 139 | 154 62 | 3 | 1 | 0.02 672 933 5 | 0.12 265 250 1 | ADIPOQ |
| upreg ulated | GO:00 72249 | metanephric glomerular visceral epithelial cell development | 139 | 154 62 | 3 | 1 | 0.02 672 933 5 | 0.12 265 250 1 | ADIPOQ |
| upreg ulated | GO:00 72244 | metanephric glomerular epithelium development | 139 | 154 62 | 3 | 1 | 0.02 672 933 5 | 0.12 265 250 1 | ADIPOQ |
| upreg ulated | GO:00 61209 | cell proliferation involved in mesonephros development | 139 | 154 62 | 3 | 1 | 0.02 672 933 5 | 0.12 265 250 1 | BMP2 |
| upreg ulated | GO:20 00623 | negative regulation of nuclear-transcribed mRNA catabolic process, nonsense-mediated decay | 139 | 154 62 | 3 | 1 | 0.02 672 933 5 | 0.12 265 250 1 | DHX34 |
| upreg ulated | GO:00 45324 | late endosome to vacuole transport | 139 | 154 62 | 3 | 1 | 0.02 672 933 5 | 0.12 265 250 1 | LRRK2 |
| upreg ulated | GO:20 00726 | negative regulation of cardiac muscle cell differentiation | 139 | 154 62 | 3 | 1 | 0.02 672 933 5 | 0.12 265 250 1 | BMP2 |
| upreg ulated | GO:00 71883 | activation of MAPK activity by adrenergic | 139 | 154 62 | 3 | 1 | 0.02 672 933 | 0.12 265 250 | ADRA2A |

| | | | | | | | | | |
|---|---|---|---|---|---|---|---|---|---|
| | | receptor signaling pathway | | | | | 5 | 1 | |
| upreg ulated | GO:00 45404 | positive regulation of interleukin-4 biosynthetic process | 139 | 154 62 | 3 | 1 | 0.02 672 933 5 | 0.12 265 250 1 | IRF4 |
| upreg ulated | GO:00 60161 | positive regulation of dopamine receptor signaling pathway | 139 | 154 62 | 3 | 1 | 0.02 672 933 5 | 0.12 265 250 1 | LRRK2 |
| upreg ulated | GO:00 90270 | regulation of fibroblast growth factor production | 139 | 154 62 | 3 | 1 | 0.02 672 933 5 | 0.12 265 250 1 | RGCC |
| upreg ulated | GO:00 61184 | positive regulation of dermatome development | 139 | 154 62 | 3 | 1 | 0.02 672 933 5 | 0.12 265 250 1 | WNT4 |
| upreg ulated | GO:00 00720 | pyrimidine dimer repair by nucleotide-excision repair | 139 | 154 62 | 3 | 1 | 0.02 672 933 5 | 0.12 265 250 1 | ERCC1 |
| upreg ulated | GO:00 35694 | mitochondrial protein catabolic process | 139 | 154 62 | 3 | 1 | 0.02 672 933 5 | 0.12 265 250 1 | BNIP3L |
| upreg ulated | GO:00 72138 | mesenchymal cell proliferation involved in ureteric bud development | 139 | 154 62 | 3 | 1 | 0.02 672 933 5 | 0.12 265 250 1 | BMP2 |
| upreg ulated | GO:00 72248 | metanephric glomerular visceral epithelial cell differentiation | 139 | 154 62 | 3 | 1 | 0.02 672 933 5 | 0.12 265 250 1 | ADIPOQ |
| upreg ulated | GO:00 03133 | endodermal-mesodermal cell signaling | 139 | 154 62 | 3 | 1 | 0.02 672 933 5 | 0.12 265 250 1 | BMP2 |
| upreg ulated | GO:00 71351 | cellular response to interleukin-18 | 139 | 154 62 | 3 | 1 | 0.02 672 933 5 | 0.12 265 250 1 | IL18R1 |
| upreg ulated | GO:20 00622 | regulation of nuclear-transcribed mRNA catabolic process, nonsense-mediated decay | 139 | 154 62 | 3 | 1 | 0.02 672 933 5 | 0.12 265 250 1 | DHX34 |
| upreg ulated | GO:20 00300 | regulation of synaptic vesicle exocytosis | 139 | 154 62 | 3 | 1 | 0.02 672 933 5 | 0.12 265 250 1 | LRRK2 |
| upreg ulated | GO:00 35441 | cell migration involved in vasculogenesis | 139 | 154 62 | 3 | 1 | 0.02 672 933 5 | 0.12 265 250 1 | TBX5 |
| upreg ulated | GO:20 00366 | positive regulation of STAT protein import into nucleus | 139 | 154 62 | 3 | 1 | 0.02 672 933 5 | 0.12 265 250 1 | IL6 |
| upreg ulated | GO:00 90269 | fibroblast growth factor production | 139 | 154 62 | 3 | 1 | 0.02 672 933 | 0.12 265 250 | RGCC |

| | | | | | | | | | |
|---|---|---|---|---|---|---|---|---|---|
| | | | | | | | 5 | 1 | |
| upregulated | GO:0045074 | regulation of interleukin-10 biosynthetic process | 139 | 15462 | 3 | 1 | 0.026729335 | 0.122652501 | IRF4 |
| upregulated | GO:0060435 | bronchiole development | 139 | 15462 | 3 | 1 | 0.026729335 | 0.122652501 | HOXA5 |
| upregulated | GO:0002378 | immunoglobulin biosynthetic process | 139 | 15462 | 3 | 1 | 0.026729335 | 0.122652501 | PTPRC |
| upregulated | GO:0060574 | intestinal epithelial cell maturation | 139 | 15462 | 3 | 1 | 0.026729335 | 0.122652501 | HOXA5 |
| upregulated | GO:2000364 | regulation of STAT protein import into nucleus | 139 | 15462 | 3 | 1 | 0.026729335 | 0.122652501 | IL6 |
| upregulated | GO:0070340 | detection of bacterial lipopeptide | 139 | 15462 | 3 | 1 | 0.026729335 | 0.122652501 | TLR1 |
| upregulated | GO:0003134 | endodermal-mesodermal cell signaling involved in heart induction | 139 | 15462 | 3 | 1 | 0.026729335 | 0.122652501 | BMP2 |
| upregulated | GO:0048743 | positive regulation of skeletal muscle fiber development | 139 | 15462 | 3 | 1 | 0.026729335 | 0.122652501 | SHOX2 |
| upregulated | GO:0006296 | nucleotide-excision repair, DNA incision, 5'-to lesion | 139 | 15462 | 3 | 1 | 0.026729335 | 0.122652501 | ERCC1 |
| upregulated | GO:0072312 | metanephric glomerular epithelial cell differentiation | 139 | 15462 | 3 | 1 | 0.026729335 | 0.122652501 | ADIPOQ |
| upregulated | GO:0015015 | heparan sulfate proteoglycan biosynthetic process, enzymatic modification | 139 | 15462 | 3 | 1 | 0.026729335 | 0.122652501 | HS3ST3B1 |
| upregulated | GO:0002540 | leukotriene production involved in inflammatory response | 139 | 15462 | 3 | 1 | 0.026729335 | 0.122652501 | ALOX5AP |
| upregulated | GO:0071639 | positive regulation of monocyte chemotactic protein-1 production | 139 | 15462 | 3 | 1 | 0.026729335 | 0.122652501 | ADIPOQ |
| upregulated | GO:0002538 | arachidonic acid metabolite production involved in inflammatory response | 139 | 15462 | 3 | 1 | 0.026729335 | 0.122652501 | ALOX5AP |
| upregulated | GO:0010035 | response to inorganic substance | 139 | 15462 | 323 | 7 | 0.026788702 | 0.122737242 | NR4A3, IL6, ALOX5AP, CD36, FOXO1, FABP4, CAMK2D |
| upreg | GO:00 | negative | 139 | 154 | 72 | 3 | 0.02 | 0.12 | MDFIC, CD36, ADIPOQ |

| | | | | | | | | | |
|---|---|---|---|---|---|---|---|---|---|
| ulated | 90317 | regulation of intracellular protein transport | | 62 | | | 702 875 3 | 364 830 3 | |
| upreg ulated | GO:00 31347 | regulation of defense response | 139 | 154 62 | 473 | 9 | 0.02 707 911 8 | 0.12 369 015 6 | TNFRSF11A, BIRC3, IL6, TLR1, ALOX5AP, IRF4, CD36, ADIPOQ, FABP4 |
| upreg ulated | GO:00 17038 | protein import | 139 | 154 62 | 254 | 6 | 0.02 728 199 1 | 0.12 442 744 1 | IL6, MDFIC, IL18R1, SEMA5A, CD36, FYB |
| upreg ulated | GO:00 44710 | single-organism metabolic process | 139 | 154 62 | 509 4 | 57 | 0.02 760 981 5 | 0.12 546 392 9 | TAGAP, GPC6, CBLC, IRF4, HS3ST3B1, FABP4, ADRA2A, COL12A1, RAB30, IGJ, NPAS2, NKD1, GLRX, RASGRF2, GALNT5, AOX1, COL1A2, CAMK2D, RGCC, PRDM6, FMO4, ERCC1, CXCL9, ADH1B, BCHE, SLC22A3, FOXO1, ARHGAP42, CRLS1, STEAP4, PRELP, SOS1, MDFIC, PPP1R3B, ALOX5AP, CD36, MMP1, DOCK8, LRRK2, PRKG1, GCNT4, SDC2, PDE1A, PLIN1, ANGPTL4, GUCY1A3, CCR1, BMP2, TNFRSF11A, GALNT14, WNT4, IL6, CHSY3, NAMPT, SLC7A5, TDO2, ADIPOQ |
| upreg ulated | GO:00 72089 | stem cell proliferation | 139 | 154 62 | 127 | 4 | 0.02 767 001 3 | 0.12 546 392 9 | LRRK2, SHOX2, PTPRC, BMP2 |
| upreg ulated | GO:00 43277 | apoptotic cell clearance | 139 | 154 62 | 29 | 2 | 0.02 780 190 4 | 0.12 546 392 9 | MEGF10, CD36 |
| upreg ulated | GO:00 03230 | cardiac atrium development | 139 | 154 62 | 29 | 2 | 0.02 780 190 4 | 0.12 546 392 9 | TBX5, SHOX2 |
| upreg ulated | GO:00 51148 | negative regulation of muscle cell differentiation | 139 | 154 62 | 29 | 2 | 0.02 780 190 4 | 0.12 546 392 9 | PRDM6, BMP2 |
| upreg ulated | GO:00 32309 | icosanoid secretion | 139 | 154 62 | 29 | 2 | 0.02 780 190 4 | 0.12 546 392 9 | TNFRSF11A, PLA2R1 |
| upreg ulated | GO:20 00514 | regulation of CD4-positive, alpha-beta T cell activation | 139 | 154 62 | 29 | 2 | 0.02 780 190 4 | 0.12 546 392 9 | IL6, IRF4 |
| upreg ulated | GO:00 71248 | cellular response to metal ion | 139 | 154 62 | 73 | 3 | 0.02 800 650 1 | 0.12 619 746 | ALOX5AP, FABP4, CAMK2D |
| upreg ulated | GO:00 71900 | regulation of protein serine/threonine kinase activity | 139 | 154 62 | 400 | 8 | 0.02 812 175 6 | 0.12 652 682 3 | LRRK2, CBLC, BMP2, TNFRSF11A, ADRA2A, RGCC, MDFIC, ADIPOQ |
| upreg ulated | GO:00 06936 | muscle contraction | 139 | 154 62 | 256 | 6 | 0.02 820 339 5 | 0.12 670 417 3 | ADRA2A, TNNI2, CHRNA1, LMOD1, CAMK2D, GUCY1A3 |
| upreg ulated | GO:20 00021 | regulation of ion homeostasis | 139 | 154 62 | 128 | 4 | 0.02 836 972 | 0.12 707 094 | LRRK2, CXCL9, PTPRC, CAMK2D |
| upreg ulated | GO:00 07623 | circadian rhythm | 139 | 154 62 | 128 | 4 | 0.02 836 972 | 0.12 707 094 | TNFRSF11A, NPAS2, IL6, ADIPOQ |
| upreg ulated | GO:00 51171 | regulation of nitrogen compound metabolic | 139 | 154 62 | 416 2 | 48 | 0.02 851 483 2 | 0.12 753 056 8 | TAGAP, SHOX2, IRF4, FABP4, ADRA2A, TNNI2, HOXA5, NFATC2, TFEC, NPAS2, GDF6, RASGRF2, IL18R1, HIVEP3, CAMK2D, DHX34, RGCC, PRDM6, BNC2, |

| | | | | | | | | | |
|---|---|---|---|---|---|---|---|---|---|
| | | process | | | | | | | ERCC1, CXCL9, FOXO1, ARHGAP42, NR4A3, SOS1, RFX2, RUNX1T1, EFEMP1, MDFIC, FOSL2, CD36, SATB2, DOCK8, LRRK2, ZFHX4, BIRC3, NR4A1, PRKG1, DLX5, CDH13, GUCY1A3, BMP2, TNFRSF11A, WNT4, IL6, TBX5, NAMPT, ADIPOQ |
| upreg ulated | GO:00 42307 | positive regulation of protein import into nucleus | 139 | 154 62 | 74 | 3 | 0.02 900 290 6 | 0.12 909 588 1 | IL6, IL18R1, SEMA5A |
| upreg ulated | GO:00 45666 | positive regulation of neuron differentiation | 139 | 154 62 | 74 | 3 | 0.02 900 290 6 | 0.12 909 588 1 | IL6, GDF6, BMP2 |
| upreg ulated | GO:00 30574 | collagen catabolic process | 139 | 154 62 | 74 | 3 | 0.02 900 290 6 | 0.12 909 588 1 | COL12A1, COL1A2, MMP1 |
| upreg ulated | GO:00 45860 | positive regulation of protein kinase activity | 139 | 154 62 | 479 | 9 | 0.02 903 912 4 | 0.12 909 588 1 | LRRK2, BMP2, PTPRC, RGCC, ITGB3, TNFRSF11A, ADRA2A, MDFIC, ADIPOQ |
| upreg ulated | GO:00 00187 | activation of MAPK activity | 139 | 154 62 | 129 | 4 | 0.02 907 991 2 | 0.12 909 588 1 | ADRA2A, LRRK2, MDFIC, BMP2 |
| upreg ulated | GO:00 51235 | maintenance of location | 139 | 154 62 | 258 | 6 | 0.02 914 517 9 | 0.12 919 450 9 | ITGB3, IL6, CXCL9, CD36, PTPRC, CAMK2D |
| upreg ulated | GO:00 44253 | positive regulation of multicellular organismal metabolic process | 139 | 154 62 | 30 | 2 | 0.02 961 514 | 0.13 089 106 6 | RGCC, WNT4 |
| upreg ulated | GO:00 50710 | negative regulation of cytokine secretion | 139 | 154 62 | 30 | 2 | 0.02 961 514 | 0.13 089 106 6 | RGCC, IL6 |
| upreg ulated | GO:00 43406 | positive regulation of MAP kinase activity | 139 | 154 62 | 192 | 5 | 0.02 970 300 2 | 0.13 108 633 8 | ADRA2A, TNFRSF11A, LRRK2, MDFIC, BMP2 |
| upreg ulated | GO:00 51282 | regulation of sequestering of calcium ion | 139 | 154 62 | 75 | 3 | 0.03 001 793 9 | 0.13 131 754 5 | CXCL9, PTPRC, CAMK2D |
| upreg ulated | GO:00 50864 | regulation of B cell activation | 139 | 154 62 | 75 | 3 | 0.03 001 793 9 | 0.13 131 754 5 | IL6, NFATC2, PTPRC |
| upreg ulated | GO:00 51209 | release of sequestered calcium ion into cytosol | 139 | 154 62 | 75 | 3 | 0.03 001 793 9 | 0.13 131 754 5 | CXCL9, PTPRC, CAMK2D |
| upreg ulated | GO:00 51283 | negative regulation of sequestering of calcium ion | 139 | 154 62 | 75 | 3 | 0.03 001 793 9 | 0.13 131 754 5 | CXCL9, PTPRC, CAMK2D |
| upreg ulated | GO:00 46824 | positive regulation of nucleocytoplasmi c transport | 139 | 154 62 | 75 | 3 | 0.03 001 793 9 | 0.13 131 754 5 | IL6, IL18R1, SEMA5A |
| upreg ulated | GO:00 07569 | cell aging | 139 | 154 62 | 75 | 3 | 0.03 001 793 9 | 0.13 131 754 5 | PLA2R1, ERCC1, PRELP |
| upreg ulated | GO:00 09607 | response to biotic stimulus | 139 | 154 62 | 643 | 11 | 0.03 044 | 0.13 298 | BIRC3, TLR1, DCLK1, GUCY1A3, PTPRC, TNFRSF11A, IL6, BNIP3L, IGJ, |

| | | | | | | | | | |
|---|---|---|---|---|---|---|---|---|---|
| | | | | | | | 384 6 | 687 3 | CXCL9, CD36 |
| upreg ulated | GO:00 51924 | regulation of calcium ion transport | 139 | 154 62 | 131 | 4 | 0.03 053 184 | 0.13 317 74 | ADRA2A, CXCL9, CCR1, CAMK2D |
| upreg ulated | GO:00 90257 | regulation of muscle system process | 139 | 154 62 | 132 | 4 | 0.03 127 361 6 | 0.13 455 017 1 | ADRA2A, FBXO32, CAMK2D, GUCY1A3 |
| upreg ulated | GO:00 32270 | positive regulation of cellular protein metabolic process | 139 | 154 62 | 898 | 14 | 0.03 129 375 9 | 0.13 455 017 1 | FAM129A, LRRK2, BIRC3, GDF6, BMP2, PTPRC, RGCC, TNFRSF11A, ADRA2A, ITGB3, IL6, MDFIC, CD36, ADIPOQ |
| upreg ulated | GO:00 07610 | behavior | 139 | 154 62 | 565 | 10 | 0.03 136 540 2 | 0.13 455 017 1 | NR4A3, NPAS2, LRRK2, CDH13, GCNT4, SEMA5A, CCR1, IL6, BCHE, CALB1 |
| upreg ulated | GO:00 51046 | regulation of secretion | 139 | 154 62 | 486 | 9 | 0.03 144 974 8 | 0.13 455 017 1 | LRRK2, TLR1, CD2, TNFRSF11A, ADRA2A, RGCC, IL6, PLA2R1, ADIPOQ |
| upreg ulated | GO:19 01571 | fatty acid derivative transport | 139 | 154 62 | 31 | 2 | 0.03 147 424 9 | 0.13 455 017 1 | TNFRSF11A, PLA2R1 |
| upreg ulated | GO:00 71829 | plasma lipoprotein particle disassembly | 139 | 154 62 | 31 | 2 | 0.03 147 424 9 | 0.13 455 017 1 | CD36, ADIPOQ |
| upreg ulated | GO:00 34381 | plasma lipoprotein particle clearance | 139 | 154 62 | 31 | 2 | 0.03 147 424 9 | 0.13 455 017 1 | CD36, ADIPOQ |
| upreg ulated | GO:00 97061 | dendritic spine organization | 139 | 154 62 | 31 | 2 | 0.03 147 424 9 | 0.13 455 017 1 | LRRK2, PDLIM5 |
| upreg ulated | GO:00 32987 | protein-lipid complex disassembly | 139 | 154 62 | 31 | 2 | 0.03 147 424 9 | 0.13 455 017 1 | CD36, ADIPOQ |
| upreg ulated | GO:00 51055 | negative regulation of lipid biosynthetic process | 139 | 154 62 | 31 | 2 | 0.03 147 424 9 | 0.13 455 017 1 | WNT4, BMP2 |
| upreg ulated | GO:20 00278 | regulation of DNA biosynthetic process | 139 | 154 62 | 31 | 2 | 0.03 147 424 9 | 0.13 455 017 1 | RGCC, ADIPOQ |
| upreg ulated | GO:00 03254 | regulation of membrane depolarization | 139 | 154 62 | 31 | 2 | 0.03 147 424 9 | 0.13 455 017 1 | LRRK2, CAMK2D |
| upreg ulated | GO:00 48644 | muscle organ morphogenesis | 139 | 154 62 | 31 | 2 | 0.03 147 424 9 | 0.13 455 017 1 | SHOX2, BMP2 |
| upreg ulated | GO:00 71715 | icosanoid transport | 139 | 154 62 | 31 | 2 | 0.03 147 424 9 | 0.13 455 017 1 | TNFRSF11A, PLA2R1 |
| upreg ulated | GO:00 34504 | protein localization to nucleus | 139 | 154 62 | 263 | 6 | 0.03 158 979 1 | 0.13 485 200 8 | MDFIC, IL6, IL18R1, SEMA5A, CD36, FYB |
| upreg ulated | GO:00 45087 | innate immune response | 139 | 154 62 | 814 | 13 | 0.03 166 501 1 | 0.13 498 110 4 | SLAMF7, BIRC3, NR4A1, TLR1, PDE1A, SOS1, IRF4, CAMK2D, LCP2, IGJ, NFATC2, CD36, FOXO1 |
| upreg | GO:00 | hematopoietic or | 139 | 154 | 567 | 10 | 0.03 | 0.13 | IL18R1, IRF4, CCR1, PTPRC, |

| | | | | | | | | | |
|---|---|---|---|---|---|---|---|---|---|
| ulated | 48534 | lymphoid organ development | | 62 | | | 202 090 7 | 569 482 7 | TNFRSF11A, CD2, WNT4, IL6, HOXA5, ADIPOQ |
| upreg ulated | GO:00 14031 | mesenchymal cell development | 139 | 154 62 | 133 | 4 | 0.03 202 595 9 | 0.13 569 482 7 | RGCC, WNT4, TBX5, BMP2 |
| upreg ulated | GO:00 10959 | regulation of metal ion transport | 139 | 154 62 | 196 | 5 | 0.03 205 519 3 | 0.13 569 482 7 | ADRA2A, CXCL9, CCR1, CNTN1, CAMK2D |
| upreg ulated | GO:00 09615 | response to virus | 139 | 154 62 | 264 | 6 | 0.03 209 431 | 0.13 569 482 7 | BIRC3, IL6, BNIP3L, CXCL9, DCLK1, PTPRC |
| upreg ulated | GO:00 60021 | palate development | 139 | 154 62 | 77 | 3 | 0.03 210 374 1 | 0.13 569 482 7 | BNC2, DLX5, SATB2 |
| upreg ulated | GO:20 00736 | regulation of stem cell differentiation | 139 | 154 62 | 77 | 3 | 0.03 210 374 1 | 0.13 569 482 7 | RGCC, TBX5, BMP2 |
| upreg ulated | GO:00 10720 | positive regulation of cell development | 139 | 154 62 | 197 | 5 | 0.03 266 095 9 | 0.13 615 991 3 | RGCC, SHOX2, SEMA5A, ADIPOQ, BMP2 |
| upreg ulated | GO:00 72593 | reactive oxygen species metabolic process | 139 | 154 62 | 134 | 4 | 0.03 278 888 5 | 0.13 615 991 3 | BIRC3, PLA2R1, CD36, AOX1 |
| upreg ulated | GO:00 01656 | metanephros development | 139 | 154 62 | 78 | 3 | 0.03 317 442 7 | 0.13 615 991 3 | WNT4, ADIPOQ, CALB1 |
| upreg ulated | GO:00 51208 | sequestering of calcium ion | 139 | 154 62 | 78 | 3 | 0.03 317 442 7 | 0.13 615 991 3 | CXCL9, PTPRC, CAMK2D |
| upreg ulated | GO:00 44344 | cellular response to fibroblast growth factor stimulus | 139 | 154 62 | 198 | 5 | 0.03 327 385 3 | 0.13 615 991 3 | WNT4, NR4A1, PDE1A, SOS1, FOXO1 |
| upreg ulated | GO:00 30501 | positive regulation of bone mineralization | 139 | 154 62 | 32 | 2 | 0.03 337 827 2 | 0.13 615 991 3 | WNT4, BMP2 |
| upreg ulated | GO:19 01019 | regulation of calcium ion transmembrane transporter activity | 139 | 154 62 | 32 | 2 | 0.03 337 827 2 | 0.13 615 991 3 | ADRA2A, CAMK2D |
| upreg ulated | GO:00 50873 | brown fat cell differentiation | 139 | 154 62 | 32 | 2 | 0.03 337 827 2 | 0.13 615 991 3 | FABP4, ADIPOQ |
| upreg ulated | GO:19 03169 | regulation of calcium ion transmembrane transport | 139 | 154 62 | 32 | 2 | 0.03 337 827 2 | 0.13 615 991 3 | ADRA2A, CAMK2D |
| upreg ulated | GO:00 35115 | embryonic forelimb morphogenesis | 139 | 154 62 | 32 | 2 | 0.03 337 827 2 | 0.13 615 991 3 | TBX5, SHOX2 |
| upreg ulated | GO:00 70169 | positive regulation of biomineral tissue development | 139 | 154 62 | 32 | 2 | 0.03 337 827 2 | 0.13 615 991 3 | WNT4, BMP2 |
| upreg ulated | GO:00 60998 | regulation of dendritic spine development | 139 | 154 62 | 32 | 2 | 0.03 337 827 | 0.13 615 991 | LRRK2, PDLIM5 |

| | | | | | | | | | |
|---|---|---|---|---|---|---|---|---|---|
| | | | | | | | 2 | 3 | |
| upreg ulated | GO:19 00542 | regulation of purine nucleotide metabolic process | 139 | 154 62 | 653 | 11 | 0.03 351 032 7 | 0.13 615 991 3 | TAGAP, LRRK2, PRKG1, RASGRF2, SOS1, GUCY1A3, ADRA2A, WNT4, CXCL9, ARHGAP42, DOCK8 |
| upreg ulated | GO:00 10565 | regulation of cellular ketone metabolic process | 139 | 154 62 | 135 | 4 | 0.03 356 241 | 0.13 615 991 3 | WNT4, IL6, ADIPOQ, BMP2 |
| upreg ulated | GO:00 33673 | negative regulation of kinase activity | 139 | 154 62 | 199 | 5 | 0.03 389 389 2 | 0.13 615 991 3 | IL6, CBLC, PTPRC, ADIPOQ, FABP4 |
| upreg ulated | GO:00 06493 | protein O-linked glycosylation | 139 | 154 62 | 79 | 3 | 0.03 426 357 5 | 0.13 615 991 3 | GALNT14, GCNT4, GALNT5 |
| upreg ulated | GO:00 32675 | regulation of interleukin-6 production | 139 | 154 62 | 79 | 3 | 0.03 426 357 5 | 0.13 615 991 3 | IL6, TLR1, CD36 |
| upreg ulated | GO:00 10811 | positive regulation of cell-substrate adhesion | 139 | 154 62 | 79 | 3 | 0.03 426 357 5 | 0.13 615 991 3 | WNT4, CDH13, CD36 |
| upreg ulated | GO:00 06140 | regulation of nucleotide metabolic process | 139 | 154 62 | 656 | 11 | 0.03 447 172 8 | 0.13 615 991 3 | TAGAP, LRRK2, PRKG1, RASGRF2, SOS1, GUCY1A3, ADRA2A, WNT4, CXCL9, ARHGAP42, DOCK8 |
| upreg ulated | GO:00 02694 | regulation of leukocyte activation | 139 | 154 62 | 341 | 7 | 0.03 447 874 5 | 0.13 615 991 3 | CD2, SLAMF7, IL6, NFATC2, IRF4, DPP4, PTPRC |
| upreg ulated | GO:00 43087 | regulation of GTPase activity | 139 | 154 62 | 417 | 8 | 0.03 472 642 6 | 0.13 615 991 3 | TAGAP, LRRK2, PRKG1, RASGRF2, SOS1, WNT4, ARHGAP42, DOCK8 |
| upreg ulated | GO:00 32388 | positive regulation of intracellular transport | 139 | 154 62 | 137 | 4 | 0.03 514 130 7 | 0.13 615 991 3 | IL6, CXCL9, IL18R1, SEMA5A |
| upreg ulated | GO:00 33124 | regulation of GTP catabolic process | 139 | 154 62 | 418 | 8 | 0.03 514 511 6 | 0.13 615 991 3 | TAGAP, LRRK2, PRKG1, RASGRF2, SOS1, WNT4, ARHGAP42, DOCK8 |
| upreg ulated | GO:00 71774 | response to fibroblast growth factor | 139 | 154 62 | 201 | 5 | 0.03 515 547 8 | 0.13 615 991 3 | WNT4, NR4A1, PDE1A, SOS1, FOXO1 |
| upreg ulated | GO:00 71277 | cellular response to calcium ion | 139 | 154 62 | 33 | 2 | 0.03 532 626 5 | 0.13 615 991 3 | ALOX5AP, CAMK2D |
| upreg ulated | GO:00 60325 | face morphogenesis | 139 | 154 62 | 33 | 2 | 0.03 532 626 5 | 0.13 615 991 3 | CRISPLD2, DLX5 |
| upreg ulated | GO:00 44243 | multicellular organismal catabolic process | 139 | 154 62 | 80 | 3 | 0.03 537 113 3 | 0.13 615 991 3 | COL12A1, COL1A2, MMP1 |
| upreg ulated | GO:00 72091 | regulation of stem cell proliferation | 139 | 154 62 | 80 | 3 | 0.03 537 113 3 | 0.13 615 991 3 | LRRK2, SHOX2, PTPRC |
| upreg ulated | GO:00 51291 | protein heterooligomeriz ation | 139 | 154 62 | 80 | 3 | 0.03 537 113 3 | 0.13 615 991 3 | BIRC3, COL1A2, ADIPOQ |
| upreg ulated | GO:00 30900 | forebrain development | 139 | 154 62 | 343 | 7 | 0.03 541 | 0.13 615 | WNT4, NR4A3, LRRK2, PRKG1, DCLK1, SEMA5A, BMP2 |

| | | | | | | | | | |
|---|---|---|---|---|---|---|---|---|---|
| | | | | | | | 511 4 | 991 3 | |
| upreg ulated | GO:00 03164 | His-Purkinje system development | 139 | 154 62 | 4 | 1 | 0.03 548 052 4 | 0.13 615 991 3 | TBX5 |
| upreg ulated | GO:19 01311 | regulation of gene expression involved in extracellular matrix organization | 139 | 154 62 | 4 | 1 | 0.03 548 052 4 | 0.13 615 991 3 | RGCC |
| upreg ulated | GO:00 35789 | metanephric mesenchymal cell migration | 139 | 154 62 | 4 | 1 | 0.03 548 052 4 | 0.13 615 991 3 | ADIPOQ |
| upreg ulated | GO:00 61183 | regulation of dermatome development | 139 | 154 62 | 4 | 1 | 0.03 548 052 4 | 0.13 615 991 3 | WNT4 |
| upreg ulated | GO:20 00467 | positive regulation of glycogen (starch) synthase activity | 139 | 154 62 | 4 | 1 | 0.03 548 052 4 | 0.13 615 991 3 | ADIPOQ |
| upreg ulated | GO:00 30885 | regulation of myeloid dendritic cell activation | 139 | 154 62 | 4 | 1 | 0.03 548 052 4 | 0.13 615 991 3 | CD2 |
| upreg ulated | GO:00 42816 | vitamin B6 metabolic process | 139 | 154 62 | 4 | 1 | 0.03 548 052 4 | 0.13 615 991 3 | AOX1 |
| upreg ulated | GO:00 70050 | neuron cellular homeostasis | 139 | 154 62 | 4 | 1 | 0.03 548 052 4 | 0.13 615 991 3 | CHRNA1 |
| upreg ulated | GO:00 60743 | epithelial cell maturation involved in prostate gland development | 139 | 154 62 | 4 | 1 | 0.03 548 052 4 | 0.13 615 991 3 | FEM1B |
| upreg ulated | GO:00 60159 | regulation of dopamine receptor signaling pathway | 139 | 154 62 | 4 | 1 | 0.03 548 052 4 | 0.13 615 991 3 | LRRK2 |
| upreg ulated | GO:00 42097 | interleukin-4 biosynthetic process | 139 | 154 62 | 4 | 1 | 0.03 548 052 4 | 0.13 615 991 3 | IRF4 |
| upreg ulated | GO:00 61054 | dermatome development | 139 | 154 62 | 4 | 1 | 0.03 548 052 4 | 0.13 615 991 3 | WNT4 |
| upreg ulated | GO:00 32966 | negative regulation of collagen biosynthetic process | 139 | 154 62 | 4 | 1 | 0.03 548 052 4 | 0.13 615 991 3 | IL6 |
| upreg ulated | GO:00 35625 | epidermal growth factor-activated receptor transactivation by G-protein coupled receptor signaling pathway | 139 | 154 62 | 4 | 1 | 0.03 548 052 4 | 0.13 615 991 3 | ADRA2A |
| upreg ulated | GO:00 14041 | regulation of neuron maturation | 139 | 154 62 | 4 | 1 | 0.03 548 052 4 | 0.13 615 991 3 | LRRK2 |

| | | | | | | | | | |
|---|---|---|---|---|---|---|---|---|---|
| upreg ulated | GO:00 90403 | oxidative stress-induced premature senescence | 139 | 154 62 | 4 | 1 | 0.03 548 052 4 | 0.13 615 991 3 | PLA2R1 |
| upreg ulated | GO:00 70278 | extracellular matrix constituent secretion | 139 | 154 62 | 4 | 1 | 0.03 548 052 4 | 0.13 615 991 3 | RGCC |
| upreg ulated | GO:19 01725 | regulation of histone deacetylase activity | 139 | 154 62 | 4 | 1 | 0.03 548 052 4 | 0.13 615 991 3 | CAMK2D |
| upreg ulated | GO:00 32345 | negative regulation of aldosterone metabolic process | 139 | 154 62 | 4 | 1 | 0.03 548 052 4 | 0.13 615 991 3 | BMP2 |
| upreg ulated | GO:20 00589 | regulation of metanephric mesenchymal cell migration | 139 | 154 62 | 4 | 1 | 0.03 548 052 4 | 0.13 615 991 3 | ADIPOQ |
| upreg ulated | GO:00 03166 | bundle of His development | 139 | 154 62 | 4 | 1 | 0.03 548 052 4 | 0.13 615 991 3 | TBX5 |
| upreg ulated | GO:00 07525 | somatic muscle development | 139 | 154 62 | 4 | 1 | 0.03 548 052 4 | 0.13 615 991 3 | NKD1 |
| upreg ulated | GO:00 60535 | trachea cartilage morphogenesis | 139 | 154 62 | 4 | 1 | 0.03 548 052 4 | 0.13 615 991 3 | HOXA5 |
| upreg ulated | GO:20 00065 | negative regulation of cortisol biosynthetic process | 139 | 154 62 | 4 | 1 | 0.03 548 052 4 | 0.13 615 991 3 | BMP2 |
| upreg ulated | GO:00 90238 | positive regulation of arachidonic acid secretion | 139 | 154 62 | 4 | 1 | 0.03 548 052 4 | 0.13 615 991 3 | PLA2R1 |
| upreg ulated | GO:00 60546 | negative regulation of necroptotic process | 139 | 154 62 | 4 | 1 | 0.03 548 052 4 | 0.13 615 991 3 | BIRC3 |
| upreg ulated | GO:19 02373 | negative regulation of mRNA catabolic process | 139 | 154 62 | 4 | 1 | 0.03 548 052 4 | 0.13 615 991 3 | DHX34 |
| upreg ulated | GO:00 71447 | cellular response to hydroperoxide | 139 | 154 62 | 4 | 1 | 0.03 548 052 4 | 0.13 615 991 3 | CD36 |
| upreg ulated | GO:00 70673 | response to interleukin-18 | 139 | 154 62 | 4 | 1 | 0.03 548 052 4 | 0.13 615 991 3 | IL18R1 |
| upreg ulated | GO:19 03054 | negative regulation of extracellular matrix organization | 139 | 154 62 | 4 | 1 | 0.03 548 052 4 | 0.13 615 991 3 | DPP4 |
| upreg ulated | GO:19 01148 | gene expression involved in extracellular matrix organization | 139 | 154 62 | 4 | 1 | 0.03 548 052 4 | 0.13 615 991 3 | RGCC |
| upreg ulated | GO:00 10716 | negative regulation of extracellular | 139 | 154 62 | 4 | 1 | 0.03 548 052 | 0.13 615 991 | DPP4 |

| | | | | | | | | | |
|---|---|---|---|---|---|---|---|---|---|
| | | matrix disassembly | | | | | 4 | 3 | |
| upregulated | GO:0035788 | cell migration involved in metanephros development | 139 | 15462 | 4 | 1 | 0.035480524 | 0.136159913 | ADIPOQ |
| upregulated | GO:0043654 | recognition of apoptotic cell | 139 | 15462 | 4 | 1 | 0.035480524 | 0.136159913 | MEGF10 |
| upregulated | GO:0035701 | hematopoietic stem cell migration | 139 | 15462 | 4 | 1 | 0.035480524 | 0.136159913 | PTPRC |
| upregulated | GO:0032348 | negative regulation of aldosterone biosynthetic process | 139 | 15462 | 4 | 1 | 0.035480524 | 0.136159913 | BMP2 |
| upregulated | GO:0061205 | paramesonephric duct development | 139 | 15462 | 4 | 1 | 0.035480524 | 0.136159913 | WNT4 |
| upregulated | GO:0042494 | detection of bacterial lipoprotein | 139 | 15462 | 4 | 1 | 0.035480524 | 0.136159913 | TLR1 |
| upregulated | GO:0045402 | regulation of interleukin-4 biosynthetic process | 139 | 15462 | 4 | 1 | 0.035480524 | 0.136159913 | IRF4 |
| upregulated | GO:1901313 | positive regulation of gene expression involved in extracellular matrix organization | 139 | 15462 | 4 | 1 | 0.035480524 | 0.136159913 | RGCC |
| upregulated | GO:2000048 | negative regulation of cell-cell adhesion mediated by cadherin | 139 | 15462 | 4 | 1 | 0.035480524 | 0.136159913 | RGCC |
| upregulated | GO:0032304 | negative regulation of icosanoid secretion | 139 | 15462 | 4 | 1 | 0.035480524 | 0.136159913 | PLA2R1 |
| upregulated | GO:0016043 | cellular component organization | 139 | 15462 | 4746 | 53 | 0.036428803 | 0.139171077 | DCLK1, SHOX2, IRF4, ADRA2A, COL12A1, RAB30, BNIP3L, PDLIM5, MFAP5, GFRA1, NPTX1, DPP4, COL1A2, CAMK2D, ITGB3, RGCC, PRDM6, ERCC1, ASIC2, CNTN1, NR4A3, TSPAN5, TENM3, SEMA5A, CHRNA1, SOS1, ADAMTS5, EFEMP1, PLA2R1, ALOX5AP, CD36, MMP1, SATB2, ATP11A, CRISPLD2, LRRK2, ELN, STAP1, BIRC3, PRKG1, DLX5, MEGF10, CDH13, SDC2, ANGPTL4, BMP2, PTPRC, WNT4, IL6, TBX5, C1QTNF7, SKAP2, ADIPOQ |
| upregulated | GO:0030111 | regulation of Wnt signaling pathway | 139 | 15462 | 203 | 5 | 0.036445854 | 0.139171077 | WNT4, MDFIC, NKD1, DLX5, BMP2 |
| upregulated | GO:0032635 | interleukin-6 production | 139 | 15462 | 81 | 3 | 0.036497047 | 0.139171077 | IL6, TLR1, CD36 |
| upregulated | GO:0045995 | regulation of embryonic development | 139 | 15462 | 81 | 3 | 0.03649704 | 0.13917107 | WNT4, NKD1, ADIPOQ |

| | | | | | | | | | |
|---|---|---|---|---|---|---|---|---|---|
| | | | | | | | 7 | 7 | |
| upregulated | GO:0001678 | cellular glucose homeostasis | 139 | 15462 | 81 | 3 | 0.036497047 | 0.139171077 | ADRA2A, NPTX1, FOXO1 |
| upregulated | GO:0097285 | cell-type specific apoptotic process | 139 | 15462 | 346 | 7 | 0.036851153 | 0.140343031 | RGCC, NR4A3, IL6, PDE1A, SEMA5A, ANGPTL4, ADIPOQ |
| upregulated | GO:0001659 | temperature homeostasis | 139 | 15462 | 34 | 2 | 0.037317295 | 0.141400508 | TNFRSF11A, FOXO1 |
| upregulated | GO:0051496 | positive regulation of stress fiber assembly | 139 | 15462 | 34 | 2 | 0.037317295 | 0.141400508 | RGCC, WNT4 |
| upregulated | GO:0043502 | regulation of muscle adaptation | 139 | 15462 | 34 | 2 | 0.037317295 | 0.141400508 | FBXO32, CAMK2D |
| upregulated | GO:0072210 | metanephric nephron development | 139 | 15462 | 34 | 2 | 0.037317295 | 0.141400508 | WNT4, ADIPOQ |
| upregulated | GO:0033674 | positive regulation of kinase activity | 139 | 15462 | 502 | 9 | 0.037478148 | 0.141830924 | LRRK2, BMP2, PTPRC, RGCC, ITGB3, TNFRSF11A, ADRA2A, MDFIC, ADIPOQ |
| upregulated | GO:0050773 | regulation of dendrite development | 139 | 15462 | 82 | 3 | 0.037641257 | 0.142089827 | LRRK2, PDLIM5, SDC2 |
| upregulated | GO:0072006 | nephron development | 139 | 15462 | 82 | 3 | 0.037641257 | 0.142089827 | WNT4, ADIPOQ, CALB1 |
| upregulated | GO:0043549 | regulation of kinase activity | 139 | 15462 | 751 | 12 | 0.038142595 | 0.143801416 | LRRK2, CBLC, BMP2, FABP4, PTPRC, RGCC, TNFRSF11A, ITGB3, ADRA2A, MDFIC, IL6, ADIPOQ |
| upregulated | GO:0002224 | toll-like receptor signaling pathway | 139 | 15462 | 141 | 4 | 0.03842679 | 0.144511337 | BIRC3, TLR1, IRF4, CD36 |
| upregulated | GO:0009059 | macromolecule biosynthetic process | 139 | 15462 | 4446 | 50 | 0.038427207 | 0.144511337 | GPC6, FAM129A, SHOX2, IRF4, HS3ST3B1, FABP4, ADRA2A, TNNI2, HOXA5, NFATC2, TFEC, NPAS2, GDF6, GALNT5, IL18R1, HIVEP3, CAMK2D, ITGB3, RGCC, PRDM6, BNC2, FOXO1, NR4A3, TLR1, PRELP, RFX2, RUNX1T1, EFEMP1, MDFIC, PPP1R3B, FOSL2, CD36, SATB2, ZFHX4, BIRC3, NR4A1, DLX5, SDC2, GCNT4, CDH13, BMP2, PTPRC, TNFRSF11A, GALNT14, WNT4, IL6, CHSY3, TBX5, NAMPT, ADIPOQ |
| upregulated | GO:0044281 | small molecule metabolic process | 139 | 15462 | 3115 | 37 | 0.038783441 | 0.145668468 | TAGAP, GPC6, HS3ST3B1, FABP4, ADRA2A, RAB30, NPAS2, GLRX, RASGRF2, AOX1, ADH1B, CXCL9, BCHE, ARHGAP42, SLC22A3, CRLS1, PRELP, SOS1, ALOX5AP, CD36, DOCK8, LRRK2, PRKG1, GCNT4, PDE1A, SDC2, PLIN1, ANGPTL4, BMP2, GUCY1A3, WNT4, IL6, CHSY3, NAMPT, SLC7A5, TDO2, ADIPOQ |
| upregulated | GO:0060828 | regulation of canonical Wnt signaling pathway | 139 | 15462 | 142 | 4 | 0.039274808 | 0.146696497 | WNT4, NKD1, DLX5, BMP2 |
| upregulated | GO:0030890 | positive regulation of B | 139 | 15462 | 35 | 2 | 0.03935 | 0.14669 | NFATC2, PTPRC |

| | | | | | | | | | |
|---|---|---|---|---|---|---|---|---|---|
| | | cell proliferation | | | | | 044 3 | 649 7 | |
| upreg ulated | GO:00 34121 | regulation of toll-like receptor signaling pathway | 139 | 154 62 | 35 | 2 | 0.03 935 044 3 | 0.14 669 649 7 | BIRC3, IRF4 |
| upreg ulated | GO:00 45912 | negative regulation of carbohydrate metabolic process | 139 | 154 62 | 35 | 2 | 0.03 935 044 3 | 0.14 669 649 7 | IL6, ADIPOQ |
| upreg ulated | GO:00 51963 | regulation of synapse assembly | 139 | 154 62 | 35 | 2 | 0.03 935 044 3 | 0.14 669 649 7 | ASIC2, PDLIM5 |
| upreg ulated | GO:00 50853 | B cell receptor signaling pathway | 139 | 154 62 | 35 | 2 | 0.03 935 044 3 | 0.14 669 649 7 | NFATC2, PTPRC |
| upreg ulated | GO:00 17145 | stem cell division | 139 | 154 62 | 143 | 4 | 0.04 013 349 4 | 0.14 924 487 6 | LRRK2, SHOX2, PTPRC, BMP2 |
| upreg ulated | GO:00 00302 | response to reactive oxygen species | 139 | 154 62 | 143 | 4 | 0.04 013 349 4 | 0.14 924 487 6 | NR4A3, IL6, CD36, FOXO1 |
| upreg ulated | GO:00 48863 | stem cell differentiation | 139 | 154 62 | 280 | 6 | 0.04 088 970 9 | 0.15 186 883 2 | RGCC, WNT4, TBX5, BCHE, BMP2, PTPRC |
| upreg ulated | GO:00 09896 | positive regulation of catabolic process | 139 | 154 62 | 144 | 4 | 0.04 100 285 1 | 0.15 191 303 1 | ADRA2A, LRRK2, NKD1, FOXO1 |
| upreg ulated | GO:00 30336 | negative regulation of cell migration | 139 | 154 62 | 144 | 4 | 0.04 100 285 1 | 0.15 191 303 1 | RGCC, WNT4, TBX5, ADIPOQ |
| upreg ulated | GO:00 46503 | glycerolipid catabolic process | 139 | 154 62 | 36 | 2 | 0.04 142 480 3 | 0.15 216 136 5 | PLIN1, FABP4 |
| upreg ulated | GO:00 51101 | regulation of DNA binding | 139 | 154 62 | 36 | 2 | 0.04 142 480 3 | 0.15 216 136 5 | IRF4, RUNX1T1 |
| upreg ulated | GO:00 60393 | regulation of pathway-restricted SMAD protein phosphorylation | 139 | 154 62 | 36 | 2 | 0.04 142 480 3 | 0.15 216 136 5 | GDF6, BMP2 |
| upreg ulated | GO:00 32890 | regulation of organic acid transport | 139 | 154 62 | 36 | 2 | 0.04 142 480 3 | 0.15 216 136 5 | TNFRSF11A, PLA2R1 |
| upreg ulated | GO:00 32760 | positive regulation of tumor necrosis factor production | 139 | 154 62 | 36 | 2 | 0.04 142 480 3 | 0.15 216 136 5 | CD2, CD36 |
| upreg ulated | GO:00 45058 | T cell selection | 139 | 154 62 | 36 | 2 | 0.04 142 480 3 | 0.15 216 136 5 | IL6, IRF4 |
| upreg ulated | GO:00 14909 | smooth muscle cell migration | 139 | 154 62 | 36 | 2 | 0.04 142 480 3 | 0.15 216 136 5 | ITGB3, ADIPOQ |
| upreg ulated | GO:00 42098 | T cell proliferation | 139 | 154 62 | 145 | 4 | 0.04 188 288 | 0.15 236 267 | WNT4, IL6, PTPRC, DOCK8 |

| | | | | | | | | | |
|---|---|---|---|---|---|---|---|---|---|
| | | | | | | | | 8 | |
| upregulated | GO:0048762 | mesenchymal cell differentiation | 139 | 15462 | 145 | 4 | 0.04188288 | 0.152362678 | RGCC, WNT4, TBX5, BMP2 |
| upregulated | GO:0006816 | calcium ion transport | 139 | 15462 | 282 | 6 | 0.042086442 | 0.152362678 | ADRA2A, SLC24A3, CXCL9, CCR1, PTPRC, CAMK2D |
| upregulated | GO:0044093 | positive regulation of molecular function | 139 | 15462 | 1573 | 21 | 0.042104745 | 0.152362678 | TAGAP, IRF4, SOS1, ADRA2A, MDFIC, ALOX5AP, DOCK8, LRRK2, BIRC3, PDE1A, IL18R1, RASGRF2, PTPRC, BMP2, TNFRSF11A, RGCC, ITGB3, WNT4, IL6, ARHGAP42, ADIPOQ |
| upregulated | GO:0032940 | secretion by cell | 139 | 15462 | 763 | 12 | 0.04220459 | 0.152362678 | LRRK2, TLR1, CCR1, RGCC, CD2, ITGB3, ADRA2A, LCP2, IL6, CBLN4, CD36, ADIPOQ |
| upregulated | GO:0045580 | regulation of T cell differentiation | 139 | 15462 | 86 | 3 | 0.042399769 | 0.152362678 | CD2, IL6, IRF4 |
| upregulated | GO:0031329 | regulation of cellular catabolic process | 139 | 15462 | 679 | 11 | 0.042501579 | 0.152362678 | TAGAP, LRRK2, PRKG1, RASGRF2, SOS1, DHX34, ADRA2A, WNT4, PPP1R3B, ARHGAP42, DOCK8 |
| upregulated | GO:0070838 | divalent metal ion transport | 139 | 15462 | 283 | 6 | 0.042693036 | 0.152362678 | ADRA2A, SLC24A3, CXCL9, CCR1, PTPRC, CAMK2D |
| upregulated | GO:0050708 | regulation of protein secretion | 139 | 15462 | 146 | 4 | 0.042773581 | 0.152362678 | RGCC, CD2, IL6, TLR1 |
| upregulated | GO:0051241 | negative regulation of multicellular organismal process | 139 | 15462 | 358 | 7 | 0.042979081 | 0.152362678 | RGCC, ADRA2A, IL6, TBX5, CCR1, GUCY1A3, ADIPOQ |
| upregulated | GO:0046651 | lymphocyte proliferation | 139 | 15462 | 213 | 5 | 0.043333634 | 0.152362678 | WNT4, IL6, NFATC2, PTPRC, DOCK8 |
| upregulated | GO:0051246 | regulation of protein metabolic process | 139 | 15462 | 1862 | 24 | 0.043348754 | 0.152362678 | FAM129A, CBLC, TLR1, IRF4, FABP4, ADRA2A, MDFIC, FEM1B, CD36, LRRK2, NKD1, BIRC3, NR4A1, GDF6, CAMK2D, PTPRC, BMP2, TNFRSF11A, ITGB3, RGCC, IL6, TFPI2, FOXO1, ADIPOQ |
| upregulated | GO:0032755 | positive regulation of interleukin-6 production | 139 | 15462 | 37 | 2 | 0.043539482 | 0.152362678 | IL6, CD36 |
| upregulated | GO:0014812 | muscle cell migration | 139 | 15462 | 37 | 2 | 0.043539482 | 0.152362678 | ITGB3, ADIPOQ |
| upregulated | GO:0050881 | musculoskeletal movement | 139 | 15462 | 37 | 2 | 0.043539482 | 0.152362678 | TNNI2, CHRNA1 |
| upregulated | GO:0050879 | multicellular organismal movement | 139 | 15462 | 37 | 2 | 0.043539482 | 0.152362678 | TNNI2, CHRNA1 |
| upregulated | GO:0090090 | negative regulation of canonical Wnt signaling pathway | 139 | 15462 | 87 | 3 | 0.043634461 | 0.152362678 | WNT4, NKD1, BMP2 |
| upregulated | GO:0034599 | cellular response to oxidative | 139 | 15462 | 147 | 4 | 0.04367 | 0.15236 | IL6, PLA2R1, CD36, FOXO1 |

| | | | | | | | | | |
|---|---|---|---|---|---|---|---|---|---|
| | | stress | | | | | 495 3 | 267 8 | |
| upreg ulated | GO:19 01897 | regulation of relaxation of cardiac muscle | 139 | 154 62 | 5 | 1 | 0.04 415 358 7 | 0.15 236 267 8 | CAMK2D |
| upreg ulated | GO:00 60745 | mammary gland branching involved in pregnancy | 139 | 154 62 | 5 | 1 | 0.04 415 358 7 | 0.15 236 267 8 | WNT4 |
| upreg ulated | GO:20 00096 | positive regulation of Wnt signaling pathway, planar cell polarity pathway | 139 | 154 62 | 5 | 1 | 0.04 415 358 7 | 0.15 236 267 8 | NKD1 |
| upreg ulated | GO:00 10739 | positive regulation of protein kinase A signaling | 139 | 154 62 | 5 | 1 | 0.04 415 358 7 | 0.15 236 267 8 | ADIPOQ |
| upreg ulated | GO:19 02803 | regulation of synaptic vesicle transport | 139 | 154 62 | 5 | 1 | 0.04 415 358 7 | 0.15 236 267 8 | LRRK2 |
| upreg ulated | GO:00 60385 | axonogenesis involved in innervation | 139 | 154 62 | 5 | 1 | 0.04 415 358 7 | 0.15 236 267 8 | NPTX1 |
| upreg ulated | GO:00 01781 | neutrophil apoptotic process | 139 | 154 62 | 5 | 1 | 0.04 415 358 7 | 0.15 236 267 8 | IL6 |
| upreg ulated | GO:00 60534 | trachea cartilage development | 139 | 154 62 | 5 | 1 | 0.04 415 358 7 | 0.15 236 267 8 | HOXA5 |
| upreg ulated | GO:00 72162 | metanephric mesenchymal cell differentiation | 139 | 154 62 | 5 | 1 | 0.04 415 358 7 | 0.15 236 267 8 | WNT4 |
| upreg ulated | GO:00 60100 | positive regulation of phagocytosis, engulfment | 139 | 154 62 | 5 | 1 | 0.04 415 358 7 | 0.15 236 267 8 | CD36 |
| upreg ulated | GO:00 70424 | regulation of nucleotide-binding oligomerization domain containing signaling pathway | 139 | 154 62 | 5 | 1 | 0.04 415 358 7 | 0.15 236 267 8 | BIRC3 |
| upreg ulated | GO:00 60126 | somatotropin secreting cell differentiation | 139 | 154 62 | 5 | 1 | 0.04 415 358 7 | 0.15 236 267 8 | WNT4 |
| upreg ulated | GO:00 61312 | BMP signaling pathway involved in heart development | 139 | 154 62 | 5 | 1 | 0.04 415 358 7 | 0.15 236 267 8 | BMP2 |
| upreg ulated | GO:00 72221 | metanephric distal convoluted tubule development | 139 | 154 62 | 5 | 1 | 0.04 415 358 7 | 0.15 236 267 8 | CALB1 |
| upreg ulated | GO:00 01302 | replicative cell aging | 139 | 154 62 | 5 | 1 | 0.04 415 358 7 | 0.15 236 267 8 | ERCC1 |
| upreg ulated | GO:00 31944 | negative regulation of glucocorticoid | 139 | 154 62 | 5 | 1 | 0.04 415 358 | 0.15 236 267 | BMP2 |

| | | | | | | | | | |
|---|---|---|---|---|---|---|---|---|---|
| | | metabolic process | | | | | 7 | 8 | |
| upreg ulated | GO:00 60480 | lung goblet cell differentiation | 139 | 154 62 | 5 | 1 | 0.04 415 358 7 | 0.15 236 267 8 | HOXA5 |
| upreg ulated | GO:00 90032 | negative regulation of steroid hormone biosynthetic process | 139 | 154 62 | 5 | 1 | 0.04 415 358 7 | 0.15 236 267 8 | BMP2 |
| upreg ulated | GO:00 72025 | distal convoluted tubule development | 139 | 154 62 | 5 | 1 | 0.04 415 358 7 | 0.15 236 267 8 | CALB1 |
| upreg ulated | GO:00 72537 | fibroblast activation | 139 | 154 62 | 5 | 1 | 0.04 415 358 7 | 0.15 236 267 8 | RGCC |
| upreg ulated | GO:00 10713 | negative regulation of collagen metabolic process | 139 | 154 62 | 5 | 1 | 0.04 415 358 7 | 0.15 236 267 8 | IL6 |
| upreg ulated | GO:00 35564 | regulation of kidney size | 139 | 154 62 | 5 | 1 | 0.04 415 358 7 | 0.15 236 267 8 | LRRK2 |
| upreg ulated | GO:00 51971 | positive regulation of transmission of nerve impulse | 139 | 154 62 | 5 | 1 | 0.04 415 358 7 | 0.15 236 267 8 | IL6 |
| upreg ulated | GO:00 45475 | locomotor rhythm | 139 | 154 62 | 5 | 1 | 0.04 415 358 7 | 0.15 236 267 8 | NPAS2 |
| upreg ulated | GO:00 35787 | cell migration involved in kidney development | 139 | 154 62 | 5 | 1 | 0.04 415 358 7 | 0.15 236 267 8 | ADIPOQ |
| upreg ulated | GO:00 51005 | negative regulation of lipoprotein lipase activity | 139 | 154 62 | 5 | 1 | 0.04 415 358 7 | 0.15 236 267 8 | ANGPTL4 |
| upreg ulated | GO:00 72033 | renal vesicle formation | 139 | 154 62 | 5 | 1 | 0.04 415 358 7 | 0.15 236 267 8 | WNT4 |
| upreg ulated | GO:00 71455 | cellular response to hyperoxia | 139 | 154 62 | 5 | 1 | 0.04 415 358 7 | 0.15 236 267 8 | FOXO1 |
| upreg ulated | GO:19 00121 | negative regulation of receptor binding | 139 | 154 62 | 5 | 1 | 0.04 415 358 7 | 0.15 236 267 8 | ADIPOQ |
| upreg ulated | GO:00 31947 | negative regulation of glucocorticoid biosynthetic process | 139 | 154 62 | 5 | 1 | 0.04 415 358 7 | 0.15 236 267 8 | BMP2 |
| upreg ulated | GO:00 35624 | receptor transactivation | 139 | 154 62 | 5 | 1 | 0.04 415 358 7 | 0.15 236 267 8 | ADRA2A |
| upreg ulated | GO:00 90237 | regulation of arachidonic acid secretion | 139 | 154 62 | 5 | 1 | 0.04 415 358 7 | 0.15 236 267 8 | PLA2R1 |
| upreg ulated | GO:00 36295 | cellular response to increased | 139 | 154 62 | 5 | 1 | 0.04 415 | 0.15 236 | FOXO1 |

| | | | | | | | | | |
|---|---|---|---|---|---|---|---|---|---|
| | | oxygen levels | | | | | 358 7 | 267 8 | |
| upreg ulated | GO:00 60481 | lobar bronchus epithelium development | 139 | 154 62 | 5 | 1 | 0.04 415 358 7 | 0.15 236 267 8 | HOXA5 |
| upreg ulated | GO:00 51128 | regulation of cellular component organization | 139 | 154 62 | 139 5 | 19 | 0.04 417 045 3 | 0.15 236 267 8 | SHOX2, TENM3, SEMA5A, PDLIM5, CD36, LRRK2, ELN, SDC2, CDH13, DPP4, CAMK2D, BMP2, RGCC, WNT4, ERCC1, TBX5, ASIC2, CNTN1, ADIPOQ |
| upreg ulated | GO:00 80090 | regulation of primary metabolic process | 139 | 154 62 | 522 4 | 57 | 0.04 436 470 8 | 0.15 285 704 6 | TAGAP, FAM129A, CBLC, SHOX2, IRF4, FABP4, ADRA2A, TNNI2, HOXA5, NFATC2, TFEC, NPAS2, NKD1, GDF6, RASGRF2, IL18R1, HIVEP3, CAMK2D, DHX34, ITGB3, RGCC, PRDM6, BNC2, ERCC1, TFPI2, CXCL9, FOXO1, ARHGAP42, NR4A3, TLR1, SOS1, RUNX1T1, RFX2, EFEMP1, MDFIC, FEM1B, PPP1R3B, FOSL2, CD36, SATB2, DOCK8, LRRK2, ZFHX4, BIRC3, NR4A1, PRKG1, DLX5, CDH13, BMP2, GUCY1A3, PTPRC, TNFRSF11A, WNT4, IL6, TBX5, NAMPT, ADIPOQ |
| upreg ulated | GO:00 51338 | regulation of transferase activity | 139 | 154 62 | 856 | 13 | 0.04 452 755 2 | 0.15 324 218 3 | LRRK2, CBLC, BMP2, FABP4, PTPRC, RGCC, TNFRSF11A, ITGB3, ADRA2A, IL6, MDFIC, FEM1B, ADIPOQ |
| upreg ulated | GO:20 00146 | negative regulation of cell motility | 139 | 154 62 | 148 | 4 | 0.04 458 699 2 | 0.15 327 097 7 | RGCC, WNT4, TBX5, ADIPOQ |
| upreg ulated | GO:00 32943 | mononuclear cell proliferation | 139 | 154 62 | 215 | 5 | 0.04 479 904 2 | 0.15 377 393 4 | WNT4, IL6, NFATC2, PTPRC, DOCK8 |
| upreg ulated | GO:00 51238 | sequestering of metal ion | 139 | 154 62 | 88 | 3 | 0.04 488 702 6 | 0.15 377 393 4 | CXCL9, PTPRC, CAMK2D |
| upreg ulated | GO:00 10594 | regulation of endothelial cell migration | 139 | 154 62 | 88 | 3 | 0.04 488 702 6 | 0.15 377 393 4 | RGCC, ITGB3, SEMA5A |
| upreg ulated | GO:00 50865 | regulation of cell activation | 139 | 154 62 | 362 | 7 | 0.04 516 034 1 | 0.15 453 384 8 | CD2, SLAMF7, IL6, NFATC2, IRF4, DPP4, PTPRC |
| upreg ulated | GO:00 02064 | epithelial cell development | 139 | 154 62 | 149 | 4 | 0.04 550 969 5 | 0.15 510 120 3 | WNT4, HOXA5, FEM1B, ADIPOQ |
| upreg ulated | GO:00 51480 | cytosolic calcium ion homeostasis | 139 | 154 62 | 216 | 5 | 0.04 554 279 | 0.15 510 120 3 | CXCL9, PTPRC, CCR1, CALB1, CAMK2D |
| upreg ulated | GO:00 35567 | non-canonical Wnt signaling pathway | 139 | 154 62 | 38 | 2 | 0.04 569 359 8 | 0.15 510 120 3 | WNT4, NKD1 |
| upreg ulated | GO:00 60323 | head morphogenesis | 139 | 154 62 | 38 | 2 | 0.04 569 359 8 | 0.15 510 120 3 | CRISPLD2, DLX5 |
| upreg ulated | GO:19 02930 | regulation of alcohol biosynthetic process | 139 | 154 62 | 38 | 2 | 0.04 569 359 8 | 0.15 510 120 3 | WNT4, BMP2 |
| upreg ulated | GO:00 50982 | detection of mechanical stimulus | 139 | 154 62 | 38 | 2 | 0.04 569 359 8 | 0.15 510 120 3 | ASIC2, PIEZO2 |

| | | | | | | | | | |
|---|---|---|---|---|---|---|---|---|---|
| upreg ulated | GO:00 32233 | positive regulation of actin filament bundle assembly | 139 | 154 62 | 38 | 2 | 0.04 569 359 8 | 0.15 510 120 3 | RGCC, WNT4 |
| upreg ulated | GO:00 43269 | regulation of ion transport | 139 | 154 62 | 441 | 8 | 0.04 573 960 8 | 0.15 510 120 3 | CCR1, CAMK2D, TNFRSF11A, ADRA2A, PLA2R1, ASIC2, CXCL9, CNTN1 |
| upreg ulated | GO:00 72511 | divalent inorganic cation transport | 139 | 154 62 | 288 | 6 | 0.04 580 869 9 | 0.15 516 016 5 | ADRA2A, SLC24A3, CXCL9, CCR1, PTPRC, CAMK2D |
| upreg ulated | GO:00 61572 | actin filament bundle organization | 139 | 154 62 | 89 | 3 | 0.04 615 738 3 | 0.15 581 361 8 | RGCC, WNT4, ELN |
| upreg ulated | GO:00 51017 | actin filament bundle assembly | 139 | 154 62 | 89 | 3 | 0.04 615 738 3 | 0.15 581 361 8 | RGCC, WNT4, ELN |
| upreg ulated | GO:00 46631 | alpha-beta T cell activation | 139 | 154 62 | 89 | 3 | 0.04 615 738 3 | 0.15 581 361 8 | IL6, IL18R1, IRF4 |
| upreg ulated | GO:00 02366 | leukocyte activation involved in immune response | 139 | 154 62 | 150 | 4 | 0.04 644 305 5 | 0.15 625 068 2 | IL6, ERCC1, IL18R1, IRF4 |
| upreg ulated | GO:00 08217 | regulation of blood pressure | 139 | 154 62 | 150 | 4 | 0.04 644 305 5 | 0.15 625 068 2 | ASIC2, GUCY1A3, COL1A2, ADIPOQ |
| upreg ulated | GO:00 02263 | cell activation involved in immune response | 139 | 154 62 | 150 | 4 | 0.04 644 305 5 | 0.15 625 068 2 | IL6, ERCC1, IL18R1, IRF4 |
| upreg ulated | GO:00 48704 | embryonic skeletal system morphogenesis | 139 | 154 62 | 90 | 3 | 0.04 744 544 6 | 0.15 944 432 8 | HOXA5, SHOX2, SATB2 |
| upreg ulated | GO:00 46637 | regulation of alpha-beta T cell differentiation | 139 | 154 62 | 39 | 2 | 0.04 788 628 3 | 0.16 002 977 3 | IL6, IRF4 |
| upreg ulated | GO:00 30199 | collagen fibril organization | 139 | 154 62 | 39 | 2 | 0.04 788 628 3 | 0.16 002 977 3 | COL12A1, COL1A2 |
| upreg ulated | GO:00 32330 | regulation of chondrocyte differentiation | 139 | 154 62 | 39 | 2 | 0.04 788 628 3 | 0.16 002 977 3 | EFEMP1, SHOX2 |
| upreg ulated | GO:00 35136 | forelimb morphogenesis | 139 | 154 62 | 39 | 2 | 0.04 788 628 3 | 0.16 002 977 3 | TBX5, SHOX2 |
| upreg ulated | GO:00 60996 | dendritic spine development | 139 | 154 62 | 39 | 2 | 0.04 788 628 3 | 0.16 002 977 3 | LRRK2, PDLIM5 |
| upreg ulated | GO:00 50808 | synapse organization | 139 | 154 62 | 152 | 4 | 0.04 834 172 1 | 0.16 065 726 | LRRK2, ASIC2, PDLIM5, CHRNA1 |
| upreg ulated | GO:00 02460 | adaptive immune response based on somatic recombination of immune receptors built | 139 | 154 62 | 152 | 4 | 0.04 834 172 1 | 0.16 065 726 | IL6, ERCC1, IL18R1, IRF4 |

| | | | | | | | | | |
|---|---|---|---|---|---|---|---|---|---|
| | | from immunoglobulin superfamily domains | | | | | | | |
| upregulated | GO:0042593 | glucose homeostasis | 139 | 15462 | 152 | 4 | 0.048341721 | 0.16065726 | ADRA2A, NPTX1, ADIPOQ, FOXO1 |
| upregulated | GO:0048754 | branching morphogenesis of an epithelial tube | 139 | 15462 | 152 | 4 | 0.048341721 | 0.16065726 | WNT4, HOXA5, SEMA5A, BMP2 |
| upregulated | GO:0033500 | carbohydrate homeostasis | 139 | 15462 | 152 | 4 | 0.048341721 | 0.16065726 | ADRA2A, NPTX1, ADIPOQ, FOXO1 |
| upregulated | GO:0015850 | organic hydroxy compound transport | 139 | 15462 | 153 | 4 | 0.04930701 | 0.162432521 | ADRA2A, CD36, ADIPOQ, SLC22A3 |
| upregulated | GO:0051271 | negative regulation of cellular component movement | 139 | 15462 | 153 | 4 | 0.04930701 | 0.162432521 | RGCC, WNT4, TBX5, ADIPOQ |
| upregulated | GO:0002696 | positive regulation of leukocyte activation | 139 | 15462 | 221 | 5 | 0.049372245 | 0.162432521 | CD2, IL6, NFATC2, DPP4, PTPRC |
| downregulated | GO:0008016 | regulation of heart contraction | 30 | 15462 | 130 | 3 | 0.001997243 | 0.322191584 | CHRM2, MYH6, GNAO1 |
| downregulated | GO:0060047 | heart contraction | 30 | 15462 | 153 | 3 | 0.003170858 | 0.322191584 | CHRM2, MYH6, GNAO1 |
| downregulated | GO:0003015 | heart process | 30 | 15462 | 154 | 3 | 0.003229644 | 0.322191584 | CHRM2, MYH6, GNAO1 |
| downregulated | GO:0072560 | type B pancreatic cell maturation | 30 | 15462 | 2 | 1 | 0.003876842 | 0.322191584 | RFX3 |
| downregulated | GO:0001895 | retina homeostasis | 30 | 15462 | 55 | 2 | 0.005070011 | 0.322191584 | GPR98, AZGP1 |
| downregulated | GO:0002071 | glandular epithelial cell maturation | 30 | 15462 | 3 | 1 | 0.005809811 | 0.322191584 | RFX3 |
| downregulated | GO:0007207 | phospholipase C-activating G-protein coupled acetylcholine receptor signaling pathway | 30 | 15462 | 3 | 1 | 0.005809811 | 0.322191584 | CHRM2 |
| downregulated | GO:2000078 | positive regulation of type B pancreatic cell development | 30 | 15462 | 3 | 1 | 0.005809811 | 0.322191584 | RFX3 |
| downregulated | GO:0060359 | response to ammonium ion | 30 | 15462 | 63 | 2 | 0.006604074 | 0.322191584 | GABRG2, GNAO1 |
| downregulated | GO:0036101 | leukotriene B4 catabolic process | 30 | 15462 | 4 | 1 | 0.00773915 | 0.32219158 | CYP4A11 |

| | | | | | | | | | |
|---|---|---|---|---|---|---|---|---|---|
| | | | | | | | 3 | 4 | |
| downregulated | GO:1901523 | icosanoid catabolic process | 30 | 15462 | 4 | 1 | 0.007739153 | 0.322191584 | CYP4A11 |
| downregulated | GO:0036100 | leukotriene catabolic process | 30 | 15462 | 4 | 1 | 0.007739153 | 0.322191584 | CYP4A11 |
| downregulated | GO:0036102 | leukotriene B4 metabolic process | 30 | 15462 | 4 | 1 | 0.007739153 | 0.322191584 | CYP4A11 |
| downregulated | GO:0035524 | proline transmembrane transport | 30 | 15462 | 4 | 1 | 0.007739153 | 0.322191584 | SLC6A15 |
| downregulated | GO:1901569 | fatty acid derivative catabolic process | 30 | 15462 | 4 | 1 | 0.007739153 | 0.322191584 | CYP4A11 |
| downregulated | GO:0051570 | regulation of histone H3-K9 methylation | 30 | 15462 | 5 | 1 | 0.009664876 | 0.322191584 | MYB |
| downregulated | GO:0051574 | positive regulation of histone H3-K9 methylation | 30 | 15462 | 5 | 1 | 0.009664876 | 0.322191584 | MYB |
| downregulated | GO:0002069 | columnar/cuboidal epithelial cell maturation | 30 | 15462 | 6 | 1 | 0.011586986 | 0.322191584 | RFX3 |
| downregulated | GO:0055009 | atrial cardiac muscle tissue morphogenesis | 30 | 15462 | 6 | 1 | 0.011586986 | 0.322191584 | MYH6 |
| downregulated | GO:0003228 | atrial cardiac muscle tissue development | 30 | 15462 | 6 | 1 | 0.011586986 | 0.322191584 | MYH6 |
| downregulated | GO:0042758 | long-chain fatty acid catabolic process | 30 | 15462 | 7 | 1 | 0.013505489 | 0.322191584 | CYP4A11 |
| downregulated | GO:0071420 | cellular response to histamine | 30 | 15462 | 7 | 1 | 0.013505489 | 0.322191584 | GABRG2 |
| downregulated | GO:2000074 | regulation of type B pancreatic cell development | 30 | 15462 | 7 | 1 | 0.013505489 | 0.322191584 | RFX3 |
| downregulated | GO:0045162 | clustering of voltage-gated sodium channels | 30 | 15462 | 7 | 1 | 0.013505489 | 0.322191584 | GLDN |
| downregulated | GO:0060287 | epithelial cilium movement involved in determination of left/right asymmetry | 30 | 15462 | 7 | 1 | 0.013505489 | 0.322191584 | RFX3 |
| downregulated | GO:0003008 | system process | 30 | 15462 | 1705 | 8 | 0.013653606 | 0.322191584 | OR52L1, CHRM2, CYP4A11, MYH6, GPR98, GNAO1, RBFOX1, AZGP1 |
| downregulated | GO:0048739 | cardiac muscle fiber development | 30 | 15462 | 8 | 1 | 0.015420393 | 0.322191584 | MYH6 |

| | | | | | | | | | |
|---|---|---|---|---|---|---|---|---|---|
| downregulated | GO:0032528 | microvillus organization | 30 | 15462 | 8 | 1 | 0.015420393 | 0.322191584 | GLDN |
| downregulated | GO:0015824 | proline transport | 30 | 15462 | 8 | 1 | 0.015420393 | 0.322191584 | SLC6A15 |
| downregulated | GO:0060285 | cilium-dependent cell motility | 30 | 15462 | 8 | 1 | 0.015420393 | 0.322191584 | RFX3 |
| downregulated | GO:0007186 | G-protein coupled receptor signaling pathway | 30 | 15462 | 1087 | 6 | 0.016448265 | 0.322191584 | GABRG2, OR52L1, NPY6R, CHRM2, GNAO1, GPR98 |
| downregulated | GO:0006816 | calcium ion transport | 30 | 15462 | 282 | 3 | 0.016942615 | 0.322191584 | MYB, GNAO1, CACHD1 |
| downregulated | GO:0070838 | divalent metal ion transport | 30 | 15462 | 283 | 3 | 0.017101974 | 0.322191584 | MYB, GNAO1, CACHD1 |
| downregulated | GO:0097267 | omega-hydroxylase P450 pathway | 30 | 15462 | 9 | 1 | 0.017331703 | 0.322191584 | CYP4A11 |
| downregulated | GO:0003091 | renal water homeostasis | 30 | 15462 | 9 | 1 | 0.017331703 | 0.322191584 | CYP4A11 |
| downregulated | GO:0006703 | estrogen biosynthetic process | 30 | 15462 | 9 | 1 | 0.017331703 | 0.322191584 | CYP19A1 |
| downregulated | GO:0002699 | positive regulation of immune effector process | 30 | 15462 | 106 | 2 | 0.017873443 | 0.322191584 | MYB, AZGP1 |
| downregulated | GO:0072511 | divalent inorganic cation transport | 30 | 15462 | 288 | 3 | 0.017911507 | 0.322191584 | MYB, GNAO1, CACHD1 |
| downregulated | GO:0048469 | cell maturation | 30 | 15462 | 109 | 2 | 0.018836785 | 0.322191584 | GLDN, RFX3 |
| downregulated | GO:0034776 | response to histamine | 30 | 15462 | 10 | 1 | 0.019239427 | 0.322191584 | GABRG2 |
| downregulated | GO:0019373 | epoxygenase P450 pathway | 30 | 15462 | 10 | 1 | 0.019239427 | 0.322191584 | CYP4A11 |
| downregulated | GO:0051571 | positive regulation of histone H3-K4 methylation | 30 | 15462 | 10 | 1 | 0.019239427 | 0.322191584 | MYB |
| downregulated | GO:0060736 | prostate gland growth | 30 | 15462 | 10 | 1 | 0.019239427 | 0.322191584 | CYP19A1 |
| downregulated | GO:0007188 | adenylate cyclase-modulating G-protein coupled receptor signaling pathway | 30 | 15462 | 118 | 2 | 0.021855358 | 0.322191584 | CHRM2, GNAO1 |

| | | | | | | | | | |
|---|---|---|---|---|---|---|---|---|---|
| downregulated | GO:0007213 | G-protein coupled acetylcholine receptor signaling pathway | 30 | 15462 | 12 | 1 | 0.023044139 | 0.322191584 | CHRM2 |
| downregulated | GO:0032305 | positive regulation of icosanoid secretion | 30 | 15462 | 12 | 1 | 0.023044139 | 0.322191584 | CYP4A11 |
| downregulated | GO:0045624 | positive regulation of T-helper cell differentiation | 30 | 15462 | 12 | 1 | 0.023044139 | 0.322191584 | MYB |
| downregulated | GO:0045161 | neuronal ion channel clustering | 30 | 15462 | 12 | 1 | 0.023044139 | 0.322191584 | GLDN |
| downregulated | GO:0060122 | inner ear receptor stereocilium organization | 30 | 15462 | 12 | 1 | 0.023044139 | 0.322191584 | GPR98 |
| downregulated | GO:0071242 | cellular response to ammonium ion | 30 | 15462 | 13 | 1 | 0.02494114 | 0.322191584 | GABRG2 |
| downregulated | GO:0044057 | regulation of system process | 30 | 15462 | 333 | 3 | 0.026158901 | 0.322191584 | CHRM2, MYH6, GNAO1 |
| downregulated | GO:0050910 | detection of mechanical stimulus involved in sensory perception of sound | 30 | 15462 | 14 | 1 | 0.026834581 | 0.322191584 | GPR98 |
| downregulated | GO:2000193 | positive regulation of fatty acid transport | 30 | 15462 | 14 | 1 | 0.026834581 | 0.322191584 | CYP4A11 |
| downregulated | GO:0007512 | adult heart development | 30 | 15462 | 14 | 1 | 0.026834581 | 0.322191584 | MYH6 |
| downregulated | GO:0043949 | regulation of cAMP-mediated signaling | 30 | 15462 | 133 | 2 | 0.027299117 | 0.322191584 | CHRM2, GNAO1 |
| downregulated | GO:0032303 | regulation of icosanoid secretion | 30 | 15462 | 15 | 1 | 0.028724467 | 0.322191584 | CYP4A11 |
| downregulated | GO:0002070 | epithelial cell maturation | 30 | 15462 | 15 | 1 | 0.028724467 | 0.322191584 | RFX3 |
| downregulated | GO:0043372 | positive regulation of CD4-positive, alpha-beta T cell differentiation | 30 | 15462 | 15 | 1 | 0.028724467 | 0.322191584 | MYB |
| downregulated | GO:0071705 | nitrogen compound transport | 30 | 15462 | 608 | 4 | 0.028833028 | 0.322191584 | SLC6A15, RFX3, SYBU, RBFOX1 |
| downregulated | GO:0003323 | type B pancreatic cell development | 30 | 15462 | 16 | 1 | 0.030610806 | 0.322191584 | RFX3 |
| downr egulat | GO:00 51567 | histone H3-K9 methylation | 30 | 154 62 | 16 | 1 | 0.03 061 | 0.32 219 | MYB |

| | | | | | | | | | |
|---|---|---|---|---|---|---|---|---|---|
| ed | | | | | | | 080 6 | 158 4 | |
| downregulated | GO:0045761 | regulation of adenylate cyclase activity | 30 | 15462 | 147 | 2 | 0.032821306 | 0.322191584 | CHRM2, GNAO1 |
| downregulated | GO:0006805 | xenobiotic metabolic process | 30 | 15462 | 148 | 2 | 0.033231367 | 0.322191584 | CYP19A1, CYP4A11 |
| downregulated | GO:0071466 | cellular response to xenobiotic stimulus | 30 | 15462 | 149 | 2 | 0.033643463 | 0.322191584 | CYP19A1, CYP4A11 |
| downregulated | GO:0007187 | G-protein coupled receptor signaling pathway, coupled to cyclic nucleotide second messenger | 30 | 15462 | 149 | 2 | 0.033643463 | 0.322191584 | CHRM2, GNAO1 |
| downregulated | GO:0031062 | positive regulation of histone methylation | 30 | 15462 | 18 | 1 | 0.034372863 | 0.322191584 | MYB |
| downregulated | GO:0006691 | leukotriene metabolic process | 30 | 15462 | 18 | 1 | 0.034372863 | 0.322191584 | CYP4A11 |
| downregulated | GO:0036465 | synaptic vesicle recycling | 30 | 15462 | 18 | 1 | 0.034372863 | 0.322191584 | SH3GL2 |
| downregulated | GO:0048488 | synaptic vesicle endocytosis | 30 | 15462 | 18 | 1 | 0.034372863 | 0.322191584 | SH3GL2 |
| downregulated | GO:0009083 | branched-chain amino acid catabolic process | 30 | 15462 | 18 | 1 | 0.034372863 | 0.322191584 | HIBCH |
| downregulated | GO:2000516 | positive regulation of CD4-positive, alpha-beta T cell activation | 30 | 15462 | 18 | 1 | 0.034372863 | 0.322191584 | MYB |
| downregulated | GO:0051569 | regulation of histone H3-K4 methylation | 30 | 15462 | 18 | 1 | 0.034372863 | 0.322191584 | MYB |
| downregulated | GO:0002068 | glandular epithelial cell development | 30 | 15462 | 18 | 1 | 0.034372863 | 0.322191584 | RFX3 |
| downregulated | GO:0019933 | cAMP-mediated signaling | 30 | 15462 | 151 | 2 | 0.034473722 | 0.322191584 | CHRM2, GNAO1 |
| downregulated | GO:0009410 | response to xenobiotic stimulus | 30 | 15462 | 152 | 2 | 0.034891869 | 0.322191584 | CYP19A1, CYP4A11 |
| downregulated | GO:0008015 | blood circulation | 30 | 15462 | 374 | 3 | 0.035184232 | 0.322191584 | CHRM2, MYH6, GNAO1 |
| downregulated | GO:0003013 | circulatory system process | 30 | 15462 | 376 | 3 | 0.035661162 | 0.322191584 | CHRM2, MYH6, GNAO1 |

| | | | | | | | | | |
|---|---|---|---|---|---|---|---|---|---|
| downregulated | GO:0055008 | cardiac muscle tissue morphogenesis | 30 | 15462 | 19 | 1 | 0.036248596 | 0.322191584 | MYH6 |
| downregulated | GO:0001916 | positive regulation of T cell mediated cytotoxicity | 30 | 15462 | 19 | 1 | 0.036248596 | 0.322191584 | AZGP1 |
| downregulated | GO:0060420 | regulation of heart growth | 30 | 15462 | 19 | 1 | 0.036248596 | 0.322191584 | MYH6 |
| downregulated | GO:0015804 | neutral amino acid transport | 30 | 15462 | 19 | 1 | 0.036248596 | 0.322191584 | SLC6A15 |
| downregulated | GO:0003351 | epithelial cilium movement | 30 | 15462 | 19 | 1 | 0.036248596 | 0.322191584 | RFX3 |
| downregulated | GO:0006811 | ion transport | 30 | 15462 | 1311 | 6 | 0.037364547 | 0.322191584 | GABRG2, SLC6A15, CYP4A11, MYB, GNAO1, CACHD1 |
| downregulated | GO:0098656 | anion transmembrane transport | 30 | 15462 | 158 | 2 | 0.037442429 | 0.322191584 | GABRG2, SLC6A15 |
| downregulated | GO:0008210 | estrogen metabolic process | 30 | 15462 | 20 | 1 | 0.038120806 | 0.322191584 | CYP19A1 |
| downregulated | GO:0003309 | type B pancreatic cell differentiation | 30 | 15462 | 20 | 1 | 0.038120806 | 0.322191584 | RFX3 |
| downregulated | GO:0001914 | regulation of T cell mediated cytotoxicity | 30 | 15462 | 20 | 1 | 0.038120806 | 0.322191584 | AZGP1 |
| downregulated | GO:0030001 | metal ion transport | 30 | 15462 | 672 | 4 | 0.039537437 | 0.322191584 | SLC6A15, MYB, GNAO1, CACHD1 |
| downregulated | GO:0050796 | regulation of insulin secretion | 30 | 15462 | 163 | 2 | 0.039621523 | 0.322191584 | RFX3, SYBU |
| downregulated | GO:0009081 | branched-chain amino acid metabolic process | 30 | 15462 | 21 | 1 | 0.0399895 | 0.322191584 | HIBCH |
| downregulated | GO:0007214 | gamma-aminobutyric acid signaling pathway | 30 | 15462 | 21 | 1 | 0.0399895 | 0.322191584 | GABRG2 |
| downregulated | GO:0002026 | regulation of the force of heart contraction | 30 | 15462 | 21 | 1 | 0.0399895 | 0.322191584 | MYH6 |
| downregulated | GO:0031279 | regulation of cyclase activity | 30 | 15462 | 166 | 2 | 0.040951901 | 0.322191584 | CHRM2, GNAO1 |
| downregulated | GO:0030817 | regulation of cAMP biosynthetic process | 30 | 15462 | 168 | 2 | 0.041848227 | 0.322191584 | CHRM2, GNAO1 |
| downregulated | GO:2000191 | regulation of fatty acid transport | 30 | 15462 | 22 | 1 | 0.04185468 | 0.32219158 | CYP4A11 |

| | | | | | | | | | |
|---|---|---|---|---|---|---|---|---|---|
| | | | | | | | 5 | 4 | |
| downregulated | GO:0035883 | enteroendocrine cell differentiation | 30 | 15462 | 22 | 1 | 0.041854685 | 0.322191584 | RFX3 |
| downregulated | GO:0032892 | positive regulation of organic acid transport | 30 | 15462 | 22 | 1 | 0.041854685 | 0.322191584 | CYP4A11 |
| downregulated | GO:0045622 | regulation of T-helper cell differentiation | 30 | 15462 | 22 | 1 | 0.041854685 | 0.322191584 | MYB |
| downregulated | GO:0051926 | negative regulation of calcium ion transport | 30 | 15462 | 22 | 1 | 0.041854685 | 0.322191584 | GNAO1 |
| downregulated | GO:0007212 | dopamine receptor signaling pathway | 30 | 15462 | 22 | 1 | 0.041854685 | 0.322191584 | GNAO1 |
| downregulated | GO:0001539 | cilium or flagellum-dependent cell motility | 30 | 15462 | 22 | 1 | 0.041854685 | 0.322191584 | RFX3 |
| downregulated | GO:0051339 | regulation of lyase activity | 30 | 15462 | 169 | 2 | 0.042299188 | 0.322191584 | CHRM2, GNAO1 |
| downregulated | GO:0006171 | cAMP biosynthetic process | 30 | 15462 | 170 | 2 | 0.042752003 | 0.322191584 | CHRM2, GNAO1 |
| downregulated | GO:0010927 | cellular component assembly involved in morphogenesis | 30 | 15462 | 171 | 2 | 0.043206663 | 0.322191584 | RFX3, MYH6 |
| downregulated | GO:0001894 | tissue homeostasis | 30 | 15462 | 172 | 2 | 0.043663159 | 0.322191584 | GPR98, AZGP1 |
| downregulated | GO:0021700 | developmental maturation | 30 | 15462 | 174 | 2 | 0.044581631 | 0.322191584 | GLDN, RFX3 |
| downregulated | GO:0046395 | carboxylic acid catabolic process | 30 | 15462 | 175 | 2 | 0.045043589 | 0.322191584 | CYP4A11, HIBCH |
| downregulated | GO:0016054 | organic acid catabolic process | 30 | 15462 | 175 | 2 | 0.045043589 | 0.322191584 | CYP4A11, HIBCH |
| downregulated | GO:0030030 | cell projection organization | 30 | 15462 | 1026 | 5 | 0.045494482 | 0.322191584 | GLDN, RFX3, GNAO1, GPR98, SH3GL2 |
| downregulated | GO:0060119 | inner ear receptor cell development | 30 | 15462 | 24 | 1 | 0.045574551 | 0.322191584 | GPR98 |
| downregulated | GO:0050974 | detection of mechanical stimulus involved in sensory perception | 30 | 15462 | 24 | 1 | 0.045574551 | 0.322191584 | GPR98 |
| downregulated | GO:0010817 | regulation of hormone levels | 30 | 15462 | 416 | 3 | 0.04590643 | 0.322191584 | CYP19A1, RFX3, SYBU |

| | | | | | | | | | |
|---|---|---|---|---|---|---|---|---|---|
| downregulated | GO:0090276 | regulation of peptide hormone secretion | 30 | 15462 | 179 | 2 | 0.046909379 | 0.322191584 | RFX3, SYBU |
| downregulated | GO:0043278 | response to morphine | 30 | 15462 | 25 | 1 | 0.047429245 | 0.322191584 | GNAO1 |
| downregulated | GO:0001913 | T cell mediated cytotoxicity | 30 | 15462 | 25 | 1 | 0.047429245 | 0.322191584 | AZGP1 |
| downregulated | GO:0034113 | heterotypic cell-cell adhesion | 30 | 15462 | 25 | 1 | 0.047429245 | 0.322191584 | GLDN |
| downregulated | GO:0060415 | muscle tissue morphogenesis | 30 | 15462 | 25 | 1 | 0.047429245 | 0.322191584 | MYH6 |
| downregulated | GO:0030814 | regulation of cAMP metabolic process | 30 | 15462 | 181 | 2 | 0.047852934 | 0.322191584 | CHRM2, GNAO1 |
| downregulated | GO:0006810 | transport | 30 | 15462 | 3838 | 12 | 0.048055387 | 0.322191584 | SLC6A15, FNBP1, SH3GL2, GABRG2, RFX3, CYP4A11, SYBU, MYB, RBFOX1, GNAO1, CACHD1, AZGP1 |
| downregulated | GO:0048002 | antigen processing and presentation of peptide antigen | 30 | 15462 | 183 | 2 | 0.048803509 | 0.322191584 | SH3GL2, AZGP1 |
| downregulated | GO:0050906 | detection of stimulus involved in sensory perception | 30 | 15462 | 428 | 3 | 0.049239168 | 0.322191584 | OR52L1, GPR98, AZGP1 |
| downregulated | GO:0002090 | regulation of receptor internalization | 30 | 15462 | 26 | 1 | 0.049280454 | 0.322191584 | SH3GL2 |
| downregulated | GO:0043370 | regulation of CD4-positive, alpha-beta T cell differentiation | 30 | 15462 | 26 | 1 | 0.049280454 | 0.322191584 | MYB |
| downregulated | GO:0003209 | cardiac atrium morphogenesis | 30 | 15462 | 26 | 1 | 0.049280454 | 0.322191584 | MYH6 |
| downregulated | GO:0014072 | response to isoquinoline alkaloid | 30 | 15462 | 26 | 1 | 0.049280454 | 0.322191584 | GNAO1 |
| downregulated | GO:0045494 | photoreceptor cell maintenance | 30 | 15462 | 26 | 1 | 0.049280454 | 0.322191584 | GPR98 |
| downregulated | GO:0002791 | regulation of peptide secretion | 30 | 15462 | 184 | 2 | 0.049281409 | 0.322191584 | RFX3, SYBU |

Table 11: AngioImmune Probeset Information

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3P.701 3.C1_s_at | Expression probeset | Sense (Fully Exonic) | 11 | ENSG000 0015165 1 | ADA M8 | 101 | ADAM metallopeptidase domain 8 [Source:HGNC Symbol;Acc:215] | Chr 10 | Revers e Strand | 303 |
| OC3P.105 46.C1_s_ at | Expression probeset | Sense (Fully Exonic) | 11 | ENSG000 0001277 9 | ALOX 5 | 240 | arachidonate 5-lipoxygenase [Source:HGNC Symbol;Acc:435] | Chr 10 | Forwar d Strand | 304 |
| OC3SNGn . 8557-31a_x_at | Expression probeset | Sense (Fully Exonic) | 11 | ENSG000 0013020 8 | APO C1 | 341 | apolipoprotein C-I [Source:HGNC Symbol;Acc:607] | Chr 19 | Forwar d Strand | 305 |
| OCMXSN G. 5067 _s _at | Expression probeset | Sense (Fully Exonic) | 11 | ENSG000 0016671 0 | B2M | 567 | beta-2-microglobulin [Source: HGNC Symbol;Acc:914] | Chr 15 | Forwar d Strand | 306 |
| OCADNP. 3105_s_a t | Expression probeset | Sense (includ es Introni c) | 11 | ENSG000 0016671 0 | B2M | 567 | beta-2-microglobulin [Source: HGNC Symbol;Acc:914] | Chr 15 | Forwar d Strand | 307 |
| OC3SNG. 1495-79a_s_at | Expression probeset | Sense (Fully Exonic) | 11 | ENSG000 0013030 3 | BST2 | 684 | bone marrow stromal cell antigen 2 [Source:HGNC Symbol;Acc: 1119] | Chr 19 | Revers e Strand | 308 |
| OC3SNGn h. 10611_ x_at | Expression probeset | Sense (Fully Exonic) | 7 | ENSG000 0013030 3 | BST2 | 684 | bone marrow stromal cell antigen 2 [Source:HGNC Symbol;Acc: 1119] | Chr 19 | Revers e Strand | 309 |
| OC3P.154 5.CB1_x_ at | Expression probeset | Sense (Fully Exonic) | 7 | ENSG000 0013030 3 | BST2 | 684 | bone marrow stromal cell antigen 2 [Source:HGNC Symbol;Acc: 1119] | Chr 19 | Revers e Strand | 310 |
| OC3P.546 8.C1_s_at | Expression probeset | Sense (Fully Exonic) | 11 | ENSG000 0017336 9 | C1Q B | 713 | complement component 1, q subcomponent, B chain [Source: HGNC Symbol;Acc:1242] | Chr 1 | Forwar d Strand | 311 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCMXSN G. 5528_s _at | Expression probeset | AntiSense | 11 | ENSG000 0015918 9 | C1Q C | 714 | complement component 1, q subcomponent, C chain [Source: HGNC Symbol;Acc:1245] | Chr 1 | Forward Strand | 312 |
| OC3SNG. 856-35a_x_at | Expression probeset | Sense (Fully Exonic) | 8 | ENSG000 0015918 9 | C1Q C | 714 | complement component 1, q subcomponent, C chain [Source: HGNC Symbol;Acc:1245] | Chr 1 | Forward Strand | 313 |
| OC3SNGn . 6006-1022a_s_ at | Expression probeset | Sense (Fully Exonic) | 7 | ENSG000 0018232 6 | C1S | 716 | complement component 1, s subcomponent [Source:HGNC Symbol;Acc:1247] | Chr 12 | Forward Strand | 314 |
| OCHP.88 7_s_at | Expression probeset | Sense (Fully Exonic) | 11 | ENSG000 0015923 1 | CBR3 | 874 | carbonyl reductase 3 [Source: HGNC Symbol;Acc:1549] | Chr 21 | Forward Strand | 315 |
| OC3P.925 1.C1_s_at | Expression probeset | Sense (Fully Exonic) | 11 | ENSG000 0001061 0 | CD4 | 920 | CD4 molecule [Source:HGNC Symbol;Acc:1678] | Chr 12 | Forward Strand | 316 |
| OC3P.473 2.C1_s_at | Expression probeset | Sense (Fully Exonic) | 11 | ENSG000 0002650 8 | CD44 | 960 | CD44 molecule (Indian blood group) [Source:HGNC Symbol; Acc:1681] | Chr 11 | Forward Strand | 317 |
| OCMX.67 O.CB2_at | Expression probeset | AntiSense | 9 | ENSG000 0001958 2 | CD74 | 972 | CD74 molecule, major histocompatibility complex, class II invariant chain [Source:HGNC Symbol;Acc:1697] | Chr 5 | Reverse Strand | 318 |
| OC3SNG. 3064-21a_x_at | Expression probeset | Sense (Fully Exonic) | 11 | ENSG000 0001958 2 | CD74 | 972 | CD74 molecule, major histocompatibility complex, class II invariant chain [Source:HGNC Symbol;Acc:1697] | Chr 5 | Reverse Strand | 319 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCHP.36 6_s_at | Expression probeset | Sense (Fully Exonic) | 11 | ENSG000 0016473 3 | CTSB | 1508 | cathepsin B [Source:HGNC Symbol;Acc:2527] | Chr 8 | Reverse Strand | 320 |
| OC3P.77. C1_s_at | Expression probeset | Sense (Fully Exonic) | 11 | ENSG000 0016473 3 | CTSB | 1508 | cathepsin B [Source:HGNC Symbol;Acc:2527] | Chr 8 | Reverse Strand | 321 |
| OCMX.24 32.C4_s_ at | Expression probeset | Sense (Fully Exonic) | 11 | ENSG000 0016473 3 | CTSB | 1508 | cathepsin B [Source:HGNC Symbol;Acc:2527] | Chr 8 | Reverse Strand | 322 |
| OCADNP. 7474_s_a t | Expression probeset | Sense (Fully Exonic) | 10 | ENSG000 0016313 1 | CTSS | 1520 | cathepsin S [Source:HGNC Sym bol;Acc:2545] | Chr 1 | Reverse Strand | 323 |
| OC3SNG. 3595-3338a_s_at | Expression probeset | Sense (Fully Exonic) | 11 | ENSG000 0008379 9 | CYLD | 1540 | cylindromatosis (turban tumor syndrome) [Source:HGNC Symbol;Acc:2584] | Chr 16 | Forward Strand | 324 |
| OCRS2.22 90 s at | Expression probeset | Sense (Fully Exonic) | 11 | ENSG000 0006535 7 | DGK A | 1606 | diacylglycerol kinase, alpha 80kDa [Source:HGNC Symbol; Acc:2849] | Chr 12 | Forward Strand | 325 |
| OC3SNG. 2053-58a_s_at | Expression probeset | Sense (Fully Exonic) | 11 | ENSG000 0016514 0 | FBP1 | 2203 | fructose-1,6-bisphosphatase 1 [Source:HGNC Symbol;Acc: 3606] | Chr 9 | Reverse Strand | 326 |
| OC3SNGn h. 2550_s _at | Expression probeset | Sense (Fully Exonic) | 11 | ENSG000 0015886 9 | FCER 1G | 2207 | Fc fragment of IgE, high affinity I, receptor for; gamma polypeptide [Source:HGNC Symbol;Acc: 3611] | Chr 1 | Forward Strand | 327 |
| OC3P.481 5.C1_s_at | Expression probeset | Sense (Fully Exonic) | 9 | ENSG000 0014322 6 | FCGR 2A | 2212 | Fc fragment of IgG, low affinity IIa, receptor (CD32) [Source:HGNC Sym bol;Acc:3616] | Chr 1 | Forward Strand | 328 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCMX.12 5.C1_s_at | Expression probeset | AntiSense | 10 | ENSG000 0011722 8 | GBP 1 | 2633 | guanylate binding protein 1, interferon-inducible [Source: HGNC Symbol;Acc:4182] | Chr 1 | Reverse Strand | 329 |
| OCADA.1 0565_s_a t | Expression probeset | Sense (Fully Exonic) | 11 | ENSG000 0011722 8 | GBP 1 | 2633 | guanylate binding protein 1, interferon-inducible [Source: HGNC Symbol;Acc:4182] | Chr 1 | Reverse Strand | 330 |
| OC3P.316 9.C1_s_at | Expression probeset | Sense (Fully Exonic) | 11 | ENSG000 0016264 5 | GBP 2 | 2634 | guanylate binding protein 2, interferon-inducible [Source: HGNC Symbol;Acc:4183] | Chr 1 | Reverse Strand | 331 |
| OCHP.14 36_s_at | Expression probeset | Sense (Fully Exonic) | 11 | ENSG000 0017322 1 | GLRX | 2745 | glutaredoxin (thioltransferase) [Source:HGNC Symbol;Acc: 4330] | Chr 5 | Reverse Strand | 332 |
| OCHP.34 5_s_at | Expression probeset | Sense (Fully Exonic) | 11 | ENSG000 0017579 3 | SFN | 2810 | stratifin [Source:HGNC Symbol; Acc:10773] | Chr 1 | Forward Strand | 333 |
| OC3P.522 7.C1_s_at | Expression probeset | Sense (Fully Exonic) | 11 | ENSG000 0018035 3 | HCLS 1 | 3059 | hematopoietic cell-specific Lyn substrate 1 [Source:HGNC Symbol;Acc:4844] | Chr 3 | Reverse Strand | 334 |
| OCRS2.25 71_s_at | Expression probeset | Sense (Fully Exonic) | 11 | ENSG000 0018035 3 | HCLS 1 | 3059 | hematopoietic cell-specific Lyn substrate 1 [Source:HGNC Symbol;Acc:4844] | Chr 3 | Reverse Strand | 335 |
| OC3SNGn . 6880-3840a_x_at | Expression probeset | Sense (Fully Exonic) | 9 | ENSG000 0020650 3 | HLA-A | 3105 | major histocompatibility complex, class I, A [Source:HGNC Symbol; Acc:4931] | Chr 6 | Forward Strand | 336 |
| OC3SNGn . 1244-62a_x_at | Expression probeset | Sense (Fully Exonic) | 11 | ENSG000 0020650 3 | HLA-A | 3105 | major histocompatibility complex, class I, A [Source:HGNC Symbol; Acc:4931] | Chr 6 | Forward Strand | 337 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3SNGn . 6460-38a_x_at | Expression probeset | Sense (Fully Exonic) | 9 | ENSG000 0020650 3 | HLA-A | 3105 | major histocompatibility complex, class I, A [Source:HGNC Symbol; Acc:4931] | Chr 6 | Forward Strand | 338 |
| OCRS2.73 1_x_at | Expression probeset | Sense (Fully Exonic) | 11 | ENSG000 0023474 5 | HLA-B | 3106 | major histocompatibility complex, class I, B [Source:HGNC Symbol; Acc:4932] | Chr 6 | Reverse Strand | 339 |
| OC3P. 141 .C12_x_at | Expression probeset | Sense (Fully Exonic) | 9 | ENSG000 0023474 5 | HLA-B | 3106 | major histocompatibility complex, class I, B [Source:HGNC Symbol; Acc:4932] | Chr 6 | Reverse Strand | 340 |
| OC3P.472 9.C1_s_at | Expression probeset | Sense (Fully Exonic) | 11 | ENSG000 0024257 4 | HLA-DMB | 3109 | major histocompatibility complex, class II, DM beta [Source:HGNC Symbol;Acc:4935] | Chr 6 | Reverse Strand | 341 |
| OC3SNGn . 2735-12a_s_at | Expression probeset | Sense (Fully Exonic) | 11 | ENSG000 0023138 9 | HLA-DPA 1 | 3113 | major histocompatibility complex, class II, DP alpha 1 [Source: HGNC Symbol;Acc:4938] | Chr 6 | Reverse Strand | 342 |
| OC3P.116 4.C1_s_at | Expression probeset | Sense (Fully Exonic) | 11 | ENSG000 0022386 5 | HLA-DPB1 | 3115 | major histocompatibility complex, class II, DP beta 1 [Source:HGNC Symbol;Acc:4940] | Chr 6 | Forward Strand | 343 |
| OC3P. 141 .C13_s_at | Expression probeset | Sense (Fully Exonic) | 11 | ENSG000 0020464 2 | HLA-F | 3134 | major histocompatibility complex, class I, F [Source:HGNC Symbol; Acc:4963] | Chr 6 | Forward Strand | 344 |
| OCRS2.28 19_x_at | Expression probeset | Sense (Fully Exonic) | 11 | ENSG000 0020464 2 | HLA-F | 3134 | major histocompatibility complex, class I, F [Source:HGNC Symbol; Acc:4963] | Chr 6 | Forward Strand | 345 |
| OCRS2.28 19_at | Expression probeset | Sense (Fully Exonic) | 11 | ENSG000 0020464 2 | HLA-F | 3134 | major histocompatibility complex, class I, F [Source:HGNC Symbol; Acc:4963] | Chr 6 | Forward Strand | 346 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3P.246 0.C1_s_at | Expression probeset | Sense (Fully Exonic) | 11 | ENSG000 0011992 2 | IFIT2 | 3433 | interferon-induced protein with tetratricopeptide repeats 2 [Source:HGNC Symbol;Acc: 5409] | Chr 10 | Forward Strand | 347 |
| OCHP.48 9_s_at | Expression probeset | Sense (Fully Exonic) | 11 | ENSG000 0013668 9 | IL1R N | 3557 | interleukin 1 receptor antagonist [Source:HGNC Symbol;Acc: 6000] | Chr 2 | Forward Strand | 348 |
| OC3P.443 5.C1-401a_s_a t | Expression probeset | Sense (Fully Exonic) | 11 | ENSG000 0012534 7 | IRF1 | 3659 | interferon regulatory factor 1 [Source:HGNC Symbol;Acc: 6116] | Chr 5 | Reverse Strand | 349 |
| OCRS2.43 10_s_at | Expression probeset | Sense (Fully Exonic) | 11 | ENSG000 0016025 5 | ITGB 2 | 3689 | integrin, beta 2 (complement component 3 receptor 3 and 4 subunit) [Source:HGNC Symbol; Acc:6155] | Chr 21 | Reverse Strand | 350 |
| OC3P.872 2.C1_s_at | Expression probeset | Sense (Fully Exonic) | 11 | ENSG000 0016025 5 | ITGB 2 | 3689 | integrin, beta 2 (complement component 3 receptor 3 and 4 subunit) [Source:HGNC Symbol; Acc:6155] | Chr 21 | Reverse Strand | 351 |
| OC3P.103 3.C1_s_at | Expression probeset | Sense (Fully Exonic) | 11 | ENSG000 0016896 1 | LGAL S9 | 3965 | lectin, galactoside-binding, soluble, 9 [Source:HGNC Symbol; Acc:6570] | Chr 17 | Forward Strand | 352 |
| OC3P.544 9.C1_at | Expression probeset | Sense (Fully Exonic) | 8 | ENSG000 0011830 8 | LRM P | 4033 | lymphoid-restricted membrane protein [Source:HGNC Symbol; Acc:6690] | Chr 12 | Forward Strand | 353 |
| OCMX.55 83.C1_s_ at | Expression probeset | AntiSense | 11 | ENSG000 0022750 7 | LTB | 4050 | lymphotoxin beta (TNF superfamily, member 3) [Source: HGNC Symbol;Acc:6711] | Chr 6 | Reverse Strand | 354 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3P. 805 .C1_s_at | Expression probeset | Sense (Fully Exonic) | 11 | ENSG000 0017958 3 | CIITA | 4261 | class II, major histocompatibility complex, transactivator [Source: HGNC Symbol;Acc:7067] | Chr 16 | Forward Strand | 355 |
| OC3SNGn . 5100-4676a_s_at | Expression probeset | Sense (Fully Exonic) | 10 | ENSG000 0013767 3 | MM P7 | 4316 | matrix metallopeptidase 7 (matrilysin, uterine) [Source: HGNC Symbol;Acc:7174] | Chr 11 | Reverse Strand | 356 |
| OC3SNGn h. 19645_ s_at | Expression probeset | Sense (Fully Exonic) | 10 | ENSG000 0015760 1 | MX1 | 4599 | myxovirus (influenza virus) resistance 1, interferon-inducible protein p78 (mouse) [Source: HGNC Symbol;Acc:7532] | Chr 21 | Forward Strand | 357 |
| OCHP.16 40_s_at | Expression probeset | Sense (Fully Exonic) | 11 | ENSG000 0016674 1 | NNM T | 4837 /// 1019 2891 6 | nicotinamide N-methyltransferase [Source:HGNC Symbol;Acc: 7861] | Chr 11 | Forward Strand | 358 |
| OC3P.601 1.C1_s_at | Expression probeset | Sense (Fully Exonic) | 11 | ENSG000 0019794 3 | PLCG 2 | 5336 | phospholipase C, gamma 2 (phosphatidylinositol-specific) [Source:HGNC Symbol;Acc: 9066] | Chr 16 | Forward Strand | 359 |
| OCRS2.45 48_s_at | Expression probeset | Sense (Fully Exonic) | 11 | ENSG000 0014046 4 | PML | 5371 | promyelocytic leukemia [Source: HGNC Symbol;Acc:9113] | Chr 15 | Forward Strand | 360 |
| OC3SNG. 1855-2142a_s_at | Expression probeset | Sense (Fully Exonic) | 11 | ENSG000 0013006 6 | SAT1 | 6303 | spermidine/spermine N1-acetyltransferase 1 [Source: HGNC Symbol;Acc:10540] | Chr X | Forward Strand | 361 |
| OCHPRC. 804_s_at | Expression probeset | AntiSense | 9 | ENSG000 0013006 6 | SAT1 | 6303 | spermidine/spermine N1-acetyltransferase 1 [Source: HGNC Symbol;Acc:10540] | Chr X | Forward Strand | 362 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3P.935 5.C1_s_at | Expression probeset | AntiSense | 11 | ENSG000 0013006 6 | SAT1 | 6303 | spermidine/spermine N1-acetyltransferase 1 [Source: HGNC Symbol;Acc:10540] | Chr X | Forward Strand | 363 |
| OCHP.18 27_s_at | Expression probeset | Sense (Fully Exonic) | 11 | ENSG000 0008882 7 | SIGLEC1 | 6614 | sialic acid binding Ig-like lectin 1, sialoadhesin [Source:HGNC Symbol;Acc:11127] | Chr 20 | Reverse Strand | 364 |
| OCHP.15 88_s_at | Expression probeset | Sense (Fully Exonic) | 11 | ENSG000 0011541 5 | STAT 1 | 6772 | signal transducer and activator of transcription 1, 91kDa [Source: HGNC Symbol;Acc:11362] | Chr 2 | Reverse Strand | 365 |
| Adx-Hs-ISGF3A-300-3_x_at | Almac redesigned standard human control | Sense (Fully Exonic) | 10 | ENSG000 0011541 5 | STAT 1 | 6772 | signal transducer and activator of transcription 1, 91kDa [Source: HGNC Symbol;Acc:11362] | Chr 2 | Reverse Strand | 366 |
| Adx-Hs-ISGF3A-400-3_x_at | Almac redesigned standard human control | Sense (Fully Exonic) | 9 | ENSG000 0011541 5 | STAT 1 | 6772 | signal transducer and activator of transcription 1, 91kDa [Source: HGNC Symbol;Acc:11362] | Chr 2 | Reverse Strand | 367 |
| OCADNP. 3111_s_a t | Expression probeset | Sense (includes Intronic) | 11 | ENSG000 0011541 5 | STAT 1 | 6772 | signal transducer and activator of transcription 1, 91kDa [Source: HGNC Symbol;Acc:11362] | Chr 2 | Reverse Strand | 368 |
| OC3SNG. 2984-24a_s_at | Expression probeset | Sense (Fully Exonic) | 10 | ENSG000 0001160 0 | TYROBP | 7305 | TYRO protein tyrosine kinase binding protein [Source:HGNC Symbol;Acc:12449] | Chr 19 | Reverse Strand | 369 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3P.728 4.C1_s_at | Expression probeset | Sense (Fully Exonic) | 11 | ENSG000 0016269 2 | VCA M1 | 7412 | vascular cell adhesion molecule 1 [Source:HGNC Symbol;Acc: 12663] | Chr 1 | Forward Strand | 370 |
| OC3P.530 .Cl-561a_s_a t | Expression probeset | Sense (Fully Exonic) | 11 | ENSG000 0010021 9 | XBP1 | 7494 | X-box binding protein 1 [Source: HGNC Symbol;Acc:12801] | Chr 22 | Reverse Strand | 371 |
| OC3P.115 19.C1_s_ at | Expression probeset | Sense (Fully Exonic) | 11 | ENSG000 0006942 4 | KCN AB2 | 8514 | potassium voltage-gated channel, shaker-related subfamily, beta member 2 [Source:HGNC Symbol;Acc:6229] | Chr 1 | Forward Strand | 372 |
| OCMXSN G. 5608_a t | Expression probeset | AntiSense | 11 | ENSG000 0010034 2 | APOL 1 | 8542 | apolipoprotein L, 1 [Source:HGNC Symbol;Acc:618] | Chr 22 | Forward Strand | 373 |
| OC3P.117 7.C1_x_at | Expression probeset | Sense (Fully Exonic) | 11 | ENSG000 0010034 2 | APOL 1 | 8542 | apolipoprotein L, 1 [Source:HGNC Symbol;Acc:618] | Chr 22 | Forward Strand | 374 |
| OC3P.706 8.C1_s_at | Expression probeset | Sense (Fully Exonic) | 11 | ENSG000 0015658 7 | UBE2 L6 | 9246 | ubiquitin-conjugating enzyme E2L 6 [Source:HGNC Symbol;Acc: 12490] | Chr 11 | Reverse Strand | 375 |
| OC3SNGn . 2005-402a_s_a t | Expression probeset | Sense (Fully Exonic) | 11 | ENSG000 0017757 5 | CD16 3 | 9332 | CD163 molecule [Source:HGNC Symbol;Acc:1631] | Chr 12 | Reverse Strand | 376 |
| OC3P.593 0.C1_at | Expression probeset | Sense (Fully Exonic) | 11 | ENSG000 0018906 7 | LITA F | 9516 | lipopolysaccharide-induced TNF factor [Source:HGNC Symbol; Acc:16841] | Chr 16 | Reverse Strand | 377 |

(continued)

| Probe Set ID | Type | Orient ation | No. prob es align ed | Ensembl Gene | Gene Sym bol | Entr ez Gene ID | Description | Chro mos ome | Strand | SEQ ID NO |
|---|---|---|---|---|---|---|---|---|---|---|
| OC3P.986 9.C1_s_at | Expression probeset | Sense (Fully Exonic) | 11 | ENSG000 0020410 3 | MAF B | 9935 | v-maf avian musculoaponeurotic fibrosarcoma oncogene homolog B [Source:HGNC Symbol;Acc: 6408] | Chr 20 | Revers e Strand | 378 |
| OC3SNG. 4002-20a_s_at | Expression probeset | Sense (Fully Exonic) | 10 | ENSG000 0006883 1 | RAS GRP 2 | 1023 5 | RAS guanyl releasing protein 2 (calcium and DAG-regulated) [Source:HGNC Symbol;Acc: 9879] | Chr 11 | Revers e Strand | 379 |
| OC3P. 616 .C1_s_at | Expression probeset | Sense (Fully Exonic) | 11 | ENSG000 0013623 5 | GPN MB | 1045 7 | glycoprotein (transmembrane) nmb [Source:HGNC Symbol;Acc: 4462] | Chr 7 | Forwar d Strand | 380 |
| OC3P.133 91.C1_s_ at | Expression probeset | Sense (Fully Exonic) | 11 | ENSG000 0019684 3 | ARID 5A | 1086 5 | AT rich interactive domain 5A (MRF1-like) [Source:HGNC Symbol;Acc:17361] | Chr 2 | Forwar d Strand | 381 |
| OC3P.131 44.C1-468a_s_a t | Expression probeset | Sense (Fully Exonic) | 11 | ENSG000 0018044 8 | HMH A1 | 2352 6 | histocompatibility (minor) HA-1 [Source:HGNC Symbol;Acc: 17102] | Chr 19 | Forwar d Strand | 382 |
| OC3SNGn . 1535-1076a_s_ at | Expression probeset | Sense (Fully Exonic) | 11 | ENSG000 0015277 8 | IFIT5 | 2413 8 | interferon-induced protein with tetratricopeptide repeats 5 [Source:HGNC Symbol;Acc: 13328] | Chr 10 | Forwar d Strand | 383 |
| OC3P.110 92.C1_s_ at | Expression probeset | Sense (Fully Exonic) | 11 | ENSG000 0019862 4 | CCD C69 | 2611 2 | coiled-coil domain containing 69 [Source:HGNC Symbol;Acc: 24487] | Chr 5 | Revers e Strand | 384 |
| OC3P.229 3.C2_x_at | Expression probeset | Sense (Fully Exonic) | 9 | ENSG000 0017604 6 | NUP R1 | 2647 1 | nuclear protein, transcriptional regulator, 1 [Source:HGNC Sym bol;Acc:29990] | Chr 16 | Revers e Strand | 385 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3SNGn . 883-5a_s_at | Expression probeset | Sense (Fully Exonic) | 9 | ENSG000 0009597 0 | TRE M2 | 5420 9 | triggering receptor expressed on myeloid cells 2 [Source:HGNC Symbol;Acc:17761] | Chr 6 | Reverse Strand | 386 |
| OC3SNGn h. 2575_s at | Expression probeset | Sense (includes Intronic) | 11 | ENSG000 0017319 3 | PARP 14 | 5462 5 | poly (ADP-ribose) polymerase family, member 14 [Source:HGNC Symbol;Acc:29232] | Chr 3 | Forward Strand | 387 |
| OC3SNG. 2605-236a_x_a t | Expression probeset | Sense (Fully Exonic) | 11 | ENSG000 0013253 0 | XAF1 | 5473 9 | XIAP associated factor 1 [Source: HGNC Sym bol;Acc:30932] | Chr 17 | Forward Strand | 388 |
| OC3P.487 3.C1_s_at | Expression probeset | Sense (Fully Exonic) | 11 | ENSG000 0013253 0 | XAF1 | 5473 9 | XIAP associated factor 1 [Source: HGNC Sym bol;Acc:30932] | Chr 17 | Forward Strand | 389 |
| OC3P.832 O.C1_s_at | Expression probeset | Sense (Fully Exonic) | 11 | ENSG000 0010895 0 | FAM 20A | 5475 7 | family with sequence similarity 20, member A [Source:HGNC Symbol;Acc:23015] | Chr 17 | Reverse Strand | 390 |
| OC3P.831 4.C1_s_at | Expression probeset | Sense (Fully Exonic) | 11 | ENSG000 0020541 3 | SAM D9 | 5480 9 | sterile alpha motif domain containing 9 [Source:HGNC Symbol;Acc:1348] | Chr 7 | Reverse Strand | 391 |
| OC3P.631 6.C1_s_at | Expression probeset | Sense (Fully Exonic) | 11 | ENSG000 0018350 8 | FAM 46C | 5485 5 | family with sequence similarity 46, member C [Source:HGNC Symbol;Acc:24712] | Chr 1 | Forward Strand | 392 |
| OCADA.1 0345_s_a t | Expression probeset | Sense (Fully Exonic) | 11 | ENSG000 0018350 8 | FAM 46C | 5485 5 | family with sequence similarity 46, member C [Source:HGNC Symbol;Acc:24712] | Chr 1 | Forward Strand | 393 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3SNG. 2106-17a_s_at | Expression probeset | Sense (Fully Exonic) | 11 | ENSG000 0011968 6 | FLVC R2 | 5564 0 | feline leukemia virus subgroup C cellular receptor family, member 2 [Source:HGNC Symbol;Acc: 20105] | Chr 14 | Forward Strand | 394 |
| OCADNP. 5178_s_a t | Expression probeset | Sense (Fully Exonic) | 11 | ENSG000 0016645 2 | AKIP 1 | 5667 2 | A kinase (PRKA) interacting protein 1 [Source:HGNC Symbol; Acc:1170] | Chr 11 | Forward Strand | 395 |
| OCADA.1 0811_s_a t | Expression probeset | Sense (Fully Exonic) | 8 | ENSG000 0002675 1 | SLA MF7 | 5782 3 | SLAM family member 7 [Source: HGNC Symbol;Acc:21394] | Chr 1 | Forward Strand | 396 |
| OC3P.102 80.C1_s_ at | Expression probeset | Sense (Fully Exonic) | 11 | ENSG000 0011526 7 | IFIH1 | 6413 5 | interferon induced with helicase C domain 1 [Source:HGNC Sym bol; Acc:18873] | Chr 2 | Reverse Strand | 397 |
| OCRS.727 _s_at | Expression probeset | Sense (Fully Exonic) | 11 | ENSG000 0019709 3 | GAL3 ST4 | 7969 0 | galactose-3-O-sulfotransferase 4 [Source:HGNC Symbol;Acc: 24145] | Chr 7 | Reverse Strand | 398 |
| OC3P.755 7.C1_s_at | Expression probeset | Sense (Fully Exonic) | 9 | ENSG000 0014085 3 | NLRC 5 | 8416 6 | NLR family, CARD domain containing 5 [Source:HGNC Symbol;Acc:29933] | Chr 16 | Forward Strand | 399 |
| OCADA.3 339_s_at | Expression probeset | Sense (Fully Exonic) | 11 | ENSG000 0009995 8 | DERL 3 | 9131 9 | derlin 3 [Source:HGNC Symbol; Acc:14236] | Chr 22 | Reverse Strand | 400 |
| OC3SNGn . 3058-31a_s_at | Expression probeset | Sense (Fully Exonic) | 11 | ENSG000 0015445 1 | GBP 5 | 1153 62 | guanylate binding protein 5 [Source:HGNC Symbol;Acc: 19895] | Chr 1 | Reverse Strand | 401 |
| OCMXSN G. 2584_s _at | Expression probeset | AntiSense | 11 | ENSG000 0016806 2 | BATF 2 | 1160 71 | basic leucine zipper transcription factor, ATF-like 2 [Source:HGNC Symbol;Acc:25163] | Chr 11 | Reverse Strand | 402 |

(continued)

| Probe Set ID | Type | Orient ation | No. prob es align ed | Ensembl Gene | Gene Sym bol | Entr ez Gene ID | Description | Chro mos ome | Strand | SEQ ID NO |
|---|---|---|---|---|---|---|---|---|---|---|
| OCADA.8 743_s_at | Expression probeset | Sense (Fully Exonic) | 11 | ENSG000 0017057 1 | EMB | 1334 18 | embigin [Source:HGNC Sym bol; Acc:30465] | Chr 5 | Revers e Strand | 403 |
| OCMXSN G. 448_s_ at | Expression probeset | AntiSe nse | 11 | ENSG000 0017740 9 | SAM D9L | 2192 85 | sterile alpha motif domain containing 9-like [Source:HGNC Symbol;Acc:1349] | Chr 7 | Revers e Strand | 404 |
| OC3P.104 87.C1_s_ at | Expression probeset | Sense (Fully Exonic) | 11 | ENSG000 0017740 9 | SAM D9L | 2192 85 | sterile alpha motif domain containing 9-like [Source:HGNC Symbol;Acc:1349] | Chr 7 | Revers e Strand | 405 |
| OC3P. 969 .C1_s_at | Expression probeset | Sense (Fully Exonic) | 11 | ENSG000 0010495 1 | IL4I1 | 2593 07 | interleukin 4 induced 1 [Source: HGNC Symbol;Acc:19094] | Chr 19 | Revers e Strand | 406 |
| OCADA.5 772_s_at | Expression probeset | Sense (Fully Exonic) | 11 | ENSG000 0017798 9 | ODF 3B | 4408 36 | outer dense fiber of sperm tails 3B [Source:HGNC Sym bol;Acc: 34388] | Chr 22 | Revers e Strand | 407 |
| OCMXSN G. 5626_s at | Expression probeset | AntiSe nse | 11 | ENSG000 0017798 9 | ODF 3B | 4408 36 | outer dense fiber of sperm tails 3B [Source:HGNC Symbol;Acc: 34388] | Chr 22 | Revers e Strand | 408 |
| OCADA.8 654_s_at | Expression probeset | Sense (Fully Exonic) | 11 | ENSG000 0018882 0 | FAM 26F | 4411 68 | family with sequence similarity 26, member F [Source:HGNC Symbol;Acc:33391] | Chr 6 | Forwar d Strand | 409 |
| OC3P.144 83.C1_s_ at | Expression probeset | Sense (Fully Exonic) | 11 | ENSG000 0011209 6 | SOD 2 | 6648 /// 1001 2951 8 | superoxide dismutase 2, mitochondrial [Source:HGNC Symbol;Acc:11180] | Chr 6 | Revers e Strand | 410 |
| OCMX.15 525.C1_s _at | Expression probeset | Sense (Fully Exonic) | 11 | ENSG000 0013795 9 | IFI44 L | 1096 4 | interferon-induced protein 44-like [Source:HGNC Symbol;Acc: 17817] | Chr 1 | Forwar d Strand | 411 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3P.690 3.C1_at | Expression probeset | Sense (Fully Exonic) | 10 | ENSG000 0013795 9 | IFI44 L | 1096 4 | interferon-induced protein 44-like [Source:HGNC Symbol;Acc: 17817] | Chr 1 | Forward Strand | 412 |
| OC3SNG. 3004-20a_s_at | Expression probeset | Sense (Fully Exonic) | 10 | ENSG000 0022196 3 | APOL 6 | 8083 0 | apolipoprotein L, 6 [Source:HGNC Symbol;Acc:14870] | Chr 22 | Forward Strand | 413 |
| OCMXSN G. 1782_a t | Expression probeset | Sense (includes Intronic) | 11 | ENSG000 0023644 9 | N/A | N/A | NOVEL antisense (Clone_based_ vega_gene) | Chr 2 | Forward Strand | 414 |
| OC3SNGn . 6005-4a_x_at | Expression probeset | Insufficient probes (< 6) | 0 | 0 | 0 | 0 | 0 | 0 | 0 | 415 |
| OC3P. 264 .C16_x_at | Expression probeset | Insufficient probes (< 6) | 0 | 0 | 0 | 0 | 0 | 0 | 0 | 416 |
| OC3SNG. 1434-27a_x_at | Expression probeset | Insufficient probes (< 6) | 0 | 0 | 0 | 0 | 0 | 0 | 0 | 417 |
| OC3P. 264 .C2_x_at | Expression probeset | Insufficient probes (< 6) | 0 | 0 | 0 | 0 | 0 | 0 | 0 | 418 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3P. 264 .CB1_x_a t | Expression probeset | Insufficient probes (< 6) | 0 | 0 | 0 | 0 | 0 | 0 | 0 | 419 |
| OC3P. 264 .CB5_x_a t | Expression probeset | Insufficient probes (< 6) | 0 | 0 | 0 | 0 | 0 | 0 | 0 | 420 |
| OC3SNGn . 5999-435a_x_a t | Expression probeset | No Genome match | 0 | 0 | 0 | 0 | 0 | 0 | 0 | 421 |
| OC3P. 264 .C10_x_at | Expression probeset | Insufficient probes (< 6) | 0 | 0 | 0 | 0 | 0 | 0 | 0 | 422 |
| OC3P.42. CB2_x_at | Expression probeset | Insufficient probes (< 6) | 0 | 0 | 0 | 0 | 0 | 0 | 0 | 423 |
| OCADNP. 9529_x_a t | Expression probeset | Insufficient probes (< 6) | 0 | 0 | 0 | 0 | 0 | 0 | 0 | 424 |
| OC3P. 121 .C9_x_at | Expression probeset | Insufficient probes (< 6) | 0 | 0 | 0 | 0 | 0 | 0 | 0 | 425 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCADNP. 6175_x_a t | Expression probeset | Insufficient probes (< 6) | 0 | 0 | 0 | 0 | 0 | 0 | 0 | 426 |
| OC3P. 264 .C21_x_at | Expression probeset | Sense (Fully Exonic) | 11 | ENSG000 0021167 9 | IGLC 3 | N/A | immunoglobulin lambda constant 3 (Kern-Oz+ marker) [Source: HGNC Sym bol;Acc:5857] | Chr 22 | Forward Strand | 427 |
| OC3SNG. 1849-16a_x_at | Expression probeset | Sense (Fully Exonic) | 8 | ENSG000 0021167 9 | IGLC 3 | N/A | immunoglobulin lambda constant 3 (Kern-Oz+ marker) [Source: HGNC Sym bol;Acc:5857] | Chr 22 | Forward Strand | 428 |
| OC3SNG. 1049-17a_x_at | Expression probeset | Sense (Fully Exonic) | 11 | ENSG000 0021167 9 | IGLC 3 | N/A | immunoglobulin lambda constant 3 (Kern-Oz+ marker) [Source: HGNC Sym bol;Acc:5857] | Chr 22 | Forward Strand | 429 |
| OCMX.26 30.C25_s _at | Expression probeset | Sense (Fully Exonic) | 11 | ENSG000 0025336 4 | N/A | N/A | KNOWN lincRNA (Clone_based_ vega_gene) | Chr 14 | Reverse Strand | 430 |
| OC3SNGn . 7538-8a_s_at | Expression probeset | Sense (Fully Exonic) | 11 | ENSG000 0021189 6 | IGHG 1 | 1019 3037 4 /// 1024 6587 1 /// 1024 6688 9 | immunoglobulin heavy constant gamma 1 (G1m marker) [Source: HGNC Symbol;Acc:5525] | Chr 14 | Reverse Strand | 431 |
| OCADNP. 6338_x_a t | Expression probeset | AntiSense | 11 | ENSG000 0020783 5 | N/A | N/A | NOVEL miRNA (Clone_based_ ensembl_gene) | Chr 22 | Forward Strand | 432 |
| OCMX.26 30.C11_s _at | Expression probeset | Sense (Fully Exonic) | 11 | ENSG000 0025336 4 | N/A | N/A | KNOWN lincRNA (Clone_based_ vega_gene) | Chr 14 | Reverse Strand | 433 |
| OC3P. 121 .CB2_x_a t | Expression probeset | Sense (Fully Exonic) | 6 | ENSG000 0021159 2 | IGKC | N/A | immunoglobulin kappa constant [Source:HGNC Symbol;Acc: 5716] | Chr 2 | Reverse Strand | 434 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3SNGn . 6560-270a_x_a t | Expression probeset | Sense (Fully Exonic) | 11 | ENSG000 0021159 2 | IGKC | N/A | immunoglobulin kappa constant [Source:HGNC Symbol;Acc: 5716] | Chr 2 | Reverse Strand | 435 |
| OC3P.103 47.C1_s_ at | Expression probeset | Sense (Fully Exonic) | 11 | ENSG000 0025559 4 | N/A | N/A | KNOWN pseudogene (Clone_ based_ensembl_gene) | Chr 22 | Reverse Strand | 436 |
| OC3P.132 21.C1_s_ at | Expression probeset | Sense (Fully Exonic) | 11 | ENSG000 0019801 9 | FCGR 1B | 2209 /// 2210 /// 1001 3241 7 | Fc fragment of IgG, high affinity Ib, receptor (CD64) [Source:HGNC Symbol;Acc:3614] | Chr 1 | Reverse Strand | 437 |
| OCMXSN G. 5045_s _at | Expression probeset | Sense (Fully Exonic) | 11 | ENSG000 0023925 7 | RPL2 3AP1 | N/A | ribosomal protein L23a pseudogene 1 [Source:HGNC Symbol;Acc:10318] | Chr 6 | Reverse Strand | 438 |
| OCHP.12 16_s_ at | Expression probeset | Sense (Fully Exonic) | 11 | ENSG000 0010779 6 | ACTA 2 | 59 | actin, alpha 2, smooth muscle, aorta [Source:HGNC Symbol;Acc: 130] | Chr 10 | Reverse Strand | 439 |
| OC3P.126 92.C1_s_ at | Expression probeset | Sense (Fully Exonic) | 10 | ENSG000 0019789 4 | ADH 5 | 128 | alcohol dehydrogenase 5 (class III), chi polypeptide [Source: HGNC Symbol;Acc:253] | Chr 4 | Reverse Strand | 440 |
| OC3P.345 8.C1_s_at | Expression probeset | Sense (Fully Exonic) | 11 | ENSG000 0010662 4 | AEBP 1 | 165 | AE binding protein 1 [Source: HGNC Symbol;Acc:303] | Chr 7 | Forward Strand | 441 |
| OC3SNGn h. 5051_a t | Expression probeset | Sense (includes Intronic) | 10 | ENSG000 0016369 7 | APBB 2 | 323 | amyloid beta (A4) precursor protein-binding, family B, member 2 [Source:HGNC Symbol;Acc: 582] | Chr 4 | Reverse Strand | 442 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3SNGn h. 5051_x _at | Expression probeset | Sense (includes Intronic) | 9 | ENSG000 0016369 7 | APBB 2 | 323 | amyloid beta (A4) precursor protein-binding, family B, member 2 [Source:HGNC Symbol;Acc: 582] | Chr 4 | Reverse Strand | 443 |
| OCHP.10 16_s_ at | Expression probeset | Sense (Fully Exonic) | 11 | ENSG000 0018905 8 | APO D | 347 | apolipoprotein D [Source:HGNC Symbol;Acc:612] | Chr 3 | Reverse Strand | 444 |
| OC3P. 373 .C1-533a_ s_a t | Expression probeset | Sense (Fully Exonic) | 11 | ENSG000 0014387 8 | RHO B | 388 | ras homolog family member B [Source:HGNC Symbol;Acc:668] | Chr 2 | Forward Strand | 445 |
| OCMX.43 59.C1_x_ at | Expression probeset | AntiSense | 8 | ENSG000 0016277 2 | ATF3 | 467 | activating transcription factor 3 [Source:HGNC Symbol;Acc:785] | Chr 1 | Forward Strand | 446 |
| OC3P.232 1.C1_s_at | Expression probeset | Sense (Fully Exonic) | 11 | ENSG000 0016277 2 | ATF3 | 467 | activating transcription factor 3 [Source:HGNC Symbol;Acc:785] | Chr 1 | Forward Strand | 447 |
| OCMX.43 59.C1_at | Expression probeset | AntiSense | 11 | ENSG000 0016277 2 | ATF3 | 467 | activating transcription factor 3 [Source:HGNC Symbol;Acc:785] | Chr 1 | Forward Strand | 448 |
| OCADNP. 7610_s_a t | Expression probeset | Sense (Fully Exonic) | 11 | ENSG000 0016487 9 | CA3 | 761 | carbonic anhydrase III, muscle specific [Source:HGNC Symbol; Acc:1374] | Chr 8 | Forward Strand | 449 |
| OC3SNGn h. 4098_a t | Expression probeset | Sense (includes Intronic) | 11 | ENSG000 0016487 9 | CA3 | 761 | carbonic anhydrase III, muscle specific [Source:HGNC Symbol; Acc:1374] | Chr 8 | Forward Strand | 450 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3P.110 93.C1_s_ at | Expression probeset | Sense (Fully Exonic) | 9 | ENSG000 0007441 0 | CA12 | 771 | carbonic anhydrase XII [Source: HGNC Symbol;Acc:1371] | Chr 15 | Reverse Strand | 451 |
| OCMX.51 7.C1_at | Expression probeset | AntiSense | 11 | ENSG000 0012278 6 | CALD 1 | 800 | caldesmon 1 [Source:HGNC Symbol;Acc:1441] | Chr 7 | Forward Strand | 452 |
| OCMX.51 7.C1_x_at | Expression probeset | AntiSense | 11 | ENSG000 0012278 6 | CALD 1 | 800 | caldesmon 1 [Source:HGNC Symbol;Acc:1441] | Chr 7 | Forward Strand | 453 |
| OCADNP. 2089_s_a t | Expression probeset | Sense (Fully Exonic) | 11 | ENSG000 0012278 6 | CALD 1 | 800 | caldesmon 1 [Source:HGNC Symbol;Acc:1441] | Chr 7 | Forward Strand | 454 |
| OCHP.14 8_s_at | Expression probeset | Sense (Fully Exonic) | 11 | ENSG000 0014093 7 | CDH 11 | 1009 | cadherin 11, type 2, OB-cadherin (osteoblast) [Source:HGNC Symbol;Acc:1750] | Chr 16 | Reverse Strand | 455 |
| OC3SNGn h. 3154_s _at | Expression probeset | Sense (Fully Exonic) | 11 | ENSG000 0012865 6 | CHN 1 | 1123 | chimerin 1 [Source:HGNC Symbol;Acc:1943] | Chr 2 | Reverse Strand | 456 |
| OC3P.713 8.C3_x_at | Expression probeset | AntiSense | 11 | ENSG000 0007420 1 | CLNS 1A | 1207 | chloride channel, nucleotide-sensitive, 1A [Source:HGNC Symbol;Acc:2080] | Chr 11 | Reverse Strand | 457 |
| OC3P.713 8.C3_at | Expression probeset | AntiSense | 11 | ENSG000 0007420 1 | CLNS 1A | 1207 | chloride channel, nucleotide-sensitive, 1A [Source:HGNC Symbol;Acc:2080] | Chr 11 | Reverse Strand | 458 |
| OC3P. 354 .CB1_s_at | Expression probeset | Sense (Fully Exonic) | 11 | ENSG000 0010882 1 | COL1 A1 | 1277 | collagen, type I, alpha 1 [Source: HGNC Symbol;Acc:2197] | Chr 17 | Reverse Strand | 459 |

(continued)

| Probe Set ID | Type | Orient ation | No. prob es align ed | Ensembl Gene | Gene Sym bol | Entr ez Gene ID | Description | Chro mos ome | Strand | SEQ ID NO |
|---|---|---|---|---|---|---|---|---|---|---|
| OCMXSN G. 5132_s _at | Expression probeset | AntiSe nse | 11 | ENSG000 0010882 1 | COL1 A1 | 1277 | collagen, type I, alpha 1 [Source: HGNC Symbol;Acc:2197] | Chr 17 | Revers e Strand | 460 |
| OCMXSN G. 5132_x _at | Expression probeset | AntiSe nse | 11 | ENSG000 0010882 1 | COL1 A1 | 1277 | collagen, type I, alpha 1 [Source: HGNC Symbol;Acc:2197] | Chr 17 | Revers e Strand | 461 |
| OC3SNGn . 2538-539a_x_a t | Expression probeset | Sense (Fully Exonic) | 11 | ENSG000 0016469 2 | COL1 A2 | 1278 | collagen, type I, alpha 2 [Source: HGNC Symbol;Acc:2198] | Chr 7 | Forwar d Strand | 462 |
| OC3SNGn . 8474-50a_x_at | Expression probeset | Sense (Fully Exonic) | 9 | ENSG000 0016469 2 | COL1 A2 | 1278 | collagen, type I, alpha 2 [Source: HGNC Symbol;Acc:2198] | Chr 7 | Forwar d Strand | 463 |
| OC3SNGn . 1211-6a_s_at | Expression probeset | Sense (Fully Exonic) | 11 | ENSG000 0016854 2 | COL3 A1 | 1281 | collagen, type III, alpha 1 [Source: HGNC Symbol;Acc:2201] | Chr 2 | Forwar d Strand | 464 |
| OC3P.81. CB2_s_at | Expression probeset | Sense (Fully Exonic) | 11 | ENSG000 0016854 2 | COL3 A1 | 1281 | collagen, type III, alpha 1 [Source: HGNC Symbol;Acc:2201] | Chr 2 | Forwar d Strand | 465 |
| OC3P. 850 .C1-1145a_ s_ at | Expression probeset | Sense (Fully Exonic) | 11 | ENSG000 0018749 8 | COL4 A1 | 1282 | collagen, type IV, alpha 1 [Source: HGNC Symbol;Acc:2202] | Chr 13 | Revers e Strand | 466 |
| OC3P.498 4.C1-787a_s_a t | Expression probeset | Sense (Fully Exonic) | 11 | ENSG000 0013063 5 | COL5 A1 | 1289 | collagen, type V, alpha 1 [Source: HGNC Symbol;Acc:2209] | Chr 9 | Forwar d Strand | 467 |
| OCHP.10 05_s_ at | Expression probeset | Sense (Fully Exonic) | 11 | ENSG000 0013063 5 | COL5 A1 | 1289 | collagen, type V, alpha 1 [Source: HGNC Symbol;Acc:2209] | Chr 9 | Forwar d Strand | 468 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3P.2713.C1_s_at | Expression probeset | Sense (Fully Exonic) | 11 | ENSG00000204262 | COL5A2 | 1290 | collagen, type V, alpha 2 [Source: HGNC Symbol;Acc:2210] | Chr 2 | Reverse Strand | 469 |
| OC3P.13652.C1_s_ at | Expression probeset | Sense (Fully Exonic) | 11 | ENSG00000144810 | COL8A1 | 1295 | collagen, type VIII, alpha 1 [Source:HGNC Symbol;Acc: 2215] | Chr 3 | Forward Strand | 470 |
| OC3P.10562.C1_s_ at | Expression probeset | Sense (Fully Exonic) | 11 | ENSG00000144810 | COL8A1 | 1295 | collagen, type VIII, alpha 1 [Source:HGNC Symbol;Acc: 2215] | Chr 3 | Forward Strand | 471 |
| OC3SNG. 1834-947a_s_a t | Expression probeset | Sense (Fully Exonic) | 11 | ENSG00000123500 | COL10A1 | 1300 | collagen, type X, alpha 1 [Source: HGNC Symbol;Acc:2185] | Chr 6 | Reverse Strand | 472 |
| OCRS.383 _s_at | Expression probeset | Sense (Fully Exonic) | 11 | ENSG00000123500 | COL10A1 | 1300 | collagen, type X, alpha 1 [Source: HGNC Symbol;Acc:2185] | Chr 6 | Reverse Strand | 473 |
| OC3P.14073.C1_s_ at | Expression probeset | Sense (Fully Exonic) | 11 | ENSG00000111799 | COL12A1 | 1303 | collagen, type XII, alpha 1 [Source: HGNC Symbol;Acc:2188] | Chr 6 | Reverse Strand | 474 |
| OC3SNGn h. 11427_ x_at | Expression probeset | Sense (includes Intronic) | 8 | ENSG00000111799 | COL12A1 | 1303 | collagen, type XII, alpha 1 [Source: HGNC Symbol;Acc:2188] | Chr 6 | Reverse Strand | 475 |
| OC3SNGn h. 11427_ at | Expression probeset | Sense (includes Intronic) | 6 | ENSG00000111799 | COL12A1 | 1303 | collagen, type XII, alpha 1 [Source: HGNC Symbol;Acc:2188] | Chr 6 | Reverse Strand | 476 |

(continued)

| Probe Set ID | Type | Orient ation | No. prob es align ed | Ensembl Gene | Gene Sym bol | Entr ez Gene ID | Description | Chro mos ome | Strand | SEQ ID NO |
|---|---|---|---|---|---|---|---|---|---|---|
| OC3P.101 57.C1_s_ at | Expression probeset | Sense (Fully Exonic) | 11 | ENSG000 0020429 1 | COL1 5A1 | 1306 | collagen, type XV, alpha 1 [Source:HGNC Symbol;Acc: 2192] | Chr 9 | Forwar d Strand | 477 |
| OCMX.43 93.C1_s_ at | Expression probeset | AntiSe nse | 11 | ENSG000 0006708 2 | KLF6 | 1316 | Kruppel-like factor 6 [Source: HGNC Symbol;Acc:2235] | Chr 10 | Revers e Strand | 478 |
| OCMX.15 173.C1_s _at | Expression probeset | Sense (Fully Exonic) | 11 | ENSG000 0003842 7 | VCA N | 1462 | versican [Source:HGNC Symbol; Acc:2464] | Chr 5 | Forwar d Strand | 479 |
| OC3P.120 0.C1_s_at | Expression probeset | Sense (Fully Exonic) | 11 | ENSG000 0003842 7 | VCA N | 1462 | versican [Source:HGNC Symbol; Acc:2464] | Chr 5 | Forwar d Strand | 480 |
| OCADNP. 9526_s_a t | Expression probeset | Sense (Fully Exonic) | 11 | ENSG000 0011852 3 | CTGF | 1490 | connective tissue growth factor [Source:HGNC Symbol;Acc: 2500] | Chr 6 | Revers e Strand | 481 |
| OC3P.117 8.C1_x_at | Expression probeset | Sense (Fully Exonic) | 9 | ENSG000 0011852 3 | CTGF | 1490 | connective tissue growth factor [Source:HGNC Symbol;Acc: 2500] | Chr 6 | Revers e Strand | 482 |
| OC3P.117 8.C1_at | Expression probeset | Sense (Fully Exonic) | 10 | ENSG000 0011852 3 | CTGF | 1490 | connective tissue growth factor [Source:HGNC Symbol;Acc: 2500] | Chr 6 | Revers e Strand | 483 |
| OC3P.282 2.C1_s_at | Expression probeset | Sense (Fully Exonic) | 11 | ENSG000 0013592 9 | CYP2 7A1 | 1593 | cytochrome P450, family 27, subfamily A, polypeptide 1 [Source:HGNC Symbol;Acc: 2605] | Chr 2 | Forwar d Strand | 484 |
| OC3P. 694 .CB1-490a_ s_a t | Expression probeset | Sense (Fully Exonic) | 8 | ENSG000 0001146 5 | DCN | 1634 | decorin [Source:HGNC Symbol; Acc:2705] | Chr 12 | Revers e Strand | 485 |

(continued)

| Probe Set ID | Type | Orient ation | No. prob es align ed | Ensembl Gene | Gene Sym bol | Entr ez Gene ID | Description | Chro mos ome | Strand | SEQ ID NO |
|---|---|---|---|---|---|---|---|---|---|---|
| OC3SNG. 461-892a_s_a t | Expression probeset | Sense (Fully Exonic) | 11 | ENSG000 0001146 5 | DCN | 1634 | decorin [Source:HGNC Symbol; Acc:2705] | Chr 12 | Revers e Strand | 486 |
| OC3SNGn h. 5811_a t | Expression probeset | Sense (includ es Introni c) | 11 | ENSG000 0019894 7 | DMD | 1756 | dystrophin [Source:HGNC Symbol;Acc:2928] | Chr X | Revers e Strand | 487 |
| OCADNP. 13759_s_ at | Expression probeset | Sense (includ es Introni c) | 11 | ENSG000 0011365 7 | DPYS L3 | 1809 | dihydropyrimidinase-like 3 [Source:HGNC Sym bol;Acc: 3015] | Chr 5 | Revers e Strand | 488 |
| OC3SNGn h. 8739_s _at | Expression probeset | Sense (Fully Exonic) | 11 | ENSG000 0012087 5 | DUS P4 | 1846 | dual specificity phosphatase 4 [Source:HGNC Symbol;Acc: 3070] | Chr 8 | Revers e Strand | 489 |
| OCADA.7 893_s_at | Expression probeset | Sense (Fully Exonic) | 11 | ENSG000 0018434 9 | EFNA 5 | 1946 | ephrin-A5 [Source:HGNC Symbol;Acc:3225] | Chr 5 | Revers e Strand | 490 |
| OC3SNGn h. 9087_a t | Expression probeset | AntiSe nse | 11 | ENSG000 0018434 9 | EFNA 5 | 1946 | ephrin-A5 [Source:HGNC Symbol;Acc:3225] | Chr 5 | Revers e Strand | 491 |
| OC3P.191 0.C1_s_at | Expression probeset | Sense (Fully Exonic) | 11 | ENSG000 0012073 8 | EGR1 | 1958 | early growth response 1 [Source: HGNC Symbol;Acc:3238] | Chr 5 | Forwar d Strand | 492 |
| OCADNP. 2432_s_a t | Expression probeset | AntiSe nse | 11 | ENSG000 0012073 8 | EGR1 | 1958 | early growth response 1 [Source: HGNC Symbol;Acc:3238] | Chr 5 | Forwar d Strand | 493 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3SNGn h. 19479_ s_at | Expression probeset | AntiSense | 11 | ENSG000 0012073 8 | EGR1 | 1958 | early growth response 1 [Source: HGNC Symbol;Acc:3238] | Chr 5 | Forward Strand | 494 |
| OCMX.8. C2_s_ at | Expression probeset | AntiSense | 11 | ENSG000 0012073 8 | EGR1 | 1958 | early growth response 1 [Source: HGNC Symbol;Acc:3238] | Chr 5 | Forward Strand | 495 |
| OC3SNGn . 469-921a_s_a t | Expression probeset | Sense (Fully Exonic) | 11 | ENSG000 0012073 8 | EGR1 | 1958 | early growth response 1 [Source: HGNC Symbol;Acc:3238] | Chr 5 | Forward Strand | 496 |
| OC3P.89. C6_s_ at | Expression probeset | Sense (Fully Exonic) | 11 | ENSG000 0004954 0 | ELN | 2006 | elastin [Source:HGNC Symbol; Acc:3327] | Chr 7 | Forward Strand | 497 |
| OC3P.129 2.C1_s_at | Expression probeset | Sense (Fully Exonic) | 11 | ENSG000 0013453 1 | EMP 1 | 2012 | epithelial membrane protein 1 [Source:HGNC Symbol;Acc: 3333] | Chr 12 | Forward Strand | 498 |
| OC3SNG. 2163-2941a_s_ at | Expression probeset | Sense (Fully Exonic) | 11 | ENSG000 0000646 8 | ETV1 | 2115 | ets variant 1 [Source:HGNC Symbol;Acc:3490] | Chr 7 | Reverse Strand | 499 |
| OC3SNGn h. 1613_a t | Expression probeset | Sense (includes Intronic) | 9 | ENSG000 0006836 6 | ACSL 4 | 2182 | acyl-CoA synthetase long-chain family member 4 [Source:HGNC Symbol;Acc:3571] | Chr X | Reverse Strand | 500 |
| OC3P.349 9.C1_s_at | Expression probeset | Sense (Fully Exonic) | 11 | ENSG000 0008385 7 | FAT1 | 2195 | FAT atypical cadherin 1 [Source: HGNC Symbol;Acc:3595] | Chr 4 | Reverse Strand | 501 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCHP.10 78_s_ at | Expression probeset | Sense (Fully Exonic) | 11 | ENSG000 0016352 0 | FBLN 2 | 2199 | fibulin 2 [Source:HGNC Symbol; Acc:3601] | Chr 3 | Forward Strand | 502 |
| OC3SNG. 6042-23a_x_at | Expression probeset | Sense (Fully Exonic) | 11 | ENSG000 0007778 2 | FGFR 1 | 2260 | fibroblast growth factor receptor 1 [Source:HGNC Symbol;Acc: 3688] | Chr 8 | Reverse Strand | 503 |
| OCADNP. 8535_s_a t | Expression probeset | Sense (Fully Exonic) | 11 | ENSG000 0007778 2 | FGFR 1 | 2260 | fibroblast growth factor receptor 1 [Source:HGNC Symbol;Acc: 3688] | Chr 8 | Reverse Strand | 504 |
| OC3SNGn . 6036-20a_x_at | Expression probeset | Sense (Fully Exonic) | 7 | ENSG000 0007778 2 | FGFR 1 | 2260 | fibroblast growth factor receptor 1 [Source:HGNC Symbol;Acc: 3688] | Chr 8 | Reverse Strand | 505 |
| OCMXSN G. 5052_s _at | Expression probeset | AntiSense | 11 | ENSG000 0011541 4 | FN1 | 2335 | fibronectin 1 [Source:HGNC Symbol;Acc:3778] | Chr 2 | Reverse Strand | 506 |
| OCMX.49 3.C1_s_at | Expression probeset | Sense (Fully Exonic) | 11 | ENSG000 0011541 4 | FN1 | 2335 | fibronectin 1 [Source:HGNC Symbol;Acc:3778] | Chr 2 | Reverse Strand | 507 |
| OCADA.9 921_s_at | Expression probeset | Sense (Fully Exonic) | 11 | ENSG000 0017034 5 | FOS | 2353 | FBJ murine osteosarcoma viral oncogene homolog [Source: HGNC Symbol;Acc:3796] | Chr 14 | Forward Strand | 508 |
| OC3SNGn h. 12139_ at | Expression probeset | Sense (Fully Exonic) | 7 | ENSG000 0001081 0 | FYN | 2534 | FYN oncogene related to SRC, FGR, YES [Source:HGNC Symbol;Acc:4037] | Chr 6 | Reverse Strand | 509 |
| OCMX.11 023.C1_s _at | Expression probeset | AntiSense | 11 | ENSG000 0018044 7 | GAS1 | 2619 | growth arrest-specific 1 [Source: HGNC Symbol;Acc:4165] | Chr 9 | Reverse Strand | 510 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCHP.18 36 s at | Expression probeset | Sense (Fully Exonic) | 11 | ENSG000 0015266 1 | GJA1 | 2697 | gap junction protein, alpha 1, 43kDa [Source:HGNC Symbol; Acc:4274] | Chr 6 | Forward Strand | 511 |
| OCADA.7 782_s_at | Expression probeset | Sense (includes Intronic) | 11 | ENSG000 0014818 0 | GSN | 2934 | gelsolin [Source:HGNC Symbol; Acc:4620] | Chr 9 | Forward Strand | 512 |
| OC3SNGn . 484-1a_s_at | Expression probeset | Sense (Fully Exonic) | 11 | ENSG000 0019775 7 | HOX C6 | 3223 | homeobox C6 [Source:HGNC Symbol;Acc:5128] | Chr 12 | Forward Strand | 513 |
| OC3P.101 27.C1_s_ at | Expression probeset | Sense (Fully Exonic) | 11 | ENSG000 0019775 7 | HOX C6 | 3223 | homeobox C6 [Source:HGNC Symbol;Acc:5128] | Chr 12 | Forward Strand | 514 |
| OC3P.187 8.C1_s_at | Expression probeset | Sense (Fully Exonic) | 11 | ENSG000 0004198 2 | TNC | 3371 | tenascin C [Source:HGNC Symbol;Acc:5318] | Chr 9 | Reverse Strand | 515 |
| OC3P.129 39.C1_s_ at | Expression probeset | Sense (Fully Exonic) | 11 | ENSG000 0001742 7 | IGF1 | 3479 | insulin-like growth factor 1 (somatomedin C) [Source:HGNC Symbol;Acc:5464] | Chr 12 | Reverse Strand | 516 |
| OCMX.11 138.C1_x _at | Expression probeset | AntiSense | 8 | ENSG000 0001742 7 | IGF1 | 3479 | insulin-like growth factor 1 (somatomedin C) [Source:HGNC Symbol;Acc:5464] | Chr 12 | Reverse Strand | 517 |
| OCMX.11 138.C1_a t | Expression probeset | AntiSense | 8 | ENSG000 0001742 7 | IGF1 | 3479 | insulin-like growth factor 1 (somatomedin C) [Source:HGNC Symbol;Acc:5464] | Chr 12 | Reverse Strand | 518 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3P. 460 .C1_s_at | Expression probeset | AntiSense | 11 | ENSG000 0016724 4 | IGF2 | 3481 | insulin-like growth factor 2 (somatomedin A) [Source:HGNC Symbol;Acc:5466] | Chr 11 | Reverse Strand | 519 |
| OC3SNG. 5134-22a_s_at | Expression probeset | Sense (Fully Exonic) | 11 | ENSG000 0014175 3 | IGFBP4 | 3487 | insulin-like growth factor binding protein 4 [Source:HGNC Symbol; Acc:5473] | Chr 17 | Forward Strand | 520 |
| OC3P.198 7.C1_x_at | Expression probeset | Sense (Fully Exonic) | 6 | ENSG000 0011546 1 | IGFBP5 | 3488 | insulin-like growth factor binding protein 5 [Source:HGNC Symbol; Acc:5474] | Chr 2 | Reverse Strand | 521 |
| OC3P.198 7.CB1_x_ at | Expression probeset | Sense (Fully Exonic) | 9 | ENSG000 0011546 1 | IGFBP5 | 3488 | insulin-like growth factor binding protein 5 [Source:HGNC Symbol; Acc:5474] | Chr 2 | Reverse Strand | 522 |
| OC3SNG. 2502-79a_s_at | Expression probeset | Sense (Fully Exonic) | 9 | ENSG000 0011546 1 | IGFBP5 | 3488 | insulin-like growth factor binding protein 5 [Source:HGNC Symbol; Acc:5474] | Chr 2 | Reverse Strand | 523 |
| OCADNP. 830_s_at | Expression probeset | Sense (Fully Exonic) | 11 | ENSG000 0011546 1 | IGFBP5 | 3488 | insulin-like growth factor binding protein 5 [Source:HGNC Symbol; Acc:5474] | Chr 2 | Reverse Strand | 524 |
| OC3SNGn h. 6980_s _at | Expression probeset | AntiSense | 11 | ENSG000 0011546 1 | IGFBP5 | 3488 | insulin-like growth factor binding protein 5 [Source:HGNC Symbol; Acc:5474] | Chr 2 | Reverse Strand | 525 |
| OCHP.13 01_s_ at | Expression probeset | Sense (Fully Exonic) | 11 | ENSG000 0016777 9 | IGFBP6 | 3489 | insulin-like growth factor binding protein 6 [Source:HGNC Symbol; Acc:5475] | Chr 12 | Forward Strand | 526 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCADNP. 3131_x_a t | Expression probeset | Sense (includes Intronic) | 6 | ENSG000 0016345 3 | IGFB P7 | 3490 | insulin-like growth factor binding protein 7 [Source:HGNC Symbol; Acc:5476] | Chr 4 | Reverse Strand | 527 |
| OC3P.184 3.C1_x_at | Expression probeset | Sense (Fully Exonic) | 10 | ENSG000 0011559 4 | IL1R 1 | 3554 | interleukin 1 receptor, type I [Source:HGNC Sym bol;Acc: 5993] | Chr 2 | Forward Strand | 528 |
| OC3P.589 3.C1_s_at | Expression probeset | Sense (Fully Exonic) | 11 | ENSG000 0016904 7 | IRS1 | 3667 | insulin receptor substrate 1 [Source:HGNC Symbol;Acc: 6125] | Chr 2 | Reverse Strand | 529 |
| OCHP.18 81_s_ at | Expression probeset | Sense (Fully Exonic) | 11 | ENSG000 0015740 4 | KIT | 3815 | v-kit Hardy-Zuckerman 4 feline sarcoma viral oncogene homolog [Source:HGNC Symbol;Acc: 6342] | Chr 4 | Forward Strand | 530 |
| OC3SNGn h. 985_s_ at | Expression probeset | Sense (Fully Exonic) | 11 | ENSG000 0009920 4 | ABLI M1 | 3983 | actin binding LIM protein 1 [Source:HGNC Symbol;Acc:78] | Chr 10 | Reverse Strand | 531 |
| OCHP.13 06 s at | Expression probeset | Sense (Fully Exonic) | 11 | ENSG000 0012903 8 | LOXL 1 | 4016 | lysyl oxidase-like 1 [Source: HGNC Symbol;Acc:6665] | Chr 15 | Forward Strand | 532 |
| OCMXSN G. 3759_x _at | Expression probeset | AntiSense | 7 | ENSG000 0012903 8 | LOXL 1 | 4016 | lysyl oxidase-like 1 [Source: HGNC Symbol;Acc:6665] | Chr 15 | Forward Strand | 533 |
| OC3P.570 0.C1_s_at | Expression probeset | Sense (Fully Exonic) | 11 | ENSG000 0011968 1 | LTBP 2 | 4053 | latent transforming growth factor beta binding protein 2 [Source: HGNC Symbol;Acc:6715] | Chr 14 | Reverse Strand | 534 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OCHP.15 34_s_at | Expression probeset | Sense (Fully Exonic) | 11 | ENSG000 0013932 9 | LUM | 4060 | lumican [Source:HGNC Symbol; Acc:6724] | Chr 12 | Reverse Strand | 535 |
| OC3P.924 8.C1_s_at | Expression probeset | Sense (Fully Exonic) | 11 | ENSG000 0015513 0 | MAR CKS | 4082 | myristoylated alanine-rich protein kinase C substrate [Source:HGNC Symbol;Acc:6759] | Chr 6 | Forward Strand | 536 |
| OC3P.104 85.C1_s_ at | Expression probeset | Sense (Fully Exonic) | 11 | ENSG000 0015513 0 | MAR CKS | 4082 | myristoylated alanine-rich protein kinase C substrate [Source:HGNC Symbol;Acc:6759] | Chr 6 | Forward Strand | 537 |
| OCADA.6 829_s_at | Expression probeset | Sense (includes Intronic) | 11 | ENSG000 0009501 5 | MAP 3K1 | 4214 | mitogen-activated protein kinase kinase kinase 1, E3 ubiquitin protein ligase [Source:HGNC Symbol;Acc:6848] | Chr 5 | Forward Strand | 538 |
| OC3SNGn . 8705-760a_x_a t | Expression probeset | Sense (Fully Exonic) | 9 | ENSG000 0011134 1 | MGP | 4256 | matrix Gla protein [Source:HGNC Symbol;Acc:7060] | Chr 12 | Reverse Strand | 539 |
| OCADNP. 7251_s_a t | Expression probeset | Sense (Fully Exonic) | 11 | ENSG000 0008724 5 | MM P2 | 4313 | matrix metallopeptidase 2 (gelatinase A, 72kDa gelatinase, 72kDa type IV collagenase) [Source:HGNC Symbol;Acc: 7166] | Chr 16 | Forward Strand | 540 |
| OC3SNGn . 2375-26a_s_at | Expression probeset | Sense (Fully Exonic) | 11 | ENSG000 0009995 3 | MM P11 | 4320 | matrix metallopeptidase 11 (stromelysin 3) [Source:HGNC Symbol;Acc:7157] | Chr 22 | Forward Strand | 541 |
| OC3P.376 4.C1_s_at | Expression probeset | Sense (Fully Exonic) | 11 | ENSG000 0009995 3 | MM P11 | 4320 | matrix metallopeptidase 11 (stromelysin 3) [Source:HGNC Symbol;Acc:7157] | Chr 22 | Forward Strand | 542 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3P.412 3.C1_x_at | Expression probeset | Sense (Fully Exonic) | 9 | ENSG000 0015722 7 | MM P14 | 4323 | matrix metallopeptidase 14 (membrane-inserted) [Source: HGNC Symbol;Acc:7160] | Chr 14 | Forward Strand | 543 |
| OCADA.6 468_s_at | Expression probeset | Sense (includes Intronic) | 11 | ENSG000 0014706 5 | MSN | 4478 | moesin [Source:HGNC Symbol; Acc:7373] | Chr X | Forward Strand | 544 |
| OCHPRC. 15_s_ at | Expression probeset | Sense (Fully Exonic) | 11 | ENSG000 0016313 2 | MSX 1 | 4487 | msh homeobox 1 [Source:HGNC Symbol;Acc:7391] | Chr 4 | Forward Strand | 545 |
| OCADNP. 1685_s_a t | Expression probeset | Sense (Fully Exonic) | 6 | ENSG000 0018495 6 | MUC 6 | 4588 | mucin 6, oligomeric mucus/gel-forming [Source:HGNC Symbol; Acc:7517] | Chr 11 | Reverse Strand | 546 |
| OC3P.400 1.C1_s_at | Expression probeset | Sense (Fully Exonic) | 11 | ENSG000 0009986 0 | GAD D45B | 4616 | growth arrest and DNA-damage-inducible, beta [Source:HGNC Symbol;Acc:4096] | Chr 19 | Forward Strand | 547 |
| OC3P.303 4.C1_s_at | Expression probeset | Sense (Fully Exonic) | 11 | ENSG000 0018263 6 | NDN | 4692 | necdin, melanoma antigen (MAGE) family member [Source: HGNC Symbol;Acc:7675] | Chr 15 | Reverse Strand | 548 |
| OC3P.128 52.C1_s_ at | Expression probeset | Sense (Fully Exonic) | 11 | ENSG000 0013119 6 | NFAT C1 | 4772 | nuclear factor of activated T-cells, cytoplasmic, calcineurin-dependent 1 [Source:HGNC Symbol;Acc:7775] | Chr 18 | Forward Strand | 549 |
| OC3P.684 2.C1_s_at | Expression probeset | Sense (Fully Exonic) | 11 | ENSG000 0017048 5 | NPA S2 | 4862 | neuronal PAS domain protein 2 [Source:HGNC Symbol;Acc: 7895] | Chr 2 | Forward Strand | 550 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3SNG. 1306-60a_s_at | Expression probeset | Sense (Fully Exonic) | 11 | ENSG000 0016273 3 | DDR 2 | 4921 | discoidin domain receptor tyrosine kinase 2 [Source:HGNC Symbol; Acc:2731] | Chr 1 | Forward Strand | 551 |
| OCHP.76 9_s_at | Expression probeset | Sense (Fully Exonic) | 11 | ENSG000 0013485 3 | PDG FRA | 5156 | platelet-derived growth factor receptor, alpha polypeptide [Source:HGNC Symbol;Acc: 8803] | Chr 4 | Forward Strand | 552 |
| OC3P.484 9.C1_s_at | Expression probeset | Sense (Fully Exonic) | 11 | ENSG000 0017052 5 | PFKF B3 | 5209 | 6-phosphofructo-2-kinase/ fructose-2,6-biphosphatase 3 [Source:HGNC Symbol;Acc: 8874] | Chr 10 | Forward Strand | 553 |
| OCHP.73 9_s_at | Expression probeset | Sense (Fully Exonic) | 11 | ENSG000 0012286 1 | PLAU | 5328 | plasminogen activator, urokinase [Source:HGNC Symbol;Acc: 9052] | Chr 10 | Forward Strand | 554 |
| OC3P.128 92.C1_s_ at | Expression probeset | Sense (Fully Exonic) | 11 | ENSG000 0011331 9 | RAS GRF2 | 5924 | Ras protein-specific guanine nucleotide-releasing factor 2 [Source:HGNC Symbol;Acc: 9876] | Chr 5 | Forward Strand | 555 |
| OCRS.167 4_s_ at | Expression probeset | Sense (Fully Exonic) | 11 | ENSG000 0013311 1 | RFXA P | 5994 | regulatory factor X-associated protein [Source:HGNC Symbol; Acc:9988] | Chr 13 | Forward Strand | 556 |
| OC3P.310 0.C1_s_at | Expression probeset | Sense (Fully Exonic) | 11 | ENSG000 0011674 1 | RGS2 | 5997 | regulator of G-protein signaling 2, 24kDa [Source:HGNC Symbol; Acc:9998] | Chr 1 | Forward Strand | 557 |
| OC3P.130 61.C1_s_ at | Expression probeset | Sense (Fully Exonic) | 11 | ENSG000 0016985 5 | ROB O1 | 6091 | roundabout, axon guidance receptor, homolog 1 (Drosophila) [Source:HGNC Symbol;Acc: 10249] | Chr 3 | Reverse Strand | 558 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3SNGn h. 14507_ x_at | Expression probeset | Sense (includes Intronic) | 7 | ENSG000 0006966 7 | ROR A | 6095 | RAR-related orphan receptor A [Source:HGNC Sym bol;Acc: 10258] | Chr 15 | Reverse Strand | 559 |
| OC3SNGn h. 14507_ at | Expression probeset | Sense (includes Intronic) | 7 | ENSG000 0006966 7 | ROR A | 6095 | RAR-related orphan receptor A [Source:HGNC Sym bol;Acc: 10258] | Chr 15 | Reverse Strand | 560 |
| OC3SNGn h. 5170_x _at | Expression probeset | Sense (includes Intronic) | 11 | ENSG000 0006966 7 | ROR A | 6095 | RAR-related orphan receptor A [Source:HGNC Sym bol;Acc: 10258] | Chr 15 | Reverse Strand | 561 |
| OC3P.837 3.C1_s_at | Expression probeset | Sense (Fully Exonic) | 11 | ENSG000 0016943 9 | SDC2 | 6383 | syndecan 2 [Source:HGNC Symbol;Acc:10659] | Chr 8 | Forward Strand | 562 |
| OC3P.136 21.C1_s_ at | Expression probeset | Sense (Fully Exonic) | 11 | ENSG000 0014542 3 | SFRP 2 | 6423 | secreted frizzled-related protein 2 [Source:HGNC Symbol;Acc: 10777] | Chr 4 | Reverse Strand | 563 |
| OC3P.706 2.C1_s_at | Expression probeset | Sense (Fully Exonic) | 11 | ENSG000 0016306 9 | SGCB | 6443 | sarcoglycan, beta (43kDa dystrophin-associated glycoprotein) [Source:HGNC Symbol;Acc:10806] | Chr 4 | Reverse Strand | 564 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3SNG. 1640-14a_s_at | Expression probeset | Sense (Fully Exonic) | 11 | ENSG000 0010203 8 | SMA RCA1 | 6594 | SWI/SNF related, matrix associated, actin dependent regulator of chromatin, subfamily a, member 1 [Source:HGNC Symbol;Acc:11097] | Chr X | Reverse Strand | 565 |
| OCRS2.11 009_x_at | Expression probeset | Sense (Fully Exonic) | 11 | ENSG000 0014959 1 | TAGL N | 6876 | transgelin [Source:HGNC Symbol;Acc:11553] | Chr 11 | Forward Strand | 566 |
| OC3P.510 1.C1_s_at | Expression probeset | Sense (Fully Exonic) | 8 | ENSG000 0017574 5 | NR2F 1 | 7025 | nuclear receptor subfamily 2, group F, member 1 [Source: HGNC Symbol;Acc:7975] | Chr 5 | Forward Strand | 567 |
| OC3SNGn h. 3734_s _at | Expression probeset | Sense (Fully Exonic) | 11 | ENSG000 0009296 9 | TGFB 2 | 7042 | transforming growth factor, beta 2 [Source:HGNC Symbol;Acc: 11768] | Chr 1 | Forward Strand | 568 |
| OC3P.102 33.C1_s_ at | Expression probeset | Sense (Fully Exonic) | 11 | ENSG000 0011969 9 | TGFB 3 | 7043 | transforming growth factor, beta 3 [Source:HGNC Symbol;Acc: 11769] | Chr 14 | Reverse Strand | 569 |
| OC3P.116 04.C1_s_ at | Expression probeset | Sense (Fully Exonic) | 9 | ENSG000 0013780 1 | THBS 1 | 7057 | thrombospondin 1 [Source:HGNC Symbol;Acc:11785] | Chr 15 | Forward Strand | 570 |
| OC3P.279 0.C1_s_at | Expression probeset | Sense (Fully Exonic) | 11 | ENSG000 0015409 6 | THY1 | 7070 | Thy-1 cell surface antigen [Source:HGNC Symbol;Acc: 11801] | Chr 11 | Reverse Strand | 571 |
| OC3P. 543 .CB1-699a_ s_a t | Expression probeset | Sense (Fully Exonic) | 11 | ENSG000 0003586 2 | TIMP 2 | 7077 | TIMP metallopeptidase inhibitor 2 [Source:HGNC Symbol;Acc: 11821] | Chr 17 | Reverse Strand | 572 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3SNGn h. 19238_ s_at | Expression probeset | Sense (Fully Exonic) | 11 | ENSG000 0003586 2 | TIMP 2 | 7077 | TIMP metallopeptidase inhibitor 2 [Source:HGNC Symbol;Acc: 11821] | Chr 17 | Reverse Strand | 573 |
| OC3P.104 70.C1_s_ at | Expression probeset | Sense (Fully Exonic) | 11 | ENSG000 0010023 4 | TIMP 3 | 7078 | TIMP metallopeptidase inhibitor 3 [Source:HGNC Symbol;Acc: 11822] | Chr 22 | Forward Strand | 574 |
| OCADA.8 344_s_at | Expression probeset | Sense (includes Intronic) | 11 | ENSG000 0014041 6 | TPM 1 | 7168 | tropomyosin 1 (alpha) [Source: HGNC Symbol;Acc:12010] | Chr 15 | Forward Strand | 575 |
| OC3SNGn h. 487_at | Expression probeset | Sense (Fully Exonic) | 7 | ENSG000 0014041 6 | TPM 1 | 7168 | tropomyosin 1 (alpha) [Source: HGNC Symbol;Acc:12010] | Chr 15 | Forward Strand | 576 |
| OC3SNGn h. 5090_a t | Expression probeset | Sense (Fully Exonic) | 10 | ENSG000 0014041 6 | TPM 1 | 7168 | tropomyosin 1 (alpha) [Source: HGNC Symbol;Acc:12010] | Chr 15 | Forward Strand | 577 |
| OCHP.64 3_s_at | Expression probeset | Sense (Fully Exonic) | 11 | ENSG000 0008251 2 | TRAF 5 | 7188 | TNF receptor-associated factor 5 [Source:HGNC Symbol;Acc: 12035] | Chr 1 | Forward Strand | 578 |
| OCADNP. 14769_s_ at | Expression probeset | AntiSense | 11 | ENSG000 0003838 2 | TRIO | 7204 | trio Rho guanine nucleotide exchange factor [Source:HGNC Symbol;Acc:12303] | Chr 5 | Forward Strand | 579 |
| OCRS2.11 542_s_at | Expression probeset | Sense (Fully Exonic) | 11 | ENSG000 0012269 1 | TWIST1 | 7291 | twist family bHLH transcription factor 1 [Source:HGNC Symbol; Acc:12428] | Chr 7 | Reverse Strand | 580 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3SNGn . 2801-166a_s_a t | Expression probeset | Sense (Fully Exonic) | 11 | ENSG000 0012269 1 | TWIS T1 | 7291 | twist family bHLH transcription factor 1 [Source:HGNC Symbol; Acc:12428] | Chr 7 | Revers e Strand | 581 |
| OCMXSN G. 2027_x _at | Expression probeset | AntiSe nse | 10 | ENSG000 0012269 1 | TWIS T1 | 7291 | twist family bHLH transcription factor 1 [Source:HGNC Symbol; Acc:12428] | Chr 7 | Revers e Strand | 582 |
| OCMXSN G. 2027_a t | Expression probeset | AntiSe nse | 9 | ENSG000 0012269 1 | TWIS T1 | 7291 | twist family bHLH transcription factor 1 [Source:HGNC Symbol; Acc:12428] | Chr 7 | Revers e Strand | 583 |
| OC3P.584 9.C1_s_at | Expression probeset | Sense (Fully Exonic) | 11 | ENSG000 0009244 5 | TYRO 3 | 7301 | TYRO3 protein tyrosine kinase [Source:HGNC Symbol;Acc: 12446] | Chr 15 | Forwar d Strand | 584 |
| OC3P.784 5.C1_s_at | Expression probeset | Sense (Fully Exonic) | 11 | ENSG000 0018795 5 | COL1 4A1 | 7373 | collagen, type XIV, alpha 1 [Source:HGNC Symbol;Acc: 2191] | Chr 8 | Forwar d Strand | 585 |
| OC3SNGn . 6594-7a_s_at | Expression probeset | Sense (Fully Exonic) | 11 | ENSG000 0018795 5 | COL1 4A1 | 7373 | collagen, type XIV, alpha 1 [Source:HGNC Symbol;Acc: 2191] | Chr 8 | Forwar d Strand | 586 |
| OCADA.7 569_s_at | Expression probeset | AntiSe nse | 11 | ENSG000 0011425 1 | WNT 5A | 7474 | wingless-type MMTV integration site family, member 5A [Source: HGNC Symbol;Acc:12784] | Chr 3 | Revers e Strand | 587 |
| OC3SNG. 1705-33a_s_at | Expression probeset | Sense (Fully Exonic) | 11 | ENSG000 0015476 4 | WNT 7A | 7476 | wingless-type MMTV integration site family, member 7A [Source: HGNC Symbol;Acc:12786] | Chr 3 | Revers e Strand | 588 |
| OCMXSN G. 4570_s _at | Expression probeset | Sense (Fully Exonic) | 11 | ENSG000 0022980 7 | XIST | 7503 | X inactive specific transcript (non-protein coding) [Source:HGNC Symbol;Acc:12810] | Chr X | Revers e Strand | 589 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3P.414 5.C1_s_at | Expression probeset | Sense (Fully Exonic) | 11 | ENSG000 0022980 7 | XIST | 7503 | X inactive specific transcript (non-protein coding) [Source:HGNC Symbol;Acc:12810] | Chr X | Reverse Strand | 590 |
| OCMXSN G. 427_s_ at | Expression probeset | Sense (Fully Exonic) | 11 | ENSG000 0022980 7 | XIST | 7503 | X inactive specific transcript (non-protein coding) [Source:HGNC Symbol;Acc:12810] | Chr X | Reverse Strand | 591 |
| OCMX.11 66.C2 at | Expression probeset | Sense (Fully Exonic) | 11 | ENSG000 0027064 1 | TSIX | 9383 | TSIX transcript, XIST antisense RNA [Source:HGNC Symbol;Acc: 12377] | Chr X | Forward Strand | 592 |
| OCADNP. 5655_s_a t | Expression probeset | Sense (Fully Exonic) | 11 | ENSG000 0022980 7 | XIST | 7503 | X inactive specific transcript (non-protein coding) [Source:HGNC Symbol;Acc:12810] | Chr X | Reverse Strand | 593 |
| OCMXSN G. 4891_s _at | Expression probeset | Sense (Fully Exonic) | 11 | ENSG000 0022980 7 | XIST | 7503 | X inactive specific transcript (non-protein coding) [Source:HGNC Symbol;Acc:12810] | Chr X | Reverse Strand | 594 |
| OC3SNGn . 1637-35a_s_at | Expression probeset | Sense (Fully Exonic) | 11 | ENSG000 0012801 6 | ZFP3 6 | 7538 | ZFP36 ring finger protein [Source: HGNC Sym bol;Acc:12862] | Chr 19 | Forward Strand | 595 |
| OC3P.404 0.C1_s_at | Expression probeset | Sense (Fully Exonic) | 11 | ENSG000 0010549 7 | ZNF1 75 | 7728 | zinc finger protein 175 [Source: HGNC Symbol;Acc:12964] | Chr 19 | Forward Strand | 596 |
| OC3P. 305 .C1_at | Expression probeset | Sense (Fully Exonic) | 11 | ENSG000 0015938 8 | BTG2 | 7832 | BTG family, member 2 [Source: HGNC Symbol;Acc:1131] | Chr 1 | Forward Strand | 597 |
| OCMX.12 937.C1_s _at | Expression probeset | AntiSense | 11 | ENSG000 0011084 1 | PPFI BP1 | 8496 | PTPRF interacting protein, binding protein 1 (liprin beta 1) [Source:HGNC Symbol;Acc: 9249] | Chr 12 | Forward Strand | 598 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OCMX.20 61.C1_s_ at | Expression probeset | Sense (Fully Exonic) | 11 | ENSG000 0017161 7 | ENC1 | 8507 | ectodermal-neural cortex 1 (with BTB domain) [Source:HGNC Symbol;Acc:3345] | Chr 5 | Reverse Strand | 599 |
| OC3P.808 7.C1_s_at | Expression probeset | Sense (Fully Exonic) | 11 | ENSG000 0000723 7 | GAS7 | 8522 | growth arrest-specific 7 [Source: HGNC Symbol;Acc:4169] | Chr 17 | Reverse Strand | 600 |
| OC3P.136 34.C1_s_ at | Expression probeset | Sense (Fully Exonic) | 11 | ENSG000 0018595 0 | IRS2 | 8660 | insulin receptor substrate 2 [Source:HGNC Sym bol;Acc: 6126] | Chr 13 | Reverse Strand | 601 |
| OCADNP. 2524_s_a t | Expression probeset | AntiSense | 6 | ENSG000 0011825 7 | NRP 2 | 8828 | neuropilin 2 [Source:HGNC Symbol;Acc:8005] | Chr 2 | Forward Strand | 602 |
| OC3P.844 5.C1_s_at | Expression probeset | Sense (Fully Exonic) | 11 | ENSG000 0009925 0 | NRP 1 | 8829 | neuropilin 1 [Source:HGNC Symbol;Acc:8004] | Chr 10 | Reverse Strand | 603 |
| OCADA.8 635_s_at | Expression probeset | Sense (includes Intronic) | 11 | ENSG000 0007268 2 | P4H A2 | 8974 /// 1019 2770 5 | prolyl 4-hydroxylase, alpha polypeptide II [Source:HGNC Symbol;Acc:8547] | Chr 5 | Reverse Strand | 604 |
| OCADNP. 2893_s_a t | Expression probeset | Sense (includes Intronic) | 11 | ENSG000 0012969 1 | ASH2 L | 9070 | ash2 (absent, small, or homeotic)-like (Drosophila) [Source:HGNC Symbol;Acc:744] | Chr 8 | Forward Strand | 605 |
| OC3P.107 90.C1_s_ at | Expression probeset | Sense (Fully Exonic) | 11 | ENSG000 0013098 8 | RGN | 9104 | regucalcin [Source:HGNC Symbol;Acc:9989] | Chr X | Forward Strand | 606 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3P.125 29.C1_s_ at | Expression probeset | Sense (Fully Exonic) | 11 | ENSG000 0017155 1 | ECEL 1 | 9427 | endothelin converting enzyme-like 1 [Source:HGNC Symbol;Acc: 3147] | Chr 2 | Reverse Strand | 607 |
| OCHPRC. 106_s_at | Expression probeset | Sense (Fully Exonic) | 11 | ENSG000 0008711 6 | ADA MTS 2 | 9509 | ADAM metallopeptidase with thrombospondin type 1 motif, 2 [Source:HGNC Symbol;Acc:218] | Chr 5 | Reverse Strand | 608 |
| OCHP.10 72_s_ at | Expression probeset | Sense (Fully Exonic) | 11 | ENSG000 0014582 4 | CXCL 14 | 9547 | chemokine (C-X-C motif) ligand 14 [Source:HGNC Symbol;Acc: 10640] | Chr 5 | Reverse Strand | 609 |
| OC3SNGn h. 868_x_ at | Expression probeset | Sense (includes Intronic) | 11 | ENSG000 0012851 2 | DOC K4 | 9732 | dedicator of cytokinesis 4 [Source: HGNC Symbol;Acc:19192] | Chr 7 | Reverse Strand | 610 |
| OC3P.617 5.C1_s_at | Expression probeset | Sense (Fully Exonic) | 11 | ENSG000 0008947 2 | HEP H | 9843 | hephaestin [Source:HGNC Symbol;Acc:4866] | Chr X | Forward Strand | 611 |
| OC3SNGn . 1784-2686a_s_ at | Expression probeset | Sense (Fully Exonic) | 11 | ENSG000 0011702 0 | AKT3 | 1000 0 | v-akt murine thymoma viral oncogene homolog 3 [Source: HGNC Symbol;Acc:393] | Chr 1 | Reverse Strand | 612 |
| OC3P.126 48.C1_s_ at | Expression probeset | Sense (Fully Exonic) | 11 | ENSG000 0011196 2 | UST | 1009 0 | uronyl-2-sulfotransferase [Source:HGNC Symbol;Acc: 17223] | Chr 6 | Forward Strand | 613 |
| OC3SNGn . 2612-800a_s_a t | Expression probeset | Sense (Fully Exonic) | 11 | ENSG000 0012264 4 | ARL4 A | 1012 4 | ADP-ribosylation factor-like 4A [Source:HGNC Symbol;Acc:695] | Chr 7 | Forward Strand | 614 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCADA.3 083_s_at | Expression probeset | Sense (Fully Exonic) | 11 | ENSG000 0013979 3 | MBN L2 | 1015 0 | muscleblind-like splicing regulator 2 [Source:HGNC Symbol;Acc: 16746] | Chr 13 | Forwar d Strand | 615 |
| OCADNP. 8513_s_a t | Expression probeset | Sense (Fully Exonic) | 11 | ENSG000 0000831 1 | AASS | 1015 7 | aminoadipate-semialdehyde synthase [Source:HGNC Symbol; Acc:17366] | Chr 7 | Revers e Strand | 616 |
| OCADA.3 572_s_at | Expression probeset | Sense (Fully Exonic) | 11 | ENSG000 0020497 7 | TRIM 13 | 1020 6 | tripartite motif containing 13 [Source:HGNC Symbol;Acc: 9976] | Chr 13 | Forwar d Strand | 617 |
| OC3P.253 7.CB1_s_ at | Expression probeset | Sense (Fully Exonic) | 11 | ENSG000 0010133 5 | MYL 9 | 1039 8 | myosin, light chain 9, regulatory [Source:HGNC Symbol;Acc: 15754] | Chr 20 | Forwar d Strand | 618 |
| OCHP.16 4_s_at | Expression probeset | Sense (Fully Exonic) | 11 | ENSG000 0010100 0 | PRO CR | 1054 4 | protein C receptor, endothelial [Source:HGNC Symbol;Acc: 9452] | Chr 20 | Forwar d Strand | 619 |
| OC3SNGn h. 17196_ s_at | Expression probeset | Sense (Fully Exonic) | 11 | ENSG000 0017359 8 | NUD T4 | 1116 3 | nudix (nucleoside diphosphate linked moiety X)-type motif 4 [Source:HGNC Symbol;Acc: 8051] | Chr 12 | Forwar d Strand | 620 |
| OC3P. 211 .C1_x_at | Expression probeset | Sense (Fully Exonic) | 6 | ENSG000 0016343 0 | FSTL 1 | 1116 7 | follistatin-like 1 [Source:HGNC Symbol;Acc:3972] | Chr 3 | Revers e Strand | 621 |
| OCMXSN G. 5380_x _at | Expression probeset | Sense (Fully Exonic) | 10 | ENSG000 0011809 4 | TREH | 1118 1 | trehalase (brush-border membrane glycoprotein) [Source: HGNC Symbol;Acc:12266] | Chr 11 | Revers e Strand | 622 |
| OC3P.117 25.C1_at | Expression probeset | Sense (Fully Exonic) | 11 | ENSG000 0016607 3 | GPR 176 | 1124 5 | G protein-coupled receptor 176 [Source:HGNC Symbol;Acc: 32370] | Chr 15 | Revers e Strand | 623 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCMX.38 83.C1_s_ at | Expression probeset | Sense (includes Intronic) | 11 | ENSG000 0011892 2 | KLF1 2 | 1127 8 | Kruppel-like factor 12 [Source: HGNC Symbol;Acc:6346] | Chr 13 | Reverse Strand | 624 |
| OC3SNG. 3488-18a_s_at | Expression probeset | Sense (Fully Exonic) | 11 | ENSG000 0010234 9 | KLF8 | 1127 9 | Kruppel-like factor 8 [Source: HGNC Symbol;Acc:6351] | Chr X | Forward Strand | 625 |
| OC3P. 164 .C1_s_at | Expression probeset | Sense (Fully Exonic) | 11 | ENSG000 0008730 3 | NID2 | 2279 5 | nidogen 2 (osteonidogen) [Source:HGNC Symbol;Acc: 13389] | Chr 14 | Reverse Strand | 626 |
| OC3SNG. 2402-2883a_s_ at | Expression probeset | Sense (Fully Exonic) | 11 | ENSG000 0016429 2 | RHO BTB3 | 2283 6 | Rho-related BTB domain containing 3 [Source:HGNC Symbol;Acc:18757] | Chr 5 | Forward Strand | 627 |
| OC3SNGn h. 14944_ at | Expression probeset | Sense (includes Intronic) | 11 | ENSG000 0015324 6 | PLA2 R1 | 2292 5 | phospholipase A2 receptor 1, 180kDa [Source:HGNC Symbol; Acc:9042] | Chr 2 | Reverse Strand | 628 |
| OC3SNGn h. 9821_s _at | Expression probeset | AntiSense | 11 | ENSG000 0010059 2 | DAA M1 | 2300 2 | dishevelled associated activator of morphogenesis 1 [Source:HGNC Symbol;Acc:18142] | Chr 14 | Forward Strand | 629 |
| OC3P.100 89.C1_at | Expression probeset | Sense (Fully Exonic) | 11 | ENSG000 0012121 0 | KIAA 0922 | 2324 0 | KIAA0922 [Source:HGNC Symbol;Acc:29146] | Chr 4 | Forward Strand | 630 |
| OC3P.114 85.C1_s_ at | Expression probeset | Sense (Fully Exonic) | 11 | ENSG000 0015601 1 | PSD3 | 2336 2 | pleckstrin and Sec7 domain containing 3 [Source:HGNC Symbol;Acc:19093] | Chr 8 | Reverse Strand | 631 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3P.267 9.C1_s_at | Expression probeset | Sense (Fully Exonic) | 11 | ENSG000 0013685 9 | ANG PTL2 | 2345 2 | angiopoietin-like 2 [Source:HGNC Symbol;Acc:490] | Chr 9 | Reverse Strand | 632 |
| OC3SNGn h. 20422_ s_at | Expression probeset | Sense (Fully Exonic) | 11 | ENSG000 0027297 2 | SPID R | 2351 4 | scaffolding protein involved in DNA repair [Source:HGNC Symbol;Acc:28971] | Chr HG1 699_ PATC H | Forward Strand | 633 |
| OCADA.2 087_s_at | Expression probeset | Sense (includes Intronic) | 11 | ENSG000 0014464 2 | RBM S3 | 2730 3 | RNA binding motif, single stranded interacting protein 3 [Source:HGNC Symbol;Acc: 13427] | Chr 3 | Forward Strand | 634 |
| OCRS.320 _s_at | Expression probeset | Sense (Fully Exonic) | 11 | ENSG000 0008699 1 | NOX 4 | 5050 7 | NADPH oxidase 4 [Source:HGNC Symbol;Acc:7891] | Chr 11 | Reverse Strand | 635 |
| OC3SNGn . 793-57a_s_at | Expression probeset | Sense (Fully Exonic) | 11 | ENSG000 0019745 7 | STM N3 | 5086 1 | stathmin-like 3 [Source:HGNC Symbol;Acc:15926] | Chr 20 | Reverse Strand | 636 |
| OC3P.346 O.C1_s_at | Expression probeset | Sense (Fully Exonic) | 11 | ENSG000 0013610 0 | VPS3 6 | 5102 8 | vacuolar protein sorting 36 homolog (S. cerevisiae) [Source: HGNC Symbol;Acc:20312] | Chr 13 | Reverse Strand | 637 |
| OC3P.641 7.C1_s_at | Expression probeset | Sense (Fully Exonic) | 11 | ENSG000 0016412 5 | FAM 198B | 5131 3 | family with sequence similarity 198, member B [Source:HGNC Symbol;Acc:25312] | Chr 4 | Reverse Strand | 638 |
| OC3P.665 9.C1_s_at | Expression probeset | Sense (Fully Exonic) | 11 | ENSG000 0018202 2 | CHST 15 | 5136 3 | carbohydrate (N-acetylgalactosamine 4-sulfate 6-O) sulfotransferase 15 [Source: HGNC Symbol;Acc:18137] | Chr 10 | Reverse Strand | 639 |

(continued)

| Probe Set ID | Type | Orient ation | No. prob es align ed | Ensembl Gene | Gene Sym bol | Entr ez Gene ID | Description | Chro mos ome | Strand | SEQ ID NO |
|---|---|---|---|---|---|---|---|---|---|---|
| OC3SNGn . 7890-859a_x_a t | Expression probeset | Sense (Fully Exonic) | 11 | ENSG000 0016255 2 | WNT 4 | 5436 1 | wingless-type MMTV integration site family, member 4 [Source: HGNC Symbol;Acc:12783] | Chr 1 | Revers e Strand | 640 |
| OC3P.119 93.C1_s_ at | Expression probeset | Sense (Fully Exonic) | 11 | ENSG000 0008882 6 | SMO X | 5449 8 | spermine oxidase [Source:HGNC Symbol;Acc:15862] | Chr 20 | Forwar d Strand | 641 |
| OC3P.523 O.C1_s_at | Expression probeset | Sense (Fully Exonic) | 11 | ENSG000 0008628 9 | EPDR 1 | 5474 9 | ependymin related 1 [Source: HGNC Symbol;Acc:17572] | Chr 7 | Forwar d Strand | 642 |
| OC3P.991 O.C1_s_at | Expression probeset | Sense (Fully Exonic) | 11 | ENSG000 0016245 8 | FBLI M1 | 5475 1 | filamin binding LIM protein 1 [Source:HGNC Symbol;Acc: 24686] | Chr 1 | Forwar d Strand | 643 |
| OC3SNG. 4208-25a_x_at | Expression probeset | Sense (Fully Exonic) | 9 | ENSG000 0019811 3 | TOR 4A | 5486 3 | torsin family 4, member A [Source: HGNC Symbol;Acc:25981] | Chr 9 | Forwar d Strand | 644 |
| OCMX.17 60.C2_s_ at | Expression probeset | AntiSe nse | 11 | ENSG000 0014754 8 | WHS C1L1 | 5490 4 | Wolf-Hirschhorn syndrome candidate 1-like 1 [Source:HGNC Symbol;Acc:12767] | Chr 8 | Revers e Strand | 645 |
| OCADNP. 12059_s_ at | Expression probeset | Sense (Fully Exonic) | 11 | ENSG000 0021454 8 | MEG 3 | 5538 4 | maternally expressed 3 (non-protein coding) [Source:HGNC Symbol;Acc:14575] | Chr 14 | Forwar d Strand | 646 |
| OC3P.953 2.C1_s_at | Expression probeset | Sense (Fully Exonic) | 11 | ENSG000 0021454 8 | MEG 3 | 5538 4 | maternally expressed 3 (non-protein coding) [Source:HGNC Symbol;Acc:14575] | Chr 14 | Forwar d Strand | 647 |
| OC3P.136 42.C1_s_ at | Expression probeset | Sense (Fully Exonic) | 11 | ENSG000 0021454 8 | MEG 3 | 5538 4 | maternally expressed 3 (non-protein coding) [Source:HGNC Symbol;Acc:14575] | Chr 14 | Forwar d Strand | 648 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3P.217 9.C1_s_at | Expression probeset | Sense (Fully Exonic) | 11 | ENSG000 0019656 2 | SULF 2 | 5595 9 | sulfatase 2 [Source:HGNC Symbol;Acc:20392] | Chr 20 | Revers e Strand | 649 |
| OC3P.100 40.C1_s_ at | Expression probeset | Sense (Fully Exonic) | 10 | ENSG000 0014543 1 | PDG FC | 5603 4 | platelet derived growth factor C [Source:HGNC Symbol;Acc: 8801] | Chr 4 | Revers e Strand | 650 |
| OCADA.1 904_s_at | Expression probeset | Sense (includ es Introni c) | 11 | ENSG000 0014543 1 | PDG FC | 5603 4 | platelet derived growth factor C [Source:HGNC Symbol;Acc: 8801] | Chr 4 | Revers e Strand | 651 |
| OCMXSN G. 126_x_ at | Expression probeset | AntiSe nse | 11 | ENSG000 0012582 7 | TMX 4 | 5625 5 | thioredoxin-related transmembrane protein 4 [Source: HGNC Symbol;Acc:25237] | Chr 20 | Revers e Strand | 652 |
| OC3P.136 3.C1_s_at | Expression probeset | Sense (Fully Exonic) | 11 | ENSG000 0010728 1 | NPD C1 | 5665 4 | neural proliferation, differentiation and control, 1 [Source:HGNC Symbol;Acc:7899] | Chr 9 | Revers e Strand | 653 |
| OCADA.1 1214_s_a t | Expression probeset | Sense (Fully Exonic) | 11 | ENSG000 0006317 6 | SPHK 2 | 5684 8 | sphingosine kinase 2 [Source: HGNC Symbol;Acc:18859] | Chr 19 | Forwar d Strand | 654 |
| OC3P.122 59.C1_x_ at | Expression probeset | Sense (Fully Exonic) | 11 | ENSG000 0001163 8 | TME M15 9 | 5714 6 | transmembrane protein 159 [Source:HGNC Symbol;Acc: 30136] | Chr 16 | Forwar d Strand | 655 |
| OC3P.138 55.C1_s_ at | Expression probeset | Sense (Fully Exonic) | 11 | ENSG000 0012027 8 | PLEK HG1 | 5748 0 | pleckstrin homology domain containing, family G (with RhoGef domain) member 1 [Source: HGNC Symbol;Acc:20884] | Chr 6 | Forwar d Strand | 656 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3SNGn h. 4306_s _at | Expression probeset | AntiSense | 11 | ENSG000 0012027 8 | PLEKHG1 | 5748 0 | pleckstrin homology domain containing, family G (with RhoGef domain) member 1 [Source: HGNC Symbol;Acc:20884] | Chr 6 | Forward Strand | 657 |
| OCADA.6 529_s_at | Expression probeset | AntiSense | 11 | ENSG000 0016891 6 | ZNF608 | 5750 7 | zinc finger protein 608 [Source: HGNC Symbol;Acc:29238] | Chr 5 | Reverse Strand | 658 |
| OC3P.142 64.C1_s_ at | Expression probeset | Sense (Fully Exonic) | 10 | ENSG000 0017128 2 | N/A | 5759 7 | BAH and coiled-coil domain-containing protein 1 [Source: RefSeq peptide;Acc:NP_ 001073988] | Chr 17 | Forward Strand | 659 |
| OC3P.135 17.C1_s_ at | Expression probeset | Sense (Fully Exonic) | 11 | ENSG000 0013108 0 | EDA2R | 6040 1 | ectodysplasin A2 receptor [Source:HGNC Symbol;Acc: 17756] | Chr X | Reverse Strand | 660 |
| OCMXSN G. 274_s_ at | Expression probeset | AntiSense | 11 | ENSG000 0014480 2 | NFKBIZ | 6433 2 | nuclear factor of kappa light polypeptide gene enhancer in B-cells inhibitor, zeta [Source:HGNC Symbol;Acc:29805] | Chr 3 | Forward Strand | 661 |
| OC3P. 697 .C1_s_at | Expression probeset | Sense (Fully Exonic) | 11 | ENSG000 0014480 2 | NFKBIZ | 6433 2 | nuclear factor of kappa light polypeptide gene enhancer in B-cells inhibitor, zeta [Source:HGNC Symbol;Acc:29805] | Chr 3 | Forward Strand | 662 |
| OC3SNG. 3829-22a_s_at | Expression probeset | Sense (Fully Exonic) | 11 | ENSG000 0014465 5 | CSRNP1 | 6465 1 | cysteine-serine-rich nuclear protein 1 [Source:HGNC Symbol; Acc:14300] | Chr 3 | Reverse Strand | 663 |
| OC3SNGn h. 17408_ s_at | Expression probeset | Sense (Fully Exonic) | 11 | ENSG000 0019734 3 | ZNF655 | 7902 7 /// 1019 2949 6 | zinc finger protein 655 [Source: HGNC Symbol;Acc:30899] | Chr 7 | Forward Strand | 664 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OCADNP. 13827_s_ at | Expression probeset | Sense (includes Intronic) | 11 | ENSG000 0025839 9 | MEG 8 | 7910 4/// 6922 15 | maternally expressed 8 (non-protein coding) [Source:HGNC Symbol;Acc:14574] | Chr 14 | Forward Strand | 665 |
| OC3P.120 74.C1_s_ at | Expression probeset | Sense (includes Intronic) | 11 | ENSG000 0025839 9 | MEG 8 | 7910 4/// 6922 15 | maternally expressed 8 (non-protein coding) [Source:HGNC Symbol;Acc:14574] | Chr 14 | Forward Strand | 666 |
| OC3SNGn h. 12321_ s_at | Expression probeset | Sense (includes Intronic) | 8 | ENSG000 0017087 6 | TME M43 | 7918 8 | transmembrane protein 43 [Source:HGNC Symbol;Acc: 28472] | Chr 3 | Forward Strand | 667 |
| OCRS2.94 32_s_at | Expression probeset | Sense (Fully Exonic) | 11 | ENSG000 0014445 1 | SPAG 16 | 7958 2 | sperm associated antigen 16 [Source:HGNC Symbol;Acc: 23225] | Chr 2 | Forward Strand | 668 |
| OCMX.13 587.C1_s at | Expression probeset | Sense (Fully Exonic) | 11 | ENSG000 0016198 1 | SNR NP25 | 7962 2 | small nuclear ribonucleoprotein 25kDa (U11/U12) [Source:HGNC Symbol;Acc:14161] | Chr 16 | Forward Strand | 669 |
| OCHP.17 9_x_at | Expression probeset | Sense (Fully Exonic) | 11 | ENSG000 0012421 2 | PTGI S | 5740 | prostaglandin I2 (prostacyclin) synthase [Source:HGNC Symbol; Acc:9603] | Chr 20 | Reverse Strand | 670 |
| OCMX.14 790.C1_a t | Expression probeset | Sense (includes Intronic) | 10 | ENSG000 0015276 3 | WDR 78 | 7981 9 | WD repeat domain 78 [Source: HGNC Symbol;Acc:26252] | Chr 1 | Reverse Strand | 671 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3SNGn h. 16119_ at | Expression probeset | Sense (includes Intronic) | 9 | ENSG000 0017096 2 | PDG FD | 8031 0 | platelet derived growth factor D [Source:HGNC Symbol;Acc: 30620] | Chr 11 | Reverse Strand | 672 |
| OC3P.576 6.C1_s_at | Expression probeset | Sense (Fully Exonic) | 11 | ENSG000 0014539 1 | SETD 7 | 8085 4 | SET domain containing (lysine methyltransferase) 7 [Source: HGNC Symbol;Acc:30412] | Chr 4 | Reverse Strand | 673 |
| OC3P.564 .Cl-358a_s_a t | Expression probeset | Sense (Fully Exonic) | 11 | ENSG000 0006271 6 | VMP 1 | 8167 1 /// 4069 91 | vacuole membrane protein 1 [Source:HGNC Symbol;Acc: 29559] | Chr 17 | Forward Strand | 674 |
| OC3P. 564 .C1_s_at | Expression probeset | Sense (Fully Exonic) | 9 | ENSG000 0006271 6 | VMP 1 | 8167 1 /// 4069 91 | vacuole membrane protein 1 [Source:HGNC Symbol;Acc: 29559] | Chr 17 | Forward Strand | 675 |
| OC3SNGn h. 4611_s _at | Expression probeset | Sense (Fully Exonic) | 11 | ENSG000 0021302 0 | ZNF6 11 | 8185 6 | zinc finger protein 611 [Source: HGNC Symbol;Acc:28766] | Chr 19 | Reverse Strand | 676 |
| OC3P. 560 .C1_s_at | Expression probeset | Sense (Fully Exonic) | 11 | ENSG000 0016608 6 | JAM 3 | 8370 0 | junctional adhesion molecule 3 [Source:HGNC Symbol;Acc: 15532] | Chr 11 | Forward Strand | 677 |
| OC3P. 925 .C1_s_at | Expression probeset | Sense (Fully Exonic) | 11 | ENSG000 0016960 4 | ANT XR1 | 8416 8 | anthrax toxin receptor 1 [Source: HGNC Symbol;Acc:21014] | Chr 2 | Forward Strand | 678 |
| OCMXSN G. 3100_s _at | Expression probeset | Sense (includes Intronic) | 11 | ENSG000 0018422 0 | CMS S1 | 8431 9 | cmsl ribosomal small subunit homolog (yeast) [Source:HGNC Symbol;Acc:28666] | Chr 3 | Forward Strand | 679 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCMX.33 29.C1_s at | Expression probeset | Sense (includes Intronic) | 11 | ENSG000 0018422 0 | CMS S1 | 8431 9 | cmsl ribosomal small subunit homolog (yeast) [Source:HGNC Symbol;Acc:28666] | Chr 3 | Forward Strand | 680 |
| OC3P.676 9.C1_s_at | Expression probeset | Sense (Fully Exonic) | 11 | ENSG000 0017147 6 | HOP X | 8452 5 | HOP homeobox [Source:HGNC Symbol;Acc:24961] | Chr 4 | Reverse Strand | 681 |
| OC3P. 632 .C1_s_at | Expression probeset | Sense (Fully Exonic) | 11 | ENSG000 0016469 4 | FND C1 | 8462 4 | fibronectin type III domain containing 1 [Source:HGNC Symbol;Acc:21184] | Chr 6 | Forward Strand | 682 |
| OC3P.439 O.C1_s_at | Expression probeset | Sense (Fully Exonic) | 11 | ENSG000 0019673 9 | COL2 7A1 | 8530 1 | collagen, type XXVII, alpha 1 [Source:HGNC Symbol;Acc: 22986] | Chr 9 | Forward Strand | 683 |
| OC3P.134 98.C1_s at | Expression probeset | Sense (Fully Exonic) | 11 | ENSG000 0013436 9 | NAV 1 | 8979 6 | neuron navigator 1 [Source: HGNC Symbol;Acc:15989] | Chr 1 | Forward Strand | 684 |
| OCADA.3 141_s_at | Expression probeset | Sense (Fully Exonic) | 11 | ENSG000 0015757 0 | TSPA N18 | 9013 9 | tetraspanin 18 [Source:HGNC Symbol;Acc:20660] | Chr 11 | Forward Strand | 685 |
| OC3P.136 29.C1_s at | Expression probeset | Sense (Fully Exonic) | 9 | ENSG000 0013860 6 | SHF | 9052 5 | Src homology 2 domain containing F [Source:HGNC Symbol;Acc: 25116] | Chr 15 | Reverse Strand | 686 |
| OCADNP. 7019_s_a t | Expression probeset | Sense (Fully Exonic) | 11 | ENSG000 0022186 6 | PLXN A4 | 9158 4 | plexin A4 [Source:HGNC Symbol; Acc:9102] | Chr 7 | Reverse Strand | 687 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3P.286 O.C1_s_at | Expression probeset | Sense (Fully Exonic) | 11 | ENSG000 0020378 0 | FAN K1 | 9256 5 | fibronectin type III and ankyrin repeat domains 1 [Source:HGNC Symbol;Acc:23527] | Chr 10 | Forwar d Strand | 688 |
| OC3P.763 8.C1_s_at | Expression probeset | Sense (Fully Exonic) | 11 | ENSG000 0015680 4 | FBXO 32 | 1149 07 | F-box protein 32 [Source:HGNC Symbol;Acc:16731] | Chr 8 | Revers e Strand | 689 |
| OCHP.19 _s_at | Expression probeset | AntiSe nse | 8 | ENSG000 0016493 2 | CTHR C1 | 1159 08 | collagen triple helix repeat containing 1 [Source:HGNC Symbol;Acc:18831] | Chr 8 | Forwar d Strand | 690 |
| OC3P.976 4.C1_s_at | Expression probeset | Sense (Fully Exonic) | 11 | ENSG000 0016329 7 | ANT XR2 | 1184 29 | anthrax toxin receptor 2 [Source: HGNC Symbol;Acc:21732] | Chr 4 | Revers e Strand | 691 |
| OC3P.896 5.C1_s_at | Expression probeset | Sense (Fully Exonic) | 11 | ENSG000 0014093 1 | CMT M3 | 1239 20 | CKLF-like MARVEL transmembrane domain containing 3 [Source:HGNC Symbol;Acc:19174] | Chr 16 | Forwar d Strand | 692 |
| OCADA.1 2049_s_a t | Expression probeset | Sense (Fully Exonic) | 10 | ENSG000 0018777 3 | FAM 69C | 1257 04 | family with sequence similarity 69, member C [Source:HGNC Symbol;Acc:31729] | Chr 18 | Revers e Strand | 693 |
| OC3SNG. 3388-17a_s_at | Expression probeset | Sense (Fully Exonic) | 11 | ENSG000 0010123 0 | ISM1 | 1408 62 | isthmin 1, angiogenesis inhibitor [Source:HGNC Symbol;Acc: 16213] | Chr 20 | Forwar d Strand | 694 |
| OCADNP. 13664_s_ at | Expression probeset | Sense (Fully Exonic) | 11 | ENSG000 0014921 2 | SESN 3 | 1436 86 | sestrin 3 [Source:HGNC Symbol; Acc:23060] | Chr 11 | Revers e Strand | 695 |
| OCHP.14 23_s_ at | Expression probeset | Sense (Fully Exonic) | 11 | ENSG000 0015485 6 | APC DD1 | 1474 95 | adenomatosis polyposis coli down-regulated 1 [Source:HGNC Symbol;Acc:15718] | Chr 18 | Forwar d Strand | 696 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3SNGn . 3144-297a_s_a t | Expression probeset | Sense (Fully Exonic) | 11 | ENSG000 0018878 5 | ZNF5 48 | 1476 94 | zinc finger protein 548 [Source: HGNC Symbol;Acc:26561] | Chr 19 | Forward Strand | 697 |
| OC3P.142 66.C1-547a_s_a t | Expression probeset | Sense (Fully Exonic) | 11 | ENSG000 0026080 4 | N/A | 1509 67 | NOVEL lincRNA (Clone_based_ vega_gene) | Chr 2 | Forward Strand | 698 |
| OC3SNG. 5645-98a_x_at | Expression probeset | Sense (Fully Exonic) | 11 | ENSG000 0009198 6 | CCD C80 | 1518 87 | coiled-coil domain containing 80 [Source:HGNC Sym bol;Acc: 30649] | Chr 3 | Reverse Strand | 699 |
| OC3P.144 03.C1_s_ at | Expression probeset | Sense (Fully Exonic) | 11 | ENSG000 0015161 2 | ZNF8 27 | 1524 85 /// 1019 2770 7 | zinc finger protein 827 [Source: HGNC Symbol;Acc:27193] | Chr 4 | Reverse Strand | 700 |
| OC3SNGn . 538-5592a_s_ at | Expression probeset | Sense (Fully Exonic) | 11 | ENSG000 0016602 5 | AMO TL1 | 1548 10 | angiomotin like 1 [Source:HGNC Symbol;Acc:17811] | Chr 11 | Forward Strand | 701 |
| OCRS.115 _s_at | Expression probeset | Sense (Fully Exonic) | 11 | ENSG000 0013285 4 | KAN K4 | 1637 82 | KN motif and ankyrin repeat domains 4 [Source:HGNC Symbol;Acc:27263] | Chr 1 | Reverse Strand | 702 |
| OC3P.591 3.C1_s_at | Expression probeset | Sense (Fully Exonic) | 11 | ENSG000 0016363 7 | PRIC KLE2 | 1663 36 | prickle homolog 2 (Drosophila) [Source:HGNC Symbol;Acc: 20340] | Chr 3 | Reverse Strand | 703 |
| OC3SNGn h. 4002_s _at | Expression probeset | Sense (Fully Exonic) | 9 | ENSG000 0025327 6 | CCD C71L | 1684 55 | coiled-coil domain containing 71-like [Source:HGNC Symbol;Acc: 26685] | Chr 7 | Reverse Strand | 704 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCADA.5 390_s_at | Expression probeset | Sense (includes Intronic) | 9 | ENSG000 0017240 3 | SYNP O2 | 1710 24 | synaptopodin 2 [Source:HGNC Symbo;Acc:17732] | Chr 4 | Forward Strand | 705 |
| OC3SNGn h. 11631_ s_at | Expression probeset | Sense (includes Intronic) | 11 | ENSG000 0014655 5 | SDK1 | 2219 35 | sidekick cell adhesion molecule 1 [Source:HGNC Symbol;Acc: 19307] | Chr 7 | Forward Strand | 706 |
| OC3SNGn . 2710-2169a_s_ at | Expression probeset | Sense (Fully Exonic) | 11 | ENSG000 0018035 4 | MTU RN | 2221 66 | maturin, neural progenitor differentiation regulator homolog (Xenopus) [Source:HGNC Symbol;Acc:25457] | Chr 7 | Forward Strand | 707 |
| OC3P.110 44.C1_at | Expression probeset | Sense (includes Intronic) | 11 | ENSG000 0016430 0 | SERI NC5 | 2569 87 | serine incorporator 5 [Source: HGNC Symbol;Acc:18825] | Chr 5 | Reverse Strand | 708 |
| OC3P.110 44.C1_x_ at | Expression probeset | Sense (includes Intronic) | 10 | ENSG000 0016430 0 | SERI NC5 | 2569 87 | serine incorporator 5 [Source: HGNC Symbol;Acc:18825] | Chr 5 | Reverse Strand | 709 |
| OCMX.11 593.C1_a t | Expression probeset | Sense (includes Intronic) | 10 | ENSG000 0016430 0 | SERI NC5 | 2569 87 | serine incorporator 5 [Source: HGNC Symbol;Acc:18825] | Chr 5 | Reverse Strand | 710 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3SNG. 1416-18a_s_at | Expression probeset | Sense (Fully Exonic) | 11 | ENSG000 0017746 9 | PTRF | 2841 19 | polymerase I and transcript release factor [Source:HGNC Symbol;Acc:9688] | Chr 17 | Reverse Strand | 711 |
| OC3P. 603 .C1_at | Expression probeset | Sense (Fully Exonic) | 11 | ENSG000 0016167 1 | EMC 10 | 2843 61 | ER membrane protein complex subunit 10 [Source:HGNC Symbol;Acc:27609] | Chr 19 | Forward Strand | 712 |
| OC3SNGn h. 20169_ s_at | Expression probeset | Sense (Fully Exonic) | 11 | ENSG000 0016316 2 | RNF1 49 | 2849 96 | ring finger protein 149 [Source: HGNC Symbol;Acc:23137] | Chr 2 | Reverse Strand | 713 |
| OCRS2.12 918_s_at | Expression probeset | Sense (Fully Exonic) | 11 | ENSG000 0018668 4 | CYP2 7C1 | 3397 61 | cytochrome P450, family 27, subfamily C, polypeptide 1 [Source:HGNC Symbol;Acc: 33480] | Chr 2 | Reverse Strand | 714 |
| OC3P.816 9.C1_s_at | Expression probeset | Sense (Fully Exonic) | 11 | ENSG000 0018368 8 | FAM 101B | 3598 45 | family with sequence similarity 101, member B [Source:HGNC Symbol;Acc:28705] | Chr 17 | Reverse Strand | 715 |
| OC3P. 536 .C4_x_at | Expression probeset | Sense (includes Intronic) | 11 | ENSG000 0022459 7 | PTCH D3P1 | 3876 47 | patched domain containing 3 pseudogene 1 [Source:HGNC Symbol;Acc:44945] | Chr 10 | Forward Strand | 716 |
| OCADNP. 9287_s_a t | Expression probeset | Sense (Fully Exonic) | 8 | ENSG000 0014659 2 | CREB 5 | 9586 /// 4013 17 | cAMP responsive element binding protein 5 [Source:HGNC Symbol; Acc:16844] | Chr 7 | Forward Strand | 717 |
| OCADNP. 7150_s_a t | Expression probeset | Sense (Fully Exonic) | 11 | ENSG000 0014659 2 | CREB 5 | 9586 /// 4013 17 | cAMP responsive element binding protein 5 [Source:HGNC Symbol; Acc:16844] | Chr 7 | Forward Strand | 718 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3P.240 9.C1_s_at | Expression probeset | Sense (Fully Exonic) | 11 | ENSG000 0006271 6 | VMP 1 | 8167 1 /// 4069 91 | vacuole membrane protein 1 [Source:HGNC Symbol;Acc: 29559] | Chr 17 | Forward Strand | 719 |
| OC3P.603 1.C1_s_at | Expression probeset | Sense (Fully Exonic) | 11 | ENSG000 0018659 4 | MIR2 2HG | 8498 1 /// 4070 04 | MIR22 host gene (non-protein coding) [Source:HGNC Symbol; Acc:28219] | Chr 17 | Reverse Strand | 720 |
| OCRS2.73 32_s_at | Expression probeset | Sense (Fully Exonic) | 11 | ENSG000 0018659 4 | MIR2 2HG | 8498 1 /// 4070 04 | MIR22 host gene (non-protein coding) [Source:HGNC Symbol; Acc:28219] | Chr 17 | Reverse Strand | 721 |
| OC3SNGn h. 18279_ x_at | Expression probeset | Sense (includes Intronic) | 10 | ENSG000 0017188 9 | MIR3 1HG | 5542 02 | MIR31 host gene (non-protein coding) [Source:HGNC Symbol; Acc:37187] | Chr 9 | Reverse Strand | 722 |
| OC3SNGn h. 18279_ at | Expression probeset | Sense (includes Intronic) | 11 | ENSG000 0017188 9 | MIR3 1HG | 5542 02 | MIR31 host gene (non-protein coding) [Source:HGNC Symbol; Acc:37187] | Chr 9 | Reverse Strand | 723 |
| OCADA.3 940_s_at | Expression probeset | Sense (Fully Exonic) | 11 | ENSG000 0020414 7 | ASA H2B | 6533 08 | N-acylsphingosine amidohydrolase (non-lysosomal ceramidase) 2B [Source:HGNC Symbol;Acc:23456] | Chr 10 | Forward Strand | 724 |
| OC3SNGn h. 7474_s _at | Expression probeset | Sense (includes Intronic) | 11 | ENSG000 0017298 6 | GXYL T2 | 7279 36 | glucoside xylosyltransferase 2 [Source:HGNC Symbol;Acc: 33383] | Chr 3 | Forward Strand | 725 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCADA.9 028_s_at | Expression probeset | Sense (Fully Exonic) | 11 | ENSG000 0017298 6 | GXYL T2 | 7279 36 | glucoside xylosyltransferase 2 [Source:HGNC Symbol;Acc: 33383] | Chr 3 | Forward Strand | 726 |
| OC3SNGn h. 6774_x _at | Expression probeset | AntiSense | 11 | ENSG000 0017298 6 | GXYL T2 | 7279 36 | glucoside xylosyltransferase 2 [Source:HGNC Symbol;Acc: 33383] | Chr 3 | Forward Strand | 727 |
| OC3P.274 2.C1_at | Expression probeset | AntiSense | 11 | ENSG000 0018210 9 | N/A | 7284 48 | NOVEL antisense (Clone_based_ vega_gene) | Chr 1 | Reverse Strand | 728 |
| OC3P.117 17.C1_s_ at | Expression probeset | Sense (includes Intronic) | 11 | ENSG000 0018182 6 | RELL 1 | 7682 11 | RELT-like 1 [Source:HGNC Symbol;Acc:27379] | Chr 4 | Reverse Strand | 729 |
| OCRS2.12 554_x_at | Expression probeset | Sense (Fully Exonic) | 11 | ENSG000 0022407 8 | SNH G14 | 3653 /// 9138 0/// 3477 46 /// 1000 3341 6/// 1000 3343 3/// 1000 3344 4/// 1000 3345 0/// 1000 3380 2/// 1000 3382 0/// 1019 3040 4 | small nucleolar RNA host gene 14 (non-protein coding) [Source: HGNC Symbol;Acc:37462] | Chr 15 | Forward Strand | 730 |
| OCRS2.10 334_x_at | Expression probeset | Sense (Fully Exonic) | 11 | ENSG000 0020709 3 | SNO RD11 6-8 | 1000 3342 0 | small nucleolar RNA, C/D box 116-8 [Source:HGNC Symbol; Acc:33074] | Chr 15 | Forward Strand | 731 |
| OCRS2.11 097_x_at | Expression probeset | Sense (Fully Exonic) | 11 | ENSG000 0020737 5 | SNO RD11 6-23 | 1000 3343 4 | small nucleolar RNA, C/D box 116-23 [Source:HGNC Symbol; Acc:33089] | Chr 15 | Forward Strand | 732 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCMXSN G. 4302_a t | Expression probeset | Sense (includes Intronic) | 11 | ENSG000 0017711 2 | MRV I1-AS1 | 1001 2982 7 | MRVI1 antisense RNA 1 [Source: HGNC Symbol;Acc:43434] | Chr 11 | Forward Strand | 733 |
| OCADA.1 0563_s_a t | Expression probeset | AntiSense | 11 | ENSG000 0019842 0 | FAM 115A | 9747 /// 1002 9403 3/// 1019 3000 3 | family with sequence similarity 115, member A [Source:HGNC Symbol;Acc:22201] | Chr 7 | Reverse Strand | 734 |
| OCMXSN G. 3737 _s _at | Expression probeset | AntiSense | 11 | ENSG000 0019842 0 | FAM 115A | 9747 /// 1002 9403 3/// 1019 3000 3 | family with sequence similarity 115, member A [Source:HGNC Symbol;Acc:22201] | Chr 7 | Reverse Strand | 735 |
| OC3P.227 8.C1_x_at | Expression probeset | Sense (Fully Exonic) | 11 | ENSG000 0016712 3 | CERC AM | 5114 8 | cerebral endothelial cell adhesion molecule [Source:HGNC Symbol; Acc:23723] | Chr 9 | Forward Strand | 736 |
| OC3P.101 47.C1_s_ at | Expression probeset | Sense (Fully Exonic) | 11 | ENSG000 0022407 8 | SNH G14 | 3653 /// 9138 0/// 3477 46 /// 1000 3341 6 /// 1000 3343 3 /// 1000 3344 4 /// 1000 3345 0/// 1000 3380 2 /// 1000 3382 0/// 1019 3040 4 | small nucleolar RNA host gene 14 (non-protein coding) [Source: HGNC Symbol;Acc:37462] | Chr 15 | Forward Strand | 737 |
| OCMX.58 42.C2_s_ at | Expression probeset | Sense (Fully Exonic) | 11 | ENSG000 0018501 5 | CA13 | 3776 77 /// 1005 0725 8 | carbonic anhydrase XIII [Source: HGNC Symbol;Acc:14914] | Chr 8 | Forward Strand | 738 |
| OC3SNGn . 812-49a_s_at | Expression probeset | Sense (Fully Exonic) | 11 | ENSG000 0007561 8 | FSCN 1 | 6624 | fascin homolog 1, actin-bundling protein (Strongylocentrotus purpuratus) [Source:HGNC Symbol;Acc:11148] | Chr 7 | Forward Strand | 739 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OCMXSN G. 5099_s _at | Expression probeset | AntiSense | 11 | ENSG000 0007561 8 | FSCN 1 | 6624 | fascin homolog 1, actin-bundling protein (Strongylocentrotus purpuratus) [Source:HGNC Symbol;Acc:11148] | Chr 7 | Forward Strand | 740 |
| OCMXSN G. 5706_a t | Expression probeset | Sense (Fully Exonic) | 11 | ENSG000 0025452 8 | N/A | N/A | NOVEL antisense (Clone_based_vega_gene) | Chr 11 | Forward Strand | 741 |
| OCMXSN G. 5706_x _at | Expression probeset | Sense (Fully Exonic) | 11 | ENSG000 0025452 8 | N/A | N/A | NOVEL antisense (Clone_based_vega_gene) | Chr 11 | Forward Strand | 742 |
| OC3P.825 8.C1_at | Expression probeset | Sense (Fully Exonic) | 8 | ENSG000 0026082 2 | N/A | N/A | NOVEL sense_overlapping (Clone_based_vega_gene) | Chr X | Forward Strand | 743 |
| OCRS2.15 06_s_at | Expression probeset | No Transcript match | 0 | 0 | 0 | 0 | 0 | 0 | 0 | 744 |
| OCHPRC. 112_s_at | Expression probeset | Sense (Fully Exonic) | 11 | ENSG000 0018353 1 | N/A | N/A | NOVEL pseudogene (Clone_based_ensembl_gene) | Chr 22 | Reverse Strand | 745 |
| OC3SNGn h. 8868_a t | Expression probeset | Sense (includes Intronic) | 11 | ENSG000 0027216 8 | CASC 15 | 4012 37 | cancer susceptibility candidate 15 (non-protein coding) [Source: HGNC Symbol;Acc:28245] | Chr 6 | Forward Strand | 746 |
| OC3P.888 9.C1_s_at | Expression probeset | Sense (Fully Exonic) | 11 | ENSG000 0016563 3 | VST M4 | 1967 40 | V-set and transmembrane domain containing 4 [Source:HGNC Symbol;Acc:26470] | Chr 10 | Reverse Strand | 747 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3SNGn h. 8417_s _at | Expression probeset | Sense (Fully Exonic) | 11 | ENSG000 0021454 8 | MEG 3 | 5538 4 | maternally expressed 3 (non-protein coding) [Source:HGNC Symbol;Acc:14575] | Chr 14 | Forward Strand | 748 |
| OC3P.544 8.C1_s_at | Expression probeset | No Transcript match | 0 | 0 | 0 | 0 | 0 | 0 | 0 | 749 |
| OCADA.8 421_s_at | Expression probeset | Sense (Fully Exonic) | 11 | ENSG000 0016904 7 | IRS1 | 3667 | insulin receptor substrate 1 [Source:HGNC Symbol;Acc: 6125] | Chr 2 | Reverse Strand | 750 |
| OCRS2.77 72_s_at | Expression probeset | No Transcript match | 0 | 0 | 0 | 0 | 0 | 0 | 0 | 751 |
| OC3P.134 44.C1_s_ at | Expression probeset | Sense (Fully Exonic) | 11 | ENSG000 0013817 5 | ARL3 | 403 | ADP-ribosylation factor-like 3 [Source:HGNC Symbol;Acc:694] | Chr 10 | Reverse Strand | 752 |
| OCADNP. 12075_s_ at | Expression probeset | Sense (Fully Exonic) | 11 | ENSG000 0021454 8 | MEG 3 | 5538 4 | maternally expressed 3 (non-protein coding) [Source:HGNC Symbol;Acc:14575] | Chr 14 | Forward Strand | 753 |
| OC3P.134 19.C1_x_ at | Expression probeset | No Transcript match | 0 | 0 | 0 | 0 | 0 | 0 | 0 | 754 |
| OCRS2.77 72_x_at | Expression probeset | No Transcript match | 0 | 0 | 0 | 0 | 0 | 0 | 0 | 755 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCRS2.19 05_x_at | Expression probeset | Sense (Fully Exonic) | 10 | ENSG000 0015675 0 | AQP 7P3 | N/A | aquaporin 7 pseudogene 3 [Source:HGNC Symbol;Acc: 31976] | Chr 9 | Forward Strand | 756 |
| OC3SNGn h. 20450_ at | Expression probeset | No Transcript match | 0 | 0 | 0 | 0 | 0 | 0 | 0 | 757 |
| OCEM.23 7_x_at | Expression probeset | Sense (includes Intronic) | 11 | ENSG000 0025802 6 | N/A | N/A | NOVEL antisense (Clone_based_ vega_gene) | Chr 12 | Reverse Strand | 758 |
| OC3P.285 8.C1_s_at | Expression probeset | No Transcript match | 0 | 0 | 0 | 0 | 0 | 0 | 0 | 759 |
| OC3SNGn . 1420-1337a_s_ at | Expression probeset | AntiSense | 11 | ENSG000 0022503 2 | N/A | N/A | NOVEL antisense (Clone_based_ vega_gene) | Chr 9 | Forward Strand | 760 |
| OC3SNGn . 5831-326a_s_a t | Expression probeset | No Transcript match | 0 | 0 | 0 | 0 | 0 | 0 | 0 | 761 |
| OCMXSN G. 2371_x _at | Expression probeset | Insufficient probes (< 6) | 0 | 0 | 0 | 0 | 0 | 0 | 0 | 762 |

(continued)

| Probe Set ID | Type | Orient ation | No. prob es align ed | Ensembl Gene | Gene Sym bol | Entr ez Gene ID | Description | Chro mos ome | Strand | SEQ ID NO |
|---|---|---|---|---|---|---|---|---|---|---|
| OCMXSN G. 2411_x _at | Expression probeset | Insufficient probes (< 6) | 0 | 0 | 0 | 0 | 0 | 0 | 0 | 763 |
| OC3SNGn h. 14683_ x_at | Expression probeset | Insufficient probes (< 6) | 0 | 0 | 0 | 0 | 0 | 0 | 0 | 764 |
| OC3SNGn h. 13886_ x_at | Expression probeset | Insufficient probes (< 6) | 0 | 0 | 0 | 0 | 0 | 0 | 0 | 765 |
| OCMXSN G. 2415_x _at | Expression probeset | No Genome match | 0 | 0 | 0 | 0 | 0 | 0 | 0 | 766 |
| OC3SNGn h. 9044_x _at | Expression probeset | Insufficient probes (< 6) | 0 | 0 | 0 | 0 | 0 | 0 | 0 | 767 |
| OCMXSN G. 2681_x _at | Expression probeset | No Genome match | 0 | 0 | 0 | 0 | 0 | 0 | 0 | 768 |
| OCMXSN G. 4333_a t | Expression probeset | Insufficient probes (< 6) | 0 | 0 | 0 | 0 | 0 | 0 | 0 | 769 |

(continued)

| Probe Set ID | Type | Orientation | No. probes aligned | Ensembl Gene | Gene Symbol | Entrez Gene ID | Description | Chromosome | Strand | SEQ ID NO |
|---|---|---|---|---|---|---|---|---|---|---|
| OC3SNGn h. 9044_a t | Expression probeset | Insufficient probes (< 6) | 0 | 0 | 0 | 0 | 0 | 0 | 0 | 770 |
| OCMXSN G. 3121_a t | Expression probeset | No Genome match | 0 | 0 | 0 | 0 | 0 | 0 | 0 | 771 |
| OC3SNGn h. 9562_x _at | Expression probeset | Insufficient probes (< 6) | 0 | 0 | 0 | 0 | 0 | 0 | 0 | 772 |
| OC3SNGn h. 6940_x _at | Expression probeset | Insufficient probes (< 6) | 0 | 0 | 0 | 0 | 0 | 0 | 0 | 773 |
| OCADA.1 0862_s_a t | Expression probeset | Insufficient probes (< 6) | 0 | 0 | 0 | 0 | 0 | 0 | 0 | 774 |
| OCMXSN G. 2184_a t | Expression probeset | AntiSense | 11 | ENSG000 0015292 6 | ZNF1 17 | 5135 1 | zinc finger protein 117 [Source: HGNC Symbol;Acc:12897] | Chr 7 | Reverse Strand | 775 |
| OC3P.310 4.C1_s_at | Expression probeset | Sense (Fully Exonic) | 10 | ENSG000 0010541 9 | MEIS 3 | 5691 7 | Meis homeobox 3 [Source:HGNC Symbol;Acc:29537] | Chr 19 | Reverse Strand | 776 |
| OCRS2.11 268_x_at | Expression probeset | Sense (Fully Exonic) | 11 | ENSG000 0020713 3 | SNO RD11 6-7 | 1000 3341 7 /// 1000 3341 9 | small nucleolar RNA, C/D box 116-7 [Source:HGNC Symbol; Acc:33073] | Chr 15 | Forward Strand | 777 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OCRS2.31 93_x_at | Expression probeset | Sense (Fully Exonic) | 11 | ENSG000 0020701 4 | SNO RD11 6-3 | 1000 3341 5 /// 1000 3342 1 | small nucleolar RNA, C/D box 116-3 [Source:HGNC Symbol; Acc:33069] | Chr 15 | Forward Strand | 778 |
| OC3SNG. 2287-17a_s_at | Expression probeset | Sense (Fully Exonic) | 9 | ENSG000 0022869 5 | CES1 P1 | 5171 6 | carboxylesterase 1 pseudogene 1 [Source:HGNC Symbol;Acc: 18546] | Chr 16 | Forward Strand | 779 |
| OCMXSN G. 2402_x _at | Expression probeset | AntiSense | 6 | ENSG000 0012996 5 | INS-IGF2 | 7239 61 | INS-IGF2 readthrough [Source: HGNC Symbol;Acc:33527] | Chr 11 | Reverse Strand | 780 |
| OCMXSN G. 2377 _x _at | Expression probeset | AntiSense | 11 | ENSG000 0012996 5 | INS-IGF2 | 7239 61 | INS-IGF2 readthrough [Source: HGNC Symbol;Acc:33527] | Chr 11 | Reverse Strand | 781 |
| OCMXSN G. 5242_x at | Expression probeset | AntiSense | 11 | ENSG000 0012996 5 | INS-IGF2 | 7239 61 | INS-IGF2 readthrough [Source: HGNC Symbol;Acc:33527] | Chr 11 | Reverse Strand | 782 |
| OCHPRC. 192_at | Expression probeset | AntiSense | 11 | ENSG000 0012996 5 | INS-IGF2 | 7239 61 | INS-IGF2 readthrough [Source: HGNC Symbol;Acc:33527] | Chr 11 | Reverse Strand | 783 |
| OCEM.12 15_s_ at | Expression probeset | AntiSense | 11 | ENSG000 0019830 0 | PEG3 | 5178 | paternally expressed 3 [Source: HGNC Symbol;Acc:8826] | Chr 19 | Reverse Strand | 784 |
| OCMXSN G. 4986_s _at | Expression probeset | AntiSense | 11 | ENSG000 0018870 7 | ZBED 6CL | 1137 63 | ZBED6 C-terminal like [Source: HGNC Symbol;Acc:21720] | Chr 7 | Forward Strand | 785 |
| OC3SNGn h. 19773_ s_at | Expression probeset | Sense (Fully Exonic) | 11 | ENSG000 0012996 5 | INS-IGF2 | 7239 61 | INS-IGF2 readthrough [Source: HGNC Symbol;Acc:33527] | Chr 11 | Reverse Strand | 786 |

(continued)

| Probe Set ID | Type | Orientation | No. probes aligned | Ensembl Gene | Gene Symbol | Entr ez Gene ID | Description | Chromosome | Strand | SEQ ID NO |
|---|---|---|---|---|---|---|---|---|---|---|
| OC3SNGn . 3705-202a_s_a t | Expression probeset | Sense (Fully Exonic) | 11 | ENSG000 0017990 9 | ZNF1 54 | 7710 | zinc finger protein 154 [Source: HGNC Symbol;Acc:12939] | Chr 19 | Revers e Strand | 787 |
| OCMXSN G. 1883_x _at | Expression probeset | AntiSe nse | 11 | ENSG000 0014519 1 | EIF2 B5 | 8893 | eukaryotic translation initiation factor 2B, subunit 5 epsilon, 82kDa [Source:HGNC Symbol;Acc: 3261] | Chr 3 | Forwar d Strand | 788 |

**Table 12: Subtype analysis: 15 Gene Signature**

| GeneID | EntrezGeneID | AUC | ANOVA (p value) | C-Index | LCI C-Index | UCI C-Index |
|---|---|---|---|---|---|---|
| CDH11 | 1009 | 0.851 | 1.35E-25 | 0.558 | 0.515 | 0.596 |
| RAB31 | 11031 | 0.816 | 1.41E-22 | 0.543 | 0.501 | 0.58 |
| COL5A1 | 1289 | 0.815 | 9.01E-22 | 0.572 | 0.534 | 0.602 |
| COL10A1 | 1300 | 0.844 | 2.30E-25 | 0.559 | 0.515 | 0.596 |
| VCAN | 1462 | 0.845 | 1.01E-23 | 0.575 | 0.533 | 0.61 |
| FAP | 2191 | 0.849 | 1.17E-26 | 0.577 | 0.536 | 0.614 |
| FN1 | 2335 | 0.818 | 1.49E-21 | 0.569 | 0.529 | 0.607 |
| ANGPTL2 | 23452 | 0.795 | 9.03E-19 | 0.561 | 0.519 | 0.599 |
| GJB2 | 2706 | 0.832 | 1.49E-24 | 0.553 | 0.512 | 0.591 |
| INHBA | 3624 | 0.825 | 4.41E-22 | 0.558 | 0.518 | 0.594 |
| MMP14 | 4323 | 0.818 | 5.29E-22 | 0.563 | 0.522 | 0.6 |
| PLAU | 5328 | 0.812 | 1.96E-20 | 0.558 | 0.513 | 0.596 |
| THBS1 | 7057 | 0.855 | 1.93E-27 | 0.541 | 0.5 | 0.583 |
| THBS2 | 7058 | 0.84 | 1.88E-25 | 0.561 | 0.516 | 0.598 |
| GFPT2 | 9945 | 0.855 | 8.37E-27 | 0.571 | 0.53 | 0.605 |

**Table 23: Subtype analysis: 45 Gene Signature**

| GeneID | AUC | ANOVA (p value) | C-Index | LCI C-Index | UCI C-Index |
|---|---|---|---|---|---|
| ALPK2 | 0.754 | 4.264E-16 | 0.529 | 0.487 | 0.566 |
| ANGPTL2 | 0.794 | 1.188E-18 | 0.560 | 0.520 | 0.599 |
| BGN | 0.817 | 4.725E-21 | 0.573 | 0.530 | 0.608 |
| BICC1 | 0.795 | 2.664E-18 | 0.551 | 0.505 | 0.592 |
| CDH11 | 0.851 | 1.917E-25 | 0.557 | 0.517 | 0.593 |
| COL10A1 | 0.844 | 3.574E-25 | 0.558 | 0.512 | 0.597 |
| COL11A1 | 0.777 | 1.927E-18 | 0.574 | 0.533 | 0.611 |
| COL1A2 | 0.832 | 8.919E-23 | 0.577 | 0.533 | 0.614 |
| COL3A1 | 0.821 | 1.998E-20 | 0.590 | 0.548 | 0.628 |
| COL5A2 | 0.816 | 5.308E-23 | 0.569 | 0.528 | 0.609 |
| COL8A1 | 0.870 | 2.680E-27 | 0.566 | 0.523 | 0.603 |
| COPZ2 | 0.810 | 8.676E-20 | 0.564 | 0.526 | 0.600 |
| CTSK | 0.804 | 1.849E-19 | 0.547 | 0.506 | 0.583 |
| FAP | 0.850 | 1.374E-26 | 0.578 | 0.538 | 0.611 |
| FN1 | 0.818 | 1.828E-21 | 0.570 | 0.529 | 0.607 |
| FZD1 | 0.780 | 2.247E-15 | 0.586 | 0.546 | 0.621 |
| GFPT2 | 0.854 | 9.031E-27 | 0.572 | 0.530 | 0.611 |
| GJB2 | 0.832 | 1.666E-24 | 0.553 | 0.511 | 0.591 |
| IGFL2 | 0.773 | 2.288E-16 | 0.536 | 0.485 | 0.571 |

(continued)

| GeneID | AUC | ANOVA (p value) | C-Index | LCI C-Index | UCI C-Index |
|---|---|---|---|---|---|
| INHBA | 0.825 | 4.651E-22 | 0.558 | 0.518 | 0.594 |
| ITGA5 | 0.807 | 2.079E-19 | 0.542 | 0.496 | 0.577 |
| KIF26B | 0.776 | 5.854E-15 | 0.552 | 0.511 | 0.591 |
| LOXL1 | 0.796 | 1.650E-17 | 0.550 | 0.508 | 0.590 |
| LUM | 0.823 | 1.073E-21 | 0.559 | 0.516 | 0.593 |
| MIR1245 | 0.825 | 3.785E-23 | 0.581 | 0.537 | 0.620 |
| MMP13 | 0.755 | 2.508E-14 | 0.566 | 0.523 | 0.603 |
| MMP14 | 0.815 | 1.208E-21 | 0.563 | 0.520 | 0.600 |
| MMP2 | 0.817 | 3.637E-23 | 0.553 | 0.512 | 0.590 |
| MRVI1 | 0.778 | 4.541E-17 | 0.529 | 0.486 | 0.569 |
| NKD2 | 0.802 | 1.892E-18 | 0.564 | 0.524 | 0.603 |
| NTM | 0.766 | 2.036E-15 | 0.563 | 0.524 | 0.598 |
| PLAU | 0.812 | 1.956E-20 | 0.558 | 0.512 | 0.597 |
| PMP22 | 0.808 | 1.183E-19 | 0.544 | 0.496 | 0.583 |
| POLD2 | 0.786 | 1.547E-16 | 0.589 | 0.545 | 0.626 |
| POSTN | 0.777 | 1.438E-17 | 0.569 | 0.527 | 0.602 |
| RAB31 | 0.815 | 1.676E-22 | 0.542 | 0.502 | 0.579 |
| RUNX2 | 0.784 | 1.463E-15 | 0.545 | 0.502 | 0.585 |
| SERPINF1 | 0.840 | 3.343E-24 | 0.563 | 0.522 | 0.599 |
| SFRP2 | 0.776 | 1.143E-17 | 0.569 | 0.527 | 0.605 |
| THBS2 | 0.840 | 1.511E-25 | 0.559 | 0.515 | 0.598 |
| TIMP3 | 0.787 | 6.196E-17 | 0.547 | 0.508 | 0.585 |
| TMEM200A | 0.842 | 4.891E-26 | 0.540 | 0.496 | 0.580 |
| TNFAIP6 | 0.795 | 1.600E-17 | 0.536 | 0.492 | 0.576 |
| VCAN | 0.845 | 9.655E-24 | 0.575 | 0.534 | 0.608 |
| VGLL3 | 0.800 | 5.200E-19 | 0.561 | 0.521 | 0.597 |

**Table 14: Table to represent data in Figure 26 - Core set analysis:**

| Tothill_HR_Final_Core Set Analysis-45 G ene | | |
|---|---|---|
| **Gene** | **Total Delta HR** | **Rank** |
| **MMP13** | 0.251541347 | 1 |
| **TNFAIP6** | 0.152036533 | 2 |
| **COL3A1** | 0.121550645 | 3 |
| **TIMP3** | 0.11735755 | 4 |
| **NKD2** | 0.114643471 | 5 |
| **KIF26B** | 0.10080423 | 6 |
| **FAP** | 0.096789296 | 7 |

(continued)

| Tothill_HR_Final_Core Set Analysis-45 G ene | | |
|---|---|---|
| **Gene** | **Total Delta HR** | **Rank** |
| **VGLL3** | 0.095605873 | 8 |
| **MMP14** | 0.095542494 | 9 |
| **POLD2** | 0.092045607 | 10 |
| **ALPK2** | 0.07567092 | 11 |
| **RUNX2** | 0.066961369 | 12 |
| **GFPT2** | 0.057289863 | 13 |
| **CTSK** | 0.048821463 | 14 |
| **NTM** | 0.045878012 | 15 |
| **ITGA5** | 0.04175161 | 16 |
| **FZD1** | 0.019664178 | 17 |
| **COPZ2** | 0.019354845 | 18 |
| **ANGPTL2** | 0.014075269 | 19 |
| **LUM** | 0.005097026 | 20 |
| **PMP22** | 0.003327351 | 21 |
| **PLAU** | -0.010756737 | 22 |
| **COL8A1** | -0.014008548 | 23 |
| **COL1A2** | -0.015474608 | 24 |
| **POSTN** | -0.02055353 | 25 |
| **CDH11** | -0.023093817 | 26 |
| **MIR1245** | -0.031717475 | 27 |
| **BICC1** | -0.03660296 | 28 |
| **IGFL2** | -0.04026505 | 29 |
| **MRVI1** | -0.040646101 | 30 |
| **LOXL1** | -0.044712308 | 31 |
| **COL11A1** | -0.051616035 | 32 |
| **RAB31** | -0.06464052 | 33 |
| **VCAN** | -0.071803963 | 34 |
| **BGN** | -0.076841005 | 35 |
| **MMP2** | -0.077505975 | 36 |
| **TMEM200A** | -0.081880796 | 37 |
| **SERPINF1** | -0.083927136 | 38 |
| **FN1** | -0.086805141 | 39 |
| **COL5A2** | -0.091752842 | 40 |
| **THBS2** | -0.115003748 | 41 |
| **INHBA** | -0.122749243 | 42 |
| **COL10A1** | -0.122975998 | 43 |
| **GJB2** | -0.147746426 | 44 |

(continued)

| Tothill_HR_Final_Core Set Analysis-45 G ene | | |
|---|---|---|
| Gene | Total Delta HR | Rank |
| SFRP2 | -0.170612716 | 45 |

Table 15: Table to represent data in Figure 27 - Core set analysis:

| ICON7_HR_Final_Core Set Analysis_45 Ge ne | | |
|---|---|---|
| Gene | Total Delta HR | Rank |
| COL11A1 | 0.230614266 | 1 |
| TNFAIP6 | 0.126558604 | 2 |
| COL8A1 | 0.113348626 | 3 |
| COL3A1 | 0.0985761 | 4 |
| THBS2 | 0.0869707 | 5 |
| POSTN | 0.082212851 | 6 |
| LUM | 0.081836943 | 7 |
| TMEM200A | 0.076763765 | 8 |
| NTM | 0.073348682 | 9 |
| ALPK2 | 0.069008899 | 10 |
| FAP | 0.063224645 | 11 |
| LOXL1 | 0.062094112 | 12 |
| GJB2 | 0.061294927 | 13 |
| RAB31 | 0.05995635 | 14 |
| INHBA | 0.056178828 | 15 |
| IGFL2 | 0.051228443 | 16 |
| TIMP3 | 0.033369809 | 17 |
| SERPINF1 | 0.024090101 | 18 |
| KIF26B | 0.0215188 | 19 |
| COPZ2 | 0.01714854 | 20 |
| POLD2 | 0.01003965 | 21 |
| BGN | 0.008585452 | 22 |
| NKD2 | 0.003216793 | 23 |
| MMP2 | -0.001775152 | 24 |
| FN1 | -0.00548725 | 25 |
| PLAU | -0.006301357 | 26 |
| ANGPTL2 | -0.006314736 | 27 |
| COL1A2 | -0.006622043 | 28 |
| RUNX2 | -0.015890395 | 29 |
| FZD1 | -0.027629684 | 30 |
| SFRP2 | -0.02829194 | 31 |

(continued)

| ICON7_HR_Final_Core Set Analysis_45 Ge ne | | |
|---|---|---|
| **Gene** | **Total Delta HR** | **Rank** |
| **MMP14** | -0.038025373 | 32 |
| **MRVI1** | -0.038171694 | 33 |
| **PMP22** | -0.04476172 | 34 |
| **COL10A1** | -0.047438573 | 35 |
| **COL5A2** | -0.053611989 | 36 |
| **BICC1** | -0.079674476 | 37 |
| **GFPT2** | -0.091136276 | 38 |
| **MIR1245** | -0.110650653 | 39 |
| **ITGA5** | -0.116066589 | 40 |
| **MMP13** | -0.119153053 | 41 |
| **VCAN** | -0.127296023 | 42 |
| **CDH11** | -0.133504128 | 43 |
| **VGLL3** | -0.191412013 | 44 |
| **CTSK** | -0.207991236 | 45 |

**Table 16: FKBP-L peptides**

| SEQUENCE | SEQ ID NO: |
|---|---|
| METPPVNTIGEKDTSQPQQEWEKNLRENLDSVIQIRQQPRDPPTETLELEVSPDPASQILEHTQGAEKLV<br>AELEGDSHKSHGSTSQMPEALQASDLWYCPDGSFVKKIVIRGHGLDKPKLGSCCRVLALGFPFGSGPPEG<br>WTELTMGVGPWREETWGELIEKCLESMCQGEEAELQLPGHSGPPVRLTLASFTQGRDSWELETSEKEALA<br>REERARGTELFRAGNPEGAARCYGRALRLLLTLPPPGPPERTVLHANLAACQLLLGQPQLAAQSCDRVLE<br>REPGHLKALYRRGVAQAALGNLEKATADLKKVLAIDPKNRAAQEELGKVVIQGKNQDAGLAQGLRKMFG | 789 |
| METPPVNTIGEKDTSQPQQEWEKNLRENLDSVIQIRQQPRDPPTETLELEVSPDPASQILEHTQGAEKLV<br>AELEGDSHKSHGSTSQMPEALQASDLWYCPDGSFVKKIVIRGHGLDKPKLGSCCRVLALGFPFGSGPPEG<br>WTELTMGVGPWREETWGELIEKCLESMCQGEEAELQLPGHTGPPVGLTLASFTQGRDSWELETSEKEALA<br>REERARGTELFRAGNPEGAARCYGRALRLLLTLPPPGPPERTVLHANLAACQLLLGQPQLAAQSCDRVLE<br>REPGHLKALYRRGVAQAALGNLEKATADLKKVLAIDPKNRAAQEELGKVVIQGKNQDAGLAQGLRKMFG | 790 |
| IRQQPRDPPTETLELEVSPDPAS (referred to herein as ALM201) | 791 |
| QIRQQPRDPPTETLELEVSPDPAS | 792 |
| METPPVNTIGEKDTSQPQQEWEKNLRENLDSVIQIRQQPRDPPTETLELEVSPDPASQILEHTQGAEKLV<br>AELEGDSHKSHGSTSQMPEALQASDLWYCPDGSFVKKIVIRGHGLDKPKLGSCCRVLALGFPFGSGPPEG<br>WTELTMGVGPWREETWGELIEKCLESMCQGEEAELQLPGHTGPPVGLTLASFTQGRDSW | 793 |
| METPPVNTIGEKDTSQPQQEWEKNLRENLDSVIQIRQQPRDPPTETLELEVSPDPASQILEHTQGAEKLV<br>AELEGDSHKSHGSTSQMPEALQASDLWYCPDGSFVKKIVIRGHGLDKPKLGSCCRVLALGFPFGSGPPEG<br>WTELTMGVGP | 794 |
| METPPVNTIGEKDTSQPQQEWEKNLRENLDSVIQIRQQPRDPPTETLELEVSPDPASQILEHTQGAEKLV<br>AELEGDSHKSHGSTS | 795 |
| METPPVNTIGEKDTSQPQQEWEKNLRENLDSVIQIRQQPRDPPTETLELEVSPDPAS | 796 |
| METPPVNTIGEKDTSQPQQEWEKNLRENLDSVIQIRQQPRDPPTETL | 797 |
| QQPRDPPTETLELEVSPD | 798 |
| QIRQQPRDPPTETLELEVSPD | 799 |
| QIRQQPRDPPTETLELEV | 800 |
| QIRQQPRDPPTETLE | 801 |

(continued)

| SEQUENCE | SEQ ID NO: |
|---|---|
| QIRQQPRDPPTE | 802 |
| QQPRDPPTETLELEVSPDPAS | 803 |
| RDPPTETLELEVSPDPAS | 804 |
| PTETLELEVSPDPAS | 805 |
| TLELEVSPDPAS | 806 |
| RQQPRDPPTETLELEVSPD | 807 |
| RQQPRDPPTETLELEVSP | 808 |
| RQQPRDPPTETLELEVS | 809 |
| PRDPPTETLELEVSPD | 810 |
| RDPPTETLELEVSPD | 811 |

**References**

Ahmed N, Abubaker K, Findlay J, Quinn M. Epithelial mesenchymal transition and cancer stem cell-like phenotypes facilitate chemoresistance in recurrent ovarian cancer. Curr Cancer Drug Targets. 2010 May;10(3):268-78. Review.

Ferlay, J. et al. GLOBOCAN 2012 v1.0, Cancer Incidence and Mortality Worldwide: IARC CancerBase. No. 11 [Internet]. Lyon, France: International Agency for Research on Cancer. 11, http://globocan.iarc.f (2013).

UK, C. R. Cancer Incidence for Common Cancers. Cancer Research UK 1 (2013). at <http://www.cancerresearchuk.org/cancer-info/cancerstats/incidence/commoncancers/uk-cancer-incidence-statistics-for-common-cancers>http://www.bbc.co.uk/news/uk-northernireland-33235308, http://www.ncri.ie/factsheets

Vaughan S, Coward JI, Bast RC Jr, Berchuck A, Berek JS, Brenton JD, Coukos G, Crum CC, Drapkin R, Etemadmoghadam D, Friedlander M, Gabra H, Kaye SB, Lord CJ, Lengyel E, Levine DA, McNeish IA, Menon U, Mills GB, Nephew KP, Oza AM, Sood AK, Stronach EA, Walczak H, Bowtell DD, Balkwill FR. Rethinking ovarian cancer: recommendations for improving outcomes. Nat Rev Cancer. 2011 Sep 23;11 (10):719-25.

McCluggage, W. G. My approach to and thoughts on the typing of ovarian carcinomas. J. Clin. Pathol. 61, 152-163 (2008).

Bowtell, D. D. The genesis and evolution of high-grade serous ovarian cancer. Nat Rev Cancer 10, 803-08, doi:10.1038/nrc2946 (2010).

Jose DG and De Kretser T. Oncogenes of human tumour cells. A review of advances in virology and molecular biology of cancer. Australas Radiol. 1984; 28(1): 43-50. PMID: 6089728.

Cohen Y, Xing M, Mambo E, et al. BRAF mutation in papillary thyroid carcinoma. J Natl Cancer Inst. 2003;95(8):625-627.

Davies H, Bignell GR, Cox C, et al. Mutations of the BRAF gene in human cancer. Nature. 2002;417(6892):949-954.

Xu X, Quiros RM, Gattuso P, et al. High prevalence of BRAF gene mutation in papillary thyroid carcinomas and thyroid tumor cell lines. Cancer Res. 2003; 63(15): 4561-4567. PMID: 12907632.

Hoshino R, Chatani Y, Yamori T,, et al. Constitutive activation of the 41-/43-kDa mitogen-activated protein kinase signaling pathway in human tumors. Oncogene, 1999; 18(3); 813-822. PMID: 9989833.

Marshall CJ. Cell signalling. Raf gets it together. Nature. 1996; 383(6596): 127-128.PMID: 8774875.

Shaul YD and Seger R. The MEK/ERK cascade: from signaling specificity to diverse functions. Biochim Biophys Acta. 2007; 1773(8): 1213-1226. PMID: 17112607. Epub 2006 Oct 19.

Lewis, TS, Shapiro PS, and Ahn NG. Signal transduction through MAP kinase cascades. Adv Cancer Res. 1998; 74: 49-139. PMID: 9561267.

Alberola-Ila J, Forbush KA, Seger R, et al. Selective requirement for MAP kinase activation in thymocyte differentiation. Nature. 1995; 373(6515): 620-623. PMID: 7854419

Johnson GL and Vaillancourt RR. Sequential protein kinase reactions controlling cell growth and differentiation. Curr Opin Cell Biol. 1994; 6(2): 230-238. PMID: 8024815.

D'Angelo G, Struman I, Martial J, et al. Activation of mitogen-activated protein kinases by vascular endothelial growth factor and basic fibroblast growth factor in capillary endothelial cells is inhibited by the antiangiogenic factor 16-kDa N-terminal fragment of prolactin. Proc Natl Acad Sci USA. 1995; 92(14): p. 6374-6378. PMID: 7541539.

Na J, Furue MK, and Andrews PW. Inhibition of ERK1/2 prevents neural and mesendodermal differentiation and promotes human embryonic stem cell self-renewal. Stem Cell Res. 2010; 5(2): 157-169. PMID: 20675210. Epub 2010 Aug 3.

TCGA. Integrated genomic analyses of ovarian carcinoma. Nature 2011;474:609-15.

J. Lopez, S. Banerjee and S. B. Kaye. New developments in the treatment of ovarian cancerfuture perspectives. Ann Oncol. 2013 Dec; 24(Suppl 10): x69-x76.

Schwarz RF, Ng CK, Cooke SL, Newman S, Temple J, Piskorz AM, Gale D, Sayal K, Murtaza M, Baldwin PJ, Rosenfeld N, Earl HM, Sala E, Jimenez-Linan M, Parkinson CA, Markowetz F, Brenton JD. Spatial and temporal heterogeneity in high-grade serous ovarian cancer: a phylogenetic analysis. PLoS Med. 2015 Feb 24;12(2):e1001789.

Patch AM, Christie EL, Etemadmoghadam D, Garsed DW, George J, Fereday S, Nones K, Cowin P, Alsop K, Bailey PJ, Kassahn KS, Newell F, Quinn MC, Kazakoff S, Quek K, Wilhelm-Benartzi C, Curry E, Leong HS; Australian Ovarian Cancer Study Group, Hamilton A, Mileshkin L, Au-Yeung G, Kennedy C, Hung J, Chiew YE, Harnett P, Friedlander M, Quinn M, Pyman J, Cordner S, O'Brien P, Leditschke J, Young G, Strachan K, Waring P, Azar W, Mitchell C, Traficante N, Hendley J, Thorne H, Shackleton M, Miller DK, Arnau GM, Tothill RW, Holloway TP, Semple T, Harliwong I, Nourse C, Nourbakhsh E, Manning S, Idrisoglu S, Bruxner TJ, Christ AN, Poudel B, Holmes O, Anderson M, Leonard C, Lonie A, Hall N, Wood S, Taylor DF, Xu Q, Fink JL, Waddell N, Drapkin R, Stronach E, Gabra H, Brown R, Jewell A, Nagaraj SH, Markham E, Wilson PJ, Ellul J, McNally O, Doyle MA, Vedururu R, Stewart C, Lengyel E, Pearson JV, Waddell N, deFazio A, Grimmond SM, Bowtell DD. Whole-genome characterization of chemoresistant ovarian cancer. Nature. 2015 May 28;521 (7553):489-94.

Eleveld TF, Oldridge DA, Bernard V, Koster J, Daage LC, Diskin SJ, Schild L, Bentahar NB, Bellini A, Chicard M, Lapouble E, Combaret V, Legoix-Né P, Michon J, Pugh TJ, Hart LS, Rader J, Attiyeh EF, Wei JS, Zhang S, Naranjo A, Gastier-Foster JM, Hogarty MD, Asgharzadeh S, Smith MA, Auvil JM, Watkins TB, Zwijnenburg DA, Ebus ME, van Sluis P, Hakkert A, van Wezel E, van der Schoot CE, Westerhout EM, Schulte JH, Tytgat GA, Dolman ME, Janoueix-Lerosey I, Gerhard DS, Caron HN, Delattre O, Khan J, Versteeg R, Schleiermacher G, Molenaar JJ, Maris JM. Relapsed neuroblastomas show frequent RAS-MAPK pathway mutations. Nat Genet. 2015 Jun 29. doi: 10.1038/ng.3333.

Li Ren Kong, Kian Ngiap Chua, Wen Jing Sim, Hsien Chun Ng, Chonglei Bi, Jingshan Ho, Min En Nga, Yin Huei Pang, Weijie Richard Ong, Ross Andrew Soo, Hung Huynh, Wee Joo Chng, Jean-Paul Thiery, and Boon Cher Goh MEK Inhibition Overcomes Cisplatin Resistance Conferred by SOS/MAPK Pathway Activation in Squamous Cell Carcinoma.. Molecular Cancer Therapeutics 2015.

Benedetti V, Perego P, Luca Beretta G, Corna E, Tinelli S, Righetti SC, Leone R, Apostoli P, Lanzi C, Zunino F. Modulation of survival pathways in ovarian carcinoma cell lines resistant to platinum compounds. Mol Cancer Ther. 2008 Mar;7(3):679-87.

Lord CJ, Ashworth A. Mechanisms of resistance to therapies targeting BRCA-mutant cancers. Nat Med. 2013 Nov;19(11):1381-8. doi: 10.1038/nm.3369. Epub 2013 Oct 7.

Siddiqui and Rimm . Pre-analytic variables and phospho-specific antibodies: the Achilles heel of immunohistochemistry. Breast Cancer Research 2010, 12:113

Pinhel IF, MacNeill FA, Hills MJ, Salter J, Detre S, A'Hern R, Nerurkar A, Osin P, Smith IE, Dowsett M. Extreme loss of immunoreactive p-Akt and p-Erk1/2 during routine fixation of primary breast cancer. Breast Cancer Res 2010, 12:R76.

Grossi V, Peserico A, Tezil T, Simone C. p38$\alpha$ MAPK pathway: a key factor in colorectal cancer therapy and chemoresistance. World J Gastroenterol. 2014 Aug 7;20(29):9744-58. doi: 10.3748/wjg.v20.i29.9744.

Kennedy RD, Bylesjo M, Kerr P, et al. Development and independent validation of a prognostic assay for stage II colon cancer using formalin-fixed paraffin-embedded tissue. J Clin Oncol 2011; 29:4620-6.

Tanney A, Oliver GR, Farztdinov V, et al. Generation of a non-small cell lung cancer transcriptome microarray. BMC medical genomics 2008; 1:20.

de Jong, S. SIMPLS: an alternative approach to partial least squares regression Chemometrics IntellLab Syst, 18, 251-263 (1993)

Irizarry RA, Bolstad BM, Collin F, Cope LM, Hobbs B, Speed TP. Summaries of Affymetrix GeneChip probe level data. Nucleic acids research 2003;31:e15.

Tibshirani R, Walther G, Hastie T: Estimating the number of clusters in a data set via the gap statistic. Journal of the Royal Statistical Society Series B-Statistical Methodology 63:411-423, 2001.

Ward JH. Hierarchical Grouping to Optimize an Objective Function. Journal of the American Statistical Association 1963;58:236-244.

Pinto FR, Melo-Cristino J, Ramirez M. A Confidence Interval for the Wallace Coefficient of Concordance and Its Application to Microbial Typing Methods. PLoS One 2008;3.

Carrico JA, Silva-Costa C, Melo-Cristino J, et al. Illustration of a common framework for relating multiple typing methods by application to macrolide-resistant Streptococcus pyogenes. J Clin Microbiol 2006;44:2524-32.

Marisa L, de Reyniès A, Duval A, Selves J, Gaub MP, Vescovo L, Etienne-Grimaldi MC, Schiappa R, Guenot D, Ayadi M, Kirzin S, Chazal M, Fléjou JF, Benchimol D, Berger A, Lagarde A, Pencreach E, Piard F, Elias D, Parc Y, Olschwang S, Milano G, Laurent-Puig P, Boige V. Gene expression classification of colon cancer into molecular subtypes: characterization, validation, and prognostic value. PLoS Med. 2013;10(5):e1001453. doi: 10.1371/journal.pmed.1001453. Epub 2013 May 21.

Jorissen RN, Gibbs P, Christie M, Prakash S, Lipton L, Desai J, Kerr D, Aaltonen LA, Arango D, Kruhøffer M, Orntoft TF, Andersen CL, Gruidl M, Kamath VP, Eschrich S, Yeatman TJ, Sieber OM. Metastasis-Associated Gene Expression Changes Predict Poor Outcomes in Patients with Dukes Stage B and C Colorectal Cancer. Clin Cancer Res. 2009 Dec 15;15(24):7642-7651.

Okayama H, Kohno T, Ishii Y, Shimada Y, Shiraishi K, Iwakawa R, Furuta K, Tsuta K, Shibata T, Yamamoto S, Watanabe S, Sakamoto H, Kumamoto K, Takenoshita S, Gotoh N, Mizuno H, Sarai A, Kawano S, Yamaguchi R, Miyano S, Yokota J. Identification of genes upregulated in ALK-positive and EGFR/KRAS/ALK-negative lung adenocarcinomas. Cancer Res. 2012 Jan 1;72(1):100-11. doi: 10.1158/0008-5472.CAN-11-1403. Epub 2011 Nov 11.

Virtakoivu R, Mai A, Mattila E, De Franceschi N, Imanishi SY, Corthals G, Kaukonen R, Saari M, Cheng F, Torvaldson E, Kosma VM, Mannermaa A, Muharram G, Gilles C, Eriksson J, Soini Y, Lorens JB, Ivaska J. Vimentin-ERK Signaling Uncouples Slug Gene Regulatory Function. Cancer Res. 2015 Jun 1;75(11):2349-62.

Haslehurst AM, Koti M, Dharsee M, Nuin P, Evans K, Geraci J, Childs T, Chen J, Li J, Weberpals J, Davey S, Squire J, Park PC, Feilotter H. EMT transcription factors snail and slug directly contribute to cisplatin resistance in ovarian cancer. BMC Cancer. 2012 Mar 19;12:91. doi: 10.1186/1471-2407-12-91.

Marchini S, Fruscio R, Clivio L, Beltrame L, Porcu L, Fuso Nerini I, Cavalieri D, Chiorino G, Cattoretti G, Mangioni C, Milani R, Torri V, Romualdi C, Zambelli A, Romano M, Signorelli M, di Giandomenico S, D'Incalci M. Resistance to platinum-based chemotherapy is associated with epithelial to mesenchymal transition in epithelial ovarian cancer. Eur J Cancer. 2013 Jan;49(2):520-30. doi: 10.1016/j.ejca.2012.06.026. Epub 2012 Aug 13.

## Claims

**1.** A method for selecting a treatment for a subject having a cancer, comprising:

(i) measuring the expression level(s) of at least 1 biomarker, wherein the at least one biomarker comprises GFPT2, in a sample from the subject;
(ii) assessing from the expression level(s) of the at least 1 biomarker whether the sample from the subject is positive or negative for a biomarker signature comprising the at least 1 biomarker, wherein:

(a) if the sample is positive for the biomarker signature a MAPK pathway inhibitor is indicated and/or if the sample is negative for the biomarker signature a MAPK pathway inhibitor is not indicated; and/or
(b) if the sample is positive for the biomarker signature an EMT pathway inhibitor is indicated and/or if the sample is negative for the biomarker signature an EMT pathway inhibitor is not indicated; and/or
(c) if the sample is positive for the biomarker signature an SRC pathway inhibitor is not indicated and/or if the sample is negative for the biomarker signature an SRC pathway inhibitor is indicated; and/or
(d) if the sample is positive for the biomarker signature a taxane is indicated and/or if the sample is negative for the biomarker signature a taxane is not indicated.

**2.** The method of claim 1(a) wherein the MAPK pathway inhibitor is combined with a platinum based chemotherapeutic agent and/or an SRC pathway inhibitor.

**3.** A method for predicting the responsiveness of a subject with cancer to a therapeutic agent comprising:

(i) measuring the expression level(s) of at least 1 biomarker(s), wherein the at least 1 biomarker comprises GFPT2, in a sample from the subject;
(ii) assessing from the expression level(s) of the at least 1 biomarker(s) whether the sample from the subject is positive or negative for a biomarker signature comprising the at least 1 biomarker;
(iii) classifying the subject as:

(a) predicted to be responsive to a MAPK pathway inhibitor if the sample is positive for the biomarker signature and/or predicted to be non-responsive to the MAPK pathway inhibitor if the sample is negative for the biomarker signature; and/or

(b) predicted to be responsive to an EMT pathway inhibitor if the sample is positive for the biomarker signature and/or predicted to be non-responsive to the EMT pathway inhibitor if the sample is negative for the biomarker signature; and/or

(c) predicted to be non-responsive to a SRC pathway inhibitor if the sample is positive for the biomarker signature and/or predicted to be responsive to the SRC inhibitor if the sample is negative for the biomarker signature; and/or

(d) predicted to be non-responsive to a platinum-based chemotherapeutic agent if the sample is positive for the biomarker signature and/or predicted to be responsive to a platinum-based chemotherapeutic agent if the sample is negative for the biomarker signature

(e) predicted to be responsive to a taxane if the sample is positive for the biomarker signature and/or predicted to be non-responsive to the taxane if the sample is negative for the biomarker signature.

**4.** A method of determining clinical prognosis of a subject with cancer comprising:

(i) measuring the expression level(s) of at least 1 biomarker(s), wherein the at least 1 biomarker comprises GFPT2, in a sample from the subject;

(ii) assessing from the expression level(s) of the at least 1 biomarker(s) whether the sample from the subject is positive or negative for a biomarker signature comprising the at least 1 biomarker;

(iii) classifying the subject as having a poor prognosis if the sample is positive for the biomarker signature and/or having a good prognosis if the sample is negative for the biomarker signature,

wherein the cancer is prostate cancer, colon cancer, bladder cancer, cervical cancer, glioblastoma, head and neck cancer, glioma, pancreatic cancer, melanoma, stomach cancer or lung cancer; and wherein the biomarker signature identifies the cancer as being in a molecular subgroup of cancer **characterized by** misregulation of the MAPK signaling pathway and the EMT pathway;

optionally wherein poor prognosis indicates decreased progression free survival and/or overall survival rates compared to samples that are negative for the biomarker signature and/or good prognosis indicates increased progression free survival and/or overall survival rates compared to samples that are positive for the biomarker signature.

**5.** The method of any previous claim wherein:

(a) assessing whether the sample is positive or negative for the biomarker signature comprises use of classification trees; and/or

(b) assessing whether the sample is positive or negative for the biomarker signature comprises:

(i) determining a sample expression score for the biomarker(s);

(ii) comparing the sample expression score to a threshold score; and

(iii) determining whether the sample expression score is above or equal to or below the threshold expression score,

wherein if the sample expression score is above or equal to the threshold expression score the sample is positive for the biomarker signature and/or if the sample expression score is below the threshold score the sample is negative for the biomarker signature; and/or

(c) an increased expression level and/or a decreased expression level of the biomarker(s) as shown in Tables C and D contributes to the determination that the sample is positive or negative for the biomarker signature.

**6.** A therapeutic agent for use in treating cancer in a subject, wherein

(a) if a sample from the subject is positive for a biomarker signature comprising the expression level(s) of at least 1 biomarker(s), wherein the at least 1 biomarker comprises GFPT2, the therapeutic agent is a MAPK pathway inhibitor; and/or

(b) if a sample from the subject is positive for the biomarker signature comprising the expression level(s) of at least 1 biomarker(s), wherein the at least 1 biomarker comprises GFPT2, the therapeutic agent is an EMT pathway inhibitor; and/or

(c) if a sample from the subject is negative for the biomarker signature comprising the expression level(s) of at least 1 biomarker(s), wherein the at least 1 biomarker comprises GFPT2, the therapeutic agent is an SRC pathway inhibitor; and/or

(d) if a sample from the subject is negative for the biomarker signature comprising the expression level(s) of at least 1 biomarker(s), wherein the at least 1 biomarker comprises GFPT2, the therapeutic agent is a platinum-based chemotherapeutic agent; and/or

(e) if a sample from the subject is positive for a biomarker signature comprising the expression level(s) of at least 1 biomarker(s), wherein the at least 1 biomarker comprises GFPT2, the therapeutic agent is a taxane.

**7.** The therapeutic agent for use of claim 6 wherein the subject is selected for treatment by:

(i) measuring the expression level(s) of at least 1 biomarker(s), wherein the at least 1 biomarker comprises GFPT2, in a sample from the subject;

(ii) assessing from the expression level(s) of the at least 1 biomarker(s) whether the sample from the subject is positive or negative for the biomarker signature comprising the at least 1 biomarker.

**8.** The therapeutic agent for use of claim 6, wherein the subject is selected for treatment on the basis of a method as claimed in any previous claim.

**9.** The method of any of claims 1-5, or therapeutic agent for use of any of claims 6-8 wherein:

(a) the MAPK pathway inhibitor is selected from Table G and/or H, the EMT pathway inhibitor is selected from Table I and/or is FKBP-L polypeptide or a biologically active peptide fragment thereof, preferably ALM201, the SRC pathway inhibitor is selected from Table J and/or the taxane is Paclitaxel and/or Docetaxel and/or

(b) the MAPK pathway inhibitor is a MEK inhibitor, optionally trametinib and/or selumetinib; and/or

(c) the SRC pathway inhibitor is not Dasatinib; and/or

(d) the platinum-based chemotherapeutic agent is cisplatin;

(e) the therapeutic agent is a MAPK pathway inhibitor combined with a platinum-based chemotherapeutic agent.

**10.** The method or therapeutic agent for use of any previous claim:

(a) wherein the cancer is prostate cancer, ovarian cancer, breast cancer, colon cancer and/or lung cancer, optionally wherein:

(i) the ovarian cancer is serous ovarian cancer, optionally high grade serous ovarian cancer, and/or

(ii) the lung cancer is non-small cell lung cancer; and/or

(b) comprising measuring the expression levels of one or more additional biomarkers from Table A and/or Table B; and/or

(c) comprising measuring the expression levels of each of the biomarkers listed in Table A and/or each of the biomarkers listed in Table B; and/or

(d) wherein the expression score is calculated using a weight value and/or a bias value for each biomarker, and wherein the weight value and the bias value are defined for each biomarker in Table A and/or Table B; and/or

(e) wherein the expression level is determined at the level of RNA, optionally wherein the expression level is determined by microarray, northern blotting, RNA-seq (RNA sequencing), in situ RNA detection or nucleic acid amplification; and/or

(f) wherein measuring the expression levels comprises contacting the sample with a set of nucleic acid probes or primers that bind to the biomarker and detecting binding of the set of nucleic acid probes or primers to the biomarker(s) by microarray, northern blotting, or nucleic acid amplification.

**11.** A system or test kit for selecting a treatment for a subject having a cancer, comprising:

(a) one or more testing devices for determining the expression level of at least 1 biomarker, wherein the at least 1 biomarker comprises GFPT2, in a sample from the subject

(b) a processor; and

(c) storage medium comprising a computer application that, when executed by the processor, is configured to:

(i) access and/or calculate the determined expression levels of the at least 1 biomarker in the sample on the one or more testing devices

(ii) calculate from the expression level(s) of the at least 1 biomarker whether the sample from the subject is positive or negative for a biomarker signature comprising the at least 1 biomarker; and
(iii) output from the processor the selected treatment, optionally wherein

(a) if the sample is positive for the biomarker signature a MAPK pathway inhibitor is selected and/or if the sample is negative for the biomarker signature a MAPK pathway inhibitor is not selected; and/or
(b) if the sample is positive for the biomarker signature an EMT pathway inhibitor is selected and/or if the sample is negative for the biomarker signature an EMT pathway inhibitor is not selected; and/or
(c) if the sample is positive for the biomarker signature an SRC pathway inhibitor is not selected and/or if the sample is negative for the biomarker signature an SRC pathway inhibitor is selected; and/or
(d) if the sample is positive for the biomarker signature a taxane is selected and/or if the sample is negative for the biomarker signature a taxane is not selected,

optionally wherein the system or test kit further comprises a display for the output from the processor.

**12.** A system or test kit for predicting the responsiveness of a subject with cancer to a therapeutic agent comprising:

(a) one or more testing devices for determining the expression level of at least 1 biomarker, wherein the at least 1 biomarker comprises GFPT2, in a sample from the subject
(b) a processor; and
(c) storage medium comprising a computer application that, when executed by the processor, is configured to:

(i) access and/or calculate the determined expression levels of the at least 1 biomarker in the sample on the one or more testing devices
(ii) calculate from the expression level(s) of the at least 1 biomarker whether the sample from the subject is positive or negative for a biomarker signature comprising the at least 1 biomarker; and
(iii) output from the processor the predicted responsiveness, optionally wherein the subject is classified as

(a) predicted to be responsive to a MAPK pathway inhibitor if the sample is positive for the biomarker signature and/or predicted to be non-responsive to the MAPK pathway inhibitor if the sample is negative for the biomarker signature; and/or
(b) predicted to be responsive to an EMT pathway inhibitor if the sample is positive for the biomarker signature and/or predicted to be non-responsive to the EMT pathway inhibitor if the sample is negative for the biomarker signature; and/or
(c) predicted to be non-responsive to a SRC pathway inhibitor if the sample is positive for the biomarker signature and/or predicted to be responsive to the SRC inhibitor if the sample is negative for the biomarker signature; and/or
(d) predicted to be non-responsive to a platinum-based chemotherapeutic agent if the sample is positive for the biomarker signature and/or predicted to be responsive to a platinum-based chemotherapeutic agent if the sample is negative for the biomarker signature
(e) predicted to be responsive to a taxane if the sample is positive for the biomarker signature and/or predicted to be non-responsive to the taxane if the sample is negative for the biomarker signature,

optionally wherein the system or test kit further comprises a display for the output from the processor.

**13.** A system or test kit for determining the clinical prognosis of a subject with cancer comprising:

a) one or more testing devices for determining the expression level of at least 1 biomarker, wherein the at least 1 biomarker comprises GFPT2, in a sample from the subject;
b) a processor; and
c) storage medium comprising a computer application that, when executed by the processor, is configured to:

(i) access and/or calculate the determined expression levels of the at least 1 biomarker in the sample on the one or more testing devices;
(ii) calculate from the expression level(s) of the at least 1 biomarker whether the sample from the subject is positive or negative for a biomarker signature comprising the at least 1 biomarker; and
(iii) output from the processor the prognosis for the subject, optionally wherein the subject is classified as having a poor prognosis if the sample is positive for the biomarker signature and/or having a good prognosis if the sample is negative for the biomarker signature,

wherein the cancer is prostate cancer, colon cancer, bladder cancer, cervical cancer, glioblastoma, head and neck cancer, glioma, pancreatic cancer, melanoma, stomach cancer or lung cancer; and wherein the biomarker signature identifies the cancer as being in a molecular subgroup of cancer **characterized by** misregulation of the MAPK signaling pathway and the EMT pathway;
optionally further comprising a display for the output from the processor.

**14.** A computer application or storage medium comprising a computer application as defined in any of claims 11-13.

**15.** A computer program product comprising a non-transitory computer-readable storage device having computer-readable program instructions embodied thereon that cause the computer to:

(i) access and/or calculate the determined expression levels of the at least 1 biomarker comprising GFPT2, in a sample on one or more testing devices;
(ii) calculate from the expression level(s) of the at least 1 biomarker whether the sample from the subject is positive or negative for a biomarker signature comprising the at least 1 biomarker in the sample; and,
(iii) provide an output, wherein the output is selection of a treatment as defined in claim 1, prediction of responsiveness to a therapeutic agent as defined in claim 3 or determination of clinical prognosis as defined in claim 4.

## Patentansprüche

**1.** Verfahren zur Auswahl einer Behandlung für ein Subjekt mit Krebs, das umfasst:

(i) Messen der(des) Expressionslevel(s) von mindestens 1 Biomarker, wobei der mindestens eine Biomarker GFPT2 umfasst, in einer Probe des Subjekts;
(ii) Beurteilen anhand der(des) Expressionslevel(s) des mindestens 1 Biomarkers, ob die Probe des Subjekts positiv oder negativ für eine Biomarker-Signatur ist, die den mindestens 1 Biomarker umfasst, wobei:

(a) wenn die Probe positiv für die Biomarker-Signatur ist, ein MAPK-Weg-Inhibitor angezeigt wird und/oder wenn die Probe negativ für die Biomarker-Signatur ist, ein MAPK-Weg-Inhibitor nicht angezeigt wird; und/oder
(b) wenn die Probe positiv für die Biomarker-Signatur ist, ein EMT-Weg-Inhibitor angezeigt wird und/oder wenn die Probe negativ für die Biomarker-Signatur ist, ein EMT-Weg-Inhibitor nicht angezeigt wird; und/oder
(c) wenn die Probe positiv für die Biomarker-Signatur ist, ein SRC-Weg-Inhibitor nicht angezeigt wird und/oder wenn die Probe für die Biomarker-Signatur negativ ist, ein SRC-Weg-Inhibitor angezeigt wird; und/oder
(d) wenn die Probe positiv für die Biomarker-Signatur ist, ein Taxan angezeigt wird und/oder wenn die Probe negativ für die Biomarker-Signatur ist, ein Taxan nicht angezeigt wird.

**2.** Verfahren nach Anspruch 1 (a), wobei der MAPK-Weg-Inhibitor mit einem platinbasierten chemotherapeutischen Wirkstoff und/oder einem SRC-Weg-Inhibitor kombiniert wird.

**3.** Verfahren zur Vorhersage, ob ein Subjekt mit Krebs auf einen therapeutischen Wirkstoff anspricht, das umfasst:

(i) Messen der(des) Expressionslevel(s) von mindestens 1 Biomarker(n), wobei der mindestens 1 Biomarker GFPT2 umfasst, in einer Probe des Subjekts;
(ii) Beurteilen anhand des/der Expressionslevel(s) der(des) mindestens 1 Biomarker(s), ob die Probe des Subjekts positiv oder negativ für eine Biomarker-Signatur ist, die den mindestens einen Biomarker umfasst;
(iii) Klassifizieren des Subjekts als:

(a) prognostiziert auf einen MAPK-Weg-Inhibitor anzusprechen, wenn die Probe positiv auf die Biomarker-Signatur ist und/oder prognostiziert nicht auf den MAPK-Weg-Inhibitor anzusprechen, wenn die Probe negativ auf die Biomarker-Signatur ist; und/oder
(b) prognostiziert auf einen EMT-Weg-Inhibitor anzusprechen, wenn die Probe positiv auf die Biomarker-Signatur ist und/oder prognostiziert nicht auf den EMT-Weg-Inhibitor anzusprechen, wenn die Probe negativ auf die Biomarker-Signatur ist; und/oder
(c) prognostiziert nicht auf einen SRC-Weg-Inhibitor anzusprechen, wenn die Probe positiv auf die Biomarker-Signatur ist und/oder prognostiziert auf den SRC-Weg-Inhibitor anzusprechen, wenn die Probe negativ auf die Biomarker-Signatur ist; und/oder

(d) prognostiziert nicht auf einen platinbasierten chemotherapeutischen Wirkstoff anzusprechen, wenn die Probe positiv auf die Biomarker-Signatur ist und/oder prognostiziert auf den platinbasierten chemotherapeutischen Wirkstoff anzusprechen, wenn die Probe negativ für die Biomarker-Signatur ist;

(e) prognostiziert auf ein Taxan anzusprechen, wenn die Probe positiv auf die Biomarker-Signatur ist und/oder prognostiziert nicht auf das Taxan anzusprechen, wenn die Probe negativ für die Biomarker-Signatur ist.

**4.** Verfahren zur Bestimmung der klinischen Prognose eines Subjekts mit Krebs, das umfasst:

(i) Messen der(des) Expressionslevel(s) von mindestens 1 Biomarker(n), wobei der mindestens 1 Biomarker GFPT2 umfasst, in einer Probe des Subjekts;

(ii) Beurteilen anhand der(des) Expressionslevel(s) der(des) mindestens 1 Biomarker(s), ob die Probe des Subjekts positiv oder negativ auf eine Biomarker-Signatur ist, die den mindestens einen Biomarker umfasst;

(iii) Klassifizieren des Subjekts als Person mit einer schlechten Prognose, wenn die Probe positiv auf die Biomarker-Signatur ist und/oder als Person mit einer guten Prognose, wenn die Probe negativ auf die Biomarker-Signatur ist,

wobei der Krebs Prostatakrebs, Dickdarmkrebs, Blasenkrebs, Gebärmutterhalskrebs, Glioblastom, Kopf- und Halskrebs, Gliom, Bauchspeicheldrüsenkrebs, Melanom, Magenkrebs oder Lungenkrebs ist; und wobei die Biomarker-Signatur den Krebs als zu einer molekularen Untergruppe von Krebs gehörend identifiziert, die durch eine Fehlregulierung des MAPK-Signalwegs und des EMT-Wegs charakterisiert ist;

wobei optional eine schlechte Prognose verringertes progressionsfreies Überleben und/oder Gesamtüberlebensraten im Vergleich zu Proben, die negativ auf die Biomarker-Signatur sind, anzeigt, und/oder eine gute Prognose erhöhtes progressionsfreies Überleben und/oder Gesamtüberlebensraten im Vergleich zu Proben, die positiv auf die Biomarker-Signatur sind, anzeigt.

**5.** Verfahren nach einem der vorhergehenden Ansprüche, wobei:

(a) das Bewerten, ob die Probe positiv oder negativ auf die Biomarker-Signatur ist, die Verwendung von Klassifikationsbäumen umfasst; und/oder

(b) das Bewerten, ob die Probe positiv oder negativ auf die Biomarker-Signatur ist, umfasst:

(i) Bestimmen eines Probenexpressionswertes für den(die) Biomarker;

(ii) Vergleichen des Probenexpressionswerts mit einem Schwellenwert; und

(iii) Feststellen, ob der Proben-Expressionswert über oder gleich oder unter dem Schwellen-Expressionswert ist,

wobei, wenn der Probenexpressionswert über oder gleich dem Schwellen-Expressionswert ist, die Probe positiv für die Biomarker-Signatur ist und/oder wenn der Proben-Expressionswert unter dem Schwellenwert ist, die Probe negativ für die Biomarker-Signatur ist; und/oder

(c) ein erhöhtes Expressionslevel und/oder ein verringertes Expressionslevel der(des) Biomarker(s) wie in den Tabellen C und D gezeigt, zur Feststellung beiträgt, dass die Probe positiv oder negativ auf die Biomarker-Signatur ist.

**6.** Therapeutisches Mittel zur Verwendung bei der Behandlung von Krebs bei einem Subjekt, wobei

(a) wenn eine Probe des Subjekts positiv auf eine Biomarker-Signatur ist, die das(die) Expressionslevel von mindestens 1 Biomarker(n) umfasst, wobei der mindestens 1 Biomarker GFPT2 umfasst, der therapeutische Wirkstoff ein MAPK-Weg-Inhibitor ist; und/oder

(b) wenn eine Probe des Subjekts positiv auf die Biomarker-Signatur ist, die das(die) Expressionslevel von mindestens 1 Biomarker(n) umfasst, wobei der mindestens 1 Biomarker GFPT2 umfasst, der therapeutische Wirkstoff ein EMT-Weg-Inhibitor ist; und/oder

(c) wenn eine Probe des Subjekts negativ auf die Biomarker-Signatur ist, die das(die) Expressionslevel von mindestens 1 Biomarker(n) umfasst, wobei der mindestens 1 Biomarker GFPT2 umfasst, der therapeutische Wirkstoff ein SRC-Weg-Inhibitor ist; und/oder

(d) wenn eine Probe des Subjekts negativ auf die Biomarker-Signatur ist, die das(die) Expressionslevel von mindestens 1 Biomarker(n) umfasst, wobei der mindestens 1 Biomarker GFPT2 umfasst, der therapeutische Wirkstoff ein platinbasierter chemotherapeutischer Wirkstoff ist; und/oder
(e) wenn eine Probe des Subjekts positiv auf die Biomarker-Signatur ist, die das(die) Expressionslevel von mindestens 1 Biomarker(n) umfasst, wobei der mindestens 1 Biomarker GFPT2 umfasst, der therapeutische Wirkstoff ein Taxan ist.

**7.** Therapeutisches Mittel zur Verwendung nach Anspruch 6, wobei das Subjekt ausgewählt wird für die Behandlung durch:

(i) Messen der(des) Expressionslevel(s) von mindestens 1 Biomarker(n), wobei der mindestens 1 Biomarker GFPT2 umfasst, in einer Probe des Subjekts;
(ii) Beurteilen anhand der(des) Expressionslevel(s) der(des) mindestens 1 Biomarker(s), ob die Probe des Subjekts positiv oder negativ für eine Biomarker-Signatur ist, die den mindestens einen Biomarker umfasst.

**8.** Therapeutisches Mittel zur Verwendung nach Anspruch 6, wobei das Subjekt für die Behandlung auf der Grundlage eines Verfahrens nach einem der vorhergehenden Ansprüche ausgewählt wird.

**9.** Verfahren nach einem der Ansprüche 1-5, oder therapeutisches Mittel zur Verwendung nach einem der Ansprüche 6-8, wobei:

(a) der MAPK-Weg-Inhibitor aus Tabelle G und/oder H ausgewählt ist, der EMT-Weg-Inhibitor aus Tabelle I ausgewählt ist und/oder FKBP-L-Polypeptid oder ein biologisch aktives Peptidfragment davon, bevorzugt ALM201, ist, der SRC-Weg-Inhibitor aus Tabelle J ausgewählt ist und/oder das Taxan Paclitaxel und/oder Docetaxel ist und/oder
(b) der MAPK-Weg-Inhibitor ein MEK-Inhibitor ist, optional Trametinib und/oder Selumetinib; und/oder
(c) der SRC-Weg-Inhibitor nicht Dasatinib ist; und/oder
(d) der platinbasierte chemotherapeutische Wirkstoff Cisplatin ist;
(e) der therapeutische Wirkstoff ein MAPK-Weg-Inhibitor in Kombination mit einem platinbasierten chemotherapeutischen Wirkstoff ist.

**10.** Verfahren oder therapeutischer Wirkstoff zur Verwendung nach einem der vorhergehenden Ansprüche:

(a) wobei der Krebs Prostatakrebs, Eierstockkrebs, Brustkrebs, Dickdarmkrebs und/oder Lungenkrebs ist, wobei optional:

(i) der Eierstockkrebs seröser Eierstockkrebs ist, optional hochgradig seröser Eierstockkrebs, und/oder
(ii) der Lungenkrebs nicht-kleinzelliger Lungenkrebs ist; und/oder

(b) umfassend das Messen der Expressionslevel eines oder mehrerer zusätzlicher Biomarker aus Tabelle A und/oder Tabelle B; und/oder
(c) umfassend das Messen der Expressionslevel jedes der in Tabelle A aufgeführten Biomarker und/oder jedes der in Tabelle B aufgeführten Biomarker; und/oder
(d) wobei der Expressionswert unter Verwendung eines Gewichtungswerts und/oder eines Verzerrungswerts für jeden Biomarker berechnet wird, und wobei der Gewichtungswert und der Verzerrungswert für jeden Biomarker in Tabelle A und/oder Tabelle B definiert sind; und/oder
(e) wobei das Expressionslevel auf dem Level von RNA bestimmt wird, wobei optional das Expressionslevel durch Microarray, Northern Blotting, RNAseq (RNA-Sequenzierung), In-situ-RNA-Detektion oder Nukleinsäureamplifikation bestimmt wird; und/oder
(f) wobei das Messen der Expressionslevel das Kontaktieren der Probe mit einem Satz von Nukleinsäuresonden oder Primern, die an den Biomarker binden, und das Detektieren des Bindens des Satzes von Nukleinsäuresonden oder Primern an den(die) Biomarker durch Microarray, Northern Blotting oder Nukleinsäureamplifikation umfasst.

**11.** System oder Testkit zur Auswahl einer Behandlung für ein Subjekt mit einem Krebs, das umfasst:

(a) eine oder mehrere Testvorrichtungen zur Bestimmung des Expressionslevels von mindestens 1 Biomarker, wobei der mindestens 1 Biomarker GFPT2 umfasst, in einer Probe des Subjekts
(b) einen Prozessor; und
(c) Speichermedium, das eine Computeranwendung umfasst, die, wenn sie vom Prozessor ausgeführt wird, so konfiguriert ist, dass sie:

(i) auf die ermittelten Expressionslevel des mindestens 1 Biomarkers in der Probe auf dem einen oder mehrere Testgeräte zugreift und/oder berechnet
(ii) aus dem(den) Expressionslevel(n) des mindestens 1 Biomarkers berechnet, ob die Probe des Subjekts positiv oder negativ für eine Biomarker-Signatur ist, die den mindestens 1 Biomarker umfasst; und
(iii) die gewählte Behandlung aus den Prozessor ausgibt, wobei optional

(a) wenn die Probe positiv für die Biomarker-Signatur ist, wird ein MAPK-Weg-Inhibitor ausgewählt und/oder wenn die Probe negativ für die Biomarker-Signatur ist, wird ein MAPK-Weg-Inhibitor nicht ausgewählt; und/oder
(b) wenn die Probe positiv für die Biomarker-Signatur ist, wird ein EMT-Weg-Inhibitor ausgewählt und/oder wenn die Probe negativ für die Biomarker-Signatur ist, wird ein EMT-Weg-Inhibitor nicht ausgewählt; und/oder
(c) wenn die Probe positiv für die Biomarker-Signatur ist, wird ein SRC-Signalweg-Inhibitor nicht ausgewählt und/oder wenn die Probe negativ für die Biomarker-Signatur ist, wird ein SRC-Weg-Inhibitor ausgewählt; und/oder
(d) wenn die Probe positiv für die Biomarker-Signatur ist, wird ein Taxan ausgewählt und/oder wenn die Probe negativ für die Biomarker-Signatur ist, wird Taxan nicht ausgewählt,

wobei optional das System oder das Testkit ferner eine Anzeige für die Ausgabe vom Prozessor umfasst.

**12.** System oder Testkit zur Vorhersage, ob ein Subjekt mit Krebs auf einen therapeutischen Wirkstoff anspricht, das umfasst:

(a) eine oder mehrere Testvorrichtungen zur Bestimmung des Expressionslevels von mindestens 1 Biomarker, wobei der mindestens 1 Biomarker GFPT2 umfasst, in einer Probe des Subjekts
(b) einen Prozessor; und
(c) Speichermedium, das eine Computeranwendung umfasst, die, wenn sie vom Prozessor ausgeführt wird, so konfiguriert ist, dass sie:

(i) auf die ermittelten Expressionslevel des mindestens 1 Biomarkers in der Probe auf dem einen oder mehrere Testgeräte zugreift und/oder berechnet
(ii) aus dem(den) Expressionslevel(n) des mindestens 1 Biomarkers berechnet, ob die Probe des Subjekts positiv oder negativ für eine Biomarker-Signatur ist, die den mindestens 1 Biomarker umfasst; und
(iii) die vorhergesagte Antwort aus dem Prozessor ausgibt, wobei optional das Subjekt klassifiziert wird als

(a) prognostiziert auf einen MAPK-Weg-Inhibitor anzusprechen, wenn die Probe positiv auf die Biomarker-Signatur ist und/oder prognostiziert nicht auf den MAPK-Weg-Inhibitor anzusprechen, wenn die Probe negativ auf die Biomarker-Signatur ist; und/oder
(b) prognostiziert auf einen EMT-Weg-Inhibitor anzusprechen, wenn die Probe positiv auf die Biomarker-Signatur ist und/oder prognostiziert nicht auf den EMT-Weg-Inhibitor anzusprechen, wenn die Probe negativ auf die Biomarker-Signatur ist; und/oder
(c) prognostiziert nicht auf einen SRC-Weg-Inhibitor anzusprechen, wenn die Probe positiv auf die Biomarker-Signatur ist und/oder prognostiziert auf den SRC-Weg-Inhibitor anzusprechen, wenn die Probe negativ auf die Biomarker-Signatur ist; und/oder
(d) prognostiziert nicht auf einen platinbasierten chemotherapeutischen Wirkstoff anzusprechen, wenn die Probe positiv auf die Biomarker-Signatur ist und/oder prognostiziert auf den platinbasierten chemotherapeutischen Wirkstoff anzusprechen, wenn die Probe negativ für die Biomarker-Signatur ist;
(e) prognostiziert auf ein Taxan anzusprechen, wenn die Probe positiv auf die Biomarker-Signatur ist und/oder prognostiziert nicht auf das Taxan anzusprechen, wenn die Probe negativ für die Biomarker-Signatur ist,

wobei optional das System oder das Testkit ferner eine Anzeige für die Ausgabe vom Prozessor umfasst.

**13.** System oder Testkit zur Bestimmung der klinischen Prognose eines Subjekts mit Krebs, das umfasst:

(a) eine oder mehrere Testvorrichtungen zur Bestimmung des Expressionslevels von mindestens 1 Biomarker, wobei der mindestens eine Biomarker GFPT2 umfasst, in einer Probe des Subjekts;
(b) einen Prozessor; und
(c) Speichermedium, das eine Computeranwendung umfasst, die, wenn sie vom Prozessor ausgeführt wird, so konfiguriert ist, dass sie:

(i) auf die ermittelten Expressionslevel des mindestens 1 Biomarkers in der Probe auf dem einen oder mehrere Testgeräte zugreift und/oder berechnet
(ii) aus dem(den) Expressionslevel(n) des mindestens 1 Biomarkers berechnet, ob die Probe des Subjekts positiv oder negativ für eine Biomarker-Signatur ist, die den mindestens 1 Biomarker umfasst; und
(iii) die Prognose des Subjekts aus den Prozessor ausgibt, wobei optional das Subjekt mit einer schlechten Prognose klassifiziert wird, wenn die Probe positiv für die Biomarker-Signatur ist, und/oder mit einer guten Prognose, wenn die Probe negativ für die Biomarker-Signatur ist,

wobei der Krebs Prostatakrebs, Dickdarmkrebs, Blasenkrebs, Gebärmutterhalskrebs, Glioblastom, Kopf- und Halskrebs, Gliom, Bauchspeicheldrüsenkrebs, Melanom, Magenkrebs oder Lungenkrebs ist; und wobei die Biomarker-Signatur den Krebs als zu einer molekularen Untergruppe von Krebs gehörend identifiziert, die durch eine Fehlregulierung des MAPK-Signalwegs und des EMT-Wegs charakterisiert ist;
optional ferner eine Anzeige für die Ausgabe vom Prozessor umfassend.

**14.** Computeranwendung oder Speichermedium, das eine Computeranwendung wie definiert in einem der Ansprüche 11-13 umfasst.

**15.** Computerprogrammprodukt, das eine nicht-transitorische, computerlesbare Speichervorrichtung mit darauf ausgeführten computerlesbaren Programmanweisungen umfasst, die den Computer dazu veranlassen:

(i) auf die ermittelten Expressionslevel des mindestens 1 Biomarkers, der GFPT2 umfasst, in der Probe auf einen oder mehrere Testgeräte zuzugreifen und/oder zu berechnen;
(ii) aus dem(den) Expressionslevel(n) des mindestens 1 Biomarkers zu berechnen, ob die Probe des Subjekts positiv oder negativ für eine Biomarker-Signatur ist, die den mindestens 1 Biomarker umfasst; und
(iii) eine Ausgabe bereitzustellen, wobei die Ausgabe die Auswahl einer Behandlung wie definiert in Anspruch 1, die Vorhersage des Ansprechens auf einen therapeutischen Wirkstoff wie definiert in Anspruch 3 oder die Bestimmung der klinischen Prognose wie definiert in Anspruch 4 ist.

## Revendications

**1.** Procédé de sélection d’un traitement pour un sujet souffrant d’un cancer, comprenant :

(i) la mesure du/des taux d’expression d’au moins 1 biomarqueur, dans lequel le au moins un biomarqueur comprend GFPT2, dans un échantillon du sujet ;
(ii) l’évaluation, à partir du/des taux d’expression du au moins 1 biomarqueur, du fait que l’échantillon du sujet est positif ou négatif pour une signature de biomarqueur comprenant le au moins 1 biomarqueur, dans laquelle :

(a) si l’échantillon est positif pour la signature de biomarqueur, un inhibiteur de la voie MAPK est indiqué et/ou si l’échantillon est négatif pour la signature de biomarqueur, un inhibiteur de la voie MAPK n’est pas indiqué ; et/ou
(b) si l’échantillon est positif pour la signature de biomarqueur, un inhibiteur de la voie EMT est indiqué et/ou si l’échantillon est négatif pour la signature de biomarqueur, un inhibiteur de la voie EMT n’est pas indiqué ; et/ou
(c) si l’échantillon est positif pour la signature de biomarqueur, un inhibiteur de la voie SRC n’est pas indiqué et/ou si l’échantillon est négatif pour la signature de biomarqueur, un inhibiteur de la voie SRC est indiqué ; et/ou
(d) si l’échantillon est positif pour la signature de biomarqueur, un taxane est indiqué et/ou si l’échantillon est négatif pour la signature de biomarqueur, un taxane n’est pas indiqué.

**2.** Procédé selon la revendication 1(a), dans lequel l’inhibiteur de la voie MAPK est combiné avec un agent de chimiothérapie à base de platine et/ou un inhibiteur de la voie SRC.

**3.** Procédé de prédiction de la réponse d'un sujet souffrant d'un cancer à un agent thérapeutique, comprenant :

(i) la mesure du/des taux d'expression d'au moins 1 biomarqueur, dans lequel le au moins 1 biomarqueur comprend GFPT2, dans un échantillon du sujet ;
(ii) l'évaluation, à partir du/des taux d'expression du au moins 1 biomarqueur, du fait que l'échantillon du sujet est positif ou négatif pour une signature de biomarqueur comprenant le au moins 1 biomarqueur ;
(iii) la classification du sujet comme étant :

(a) prédit pour répondre à un inhibiteur de la voie MAPK si l'échantillon est positif pour la signature de biomarqueur et/ou prédit pour ne pas répondre à l'inhibiteur de la voie MAPK si l'échantillon est négatif pour la signature de biomarqueur ; et/ou
(b) prédit pour répondre à un inhibiteur de la voie EMT si l'échantillon est positif pour la signature de biomarqueur et/ou prédit pour ne pas répondre à l'inhibiteur de la voie EMT si l'échantillon est négatif pour la signature de biomarqueur ; et/ou
(c) prédit pour ne pas répondre à un inhibiteur de la voie SRC si l'échantillon est positif pour la signature de biomarqueur et/ou prédit pour répondre à l'inhibiteur de SRC si l'échantillon est négatif pour la signature de biomarqueur ; et/ou
(d) prédit pour ne pas répondre à un agent de chimiothérapie à base de platine si l'échantillon est positif pour la signature de biomarqueur et/ou prédit pour répondre à un agent de chimiothérapie à base de platine si l'échantillon est négatif pour la signature de biomarqueur
(e) prédit pour répondre à un taxane si l'échantillon est positif pour la signature de biomarqueur et/ou prédit pour ne pas répondre au taxane si l'échantillon est négatif pour la signature de biomarqueur.

**4.** Procédé de détermination du pronostic clinique d'un sujet souffrant d'un cancer, comprenant :

(i) la mesure du/des taux d'expression d'au moins 1 biomarqueur, dans lequel le au moins 1 biomarqueur comprend GFPT2, dans un échantillon du sujet ;
(ii) l'évaluation, à partir du/des taux d'expression du au moins 1 biomarqueur, du fait que l'échantillon du sujet est positif ou négatif pour une signature de biomarqueur comprenant le au moins 1 biomarqueur ;
(iii) la classification du sujet comme présentant un mauvais pronostic si l'échantillon est positif pour la signature de biomarqueur et/ou comme présentant un bon pronostic si l'échantillon est négatif pour la signature de biomarqueur,

dans lequel le cancer est le cancer de la prostate, le cancer du côlon, le cancer de la vessie, le cancer du col de l'utérus, un glioblastome, le cancer de la tête et du cou, un gliome, le cancer du pancréas, un mélanome, le cancer de l'estomac ou le cancer du poumon ; et dans lequel la signature de biomarqueur identifie le cancer comme étant dans un sous-groupe moléculaire de cancer **caractérisé par** un dérèglement de la voie de signalisation MAPK et la voie EMT ;
facultativement dans lequel un mauvais pronostic indique une diminution des taux de survie sans progression et/ou de survie globale par comparaison à des échantillons qui sont négatifs pour la signature de biomarqueur et/ou un bon pronostic indique une augmentation des taux de survie sans progression et/ou de survie globale par comparaison à des échantillons qui sont positifs pour la signature de biomarqueur.

**5.** Procédé selon une quelconque revendication précédente, dans lequel :

(a) l'évaluation du fait que l'échantillon est positif ou négatif pour la signature de biomarqueur comprend l'utilisation d'arbres de classification ; et/ou
(b) l'évaluation du fait que l'échantillon est positif ou négatif pour la signature de biomarqueur comprend :

(i) la détermination d'un score d'expression de l'échantillon pour le(s) biomarqueur(s) ;
(ii) la comparaison du score d'expression de l'échantillon avec un score seuil ; et
(iii) la détermination du fait que le score d'expression de l'échantillon est supérieur ou égal ou inférieur au score d'expression de l'échantillon,
dans lequel si le score d'expression de l'échantillon est supérieur ou égal au score d'expression seuil, l'échantillon est positif pour la signature de biomarqueur et/ou si le score d'expression de l'échantillon est inférieur au score seuil, l'échantillon est négatif pour la signature de biomarqueur ; et/ou

(c) une augmentation du taux d'expression et/ou une réduction du taux d'expression du/des biomarqueur(s) tels que montrés dans les Tableaux C et D contribue à la détermination du fait que l'échantillon est positif ou négatif pour la signature de biomarqueur.

**6.** Agent thérapeutique destiné à être utilisé dans le traitement du cancer chez un sujet, dans lequel

(a) si un échantillon du sujet est positif pour une signature de biomarqueur comprenant le(s) taux d'expression du au moins 1 biomarqueur, dans lequel le au moins 1 biomarqueur comprend GFPT2, l'agent thérapeutique est un inhibiteur de la voie MAPK ; et/ou
(b) si un échantillon du sujet est positif pour la signature de biomarqueur comprenant le(s) taux d'expression du au moins 1 biomarqueur, dans lequel le au moins 1 biomarqueur comprend GFPT2, l'agent thérapeutique est un inhibiteur de la voie EMT ; et/ou
(c) si un échantillon du sujet est négatif pour la signature de biomarqueur comprenant le(s) taux d'expression du au moins 1 biomarqueur, dans lequel le au moins 1 biomarqueur comprend GFPT2, l'agent thérapeutique est un inhibiteur de la voie SRC ; et/ou
(d) si un échantillon du sujet est négatif pour la signature de biomarqueur comprenant le(s) taux d'expression du au moins 1 biomarqueur, dans lequel le au moins 1 biomarqueur comprend GFPT2, l'agent thérapeutique est un agent de chimiothérapie à base de platine ; et/ou
(e) si un échantillon du sujet est positif pour une signature de biomarqueur comprenant le(s) taux d'expression du au moins 1 biomarqueur, dans lequel le au moins 1 biomarqueur comprend GFPT2, l'agent thérapeutique est un taxane.

**7.** Agent thérapeutique destiné à être utilisé selon la revendication 6, dans lequel le sujet est sélectionné pour un traitement par :

(i) la mesure du/des taux d'expression du au moins 1 biomarqueur, dans lequel le au moins 1 biomarqueur comprend GFPT2, dans un échantillon du sujet ;
(ii) l'évaluation, à partir du/des taux d'expression du au moins 1 biomarqueur, du fait que l'échantillon du sujet est positif ou négatif pour la signature de biomarqueur comprenant le au moins 1 biomarqueur.

**8.** Agent thérapeutique destiné à être utilisé selon la revendication 6, dans lequel le sujet est sélectionné pour un traitement en se basant sur un procédé tel que revendiqué dans une quelconque revendication précédente.

**9.** Procédé selon l'une quelconque des revendications 1-5, ou agent thérapeutique destiné à être utilisé selon l'une quelconque des revendications 6-8, dans lequel :

(a) l'inhibiteur de la voie MAPK est sélectionné dans le Tableau G et/ou H, l'inhibiteur de la voie EMT est sélectionné dans le Tableau I et/ou est un polypeptide FKBP-L ou un fragment peptidique biologiquement actif de celui-ci, de préférence ALM201, l'inhibiteur de la voie SRC est sélectionné dans le Tableau J et/ou le taxane est le paclitaxel et/ou le docétaxel et/ou
(b) l'inhibiteur de la voie MAPK est un inhibiteur de MEK, facultativement le tramétinib et/ou le sélumétinib ; et/ou
(c) l'inhibiteur de la voie SRC n'est pas le dasatinib ; et/ou
(d) l'agent de chimiothérapie à base de platine est le cisplatine ;
(e) l'agent thérapeutique est un inhibiteur de la voie MAPK combiné avec un agent de chimiothérapie à base de platine.

**10.** Procédé ou agent thérapeutique destiné à être utilisé selon une quelconque revendication précédente :

(a) dans lequel le cancer est le cancer de la prostate, le cancer de l'ovaire, le cancer du sein, le cancer du côlon et/ou le cancer du poumon, facultativement dans lequel :

(i) le cancer de l'ovaire est le cancer de l'ovaire séreux, facultativement le cancer de l'ovaire séreux de haut grade, et/ou
(ii) le cancer du poumon est le cancer du poumon non à petites cellules ; et/ou

(b) comprenant la mesure des taux d'expression d'un ou de plusieurs biomarqueurs supplémentaires du Tableau A et/ou Tableau B ; et/ou
(c) comprenant la mesure des taux d'expression de chacun des biomarqueurs répertoriés dans le Tableau A et/ou de chacun des biomarqueurs répertoriés dans le Tableau B ; et/ou

(d) dans lequel le score d'expression est calculé en utilisant une valeur de poids et/ou une valeur de biais pour chaque biomarqueur, et dans lequel la valeur de poids et la valeur de biais sont définies pour chaque biomarqueur dans le Tableau A et/ou le Tableau B ; et/ou
(e) dans lequel le taux d'expression est déterminé au niveau de l'ARN, facultativement dans lequel le taux d'expression est mesuré par microréseau, transfert de Northern, ARN-seq (séquençage de l'ARN), détection d'ARN *in situ* ou amplification d'acide nucléique ; et/ou
(f) dans lequel la mesure des taux d'expression comprend la mise en contact de l'échantillon avec un jeu de sondes ou d'amorces d'acide nucléique qui se lient au biomarqueur et la détection de la liaison du jeu de sondes ou d'amorces d'acide nucléique au(x) biomarqueur(s) par microréseau, transfert de Northern, ou amplification d'acide nucléique.

**11.** Système ou kit de test pour sélectionner un traitement pour un sujet souffrant d'un cancer, comprenant :

(a) un ou plusieurs dispositifs de test pour déterminer le taux d'expression d'au moins 1 biomarqueur, dans lequel le au moins 1 biomarqueur comprend GFPT2, dans un échantillon du sujet
(b) un processeur ; et
(c) un support de stockage comprenant une application informatique qui, lorsqu'elle est exécutée par le processeur, est configurée pour :

(i) accéder aux et/ou calculer les taux d'expression déterminés du au moins 1 biomarqueur dans l'échantillon sur le un ou plusieurs dispositifs de test
(ii) calculer, à partir du/des taux d'expression du au moins 1 biomarqueur, si l'échantillon du sujet est positif ou négatif pour une signature de biomarqueur comprenant le au moins 1 biomarqueur ; et
(iii) générer des données, à partir du processeur, sur le traitement sélectionné,
facultativement dans lequel

(a) si l'échantillon est positif pour la signature de biomarqueur, un inhibiteur de la voie MAPK est sélectionné et/ou si l'échantillon est négatif pour la signature de biomarqueur, un inhibiteur de la voie MAPK n'est pas sélectionné ; et/ou
(b) si l'échantillon est positif pour la signature de biomarqueur, un inhibiteur de la voie EMT est sélectionné et/ou si l'échantillon est négatif pour la signature de biomarqueur, un inhibiteur de la voie EMT n'est pas sélectionné ; et/ou
(c) si l'échantillon est positif pour la signature de biomarqueur, un inhibiteur de la voie SRC n'est pas sélectionné et/ou si l'échantillon est négatif pour la signature de biomarqueur, un inhibiteur de la voie SRC est sélectionné ; et/ou
(d) si l'échantillon est positif pour la signature de biomarqueur, un taxane est sélectionné et/ou si l'échantillon est négatif pour la signature de biomarqueur, un taxane n'est pas sélectionné,
facultativement dans lequel le système ou le kit de test comprend en outre un affichage pour les données générées par le processeur.

**12.** Système ou kit de test pour prédire la réponse d'un sujet souffrant d'un cancer à un agent thérapeutique comprenant :

(a) un ou plusieurs dispositifs de test pour déterminer le taux d'expression d'au moins 1 biomarqueur, dans lequel le au moins 1 biomarqueur comprend GFPT2, dans un échantillon du sujet
(b) un processeur ; et
(c) un support de stockage comprenant une application informatique qui, lorsqu'elle est exécutée par le processeur, est configurée pour :

(i) accéder aux et/ou calculer les taux d'expression déterminés du au moins 1 biomarqueur dans l'échantillon sur le un ou plusieurs dispositifs de test
(ii) calculer, à partir du/des taux d'expression du au moins 1 biomarqueur, si l'échantillon du sujet est positif ou négatif pour une signature de biomarqueur comprenant le au moins 1 biomarqueur ; et
(iii) générer des données, à partir du processeur, sur la réponse prédite,
facultativement dans lequel le sujet est classé comme étant

(a) prédit pour répondre à un inhibiteur de la voie MAPK si l'échantillon est positif pour la signature de biomarqueur et/ou prédit pour ne pas répondre à l'inhibiteur de la voie MAPK si l'échantillon est négatif pour la signature de biomarqueur ; et/ou

(b) prédit pour répondre à un inhibiteur de la voie EMT si l'échantillon est positif pour la signature de biomarqueur et/ou prédit pour ne pas répondre à l'inhibiteur de la voie EMT si l'échantillon est négatif pour la signature de biomarqueur ; et/ou
(c) prédit pour ne pas répondre à un inhibiteur de la voie SRC si l'échantillon est positif pour la signature de biomarqueur et/ou prédit pour répondre à l'inhibiteur de SRC si l'échantillon est négatif pour la signature de biomarqueur ; et/ou
(d) prédit pour ne pas répondre à un agent de chimiothérapie à base de platine si l'échantillon est positif pour la signature de biomarqueur et/ou prédit pour répondre à un agent de chimiothérapie à base de platine si l'échantillon est négatif pour la signature de biomarqueur
(e) prédit pour répondre à un taxane si l'échantillon est positif pour la signature de biomarqueur et/ou prédit pour ne pas répondre au taxane si l'échantillon est négatif pour la signature de biomarqueur,
facultativement dans lequel le système ou le kit de test comprend en outre un affichage pour les données générées par le processeur.

**13.** Système ou kit de test pour déterminer le pronostic clinique d'un sujet souffrant d'un cancer, comprenant :

a) un ou plusieurs dispositifs de test pour déterminer le taux d'expression d'au moins 1 biomarqueur, dans lequel le au moins 1 biomarqueur comprend GFPT2, dans un échantillon du sujet ;
(b) un processeur ; et
(c) un support de stockage comprenant une application informatique qui, lorsqu'elle est exécutée par le processeur, est configurée pour :

(i) accéder aux et/ou calculer les taux d'expression déterminés du au moins 1 biomarqueur dans l'échantillon sur le un ou plusieurs dispositifs de test ;
(ii) calculer, à partir du/des taux d'expression du au moins 1 biomarqueur, si l'échantillon du sujet est positif ou négatif pour une signature de biomarqueur comprenant le au moins 1 biomarqueur ; et
(iii) générer des données, à partir du processeur, sur le pronostic du sujet, facultativement dans lequel le sujet est classé comme présentant un mauvais pronostic si l'échantillon est positif pour la signature de biomarqueur et/ou comme présentant un bon pronostic si l'échantillon est négatif pour la signature de biomarqueur,

dans lequel le cancer est le cancer de la prostate, le cancer du côlon, le cancer de la vessie, le cancer du col de l'utérus, un glioblastome, le cancer de la tête et du cou, un gliome, le cancer du pancréas, un mélanome, le cancer de l'estomac ou le cancer du poumon ; et dans lequel la signature de biomarqueur identifie le cancer comme étant dans un sous-groupe moléculaire de cancer **caractérisé par** un dérèglement de la voie de signalisation MAPK et la voie EMT ;
facultativement comprenant en outre un affichage pour les données générées par le processeur.

**14.** Application informatique ou support de stockage comprenant une application informatique telle que définie dans l'une quelconque des revendications 11 à 13.

**15.** Produit de programme informatique comprenant un dispositif de stockage non transitoire lisible par ordinateur sur lequel sont enregistrées des instructions de programme lisibles par un ordinateur qui permettent à l'ordinateur :

(i) d'accéder aux et/ou de calculer les taux d'expression déterminés du au moins 1 biomarqueur comprenant GFPT2, dans un échantillon sur un ou plusieurs dispositifs de test ;
(ii) de calculer, à partir du/des taux d'expression du au moins 1 biomarqueur, si l'échantillon du sujet est positif ou négatif pour une signature de biomarqueur comprenant le au moins 1 biomarqueur dans l'échantillon ; et,
(iii) de générer des données, dans lequel les données sont la sélection d'un traitement tel que défini dans la revendication 1, la prédiction d'une réponse à un agent thérapeutique tel que défini dans la revendication 3 ou la détermination d'un pronostic clinique tel que défini dans la revendication 4.

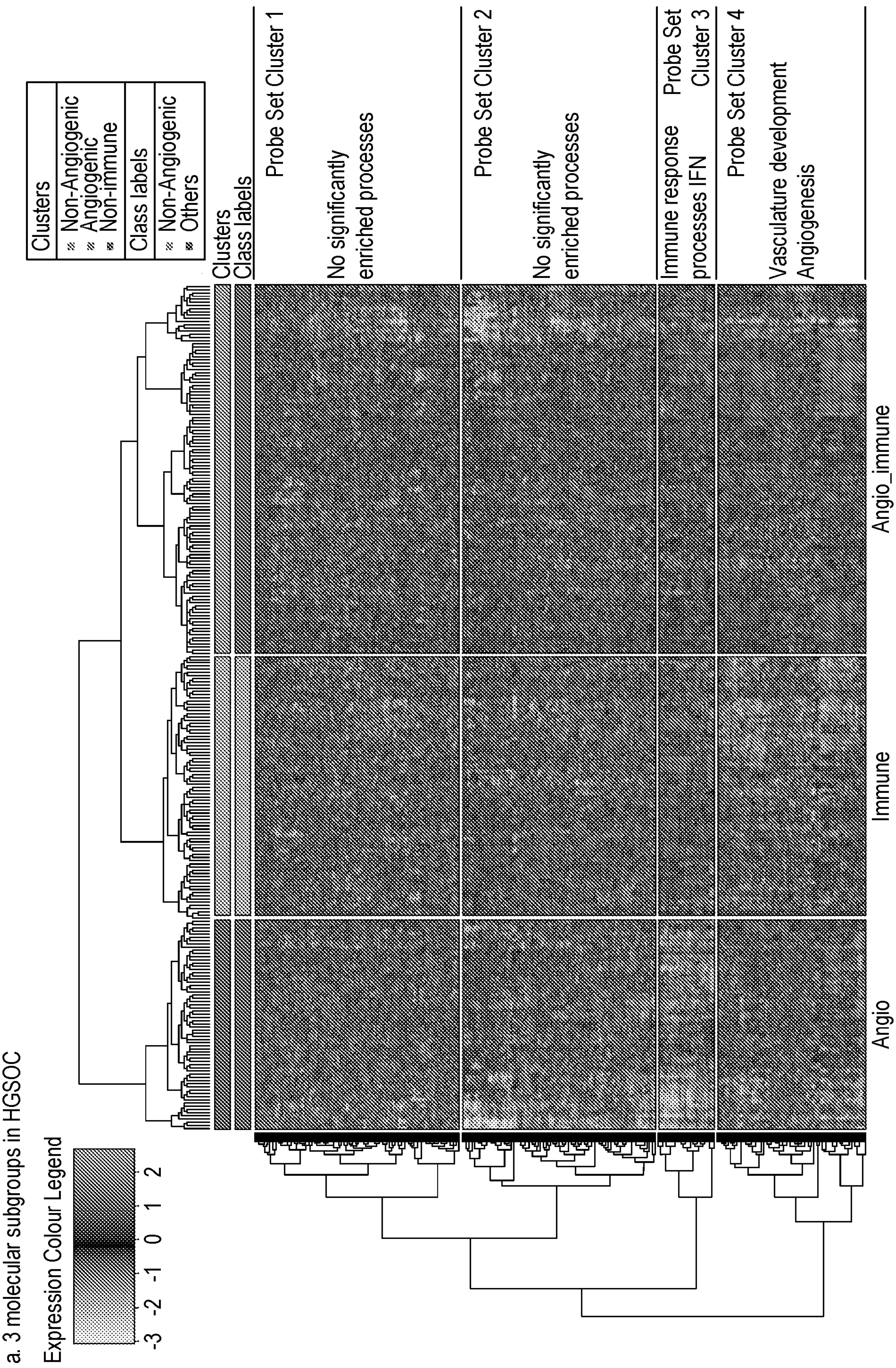
a. 3 molecular subgroups in HGSOC
Expression Colour Legend
-3 -2 -1 0 1 2
Clusters
Non-Angiogenic
Angiogenic
Non-immune
Class labels
Non-Angiogenic
Others
Clusters
Class labels
Probe Set Cluster 1
No significantly enriched processes
Probe Set Cluster 2
No significantly enriched processes
Immune response processes IFN
Probe Set Cluster 3
Probe Set Cluster 4
Vasculature development Angiogenesis
Angio
Immune
Angio_immune

FIGURE 1

b. Survival analysis of molecular subgroups

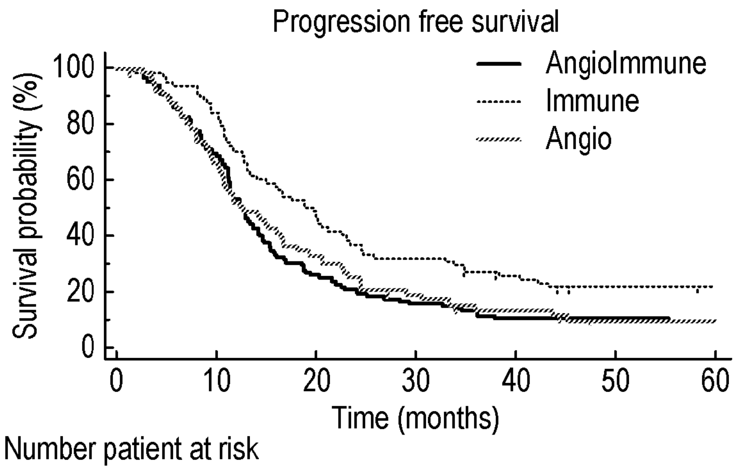


Number patient at risk

| | | | | | | | |
|---|---|---|---|---|---|---|---|
| AngioImmune | 116 | 79 | 30 | 18 | 12 | 11 | 10 |
| Immune | 83 | 70 | 38 | 26 | 19 | 14 | 13 |
| Angio | 66 | 42 | 21 | 11 | 7 | 4 | 4 |

PFS for Immune angiogenesis inactive group compared to:

AngioImmune Angiogenesis active subtype:
HR=0.60 (0.44-0.82)

Angio Angiogenesis active subtype:
HR=0.64 (0.49-0.92)

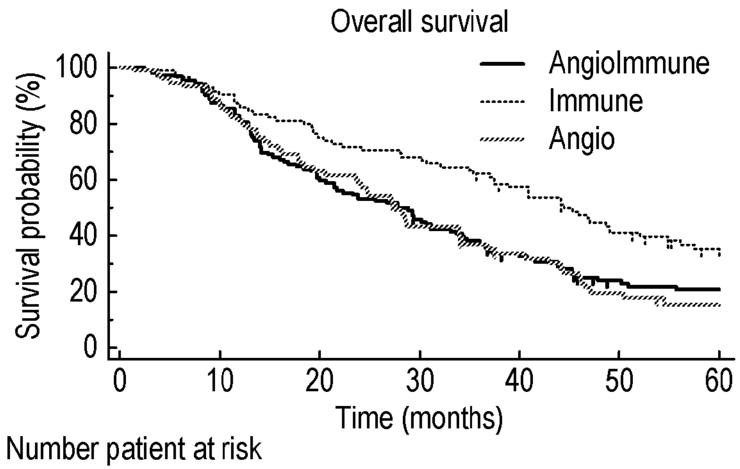


Number patient at risk

| | | | | | | | |
|---|---|---|---|---|---|---|---|
| AngioImmune | 116 | 100 | 69 | 51 | 34 | 20 | 17 |
| Immune | 83 | 75 | 62 | 56 | 45 | 31 | 22 |
| Angio | 66 | 56 | 41 | 27 | 19 | 10 | 7 |

OS for Immune angiogenesis inactive group compared to:

AngioImmune Angiogenesis active subtype:
HR=0.58 (0.41-0.82)

Angio Angiogenesis active subtype:
HR=0.55 (0.37-0.80)

**FIGURE 1 CONTINUED**

c. Prognostic significance of 45 gene signature

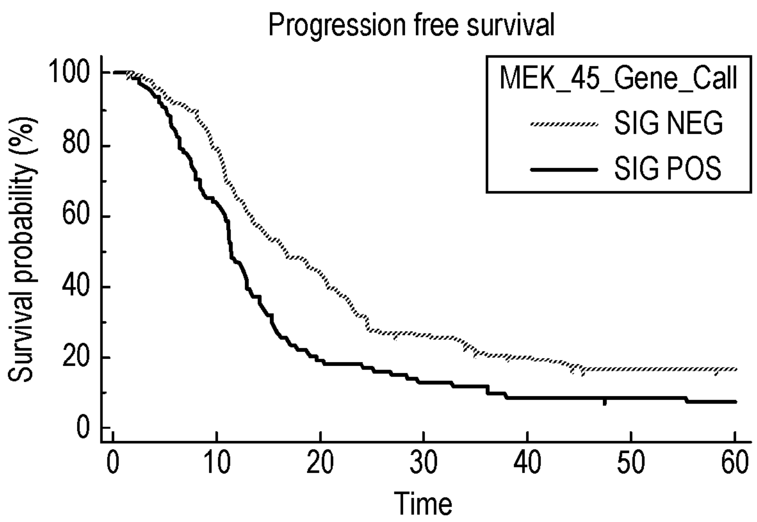


Number patient at risk

| | | | | | | | |
|---|---|---|---|---|---|---|---|
| Group: SIG NEG | | | | | | | |
| | 170 | 132 | 71 | 43 | 30 | 22 | 21 |
| Group: SIG POS | | | | | | | |
| | 95 | 59 | 18 | 12 | 8 | 7 | 6 |

HR=1.6403 [1.2252-2.1960]; p=0.0002

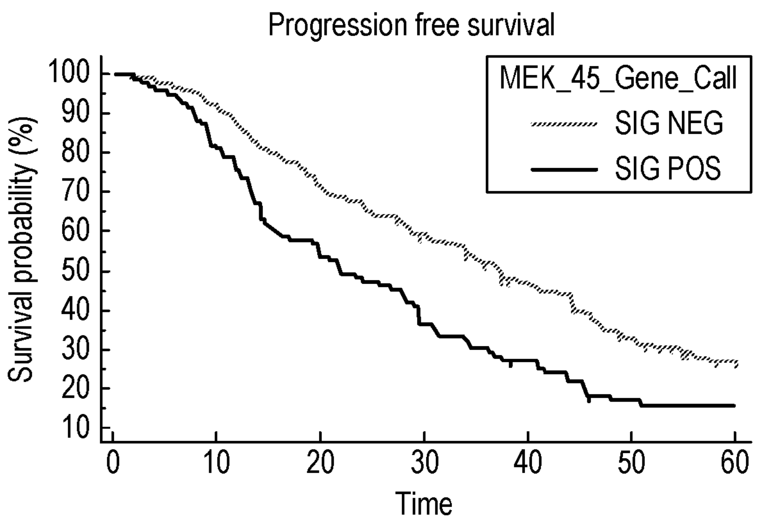


Number patient at risk

| | | | | | | | |
|---|---|---|---|---|---|---|---|
| Group: SIG NEG | | | | | | | |
| | 170 | 154 | 121 | 99 | 73 | 49 | 35 |
| Group: SIG POS | | | | | | | |
| | 95 | 77 | 51 | 35 | 25 | 12 | 11 |

HR=1.6562 [1.2169-2.2540]; p=0.0004

**FIGURE 1 CONTINUED**

d. HGSOC molecular subgroups are dynamic in the context of chemotherapy. Patients move into the bad prognosis subgroup, the Angio_immune subgroup after chemotherapy treatment

Classification using raw scores

| | | Post Chemo | | |
|---|---|---|---|---|
| Pre Chemo | | Immune | Angio | AngioImmune |
| | Immune (21 pairs) | 10 | 0 | 11 |
| | Angio (8 pairs) | 1 | 3 | 4 |
| | AngioImmune (19 pairs) | 3 | 5 | 11 |

- Immune
  - 48% of samples remained classified as Immune post chemo
  - 52% of samples classified as AngioImmune post chemo
  - No samples classified as Angio post chemo

- Angio
  - 38% of samples remained classified as Angio post chemo
  - 50% of samples classified as AngioImmune post chemo
  - 12% of samples classified as Immune post chemo

- AngioImmune
  - 58% of samples remained classified as AngioImmune post chemo
  - 16% of samples classified as Immune post chemo
  - 26% of samples classified as Angio post chemo

**FIGURE 1 CONTINUED**

a. Generation of the OVCAR3 cisplatin resistant cells

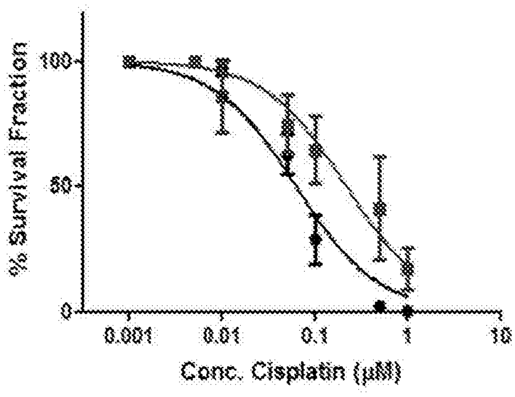


| Cell Lines | Cisplatin Alone |
|---|---|
| OVCAR3-WT | 0.066uM |
| OVCAR3-CP | 0.29uM |

b. 3 gene signature scores in A2780

Angio_immune | Angiogenesis | Immune

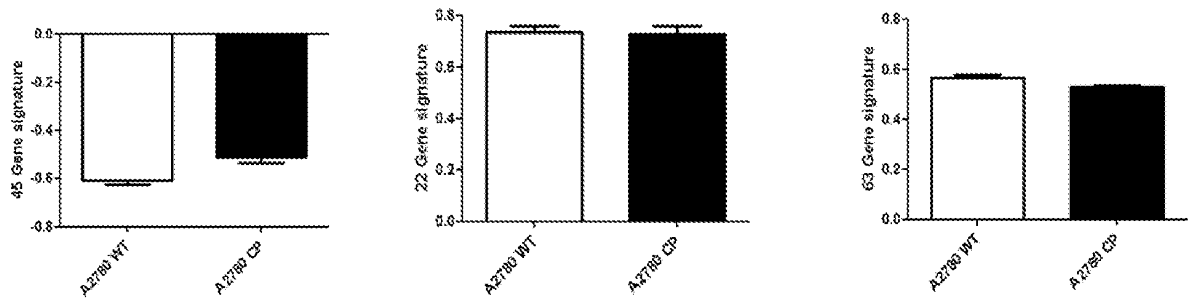


P=0.0057 | P=0.3959 | P=0.0124

c. 3 gene signature scores in OVCAR3

Angio_immune | Angiogenesis | Immune

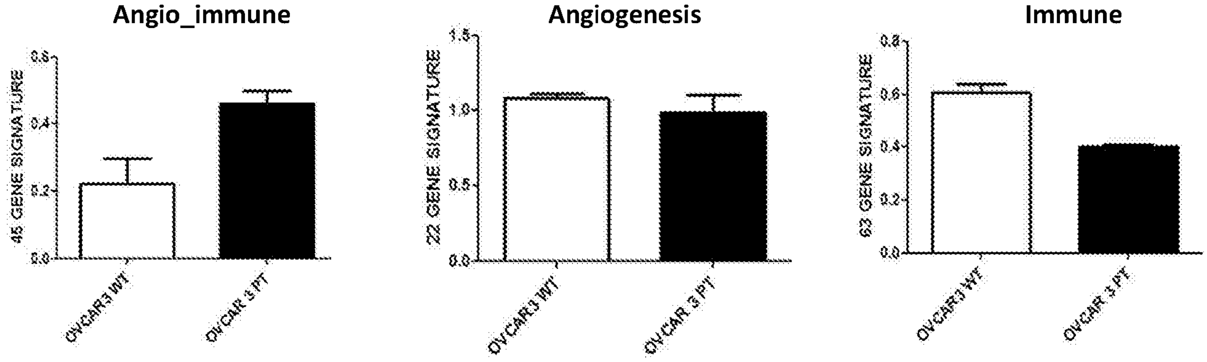


P=0.0244 | P=0.2478 | P=0.028

**FIGURE 2**

d. Characterisation of the cisplatin resistant cells

**A2780 SENSITIVE AND RESISTANT**

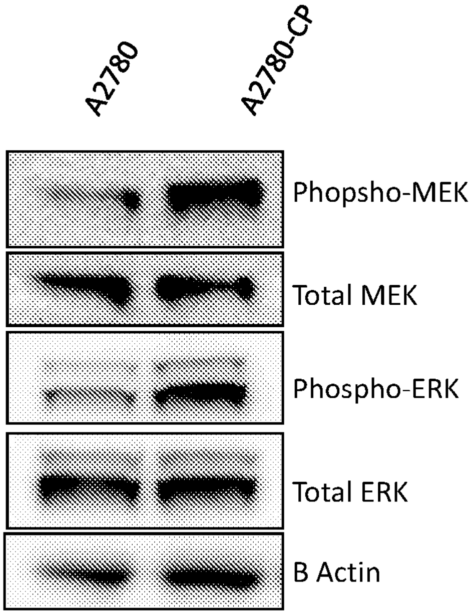


**OVCAR3 SENSITIVE AND RESISTANT**

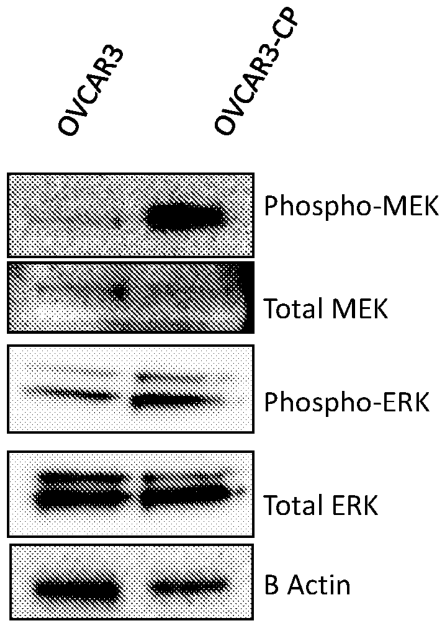


e. Colony formation assays with Cisplatin in 15 ovarian cancer cell lines

Median centre IC50

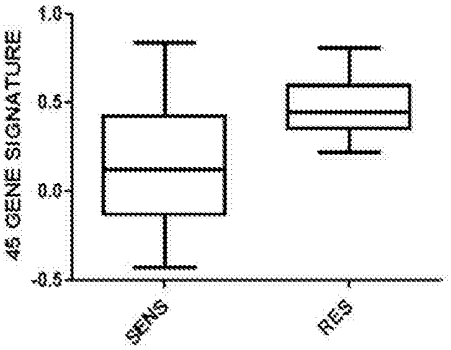


AUC .0.7917
(0.6350-0.9483)
P= 0.0008308

Median centre signature score

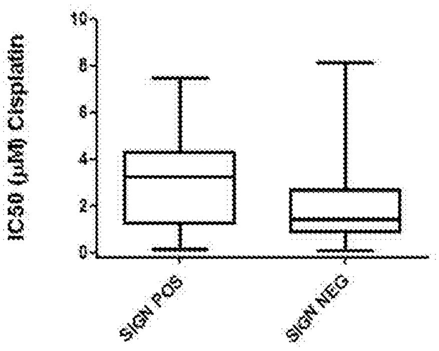


AUC .0.6838
(0.5184-0.8492)
P= 0.03477

**FIGURE 2 CONTINUED**

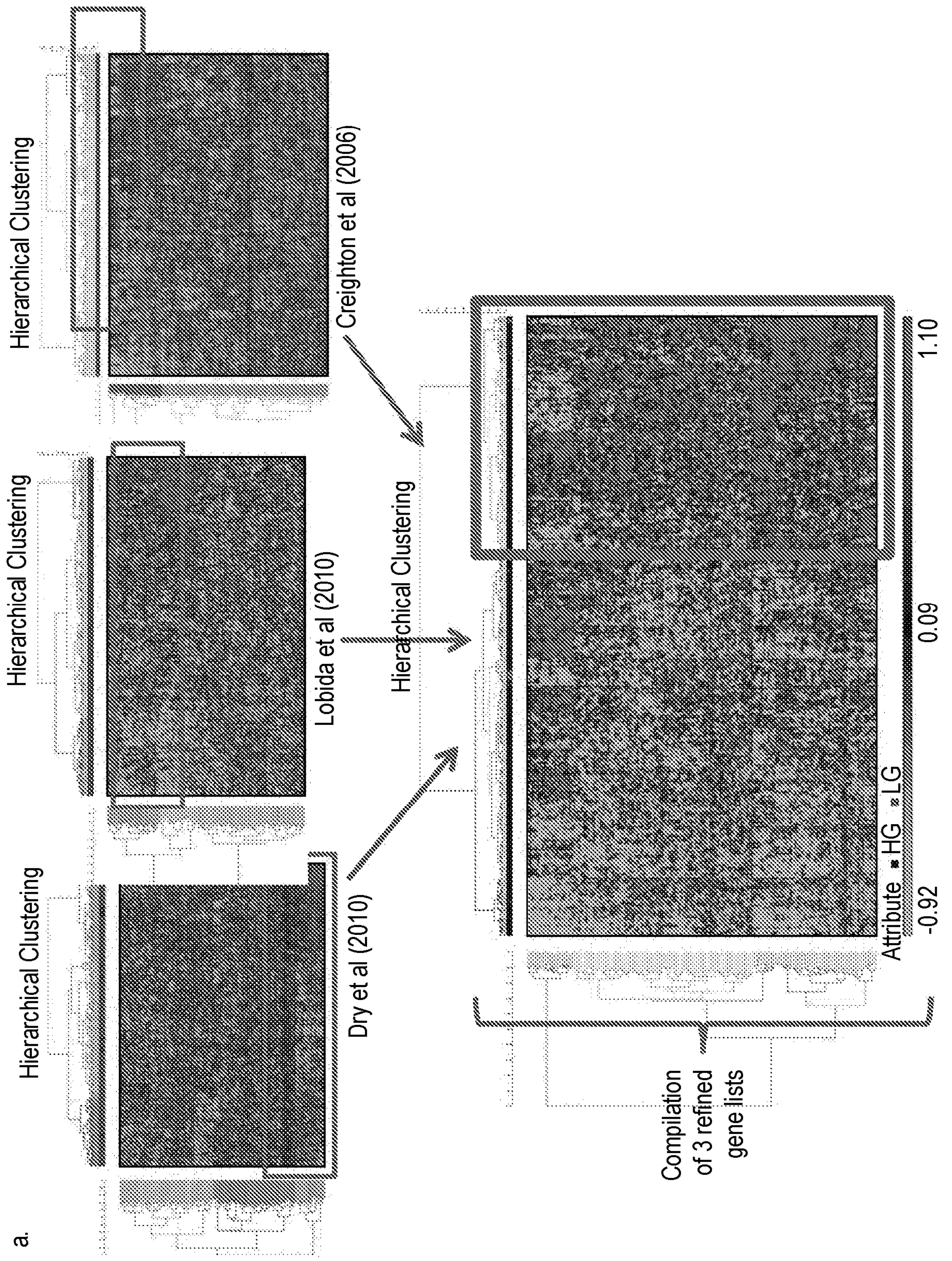
a.
Hierarchical Clustering
Hierarchical Clustering
Hierarchical Clustering
Dry et al (2010)
Lobida et al (2010)
Creighton et al (2006)
Hierarchical Clustering
Compilation
of 3 refined
gene lists
Attribute HG LG
-0.92
0.09
1.10

FIGURE 3

b.

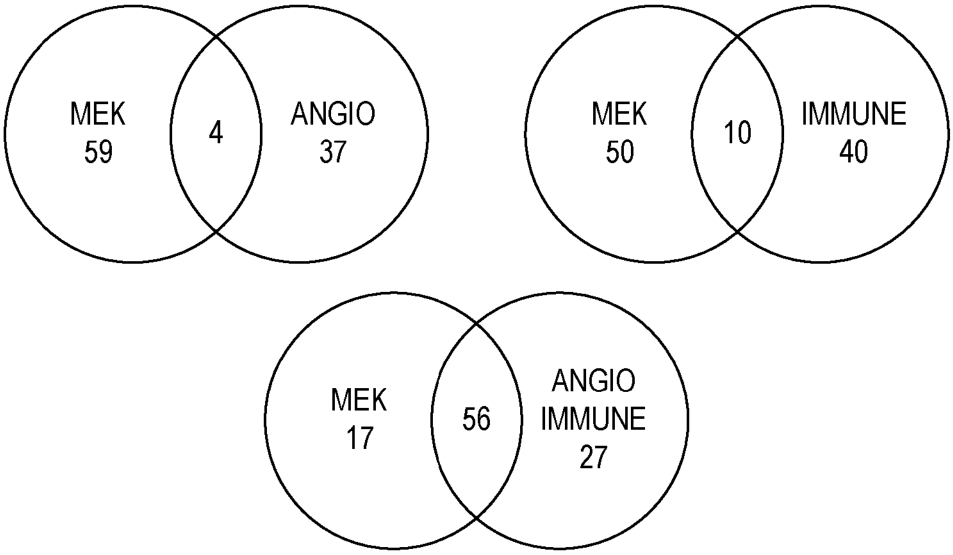


- 76% of ‘MEK ON’ patients fall within the Angio_immune group
- No/little overlap with other 2 molecular subgroups
- Angio_immune group is the MEK ON group

# FIGURE 3 CONTINUED

c. The Angioimmune group is associated with pMAPK expression

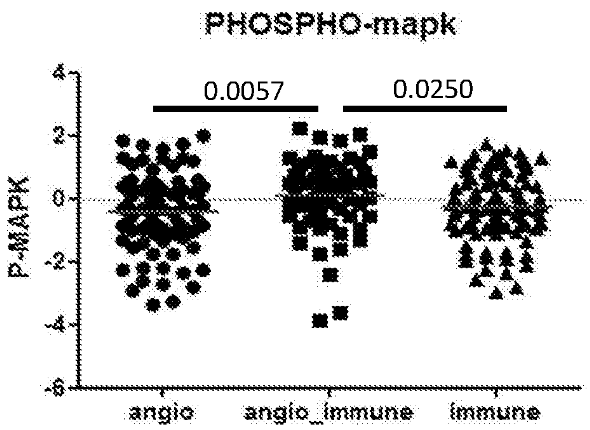


d) (i) Internal data (breast and ovarian cell lines)

Median centre IC50

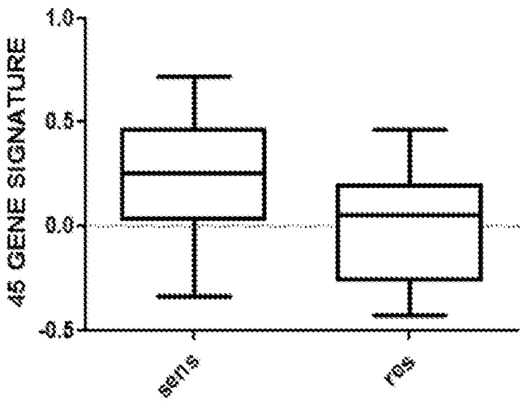


AUC 0.7234
(0.5778-0.8690)
P= 0.009066

Median centre signature score

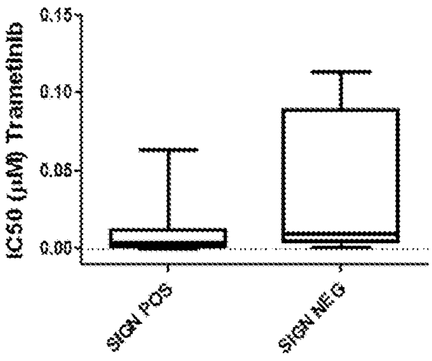


AUC 0.7147
(0.5674-0.8620)
P= 0.01170

**FIGURE 3 CONTINUED**

**ii. Trametinib: External Sanger cell line data**

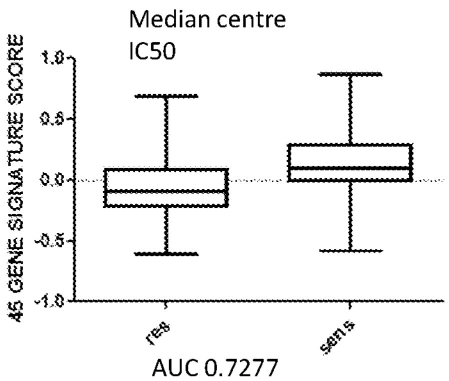


AUC 0.7277
(0.6945-0.7609)
P= < 0.0001

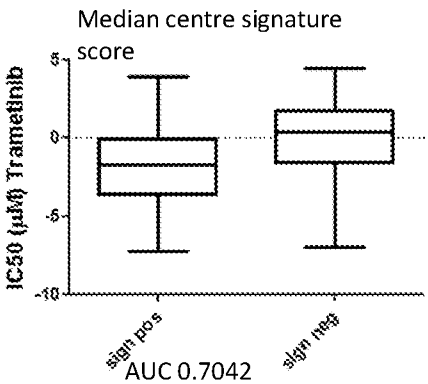


AUC 0.7042
(0.6695-0.7389)
P= < 0.0001

**ii. Selumetinib: External Sanger cell line data**

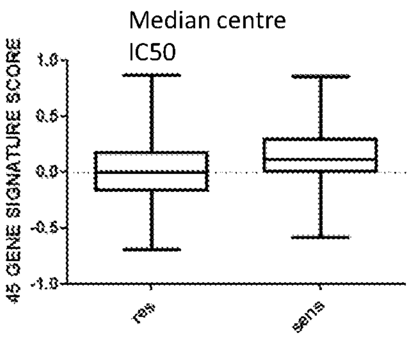


AUC 0.6598
(0.6213-0.6983)
P= < 0.0001

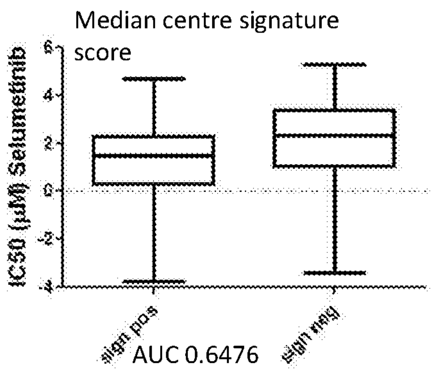


AUC 0.6476
(0.6083-0.6869)
P= < 0.0001

# FIGURE 3 CONTINUED

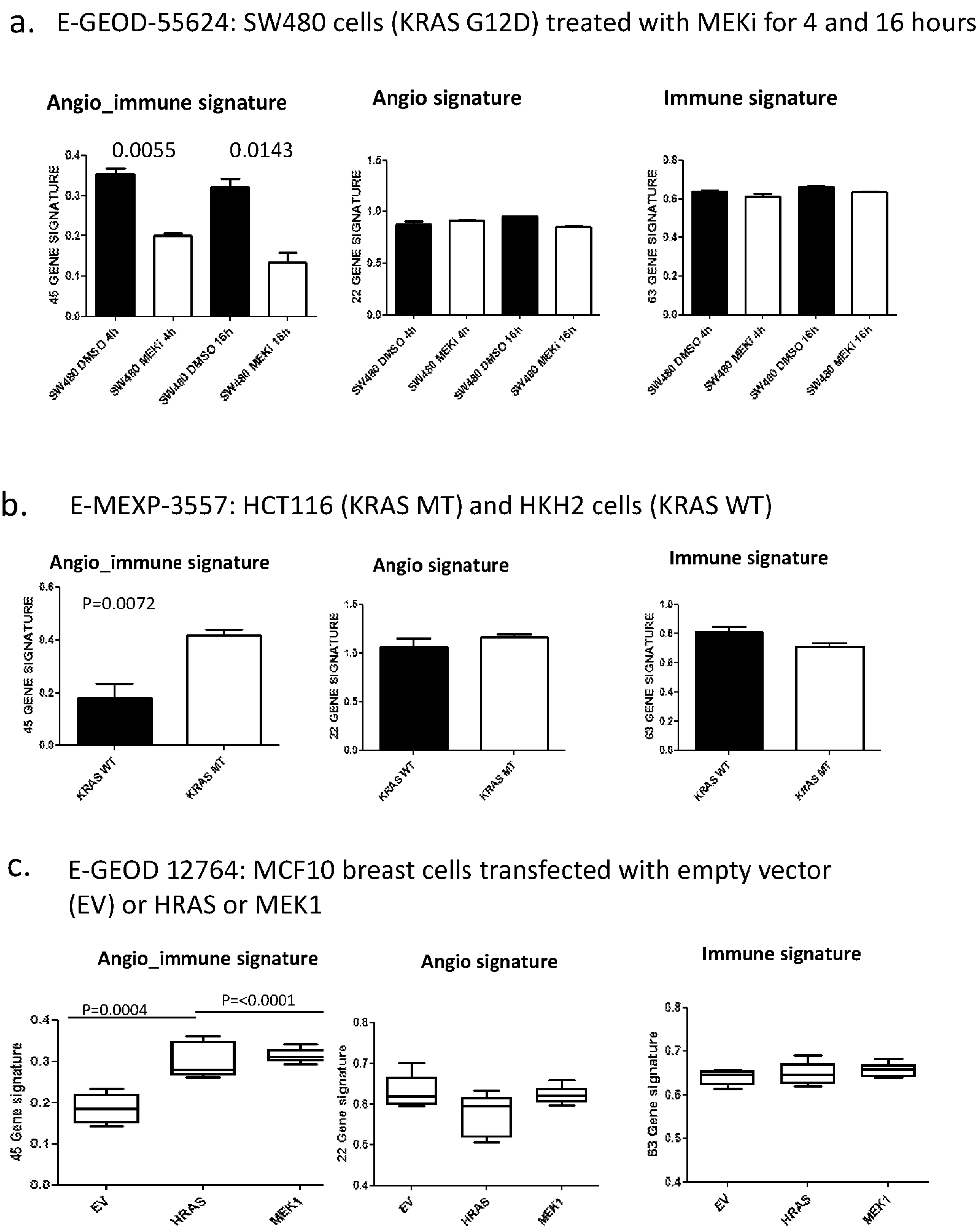
a. E-GEOD-55624: SW480 cells (KRAS G12D) treated with MEKi for 4 and 16 hours
Angio_immune signature
Angio signature
Immune signature
0.0055
0.0143
45 GENE SIGNATURE
22 GENE SIGNATURE
63 GENE SIGNATURE
SW480 DMSO 4h
SW480 MEKi 4h
SW480 DMSO 16h
SW480 MEKi 16h
b. E-MEXP-3557: HCT116 (KRAS MT) and HKH2 cells (KRAS WT)
Angio_immune signature
Angio signature
Immune signature
P=0.0072
45 GENE SIGNATURE
22 GENE SIGNATURE
63 GENE SIGNATURE
KRAS WT
KRAS MT
c. E-GEOD 12764: MCF10 breast cells transfected with empty vector (EV) or HRAS or MEK1
Angio_immune signature
Angio signature
Immune signature
P=0.0004
P=<0.0001
45 Gene signature
22 Gene signature
63 Gene signature
EV
HRAS
MEK1

FIGURE 4

d. Inhibition of MEK with Trametinib decreases 45 gene Angioimmune score in OVCAR3

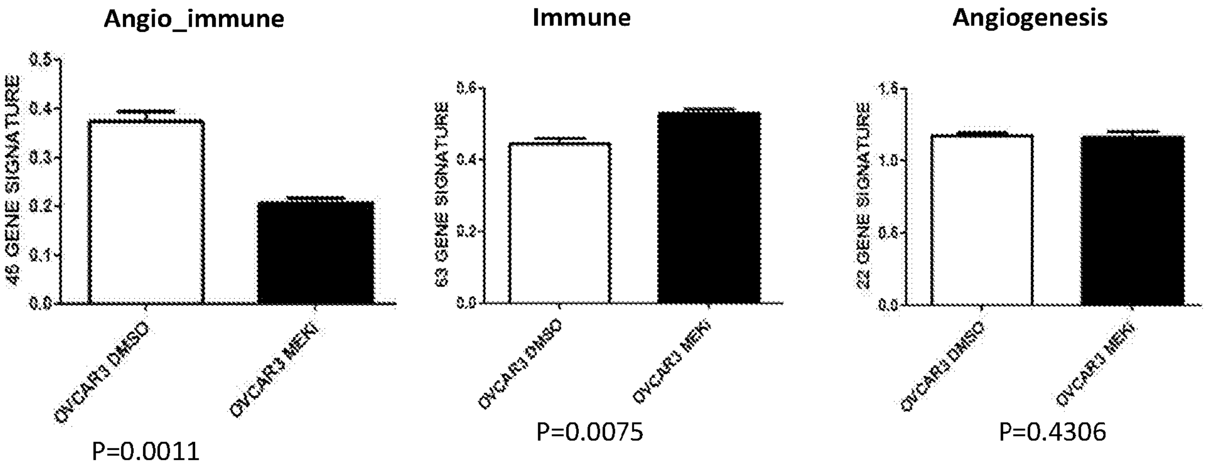


FIGURE 4 CONTINUED

a. OVCAR3 MEK assay positive (cisplatin resistant ) cells are sensitive to MEK inhibitor and resistant to cisplatin

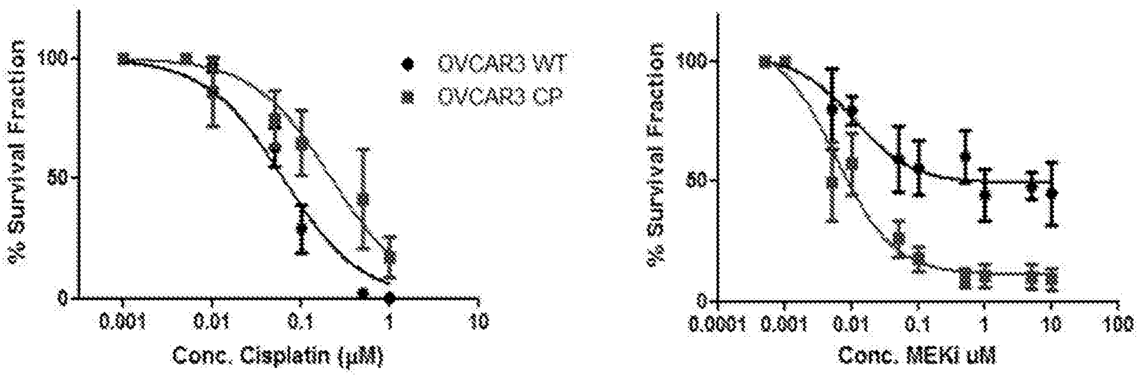


b. MEK inhibition resensitises OVCAR3 MEK assay positive (cisplatin resistant ) cells to cisplatin

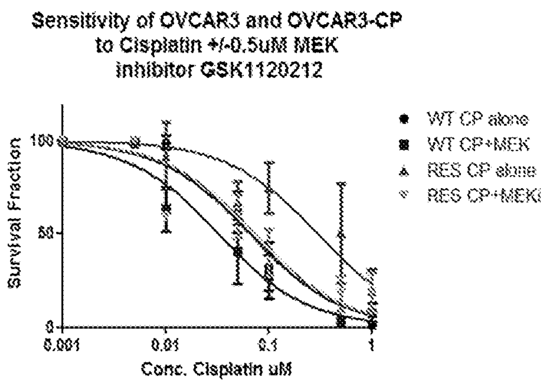


| Cell Lines | Cisplatin Alone | Cisplatin + 0.5uM MEKi | Fold Chang e |
|---|---|---|---|
| OVCAR3-WT | 0.066uM | 0.033uM | 2 |
| OVCAR3-CP | 0.29uM | 0.06uM | 4.8 |

# FIGURE 5

a. Expression of EMT related genes across 3 HGSOC molecular subgroups

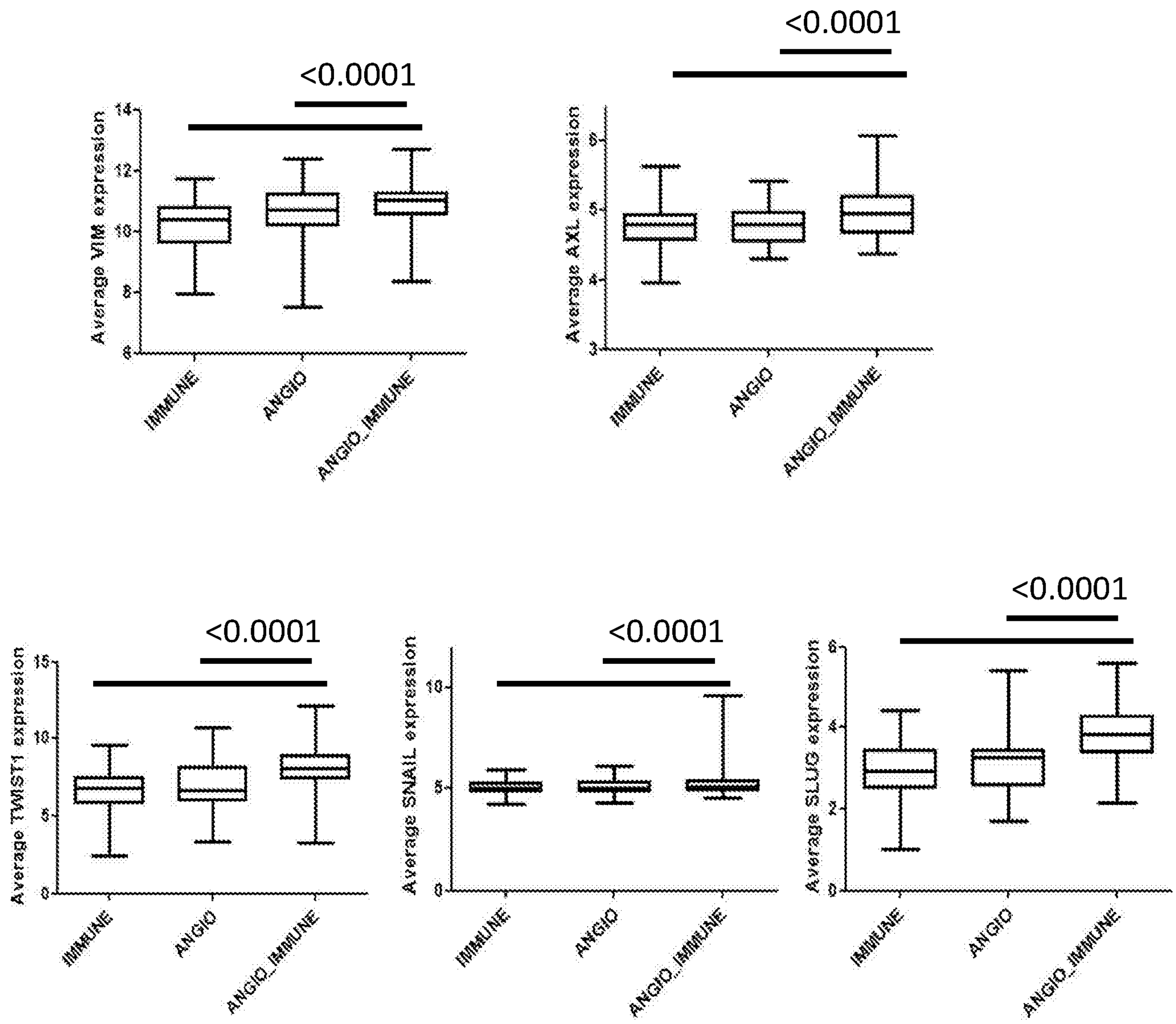


b. 45 gene signature is elevated by SNAIL overexpression

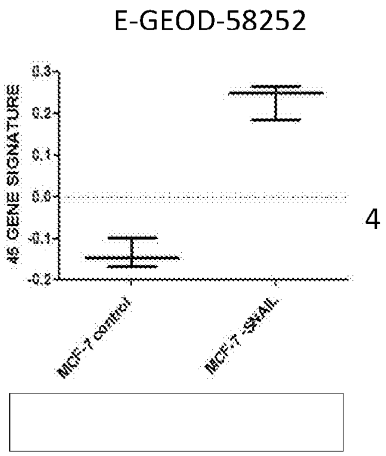


FIGURE 6

a. Characterisation of the OVCAR4 cisplatin resistant cells (Ovcar3 data Figure 2d.).

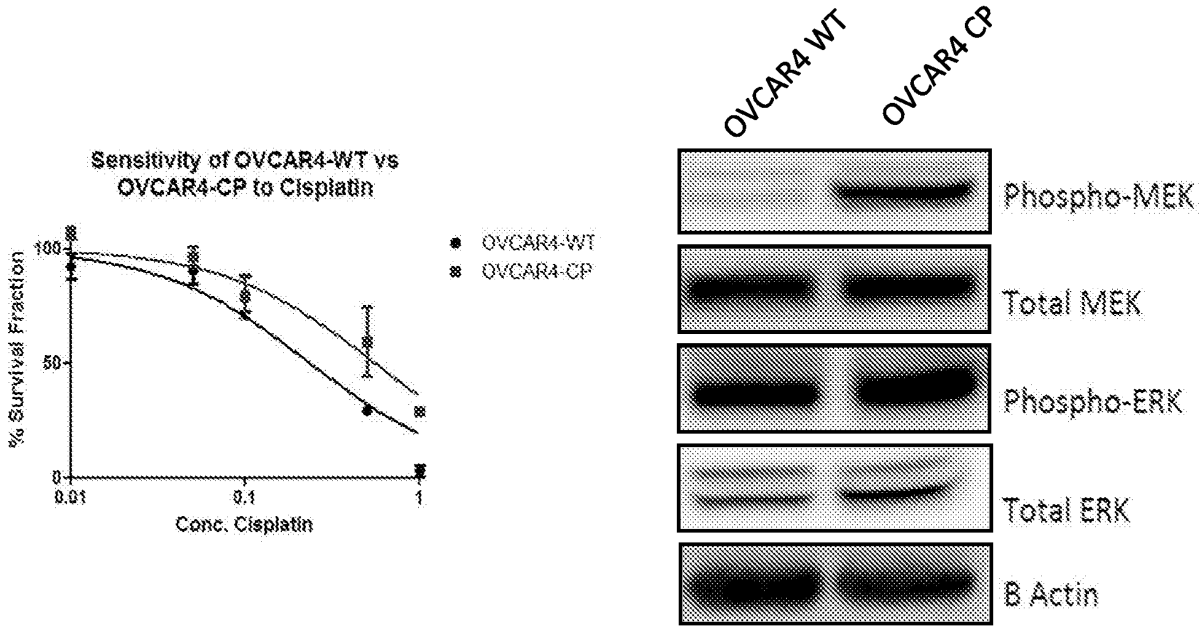


b. Activation of EMT protein markers in Cisplatin resistant OVCAR3 and OVCAR4

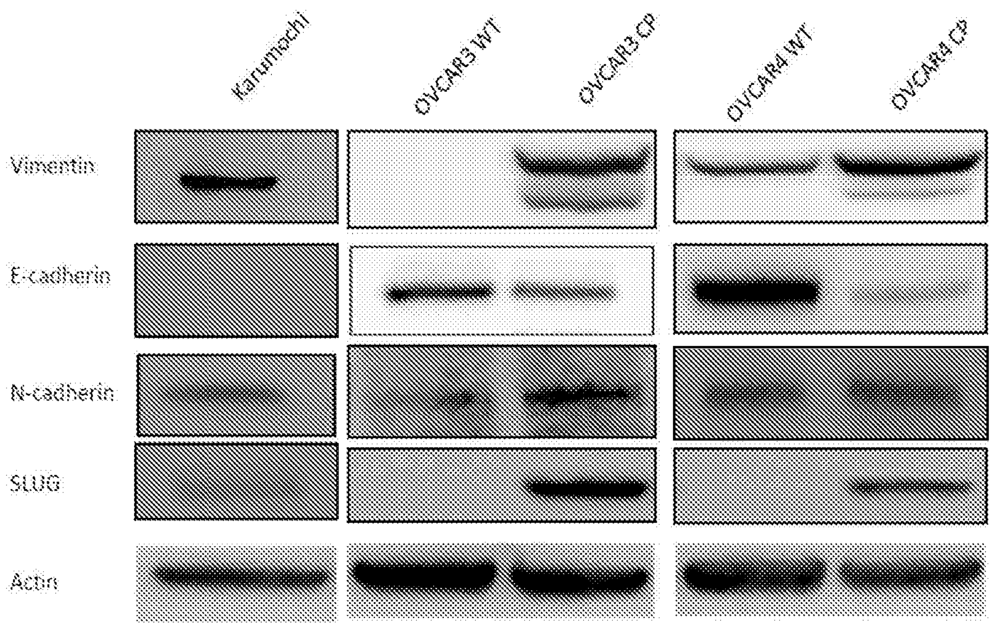


FIGURE 7

c. Increased expression of EMT markers in Cisplatin resistant OVCAR3 cells

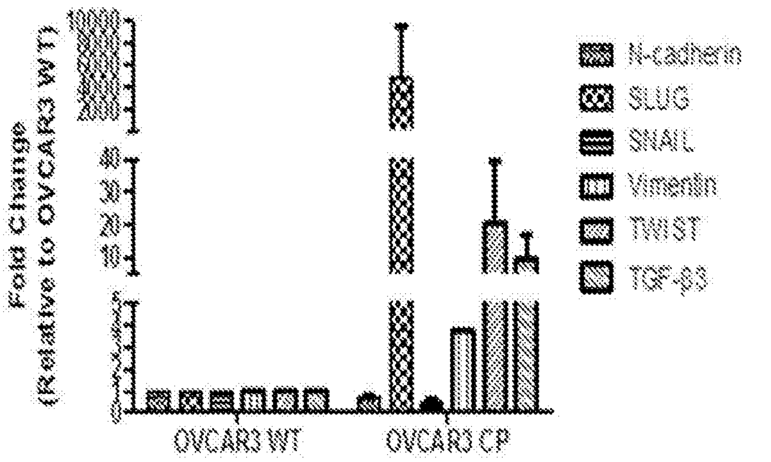


d. Increased expression of EMT markers in Cisplatin resistant OVCAR4 cells

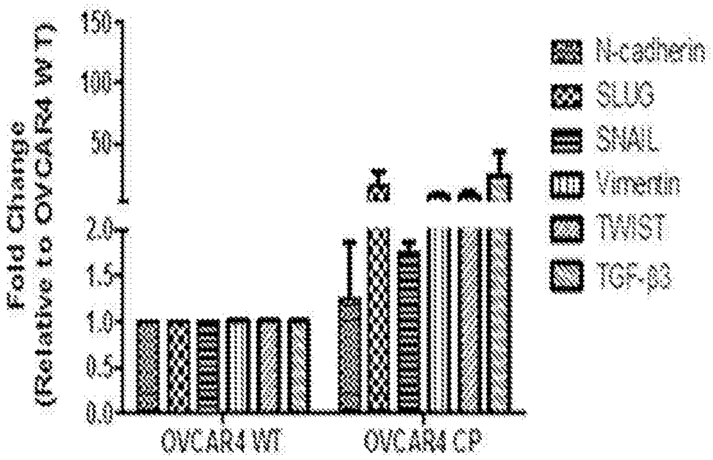


**FIGURE 7 CONTINUED**

e. Cisplatin Resistance of OVCAR3 and OVCAR4 confers a migratory phenotype in vitro

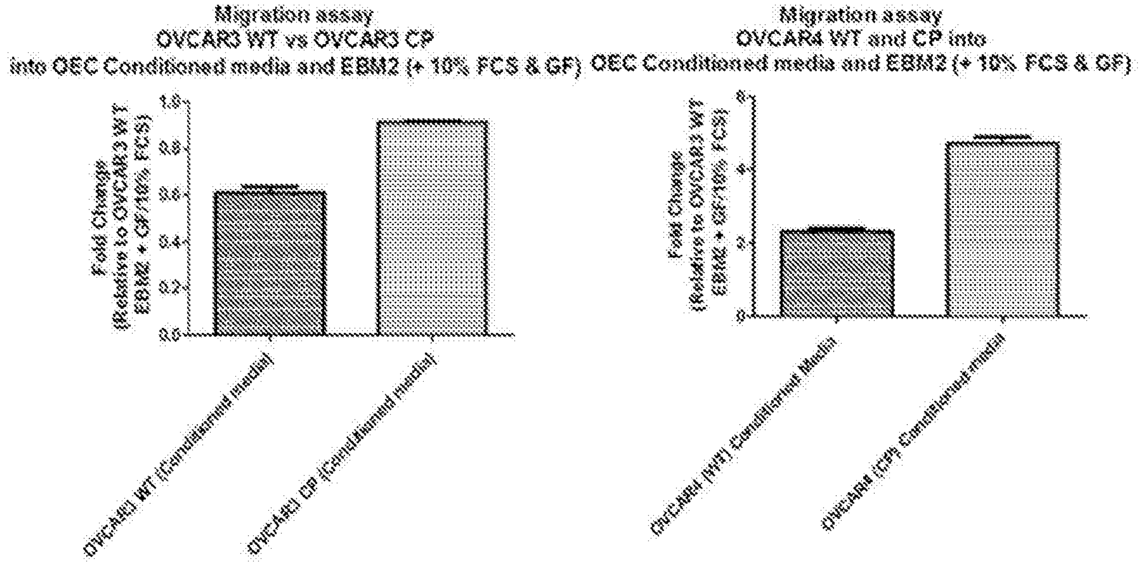


FIGURE 7 CONTINUED

a. MAPK and SRC are parallel pathway

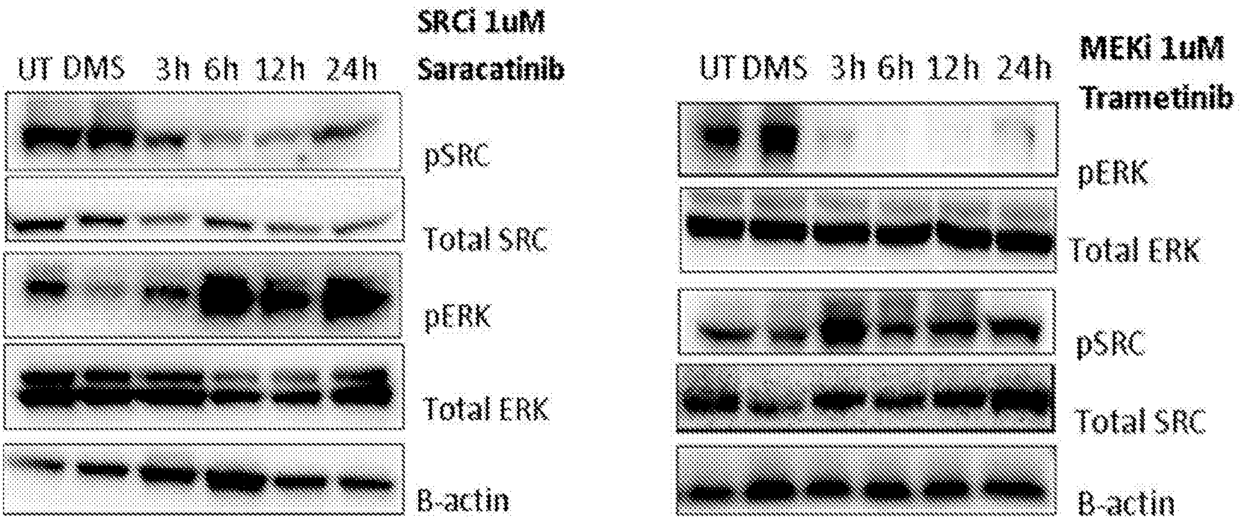


b. The 45-gene signature predicts resistance to SRC inhibitors

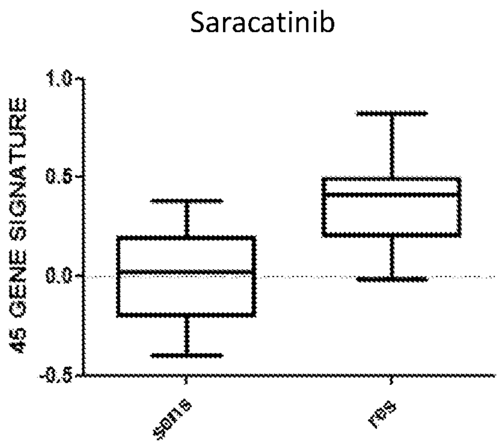


| | |
|---|---|
| Area | 0.8867 |
| Std. Error | 0.05665 |
| 95% confidence interval | 0.7757 to 0.9978 |
| P value | 0.0001927 |

**FIGURE 8**

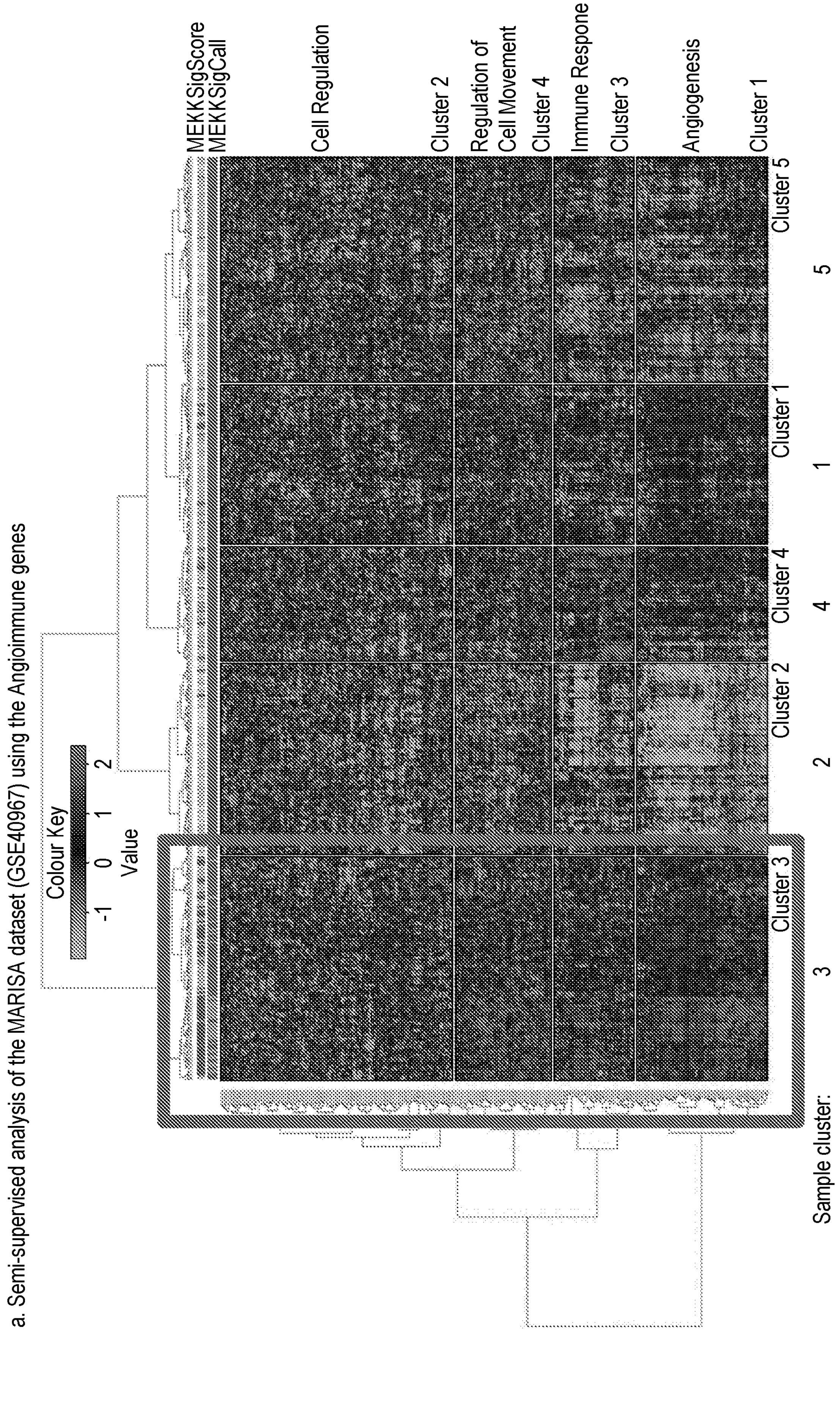
a. Semi-supervised analysis of the MARISA dataset (GSE40967) using the Angioimmune genes
Colour Key
-1 0 1 2
Value
MEKKSigScore
MEKKSigCall
Cell Regulation
Cluster 2
Regulation of
Cell Movement
Cluster 4
Immune Respone
Cluster 3
Angiogenesis
Cluster 1
Cluster 3
Cluster 2
Cluster 4
Cluster 1
Cluster 5
Sample cluster:
3
2
4
1
5

**FIGURE 9**

b. Relapse free survival of the 5 subtypes

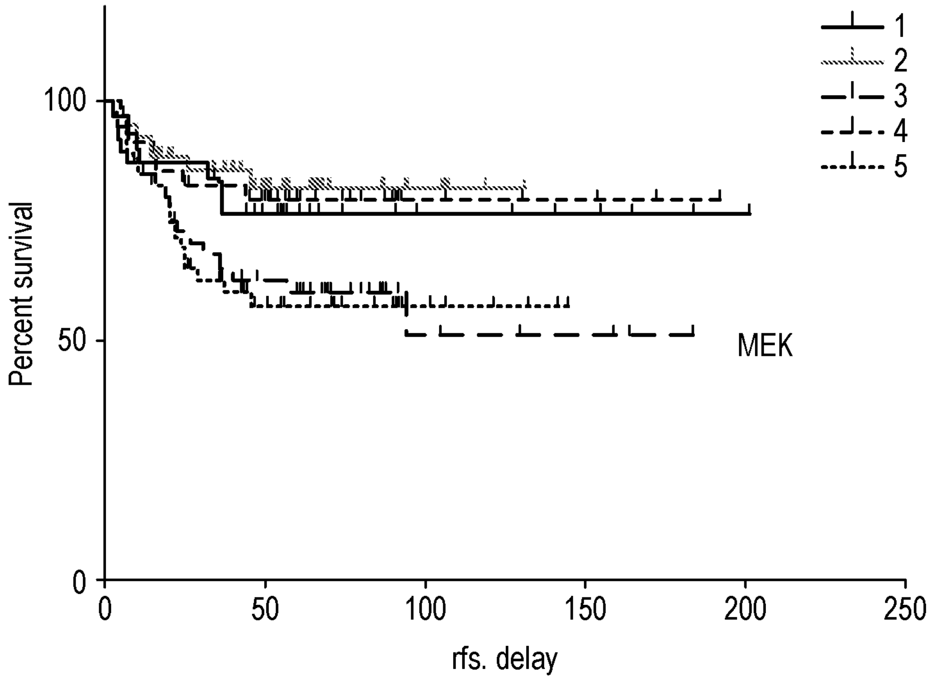


| | C1 | C2 | C3 | C4 | C5 |
|---|---|---|---|---|---|
| STAGE 1 | 0 | 0 | 0 | 0 | 0 |
| STAGE 2 | 26 | 47 | 35 | 26 | 45 |
| STAGE 3 | 4 | 8 | 7 | 9 | 53 |
| STAGE 4 | 5 | 2 | 3 | 2 | 11 |

p=0.037

# FIGURE 9 CONTINUED

c. Relapse free survival using 45 gene signature score in Marisa (GSE40967)

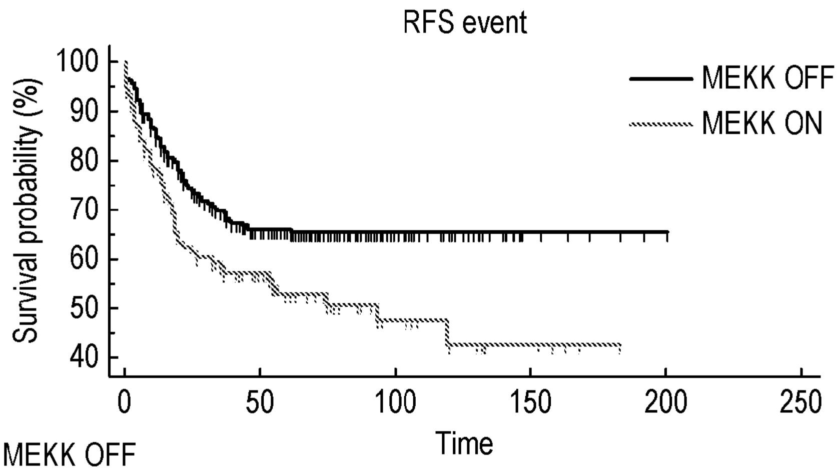


| | | | | | | |
|---|---|---|---|---|---|---|
| MEKK OFF | | | | | | |
| | 301 | 128 | 38 | 6 | 1 | 0 |
| MEKK ON | | | | | | |
| | 100 | 44 | 13 | 5 | 0 | 0 |

HR=1.5949 [1.0951-2.3228]
P=0.0063

d. Disease free survival using 45 gene signature score in Jorissen (GSE14333)

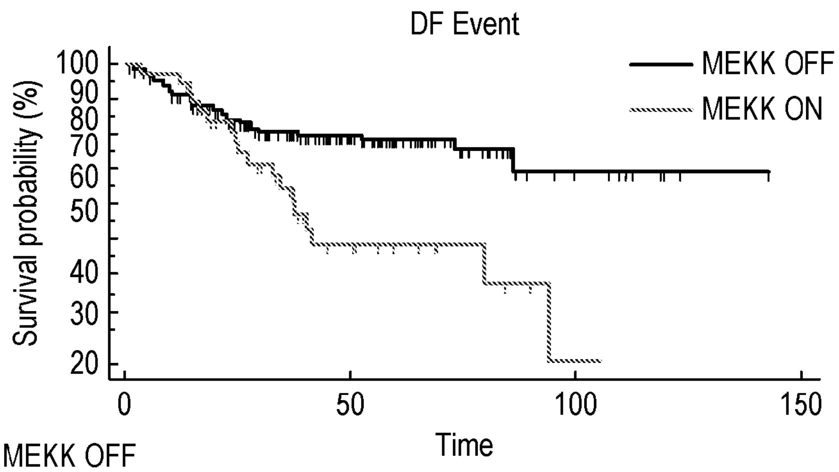


| | | | | |
|---|---|---|---|---|
| MEKK OFF | | | | |
| | 185 | 74 | 8 | 0 |
| MEKK ON | | | | |
| | 41 | 12 | 1 | 0 |

HR=2.4543 [1.2049-4.9994]
P=0.0014

# FIGURE 9 CONTINUED

a. Semi-supervised analysis of the Okayama dataset (GSE31210) using the Angioimmune genes

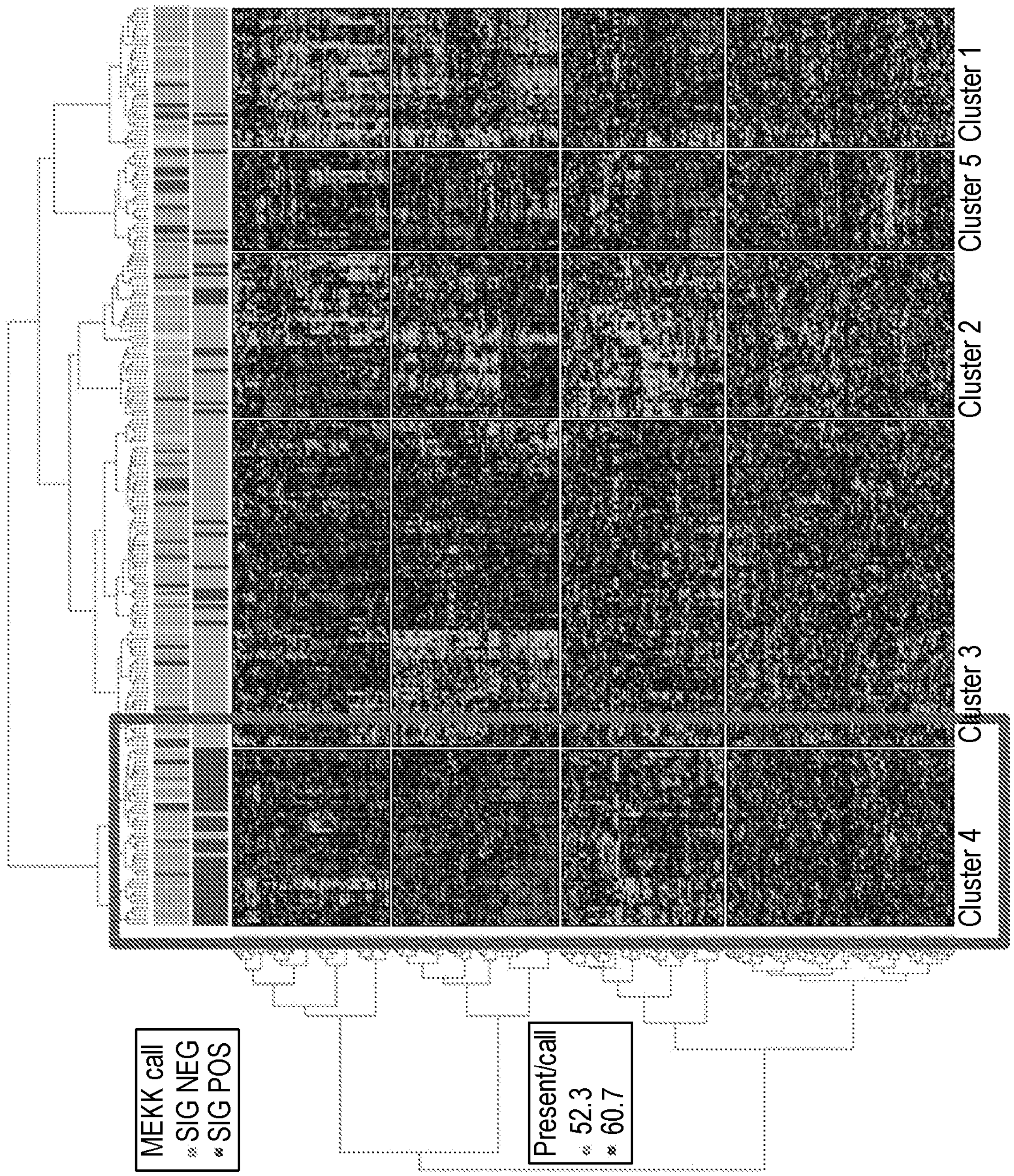


FIGURE 10

b. Relapse free survival of the 5 subtypes

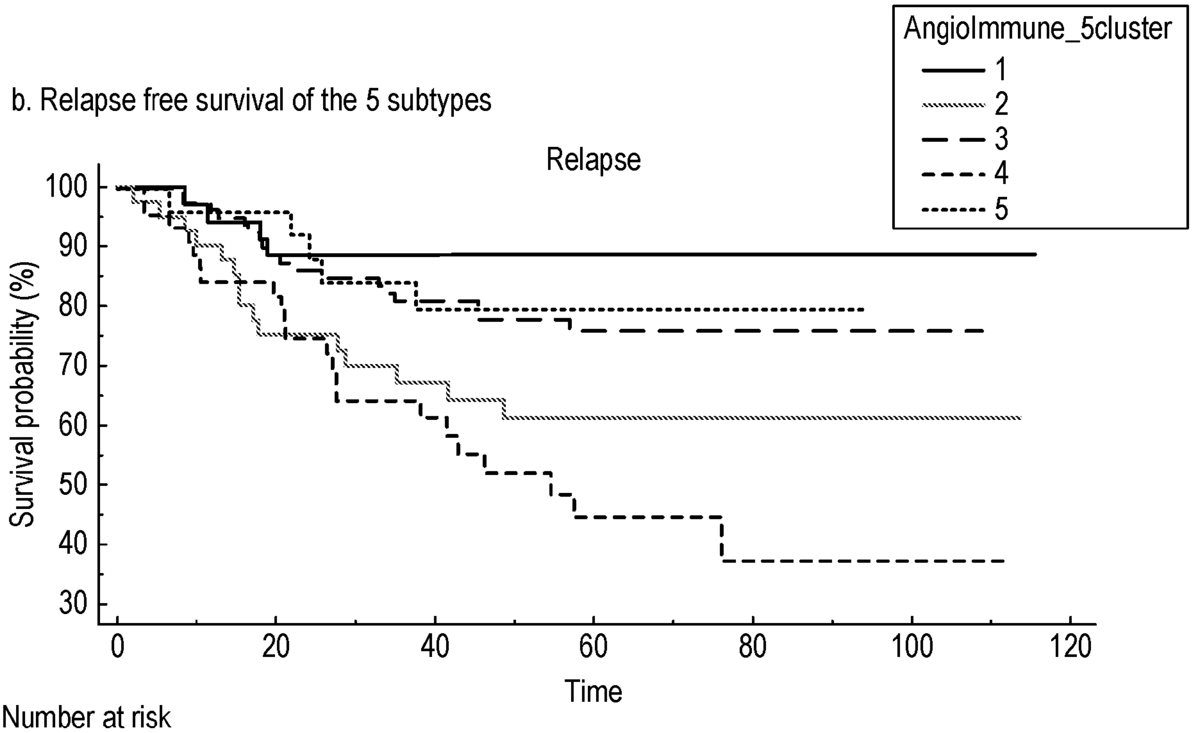


Number at risk

| | | | | | | | |
|---|---|---|---|---|---|---|---|
| Group: 1 | | | | | | | |
| | 35 | 31 | 29 | 24 | 6 | 3 | 0 |
| Group: 2 | | | | | | | |
| | 41 | 30 | 22 | 16 | 4 | 2 | 0 |
| Group: 3 | | | | | | | |
| | 81 | 70 | 58 | 35 | 11 | 4 | 0 |
| Group: 4 | | | | | | | |
| | 44 | 35 | 21 | 11 | 5 | 1 | 0 |
| Group: 5 | | | | | | | |
| | 25 | 24 | 17 | 9 | 3 | 0 | 0 |

Comparison of survival curves (Logrank test)

| | |
|---|---|
| Chi-squared | 20.5789 |
| DF | 4 |
| Significance | P=0.0004 |

# FIGURE 10 CONTINUED

c. Progression free survival using 45 gene signature score in Okayama dataset (GSE31210)

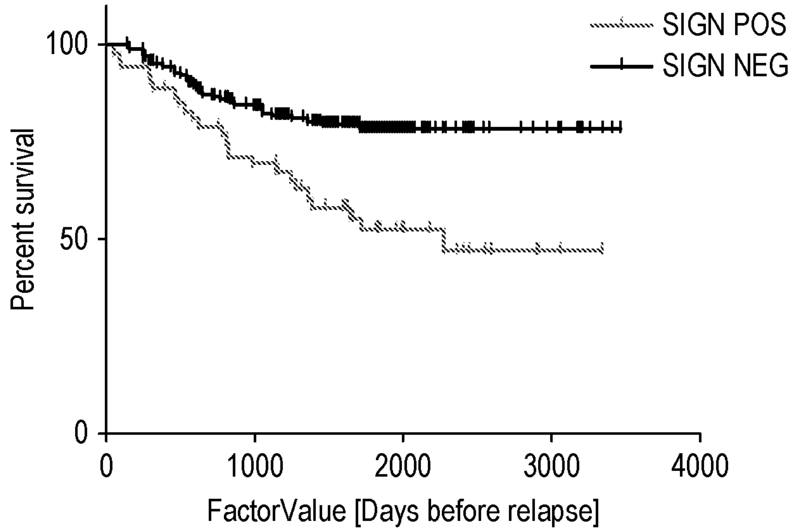


| Hazard Ratio | |
|---|---|
| Ratio | 3.045 |
| 95% CI of ratio | 1.631 to 5.686 |

p=0.0005

d. Overall survival using 45 gene signature score in Okayama dataset (GSE31210)

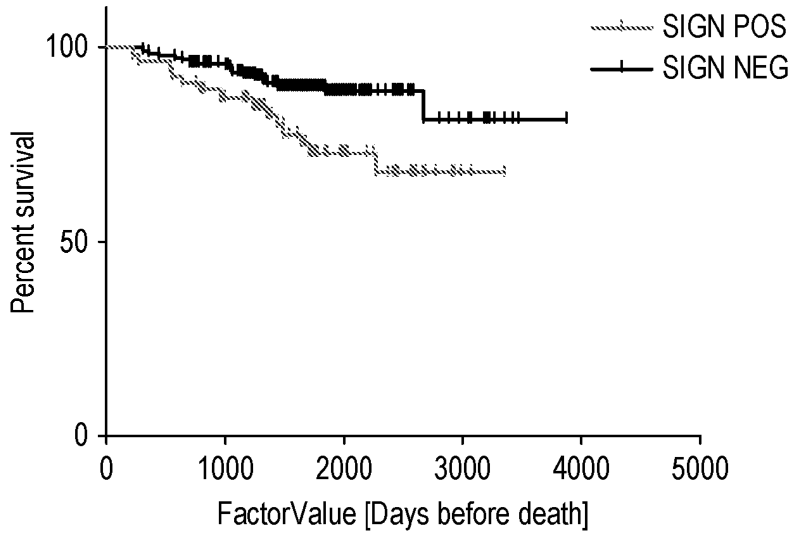


| Hazard Ratio | |
|---|---|
| Ratio | 2.872 |
| 95% CI of ratio | 1.271 to 6.489 |

p=0.0312

# FIGURE 10 CONTINUED

a. Prognostic significance of 15 gene signature in HGSOC

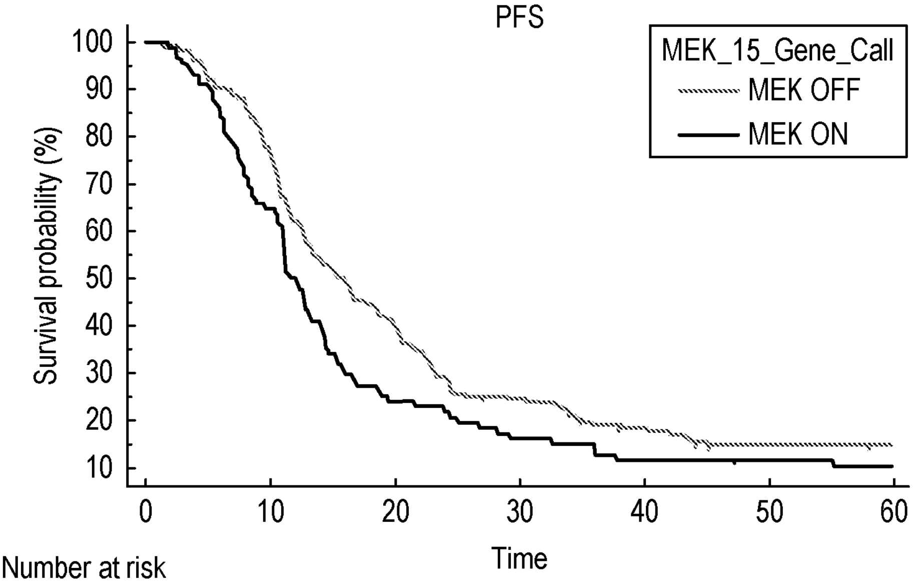


Number at risk
Group: MEK OFF
177 134 33 41 26 20 19
Group: MEK OFF
88 57 21 14 10 9 8
HR=1.3564 [1.0156-1.8117];p=0.0279

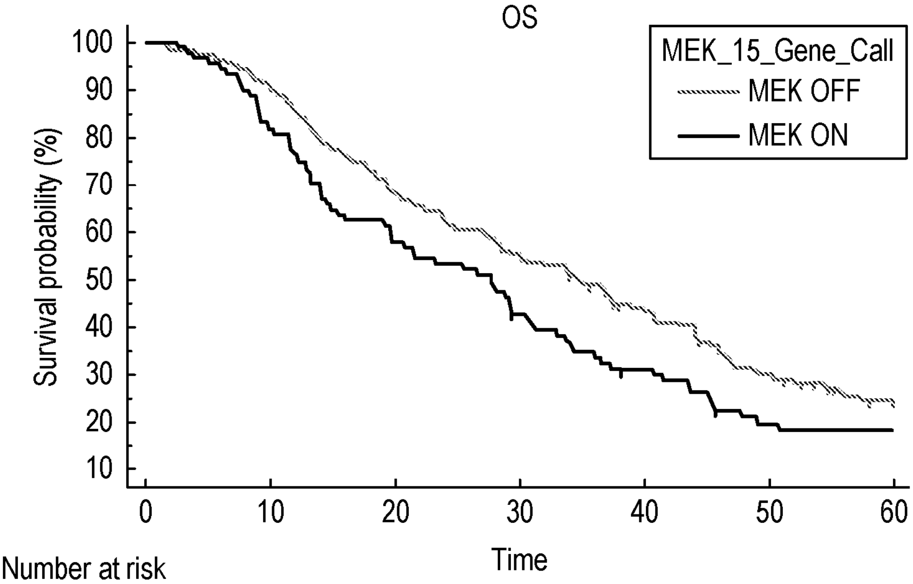


Number at risk
Group: MEK OFF
177 159 121 97 72 47 33
Group: MEK OFF
88 72 51 37 26 14 13
HR=1.3464 [0.9901-1.8308];p=0.0441

**FIGURE 11**

b. Colony formation assays with Cisplatin in 15 ovarian cancer cell lines

Median centre IC50

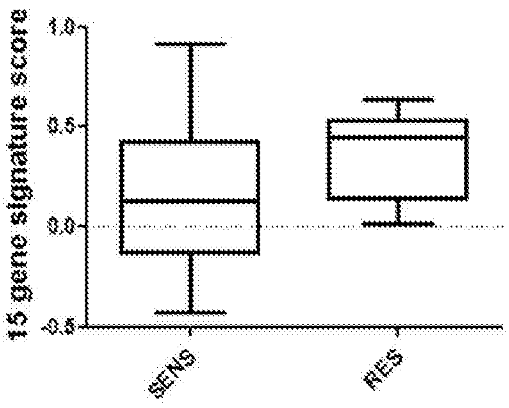


AUC .0.6905
(0.5254-0.8556)
P= 0.02900

Median centre signature score

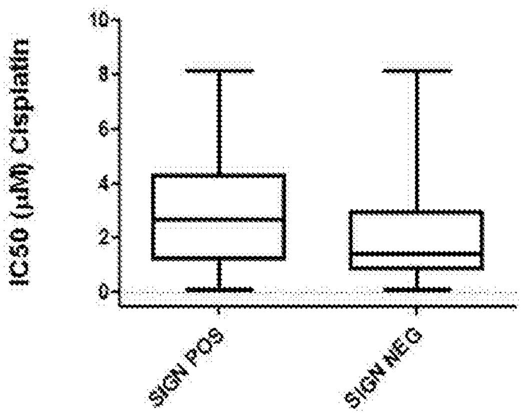


AUC .0.6897
(0.5326-0.8468)
P= 0.02932

c. 15 gene signature scores in OVCAR3 cisplatin sensitive and resistant cells

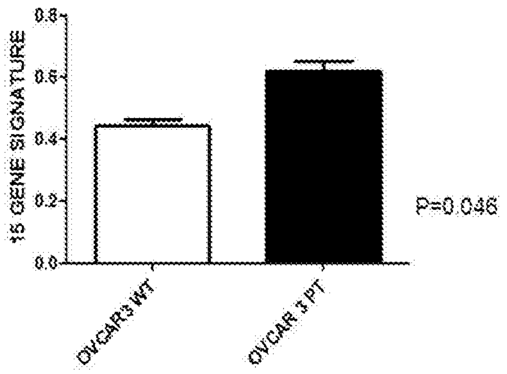


**FIGURE 11**

a. The 15 gene signature score is elevated by mutant KRAS

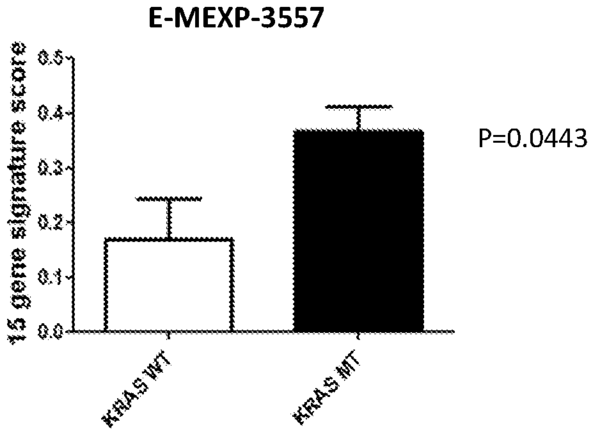


b. The 15 gene signature score is elevated by KRAS and MEK1 overexpression

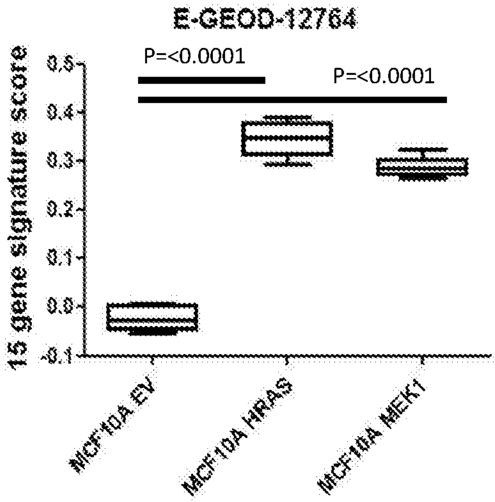


c. The 15 gene signature score is decreased by MEK inhibition

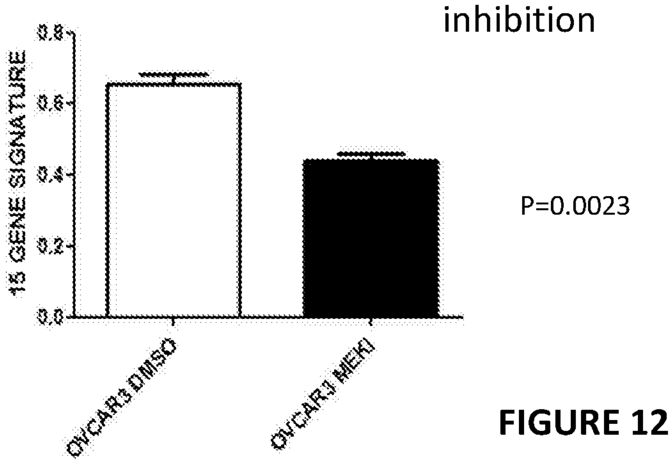


FIGURE 12

d. The 15 gene signature predicts response to MEK inhibitor Trametinib

Median centre IC50

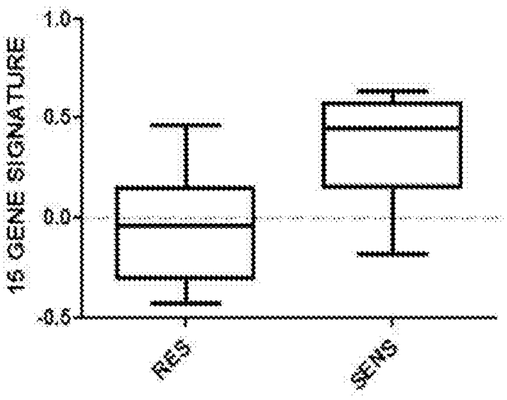


AUC 0.850
(0.7366-0.9636)
P= <0.0001

Median centre signature score

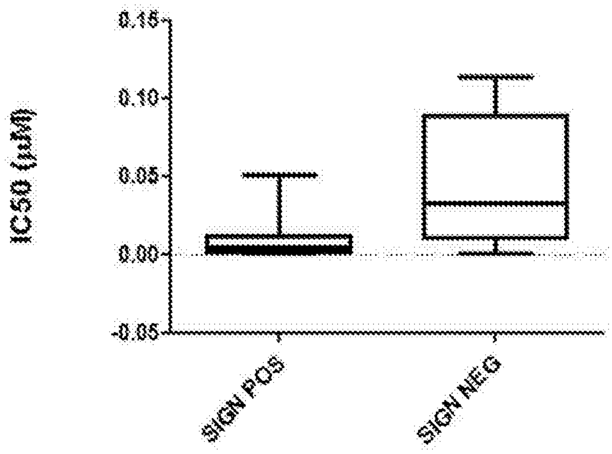


AUC 0.7397
(0.5820-0.8974)
P= 0.006495

FIGURE 12 CONTINUED

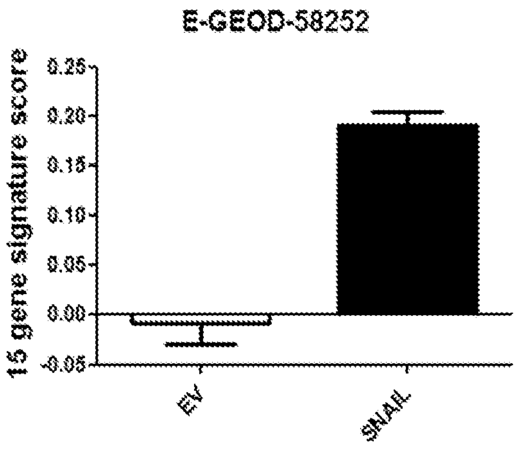


P=0.0015

FIGURE 13

a.

Median centre signature score

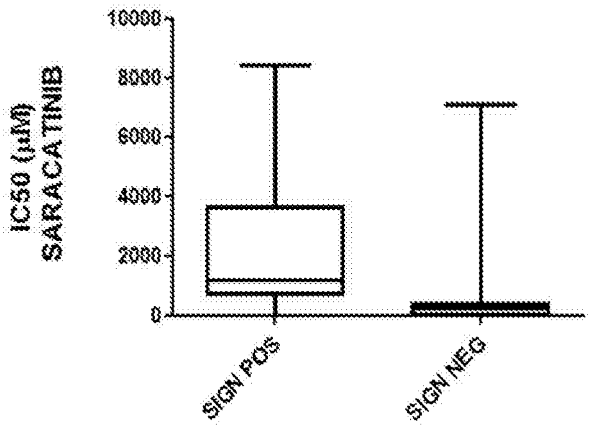


| Area under the ROC curve | |
|---|---|
| Area | 0.7289 |
| Std. Error | 0.08904 |
| 95% confidence interval | 0.5544 to 0.9035 |
| P value | 0.01454 |

b.

Median centre IC50

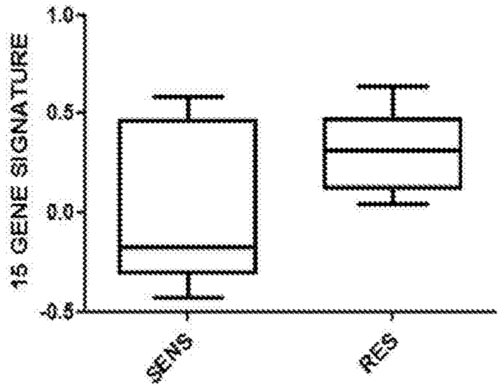


| Area under the ROC curve | |
|---|---|
| Area | 0.7698 |
| Std. Error | 0.08369 |
| 95% confidence interval | 0.6054 to 0.9343 |
| P value | 0.004076 |

FIGURE 14

a. Jorissen

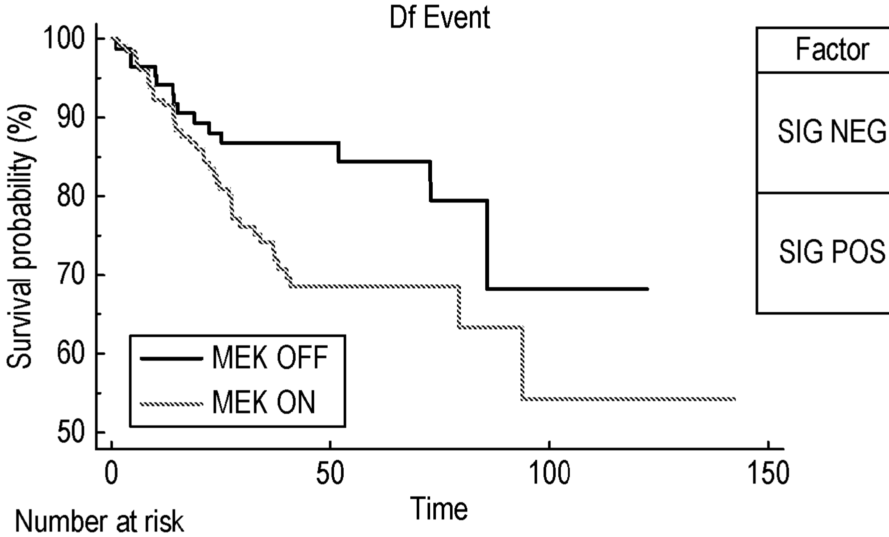


| Factor | SIG POS | SIG NEG |
|---|---|---|
| SIG NEG | - | 1.9243 1.1084 to 3.3409 |
| SIG POS | 0.5197 0.2993 to 0.9022 | - |

P=0.0328

Number at risk

Group: MEKK OFF

89 38 4 0

Group: MEKK ON

137 48 5 0

b. Marissa

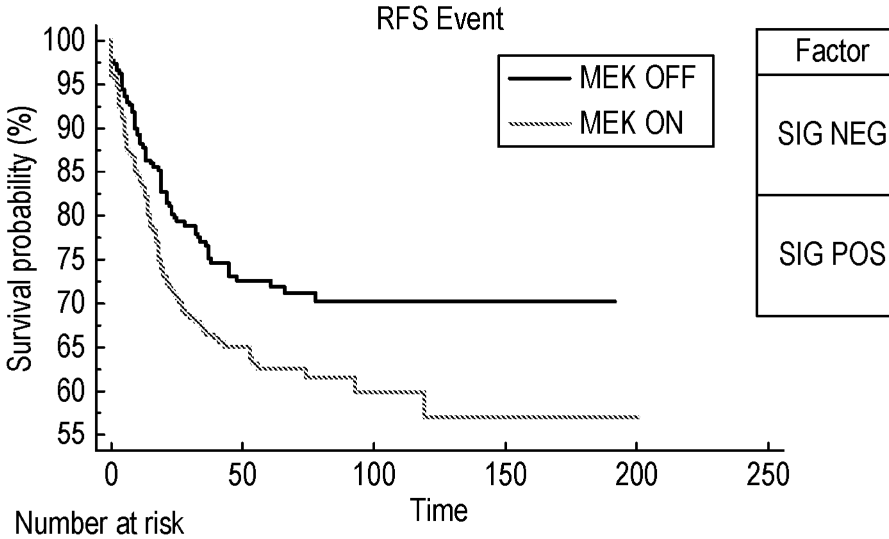


| Factor | SIG POS | SIG NEG |
|---|---|---|
| SIG NEG | - | 1.4567 1.0696 to 1.9839 |
| SIG POS | 0.6865 0.5041 to 0.9349 | - |

P=0.0161

Number at risk

Group: MEKK OFF

277 129 26 2 0 0

Group: MEKK ON

241 114 30 9 1 0

**FIGURE 15**

a. Okayama (PFS)

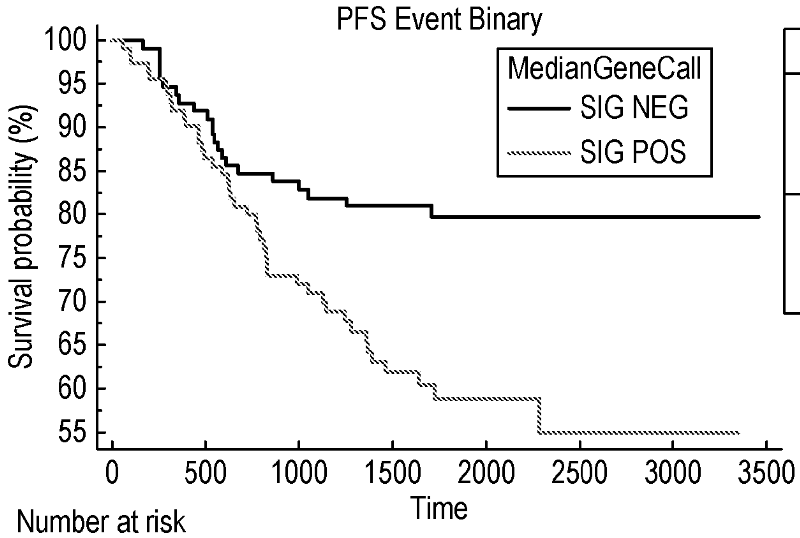


| Factor | SIG POS | SIG NEG |
|---|---|---|
| SIG NEG | - | 2.1787 1.3333 to 3.5602 |
| SIG POS | 0.4590 0.2809 to 0.7500 | - |

P=0.0024

Number at risk

Group: SIG NEG

| | | | | | | | |
|---|---|---|---|---|---|---|---|
| 113 | 103 | 91 | 74 | 29 | 11 | 5 | 0 |

Group: SIG POS

| | | | | | | | |
|---|---|---|---|---|---|---|---|
| 113 | 95 | 70 | 49 | 23 | 10 | 5 | 0 |

b. Okayama (OS)

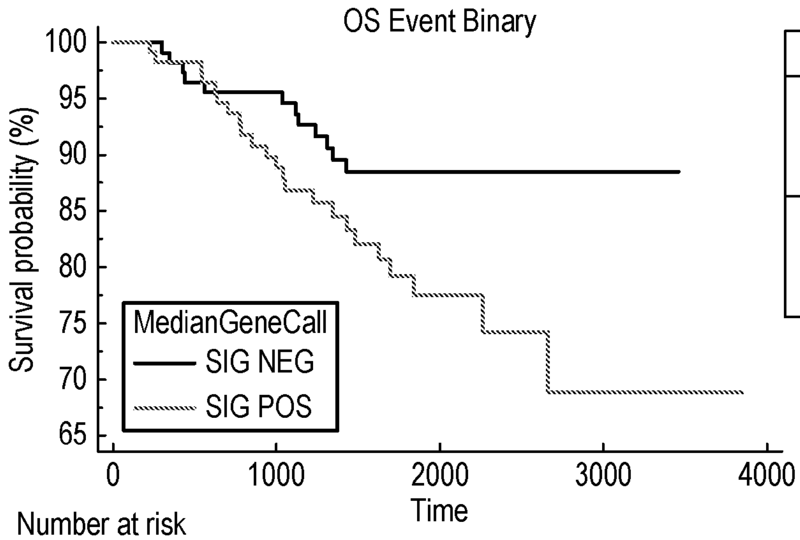


| Factor | SIG POS | SIG NEG |
|---|---|---|
| SIG NEG | - | 2.0463 1.0545 to 3.9709 |
| SIG POS | 0.4887 0.2518 to 0.9483 | - |

P=0.0396

Number at risk

Group: SIG NEG

| | | | | |
|---|---|---|---|---|
| 113 | 103 | 29 | 5 | 0 |

Group: SIG POS

| | | | | |
|---|---|---|---|---|
| 113 | 90 | 33 | 6 | 0 |

**FIGURE 16**

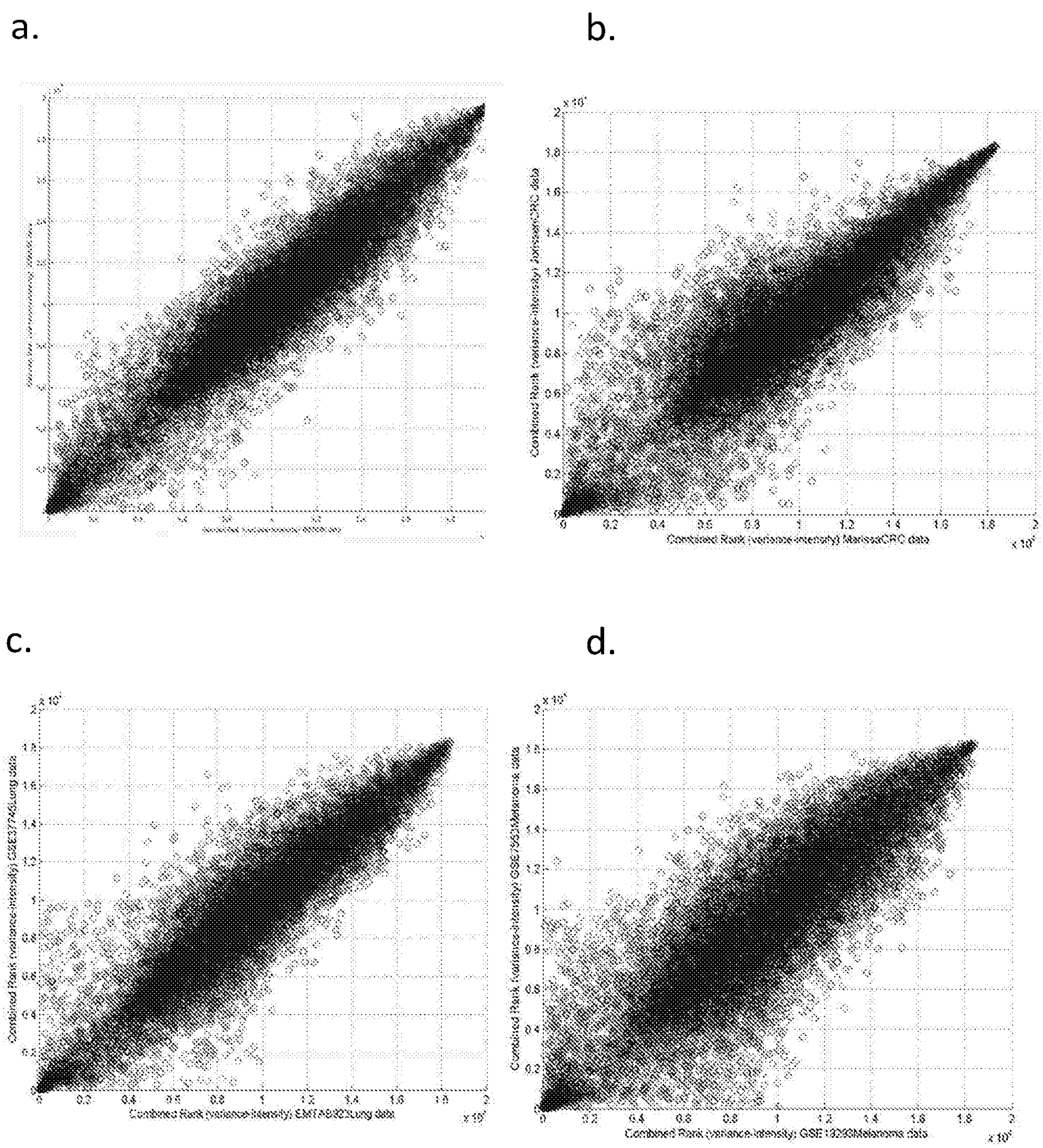


FIGURE 17

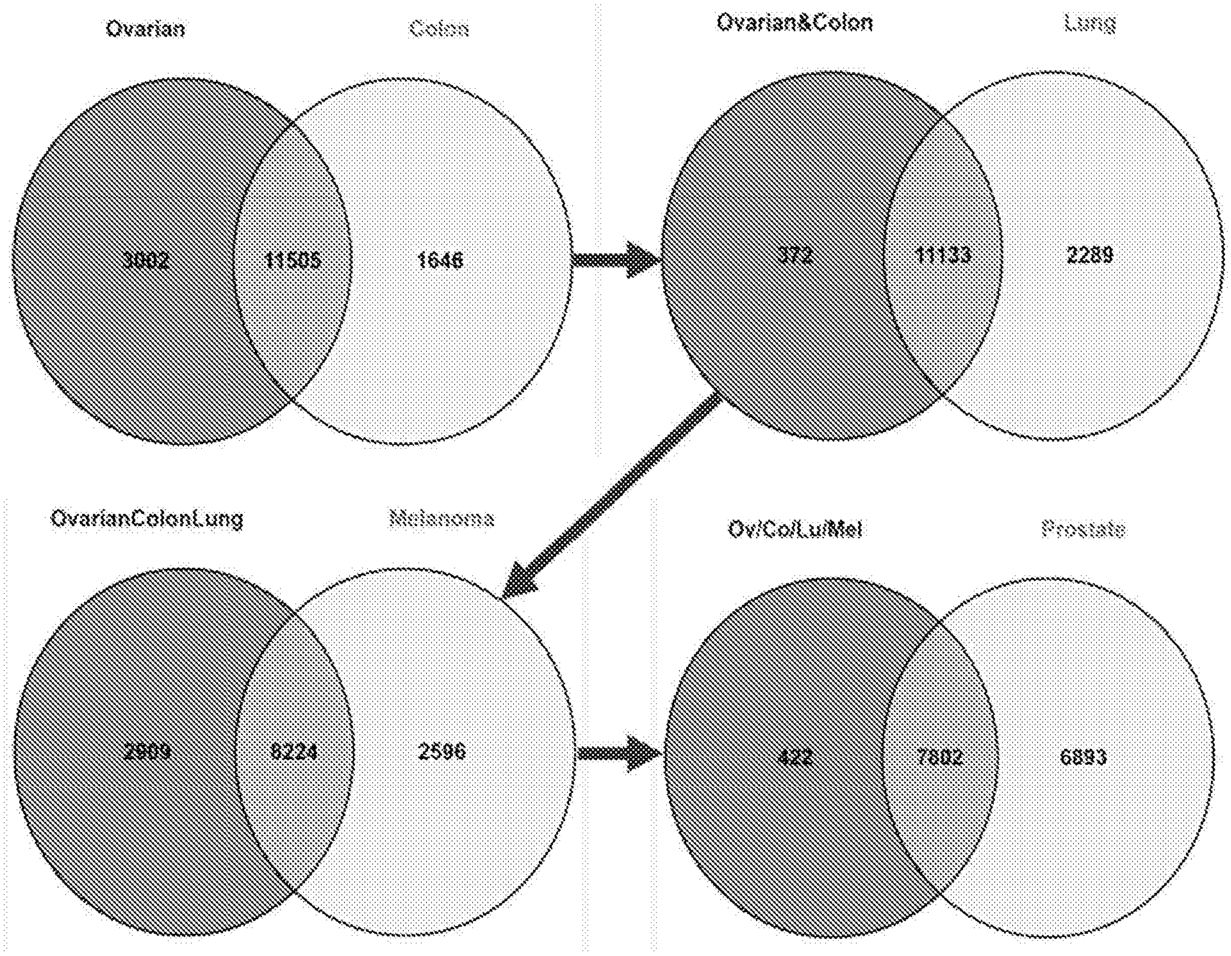
Ovarian
Colon
3002
11505
1646
Ovarian&Colon
Lung
372
11133
2289
OvarianColonLung
Melanoma
2909
8224
2596
Ov/Co/Lu/Mel
Prostate
422
7802
6893

FIGURE 18

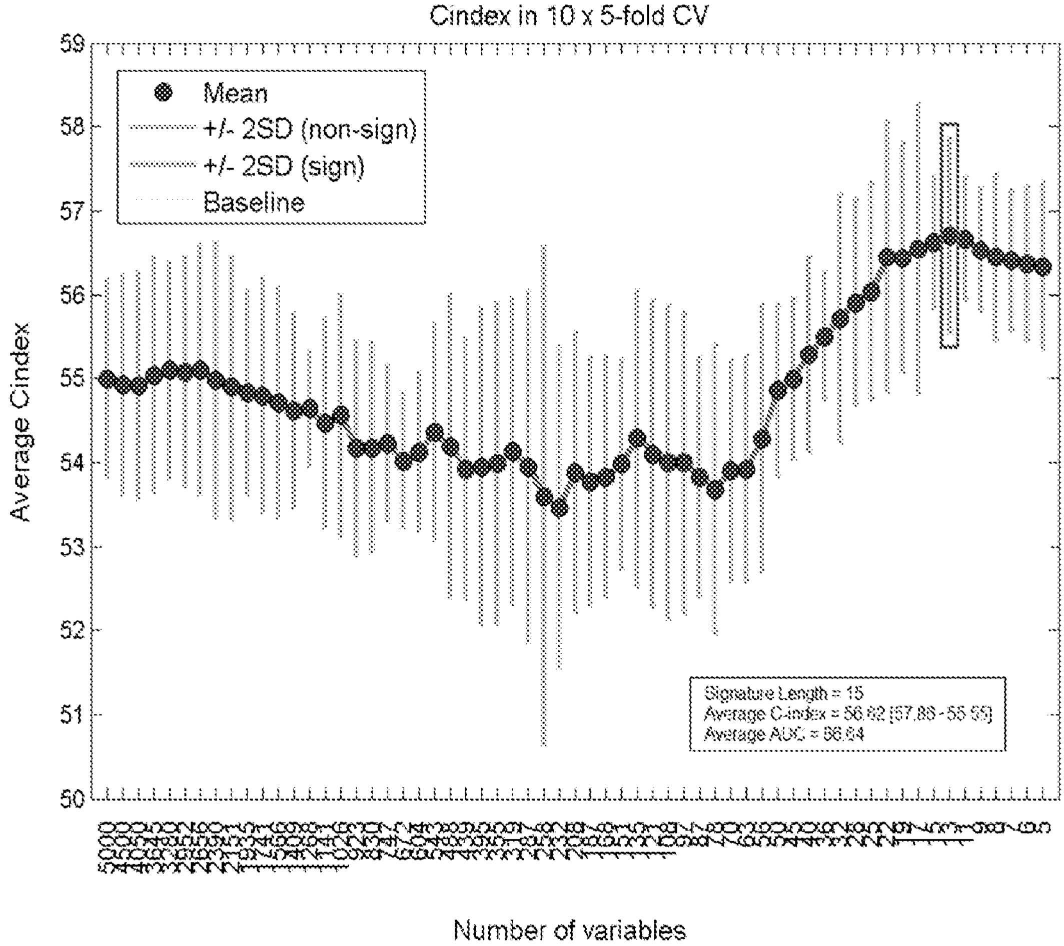
Cindex in 10 x 5-fold CV
59
58
57
56
55
54
53
52
51
50
Mean
+/- 2SD (non-sign)
+/- 2SD (sign)
Baseline
Average Cindex
Signature Length = 15
Average C-index = 56.62 [57.88 - 55.55]
Average AUC = 56.64
Number of variables

**FIGURE 19**

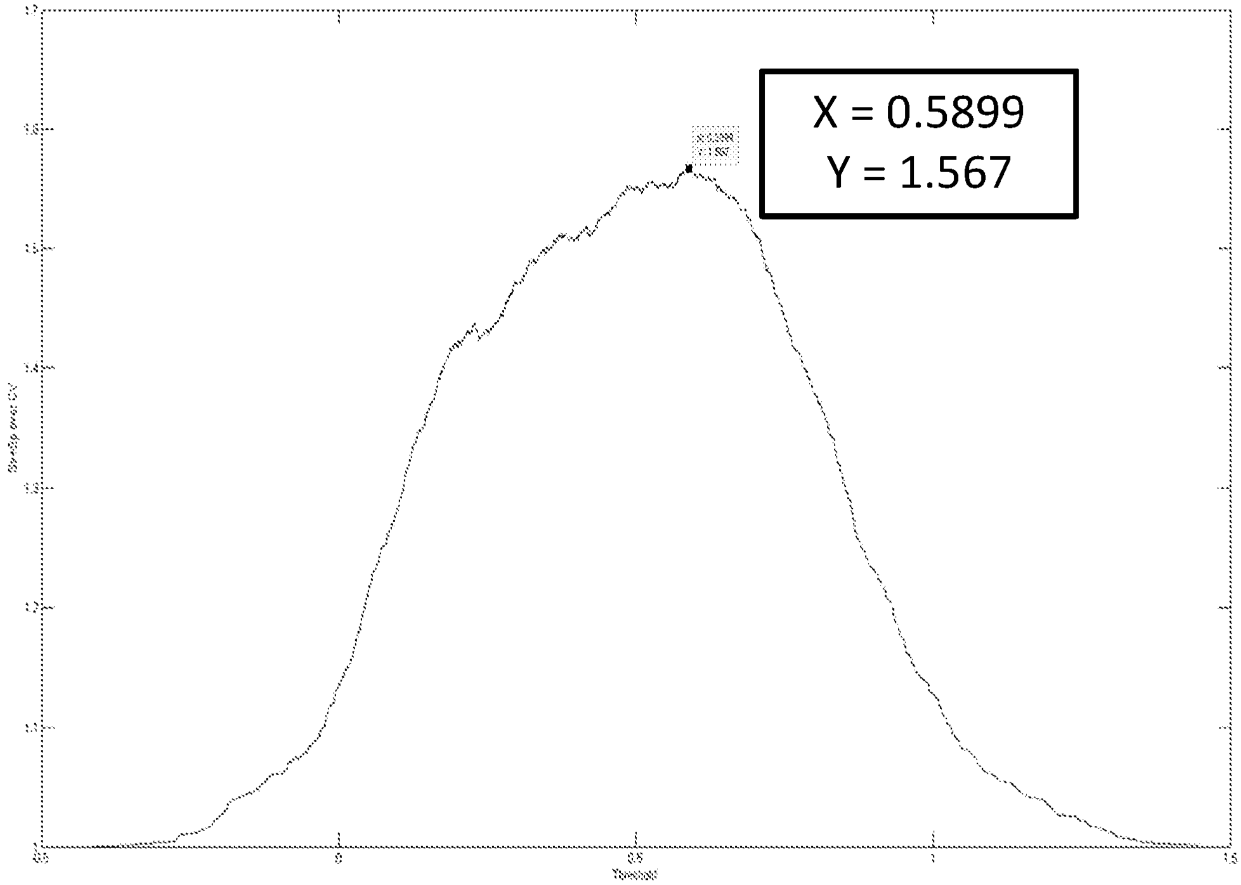


**FIGURE 20**

Functional analysis results All probe sets - HGS - Biological processes - FDR applied

| term | probe set cluster all | 1 | 2 | 3 | 4 |
|---|---|---|---|---|---|
| anatomical structure development | 6.35E-13 | 2.74E-01 | 3.13E-01 | 1.16E-01 | 7.5 |
| developmental process | 9.90E-13 | 2.26E-01 | 3.13E-01 | 1.68E-01 | 1.6 |
| response to chemical stimulus | 4.83E-12 | 4.27E-01 | 3.36E-01 | 2.13E-11 | 1.6 |
| system development | 1.91E-11 | 4.42E-01 | 3.13E-01 | 1.13E-01 | 3.8 |
| organ development | 1.43E-10 | 2.50E-01 | 2.40E-01 | 7.18E-02 | 1.9 |
| multicellular organismal development | 1.66E-10 | 4.13E-01 | 3.13E-01 | 1.26E-01 | 2.7 |
| cellular response to chemical stimulus | 5.04E-10 | 4.37E-01 | 4.21E-01 | 9.57E-13 | 2.3 |
| tissue development | 5.36E-10 | 5.81E-02 | 2.28E-01 | 4.25E-01 | 3.7 |
| collagen fibril organization | 7.72E-09 | 1.75E-01 | 6.09E-01 | | 1.9 |
| cell differentiation | 4.61E-09 | 1.79E-01 | 4.42E-01 | 1.03E-01 | 2.6 |

Functional analysis results Top10 probe set cluster 1 - HGS - Biological processes - FDR applied

| term | probe set cluster all | 1 | 2 | 3 | 4 |
|---|---|---|---|---|---|
| interphase of mitotic cell cycle | 2.23E-01 | 1.40E-02 | 8.21E-01 | 9.07E-01 | 9.9 |
| stem cell maintenance | 1.78E-02 | 1.50E-02 | 7.26E-01 | | 5.7 |
| interphase | 1.65E-01 | 1.50E-02 | 7.32E-01 | 9.10E-01 | 9.7 |
| G2/M transition of mitotic cell cycle | 3.16E-01 | 2.81E-02 | | | |
| negative regulation of macromolecule metabolic process | 4.80E-02 | 4.16E-02 | 5.68E-01 | 6.41E-01 | 5.8 |
| negative regulation of biosynthetic process | 5.37E-02 | 4.79E-02 | 5.75E-01 | 6.54E-01 | 5.5 |
| negative regulation of metabolic process | 8.99E-02 | 4.79E-02 | 5.93E-01 | 5.85E-01 | 7.1 |
| cellular response to zinc ion | 8.52E-02 | 4.79E-02 | | | |

**Only 8 significantly enriched processes**

Functional analysis results Top10 probe set cluster 2 - HGS - Biological processes - FDR applied

| term | probe set cluster all | 1 | 2 | 3 | 4 |
|---|---|---|---|---|---|

**No significantly enriched processes**

# FIGURE 21

| Functional analysis results Top10 probe set cluster 3 - HGS - Biological processes - FDR applied | | | | | |
|---|---|---|---|---|---|
| | probe set cluster | | | | |
| term | all | 1 | 2 | 3 | 4 |
| immune response | 1.54E-05 | 9.63E-01 | 4.74E-01 | 1.89E-26 | 3.8… |
| defense response | 7.75E-04 | 9.99E-01 | 5.68E-01 | 8.47E-25 | 4.3… |
| innate immune response | 6.05E-05 | 9.87E-01 | 5.91E-01 | 1.37E-24 | 5.4… |
| immune system process | 5.34E-06 | 6.29E-01 | 3.67E-01 | 8.23E-22 | 5.44E-… |
| cellular response to interferon-gamma | 1.86E-05 | | 8.52E-01 | 7.94E-20 | 7.50E-… |
| interferon-gamma-mediated signaling pathway | 2.82E-05 | | 8.15E-01 | 1.55E-19 | |
| response to interferon-gamma | 4.18E-05 | | 6.85E-01 | 9.24E-19 | 7.95E-… |
| cytokine-mediated signaling pathway | 3.70E-05 | 9.43E-01 | 5.93E-01 | 3.03E-17 | 2.0… |
| cellular response to cytokine stimulus | 3.57E-04 | 9.78E-01 | 6.92E-01 | 1.07E-16 | 2.0… |
| response to stress | 1.44E-06 | 7.02E-01 | 4.65E-01 | 1.22E-16 | 3.2… |

| Functional analysis results Top17 probe set cluster 4 - HGS - Biological processes - FDR applied | | | | | |
|---|---|---|---|---|---|
| | probe set cluster | | | | |
| term | all | 1 | 2 | 3 | 4 |
| anatomical structure development | 6.35E-13 | 2.74E-01 | 3.13E-01 | 1.16E-01 | 7.5… |
| developmental process | 9.90E-13 | 2.26E-01 | 3.13E-01 | 1.68E-01 | 1.6… |
| system development | 1.91E-11 | 4.47E-01 | 3.13E-01 | 1.13E-01 | 3.3… |
| multicellular organismal development | 1.66E-10 | 4.13E-01 | 3.13E-01 | 1.26E-01 | 7.7… |
| anatomical structure morphogenesis | 4.61E-09 | 2.08E-01 | 5.23E-01 | 4.45E-01 | 3.6… |
| biological adhesion | 4.03E-07 | 7.41E-01 | 5.83E-01 | 6.58E-02 | 5.2… |
| cell adhesion | 4.03E-07 | 7.41E-01 | 5.83E-01 | 6.58E-02 | 5.2… |
| locomotion | 7.06E-07 | 6.97E-01 | 4.52E-01 | 1.57E-01 | 1.6… |
| cellular component morphogenesis | 9.06E-06 | 3.34E-01 | 8.76E-01 | 4.00E-01 | 1.9… |
| multicellular organismal process | 2.06E-04 | 4.27E-01 | 3.55E-01 | 3.58E-02 | 4.3… |
| cell morphogenesis | 1.16E-05 | 3.34E-01 | 8.88E-01 | 3.53E-01 | 4.6… |
| vasculature development | 1.07E-04 | 8.43E-01 | 5.80E-01 | 5.66E-01 | 1.9… |

**FIGURE 21 CONTINUED**

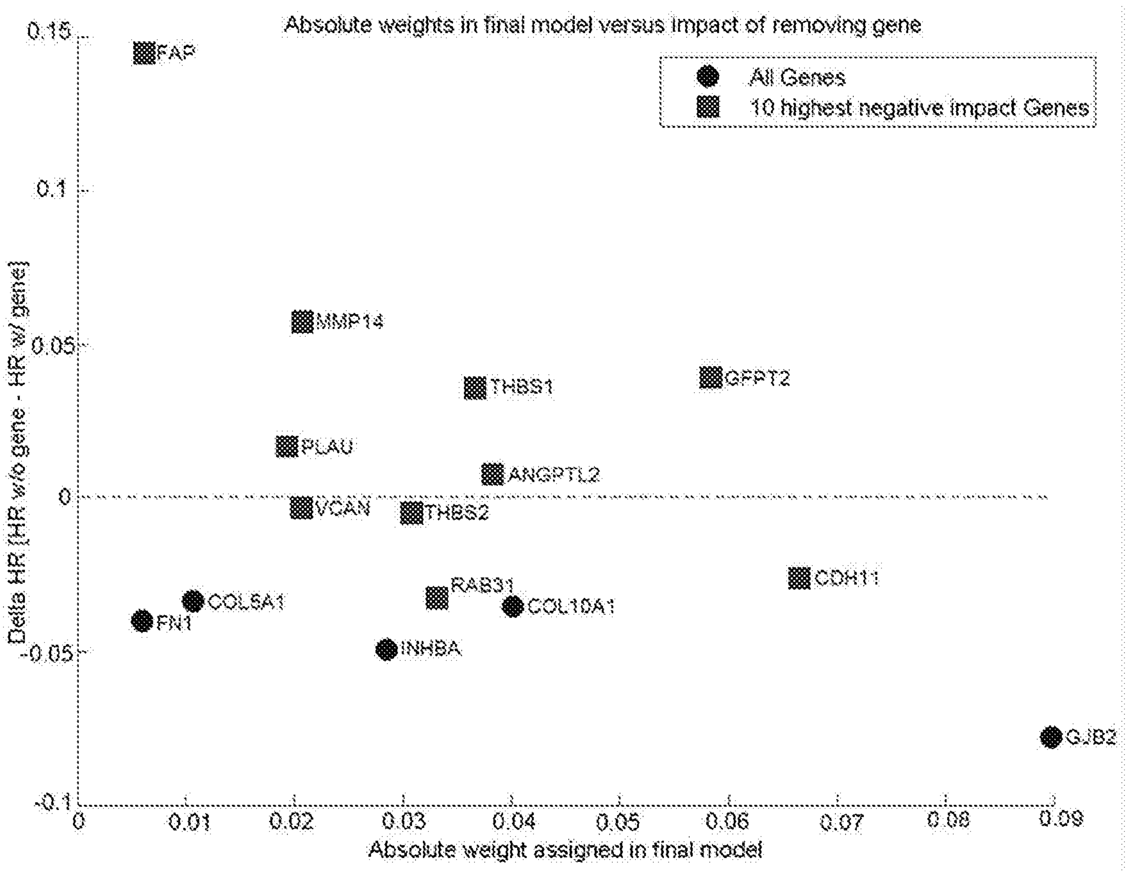
Absolute weights in final model versus impact of removing gene
All Genes
10 highest negative impact Genes
FAP
MMP14
THBS1
GFPT2
PLAU
ANGPTL2
VCAN
THBS2
CDH11
RAB31
COL5A1
COL10A1
FN1
INHBA
GJB2
Delta HR [HR w/o gene - HR w/ gene]
Absolute weight assigned in final model
0.15
0.1
0.05
0
-0.05
-0.1
0
0.01
0.02
0.03
0.04
0.05
0.06
0.07
0.08
0.09

FIGURE 22

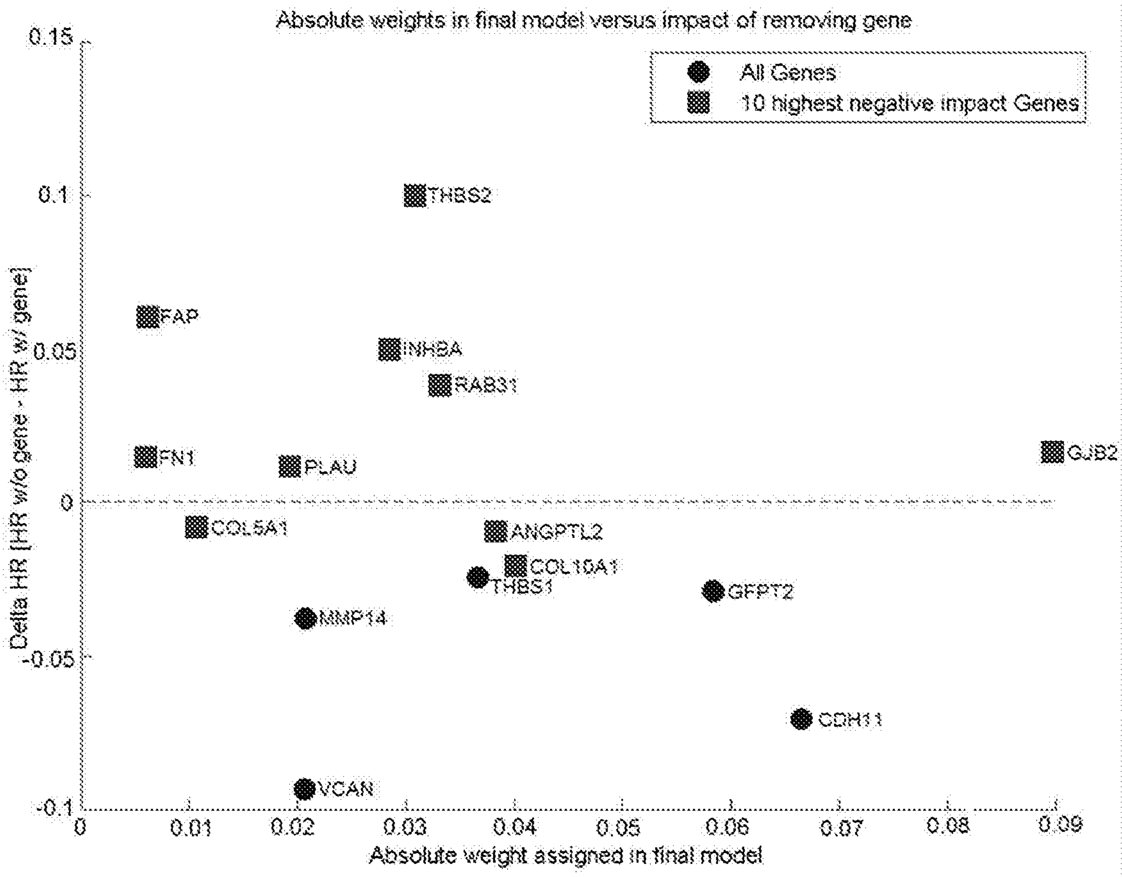
Absolute weights in final model versus impact of removing gene
All Genes
10 highest negative impact Genes
THBS2
FAP
INHBA
RAB31
FN1
PLAU
GJB2
COL5A1
ANGPTL2
COL10A1
THBS1
GFPT2
MMP14
CDH11
VCAN
Delta HR [HR w/o gene - HR w/ gene]
Absolute weight assigned in final model
0.15
0.1
0.05
0
-0.05
-0.1
0
0.01
0.02
0.03
0.04
0.05
0.06
0.07
0.08
0.09

# FIGURE 23

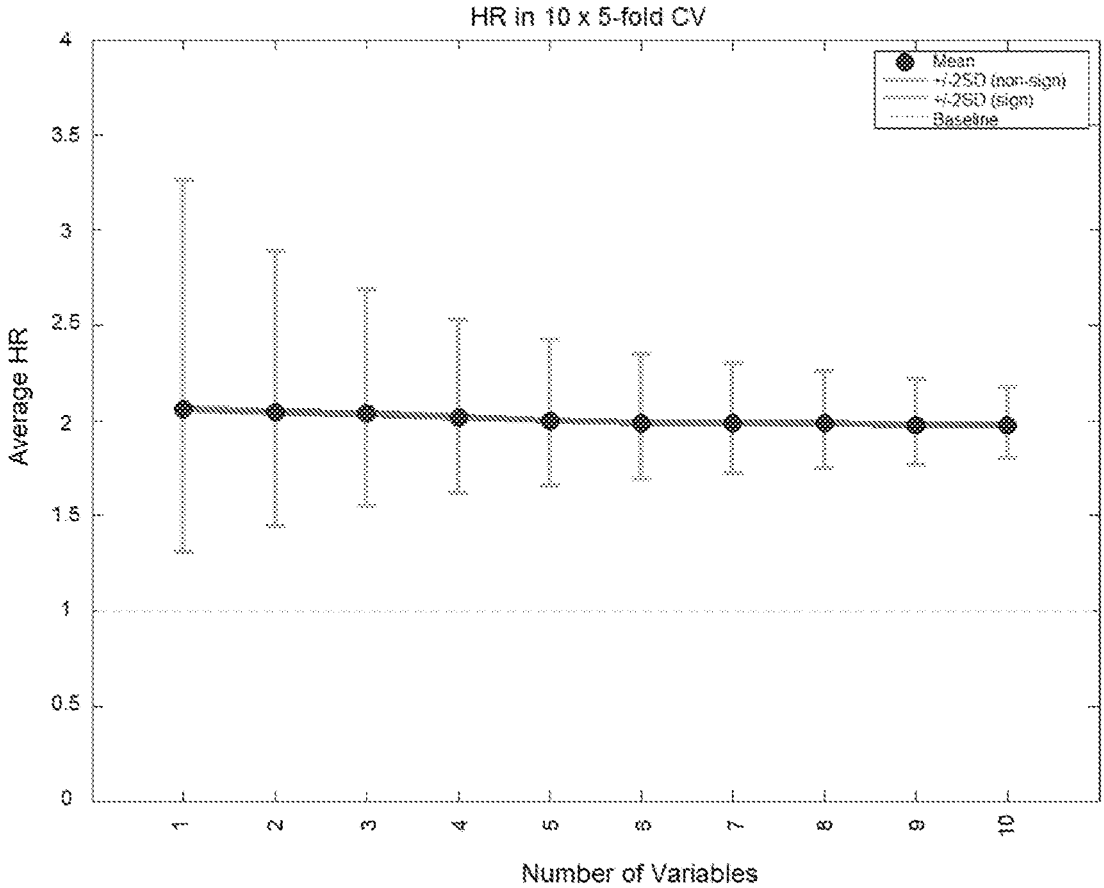
HR in 10 x 5-fold CV
Mean
+/-2SD (non-sign)
+/-2SD (sign)
Baseline
Average HR
Number of Variables
0
0.5
1
1.5
2
2.5
3
3.5
4
1
2
3
4
5
6
7
8
9
10

FIGURE 24

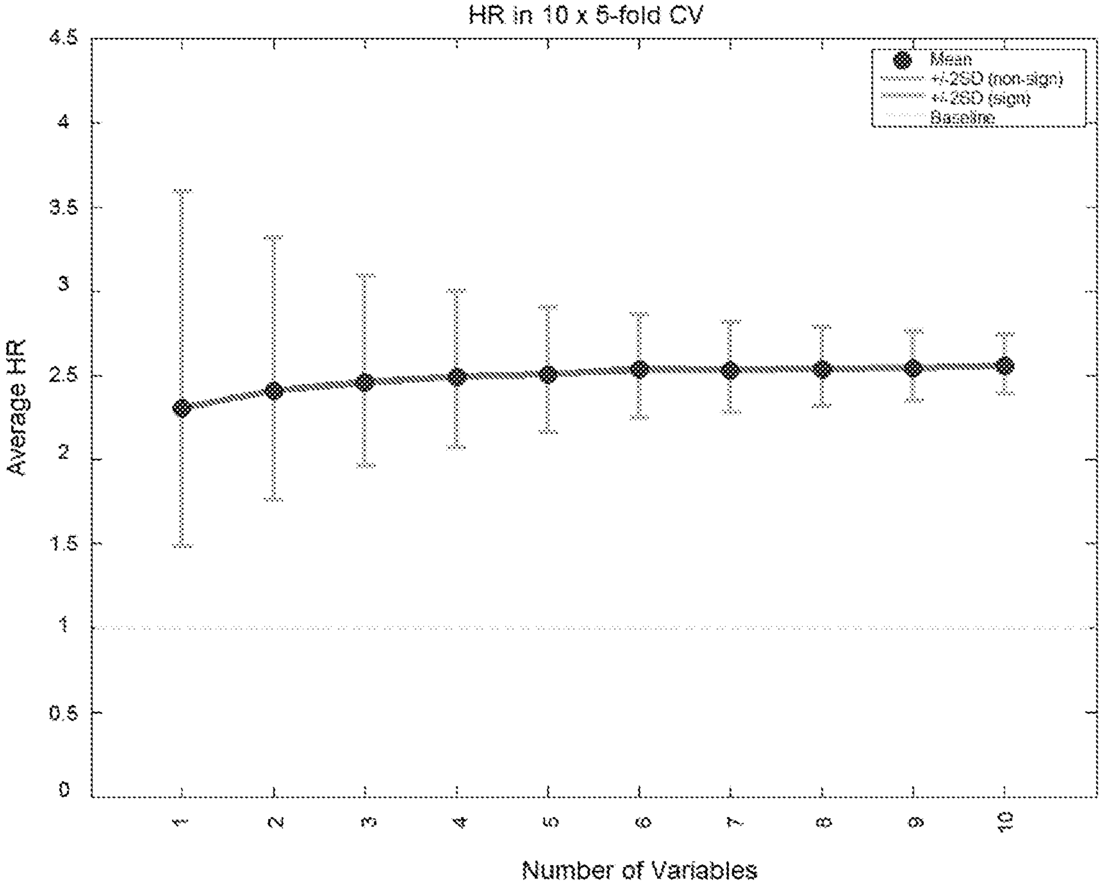
HR in 10 x 5-fold CV
Mean
+/-2SD (non-sign)
+/-2SD (sign)
Baseline
Average HR
0
0.5
1
1.5
2
2.5
3
3.5
4
4.5
1
2
3
4
5
6
7
8
9
10
Number of Variables

FIGURE 25

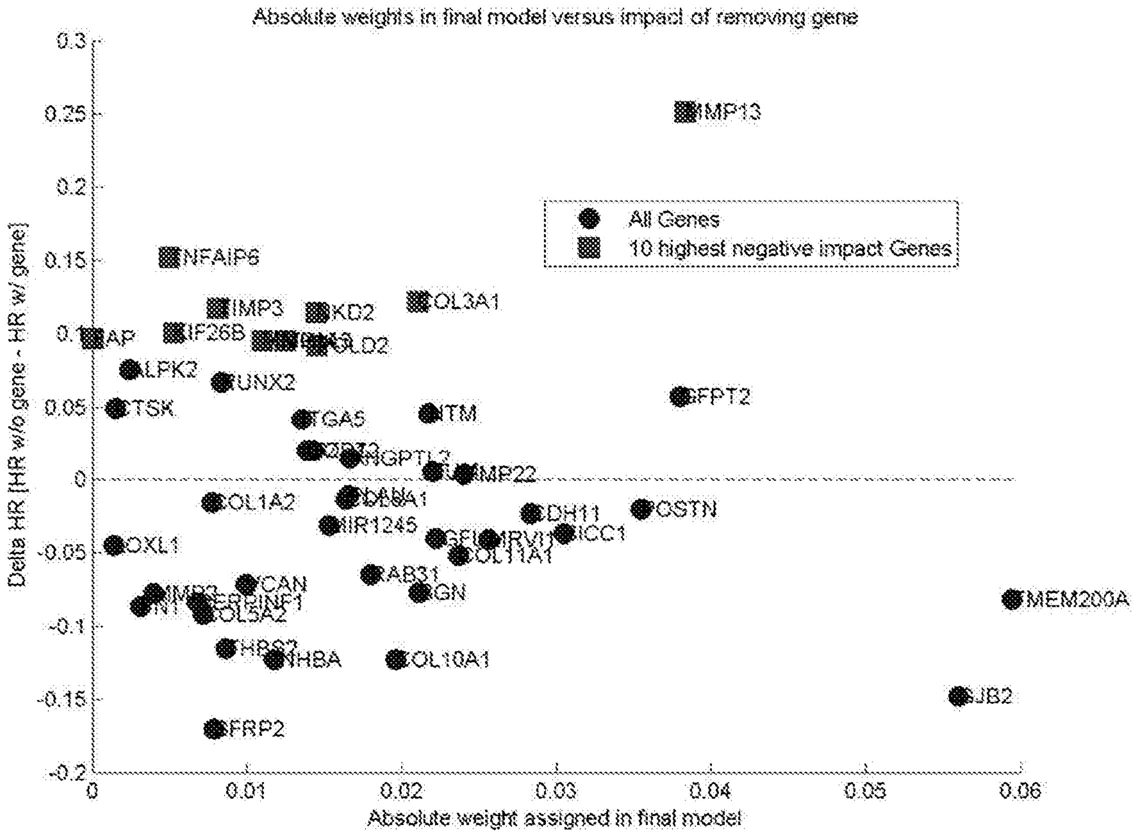
Absolute weights in final model versus impact of removing gene
Delta HR [HR w/o gene - HR w/ gene]
Absolute weight assigned in final model
All Genes
10 highest negative impact Genes
0.3
0.25
0.2
0.15
0.1
0.05
0
-0.05
-0.1
-0.15
-0.2
0
0.01
0.02
0.03
0.04
0.05
0.06
MMP13
TNFAIP6
TIMP3
COL3A1
CTSK
RUNX2
ITGA5
COL1A2
CDH11
POSTN
TMEM200A
GJB2
SFRP2
COL10A1
INHBA
COL11A1
COL5A2
SERPINF1
VCAN
RAB31

# FIGURE 26

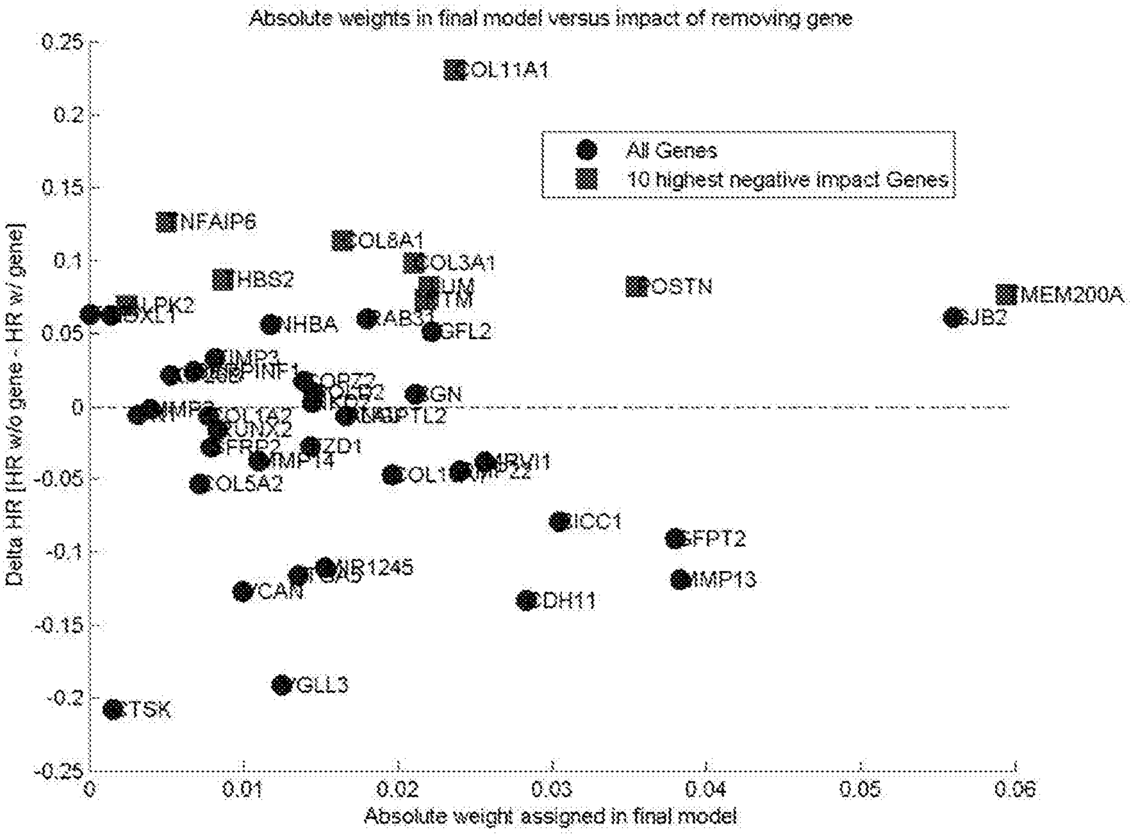
Absolute weights in final model versus impact of removing gene
0.25
0.2
0.15
0.1
0.05
0
-0.05
-0.1
-0.15
-0.2
-0.25
Delta HR [HR w/o gene - HR w/ gene]
0
0.01
0.02
0.03
0.04
0.05
0.06
Absolute weight assigned in final model
All Genes
10 highest negative impact Genes
OL11A1
NFAIP6
OL8A1
OL3A1
HBS2
OSTN
MEM200A
NHBA
GFL2
JB2
GN
ZD1
MP14
OL5A2
ICC1
FPT2
IMP13
CDH11
CAN
GLL3
TSK

# FIGURE 27

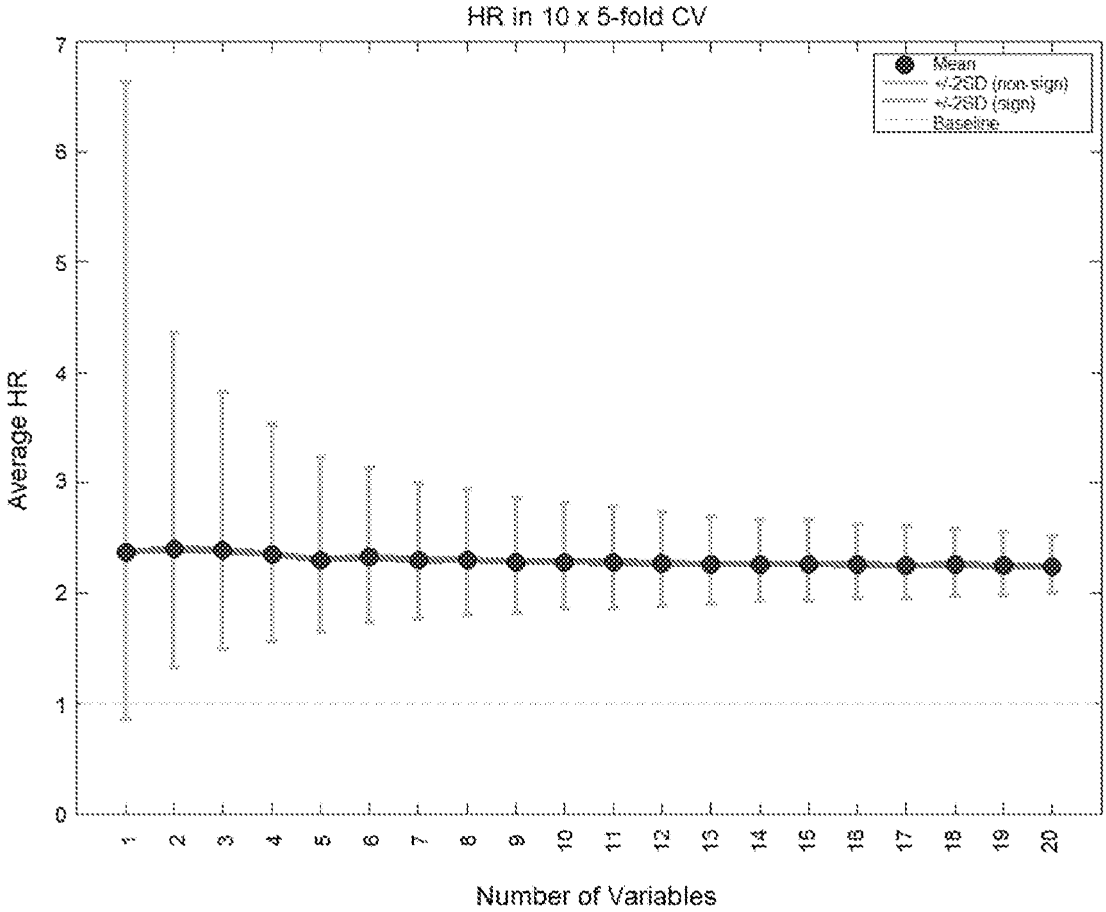
HR in 10 x 5-fold CV
Mean
+/-2SD (non-sign)
+/-2SD (sign)
Baseline
Average HR
0
1
2
3
4
5
6
7
1
2
3
4
5
6
7
8
9
10
11
12
13
14
15
16
17
18
19
20
Number of Variables

# FIGURE 28

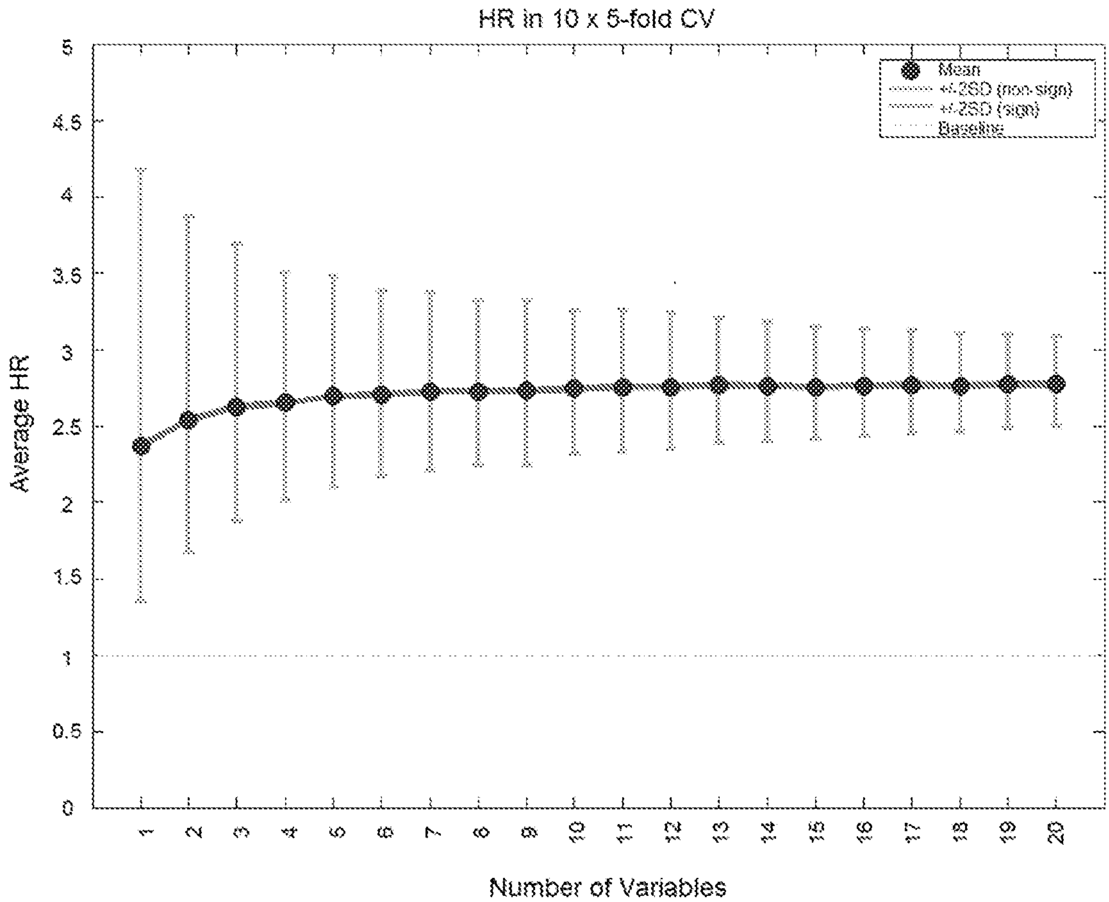
HR in 10 x 5-fold CV
Mean
+/-2SD (non-sign)
+/-2SD (sign)
Baseline
Average HR
Number of Variables
0
0.5
1
1.5
2
2.5
3
3.5
4
4.5
5
1
2
3
4
5
6
7
8
9
10
11
12
13
14
15
16
17
18
19
20

# FIGURE 29

A. ALM201 reverses EMT and downregulates MAPK pathway activation in Kuramochi cells

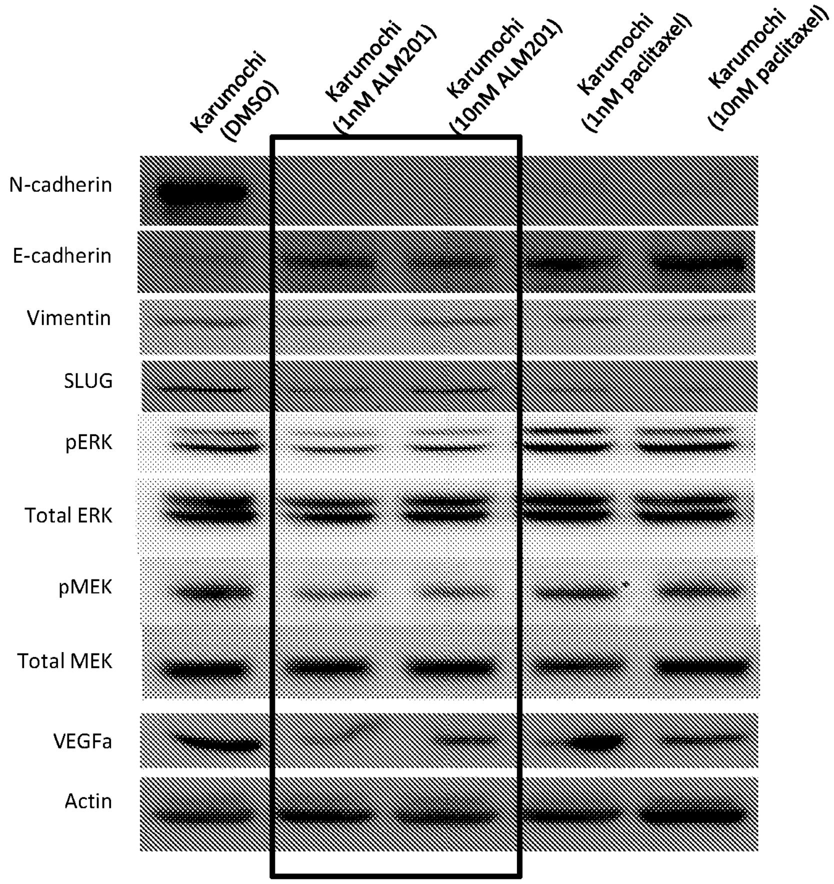


# FIGURE 30

B. ALM201 reverses EMT and downregulates MAPK pathway activation in OVCAR3 cisplatin resistant cells

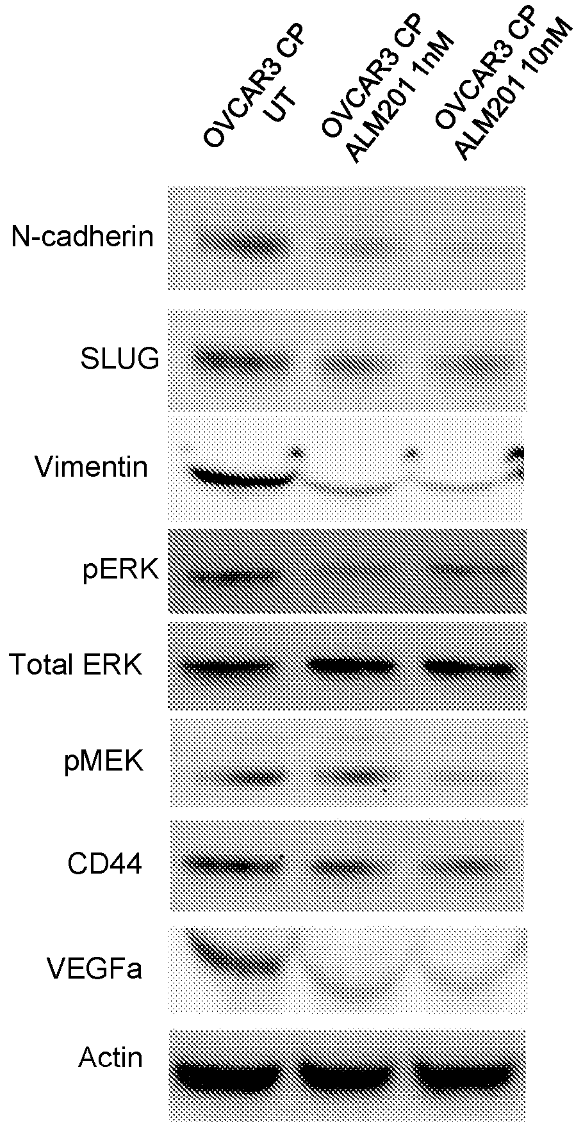


# FIGURE 30 CONTINUED

c. ALM201 reduces colony formation in OVCAR3 and OVCAR4 cisplatin resistant cells

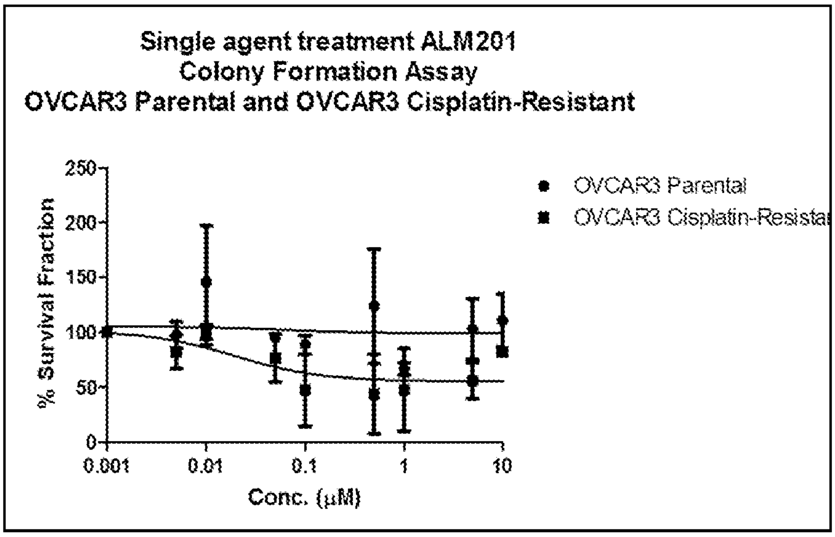


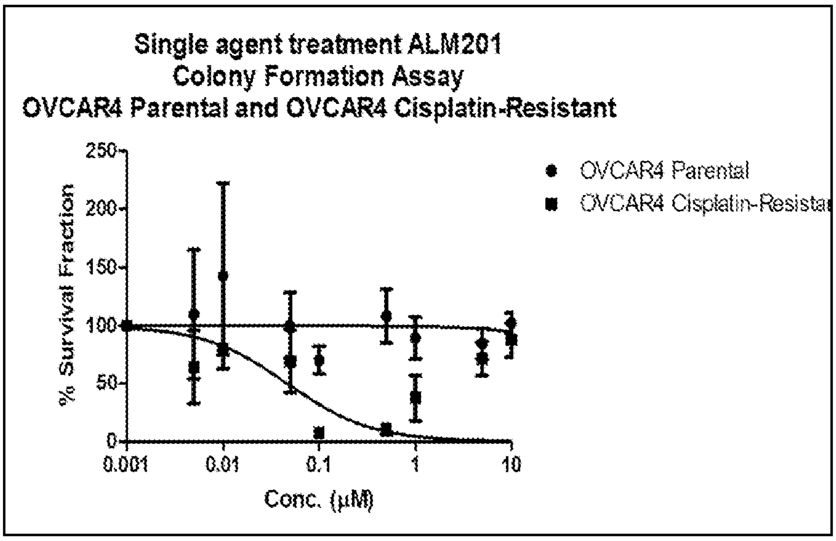


| Cell Lines | IC50 µM |
|---|---|
| OVCAR3 WT | >10 |
| OVCAR3 CP | 0.020 |
| OVCAR4 WT | >10 |
| OVCAR4 CP | 0.317 |

**FIGURE 30 CONTINUED**

d. ALM201 inhibits Migration and Invasion of the OVCAR3 platinum resistant cell lines

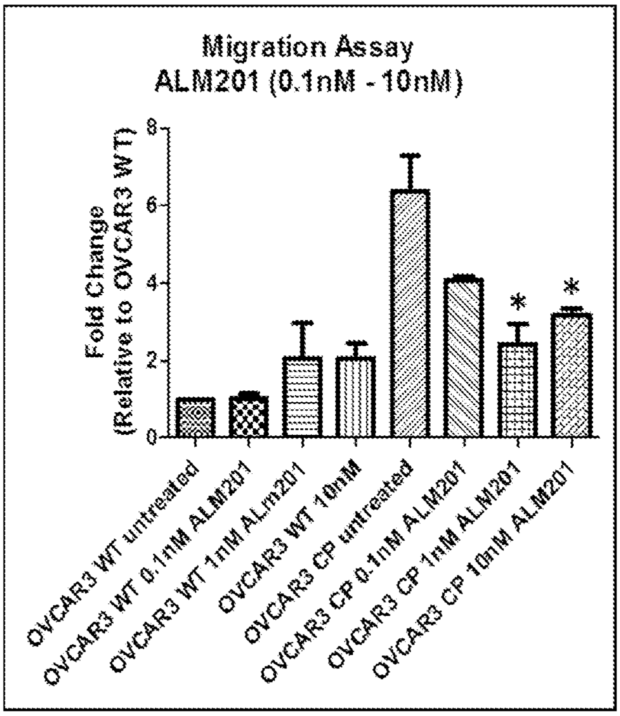


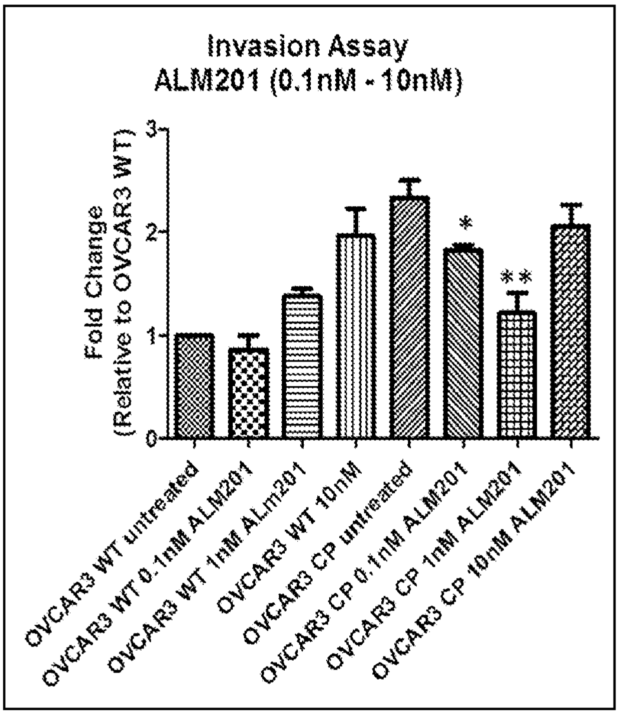


## FIGURE 30 CONTINUED

**A. Trametinib: External 'The Genomics of Drug Sensitivity in Cancer Project' cell line data**

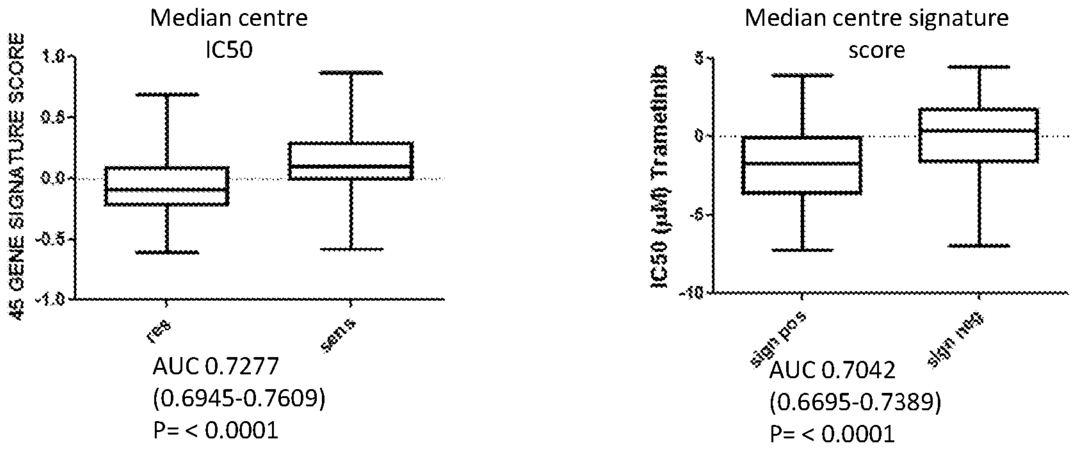


**B. Selumetinib: External 'The Genomics of Drug Sensitivity in Cancer Project' cell line data**

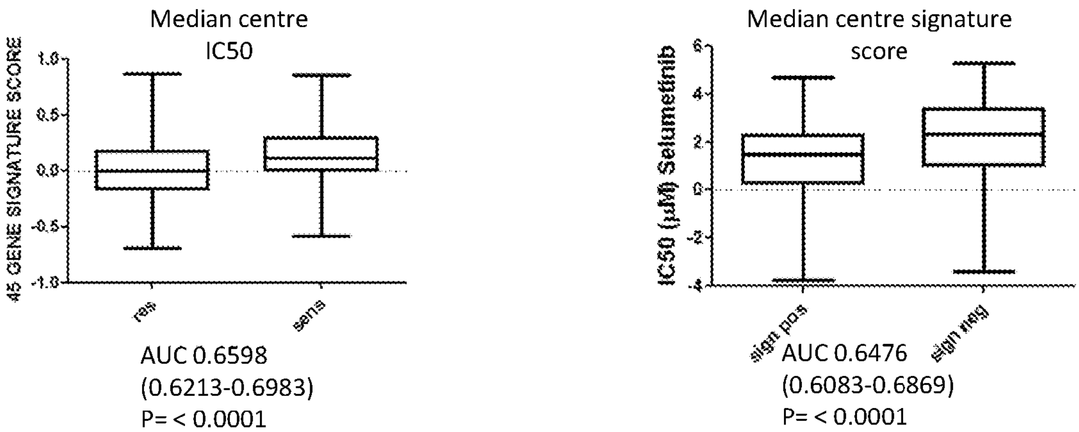


# FIGURE 31

**C. Trametinib: External 'The Genomics of Drug Sensitivity in Cancer Project' cell line data**

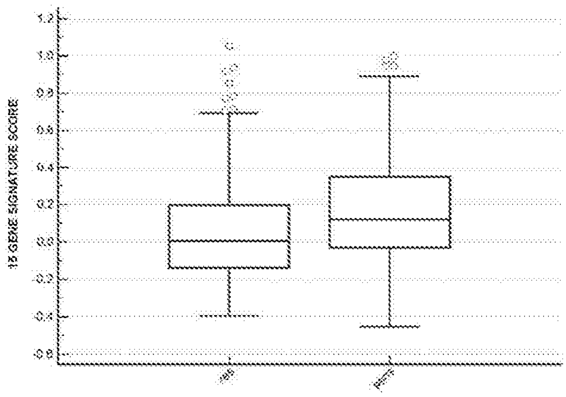

AUC = 0.629
(0.593 to 0.664)
P<0.0001

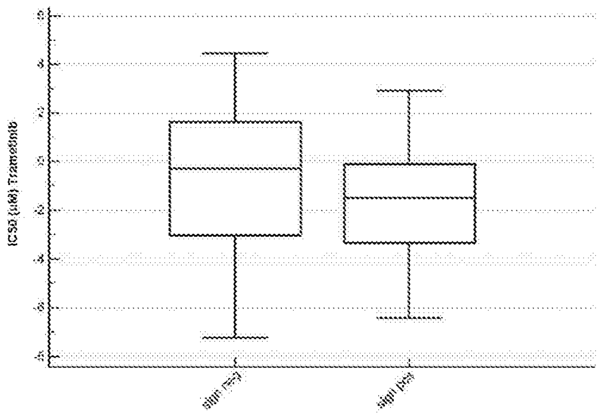

AUC = 0.627
(0.591 to 0.662)
P<0.0001

**D. Selumetinib: External 'The Genomics of Drug Sensitivity in Cancer Project' cell line data**

Median centre
IC50

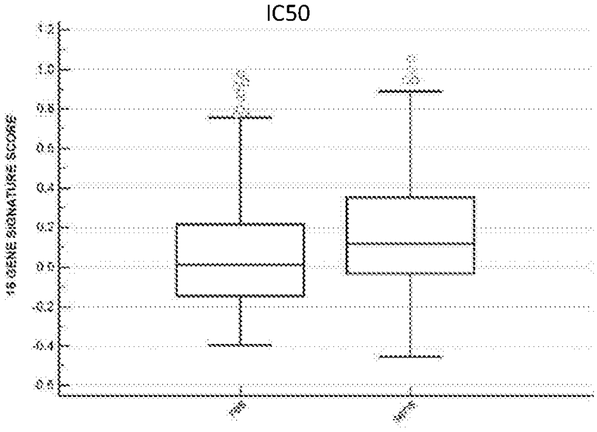

AUC = 0.619
(0.584 to 0.654)
P<0.0001

Median centre signature
score

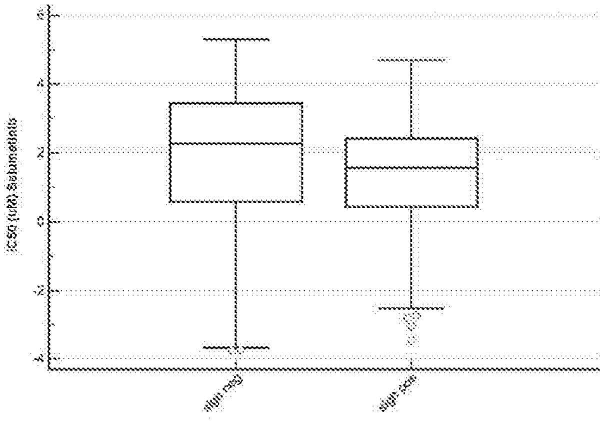

AUC = 0.609
(0.573 to 0.644)
P<0.0001

# FIGURE 31 CONTINUED

a.

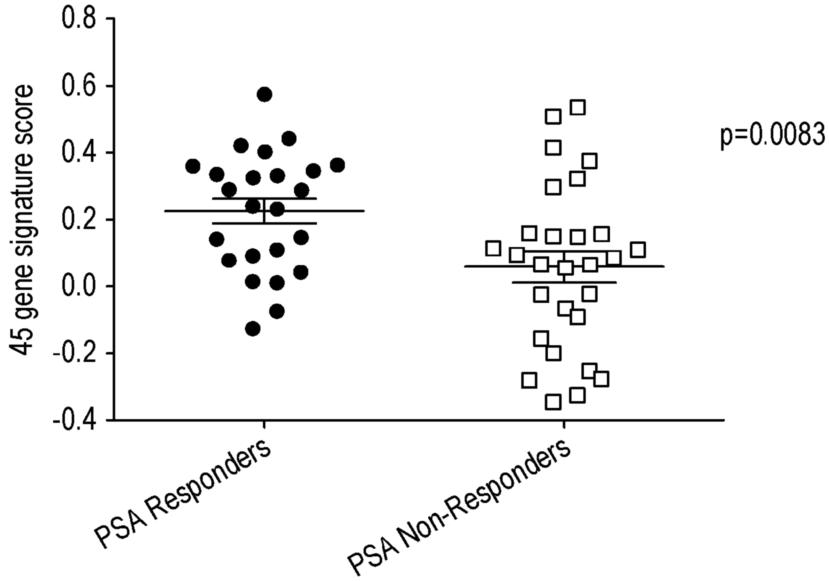


b.

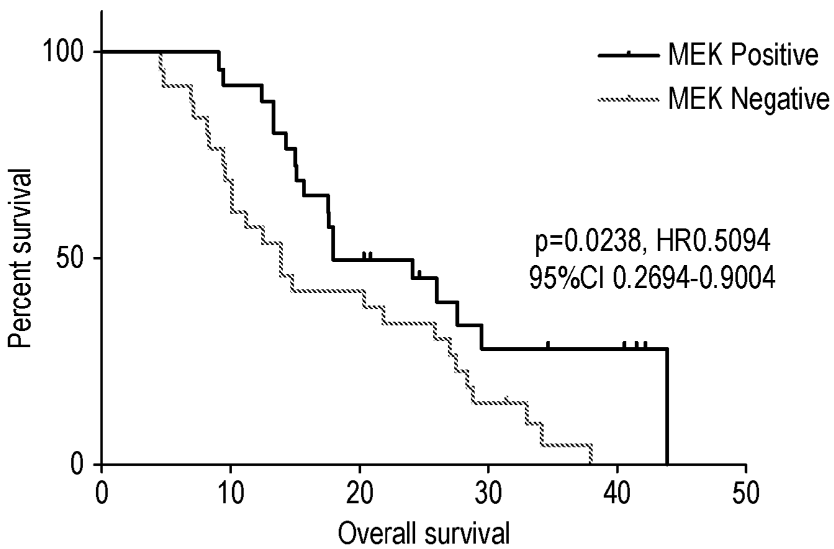


c.

| | PSA Responder | PSA Non-Responder | Total |
|---|---|---|---|
| MEK positive | 16 | 10 | 26 |
| MEK negative | 8 | 16 | 24 |
| | 24 | 28 | 50 |

Finishers Exact p=0.0505

# FIGURE 32

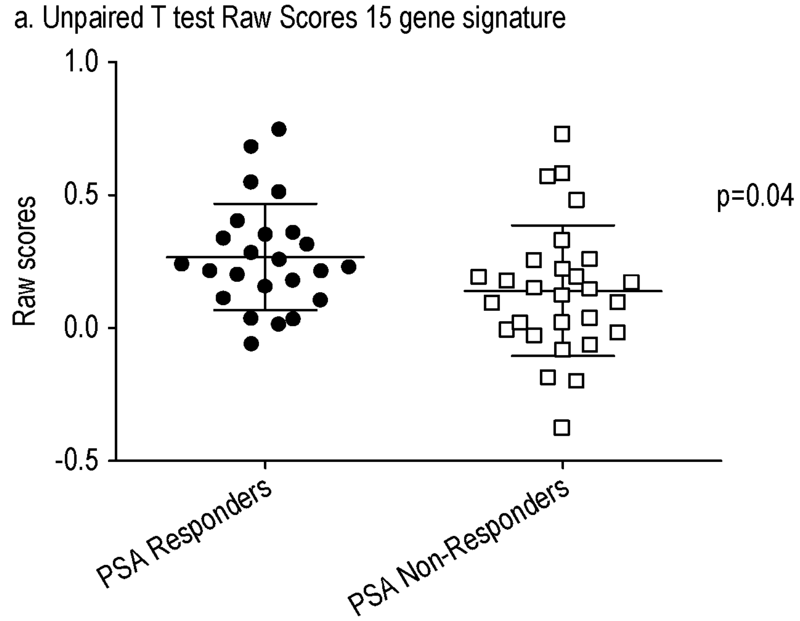


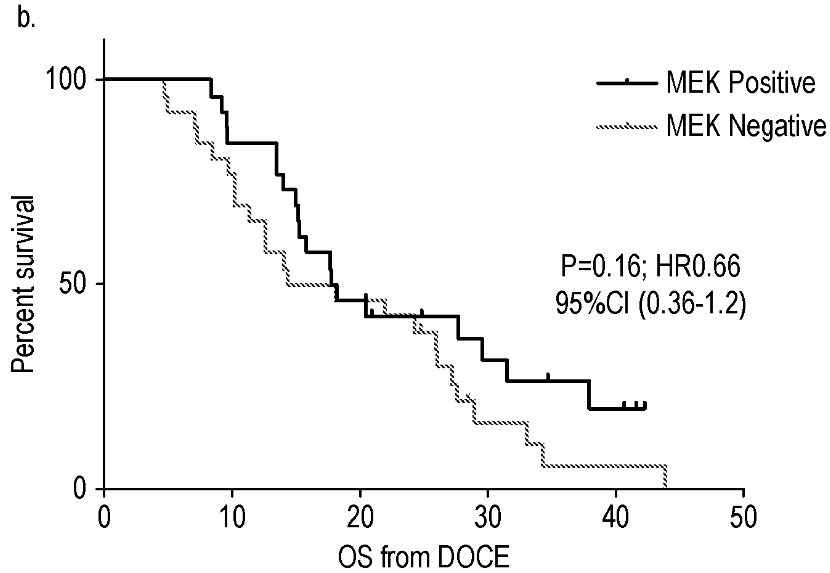


c.

| | PSA Responder | PSA Non-Responder | Total |
|---|---|---|---|
| MEK positive | 16 | 10 | 26 |
| MEK negative | 8 | 16 | 24 |
| | 24 | 28 | 50 |

Finishers Exact p=0.0505

**FIGURE 33**

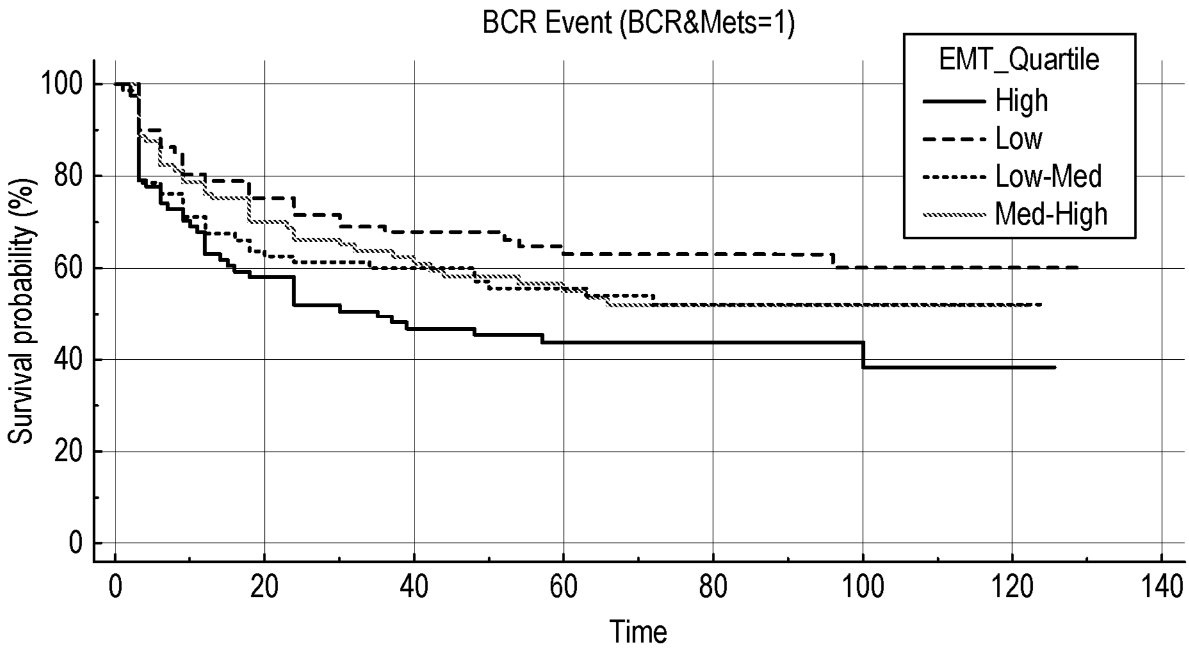


Number at risk

| | | | | | | | |
|---|---|---|---|---|---|---|---|
| Group: High | | | | | | | |
| 81 | 47 | 35 | 28 | 19 | 7 | 1 | 0 |
| Group: Low | | | | | | | |
| 81 | 61 | 44 | 40 | 35 | 14 | 3 | 0 |
| Group: Low-Med | | | | | | | |
| 80 | 50 | 41 | 34 | 26 | 7 | 3 | 0 |
| Group: Med-High | | | | | | | |
| 80 | 56 | 43 | 35 | 24 | 7 | 1 | 0 |

HR=1.8095 [1.1499-2.8474]; p=0.0658

**FIGURE 34**

A.

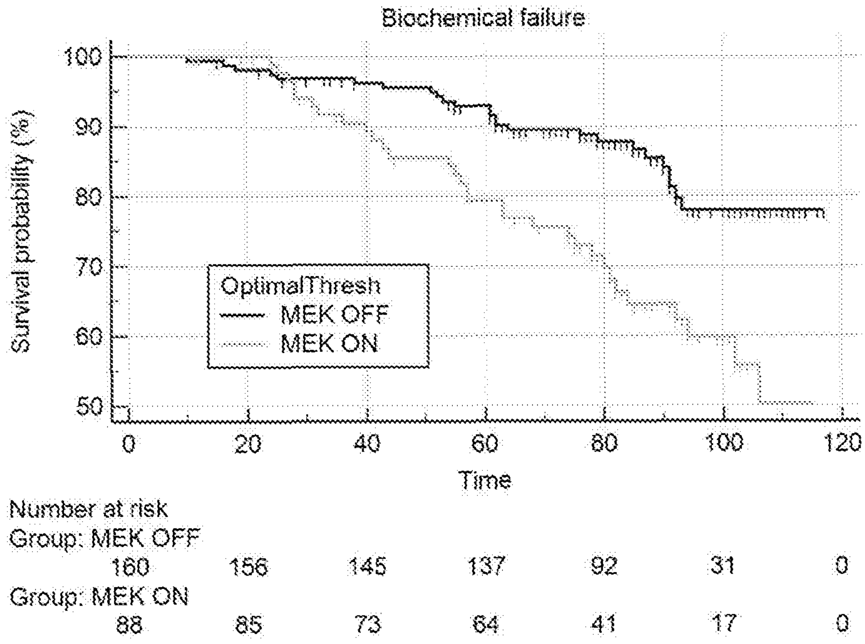


HR = 2.4924 [1.4293-4.3463]; p=0.004

B.

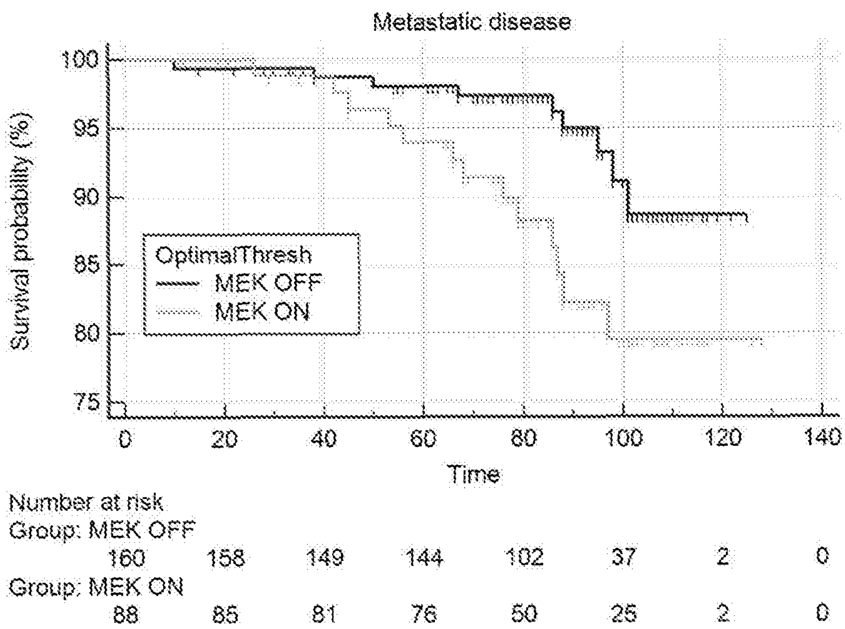


HR = 2.6351 [1.0998-6.3137]; p=0.020

**FIGURE 35**

A.

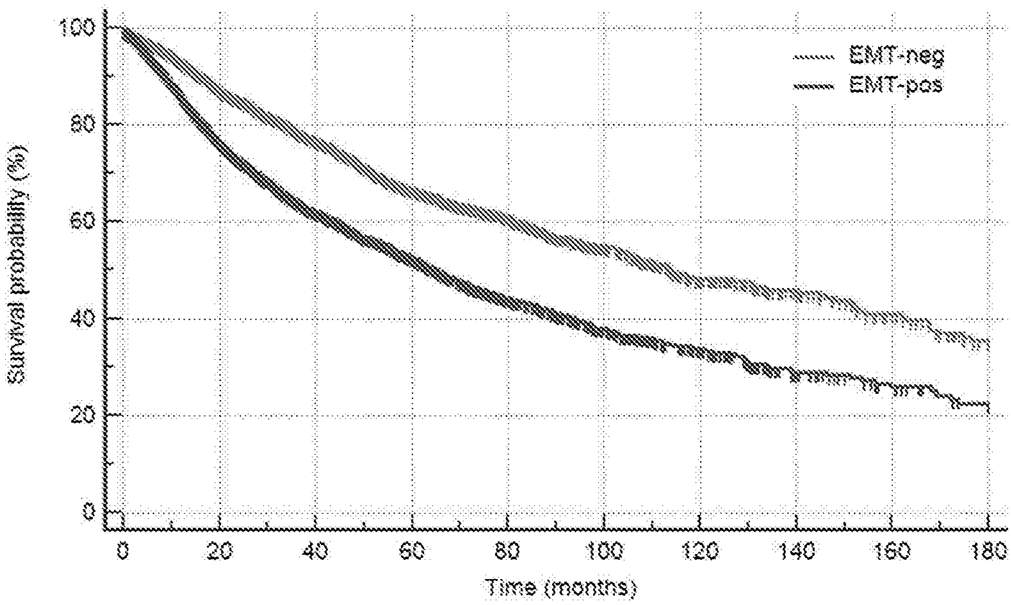


B.

| | Hazard ratio | P-value |
|---|---|---|
| All diseases | 1.78 [1.65-1.92] | <0.0001 |
| Bladder** | 1.63 [1.22-2.18] | 0.0011 |
| Cervical** | 2.16 [1.16-4.04] | 0.016 |
| Colorectal** | 1.83 [1.04-3.25] | 0.039 |
| Glioblastoma** | 2.12 [1.17-3.83] | 0.01 |
| Head and Neck** | 1.54 [1.02-2.30] | 0.039 |
| Renal clear cell** | 4.12 [2.58-6.57] | <0.0001 |
| Renal papillary** | 2.98 [1.12-7.93] | 0.029 |
| Lower grade glioma** | 2.42 [1.22-4.81] | 0.012 |
| Lung adeno** | 1.77 [1.21-2.60] | 0.003 |
| Pancreatic** | 2.58 [1.52-4.38] | 0.0005 |
| Melanoma** | 1.42 [1.07-1.88] | 0.015 |
| Ovarian** | 1.36 [0.99-1.86] | 0.050 |
| Stomach** | 1.73 [1.09-2.75] | 0.020 |

# FIGURE 36

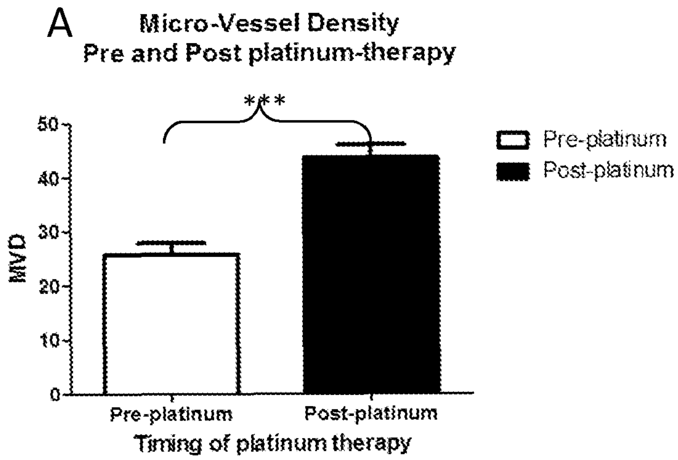

P-value: <0.0001

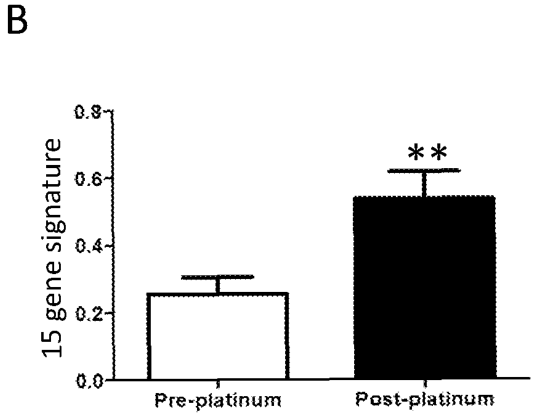

P-value: 0.0094

**FIGURE 37**

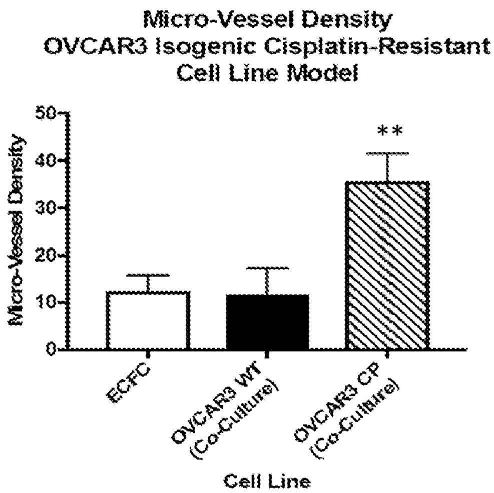


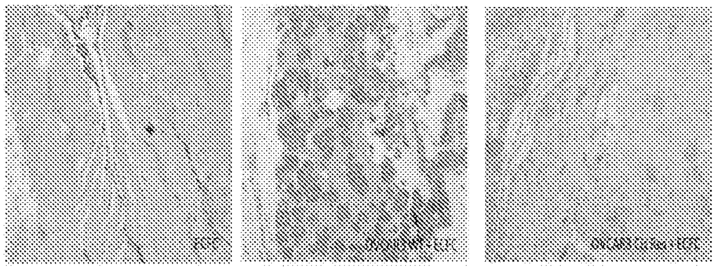

| Cell Line | P-Value (95% CI) |
|---|---|
| OVCAR3 WT (Co-Culture) | 0.8712 (-11.38 to 10.05) |
| OVCAR3 CP (Co-culture) | 0.0041 (12.38 to 34.28) |

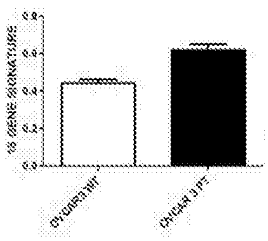

P-value: 0.046

**FIGURE 38**

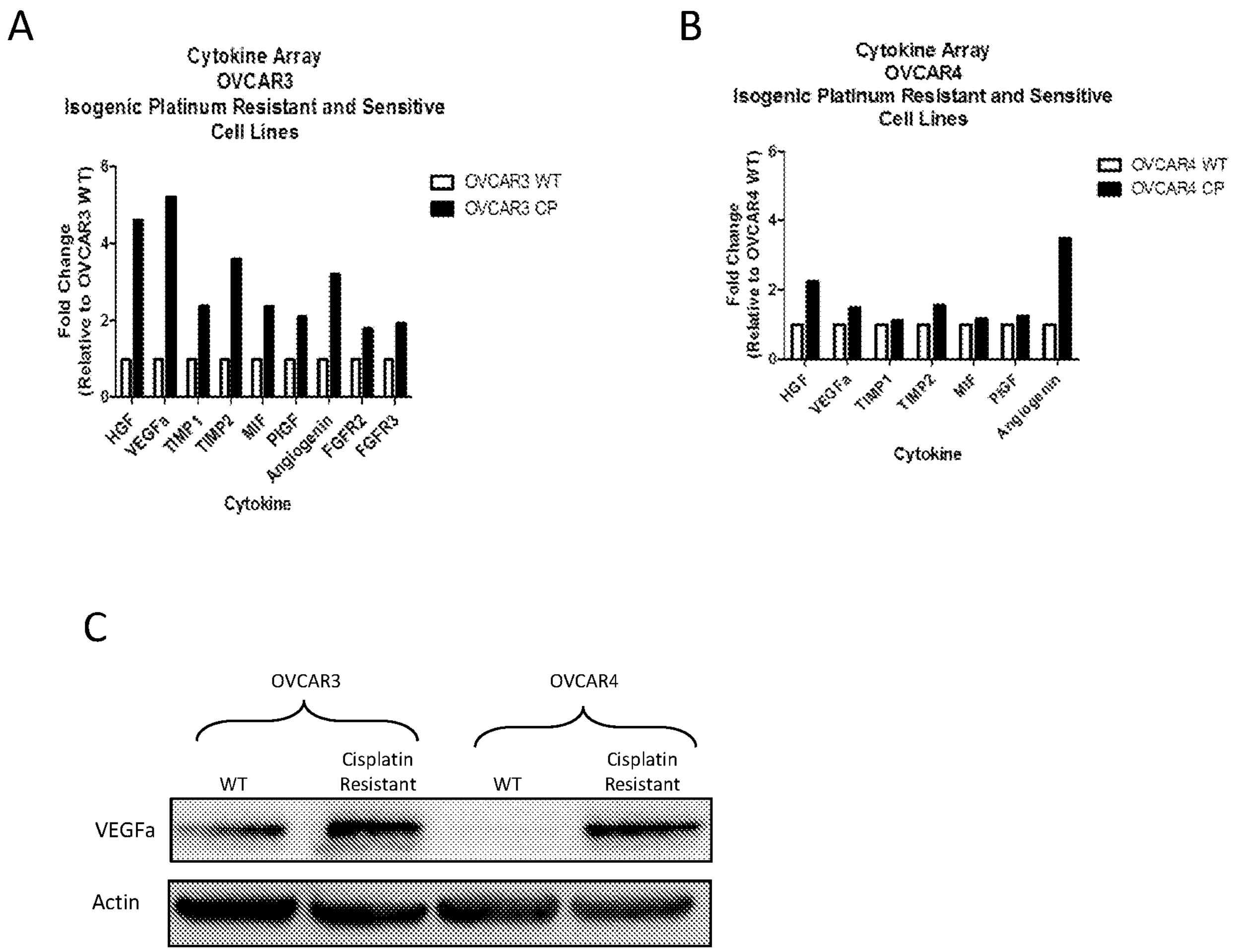
A
Cytokine Array
OVCAR3
Isogenic Platinum Resistant and Sensitive
Cell Lines
Fold Change
(Relative to OVCAR3 WT)
OVCAR3 WT
OVCAR3 CP
HGF
VEGFa
TIMP1
TIMP2
MIF
PlGF
Angiogenin
FGFR2
FGFR3
Cytokine
B
Cytokine Array
OVCAR4
Isogenic Platinum Resistant and Sensitive
Cell Lines
Fold Change
(Relative to OVCAR4 WT)
OVCAR4 WT
OVCAR4 CP
HGF
VEGFa
TIMP1
TIMP2
MIF
PlGF
Angiogenin
Cytokine
C
OVCAR3
OVCAR4
WT
Cisplatin Resistant
WT
Cisplatin Resistant
VEGFa
Actin

# FIGURE 39

A

| Gene | Gene Function | AUC CI | P VALUE |
|---|---|---|---|
| PDGFRA | Receptor Tyrosine Kinase | 0.7783 (0.7224 to 0.8342) | P= < 0.0001 |
| PDGFRB | Receptor Tyrosine Kinase | 0.7978 (0.7432 to 0.8523) | P= < 0.0001 |
| FGFR2 | Receptor Tyrosine Kinase | 0.594 (0.5259 to 0.6622) | P= 0.008683 |
| FGFR4 | Receptor Tyrosine Kinase | 0.5954 (0.5268 to 0.6630) | P= 0.008085 |
| VEGFR1 | Receptor Tyrosine Kinase | 0.6707 (0.6053 to 0.7363) | P= < 0.0001 |
| VEGFR2 | Receptor Tyrosine Kinase | 0.6543 (0.5866 to 0.7220) | P= < 0.0001 |
| EGFR | Receptor Tyrosine Kinase | 0.5706 (0.5051 to 0.6398) | P= 0.04859 |
| MET | Receptor Tyrosine Kinase | 0.5794 (0.5095 to 0.6494) | P= 0.02659 |
| EPHA4 | Receptor Tyrosine Kinase | 0.6337 (0.5662 to 0.7012) | P= 0.00019 |
| FZD1 | Receptor Tyrosine Kinase | 0.772 (0.7155 to 0.8286) | P= < 0.0001 |
| AXL | Receptor Tyrosine Kinase | 0.6652 0.6040 to 0.7348) | P= < 0.0001 |

B

| Gene | Gene Function | P VALUE |
|---|---|---|
| PDGFRA | Receptor Tyrosine Kinase | P= 0.007074 |
| PDGFRB | Receptor Tyrosine Kinase | P= 0.004868 |

# FIGURE 40

C

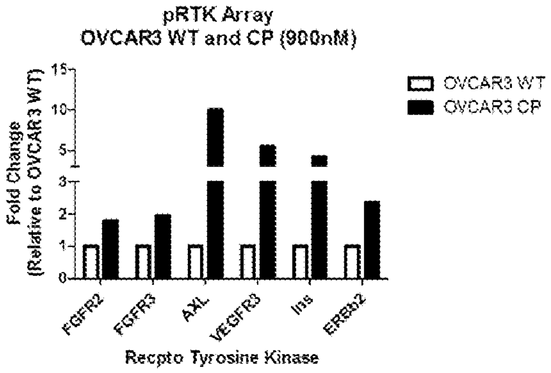
pRTK Array
OVCAR3 WT and CP (900nM)
Fold Change
(Relative to OVCAR3 WT)
OVCAR3 WT
OVCAR3 CP
FGFR2
FGFR3
AXL
VEGFR3
Ins
ERBb2
Recpto Tyrosine Kinase

D

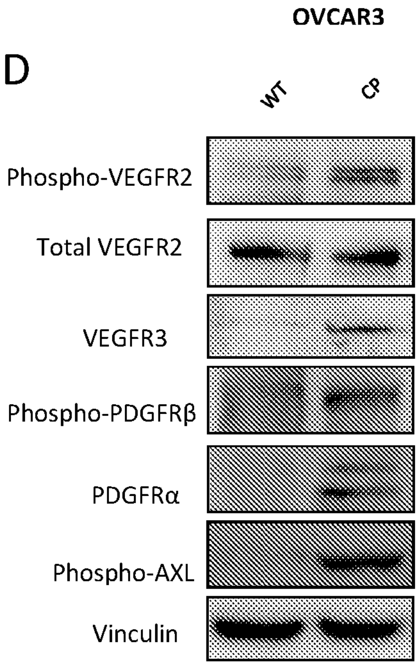
OVCAR3
WT
CP
Phospho-VEGFR2
Total VEGFR2
VEGFR3
Phospho-PDGFRβ
PDGFRα
Phospho-AXL
Vinculin

FIGURE 41

A

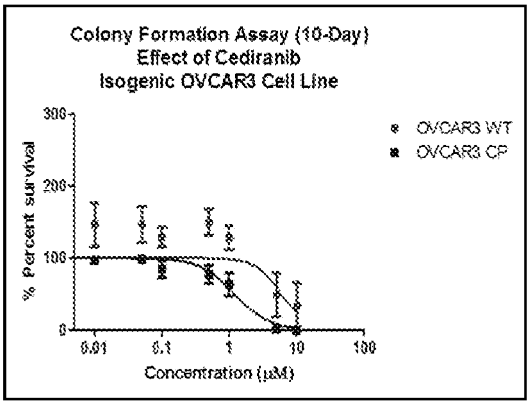


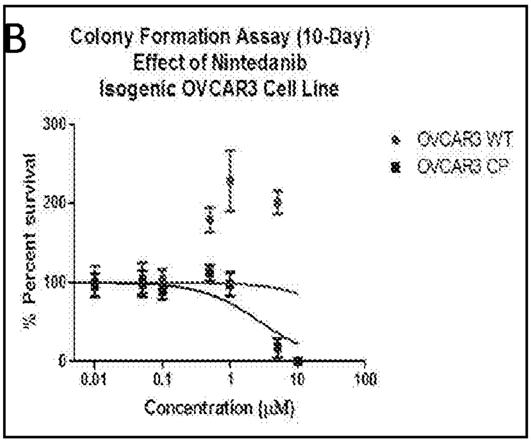


| Cell Line | Cediranib (IC50) |
|---|---|
| OVCAR3 WT | 4.994 |
| OVCAR3 CP | 1.194 |
| Fold Change | 0.2390869 |

| Cell Line | Nintedanib (IC50) |
|---|---|
| OVCAR3 WT | >10 |
| OVCAR3 CP | 3.777 |
| Fold Change | 0.3777 |

# FIGURE 42

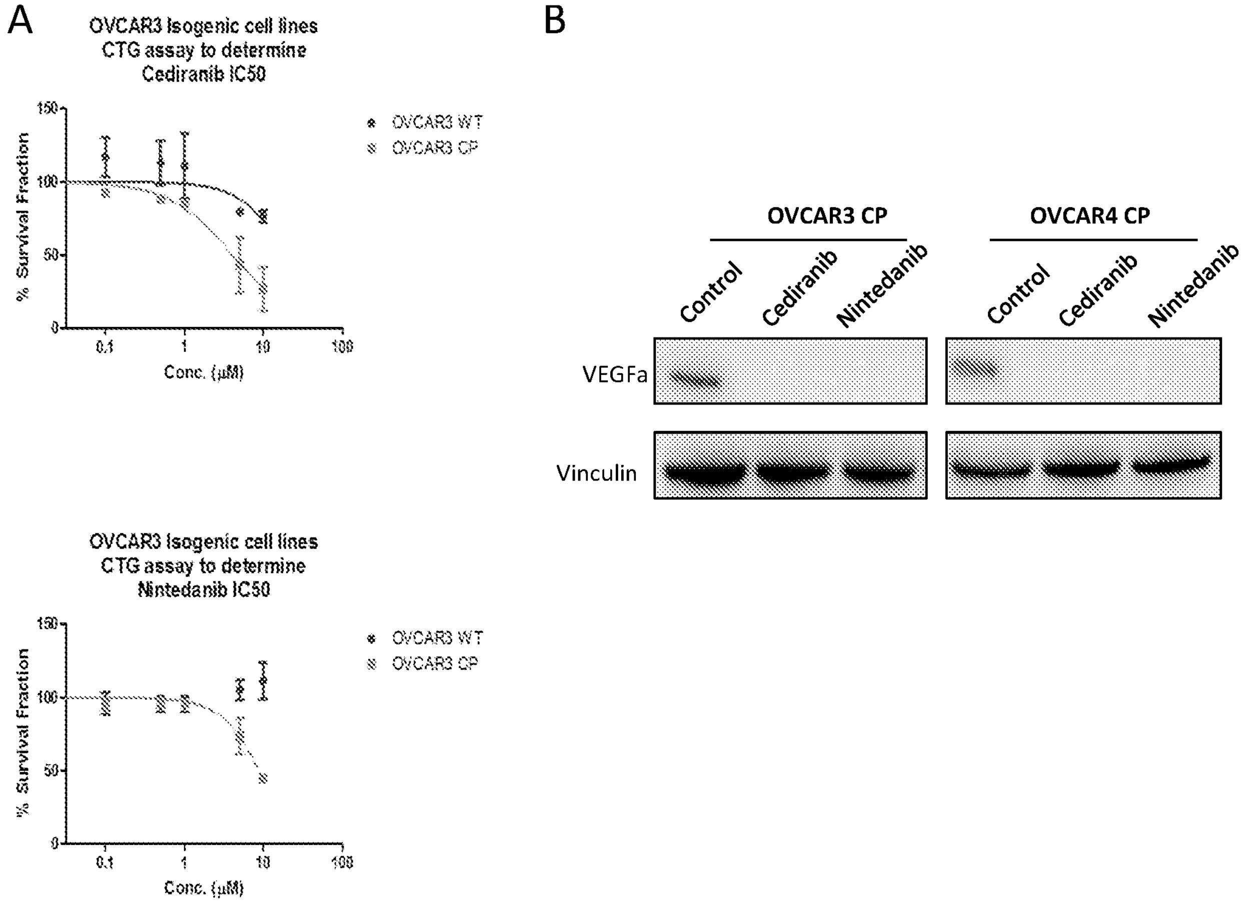
A
OVCAR3 Isogenic cell lines
CTG assay to determine
Cediranib IC50
150
100
50
0
% Survival Fraction
0.1
1
10
100
Conc. (μM)
OVCAR3 WT
OVCAR3 CP
OVCAR3 Isogenic cell lines
CTG assay to determine
Nintedanib IC50
150
100
50
0
% Survival Fraction
0.1
1
10
100
Conc. (μM)
OVCAR3 WT
OVCAR3 CP
B
OVCAR3 CP
OVCAR4 CP
Control
Cediranib
Nintedanib
Control
Cediranib
Nintedanib
VEGFa
Vinculin

# FIGURE 43

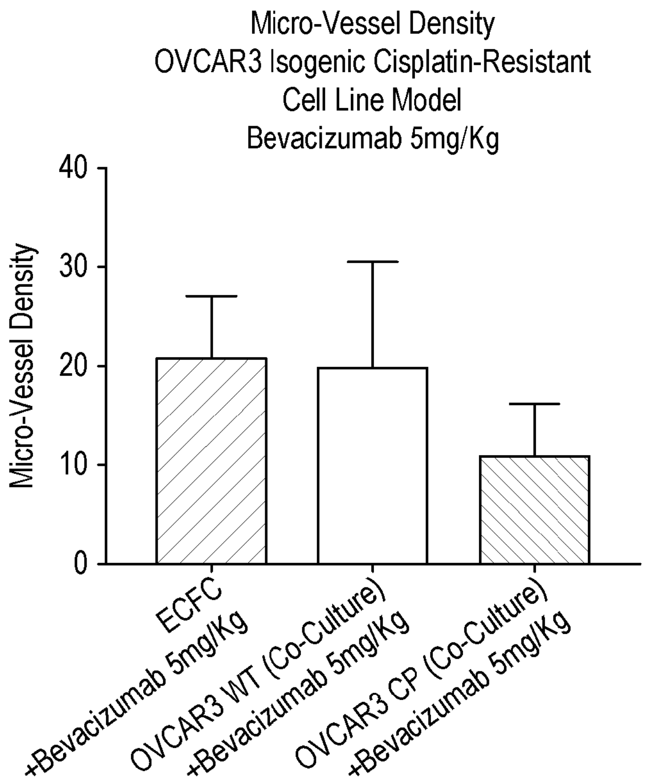


| Cell Line | P-Value (95% CI) |
|---|---|
| OVCAR3 WT | 0.84525 (-10.15 to 8.398) |
| OVCAR3 CP | 0.0024 (-15.46 to -4.067) |

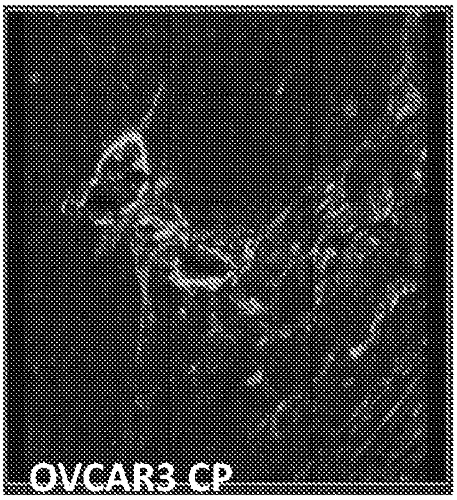


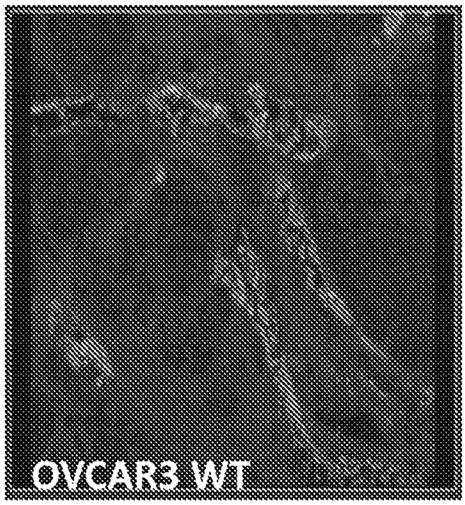


# FIGURE 44

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012142330 A
- WO 2015050500 A
- US 2014073523 A
- EP 2715348 A
- US 20060134663
- US 20090082218
- AR 00457679
- WO 2007141533 A
- WO 2012167278 A
- US 6410276 B
- US 4437975 A
- WO 9006995 A
- WO 2004067726 A
- US 5143854 A
- US 5288644 A
- US 5324633 A
- US 5432049 A
- US 5470710 A
- US 5492806 A
- US 5503980 A
- US 5510270 A
- US 5525464 A
- US 5547839 A
- US 5580732 A
- US 5661028 A
- US 5800992 A
- WO 9521265 A
- WO 9631622 A
- WO 9710365 A
- WO 9727317 A
- EP 373203 A
- EP 785280 A


### Non-patent literature cited in the description


- **HASTIE et al.** The Elements of Statistical Learning. Springer, 2001
- **BREIMAN.** *Random Forests, Machine Learning,* 2001, vol. 45, 5
- Bishop, Neural Networks for Pattern Recognition. Clarendon Press, 1995
- **DUDA et al.** Pattern Classification. John Wiley, 2001
- **TIBSHIRANI et al.** *Proc. Natl. Acad. Sci. USA,* 2002, vol. 99, 6567-6572
- **WOLD.** *Pattern Recogn.,* 1976, vol. 8, 127-139
- Classification and regression trees. **BREIMAN, LEO ; FRIEDMAN, J. H. ; OLSHEN, R. A ; STONE, C. J.** Monterey. Wadsworth & Brooks/Cole Advanced Books & Software, 1984
- **BREIMAN, LEO.** Random Forests. *Machine Learning,* 2001, vol. 45 (1), 5-32
- **NGUYEN et al.** *Bioinformatics,* 2002, vol. 18, 39-50
- **SCHÖLKOPF et al.** Learning with Kernels. MIT Press, 2002
- **AHDESMÄKI et al.** *Annals of applied statistics,* 2010, vol. 4, 503-519
- **L. STÅHLE ; S. WOLD.** *J. Chemom.,* 1987, vol. 1, 185-196
- **D. V. NGUYEN ; D.M. ROCKE.** *Bioinformatics,* 2002, vol. 18, 39-50
- **MCKEEN et al.** *Endocrinology,* 2008, vol. 149 (11), 5724-34
- **JONES et al.** *Nature,* 29 May 1986, vol. 321 (6069), 522-5
- **ROGUSKA et al.** *Protein Engineering,* 1996, vol. 9 (10), 895-904
- **STUDNICKA et al.** Humanizing Mouse Antibody Frameworks While Preserving 3-D Structure. *Protein Engineering,* 1994, vol. 7, 805
- ImmunoAssay: A Practical Guide. Taylor & Francis, Ltd, 2005
- **AHMED N ; ABUBAKER K ; FINDLAY J ; QUINN M.** Epithelial mesenchymal transition and cancer stem cell-like phenotypes facilitate chemoresistance in recurrent ovarian cancer. *Curr Cancer Drug Targets,* May 2010, vol. 10 (3), 268-78
- **FERLAY, J. et al.** GLOBOCAN 2012 v1.0, Cancer Incidence and Mortality Worldwide: IARC CancerBase. *Lyon, France: International Agency for Research on Cancer,* 2013, (11), 11, http://globocan.iarc.f
- **UK, C. R.** Cancer Incidence for Common Cancers. *Cancer Research UK,* 2013, vol. 1, http://www.cancerresearchuk.org/cancer-info/cancerstats/incidence/commoncancers/uk-cancer-incidence-statistics-for-common-cancers>http://www.bbc.co.uk/news/uk-northernireland-33235308

- **VAUGHAN S ; COWARD JI ; BAST RC JR ; BERCHUCK A ; BEREK JS ; BRENTON JD ; COUKOS G ; CRUM CC ; DRAPKIN R ; ETEMADMOGHADAM D.** Rethinking ovarian cancer: recommendations for improving outcomes. *Nat Rev Cancer.,* 23 September 2011, vol. 11 (10), 719-25
- **MCCLUGGAGE, W. G.** My approach to and thoughts on the typing of ovarian carcinomas. *J. Clin. Pathol.,* 2008, vol. 61, 152-163
- **BOWTE.** ll, D. D. The genesis and evolution of high-grade serous ovarian cancer. *Nat Rev Cancer,* 2010, vol. 10, 803-08
- **JOSE DG ; DE KRETSER T.** Oncogenes of human tumour cells. A review of advances in virology and molecular biology of cancer. *Australas Radiol,* 1984, vol. 28 (1), 43-50
- **COHEN Y ; XING M ; MAMBO E et al.** BRAF mutation in papillary thyroid carcinoma. *J Natl Cancer Inst,* 2003, vol. 95 (8), 625-627
- **DAVIES H ; BIGNELL GR ; COX C et al.** Mutations of the BRAF gene in human cancer. *Nature,* 2002, vol. 417 (6892), 949-954
- **XU X ; QUIROS RM ; GATTUSO P et al.** High prevalence of BRAF gene mutation in papillary thyroid carcinomas and thyroid tumor cell lines. *Cancer Res.,* 2003, vol. 63 (15), 4561-4567
- **HOSHINO R ; CHATANI Y ; YAMORI T et al.** Constitutive activation of the 41-/43-kDa mitogen-activated protein kinase signaling pathway in human tumors. *Oncogene,* 1999, vol. 18 (3), 813-822
- **MARSHALL CJ.** Cell signalling. Raf gets it together. *Nature,* 1996, vol. 383 (6596), 127-128
- **SHAUL YD ; SEGER R.** The MEK/ERK cascade: from signaling specificity to diverse functions. *Biochim Biophys Acta,* 19 October 2006, vol. 1773 (8), 1213-1226
- **LEWIS, TS ; SHAPIRO PS ; AHN NG.** Signal transduction through MAP kinase cascades. *Adv Cancer Res.,* 1998, vol. 74, 49-139
- **ALBEROLA-ILA J ; FORBUSH KA ; SEGER R et al.** Selective requirement for MAP kinase activation in thymocyte differentiation. *Nature,* 1995, vol. 373 (6515), 620-623
- **JOHNSON GL ; VAILLANCOURT RR.** Sequential protein kinase reactions controlling cell growth and differentiation. *Curr Opin Cell Biol.,* 1994, vol. 6 (2), 230-238
- **D'ANGELO G ; STRUMAN I ; MARTIAL J et al.** Activation of mitogen-activated protein kinases by vascular endothelial growth factor and basic fibroblast growth factor in capillary endothelial cells is inhibited by the antiangiogenic factor 16-kDa N-terminal fragment of prolactin. *Proc Natl Acad Sci USA.,* 1995, vol. 92 (14), 6374-6378
- **NA J ; FURUE MK ; ANDREWS PW.** Inhibition of ERK1/2 prevents neural and mesendodermal differentiation and promotes human embryonic stem cell self-renewal. *Stem Cell Res.,* 03 August 2010, vol. 5 (2), 157-169
- **TCGA.** Integrated genomic analyses of ovarian carcinoma. *Nature,* 2011, vol. 474, 609-15
- **J. LOPEZ ; S. BANERJEE ; S. B. KAYE.** New developments in the treatment of ovarian cancerfuture perspectives. *Ann Oncol.,* December 2013, vol. 24 (10), x69-x76
- **SCHWARZ RF ; NG CK ; COOKE SL ; NEWMAN S ; TEMPLE J ; PISKORZ AM ; GALE D ; SAYAL K ; MURTAZA M ; BALDWIN PJ.** Spatial and temporal heterogeneity in high-grade serous ovarian cancer: a phylogenetic analysis. *PLoS Med,* 24 February 2015, vol. 12 (2), e1001789
- **PATCH AM ; CHRISTIE EL ; ETEMADMOGHADAM D ; GARSED DW ; GEORGE J ; FEREDAY S ; NONES K ; COWIN P ; ALSOP K ; BAILEY PJ.** Whole-genome characterization of chemoresistant ovarian cancer. *Nature,* 28 May 2015, vol. 521 (7553), 489-94
- **ELEVELD TF ; OLDRIDGE DA ; BERNARD V ; KOSTER J ; DAAGE LC ; DISKIN SJ ; SCHILD L ; BENTAHAR NB ; BELLINI A ; CHICARD M.** Relapsed neuroblastomas show frequent RAS-MAPK pathway mutations. *Nat Genet.,* 29 June 2015
- **LI REN KONG ; KIAN NGIAP CHUA ; WEN JING SIM ; HSIEN CHUN NG ; CHONGLEI BI ; JINGSHAN HO ; MIN EN NGA ; YIN HUEI PANG ; WEIJIE RICHARD ONG ; ROSS ANDREW SOO.** MEK Inhibition Overcomes Cisplatin Resistance Conferred by SOS/MAPK Pathway Activation in Squamous Cell Carcinoma. *Molecular Cancer Therapeutics,* 2015
- **BENEDETTI V ; PEREGO P ; LUCA BERETTA G ; CORNA E ; TINELLI S ; RIGHETTI SC ; LEONE R ; APOSTOLI P ; LANZI C ; ZUNINO F.** Modulation of survival pathways in ovarian carcinoma cell lines resistant to platinum compounds. *Mol Cancer Ther.,* March 2008, vol. 7 (3), 679-87
- **LORD CJ ; ASHWORTH A.** Mechanisms of resistance to therapies targeting BRCA-mutant cancers. *Nat Med.,* 07 October 2013, vol. 19 (11), 1381-8
- **SIDDIQUI ; RIMM.** Pre-analytic variables and phospho-specific antibodies: the Achilles heel of immunohistochemistry. *Breast Cancer Research,* 2010, vol. 12, 113
- **PINHEL IF ; MACNEILL FA ; HILLS MJ ; SALTER J ; DETRE S ; A'HERN R ; NERURKAR A ; OSIN P ; SMITH IE ; DOWSETT M.** Extreme loss of immunoreactive p-Akt and p-Erk1/2 during routine fixation of primary breast cancer. *Breast Cancer Res,* 2010, vol. 12, R76

- **GROSSI V ; PESERICO A ; TEZIL T ; SIMONE C.** p38$\alpha$ MAPK pathway: a key factor in colorectal cancer therapy and chemoresistance. *World J Gastroenterol.,* 07 August 2014, vol. 20 (29), 9744-58
- **KENNEDY RD ; BYLESJO M ; KERR P et al.** Development and independent validation of a prognostic assay for stage II colon cancer using formalin-fixed paraffin-embedded tissue. *J Clin Oncol,* 2011, vol. 29, 4620-6
- **TANNEY A ; OLIVER GR ; FARZTDINOV V et al.** Generation of a non-small cell lung cancer transcriptome microarray. *BMC medical genomics,* 2008, vol. 1, 20
- **DE JONG, S.** SIMPLS: an alternative approach to partial least squares regression. *Chemometrics IntellLab Syst,* 1993, vol. 18, 251-263
- **IRIZARRY RA ; BOLSTAD BM ; COLLIN F ; COPE LM ; HOBBS B ; SPEED TP.** Summaries of Affymetrix GeneChip probe level data. *Nucleic acids research,* 2003, vol. 31, e15
- **TIBSHIRANI R ; WALTHER G ; HASTIE T.** Estimating the number of clusters in a data set via the gap statistic. *Journal of the Royal Statistical Society Series B-Statistical Methodology,* 2001, vol. 63, 411-423
- **WARD JH.** Hierarchical Grouping to Optimize an Objective Function. *Journal of the American Statistical Association,* 1963, vol. 58, 236-244
- **PINTO FR ; MELO-CRISTINO J ; RAMIREZ M.** A Confidence Interval for the Wallace Coefficient of Concordance and Its Application to Microbial Typing Methods. *PLoS One,* 2008, 3
- **CARRICO JA ; SILVA-COSTA C ; MELO-CRISTINO J et al.** Illustration of a common framework for relating multiple typing methods by application to macrolide-resistant Streptococcus pyogenes. *J Clin Microbiol,* 2006, vol. 44, 2524-32
- **MARISA L ; DE REYNIÈS A ; DUVAL A ; SELVES J ; GAUB MP ; VESCOVO L ; ETIENNE-GRIMALDI MC ; SCHIAPPA R ; GUENOT D ; AYADI M.** Gene expression classification of colon cancer into molecular subtypes: characterization, validation, and prognostic value. *PLoS Med.,* 21 May 2013, vol. 10 (5), e1001453
- **JORISSEN RN ; GIBBS P ; CHRISTIE M ; PRAKASH S ; LIPTON L ; DESAI J ; KERR D ; AALTONEN LA ; ARANGO D ; KRUHØFFER M.** Metastasis-Associated Gene Expression Changes Predict Poor Outcomes in Patients with Dukes Stage B and C Colorectal Cancer. *Clin Cancer Res.,* 15 December 2009, vol. 15 (24), 7642-7651
- **OKAYAMA H ; KOHNO T ; ISHII Y ; SHIMADA Y ; SHIRAISHI K ; IWAKAWA R ; FURUTA K ; TSUTA K ; SHIBATA T ; YAMAMOTO S.** Identification of genes upregulated in ALK-positive and EGFR/KRAS/ALK-negative lung adenocarcinomas. *Cancer Res.,* 01 January 2012, vol. 72 (1), 100-11
- **VIRTAKOIVU R ; MAI A ; MATTILA E ; DE FRANCESCHI N ; IMANISHI SY ; CORTHALS G ; KAUKONEN R ; SAARI M ; CHENG F ; TORVALDSON E.** Vimentin-ERK Signaling Uncouples Slug Gene Regulatory Function. *Cancer Res.,* 01 June 2015, vol. 75 (11), 2349-62
- **HASLEHURST AM ; KOTI M ; DHARSEE M ; NUIN P ; EVANS K ; GERACI J ; CHILDS T ; CHEN J ; LI J ; WEBERPALS J.** EMT transcription factors snail and slug directly contribute to cisplatin resistance in ovarian cancer. *BMC Cancer,* 19 March 2012, vol. 12, 91
- **MARCHINI S ; FRUSCIO R ; CLIVIO L ; BELTRAME L ; PORCU L ; FUSO NERINI I ; CAVALIERI D ; CHIORINO G ; CATTORETTI G ; MANGIONI C.** Resistance to platinum-based chemotherapy is associated with epithelial to mesenchymal transition in epithelial ovarian cancer. *Eur J Cancer,* 13 August 2012, vol. 49 (2), 520-30

SEQUENCE LISTING

<110> Almac Diagnnostics Limited

<120> GENE SIGNATURES FOR CANCER DETECTION AND TREATMENT

<130> P144790WO00

<160> 811

<170> PatentIn version 3.5

<210> 1
<211> 260
<212> DNA
<213> Homo sapiens

<400> 1

aagttcccag gtgatctatc gggagaacca cgattgagaa ccactgataa aggccgcctg 60

taggcatgtc tgttggaggg gaatgtgatg aaggtgcttt tccgtttcat tatcctcttt 120

tacaaaatct atttaaaaat ataaacagaa gcattaatca gtgtagctaa ttagtataat 180

gaaatagacc atgcactaag caggatttgt ggaaatgaat attttggtac aaattgggat 240

tctcctcttt aatcactgat 260

<210> 2
<211> 255
<212> DNA
<213> Homo sapiens

<400> 2

atttgtgtat ttgcacatgg ttgtgctgtc gaggacctgg tgctgagaag agtctgttca 60

cagccaaaat tcttcccact gtcattccta acctgggatt tctagacaca tcctgctgtg 120

atgtaaacag aaatcacgaa ttcgctcact ggatcaagtt gttccactgg tgtctaatac 180

gctattgttg ccggaggtgg gttctgtgac gtgaagccat ttcccatcat tcaacagcca 240

gttacaattt tctgt 255

<210> 3
<211> 239
<212> DNA
<213> Homo sapiens

<400> 3

aagaacaact cctcaccagt tcatcctgag gctgggagga ccgggatgct ggattctgtt 60

ttccgaagtc actgcagcgg atgatggaac tgaatcgata cggtgttttc tgtccctcct 120

actttccttc acaccagaca gcccctcatg tctccaggac aggacaggac tacagacaac 180

tctttcttta aataaattaa gtctttacaa taaaaacaca actgcaaagt accttcata 239

<210> 4
<211> 244
<212> **DNA**
<213> Homo sapiens

<400> 4

```
cactcccggg actattgcca agaaggggca agggatgagt caagaaggtg agacccttcc        60
cggtgggcac gtgggccagg ctgtgtgaga tgttggatgt ttggtactgt ccatgtctgg       120
gtgtgtgcct attacctcag catttctcac aaagtgtacc atgtagcatg ttttgtgtat       180
ataaaaggga gggttttttt aaaaatatat tcccagatta tccttgtaat gacacgaatc       240
tgca                                                                    244
```

<210> 5
<211> 192
<212> DNA
<213> Homo sapiens

<400> 5

```
agatcatccg ccagcgggaa caacgcgttg gagctctccc agctggagta acagatcctg        60
gaaccaacag ccgacatgtt gcaccttgcc cgcaagtaca aggaccctgg agcacaagtc       120
cccgcccctg ggcccattgg cccccaaccc aatcaaaaat ctttccccca ccttgaggaa       180
gcactgccca aa                                                           192
```

<210> 6
<211> 214
<212> DNA
<213> Homo sapiens

<400> 6

```
ggatcttgat gcctgaaaat cccaagattg gtacttggca aactgaaaga aatctagaaa        60
accctagaga tcaggcatct gtggccagct aactggtcat acaaatggat tgttgtggtg       120
aacttgtata gtattaatcc tgagatgctg tccccctcca cccccacccc cacaaaaaaa       180
ataaataaag tagtattaag ttagcctcat acaa                                   214
```

<210> 7
<211> 164
<212> DNA
<213> Homo sapiens

<400> 7

```
ccatggcaat gcgggtgact cctttacatg gcacaacggc aagcagttca ccaccctgga        60
cagagatcat gatgtctaca caggaaactg tgcccactac cagaagggag gctggtggta       120
taacgcctgt gcccactcca acctcaacgg ggtctggtac cgcg                        164
```

<210> 8
<211> 225
<212> DNA

<213> Homo sapiens

<400> 8

```
cccaaacctg tactgtcccg gaggaggttg ggaggtggag gcccagcatc ccgcgcagat      60
gacaccatca accgccagag tcccagacac cggttttcct agaagcccct cacccccact     120
ggcccactgg tggctaggtc tccccttatc cttctggtcc agcgcaagga ggggctgctt     180
ctgaggtcgg tggctgtctt tccattaaag aaacaccgtg caacg                     225
```

<210> 9
<211> 219
<212> DNA
<213> Homo sapiens

<400> 9

```
ggcccggccc ccatcacatg ttcccttggc ctcagagctg cccctgctct cccaccacag      60
ccacccagag gcaccccatg aagctttttt ctcgttcact cccaaaccca agtgtccaaa     120
gctccagtcc taggagaaca gtccctgggt cagcagccag gaggcggtcc ataagaatgg     180
ggacagtggg ctctgccagg gctgccgcac ctgtccaga                            219
```

<210> 10
<211> 239
<212> DNA
<213> Homo sapiens

<400> 10

```
gcaagagtgt ttggcagacg ttttttggat gtgctacagg ctttcctccc ttgttgctta      60
tttttattga caaacatccc taatgtgtgt catgtctttc tctgtctctc cccttccctc     120
tcttttttc tcaaattatg taacttttga ggacagttac ttctgtgata aactcttcaa     180
gtaagaccgt tataagatag atttggaaat taaagcccca gttgcatttc agtaactca      239
```

<210> 11
<211> 259
<212> DNA
<213> Homo sapiens

<400> 11

```
ggtatcccca gattagaatg tgtttatttg ggttggagta tcactgactg tggcctctct      60
gacttcaagt gcttggtgta attcacatgc catctcctct gtgaagcctc cttgtttctc     120
ccagtgaagc aggccacctg gttctctttc ctctcactcc attttgtgcg tgctttcact     180
ctagcacttg ccataccaaa gggcaattat ttatctacct atctgtatct ccttgtgaaa     240
tttgtgctcc acaaaccaa                                                  259
```

<210> 12
<211> 175
<212> DNA
<213> Homo sapiens

<220>
<221> misc_feature
<222> (76).. (76)
<223> n is a, c, g, or t

<400> 12

```
ccaccgcgcc tagccagcca attttttttt tttttgtatt tttagtagag acagggtttt      60
accatgttgg ccaggntggt cttgaactcc tgacctcaag tgatctgcct gccatggcct     120
cccaaagtgc tgggattaca ggtttgagcc accatgcctg ggccaatatt taatt          175
```

<210> 13
<211> 224
<212> DNA
<213> Homo sapiens

<400> 13

```
tttctcttga tgcaatgaag ggaatatgac actacagtat acagagttgt tgttatatga      60
tgcacaaaat attttgatga tttgaataaa tgttaacttt taatctgcgc cttaataatg     120
actggttatc tgtaaatata gaacagatac agattgtatt tttctgtggg tttttgtcct     180
tttagtgatt tttttccaaa aacgagagat ggaattaaca ttga                      224
```

<210> 14
<211> 293
<212> DNA
<213> Homo sapiens

<400> 14

```
tttgaatcgc caaatgcacg cacctctttc ctggaaggtg gagcgagtgg aaggctaccc      60
cgtcagtatc actcagacat tgctagtgtc agtggccgct ggtagcagca ccctcttggc     120
acatgcccgc tgactaactg taaagtggac acaggagatg tatgaacagc cttcacagca     180
caccatcctt agcactctgg gtgtctggta tcaggaccaa agcattttat tcgcacctgt     240
actttatggc aaaaaggaag aagagagaga agatgttctt atgatgtcat aca            293
```

<210> 15
<211> 122
<212> DNA
<213> Homo sapiens

<400> 15

tctgttctgt cagtcatggc atggctggac agaattcagg gacatctatc aggcctctca 60

ggagcaatta aattattttc atgaaggcaa tttgcagttg taaacaatat tgaagacact 120

gt 122

<210> 16
<211> 175
<212> DNA
<213> Homo sapiens
<400> 16

ctccccaata gctaggacta tggttatgtg ccactaggct cagctaattt ttttaggttt 60

tgtagagaca gtcttgccat gttgcccagg ctagtgttga acttctggcc tcaagcgatc 120

ctcctgtctc ggcctctcaa agcactggga ttacagggtg tgaaccacta tgcct 175

<210> 17
<211> 224
<212> DNA
<213> Homo sapiens

<400> 17

tcagtgcacg atgctccagc cacactggcc atctttcggt tcctgataca aaaaaaaaca 60

cgttcctttt ccatggaaag caggtcaccc ttgttatttt gtatcgatga caactcttta 120

aacttatttt gctttttggc tttatgtatg tgtgtgggtg ggtgggactg actgccccac 180

tagaatgtaa gctccatgag ggcagggaat cttgctttct tgtt 224

<210> 18
<211> 267
<212> DNA
<213> Homo sapiens

<400> 18

ggaggcagac aatgacccca cggctcctcc ttatgactcc attcaaatct acggttatga 60

aggcaggggc tcagtggccg ggtccctgag ctccctagag tcggccacca cagattcaga 120

cttggactat gattatctac agaactgggg acctcgtttt aagaaactag cagatttgta 180

tggttccaaa gacacttttg atgacgattc ttaacaataa cgatacaaat ttggccttaa 240

gaactgtgtc tggcgttctc aagaatc 267

<210> 19
<211> 257
<212> DNA
<213> Homo sapiens

<400> 19

ttgttactgc tgattcttgt aaatcttttt gcttctactt tcatcttaaa ctaatacgtg 60
ccagatataa ctgtcttgtt tcagtgagag acgccctatt tctatgtcat ttttaatgta 120
tctatttgta caattttaaa gttcttattt tagtatacgt ataaatatca gtattctgac 180
atgtaagaaa atgttacggc atcacactta tattttatga acattgtact gttgctttaa 240
tatgagcttc aatataa 257

<210> 20
<211> 256
<212> DNA
<213> Homo sapiens

<400> 20

actgcattgc tggaccctct gtgaaactga agccttctct acttgttttt atctcaagtg 60
aacctggaga agcaacaata atggaccttc tcccctagtc aaatagcctg tggacctccc 120
ctcatagtca gtctccaaaa acatgtatct ggaattaatt tattacaaag agaagtttag 180
tgtttcttct tttttacatg cgctcaatac tgactactgg ccagacacag caccatcctc 240
ttacaaacat catttc 256

<210> 21
<211> 260
<212> DNA
<213> Homo sapiens

<400> 21

cttgaacatg ggaaggagtt gttgcagtaa gccaagatgt gccactgtac tccagcctgg 60
gagacagagt gagactctgt ctcaaaaaat gaatgaataa ataaataaat aaataataaa 120
aaagatgatt attaacaatg ccagttaata ttaacaatat taatagtatt atttattact 180
aatgccattt tttcattttt gttaaagtat ttttattatt ttagtttaaa ttattattat 240
gagacaaagg cctccatttc 260

<210> 22
<211> 273
<212> DNA
<213> Homo sapiens

<400> 22

ccttgaaaag cagtgtcaga gcagaattat aaggcatttt taatattccc tgttttaata 60

aaactttttt tatgtttgat ttttttttat attttttgtc cgcacgtata tagatgtgga 120

tacataacat ttaacacggt tgcaatcaga gggtgatttg atttgttaac ttaatgtcac 180

atcataaaca ttttacatgc tgttatataa tgtacataat cattttttaat gactacataa 240

catcccatcc tattgacgaa tcattatgtc ctt 273

<210> 23
<211> 240
<212> DNA
<213> Homo sapiens

<220>
<221> misc_feature
<222> (28)..(28)
<223> n is a, c, g, or t

<220>
<221> misc_feature
<222> (163)..(163)
<223> n is a, c, g, or t

<400> 23

aagtatacag ctgtaagtaa ccctgtcncc atggatgatc cttttctcta ggaatgtatt 60

tggattagag atgacaacta cattttcgca tttttatgtt gaagtctttt ttaaaaaggc 120

tgtttacttt tcagtagtta agaatacttg tttttctttt tcnttttttt tttttttaac 180

cttttatttt ttcgttaagc ctctattgtt tgtagaacac tcttagaaac ttggaaataa 240

<210> 24
<211> 221
<212> DNA
<213> Homo sapiens

<400> 24

ggcagccctg acgtgatgag ctcaaccagc agagacattc catcccaaga gaggtctgcg 60

tgacgcgtcc gggaggccac cctcagcaag accaccgtac aattggtgga aggggtgaca 120

gctgcattct cctgtgccta ccacgtaacc aaaaatgaag gagaactact gtttacaagc 180

cgccctggtg tgcctgggca tgctgtgcca cagccatgcc t 221

<210> 25
<211> 129
<212> DNA
<213> Homo sapiens

<400> 25

gatccagaac tgtggctggg caccgtggct cacatctgta atctcatcac tttgggaagg 60

ctaaggcggg tggatcacct aaggtaagga gttcgaaccc agcctttaca atgtaatgaa 120

accctgcct 129

<210> 26
<211> 94
<212> DNA
<213> Homo sapiens

<400> 26

gcctcccatt caagtgaagt tataatttac actgagggtt tcaaaattcg actagaagtg 60

gagatatatt atttatttat gcactgtact gtat 94

<210> 27
<211> 129
<212> DNA
<213> Homo sapiens

<400> 27

aatgcatatg gaggtaggct gaaaagaatg taatttttat tttctgaaat acagatttga 60

gctatcagac caacaaacct tccccctgaa aagtgagcag caacgtaaaa acgtatgtga 120

agcctctct 129

<210> 28
<211> 115
<212> DNA
<213> Homo sapiens

<400> 28

tcatccatcc aggatgtact aaaacagtgt gtttaataaa ttgtaattat tttgtgtaca 60

gttttatact gttatctgtg tccatttcca aaacttgcac gtgtccctga attcc 115

<210> 29
<211> 176
<212> DNA
<213> Homo sapiens

<400> 29

gttaaattaa ttgataccag atttcactgg aacagtttca actgataatt tatgacaaaa 60

gaacatacct gtaatattga aattaaaaag tgaaatttgt cataaagaat ttcttttatt 120

tttgaaatcg agtttgtaaa tgtcctttta agaagggaga tatgaatcca ataaat 176

<210> 30
<211> 252

<212> DNA
<213> Homo sapiens

<400> 30

tttcaacttt ccttttccta tttgaatttc tttggtgctg tagaaaacaa aaaaagaaaa 60
atatatattc ataaaaaata tggtgctcat tttcatccat ccaggatgta ctaaaacagt 120
gtgtttaata aattgtaatt attttgtgta cagttttata ctgttatctg tgtccatttc 180
caaaacttgc acgtgtccct gaattccatt tgactttaat tttatgagaa ttgcagaact 240
ttgatggcaa ta 252

<210> 31
<211> 230
<212> DNA
<213> Homo sapiens

<400> 31

tacaaccatg acatttcaac ttttgatatg ggcaactaac agtgacccat ctgtcagtca 60
gaagtcctcg tgggtccctc tgtctgtctg ctaaaagtcc tcatgggtcc gtctgtctgt 120
gttctaaatg aagtacttct tttctggcct actgcaattt tacaaattac cttgtagaga 180
ttagatacat ttgtcccctc tagacagagg taacacctga gtacagacct 230

<210> 32
<211> 280
<212> DNA
<213> Homo sapiens

<400> 32

ttaaaaatta ggcaaagctg ccagggtgtg gtggctcgca catgtaatcc caacactttg 60
ggagaccaag gcgggtggat cacctcaggt cgggagttcg agaccagcct gatcaacatg 120
gagaaaaccc gtctttacca aatatacaaa attacccagg catggtggtg catgcctgta 180
atcctagcta ctcgggaggc tgaggcagga gaatcacttg aacccaggag gtggaggttg 240
ctgtgaacca agattgcgcc cttgcacaac aagagtgaaa 280

<210> 33
<211> 211
<212> DNA
<213> Homo sapiens

<400> 33

gaaaaaactt tctctttgcc atttcttctt cttctttttt aactgaaagc tgaatccttc 60

catttcttct gcacatctac ttgcttaaat tgtgggcaaa agagaaaaag aaggattgat 120

cagagcattg tgcaatacag tttcattaac tccttccctc gctcccccaa aaatttgaat 180

tttttttca acactcttac acctgttatg g 211

<210> 34
<211> 130
<212> DNA
<213> Homo sapiens

<400> 34

tctttttca gaatctatta aggacacttg aaagttttga aatttttggt aaatttggac 60

taccatgagg aaacttttga gattcaagtt cattctattc agagcaattc cgatattgat 120

gttaacttga 130

<210> 35
<211> 233
<212> DNA
<213> Homo sapiens

<400> 35

caattttgtg cctaaattgc acattagaag atggattgat tggacacatc catgtaattc 60

aaagttatta ttcaaatttg acttaattgg taatcattga aaaaactgac taatgtcatt 120

tagtgtgaag gagcactggc cagctatatg ccacactcat acatatgcat tttcagaatg 180

tgagcagctt ttctgaattt ttaatcaaac cttttcacca actttactga atg 233

<210> 36
<211> 152
<212> DNA
<213> Homo sapiens

<400> 36

gtaaatcact gtaaacgtgt cttcatttac tctagccaaa aggcctggct tctgatagga 60

aactggtaag aaactcttca tgaaaacaca tcactaatat tcgctattac tctcctggtc 120

tgaagtcagc ttttctgaac cattaaggta tt 152

<210> 37
<211> 175
<212> DNA
<213> Homo sapiens

<400> 37

gagttgtatc gtgtggtgta ttttttaaaa aatttgattt agcattcata ttttccattt 60

tattcccaat taaaagtatg cagattattt gcccaaagtt gtccttttttt tcagattcag 120

catttgtttt ttgccagttt cattttcatt tttttccatg gttccacaga agctt 175

<210> 38
<211> 226
<212> DNA
<213> Homo sapiens

<400> 38

taaaggtggt ttggcctcta atttaatttt gattcagact ctcctgtcag gactcaagaa 60

aatttaatta attaccaagg attaagtttt ttggttaagg tttttgggaa aaaaaaatag 120

caaagatgtt gatttcttgg aatcctttta caggttcata acagaaaaat cttcattccc 180

tgtaggcatt taattaaacc tagttgagaa gtgtgtggga ttcctc 226

<210> 39
<211> 270
<212> DNA
<213> Homo sapiens

<400> 39

ctgttccttt cctgggttcc aattttgtgc ctaaattgca cattagaaga tggattgatt 60

ggacacatcc atgtaattca aagttattat tcaaatttga cttaattggt aatcattgaa 120

aaaactgact aatgtcattt agtgtgaagg agcactggcc agctatatgc cacactcata 180

catatgcatt ttcagaatgt gagcagcttt tctgaatttt taatcaaacc ttttcaccaa 240

ctttactgaa tgcctactgg aattccataa 270

<210> 40
<211> 215
<212> DNA
<213> Homo sapiens

<400> 40

caaggccctg ctgtaagtat gatttgggga aataataaag aagatcacgg acctaggaat 60

gttttcttca gactaaacca agacaacttt gacaacccat taagttagcc ccatttcaat 120

atatcctcta aaatatctgg aaattgtcta aatgcaatgg gctgtaagtc catccctgca 180

gtgggcctgg gggctcgtta tttatttatg gtgaa 215

<210> 41
<211> 181
<212> DNA
<213> Homo sapiens

<400> 41

```
gagttgtatc gtgtggtgta ttttttaaaa aatttgattt agcattcata ttttccatct      60
tattcccaat taaaagtatg cagattattt gcccaaatct tcttcagatt cagcatttgt     120
tctttgccag tctcattttc atcttcttcc atggttccac agaagctttg tttcttgggc     180
a                                                                     181
```

<210> 42
<211> 225
<212> DNA
<213> Homo sapiens

<400> 42

```
ttttctgcac atttacttgc ttaaattgtg ggcaaaagag aaaaagaagg attgatcaga      60
gcattgtgca atacagtttc attaactcct tccctcgctc ccccaaaaat ttgaattttt     120
ttttcaacac tcttacacct gttatggaaa atgtcaacct ttgtaagaaa accaaaataa     180
aaattgaaaa ataaaaacca taaacatttg ccaactttct tgtac                     225
```

<210> 43
<211> 211
<212> DNA
<213> Homo sapiens

<400> 43

```
gaaaaaactt tctctttgcc atttcttctt cttctttttt aactgaaagc tgaatccttc      60
catttcttct gcacatctac ttgcttaaat tgtgggcaaa agagaaaaag aaggattgat     120
cagagcattg tgcaatacag tttcattaac tccttccccc gctcccccaa aaatttgaat     180
tttttttca acactcttac acctgttatg g                                     211
```

<210> 44
<211> 68
<212> DNA
<213> Homo sapiens

<400> 44

```
ctggaaaaga tggtcgcact ggacatcctg gtacagttgg acctgctggc attcgaggcc      60
ctcagggt                                                               68
```

<210> 45
<211> 83
<212> DNA
<213> Homo sapiens

<400> 45

taataacatg gcacgatgaa tgcttcttta gagtaaaaag gttttcttta acttgttaag 60

tcagagttgt ctaagtaatt gta 83

<210> 46
<211> 275
<212> DNA
<213> Homo sapiens

<400> 46

gttatggtgc taatgtactt tcacttttaa actctagatc agaattgttg acttgcattc 60

agaacataaa tgcacaaaat ctgtacatgt ctcccatcag aaagattcat tggcatgcca 120

caggggattt tcctccttca tcctgtaaag gtcaacaata aaaaccaaat tatggggctg 180

cttttgtcac actagcatag agaatgtgtt gaaatttaac tttgtaagct tgtatgtggt 240

tgttgatctt ttttttcctt acagacaccc ataat 275

<210> 47
<211> 65
<212> DNA
<213> Homo sapiens

<400> 47

ttaactccat atgtgttcct cttgttttaa ttttgtcaac cagtgcaagt gaccgacaaa 60

attcc 65

<210> 48
<211> 286
<212> DNA
<213> Homo sapiens

<400> 48

taaagacgca tgttatggtg ctaatgtact ttcactttta aactctagat cagaattgtt 60

gacttgcatt cagaacataa atgcacaaaa tctgtacatg tctcccatca gaaagattca 120

ttggcatgcc acaggggatt ctcctccttc atcctgtaaa ggtcaacaat aaaaaccaaa 180

ttatggggct gcttttgtca cactagcata gagaatgtgt tgaaatttaa ctttgtaagc 240

ttgtatgtgg ttgttgatct tttttttcct tacagacacc cataat 286

<210> 49
<211> 143
<212> DNA
<213> Homo sapiens

<400> 49

tctgttgtta gattgctgtc taattacatt gcacaacctt gcctaagttt ttatatacat 60

agcattgagc ttaaaaacat ggaaccagct cttatgcata atttagctat atttagcatc 120

tttggtccaa agccttataa aaa 143

<210> 50
<211> 273
<212> DNA
<213> Homo sapiens
<400> 50

aatgaccacc gccattcaca agaactttga ctgtttgaag ttgatcctga gactcttgaa 60

gtaatggctg atcctgcatc agcattgtat atatggtctt aagtgcctgg cctccttatc 120

cttcagaata tttattttac ttacaatcct caagttttaa ttgattttaa atatttttca 180

atacaacagt ttaggtttaa gatgaccaat gacaatgacc acctttgcag aaagtaaact 240

gattgaataa ataaatctcc gttttcttca att 273

<210> 51
<211> 193
<212> DNA
<213> Homo sapiens

<400> 51

ctaaactggt tatattttga cctgtatatc ttaaatttga gtatttatat gcctaaatac 60

atgtgtgagt tttgtttgac ttccaagtcc aaactataag attatataag ttcatataga 120

tgaatcagaa atatgtggta atactattaa gtcacaaaca ctaacaattt ccaactatag 180

aaataacagt tct 193

<210> 52
<211> 207
<212> DNA
<213> Homo sapiens

<400> 52

agtttgtgac tgatatttca agcaaaatat attttcttca tgtttcaaaa ttaagtgctt 60

tttctctgtt gttagattgc tgtctaatta cattgcacaa ccttgcctaa gtttttatat 120

acatagcatt gagcttaaaa acatggaacc agctcttatg cataatttag ctatatttag 180

catctttggt ccaaagcctt ataaaaa 207

<210> 53
<211> 270
<212> DNA
<213> Homo sapiens

<400> 53

gaaagagcat cgttccaatg cttgttcact gttcctctgt catactgtat ctggaatgct 60

ttgtaatact tgcatgcttc ttagaccaga acatgtaggt ccccttgtgt ctcaatactt 120

tttttttctt aattgcattt gttggctcta ttttaatttt tttcttttaa aataaacagc 180

tgggaccatc ccaaaagaca agccatgcat acaactttgg tcatgtatct ctgcaaagca 240

tcaaattaaa tgcacgcttt tgtcatgtca 270

<210> 54
<211> 269
<212> DNA
<213> Homo sapiens

<400> 54

tataaaaatt tatgtaaggc cgggcatggt ggctcacgcc tgtaatccca gcacttaggg 60

aggccagggt aggtggatta cctgaggtca ggagttcaag accagcctgg ccaacatggt 120

gaaaccctgt ctctattaaa aatacaaaaa ttagcctggc atggtggcat gtgcctgtaa 180

tcctagctat tcaggaggct gaggtaggag aatcacttga acccgggagg cggaggttgt 240

agtgaaccaa aattacaccg ttgcactcc 269

<210> 55
<211> 226
<212> DNA
<213> Homo sapiens

<400> 55

tcactgccaa ttccagcacg aagttgggcg actgccaggc aggcactccc agtcgtcaaa 60

aagtgcaaat gttactcagg gaacaattaa tgtgagttgt gtaatgtaat atgggtcaaa 120

aacatgaaaa gacgtttaaa atgtcagcgg atggctcagc ccacccatca gccagccaga 180

gagcagaaca cctgttttgc actcagtggc acagaagcca caattt 226

<210> 56
<211> 263
<212> DNA
<213> Homo sapiens

<400> 56

tcaggcccgg agaccgggtg ttcctccaga tgccctcaga acaggctgca ggactgtatg 60

ccgggcagta tgtccactcc tccttttcag gatatttatt gtatcccatg taaaaacaaa 120

aaaacaaaaa acaaagaaaa gaaagagatt ttatagaaga aaatgacaca ccaaaaaatc 180

caaatgaaaa acataattgc ttcaaaacac ttacacagtt ggaaagttat atgtaagtga 240

aaatttggac cattgtgtac aaa 263

<210> 57
<211> 245
<212> DNA
<213> Homo sapiens

<400> 57

```
attgtgctat aatccctatt tagttcaaaa ttaaccagaa tttttccatg tgaaatggac        60
caaactcata ttattgttat gtaaatacag agttttaatg cagtatgaca tcccacaggg       120
gaaaagaatg tctgtagtgg gtgactgtta tcaaatattt tatagaatac aatgaacggt       180
gaacagactg gtaacttgtt tgagttccca tgacagattt gagacttgtc aatagcaaat       240
cattt                                                                   245
```

<210> 58
<211> 212
<212> DNA
<213> Homo sapiens

<220>
<221> misc_feature
<222> (125)..(125)
<223> n is a, c, g, or t

<400> 58

```
acaggccaga ttatacaata cttctgtgca caccagatgg ttgtaaagaa agatcagcga        60
gaaaggcttt ggaagtttta atccttcttt ccaccttttt tcccccgcat ttagtaaatc       120
acaancctac ctgactggca tccaattatc agagatattc agtgtttaag ctaccctctt       180
taaaagaaaa tgatctcttc ttattcctaa gg                                     212
```

<210> 59
<211> 221
<212> DNA
<213> Homo sapiens

<400> 59

```
taatgtcatc ctgtactcgg cacaaatcaa aggccaatac aagtctgaaa agcagaaata        60
aatatttttc caggtttttg ctcgggcaca tactaactgc tttgggcatt ttaatctggt       120
ctccaaacac caaagaccca tttcgagcct gctattagcc tgctgctgac tctatcactt       180
ggagcaataa tgtggggtta tggtggtgga atcttgtata t                           221
```

<210> 60
<211> 248
<212> DNA
<213> Homo sapiens

<400> 60

aggctgtgga ttcaaggctc cctgcccccc agatcatttc cccaatcctg gcaaaagccc 60

aaagatccca gggtcaggag agacccctct gtatccccag gtccctccca gaactgactc 120

ctaaggtctc cagccagggc ttttgagatg caaaggtttg gcctcaggag agtcaccttt 180

tctcacggcc ctggccttaa ctcatatttt aggcattcct ggccccaggg ccctaataaa 240

cctgcttt 248

<210> 61
<211> 229
<212> DNA
<213> Homo sapiens

<400> 61

agccaaacaa gagactaacc tgatgtcaaa ctatgggaga ctgttgaata ggcagggttt 60

ggggaaaatc cagtttggtc tccaatgccc aggggaagca ctggcacaga tgctggccct 120

ctctggaagg acttcagaca gtcccaccag ccccacatgt cctgctctta gggctcaatt 180

ccaacacaag ctagatgcgt gacctgggtc aattactgac cctccccga 229

<210> 62
<211> 222
<212> DNA
<213> Homo sapiens

<400> 62

tttcacatag gttagattct cattcacggg actagttagc tttaagcacc ctagaggact 60

agggtaatct gacttctcac ttcctaagtt cccttctata tcctcaaggt agaaatgtct 120

atgtttttta ctccaattca taaatctatt cataagtctt tggtacaagt ttacatgata 180

aaaagaaatg tgatttgtct tcccttcttt gcacttttga aa 222

<210> 63
<211> 32
<212> DNA
<213> Homo sapiens

<400> 63
tgaggactca gaagttcaag ctaaatattg tt 32

<210> 64
<211> 72
<212> DNA
<213> Homo sapiens

<400> 64

gtgcccagcc tatatctact tgagcctgtg tgcccactga agagatgaag aataaaagcc 60

taagctctca tc 72

<210> 65
<211> 162
<212> DNA
<213> Homo sapiens

<400> 65

gattgctaaa gctctggtta atgcacaagt ggatttccag gcaatgtggt actctgacca 60

gaaccacggc ttatccggcc tgtccacgaa ccacttatac acccacatga cccacttcct 120

aaagcagtgt ttctctttgt cagactaaaa acgatgcaga tg 162

<210> 66
<211> 112
<212> DNA
<213> Homo sapiens

<400> 66

ttacaggtaa ttaattgttc tcttcacttc tcatggggca gcacagaaag gaataagtta 60

ggtaactgaa gtgaccagcc ctcgaataaa aagtggcttc atggccgggt gt 112

<210> 67
<211> 145
<212> DNA
<213> Homo sapiens

<400> 67

gataccgact gaccgtgggc cttacccgaa gaggacagcc catgcagtac aatgtgggtc 60

cctctgtctt caagtaccca ctgaggaatc tgcagcctgc atctgagtac accgtattcc 120

tcgtggccat aaagggcaac caaga 145

<210> 68
<211> 269
<212> DNA
<213> Homo sapiens

<400> 68

atcactaact acaaaatccg ccatcatccc gagcacttca atgggaaacc tcgagaaaat 60

cgggtgcccc actctcggaa ttccatcacc ctcaccaacc tcactccagg cacagagtat 120

gtggtcagca tcgttgcttt taatggcaga gaggaaagtc ccttattgat tggccaacaa 180

tcaacaggta acttttcttg tctgcaaaga aactcagaag actttcctac ccagttggta 240

gattctgtaa agtagcttgc tgttgtctg 269

<210> 69
<211> 238
<212> DNA
<213> Homo sapiens

<400> 69

tatatcaatg atggcaaaaa ggttaaaggg ggcctaacag tactgtgtgt agtgttttat 60

ttttaacagt agtacactat aacttaaaat agacttagat tagactgttt gcatgattat 120

gattctgttt cctttatgca tgaaatattg attttacctt tccagctact tcgttagctt 180

taattttaaa atacattaac tgagtcttcc ttcttgttcg aaaccagctg ttcctcct 238

<210> 70
<211> 241
<212> DNA
<213> Homo sapiens

<400> 70

gcatttttc tgtagtcttg tccaaaattg ggtaaccact tctgatgggg tagctcatat 60

ccaagaatga gtcacaaaac cagactcgtt gaacctggta tatgatgagt cacaaagcaa 120

cattctgcct ttgtttttc aggacaagaa acttgaattg tatcccactg agttaaaaga 180

taaaatatat ggcattggca tttctgtact tcagagagga atatatctgt ttgtggtagg 240

a 241

<210> 71
<211> 69
<212> DNA
<213> Homo sapiens

<400> 71

tcatttcatt tgactattct gatgacatga ttgtggcaga ataaattggg tcttaaaatg 60

ccctagaaa 69

<210> 72
<211> 129
<212> DNA
<213> Homo sapiens

<400> 72

```
gtctcctaat cttatcaatt ctgatggttt cttttttcc cagcttttga gccaacaact      60
ctgattaact attcctatag catttactat atttgtttag tgaacaaaca atatgtggtc     120
aattaaatt                                                             129
```

<210> 73
<211> 233
<212> DNA
<213> Homo sapiens

<400> 73

```
tctgtagtct tgtccaaaat tgggtaacca cttctgatgg ggtagctcat atccaagaat      60
gagtcacaaa accagactcg ttgaacctgg tatatgatga gtcacaaagc aacattctgc     120
ctttgttttt tcaggacaag aaacttgaat tgtatcccac tgagttaaaa gataaaatat     180
atggcattgg catttctgta cttcagagag gaatatatct gtttgtggta gga            233
```

<210> 74
<211> 51
<212> DNA
<213> Homo sapiens

<400> 74
ttgcaatggg agacttaaaa gtggtataaa atgtactttg ggccaggcgc a 51

<210> 75
<211> 169
<212> DNA
<213> Homo sapiens

<400> 75

```
gaccacatcg agcggtgagg cacaggacga gcaggggcgg gaaatgggga agcaggtcaa      60
gaaatatttc cgcaaatcca tctttccttt gacatgccat ttgaggataa tttgcagtgt     120
ttcagctaat aacctaagat aatttacact attattggtt gttaaaact                 169
```

<210> 76
<211> 186
<212> DNA
<213> Homo sapiens

<400> 76

```
tattcctgca ccaactgacc tgaagttcac tcaggtcaca cccacaagcc tgagcgccca      60
gtggacacca cccaatgttc agctcactgg atatcgagtg cgggtgaccc ccaaggagaa     120
gaccggacca atgaaagaag tcaaccttgc tcctgacagc tcatccgtgg ttgtatcagg     180
acttat                                                                186
```

<210> 77
<211> 251
<212> DNA
<213> Homo sapiens

<400> 77

gaggctaagc cgggagcact gattgaggcc aggagttcat gatcagcctg ggcaatgaag 60

tgagaccccg tctctacaaa aaaatatgaa aaaattagcg aggtgtggtg acacatgcct 120

gtagtcccag ctactcaaga ggctgaggta gaggatcact tgagcctacg agttcaaggc 180

tgcagtgagc tatgataact ccactgcact gccgcctgga tgacacagag agaccgtttc 240

taaattaatt a 251

<210> 78
<211> 122
<212> DNA
<213> Homo sapiens

<400> 78

ggaacccacc tagcccaaca agaacaatcc attctacttc ttggaactac gtttattttc 60

cttttccccc atttcctata agataacctc taaccaatga caatctcgac agctattcct 120

gc 122

<210> 79
<211> 54
<212> DNA
<213> Homo sapiens

<400> 79
tgttgaaaat gtagaaaatt ggccggacgg ggtggctcac gtcagtaatt tcag 54

<210> 80
<211> 196
<212> DNA
<213> Homo sapiens

<400> 80

tctggcccgc aatactgtag gaacaagcat gatcttgtta ctgtgatatt ttaaatatcc 60

acagtactca ctttttccaa atgatcctag taattgccta gaaatatctt tttcttacct 120

gttatttatc aatttttccc agtattttta tacggaaaaa attgtattga aaacacttag 180

tatgcagttg ataaga 196

<210> 81
<211> 248
<212> DNA
<213> Homo sapiens

<400> 81

```
agttgctaca aaaactgatt ggtttttgtc acttcatctc ttcactaatg gagatagctt 60
tacactttct gctttaatag atttaagtgg accccaatat ttattaaaat tgctagttta 120
ccgttcagaa gtataataga aataatcttt agttgctctt ttctaaccat tgtaattctt 180
cccttcttcc ctccaccttt ccttcattga ataaacctct gttcaaagag attgcctgca 240
agggaaat 248
```

<210> 82
<211> 291
<212> DNA
<213> Homo sapiens

<400> 82

```
tcaaaccacc atcactaact acaaaatccg ccatcatccc gagcacttca atgggaaacc 60
tcgagaaaat cgggtgcccc actctcggaa ttccatcacc ctcaccaacc tcactccagg 120
cacagagtat gtggtcagca tcgttgcttt taatggcaga gaggaaagtc ccttattgat 180
tggccaacaa tcaacaggta acttttcttg tctgcaaaga aactcagaag actttcctac 240
ccagttggta gattctgtaa agtagcttgc tgttgtctgt catcagctct c 291
```

<210> 83
<211> 209
<212> DNA
<213> Homo sapiens

<400> 83

```
gggcctaaca gtactgtgtg tagtgtttta tttttaacag tagtacacta taacttaaaa 60
tagacttaga ttagactgtt tgcatgatta tgattctgtt tcctttatgc atgaaatatt 120
gattttacct ttccagctac ttcgttagct ttaattttaa aatacattaa ctgagtcttc 180
cttcttgttc gaaaccagct gttcctcct 209
```

<210> 84
<211> 129
<212> DNA
<213> Homo sapiens

<400> 84

```
aggacagctg ctgaaggcac cctctgatga gctcggttac tcagaagagt gaggatgtgt 60
tgaaggtatc tgctgtatgg agtggcagga tgatgtctgt gattgagaaa tataatcccg 120
```

gccaggcga 129

<210> 85
<211> 89
<212> DNA
<213> Homo sapiens

<400> 85

```
aaagtctacc cttaaaccct cagatcagtc tttccaaaga attactctgt ttgcattgtt        60

gtgattgaca tttgtgaagt cccaagaaa                                          89
```

<210> 86
<211> 123
<212> DNA
<213> Homo sapiens

<400> 86

```
aatttatcaa aatgccggta cttaggacct aaatttatct atgtctgtca tacgctaaaa        60

tgatattggt ctttgaattt ggtatacatt tattctgttc actatcacaa aatcatctat       120

att                                                                     123
```

<210> 87
<211> 233
<212> DNA
<213> Homo sapiens

<400> 87

```
aagaccctca actcctggag gaagttctac acgaggctca ccaacagcaa acaaggggag        60

actacagtct gagacccggg gctcagccca tgcccaggcc tcggccgggg cgcaacgatc       120

ccccaaagcc agcgccgtgg agttcgtgcc aatcctgaca tctcgaggtt tcctcactaa       180

caactctctt tcgcaggctc ctttgaacaa ctcagctcct gcaaaagctt ccg              233
```

<210> 88
<211> 204
<212> DNA
<213> Homo sapiens

<400> 88

```
tacattttat ggtgtttcat agccaatccc acagtgtaaa aattcaggaa ttcaatgaaa        60

aaagtctacc cttaaaccct cagatcagtc tttccaaaga attactctgt ttgcattgtt       120

gtgattgaca tttgtgaagt cccaagaaaa gatctgtttt catgacagta gaaaatagaa       180

gtttgcaaat tatttcttta ctca                                              204
```

<210> 89
<211> 240
<212> DNA

<213> Homo sapiens

<400> 89

```
ttctctggaa gaactagcag atcggggcat gctgtgcagc cctctctcag gtggcaggtg        60
tctggatctg ctcaaaggct gccccctta gatgagacaa ctgtgtctag acctcagtcc        120
ctcccatgcc cctccctgcc tttgcctggg gtgagaccat ttgtgaaaga cagagccaag        180
acacagggag tttttcaatt gattctaaag tccatgctct catcaatcca ctgaataatg        240
```

<210> 90
<211> 296
<212> DNA
<213> Homo sapiens

<400> 90

```
cagcggctgc tggaacgcgt tgaccctcac tgtttgtgtg ttttcaggaa ggtgcattcg        60
cgctggtttt caagagtgtc cactacccag gagaagccgt tgccacacgg tgaggcaaaa       120
cacactggca tttcccataa gaaagaacga aaataaaatc acttagctgg agggagacgg       180
gagaggtagg gcctgtggca cgctgaaagg tgctcgtggt gtctaaagaa caactgtggc       240
tcagccccgg ggaccgcgcc ccaccatctc catggctggt ggtgctacac ttttga           296
```

<210> 91
<211> 238
<212> DNA
<213> Homo sapiens

<400> 91

```
acctatgcct cctataagtc cagttgaaat ctcagcctcc ttcaacattt tcttctcgtg        60
tgtggcccac atccctccac ttctccaact tctgtttaat ctgatcacgg ctctttttaa       120
gccctggcag cattttggtc cctgctcctt gcccatagta aaacagcttg aaatatccca       180
tgcaagagag tagtttcaag tgggcgactc tgctctctat ttaaaagcgt gcacaatc         238
```

<210> 92
<211> 183
<212> DNA
<213> Homo sapiens

<400> 92

```
attgagcaga tctataggaa gattgaacct gaatattgcc attatgcttg acatggtttc        60
caaaaaatgg tactccacat atttcagtga gggtaagtat tttcctgttg tcaagaatag       120
cattgtaaaa gcattttgta ataataaaga atagctttaa tgatatgctt gtaactaaaa       180
taa                                                                     183
```

<210> 93
<211> 254
<212> DNA
<213> Homo sapiens

<400> 93

```
gaaatacaga ctggatgtac caccaactac tacctgtaat gacaggcctg tccaacacat        60
ctcccttttc catgactgtg gtagccagca tcggaaagaa cgctgattta aagaggtcgc       120
ttgggaattt tattgacaca gtaccattta atggggagga caaaatgggg caggggaggg       180
agaagtttct gtcgttaaaa acagatttgg aaagactgga ctctaaattc tgttgattaa       240
agatgagctt tgtc                                                         254
```

<210> 94
<211> 199
<212> DNA
<213> Homo sapiens

<400> 94

```
agcagaagac gttttccctg agaagacata gaaagaaaat caactttcac taaggcattt        60
cagaaacata ggctagggta atatgtgtac cagtagagaa gcctgaggaa tttacaaaat       120
gatgcagctc caagccattg tatggcccat gtgggagact gatgggacat ggagaatgac       180
agtagattat caggaaata                                                    199
```

<210> 95
<211> 237
<212> DNA
<213> Homo sapiens

<400> 95

```
atgagtgcat ttcaactatg tcaatggttt cttaatattt attgtgtaga agtactggta        60
atttttttat ttacaatatg tttaaagaga taacagtttg atatgttttc atgtgtttat       120
agcagaagtt atttattttt atggcattcc agcggatatt ttggtgtttg cgaggcatgc       180
agtcaatatt ttgtacagtt agtggacagt attcagcaac gcctgatagc ttctttg          237
```

<210> 96
<211> 188
<212> DNA
<213> Homo sapiens

<400> 96

gtcactactg ggcaaataca cttactgtgt tctagaggca gccctttctt atgcagaaaa 60

tacaatacgc actgcatgag aagcttgaga gtggattcta atccaggtct gtcgaccttg 120

gatatcatgc atgtgggaag gtgggtgtgg tgagaaaagt tttaaggcaa gagtagatgg 180

ccatgttc 188

<210> 97
<211> 48
<212> DNA
<213> Homo sapiens

<400> 97
cttccttctc tcttactcgg agacagtcag aactctcctc cctgacag 48
<210> 98
<211> 167
<212> DNA
<213> Homo sapiens

<400> 98

tacccaatta cccaggtcat aaggtatgtc tgtgtgacac ttatctctgt gtatatcagc 60

atacacacac acacacacac acacacacac acacacaggc atttccacac attacatata 120

tacacatact ggtaaaagaa caatcgtgtg caggtggtca cacttcc 167

<210> 99
<211> 34
<212> DNA
<213> Homo sapiens

<400> 99
gaaaaatata ctaagttggt atactataac actt 34

<210> 100
<211> 277
<212> DNA
<213> Homo sapiens

<400> 100

tgtccttcca ctcaacagtc atcaaccact accgcatgcg gggccatagc ccctttgcca 60

acctcaaatc gtgctgtgtg cccaccaagc tgagacccat gtccatgttg tactatgatg 120

atggtcaaaa catcatcaaa aaggacattc agaacatgat cgtggaggag tgtgggtgct 180

catagagttg cccagcccag ggggaaaggg agcaagagtt gtccagagaa gacagtggca 240

aaatgaagaa atttttaagg tttctgagtt aaccaga 277

<210> 101
<211> 193
<212> DNA
<213> Homo sapiens

<400> 101

```
aagggagaat ggtgtaccct ttatttcttc tgaaatcaca ctgatgacat cagttgttta      60
aacggggtat tgtcctttcc ccccttgagg ttcccttgtg agcttgaatc aaccaatctg     120
atctgcagta gtgtggacta gaacaaccca aatagcatct agaaagccat gagtttgaaa     180
gggcccatca cag                                                        193
```

<210> 102
<211> 125
<212> DNA
<213> Homo sapiens

<400> 102

```
ttccttctct cttactcgga gacagtcaga actctcctcc ctgacagcca caaacctaca      60
gcactgactg cattcagaga ggaacctgca aacaaaactt cacagaaaac tttttgttct     120
tgttc                                                                 125
```

<210> 103
<211> 264
<212> DNA
<213> Homo sapiens

<400> 103

```
gcccagttca ccctgattta ggagaagcca ggaatttccc aggaccctga aggggccatg      60
atggcaacag atttggaacc tcagcctggc cagacacagg ccctccctgt tccccagaga     120
aaggggagcc cactgtcctg ggcctgcaga atttgggttt tgcctgccag ctgcactgat     180
gctgcccctc atctttctgc ccaacccttc cctcaccttg gcaccagaca cccaggactt     240
atttaaactc tgttgcaagt gcaa                                           264
```

<210> 104
<211> 289
<212> DNA
<213> Homo sapiens

<400> 104

```
tctgaactga atgtgactgg tctgtataaa atacatatta gagttcaaag acttggtaca      60
ttaaaaagaa tgtaaaatat ctcattagta gacttttta tattgattac atgttgaaat     120
gataatattt tggatatgca gggttaaatg aaatgttatt aaagttaatt tcacctgttt     180
ctttttaatt ttttgatgtg gctactagaa aaccccaaat tccatatgtg gtttgcattt     240
gtggctccca ttatatttct gttggaaagc tcagctctag aaggaaaga                 289
```

<210> 105
<211> 61
<212> DNA
<213> Homo sapiens

<400> 105

tagtctgaac tgaatgtgac tggtctgtat aaaatacata ttagagttca aagacttggt 60

a 61

<210> 106
<211> 300
<212> DNA
<213> Homo sapiens

<400> 106

agaaataatg tttcgggctg ggcgcggtgg ctcacacctg taatcccagc aatttgggag 60

gctggggcgg gcagatcacc tgaggtcagg agtttgagac tccagcctgg ccaacgtggt 120

aaaaccctgt ctttactaaa aatacaaaaa ttagctgggc atggtggcgg gcacctgtaa 180

tcccagctgc tcagggggct gaggcaggag aattgcttga acctaggagg tggaggttgc 240

agtgagccaa gatcgcgcca ctgcacttca gcctggcctt tagaacgaga ctccctccaa 300

<210> 107
<211> 288
<212> DNA
<213> Homo sapiens

<400> 107

tcaaaagcag atggcctctg gatcagtgcc agagccattc cccgggcaga gaagcatcta 60

gaccttgtca ctaccaaggg tgcggctcca cacccgcggg acccgagcct ctagaaatga 120

gggagaagac gactctgcct ttctgcaatt tagacgtcgc aaggatgaaa acgatgcgga 180

caggctttcc agcagtgagc ctggggaaat ctgcgtcaca tgggcgacag aaggtccctc 240

gaatccctca atccccgtgg cccgcaccac tgtgtaataa tctccaaa 288

<210> 108
<211> 35
<212> DNA
<213> Homo sapiens

<400> 108
ggcgggcgcc tatactccca gttgctcggg aggct 35

<210> 109
<211> 130
<212> DNA
<213> Homo sapiens

<400> 109

gaaagagcat caacagtaac ggcaaacagg ggagctaatg tcaccaacaa aacttgtgag 60

gtcaacccaa agaaagaagg gaataaatga gcaaaacttt ggaaagagtt tctcacatgc 120

tgaaaacact 130

<210> 110
<211> 253
<212> DNA
<213> Homo sapiens

<400> 110

tgtccaagcc ctgtgagact gaaaaagcac tttgaggaac cttaaagacc ttgtttgtac 60

ataagaactg ctagcaaaag agacctcact cttctcttgc tttcgtgaga aaggaggggc 120

gtggatgtag gattgctgtg gaaagcgaac acaaaacaac ccagaatgac tgattaagtg 180

ccttgcaaat ctttattatt atccaaacat ttatgttcat actttcttgt gtacagatgg 240

tgctagtcaa gat 253

<210> 111
<211> 37
<212> DNA
<213> Homo sapiens

<400> 111
cactgcattc ctgggcaacg acaacagtga aactcca 37

<210> 112
<211> 263
<212> DNA
<213> Homo sapiens

<400> 112

gtagtttcca gctaatggat cacctggata agattgcccc tgtctttgtg agagcagcaa 60

ttagctctca ccctgagaag gcggcgtgtt ttgttgcttt gcagccttgg tgtagaggat 120

ggattgggtt tgcgtctttt catttgaaag taggttatcg attagcacgg atgccaattt 180

actacagagg ctgttgccaa tgcctcttga tctcattatg gtgtaaattt tcaagattgt 240

atcacattag ctcaggagaa ttc 263

<210> 113
<211> 165
<212> DNA
<213> Homo sapiens

<400> 113

ctcagggagc gaacgtggat gaaaaccaca gggattccgg acgccagacc ccattttata 60

cttcactttt ctctacagtg ttgttttgtt gttgttggtt tttatttttt atactttggc 120

cataccacag agctagattg cccaggtctg ggctgaataa aacaa 165

<210> 114
<211> 109
<212> DNA
<213> Homo sapiens

<400> 114

aacatagcaa atggcatcac tgtgtttgac ttcttgtgaa atttctgtac tttgtatata 60

aaatacataa aacaatagat tagaaatcaa aagatatctc tggcctgca 109

<210> 115
<211> 191
<212> DNA
<213> Homo sapiens

<400> 115

taggtggtag atattgaggc caagaatatt gcaaaataca tgaagcttca tgcacttaaa 60

gaagtatttt tagaataaga atttgcatac ttacctagtg aaacttttct agaattattt 120

ttcactctaa gtcatgtatg tttctctttg attatttgca tgttatgttt aataagctac 180

tagcaaaata a 191

<210> 116
<211> 277
<212> DNA
<213> Homo sapiens

<400> 116

gtgccatcta ccgaaactcc acagtttcca ttgtgaatgg cttctttggt gcagagttcc 60

aaaaattatg tagcccagct ctttaatttt gtaacatcta atgatatcac cgccttgaag 120

tgattaaagt agattgctta aagaattaaa gctttaaaga tgaaagatgt tattgctttt 180

gctggacatg aggaacagtt gtaaagtttc caggtttaca ataactttct ggaaccctct 240

cagtgaactg tttcttgtaa aagttttccc taagata 277

<210> 117
<211> 246
<212> DNA
<213> Homo sapiens

<400> 117

aagcagtcta ctagattgtg atcccttgag atatggaagg atgccttttt ttctctgcat 60

ttaaaaaaat cccccagcac ttcccacagt gcctattgat acttggggag ggtgcttggc 120

acttattgaa tatatgatcg gccatcaagg gaagaactat tgtgctcaga gacactgttg 180

ataaaaactc aggcaaagaa aatgaaatgc atatttgcaa agtgtattag gaagtgttta 240

tgttgt 246

<210> 118
<211> 172
<212> DNA
<213> Homo sapiens

<400> 118

agcccaccca ttgaagtctc cttgggccac caaaggtggt ggccatggta ccggggactt 60

gggagagtga gacccagtgg agggagcaag aggagaggga tgtcgggggg gtggggcacg 120

gggtagggga aatggggtga acggtgctgg cagttcggct agatttctgt ct 172

<210> 119
<211> 157
<212> DNA
<213> Homo sapiens

<400> 119

tatgaattcc attcaaatcg ttcctttttg ttaacaaggg gcatggggag gggtgggggt 60

gggggggcag aggcgtctga ccccaggaac ctgcagggcg gggctgggtc ggtgcccttt 120

aaggacaatt ttgaccttgt tcaacctttc cacaaag 157

<210> 120
<211> 36
<212> DNA
<213> Homo sapiens
<400> 120
tgggggggca gaggcgtctg accccaggaa cctgca 36

<210> 121
<211> 251
<212> DNA
<213> Homo sapiens

<400> 121

aacctcaggg agagtaagct ctagtccctc tgtcctgtag aaagagccct gaagaatcag 60

caattttgtt gctttattgt ggcatctgtt cgaggtttgc ttcctcttta agtctgtttc 120

ttcattagca atcatatcag ttttaatgct actactaaca atgaacagta acaataatat 180

ccccctcaat taatagagtg ctttctatgt gcaaggcact tttcacgtgt cacctatttt 240

aacctttcca a 251

<210> 122
<211> 219
<212> DNA
<213> Homo sapiens

<400> 122

tgttgcttta ttgtggcatc tgttcgaggt ttgcttcctc tttaagtctg tttcttcatt 60

agcaatcata tcagttttaa tgctactact aacaatgaac agtaacaata atatccccct 120

caattaatag agtgctttct atgtgcaagg cacttttcac gtgtcaccta ttttaacctt 180

tccaaccaca taaataaaaa aggccattat tagttgaat 219

<210> 123
<211> 267
<212> DNA
<213> Homo sapiens

<400> 123

ttgctttgta tgcactttgt ttttttcttt gggtcttgtt ttttttttcc acttagaaat 60

tgcatttcct gacagaagga ctcaggttgt ctgaagtcac tgcacagtgc atctcagccc 120

acatagtgat ggttcccctg ttcactctac ttagcatgtc cctaccgagt ctcttctcca 180

ctggatggag gaaaaccaag ccgtggcttc ccgctcagcc ctccctgccc ctcccttcaa 240

ccattcccca tgggaaatgt caacaag 267

<210> 124
<211> 152
<212> DNA
<213> Homo sapiens

<400> 124

caccatgggc ggcaacgctg aaggacagcc ctgcaagttt ccattccgct tccagggcac 60

atcctatgac agctgcacca ctgagggccg cacggatggc taccgctggt gcggcaccac 120

tgaggactac gaccgcgaca agaagtatgg ct 152

<210> 125

<211> 126
<212> DNA
<213> Homo sapiens

<400> 125

ttcctctctg taattggtta tcaggaagaa tttgcttaat gactaacacc ctaagcatca 60

gacctggaat ttggagttgc aaagtgacta tcttcccatt tcccatctca ttttcaataa 120

cttcag 126

<210> 126
<211> 199
<212> DNA
<213> Homo sapiens

<400> 126

tctgcttagc cctaggagtc tggttccctg ccttcagttg cagagtgatg tgtttgtgtc 60

attgttgatg tcacctccta aaaagacctt cactttctgg ctgccacaaa gccatatgtg 120

ttgctcccca tatacagcct gacagagtaa atggagagga agtgctggat ttgtgtatca 180

ctggctatca gttcctcat 199

<210> 127
<211> 224
<212> DNA
<213> Homo sapiens

<400> 127

ccagtataat taggggtgtt tcagagcatc cccagttatt tagcacaaca ctgaaggagc 60

acatcccctc tccattttga cttctctccc cacttttaca gccactgcct tcgtcagttt 120

tgtagaggtt tgatttccat gtgggttttg ttgtcattgt tttgcatttt tgttttgtta 180

ttgatattgt ttgctttcat tgctaaaact catatacgac ttac 224

<210> 128
<211> 230
<212> DNA
<213> Homo sapiens

<400> 128

ttggtccatt tcttacaagt catgtatccc aaactgtgtc agcactggca agtgatctgg 60

atgatggctg cagtgatgct ggtcttgact gttgtgctgg ggctctacaa ttcctataac 120

tcttgtgcag agcaggctga tgggcccctt ggaagatcca cttgctcggc agcccagaag 180

gactcctggt ggagctcagg actccagcat gagcagccta cagagcagta 230

<210> 129

<211> 249
<212> DNA
<213> Homo sapiens

<220>
<221> misc_feature
<222> (150)..(151)
<223> n is a, c, g, or t

<220>
<221> misc_feature
<222> (153)..(154)
<223> n is a, c, g, or t

<220>
<221> misc_feature
<222> (156)..(160)
<223> n is a, c, g, or t

<220>
<221> misc_feature
<222> (162)..(167)
<223> n is a, c, g, or t

<400> 129

```
tggtgcacct tggacacgtg gacaaggccc aggcgccctc tgctcttctg ccctcgatgc      60
cacatggcgg tgaacacatc tgaagccact atgtttcctg gctctaaggc tcgtctgtgt     120
aacccataaa acctgctttg attccaaaan nannannnnn annnnnnaag gaggtgcccc     180
gatgcttggg gtgggtgttc ctgccggggc ttcaaggggt gacttctgta ggctgtccag     240
gtccgaggc                                                             249
```

<210> 130
<211> 89
<212> DNA
<213> Homo sapiens

<220>
<221> misc_feature
<222> (60)..(60)
<223> n is a, c, g, or t

<400> 130

```
acagaatgag accttccggc ccaagcgtgg cgctgcgggc actttggtag actgtgccan      60
cacggcgtgt gttgtgaaac gtgaaataa                                        89
```

<210> 131
<211> 91
<212> DNA
<213> Homo sapiens

<220>
<221> misc_feature
<222> (28)..(28)
<223> n is a, c, g, or t

<400> 131

```
acaagcagta ccgtcagtac ccacttgnct ttagcccatt atgggaatct ctgatgcctt        60
tgtgaccact ggagagttta atcctcttgg t                                      91
```

<210> 132
<211> 228
<212> DNA
<213> Homo sapiens

<400> 132

```
ggaaaggtga tctgcactgt atcttgggtt tgttggctat gcttcctttg atgacatata        60
ttatacagta tatatataca tatatttttt ttgttagagt tctagccatt ttatttctcc       120
gcagggtcct ttctcagaca ttactgcatg ctgtatatgg cgttagctgt gtgttgatct       180
tctaaaagat gatagagttt actggtaatt gtgtaatcag ctcctgcc                   228
```

<210> 133
<211> 183
<212> DNA
<213> Homo sapiens

<400> 133

```
acagagtcta cagacattaa aaggataata atggaatttt taaaaaataa gagcactttg        60
ggaggccgag gtgggtggat catttgaggc caggagttca agaccagcct ggccaacatg       120
gtgaaaccct gtctcgacta aaaatacaaa aaattagctg agcatggtgg cagacacttg       180
taa                                                                     183
```

<210> 134
<211> 179
<212> DNA
<213> Homo sapiens

<400> 134

```
agaggttata ggtcactcct ggggcctctt gggtccccca cgtgacagtg cctgggaatg        60
tattattctg cagcatgacc tgtgaccagc actgtctcag tttcactttc acatagatgt       120
ccctttcttg gccagttatc ccttcctttt agcctagttc atccaatcct cactgggtg        179
```

<210> 135
<211> 274
<212> DNA

<213> Homo sapiens

<400> 135

```
aaacagattc ctgccaggga gactcagggg gacccctcgt ctgttccctc caaggccgca      60
tgactttgac tggaattgtg agctggggcc gtggatgtgc cctgaaggac aagccaggcg     120
tctacacgag agtctcacac ttcttaccct ggatccgcag tcacaccaag gaagagaatg     180
gcctggccct ctgagggtcc ccagggagga aacgggcacc acccgctttc ttgctggttg     240
tcatttttgc agtagagtca tctccatcag ctgt                                 274
```

<210> 136
<211> 282
<212> DNA
<213> Homo sapiens

<400> 136

```
ggatggcaca catcagcttt caggaaaagc acatgaaagg tgctagggct cttggagctg      60
actgtaggtt tgggagttgt ctgtctcctt gctctagatt acagctctgt gtgttgtgtg     120
gggtctccat ggtttgccaa attatctctt cctcacttag ccacaaggct gacagttagg     180
aactatctct tcttgattgc attaggttgg ctgcttcctg aatgcatatc aaaaggctcc     240
ttcctttagt tcagtgcttt caacctgagc tgtgcatcag aa                        282
```

<210> 137
<211> 203
<212> DNA
<213> Homo sapiens

<400> 137

```
aaatcataaa gccttcatca ctcccacatt tttcttacgg tcggagcatc agaacaagcg      60
tctagactcc ttgggaccgt gagttcctag agcttggctg ggtttaggct gttctgtgcc     120
tccaaggact gtctggcaat gacttgtatt ggccaccaac tgtagatgta tatatggtgc     180
ccttctgatg ctaagactcc aga                                             203
```

<210> 138
<211> 245
<212> DNA
<213> Homo sapiens

<400> 138

tgggcttccc tctacagtgt ggaactcagg ttgtttcttc tttcctccct gaaatgacat 60

gagtttgcag cggatggtga actgaagaaa ccataggagg ctctgtcttc ttgcctgaat 120

ttcagttgga agcttggaga tttggggttc aacagagata aggaagtgta agccttcatc 180

ccgtctggtg gttggcgatc acacaccgct ctgtgctgag gctaatggcc atgatcagag 240

ttgac 245

<210> 139
<211> 92
<212> DNA
<213> Homo sapiens

<400> 139

ctcaaaaaag tggttttgac cagagaggcc cagatggagg ctgttcattc cctgcagtgt 60

cggcattgta aataaagcct gagcacttgc tg 92

<210> 140
<211> 238
<212> DNA
<213> Homo sapiens

<400> 140

gagcaggagc ctctacacag cctctggctc ttaggtccca gtcatgtttg caccccctca 60

aaggggcagg accagccctt cctttcagtg tccataccag gggccttcca tgtgctgatg 120

ggtgatgtga ctgtggtcag caggcttggg aagtgctgct gctgtagctt gagttgggct 180

ggggtcttgg taggacgctg atctcagaag tccccaaagt tcactgtgta ggtctcta 238

<210> 141
<211> 128
<212> DNA
<213> Homo sapiens

<400> 141

gaccagacag tgctgttggt gactgtccct gacttcagtg ccacgcagac cgcctgcctt 60

gtgaacctgc gcagcctggc ctgccagccc atcagcttct cgggcttcgg ggcagaggac 120

gatgacct 128

<210> 142
<211> 219
<212> DNA
<213> Homo sapiens

<220>
<221> misc_feature
<222> (48)..(48)

<223> n is a, c, g, or t

<400> 142

attgctagct gttaaacaag cccaaatttg atatactttt tatatttnaa aaattatatt 60

cactgccccc ataagagcaa tcaaggcatg tctttaaatt ctatacatag atatagccaa 120

aaatagtgca tttagtaaca ttcttttcca aaactatatt cttgggaatg aatatctgtt 180

tcttctaaca gtttgagtga taatctatac ctgtagata 219

<210> 143
<211> 245
<212> DNA
<213> Homo sapiens

<400> 143

atgggtggct aactctggga agatacttgt gttaaacttt atatgacatt taataaccct 60

tcatcataag gcaatgtttt ttacaaaaag attgaaaaaa tcatgtaagt catttactct 120

gcaaaaatgg cacattaggt ggggttccaa aatccataat gaaacaatgt gttttgcaac 180

taagaaacat tcattatgat atatggaaaa cactgtctgt ctacttgtcc tttacgaaaa 240

aatgt 245

<210> 144
<211> 227
<212> DNA
<213> Homo sapiens

<400> 144

ttataaaaat aggtgcttgg tgtacaaaac tttttatatc aaaaggcttt gcacatttct 60

atatgagtgg gtttactggt aaattatgtt attttttaca actaattttg tactctcaga 120

atgtttgtca tatgcttctt gcaatgcata ttttttaatc tcaaacgttt caataaaacc 180

atttttcaga tataaagaga attacttcaa attgagtaat tcagaaa 227

<210> 145
<211> 62
<212> DNA
<213> Homo sapiens

<400> 145

ggacttggga acaggacttt atacctcttt tactgtaaca agtactcatt aaaggaaatt 60

ga 62

<210> 146

<211> 66
<212> DNA
<213> Homo sapiens

<400> 146

```
gtttggactt gggaacagga ctttatacct tttttactgt aacaagtact cattaaagga        60
aattga                                                                   66
```

<210> 147
<211> 225
<212> DNA
<213> Homo sapiens

<220>
<221> misc_feature
<222> (114)..(114)
<223> n is a, c, g, or t

<220>
<221> misc_feature
<222> (141)..(141)
<223> n is a, c, g, or t

<220>
<221> misc_feature
<222> (146)..(147)
<223> n is a, c, g, or t
<400> 147

```
caaattcaag tcactagact tcagagttca acacctggac atgagaagat attatattat        60
gtaccataaa tatttcctgt atctgactgc ctgaacatat taccacatcc tgtnccttag       120
tatcctacag agacaaaaaa naaaannaaa aaaaaaaaaa acctttgttc aggtttttat       180
gtaaatattt ttcatgttcc actttctata ctttcagcaa ctaaa                       225
```

<210> 148
<211> 167
<212> DNA
<213> Homo sapiens

<400> 148

```
atatgagtgg gtttactggt aaattatgtt attttttaca actaattttg tactttcaga        60
atgtttgtca tatgcttctt gcaatgcata ttttttaatt tcaaacgttt caataaaacc       120
atttttcaga tataaagaga attacttcaa attgagtaat tcagaaa                     167
```

<210> 149
<211> 256
<212> DNA
<213> Homo sapiens

<400> 149

```
atgactgcaa aaatgggtgg ctaactctgg gaagatactt gtgttaaact ttatatgaca      60
tttaataacc cttcatcata aggcaatgtt ttttacaaaa agattgaaaa aatcatgtaa     120
gtcatttact ctgcaaaaat ggcacattag gtggggttcc aaaatccata atgaaacaat     180
gtgttttgca actaagaaac attcattatg atatatggaa aacactgtct gtctacttgt     240
cctttacgaa aaaatg                                                     256
```

<210> 150
<211> 266
<212> DNA
<213> Homo sapiens

<400> 150

```
gcaagccagc cgccggtgct gttgacccaa gggccgtggt ccacggtact tgaagaagcc      60
agagcccaca tcctgtgcac tgctgaagga ccctacgctc ggtggcctgg cacctcactt     120
tgagaagagt gagcacactg gctttgcatc ctggaagacc tgcagggggc ggggcaggaa     180
atgtacctga aaaggatttt agaaaaccct gggaaaaccc accacaccac cacaaaatgg     240
cctttagtgt atgaaatgca catgga                                          266
```

<210> 151
<211> 133
<212> DNA
<213> Homo sapiens

<400> 151

```
gatgggcctt cagggaagtt cctggcagga acagtccttc catttgtttg ggcggatggt      60
ccggaagagc aaagccatgc taatgagaca gaggaatagc aagtcccctt caagccgaca     120
ggaaggggcc tca                                                        133
```

<210> 152
<211> 287
<212> DNA
<213> Homo sapiens

<400> 152

```
gattggatta aagacctggc acttcagtaa ctcagcacgc ttccacttca ctcaacttaa      60
gagagttcat tgacagtgtt aggatgtgaa ggctgggaaa cacttatttt gcttcaagag     120
ttccacttgg ctctcccaaa taggtacctc aaaaactgtt agcaagcggc atttggatgt     180
cttgacaggg gctttgcagg gatttttagg gtttttttcca cattgtccac attaatggtt     240
ggcatgattg tgcttgcagg ccaagaaatg atcatacccc ttgccaa                   287
```

<210> 153
<211> 243
<212> DNA
<213> Homo sapiens

<400> 153

**acatcattgt tgaaaccaga ccctgtagtc cagtggtgct gccctgttgt gcaaactgct 60**

**cctttttctc gtgtttttgt aaagagcttc catctgggct ggacccagtt cttgcacata 120**

**caagacaccg ctgcagtcag ctaggacctt tccgccatgt attctattct gtagtaaagc 180**

**atttccatca acaatgccta attgtatctg ttatttttgg tttaacacac actgattcat 240**

**act 243**

<210> 154
<211> 284
<212> DNA
<213> Homo sapiens

<400> 154

**ttcgcattta aggtataggt tgttttaggc atgggcagta ggcgctttca gcacaggctc 60**

**cctgctggtc actctttgct gtagtttcct gctccatcca tggggtcgga ggtcttacct 120**

**cctgacgtta gagtcatttg cagcaggtgg gagtgaaagt gggccgggtg ccaggtcttc 180**

**cttacctgaa cctctcctgt cttcctggca cattaggaat gtccaagctt atttctattg 240**

**gtgaatgctc ccacatggga tggataatgg cctgtggaac attc 284**

<210> 155
<211> 35
<212> DNA
<213> Homo sapiens
<400> 155
tcacatcaga aaactaaatt ccggctgggt gcggt 35

<210> 156
<211> 128
<212> DNA
<213> Homo sapiens

<400> 156

**actgaagtcc agagggtttt cttgaactgc ccaaggacac actactattt agtgatacaa 60**

**gctgggacta aaactgaggt cccccgacac ctggtccaga gttctttcta ctatacatca 120**

**aagaatat 128**

<210> 157
<211> 209
<212> DNA
<213> Homo sapiens

<400> 157

gatcctatca gtttcccatg gtgccggggg gagaccggtc tccttccaga atgcttccgc 60

catgcaccac cacctcgaat ggcagcacgc tattaaatcc aaatttgcct aaccagaatg 120

atggtgttga cgctgatgga agccacagca gttccccaac tgttttgaat tctagtggca 180

gaatggatga atctgtttgg cgaccatat 209

<210> 158
<211> 208
<212> DNA
<213> Homo sapiens

<400> 158

gacagaagct tgatgactct aaacctagtt tgttctctga ccgcctcagt gatttagggc 60

gcattcctca tcccagtatg agagtaggtg tcccgcctca gaacccacgg ccctccctga 120

actctgcacc aagtccttgt aatccacaag gacagagtca gattacagac cccaggcagg 180

cacagtcttc cccgccgtgg tcctatga 208

<210> 159
<211> 180
<212> DNA
<213> Homo sapiens

<400> 159

tttttccaa ttgctattgc ccaagaattg ctttccatgc acatattgta aaaattccgc 60

tttgtgccac aggtcatgat tgtggatgag tttactctta acttcaaagg gactatttgt 120

attgtatgtt gcaactgtaa attgaattat ttggcatttt tctcatgatt gtaatattaa 180

<210> 160
<211> 127
<212> DNA
<213> Homo sapiens

<400> 160

gggcccctaa tatcccagtt taatattcca ataccctaga agaaaacccg agggacagca 60

gattccacag gacacgaagg ctgcccctgt aaggtttcaa tgcatacaat aaaagagctt 120

tatccct 127

<210> 161
<211> 224
<212> DNA
<213> Homo sapiens

<400> 161

tgttacttcg ttcctcctcc tattttgagc tatgcgaaat atcatatgaa gagaaacagc 60

tcttgaggaa tttggtggtc ctctacttct agcctggttt tatctaaaca ctgcaggaag 120

tcaccgttca taagaactct tagttacctg tgttggataa ggcacggaca gcttctctgc 180

tctgggggta tttctgtact aggatcagtg atcctcccgg gagg 224

<210> 162
<211> 237
<212> DNA
<213> Homo sapiens

<400> 162

tccagctaca atacttccat ttattagaag cacattaacc atttctatag catgatttct 60

tcaagtaaaa ggcaaaagat ataaatttta taattgactt gagtacttta agccttgttt 120

aaaacatttc ttacttaact tttgcaaatt aaacccattg tagcttacct gtaatataca 180

tagtagttta cctttaaaag ttgtaaaaat attgctttaa ccaacactgt aaatatt 237

<210> 163
<211> 172
<212> DNA
<213> Homo sapiens

<400> 163

tgcagtgcta gtcccggcat cctgatggct ccgacaggcc tgctccagag cacggctgac 60

catttttgct ccgggatctc agctcccgtt ccccaagcac actcctagct gctccagtct 120

cagcctgggc agcttccccc tgccttttgc acgtttgcat ccccagcatt tc 172

<210> 164
<211> 227
<212> DNA
<213> Homo sapiens

<400> 164

gttgtggggt caaccgtaca atggtgtggg agtgacgatg atgtgaatat ttagaatgta 60

ccatattttt tgtaaattat ttatgttttt ctaaacaaat ttatcgtata ggttgatgaa 120

acgtcatgtg ttttgccaaa gactgtaaat atttatttat gtgttcacat ggtcaaaatt 180

tcaccactga aaccctgcac ttagctagaa cctcattttt aaagatt 227

<210> 165
<211> 116
<212> DNA
<213> Homo sapiens

<400> 165

ttcctttgag gttgatcgtt gtgttgtttt gctgcacttt ttactttttt gcgtgtggag 60

ctgtattccc gagaccaacg aagcgttggg atacttcatt aaatgtagcg actgtc 116

<210> 166
<211> 224
<212> DNA
<213> Homo sapiens

<400> 166

gcggtgttcc taatcacatg tttgtactat aatttcacgt gggttcagtt aaactagagc 60

taaggttgtc catggcatgt tgaatcaaaa ataaaaatgg tcagtgagta aaagtaacgt 120

actatgacaa acgtgactta cacctgtatg tgagcaaaca ccttgtgtgt gtgtgtgtgt 180

gtgtgtgtgt gttaccagca tacaccctag taggtgcagc ctcc 224

<210> 167
<211> 51
<212> DNA
<213> Homo sapiens

<400> 167
ttcacgtggg ttcagttaaa ctagagctaa ggttgtccat ggcatgttga a 51

<210> 168
<211> 144
<212> DNA
<213> Homo sapiens

<400> 168

ggcacaccca ataatcagtc atgtgtaata tgcacaagtt tgtttttgtt tttgtttttt 60

ttgttggttg gtttgttttt ttgctttaag ttgcatgatc tttctgcagg aaatagtcac 120

tcatcccact ccacataagg ggtt 144

<210> 169
<211> 246
<212> DNA
<213> Homo sapiens

<400> 169

accccaaacc caagtgcctt cagaggataa atatcaatgg aactcagaga tgaacatcta 60

acccactaga ggaaaccagt ttggtgatat atgagacttt atgtggagtg aaaattgggc 120

atgccattac attgcttttt cttgtttgtt taaaaagaat gacgtttaca tataaaatgt 180

aattacttat tgtatttatg tgtatatgga gttgaaggga atactgtgca taagccatta 240

tgataa 246

<210> 170
<211> 217
<212> DNA
<213> Homo sapiens

<220>
<221> misc_feature
<222> (29).. (29)
<223> n is a, c, g, or t

<220>
<221> misc_feature
<222> (68)..(68)
<223> n is a, c, g, or t

<220>
<221> misc_feature
<222> (177)..(177)
<223> n is a, c, g, or t

<400> 170

tgaaaacaag catgagtaga tcctgcttnc caatgggggc cttcccgtct gcttactgct 60

taggtttnct gcggtgggtc tgagcaatgc agaaagtttc ttacaatttc tctggattga 120

aacaagaaat agctccctcg ctcctcttca taaatttctc agagaaaatc taagttncca 180

ggaagtagcc aaagaccagg agattgtgga atcaaaa 217

<210> 171
<211> 254
<212> DNA
<213> Homo sapiens

<400> 171

tgagcttccc ttggacacta actcttccca gatgatgaca atgaaattag tgcctgtttt 60

cttgcaaatt tagcacttgg aacatttaaa gaaaggtcta tgctgtcata tggggtttat 120

tgggaactat cctcctggcc ccaccctgcc ccttcttttt ggttttgaca tcattcattt 180

ccacctggga atttctggtg ccatgccaga aagaatgagg aacctgtatt cctcttcttc 240

gtgataatat aatc 254

<210> 172
<211> 225
<212> DNA
<213> Homo sapiens

<400> 172

tagaggcttc ttagattctc ccagcatccg cctttccctt tagccagtct gctgtcctga 60

aacccagaag tgatggagag aaaccaacaa gagatctcga accctgtcta gaaggaatgt 120

atttgttgct aaatttcgta gcactgttta cagttttcct ccatgttatt tatgaatttt 180

atattccgtg aatgtatatt gtcttgtaat gttgcataat gttca 225

<210> 173
<211> 257
<212> DNA
<213> Homo sapiens

<400> 173

gaaagcctga gtttgcaacc agttgtaggg tttttgttgt gttttttttt ttttttgaa 60

ataaaactat aatataaatt ctcctattaa ataaaattat tttaagtttt agtgtcaaaa 120

gtgagatgct gagagtaggt gataatgtat attttacaga gtgggggttg gcaggatggt 180

gacattgaac atgattgctc tctgtctctt ttttcagctt atgggtattt atcttctatt 240

agtatttgta tcttcag 257

<210> 174
<211> 267
<212> DNA
<213> Homo sapiens

<400> 174

tcctgtaaaa tacagtgcag gccaggcgcg gtgactcacg cctgtaatcc cagcattttg 60

ggaggccaag ggtggcggat catgaggtca ggagatcgag accatcctgg ctaacacggt 120

gaaaccccat ctttactaaa aatacaaaaa attaaccagg tgtggtggcg cacatctgaa 180

gtcccagcta cttgggaggc tgaggcagga taatcactta aacttgggag gtggaggttg 240

cagtgagacg acacctcgcc actgcac 267

<210> 175
<211> 169
<212> DNA
<213> Homo sapiens

<400> 175

atccacagat atagccaagt agattgggta gaggatacta tttccagaat agtgtttagc 60

tcacctaggg ggatatgttg tatacacatt tgcatatacc cacatggggg acataagcta 120

atctttttac aggacacaga attctgttca atgctgttaa atatgccaa 169

<210> 176
<211> 237
<212> DNA
<213> Homo sapiens

<400> 176

```
tctgaacatc agtattgcag ttgtggaaga gcagaaggag gatacatttg tttgtgttgc      60
tccccaaaat tccaccttgc atttgcatca caaacttccc tcaattgagg cagttttctt     120
tgttagaaca ttaagtctgt gtattgtaat agagtgggct caatatttta ctataaagca     180
tttaataaac tgttattaat agaagtttgt gttcttcaca cctttgctat tgctttt        237
```

<210> 177
<211> 225
<212> DNA
<213> Homo sapiens

<400> 177

```
tactgtccaa tagcacctca ttcccacatt ttattttccc ccggtattct ttagatccta      60
gtatttggaa aacaatcggc taaccttgac atttcttttt accttcatat gccactatct     120
cggtagttca aaaaaattta gttcttgata aattgccttg aagtttacct tgtgctggag     180
agccttatga taactccaaa gactttctta cggtataata catgt                     225
```

<210> 178
<211> 280
<212> DNA
<213> Homo sapiens

<400> 178

```
atttttctat ttgtggtata tgtatatatg tacctatatg tatttgcatt tgaaattttg      60
gaatcctgct ctatgtacag ttttgtatta tactttttaa atcttgaact ttataaacat     120
tttctgaaat cattgattat tctacaaaaa catgatttta aacagctgta aaatattcta     180
tgatatgaat gttttatgca ttatttaagc ctgtctctat tgttggaatt tcaggtcatt     240
ttcataaata ttgttgcaat aaatatcctt gaacacaaaa                           280
```

<210> 179
<211> 204
<212> DNA
<213> Homo sapiens

<400> 179

agaaggaatg actctagcta caataatacc cagtatgttt aagcaggttc ccttggttgt 60

tgcattaaaa gtaatccccc tttaggtatt ttagagccca gaacaaccct gtgttgattt 120

agtaggtttt tatttttcct ttttttttac aatgcccata atactttcct gtatttatat 180

cataacgtgt atagtgtaaa atgt 204

<210> 180
<211> 157
<212> DNA
<213> Homo sapiens

<400> 180

ttacctgcat gaacccatct gcataccaaa ggacttattc tatgaaatac tttaaaaatt 60

cctcatcagc aaaggacaat tcaataaata catccaaaca tgatcatcgt tggagccgga 120

ggtggcagga gtcgaggcgc tgatccctaa aatggcg 157

<210> 181
<211> 237
<212> DNA
<213> Homo sapiens

<400> 181

acaaaagctc ccctgatcca actagcacac tgtcaaatac agtgtcatat gagaggtcca 60

cagacggtag tttccaagac cgtttcaggg aattcgagga ttccacctta aaacctaaca 120

gaaaaaaacc cactgaaaat attatcatag acctggacaa agaggacaag gatttaatat 180

tgacaattac agagagtacc atccttgaaa ttctacctga gctgacatcg gataaaa 237

<210> 182
<211> 115
<212> DNA
<213> Homo sapiens

<220>
<221> misc_feature
<222> (81)..(81)
<223> n is a, c, g, or t

<400> 182

ttacctgcat gaaccgtaag tggtccttta gaaagaatgg actaccgtgc tataacaact 60

actagacacc ttcattttac nggctgtggt atcggcagtt tagggtatgg agcaa 115

<210> 183
<211> 230
<212> DNA

<213> Homo sapiens

<400> 183

ttgctctttg actgtcagca agactgaaga tggcttttcc tggacagcta gaaaacacaa 60

aatcttgtag gtcattgcac ctatctcagc cataggtgca gtttgcttct acatgatgct 120

aaaggctgcg aatgggatcc tgatggaact aaggactcca atgtcgaact cttctttgct 180

gcattccttt ttcttcactt acaagaaagg cctgaatgga ggacttttct 230

<210> 184
<211> 272
<212> DNA
<213> Homo sapiens

<400> 184

tgaataataa aatttaagtg gccaggcatg gtggcttacg cctgtaatcc cagctctttg 60

ggaggtcaag gcgggcggat caactgaggt cagaagttcg agaccagcct ggccaacatg 120

gcgaaacccc atctttacta aaagtacaaa aattagccgg gtgtggtggc gggtgcctgt 180

aatttcagct aatcaggagg ccgaggcagg agaatagctt gaacctggga ggcagaggtt 240

gcagtgagca gagatcatgc caccccactc ca 272

<210> 185
<211> 113
<212> DNA
<213> Homo sapiens

<400> 185

caaagtgcta ataattaact caaccaggtc tactttttaa tggctttcat aacactaact 60

cataaggtta ccgatcaatg catttcatac ggatatagac ctagggctct gga 113

<210> 186
<211> 238
<212> DNA
<213> Homo sapiens

<400> 186

agttgtgcta ccttaatttt gtgtttccag aaataaaaat taaccttagt tatgctgtca 60

tttttaacta ataaaaaaag tataattcat aaaacttttg gctttataag ataattataa 120

aattatatat ttttttctgt ttttgtgggg ttgggaaaac attttcttat ttctattcac 180

tcttcaaatg caggtctcat aatatgtgtc aatgatataa gatgatggaa gactttgt 238

<210> 187

<211> 265
<212> DNA
<213> Homo sapiens

<400> 187

aaaatacttc tagttatggt tgtattaatt aatttgattg tgataatgat tacacaatgt 60
ttacctatat caaatcatca tatcgtatac cttaaatata tataactttt atgtgtcaat 120
tataactcag taagtctggg aaaatatcgt taagtcaaag attagagtca acagaaataa 180
agaaaaatca tactttgata acttcaggac taatcaagga tcaatgggtg acatgatatc 240
atgctatgtg ccattttgtg ttaaa 265

<210> 188
<211> 224
<212> DNA
<213> Homo sapiens

<400> 188

tcagtgcacg atgctccagc cacactggcc atctttcggt tcctgataca aaaaaaaaca 60
cgttcctttt ccatggaaag caggtcaccc ttgttatttt gtatcgatga caactcttta 120
aacttatttt gctttttggc tttatgtatg tgtgtgggtg ggtgggactg actgccccac 180
tagaatgtaa gctccatgag ggcagggaat cttgctttct tgtt 224

<210> 189
<211> 273
<212> DNA
<213> Homo sapiens

<400> 189

ccttgaaaag cagtgtcaga gcagaattat aaggcatttt taatattccc tgttttaata 60
aaactttttt tatgtttgat ttttttttat attttttgtc cgcacgtata tagatgtgga 120
tacataacat ttaacacggt tgcaatcaga gggtgatttg atttgttaac ttaatgtcac 180
atcataaaca ttttacatgc tgttatataa tgtacataat catttttaat gactacataa 240
catcccatcc tattgacgaa tcattatgtc ctt 273

<210> 190
<211> 260
<212> DNA
<213> Homo sapiens

<400> 190

cttgaacatg ggaaggagtt gttgcagtaa gccaagatgt gccactgtac tccagcctgg 60

gagacagagt gagactctgt ctcaaaaaat gaatgaataa ataaataaat aaataataaa 120

aaagatgatt attaacaatg ccagttaata ttaacaatat taatagtatt atttattact 180

aatgccattt tttcattttt gttaaagtat ttttattatt ttagtttaaa ttattattat 240

gagacaaagg cctccatttc 260

<210> 191
<211> 256
<212> DNA
<213> Homo sapiens

<400> 191

actgcattgc tggaccctct gtgaaactga agccttctct acttgttttt atctcaagtg 60

aacctggaga agcaacaata atggaccttc tcccctagtc aaatagcctg tggacctccc 120

ctcatagtca gtctccaaaa acatgtatct ggaattaatt tattacaaag agaagtttag 180

tgtttcttct tttttacatg cgctcaatac tgactactgg ccagacacag caccatcctc 240

ttacaaacat catttc 256

<210> 192
<211> 129
<212> DNA
<213> Homo sapiens

<400> 192

gatccagaac tgtggctggg caccgtggct cacatctgta atctcatcac tttgggaagg 60

ctaaggcggg tggatcacct aaggtaagga gttcgaaccc agcctttaca atgtaatgaa 120

accctgcct 129

<210> 193
<211> 267
<212> DNA
<213> Homo sapiens

<400> 193

ggaggcagac aatgacccca cggctcctcc ttatgactcc attcaaatct acggttatga 60

aggcaggggc tcagtggccg ggtccctgag ctccctagag tcggccacca cagattcaga 120

cttggactat gattatctac agaactgggg acctcgtttt aagaaactag cagatttgta 180

tggttccaaa gacacttttg atgacgattc ttaacaataa cgatacaaat ttggccttaa 240

gaactgtgtc tggcgttctc aagaatc 267

<210> 194

<211> 257
<212> DNA
<213> Homo sapiens

<400> 194

ttgttactgc tgattcttgt aaatcttttt gcttctactt tcatcttaaa ctaatacgtg 60
ccagatataa ctgtcttgtt tcagtgagag acgccctatt tctatgtcat ttttaatgta 120
tctatttgta caattttaaa gttcttattt tagtatacgt ataaatatca gtattctgac 180
atgtaagaaa atgttacggc atcacactta tattttatga acattgtact gttgctttaa 240
tatgagcttc aatataa 257

<210> 195
<211> 240
<212> DNA
<213> Homo sapiens

<220>
<221> misc_feature
<222> (28)..(28)
<223> n is a, c, g, or t

<220>
<221> misc_feature
<222> (163)..(163)
<223> n is a, c, g, or t

<400> 195

aagtatacag ctgtaagtaa ccctgtcncc atggatgatc cttttctcta ggaatgtatt 60
tggattagag atgacaacta cattttcgca tttttatgtt gaagtctttt ttaaaaaggc 120
tgtttacttt tcagtagtta agaatacttg tttttctttt tcnttttttt tttttttaac 180
cttttatttt ttcgttaagc ctctattgtt tgtagaacac tcttagaaac ttggaaataa 240

<210> 196
<211> 221
<212> DNA
<213> Homo sapiens

<400> 196

ggcagccctg acgtgatgag ctcaaccagc agagacattc catcccaaga gaggtctgcg 60
tgacgcgtcc gggaggccac cctcagcaag accaccgtac aattggtgga aggggtgaca 120
gctgcattct cctgtgccta ccacgtaacc aaaaatgaag gagaactact gtttacaagc 180
cgccctggtg tgcctgggca tgctgtgcca cagccatgcc t 221

<210> 197
<211> 287

<212> DNA
<213> Homo sapiens

<400> 197

```
gattggatta aagacctggc acttcagtaa ctcagcacgc ttccacttca ctcaacttaa        60
gagagttcat tgacagtgtt aggatgtgaa ggctgggaaa cacttatttt gcttcaagag       120
ttccacttgg ctctcccaaa taggtacctc aaaaactgtt agcaagcggc atttggatgt       180
cttgacaggg gctttgcagg gatttttagg gtttttccca cattgtccac attaatggtt       240
ggcatgattg tgcttgcagg ccaagaaatg atcatacccc ttgccaa                     287
```

<210> 198
<211> 266
<212> DNA
<213> Homo sapiens

<400> 198

```
gcaagccagc cgccggtgct gttgacccaa gggccgtggt ccacggtact tgaagaagcc        60
agagcccaca tcctgtgcac tgctgaagga ccctacgctc ggtggcctgg cacctcactt       120
tgagaagagt gagcacactg gctttgcatc ctggaagacc tgcagggggc ggggcaggaa       180
atgtacctga aaaggatttt agaaaaccct gggaaaaccc accacaccac cacaaaatgg       240
cctttagtgt atgaaatgca catgga                                            266
```

<210> 199
<211> 133
<212> DNA
<213> Homo sapiens

<400> 199

```
gatgggcctt cagggaagtt cctggcagga acagtccttc catttgtttg ggcggatggt        60
ccggaagagc aaagccatgc taatgagaca gaggaatagc aagtcccctt caagccgaca       120
ggaaggggcc tca                                                          133
```

<210> 200
<211> 243
<212> DNA
<213> Homo sapiens
<400> 200

```
acatcattgt tgaaaccaga ccctgtagtc cagtggtgct gccctgttgt gcaaactgct      60
ccttttctc gtgtttttgt aaagagcttc catctgggct ggacccagtt cttgcacata      120
caagacaccg ctgcagtcag ctaggacctt tccgccatgt attctattct gtagtaaagc     180
atttccatca acaatgccta attgtatctg ttatttttgg tttaacacac actgattcat     240
act                                                                   243
```

<210> 201
<211> 284
<212> DNA
<213> Homo sapiens

<400> 201

```
ttcgcattta aggtataggt tgttttaggc atgggcagta ggcgctttca gcacaggctc      60
cctgctggtc actctttgct gtagtttcct gctccatcca tggggtcgga ggtcttacct     120
cctgacgtta gagtcatttg cagcaggtgg gagtgaaagt gggccgggtg ccaggtcttc     180
cttacctgaa cctctcctgt cttcctggca cattaggaat gtccaagctt atttctattg     240
gtgaatgctc ccacatggga tggataatgg cctgtggaac attc                      284
```

<210> 202
<211> 255
<212> DNA
<213> Homo sapiens

<400> 202

```
gatgccagca tgtgccaatg actgtcacct tcatctcttc aaaagaaaag ccatagccga      60
ggactgtccc gcgacccccg tggactgcgt ttaggtcatg tgattctgtt ttcatttctc     120
atcccatcca atttgtcctt ttctcctgtc attttcttcc tctgtggtcc cttcaaagtt     180
gttataattt gtactgaact tcaaaatgtg tcccgttctc cccagaccac tctagccaca     240
gtatattgca ataaa                                                      255
```

<210> 203
<211> 238
<212> DNA
<213> Homo sapiens

<400> 203

```
acccatcctg ttcgtgaata ggtctcaggg gttgggggag ggactgccag atttggacac      60
tatatttttt tttaaattca acttgaagat gtgtatttcc cctgaccttc aaaaaatgtt     120
ccaaggtaag cctcgtaaag gtcatcccac catcaccaaa gcctccgttt ttaacaacct     180
ccaacacgat ccatttagag gccaaatgtc attctgcagg tgccttcccg atggatta       238
```

<210> 204
<211> 239
<212> DNA
<213> Homo sapiens

<400> 204

tctgttttaa ggtctccttg aaggcgcact ggggaccctg gccatgcctc gttctccctg 60

ctttctttat cctgttattg cctccacagt ctgttgccaa ggactctaag atcaatgcac 120

gtcactttcc tttccactgg gcaggatagc caagcacact ccctcctgcg ctctcccgcc 180

ccggtgcgtc cactcccgag ggctgttatg aggactgggt tgtgcctact tgatttgaa 239

<210> 205
<211> 192
<212> DNA
<213> Homo sapiens

<400> 205

ggccaacgtg gcaaaacccc atctttacta aaaatacaaa aattagctgg gcatagtggt 60

gtgcacctgt aattccagct actcgggagg ctgaggcagg agaatctctt gaacccagga 120

ggcagaggtt gcagtgagct gagatcgcgc cactgcactc catcctgggg gacagggtga 180

gactccgtct cc 192

<210> 206
<211> 178
<212> DNA
<213> Homo sapiens

<400> 206

aaggacctaa tataaccaaa acaattttga aaaagaagaa catcggaggg cttatgcttt 60

gtgattttga gacttgcttt aaagctatag tgatgaagac agtgtagtgt tgacagaaag 120

acatatagac caatggaata gaatcaagtc cagaaataga ctcacatgta aataattt 178

<210> 207
<211> 185
<212> DNA
<213> Homo sapiens

<400> 207

gggatgcaaa ggacctaata taaccaaaac aattttgaaa aagaagaaca tcggagggct 60

tatgctttgt gattttgaga cttgctttaa agctatagtg atgaagacag tgtagtgttg 120

acagaaagac atatagacca atggaataga atcaagtcca gaaatagact cacatgtaaa 180

taatt 185

<210> 208
<211> 272
<212> DNA
<213> Homo sapiens

<400> 208

```
tccctccagc ggcttctgtt ttttaggatg ttaaggcgag tcccaggatg gcattcaact      60
ggacacggat gccagtgttg tgagtgctcc atgctgaaag ccgcctttc tggggcggtt      120
actcacgcag aggcgggaac ctgactctgt gcctgtctgt tggcttccgt aatccagccc     180
tccctgtgct gagtttaagc ccaaatgaaa ttactgggct ctaagtatgt gcctttctcc     240
ataaaatgtg atgcctggtc aatatcttgg cc                                   272
```

<210> 209
<211> 213
<212> DNA
<213> Homo sapiens

<400> 209

```
cacatcacaa actgcccttt cttggtcaga agaagcagag tggagccttc tcatccccac      60
gcgcgcagct gtggggcccc gtggtcacct ggccacatgg gagtttgcat actgagtggt     120
tcatcttttc caatgtgttg tgtcctttaa tttacattta tatttcattg ccctttctaa     180
tgatcagaac agcacatctt gttatagaaa att                                  213
```

<210> 210
<211> 251
<212> DNA
<213> Homo sapiens

<400> 210

```
atataattgg acaaacgctg gcaaaaagaa aaaaatggta agcaaaaaac ccaagataaa      60
gtttcgagga catcaggcct tttgaaatac aatgtcaaat gacacattgt acggtttcaa     120
aaaatccgct agacatgtca taagttttaa ctgtaatgcc caggaaagga tatcttaaaa     180
tattctaaac ttgtgtaaca aaggaataat taactgtaat agtttttcaa taaatcgagt     240
tgggtgtttc c                                                          251
```

<210> 211
<211> 232
<212> DNA
<213> Homo sapiens

<400> 211

atggaagaga tcttcggctc tctcaactct ctgaagctgg agattgagca gatgaaacgg 60

cccctgggca cgcagcagaa ccccgcccgc acctgcaagg acctgcagct ctgccacccc 120

gacttcccag atggtgaata ctgggtcgat cctaaacaat gatgctccag ggattccttc 180

aacgtttact gcaacttcac agccgggggg tcgacatgcg tcttccctga ca 232

<210> 212
<211> 129
<212> DNA
<213> Homo sapiens
<400> 212

aatgcatatg gaggtaggct gaaaagaatg taatttttat tttctgaaat acagatttga 60

gctatcagac caacaaacct tccccctgaa aagtgagcag caacgtaaaa acgtatgtga 120

agcctctct 129

<210> 213
<211> 94
<212> DNA
<213> Homo sapiens

<400> 213

gcctcccatt caagtgaagt tataatttac actgagggtt tcaaaattcg actagaagtg 60

gagatatatt atttatttat gcactgtact gtat 94

<210> 214
<211> 113
<212> DNA
<213> Homo sapiens

<400> 214

caaagtgcta ataattaact caaccaggtc tactttttaa tggctttcat aacactaact 60

cataaggtta ccgatcaatg catttcatac ggatatagac ctagggctct gga 113

<210> 215
<211> 204
<212> DNA
<213> Homo sapiens

<400> 215

agaaggaatg actctagcta caataatacc cagtatgttt aagcaggttc ccttggttgt 60

tgcattaaaa gtaatccccc tttaggtatt ttagagccca gaacaaccct gtgttgattt 120

agtaggtttt tatttttcct tttttttac aatgcccata atactttcct gtatttatat 180

cataacgtgt atagtgtaaa atgt 204

<210> 216
<211> 272
<212> DNA
<213> Homo sapiens

<400> 216

tgaataataa aatttaagtg gccaggcatg gtggcttacg cctgtaatcc cagctctttg 60

ggaggtcaag gcgggcggat caactgaggt cagaagttcg agaccagcct ggccaacatg 120

gcgaaacccc atctttacta aaagtacaaa aattagccgg gtgtggtggc gggtgcctgt 180

aatttcagct aatcaggagg ccgaggcagg agaatagctt gaacctggga ggcagaggtt 240

gcagtgagca gagatcatgc caccccactc ca 272

<210> 217
<211> 115
<212> DNA
<213> Homo sapiens

<220>
<221> misc_feature
<222> (81)..(81)
<223> n is a, c, g, or t

<400> 217

ttacctgcat gaaccgtaag tggtccttta gaaagaatgg actaccgtgc tataacaact 60

actagacacc ttcattttac nggctgtggt atcggcagtt tagggtatgg agcaa 115

<210> 218
<211> 157
<212> DNA
<213> Homo sapiens

<400> 218

ttacctgcat gaacccatct gcataccaaa ggacttattc tatgaaatac tttaaaaatt 60

cctcatcagc aaaggacaat tcaataaata catccaaaca tgatcatcgt tggagccgga 120

ggtggcagga gtcgaggcgc tgatccctaa aatggcg 157

<210> 219
<211> 237
<212> DNA
<213> Homo sapiens

<400> 219

acaaaagctc ccctgatcca actagcacac tgtcaaatac agtgtcatat gagaggtcca 60

cagacggtag tttccaagac cgtttcaggg aattcgagga ttccacctta aaacctaaca 120

gaaaaaaacc cactgaaaat attatcatag acctggacaa agaggacaag gatttaatat 180

tgacaattac agagagtacc atccttgaaa ttctacctga gctgacatcg gataaaa 237

<210> 220
<211> 230
<212> DNA
<213> Homo sapiens

<400> 220

ttgctctttg actgtcagca agactgaaga tggcttttcc tggacagcta gaaaacacaa 60

aatcttgtag gtcattgcac ctatctcagc cataggtgca gtttgcttct acatgatgct 120

aaaggctgcg aatgggatcc tgatggaact aaggactcca atgtcgaact cttctttgct 180

gcattccttt ttcttcactt acaagaaagg cctgaatgga ggacttttct 230

<210> 221
<211> 162
<212> DNA
<213> Homo sapiens
<400> 221

gattgctaaa gctctggtta atgcacaagt ggatttccag gcaatgtggt actctgacca 60

gaaccacggc ttatccggcc tgtccacgaa ccacttatac acccacatga cccacttcct 120

aaagcagtgt ttctctttgt cagactaaaa acgatgcaga tg 162

<210> 222
<211> 32
<212> DNA
<213> Homo sapiens

<400> 222
tgaggactca gaagttcaag ctaaatattg tt 32

<210> 223
<211> 72
<212> DNA
<213> Homo sapiens

<400> 223

gtgcccagcc tatatctact tgagcctgtg tgcccactga agagatgaag aataaaagcc 60

taagctctca tc 72

<210> 224
<211> 196
<212> DNA

<213> Homo sapiens

<400> 224

```
tctggcccgc aatactgtag gaacaagcat gatcttgtta ctgtgatatt ttaaatatcc      60
acagtactca ctttttccaa atgatcctag taattgccta gaaatatctt tttcttacct     120
gttatttatc aatttttccc agtattttta tacggaaaaa attgtattga aaacacttag     180
tatgcagttg ataaga                                                     196
```

<210> 225
<211> 51
<212> DNA
<213> Homo sapiens

<400> 225
ttgcaatggg agacttaaaa gtggtataaa atgtactttg ggccaggcgc a 51

<210> 226
<211> 112
<212> DNA
<213> Homo sapiens

<400> 226

```
ttacaggtaa ttaattgttc tcttcacttc tcatggggca gcacagaaag gaataagtta      60
ggtaactgaa gtgaccagcc ctcgaataaa aagtggcttc atggccgggt gt             112
```

<210> 227
<211> 269
<212> DNA
<213> Homo sapiens

<400> 227

```
atcactaact acaaaatccg ccatcatccc gagcacttca atgggaaacc tcgagaaaat      60
cgggtgcccc actctcggaa ttccatcacc ctcaccaacc tcactccagg cacagagtat     120
gtggtcagca tcgttgcttt taatggcaga gaggaaagtc ccttattgat tggccaacaa     180
tcaacaggta acttttcttg tctgcaaaga aactcagaag actttcctac ccagttggta     240
gattctgtaa agtagcttgc tgttgtctg                                       269
```

<210> 228
<211> 291
<212> DNA
<213> Homo sapiens

<400> 228

tcaaaccacc atcactaact acaaaatccg ccatcatccc gagcacttca atgggaaacc 60

tcgagaaaat cgggtgcccc actctcggaa ttccatcacc ctcaccaacc tcactccagg 120

cacagagtat gtggtcagca tcgttgcttt taatggcaga gaggaaagtc ccttattgat 180

tggccaacaa tcaacaggta acttttcttg tctgcaaaga aactcagaag actttcctac 240

ccagttggta gattctgtaa agtagcttgc tgttgtctgt catcagctct c 291

<210> 229
<211> 54
<212> DNA
<213> Homo sapiens

<400> 229
tgttgaaaat gtagaaaatt ggccggacgg ggtggctcac gtcagtaatt tcag 54

<210> 230
<211> 169
<212> DNA
<213> Homo sapiens

<400> 230

gaccacatcg agcggtgagg cacaggacga gcaggggcgg gaaatgggga agcaggtcaa 60

gaaatatttc cgcaaatcca tctttccttt gacatgccat ttgaggataa tttgcagtgt 120

ttcagctaat aacctaagat aatttacact attattggtt gttaaaact 169

<210> 231
<211> 129
<212> DNA
<213> Homo sapiens

<400> 231

aggacagctg ctgaaggcac cctctgatga gctcggttac tcagaagagt gaggatgtgt 60

tgaaggtatc tgctgtatgg agtggcagga tgatgtctgt gattgagaaa tataatcccg 120

gccaggcga 129

<210> 232
<211> 251
<212> DNA
<213> Homo sapiens

<400> 232

gaggctaagc cgggagcact gattgaggcc aggagttcat gatcagcctg ggcaatgaag 60

tgagaccccg tctctacaaa aaaatatgaa aaaattagcg aggtgtggtg acacatgcct 120

gtagtcccag ctactcaaga ggctgaggta gaggatcact tgagcctacg agttcaaggc 180

tgcagtgagc tatgataact ccactgcact gccgcctgga tgacacagag agaccgtttc 240

taaattaatt a 251

<210> 233
<211> 69
<212> DNA
<213> Homo sapiens

<400> 233

tcatttcatt tgactattct gatgacatga ttgtggcaga ataaattggg tcttaaaatg 60

ccctagaaa 69

<210> 234
<211> 238
<212> DNA
<213> Homo sapiens

<400> 234

tatatcaatg atggcaaaaa ggttaaaggg ggcctaacag tactgtgtgt agtgttttat 60

ttttaacagt agtacactat aacttaaaat agacttagat tagactgttt gcatgattat 120

gattctgttt cctttatgca tgaaatattg attttacctt tccagctact tcgttagctt 180

taattttaaa atacattaac tgagtcttcc ttcttgttcg aaaccagctg ttcctcct 238

<210> 235
<211> 209
<212> DNA
<213> Homo sapiens

<400> 235

gggcctaaca gtactgtgtg tagtgtttta tttttaacag tagtacacta taacttaaaa 60

tagacttaga ttagactgtt tgcatgatta tgattctgtt tcctttatgc atgaaatatt 120

gattttacct ttccagctac ttcgttagct ttaattttaa aatacattaa ctgagtcttc 180

cttcttgttc gaaaccagct gttcctcct 209

<210> 236
<211> 122
<212> DNA
<213> Homo sapiens

<400> 236

ggaacccacc tagcccaaca agaacaatcc attctacttc ttggaactac gtttattttc 60

cttttccccc atttcctata agataacctc taaccaatga caatctcgac agctattcct 120

gc 122

<210> 237
<211> 186
<212> DNA
<213> Homo sapiens

<400> 237

tattcctgca ccaactgacc tgaagttcac tcaggtcaca cccacaagcc tgagcgccca 60

gtggacacca cccaatgttc agctcactgg atatcgagtg cgggtgaccc ccaaggagaa 120

gaccggacca atgaaagaag tcaaccttgc tcctgacagc tcatccgtgg ttgtatcagg 180

acttat 186

<210> 238
<211> 233
<212> DNA
<213> Homo sapiens

<400> 238

tctgtagtct tgtccaaaat tgggtaacca cttctgatgg ggtagctcat atccaagaat 60

gagtcacaaa accagactcg ttgaacctgg tatatgatga gtcacaaagc aacattctgc 120

ctttgttttt tcaggacaag aaacttgaat tgtatcccac tgagttaaaa gataaaatat 180

atggcattgg catttctgta cttcagagag gaatatatct gtttgtggta gga 233

<210> 239
<211> 241
<212> DNA
<213> Homo sapiens

<400> 239

gcatttttc tgtagtcttg tccaaaattg ggtaaccact tctgatgggg tagctcatat 60

ccaagaatga gtcacaaaac cagactcgtt gaacctggta tatgatgagt cacaaagcaa 120

cattctgcct ttgtttttc aggacaagaa acttgaattg tatcccactg agttaaaaga 180

taaaatatat ggcattggca tttctgtact tcagagagga atatatctgt ttgtggtagg 240

a 241

<210> 240
<211> 129
<212> DNA
<213> Homo sapiens

<400> 240

gtctcctaat cttatcaatt ctgatggttt cttttttcc cagcttttga gccaacaact 60

ctgattaact attcctatag catttactat atttgtttag tgaacaaaca atatgtggtc 120

aattaaatt 129

<210> 241
<211> 248
<212> DNA
<213> Homo sapiens

<400> 241

agttgctaca aaaactgatt ggtttttgtc acttcatctc ttcactaatg gagatagctt 60

tacactttct gctttaatag atttaagtgg accccaatat ttattaaaat tgctagttta 120

ccgttcagaa gtataataga aataatcttt agttgctctt ttctaaccat tgtaattctt 180

cccttcttcc ctccaccttt ccttcattga ataaacctct gttcaaagag attgcctgca 240

agggaaat 248

<210> 242
<211> 145
<212> DNA
<213> Homo sapiens

<400> 242

gataccgact gaccgtgggc cttacccgaa gaggacagcc catgcagtac aatgtgggtc 60

cctctgtctt caagtaccca ctgaggaatc tgcagcctgc atctgagtac accgtattcc 120

tcgtggccat aaagggcaac caaga 145

<210> 243
<211> 239
<212> DNA
<213> Homo sapiens

<400> 243

aagaacaact cctcaccagt tcatcctgag gctgggagga ccgggatgct ggattctgtt 60

ttccgaagtc actgcagcgg atgatggaac tgaatcgata cggtgttttc tgtccctcct 120

actttccttc acaccagaca gcccctcatg tctccaggac aggacaggac tacagacaac 180

tctttcttta aataaattaa gtctttacaa taaaaacaca actgcaaagt accttcata 239

<210> 244
<211> 244
<212> DNA
<213> Homo sapiens

<400> 244

```
cactcccggg actattgcca agaaggggca agggatgagt caagaaggtg agacccttcc      60
cggtgggcac gtgggccagg ctgtgtgaga tgttggatgt ttggtactgt ccatgtctgg     120
gtgtgtgcct attacctcag catttctcac aaagtgtacc atgtagcatg ttttgtgtat     180
ataaaaggga gggttttttt aaaaatatat tcccagatta tccttgtaat gacacgaatc     240
tgca                                                                  244
```

<210> 245
<211> 214
<212> DNA
<213> Homo sapiens

<400> 245

```
ggatcttgat gcctgaaaat cccaagattg gtacttggca aactgaaaga aatctagaaa      60
accctagaga tcaggcatct gtggccagct aactggtcat acaaatggat tgttgtggtg     120
aacttgtata gtattaatcc tgagatgctg tccccctcca cccccacccc cacaaaaaaa     180
ataaataaag tagtattaag ttagcctcat acaa                                 214
```

<210> 246
<211> 164
<212> DNA
<213> Homo sapiens

<400> 246

```
ccatggcaat gcgggtgact cctttacatg gcacaacggc aagcagttca ccaccctgga      60
cagagatcat gatgtctaca caggaaactg tgcccactac cagaagggag gctggtggta     120
taacgcctgt gcccactcca acctcaacgg ggtctggtac cgcg                      164
```

<210> 247
<211> 254
<212> DNA
<213> Homo sapiens

<400> 247

```
gaaatacaga ctggatgtac caccaactac tacctgtaat gacaggcctg tccaacacat      60
ctccttttc catgactgtg gtagccagca tcggaaagaa cgctgattta aagaggtcgc      120
ttgggaattt tattgacaca gtaccattta atggggagga caaaatgggg caggggaggg     180
agaagtttct gtcgttaaaa acagatttgg aaagactgga ctctaaattc tgttgattaa     240
agatgagctt tgtc                                                       254
```

<210> 248

<211> 183
<212> DNA
<213> Homo sapiens

<400> 248

```
attgagcaga tctataggaa gattgaacct gaatattgcc attatgcttg acatggtttc        60

caaaaaatgg tactccacat atttcagtga gggtaagtat tttcctgttg tcaagaatag       120

cattgtaaaa gcattttgta ataataaaga atagctttaa tgatatgctt gtaactaaaa       180

taa                                                                     183
```

<210> 249
<211> 277
<212> DNA
<213> Homo sapiens

<400> 249

```
tgtccttcca ctcaacagtc atcaaccact accgcatgcg gggccatagc cccttttgcca       60

acctcaaatc gtgctgtgtg cccaccaagc tgagacccat gtccatgttg tactatgatg       120

atggtcaaaa catcatcaaa aaggacattc agaacatgat cgtggaggag tgtgggtgct       180

catagagttg cccagcccag ggggaaaggg agcaagagtt gtccagagaa gacagtggca       240

aaatgaagaa atttttaagg tttctgagtt aaccaga                                277
```

<210> 250
<211> 237
<212> DNA
<213> Homo sapiens

<400> 250

```
atgagtgcat ttcaactatg tcaatggttt cttaatattt attgtgtaga agtactggta        60

atttttttat ttacaatatg tttaaagaga taacagtttg atatgttttc atgtgtttat       120

agcagaagtt atttattttt atggcattcc agcggatatt ttggtgtttg cgaggcatgc       180

agtcaatatt ttgtacagtt agtggacagt attcagcaac gcctgatagc ttctttg          237
```

<210> 251
<211> 193
<212> DNA
<213> Homo sapiens

<400> 251

aagggagaat ggtgtaccct ttatttcttc tgaaatcaca ctgatgacat cagttgttta 60

aacggggtat tgtcctttcc ccccttgagg ttcccttgtg agcttgaatc aaccaatctg 120

atctgcagta gtgtggacta gaacaaccca aatagcatct agaaagccat gagtttgaaa 180

gggcccatca cag 193

<210> 252
<211> 48
<212> DNA
<213> Homo sapiens

<400> 252
cttccttctc tcttactcgg agacagtcag aactctcctc cctgacag 48

<210> 253
<211> 125
<212> DNA
<213> Homo sapiens

<400> 253

ttccttctct cttactcgga gacagtcaga actctcctcc ctgacagcca caaacctaca 60

gcactgactg cattcagaga ggaacctgca aacaaaactt cacagaaaac tttttgttct 120

tgttc 125

<210> 254
<211> 34
<212> DNA
<213> Homo sapiens

<400> 254
gaaaaatata ctaagttggt atactataac actt 34

<210> 255
<211> 188
<212> DNA
<213> Homo sapiens

<400> 255

gtcactactg ggcaaataca cttactgtgt tctagaggca gccctttctt atgcagaaaa 60

tacaatacgc actgcatgag aagcttgaga gtggattcta atccaggtct gtcgaccttg 120

gatatcatgc atgtgggaag gtgggtgtgg tgagaaaagt tttaaggcaa gagtagatgg 180

ccatgttc 188

<210> 256
<211> 167
<212> DNA
<213> Homo sapiens

<400> 256

tacccaatta cccaggtcat aaggtatgtc tgtgtgacac ttatctctgt gtatatcagc 60

atacacacac acacacacac acacacacac acacacaggc atttccacac attacatata 120

tacacatact ggtaaaagaa caatcgtgtg caggtggtca cacttcc 167

<210> 257
<211> 36
<212> DNA
<213> Homo sapiens

<400> 257
tgggggggca gaggcgtctg accccaggaa cctgca 36

<210> 258
<211> 157
<212> DNA
<213> Homo sapiens

<400> 258

tatgaattcc attcaaatcg ttccttttttg ttaacaaggg gcatggggag gggtgggggt 60

gggggggcag aggcgtctga ccccaggaac ctgcagggcg gggctgggtc ggtgcccttt 120

aaggacaatt ttgaccttgt tcaacctttc cacaaag 157

<210> 259
<211> 172
<212> DNA
<213> Homo sapiens

<400> 259

agcccaccca ttgaagtctc cttgggccac caaaggtggt ggccatggta ccggggactt 60

gggagagtga gacccagtgg agggagcaag aggagaggga tgtcgggggg gtggggcacg 120

gggtagggga aatggggtga acggtgctgg cagttcggct agatttctgt ct 172

<210> 260
<211> 274
<212> DNA
<213> Homo sapiens

<400> 260

aaacagattc ctgccaggga gactcagggg gacccctcgt ctgttccctc caaggccgca 60

tgactttgac tggaattgtg agctggggcc gtggatgtgc cctgaaggac aagccaggcg 120

tctacacgag agtctcacac ttcttaccct ggatccgcag tcacaccaag gaagagaatg 180

gcctggccct ctgagggtcc ccagggagga aacgggcacc acccgctttc ttgctggttg 240

tcatttttgc agtagagtca tctccatcag ctgt 274

<210> 261

<211> 179
<212> DNA
<213> Homo sapiens

<400> 261

agaggttata ggtcactcct ggggcctctt gggtccccca cgtgacagtg cctgggaatg 60

tattattctg cagcatgacc tgtgaccagc actgtctcag tttcactttc acatagatgt 120

ccctttcttg gccagttatc ccttcctttt agcctagttc atccaatcct cactgggtg 179

<210> 262
<211> 245
<212> DNA
<213> Homo sapiens

<400> 262

gaaatacgaa tgtagagatc cctaatcatc aaattgttga ttgaaagact gatcataaac 60

caatgctggt attgcacctt ctggaactat gggcttgaga aaacccccag gatcacttct 120

ccttggcttc cttcttttct gtgcttgcat cagtgtggac tcctagaacg tgcgacctgc 180

ctcaagaaaa tgcagttttc aaaaacagac tcagcattca gcctccaatg aataagacat 240

cttcc 245

<210> 263
<211> 271
<212> DNA
<213> Homo sapiens

<400> 263

aggggcagcc gtgcttatat ttttatggtt acaatggcac aaaattatta tcaacctaac 60

taaaacattc cttttctctt ttttcctgaa ttatcatgga gtttttttaat tctctctttt 120

ggaatgtaga tttttttttaa atgctttacg atgtaaaata tttatttttt acttattttg 180

gaagatctgg ctgaaggatt attcatggaa caggaagaag cgtaaagact atccatgtca 240

tctttgttga gagtcttcgt gactgtaaga t 271

<210> 264
<211> 205
<212> DNA
<213> Homo sapiens

<400> 264

```
tgcttttcat cctgtataac tgcaactttg tgccggctca tattatgttc tagagagctc       60

atgtacacca tggtgaccat agttaataat actggattgt atgcttaaaa tttgctaaga      120

gagtagatct taagtcatca ccaaaaaaag taactgtaag atgatggaga tgtgttaatt      180

agcttgacgg tggtaatcac aatat                                            205
```

<210> 265
<211> 270
<212> DNA
<213> Homo sapiens

<220>
<221> misc_feature
<222> (114)..(117)
<223> n is a, c, g, or t

<220>
<221> misc_feature
<222> (119)..(119)
<223> n is a, c, g, or t

<220>
<221> misc_feature
<222> (121)..(125)
<223> n is a, c, g, or t

<220>
<221> misc_feature
<222> (128)..(130)
<223> n is a, c, g, or t

<220>
<221> misc_feature
<222> (133)..(139)
<223> n is a, c, g, or t

<220>
<221> misc_feature
<222> (141)..(144)
<223> n is a, c, g, or t

<220>
<221> misc_feature
<222> (186)..(186)
<223> n is a, c, g, or t

<400> 265

tgttccgcat tcacttaaca tgtgcgttaa aaggaacaaa gctagctgga ggtagcaaag 60

ttttgaactt gaatcaggga ttgctaagaa aacttacaac taatgaccac agannnnang 120

nnnnntgnnn tcnnnnnnnt nnnnctagat gtgattgtag ccgtggtgcc tgggcagatg 180

gtaaanaaac atgctgtcct cttatgacaa tatgtgcaga gaaggcccca aacgctgggg 240

tgagaggaac aacaaactga ctaactcacg 270

<210> 266
<211> 225
<212> DNA
<213> Homo sapiens

<400> 266

aatgtttgca cactgaattg aagaattgtt ggtttttct tttttttgtt ttgttttttt 60

tttttttttt ttttgctttt gacctcccat ttttactatt tgccaatacc tttttctagg 120

aatgtgcttt tttttgtaca catttttatc cattttacat tctaaagcag tgtaagttgt 180

atattactgt ttcttatgta caaggaacaa caataaatca tatgg 225

<210> 267
<211> 224
<212> DNA
<213> Homo sapiens

<400> 267

ttcggccagg gtgtcgaaca tgccacggcc aacaaacagg tgtgcaagcc ccgtaacccc 60

tgcacggatg ggacccacga ctgcaacaag aacgccaagt gcaactacct gggccactat 120

agcgacccca tgtaccgctg cgagtgcaag cctggacgct ggcaatggca tcatctgcgg 180

ggaggacaca gacctggatg ctggcccaat gagaacctgg tgtg 224

<210> 268
<211> 223
<212> DNA
<213> Homo sapiens
<400> 268

gaacaaagag ttggccaatg agctgaggcg gcctccccta tgctatcaca acggagttca 60

gtacagaaat aacgaggaat ggactgttga tagctgcact gagtgtcact gtcagaactc 120

agttaccatc tgcaaaaagg tgtcctgccc catcatgccc tgctccaatg ccacagttcc 180

tgatggagaa tgctgtcctc gctgttggcc cagcgactct gcg 223

<210> 269
<211> 144
<212> DNA
<213> Homo sapiens

<400> 269

ggcacaccca ataatcagtc atgtgtaata tgcacaagtt tgtttttgtt tttgtttttt 60

ttgttggttg gtttgttttt ttgctttaag ttgcatgatc tttctgcagg aaatagtcac 120

tcatcccact ccacataagg ggtt 144

<210> 270
<211> 116
<212> DNA
<213> Homo sapiens

<400> 270

ttcctttgag gttgatcgtt gtgttgtttt gctgcacttt ttactttttt gcgtgtggag 60

ctgtattccc gagaccaacg aagcgttggg atacttcatt aaatgtagcg actgtc 116

<210> 271
<211> 246
<212> DNA
<213> Homo sapiens

<400> 271

accccaaacc caagtgcctt cagaggataa atatcaatgg aactcagaga tgaacatcta 60

acccactaga ggaaaccagt ttggtgatat atgagacttt atgtggagtg aaaattgggc 120

atgccattac attgcttttt cttgtttgtt taaaaagaat gacgtttaca tataaaatgt 180

aattacttat tgtatttatg tgtatatgga gttgaaggga atactgtgca taagccatta 240

tgataa 246

<210> 272
<211> 51
<212> DNA
<213> Homo sapiens

<400> 272
ttcacgtggg ttcagttaaa ctagagctaa ggttgtccat ggcatgttga a 51

<210> 273
<211> 224
<212> DNA
<213> Homo sapiens

<400> 273

gcggtgttcc taatcacatg tttgtactat aatttcacgt gggttcagtt aaactagagc 60
taaggttgtc catggcatgt tgaatcaaaa ataaaaatgg tcagtgagta aaagtaacgt 120
actatgacaa acgtgactta cacctgtatg tgagcaaaca ccttgtgtgt gtgtgtgtgt 180
gtgtgtgtgt gttaccagca tacaccctag taggtgcagc ctcc 224

<210> 274
<211> 227
<212> DNA
<213> Homo sapiens

<400> 274

gttgtggggt caaccgtaca atggtgtggg agtgacgatg atgtgaatat ttagaatgta 60
ccatattttt tgtaaattat ttatgttttt ctaaacaaat ttatcgtata ggttgatgaa 120
acgtcatgtg ttttgccaaa gactgtaaat atttatttat gtgttcacat ggtcaaaatt 180
tcaccactga aaccctgcac ttagctagaa cctcattttt aaagatt 227

<210> 275
<211> 296
<212> DNA
<213> Homo sapiens

<400> 275

cagcggctgc tggaacgcgt tgaccctcac tgtttgtgtg ttttcaggaa ggtgcattcg 60
cgctggtttt caagagtgtc cactacccag gagaagccgt tgccacacgg tgaggcaaaa 120
cacactggca tttcccataa gaaagaacga aaataaaatc acttagctgg agggagacgg 180
gagaggtagg gcctgtggca cgctgaaagg tgctcgtggt gtctaaagaa caactgtggc 240
tcagccccgg ggaccgcgcc ccaccatctc catggctggt ggtgctacac ttttga 296

<210> 276
<211> 240
<212> DNA
<213> Homo sapiens

<400> 276

ttctctggaa gaactagcag atcggggcat gctgtgcagc cctctctcag gtggcaggtg 60
tctggatctg ctcaaaggct gccccctta gatgagacaa ctgtgtctag acctcagtcc 120
ctcccatgcc cctccctgcc tttgcctggg gtgagaccat ttgtgaaaga cagagccaag 180
acacagggag tttttcaatt gattctaaag tccatgctct catcaatcca ctgaataatg 240

<210> 277
<211> 238
<212> DNA

<213> Homo sapiens

<400> 277

```
acctatgcct cctataagtc cagttgaaat ctcagcctcc ttcaacattt tcttctcgtg    60
tgtggcccac atccctccac ttctccaact tctgtttaat ctgatcacgg ctctttttaa   120
gccctggcag cattttggtc cctgctcctt gcccatagta aaacagcttg aaatatccca   180
tgcaagagag tagtttcaag tgggcgactc tgctctctat ttaaaagcgt gcacaatc     238
```

<210> 278
<211> 18
<212> DNA
<213> Homo sapiens

<400> 278
ctccatgtgc cggatagc 18

<210> 279
<211> 22
<212> DNA
<213> Homo sapiens

<400> 279
cgatttcacc agaagcctct ac 22

<210> 280
<211> 20
<212> DNA
<213> Homo sapiens

<400> 280
tgttgcagtg agggcaagaa 20

<210> 281
<211> 20
<212> DNA
<213> Homo sapiens

<400> 281
gaccctggtt gcttcaagga 20

<210> 282
<211> 19
<212> DNA
<213> Homo sapiens

<400> 282
accactatgc cgcgctctt 19

<210> 283
<211> 19
<212> DNA
<213> Homo sapiens

<400> 283

ggtcgtaggg ctgctggaa 19

<210> 284
<211> 20
<212> DNA
<213> Homo sapiens

<400> 284
tggtctaacg gtttccccta 20

<210> 285
<211> 18
<212> DNA
<213> Homo sapiens

<400> 285
gacctcggag cgagagtg 18

<210> 286
<211> 19
<212> DNA
<213> Homo sapiens

<400> 286
agctacgcct tctcggtct 19

<210> 287
<211> 23
<212> DNA
<213> Homo sapiens

<400> 287
ccttctctgg aaacaatgac atc 23

<210> 288
<211> 20
<212> DNA
<213> Homo sapiens

<400> 288
aagtgggtcc atgaacctaa 20

<210> 289
<211> 21
<212> DNA
<213> Homo sapiens

<400> 289
aaattcactc tgcccaggac g 21

<210> 290
<211> 24
<212> DNA
<213> Homo sapiens

<400> 290
ccagaaagct cttgagttta ttcc 24

<210> 291
<211> 22
<212> DNA
<213> Homo sapiens

<400> 291
catctagttc ccgaaccatc tc 22

<210> 292
<211> 226
<212> DNA
<213> Homo sapiens

<400> 292

taataatgcc cttaatgtga gggtttgtaa tggtgcttat taagaccaaa gacttgttaa 60

atgtatacac caagtggtaa tgaaatttct gtgactggcc cacacgtgca tagaggtctg 120

ggaggaccag gaaacagcct cagtggccag aggatcacca gtgcatcctt catcacagca 180

tgtgcaatat gccaagatta ccctcggtca ttcctgtcaa caaggg 226

<210> 293
<211> 276
<212> DNA
<213> Homo sapiens

<400> 293

actgggcagc aaaagttgtt ccacagtgga aatttaggca tcctcaagtt tcttcccagc 60

ttctgctgtg ttttcttaga gtaaattgcc aatttctgtt tttacaggaa atcctttttt 120

aaaaatggaa tcagtgtggt ccccatctac tctgcaaaaa ttgcattttt ctctattttc 180

aaatgagatt tgttcaagtt tcaaaaccac gtgaaataat aaatgtatag tagttttctt 240

ttccttgggc attgcttgat atgtgaaatg ggttta 276

<210> 294
<211> 297
<212> DNA
<213> Homo sapiens

<400> 294

gtgttgtgta catgcaactg accatttcac tggcttccca tgccgctgtt acagatgttt 60

cagccggaaa aattttaatt agacttgact aagggttgtc attttcctgt attgaaggac 120

agtgggaata catttccttt aagagaaatt tgtctgcagt tgtttaagtt aaagcctcca 180

tggaaacaac aggatggact gtcagtgacc ccttttatct tcttggagga gcctgttaaa 240

taaattcaga cagtatatat agggagattt tgtattaaat tcttaccatt ctcgctg 297

<210> 295

<211> 227
<212> DNA
<213> Homo sapiens

<400> 295

```
ggtagttgtc aatgagggta tcagtcaggt ggttggaaaa agagaaatag ttttctccgg      60
gaatcggaag catgactcct ccccgcaacc ctccaccaca cacatactca cacttttaat     120
tggcagtcaa agagtcaaat gacctgggat caactaaggc agttctgggt ctgtgattaa     180
gcttggtttc ttgttgtgta acttgctggc tcagttatcc aaactat                   227
```

<210> 296
<211> 287
<212> DNA
<213> Homo sapiens

<400> 296

```
atttcacttt caatagttga agaacttggc tttgtaaatc tctcagaagc ttgaaaatat      60
cttgtctcta ccccctcagc ccatttcatt tgccaataat tattttgtaa gtagggttga     120
aatgaactca gctggccttg tgaaatgttt aaacttgcac aaacaactac atttttgttc     180
aacaaatagc agtttactca gccaaaatca ctttggatat tgccattaca aatactgtta     240
aacttcagaa atcatgtctg taaattagat gagccaaaat aaaggac                   287
```

<210> 297
<211> 297
<212> DNA
<213> Homo sapiens

<400> 297

```
gactgtatcc aattgctgta acttttggtt tcttaatgtc ataattttta aagtctgttt      60
tattttaagt gcaatattga gtatttagct gttaggctca atccgtcgat atgaaataat     120
tttttaaatc cctaagggca ggaaagcatt tcgtggtagt gaaaataaga ggaaataaga     180
tggcatgaag gtggtgggcg gagaaactag gtagcacaca ggaaagtgct ctcaaaaatc     240
tttgaagagc tcagctagaa aaaaatggag tagatttggc tcatactatt ccggaag        297
```

<210> 298
<211> 266
<212> DNA
<213> Homo sapiens

<400> 298

gtggctgtcc acctggttac ttccgcatag gccaaaggca ctgtgtttct ggaatgggca 60
tgggccgagg aaacccacag ccacctgtca gtggtgaaat ggatgacaaa tcactctccc 120
ccacaggctt gttacgagtg taagatcaat ggctaccccc cacggggcag gaaacggaga 180
agcacaaacg aaactgatgc ctcccatatc gaggatcagt ctgagacaga aaccaatgtg 240
agtcttgcaa gttgggatgt tgagaa 266

<210> 299
<211> 266
<212> DNA
<213> Homo sapiens

<400> 299

cagtgcccac tgtagccatg gaggtgcggc catcaccaag cacccccctg gaggtccctc 60
tcaatgcctc cagcaccaat gccacagagg atcacccaga aaccaatgcc gtagatgaac 120
cctacaaaat ccatggcctg gaagatgaag tccagccacc caacctaatc cctcgacgac 180
cgagccccta tgaaaagacc agaaaccaaa gaatccaaga gctcattgat gagatgcgga 240
agaaagagat gctcggaaag ttctaa 266

<210> 300
<211> 276
<212> DNA
<213> Homo sapiens

<220>
<221> misc_feature
<222> (42)..(43)
<223> n is a, c, g, or t

<400> 300

atctcacgac tgttaacttc caactttgac gaggccatgc gnncgacacg cgcgactggg 60
gaaggtgcaa tactgtcctg ggcatatgca ctttggttta tcaggatat tttattagtg 120
ttttgtatgt atttaatgac gtattctata ggttccattg tctttgcttt tctggttttt 180
tttatgagac gttagcgtgt gccaaagtga ctgtgaaaat gacctttcat tcttactaat 240
gatggaaaga cctcctgtca tgcatcccac aaataa 276

<210> 301
<211> 38
<212> DNA
<213> Homo sapiens

<400> 301
ggcccccctt ggacgggact atgtgtgcac ctggcaag 38

<210> 302

<211> 147
<212> DNA
<213> Homo sapiens

<400> 302

tatatacatg gtgctcaata gcaacatctt agcagatgaa gcagtttatg attccactcc 60

ctcctgtatg acaggtagcc actatactga atcaaggtgc tgaactcaaa tcacaaaatt 120

ctggcttacc gatacaacaa ccaatac 147

<210> 303
<211> 163
<212> DNA
<213> Homo sapiens

<400> 303

tgagcagact gcaccccaag ctcccgactc caggtcccct gatcttgggg cctgtttccc 60

atgggattca agagggacag ccccagcttt gtgtgtgttt aagcttagga atcgccttta 120

tggaaagggc tatgtgggag agtcagctat cttgtctggt ttt 163

<210> 304
<211> 156
<212> DNA
<213> Homo sapiens

<400> 304

gcagcaacag caaatcacga ccactgatag atgtttattt ttgttggaga catgggatga 60

ttattttctg ttctatttgt gcttagtcca attccttgca catagtaggt acccaattca 120

attactattg aatgaattaa gaattggttg ccataa 156

<210> 305
<211> 105
<212> DNA
<213> Homo sapiens

<400> 305

ggacctgaag ggtgacatcc caggaggggc ctctgaaatt tcccacaccc cagcgcctgt 60

gctgaggact ccctccatgt ggccccaggt gccaccaata aaaat 105

<210> 306
<211> 45
<212> DNA
<213> Homo sapiens

<400> 306
tgcttataca cttacacttt atgcacaaaa tgtagggtta taata 45

<210> 307
<211> 296
<212> DNA
<213> Homo sapiens

<220>
<221> misc_feature
<222> (75)..(75)
<223> n is a, c, g, or t

<220>
<221> misc_feature
<222> (262)..(262)
<223> n is a, c, g, or t

<400> 307

```
atgggtgtag atcaaggcag gagcaggaac caaaaagaaa ggcataaaca taagaaaaaa      60
aatggaaggg gtggnaaaca gagtacaata acatgagtaa tttgatgggg gctattatga     120
actgagaaat gaactttgaa aagtatcttg gggccaaatc atgtagactc ttgagtgatg     180
tgttaaggaa tgctatgagt gctgagaggg catcagaagt ccttgagagc ctccagagaa     240
aggctcttaa aaatgcagcg cnatctccag tgacagaaga tactgctaga aatctg         296
```

<210> 308
<211> 167
<212> DNA
<213> Homo sapiens

<400> 308

```
tctgagcggg tcatggggca acacggttag cggggagagc acggggtagc cggagaaggg      60
cctctggagc aggtctggag gggccatggg gcagtcctgg gtgtggggac acagtcgggt     120
tgacccaggg ctgtctccct ccagagcctc cctccggaca atgagtc                  167
```

<210> 309
<211> 60
<212> DNA
<213> Homo sapiens

<400> 309
tttctggggt ctttgagctc caaaaaataa acacttcctt tgagggagag cccccccca 60

<210> 310
<211> 222
<212> DNA
<213> Homo sapiens

<400> 310

gacacagtcg ggttgaccca gggctgtctc cctccagagc ctccctccgg acaatgagtc 60

ccccctcttg tctcccaccc tgagattggg catggggtgc ggtgtggggg gcatgtgctg 120

cctgttgtta tgggtttttt ttgcgggggg ggttgctttt ttctggggtc tttgagctcc 180

aaaaaataaa cacttccttt gagggagagc acaccttccc aa 222

<210> 311
<211> 264
<212> DNA
<213> Homo sapiens

<400> 311

gccaacagca tcttttccgg gttcctgctc tttccagata tggaggcctg acctgtgggc 60

tgcttcacat ccaccccggc tccccctgcc agcaacgctc actctacccc caacaccacc 120

ccttgcccag ccaatgcaca cagtagggct tggtgaatgc tgctgagtga atgagtaaat 180

aaacttttca aggccaaggg acagtggttt aattcaactc tgtgtcccag cacctggcac 240

accagaagtg ccatgctcag aaat 264

<210> 312
<211> 72
<212> DNA
<213> Homo sapiens

<400> 312

gaatggcagt accagaaggc attggttaag tgtcccagga accacacaag cagtgactcc 60

taaagaagtt ca 72

<210> 313
<211> 239
<212> DNA
<213> Homo sapiens

<400> 313

agatgcgctc gagacccacg ggccttccac ctccctcagc ttcctgcatg gacccacctt 60

actggccagt ctgcatcctt gcctagacca ttctcccctc cagggagccc accctgaccc 120

acccccactg caccccctcc ccatgggttc tctccttcct ctgaacttct ttaggagtca 180

ctgcttgtgt ggttcctggg acacttaacc aatgccttct ggtactgcca ttcttttt 239

<210> 314
<211> 284
<212> DNA
<213> Homo sapiens

<400> 314

aactgaaatg tcaacctgtg gactgtggca ttcctgaatc cattgagaat ggtaaagttg 60

aagacccaga gagcactttg tttggttctg tcatccgcta cacttgtgag gagccatatt 120

actacatgga aaatggagga ggtggggagt atcactgtgc tggtaacggg agctgggtga 180

atgaggtgct gggcccggag ctgccgaaat gtgttccagt ctgtggagtc cccagagaac 240

cctttgaaga aaaacagagg ataattggag gatccgatgc agat 284

<210> 315
<211> 201
<212> DNA
<213> Homo sapiens

<400> 315

tggtgaatgc gtgctgccca ggaccagtga agacagacat ggatgggaaa gacagcatca 60

ggactgtgga ggaggggct gagaccctg tctacttggc cctcttgcct ccagatgcca 120

ctgagccaca aggccagttg gtccatgaca aagttgtgca aaactggtaa acgtctgctt 180

cggagcttgc tgcttaataa a 201

<210> 316
<211> 190
<212> DNA
<213> Homo sapiens

<400> 316

cacagggaaa tcagggttac aaatcttctt gatccacttc tctcaggatc ccctctcttc 60

ctacccttcc tcaccacttc cctcagtccc aactcctttt ccctatttcc ttctcctcct 120

gtctttaaag cctgcctctt ccaggaagac ccccctattg ctgctggggc tccccatttg 180

cttactttgc 190

<210> 317
<211> 115
<212> DNA
<213> Homo sapiens

<400> 317

cagaaagcca agtggactca acggagaggc cagcaagttt caggaaatgg tgcatttggt 60

gaacaaggag tcgtcagaaa ctccagacca gtttatgaca gctgatgaga caagg 115

<210> 318
<211> 241
<212> DNA
<213> Homo sapiens

<400> 318

ttcccatctc gtccatgagc ctaggtcttg gagccttgtg ttggaggctg ctgtgatgtc 60

aggaacgggg atctttctag cttttggcca cttcctggga cctcacgccc ctgttgacag 120

atggagattg ggcagcaggg ccttgctgca ttgttatctg ctgttccgac ttggtttgtc 180

ttgtccaagg gtgacgaaag agccaggcac cagggtctca tgggatgagg tccaactttt 240

t 241

<210> 319
<211> 285
<212> DNA
<213> Homo sapiens

<400> 319

caagacctag gctcatggac gagatgggaa ggcacaggga gaagggataa ccctacaccc 60

agaccccagg ctggacatgc tgactgtcct ctcccctcca gcctttggcc ttggcttttc 120

tagcctattt acctgcaggc tgagccactc tcttcccttt ccccagcatc actccccaag 180

gaagagccaa tgttttccac ccataatcct ttctgccgac ccctagttcc ctctgctcag 240

ccaagcttgt tatcagcttt cagggccatg gttcacatta gaata 285

<210> 320
<211> 37
<212> DNA
<213> Homo sapiens

<400> 320
gatgcttaac aaaggttacc ataagccaca aattcat 37

<210> 321
<211> 189
<212> DNA
<213> Homo sapiens

<400> 321

ccctgcagag aatcacgtcc tggaactgca tgttcttgcg actcttggga cttcatttta 60

acttctcgct gccccagcca tgttttcaac catggcatcc ctccccccaat tagttccctg 120

tcatcctcgt caaccttctc tgtaagtgcc tggtaagctt gcccttgctt aagaactcaa 180

aacatagct 189

<210> 322
<211> 82
<212> DNA
<213> Homo sapiens

<400> 322

tttaaaatac tcagaggaca ggatcactgt ggaatcgaat cagaagtggt ggctggaatt 60

ccacgcaccg atcagtactg gg 82

<210> 323
<211> 34
<212> DNA
<213> Homo sapiens

<400> 323
gaaatcattg tgggattgct agctttccct ctta 34

<210> 324
<211> 82
<212> DNA
<213> Homo sapiens

<400> 324

cagatgttgg tatctgcagg gatcctggaa ccaaacccct gcagatacta agggctgacg 60

atctaggtaa gactggattt aa 82

<210> 325
<211> 107
<212> DNA
<213> Homo sapiens

<400> 325

tactcccgag gctctgtaca ttgctgccac atactcctgc cagcttgggg gagtgttcct 60

tcaccctcac agtatttatt atcctgcacc acctcactgt tcccccat 107

<210> 326
<211> 83
<212> DNA
<213> Homo sapiens

<400> 326

ttccaccatc aaatgctgta gaatgcttgg cactccctaa ccaaatgctg tctccataat 60

gccactggtg ttaagatata ttt 83

<210> 327
<211> 88
<212> DNA
<213> Homo sapiens

<400> 327

gccctcatgg ttggcatcac atatgcctgc atgccattaa caccagctgg ccctacccct 60

ataatgatcc tgtgtcctaa attaatat 88

<210> 328
<211> 33
<212> DNA
<213> Homo sapiens

<400> 328
ttctgtgcct cagccgttct tgacatcaag aat 33

<210> 329
<211> 241
<212> DNA
<213> Homo sapiens

<400> 329

aactggaccc tgtcgttctc catcttctca gtcagttgtt tcaagtgttc ctgataactc 60

ctctccttct gttccatcat ctgctcattc tttctttgca tttcctgcaa catttttgct 120

gaagcctgtg cagactcagc tttcacaagt tccacttcaa tctccttttc tttttctgtg 180

agagtctggt ctgtctggag aattgcatca gtcatagact ccttggattt caagtatgtc 240

t 241

<210> 330
<211> 251
<212> DNA
<213> Homo sapiens

<400> 330

gaacaggagc aactactaaa agagggattt caaaaagaaa gcagaataat gaaaaatgag 60

atacaggatc tccagacgaa aatgagacga cgaaaggcat gtaccataag ctaaagacca 120

gagccttcct gtcaccccta accaaggcat aattgaaaca attttagaat ttggaacaag 180

cgtcactaca tttgataata attagatctt gcatcataac accaaaagtt tataaaggca 240

tgtggtacaa t 251

<210> 331
<211> 220
<212> DNA
<213> Homo sapiens

<400> 331

gaacaggaac gccttctcaa ggagggattc gagaatgaga gcaagagact tcaaaaagac 60

atatgggata tccagatgag aagcaaatca ttggagccaa tatgtaacat actttaaaag 120

tccaaggagc aaaatttgcc tgtccagctc cctctcccca agaaacaaca tgaatgagca 180

acttcagagt gtcaaacaac tgccattaaa cttaactcaa 220

<210> 332
<211> 82
<212> DNA
<213> Homo sapiens

<400> 332

gttatttaag atggctatcc agataatcct gaacactgtg tatttatttt atttagacta 60

ccagcaaaga ttaaagcatg aa 82

<210> 333
<211> 201
<212> DNA
<213> Homo sapiens

<400> 333

ccctgctgcc tctgatcgta ggaattgagg agtgtcccgc cttgtggctg agaactggac 60

agtggcaggg gctggagatg ggtgtgtgtg tgtgtgtgtg tgtgtgtgtg tgtgtgcgcg 120

cgcgccagtg caagaccgag attgagggaa agcatgtctg ctgggtgtga ccatgtttcc 180

tctcaataaa gttcccctgt g 201

<210> 334
<211> 262
<212> DNA
<213> Homo sapiens

<400> 334

gtcctctcta tcctggatga gctcatgaac atttctcttg tgttcctgac tccttcccaa 60

tgaacacctt tctgccaccc caagctttgc tctcctcctc tgtgagctct gggcttccca 120

gtttgtttac ccgggaaagt acgtctagat tgtgtggttt gcctcattgt gctatttgcc 180

cactttcctt ccctgaagaa atatctgtga accttctttc tgttcagtcc taaaattcga 240

aataaagtga gactatggtt ca 262

<210> 335
<211> 135
<212> DNA
<213> Homo sapiens

<400> 335

tacccgggaa agtacgtcta gattgtgtgg tttgcctcat tgtgctattt gcccactttc 60

cttccctgaa gaaatatctg tgaaccttct ttctgttcag tcctaaaatt cgaaataaag 120

tgagactatg gttca 135

<210> 336
<211> 278
<212> DNA
<213> Homo sapiens

<400> 336

tgagaggcaa gagttgttcc tgcccttccc tttgtgactt gaagaaccct gactttgttt 60

ctgcaaaggc acctgcatgt gtctgtgttc gtgtaggcat aatgtgagga ggtggggaga 120

gcaccccacc cccatgtcca ccatgaccct cttcccacgc tgacctgtgc tccctctcca 180

atcatctttc ctgttccaga gaggtggggc tgaggtgtct ccatctctgt ctcaacttca 240

tggtgcactg agctgtaact tcttccttcc ctattaaa 278

<210> 337
<211> 63
<212> DNA
<213> Homo sapiens

<400> 337

attagaacct tagtataaat ttactttctc aaattcttgc catgagaggt tgatgagtta 60

att 63

<210> 338
<211> 75
<212> DNA
<213> Homo sapiens

<400> 338

agaacctgag tataaattta ctttctcaaa ttcttgccat gagaggttga tgagttaatt 60

aaaggagaag attcc 75

<210> 339
<211> 132
<212> DNA
<213> Homo sapiens

<400> 339

acagcccacc cttgtgtcca ctgtgacccc tgttcccatg ctgacctgtg tttcctcccc 60

agtcatcttt cttgttccag agaggtgggg ctggatgtct ccatctctgt ctcaacttta 120

cgtgcactga gc 132

<210> 340
<211> 132
<212> DNA
<213> Homo sapiens
<400> 340

acagcccacc cttgtgtcca ctgtgacccc tgttcccatg ctgacctgtg tttcctcccc 60

agtcatcttt cttgttccag agaggtgggg ctggatgtct ccatctctgt ctcaacttta 120

tgtgcactga gc 132

<210> 341
<211> 273
<212> DNA
<213> Homo sapiens

<400> 341

ttcaaactca atgatgctac catgcctctc caacattttc aaccccctga cattatcttg 60

gatcctatgg tttctccatc caattctttg aatttcccag tctcccctat gtaaaactta 120

gcaacttggg ggacctcatt cctgggacta tgctgtaacc aaattattgt ccaaggctat 180

atttttggga tgaatataat ttgaggaagg gagttaaaga ccctcctggg gctctcagtg 240

tgccatagag gacagcaact ggtgattgtt tca 273

<210> 342
<211> 89
<212> DNA
<213> Homo sapiens

<400> 342

tgactgaatt gctgaccctt caagctctgt ccttatccat tacctcaaag cagtcattcc 60

ttagtaaagt ttccaacaaa tagaaatta 89

<210> 343
<211> 279
<212> DNA
<213> Homo sapiens

<400> 343

gcatttgctg tgtttcgtta gcatctggct ccaggacaga ccttcaactt ccaaattgga 60

tactgctgcc aagaagttgc tctgaagtca gtttctatca ttttgctctt tgattcaaag 120

cactgtttct ctcactgggc ctccaaccat gttccctttt ttttagcacc acaaataatc 180

aaaacccaac atgactgttt gttttccttt aaaaatatgc accaaatcat ctctcatcac 240

ttttctttga gggttttagt agacagtagg agttaataa 279

<210> 344
<211> 53
<212> DNA
<213> Homo sapiens

<400> 344
aagcaagata tcaatgtagc agaattgcac ttgtgcctca cgaacataca taa 53

<210> 345
<211> 159
<212> DNA
<213> Homo sapiens

<400> 345

gagcttcctt cttcgttctt ggcaccatct tatgaaaagg gtccagatta agatttttga 60

ctgagtcatt ctaaagtaag ttgcaagacc catgatacta gaccactaaa tacttcatca 120

cacacctcct aagaataaga accaacatta tcacaccaa 159

<210> 346
<211> 159
<212> DNA
<213> Homo sapiens

<400> 346

gagcttcctt cttcgttctt ggcaccatct tatgaaaagg gtccagatta agatttttga 60

ctgagtcatt ctaaagtaag ttgcaagacc catgatacta gaccactaaa tacttcatca 120

cacacctcct aagaataaga accaacatta tcacaccaa 159

<210> 347
<211> 232
<212> DNA
<213> Homo sapiens

<400> 347

tgaaccaaaa tagagtcagc tgacccagca tcagccacac tttgggttgg aaaatgtttg 60

cctgttggaa ttaatttaag cttaagtata tatcaacatt attttattgt gcaattaaaa 120

caatacaaat tcatggtttt ttaaagttaa aaattttaac cactgtaaca acagtttttg 180

tgttattttc tgtattaaac atcttgttgc acgcatttga ggtcatcagg gt 232

<210> 348
<211> 186
<212> DNA
<213> Homo sapiens

<400> 348

gagacttgta tgaaagatgg ctgtgcctct gcctgtctcc cccaccgggc tgggagctct 60

gcagagcagg aaacatgact cgtatatgtc tcaggtccct gcagggccaa gcacctagcc 120

tcgctcttgg caggtactca gcgaatgaat gctgtatatg ttgggtgcaa agttccctac 180

ttcctg 186

<210> 349
<211> 243
<212> DNA
<213> Homo sapiens

<400> 349

aaaacttggc actttttcgt gtggatcttg ccacatttct gatcagaggt gtacactaac 60

atttcccccg agctcttggc ctttgcattt atttatacag tgccttgctc ggcgcccacc 120

accccctcaa gccccagcag ccctcaacag gcccagggag ggaagtgtga gcgccttggt 180

atgacttaaa attggaaatg tcatctaacc attaagtcat gtgtgaacac ataaggacgt 240

gtg 243

<210> 350
<211> 174
<212> DNA
<213> Homo sapiens

<400> 350

ttgcatggag acttgaggag ggagggcttg aggttggtga ggttaggtgc gtgtttcctg 60

tgcaagtcag gacatcagtc tgattaaagg tggtgccaat ttatttacat ttaaacttgt 120

cagggtataa aatgacatcc cattaattat attgttaatc aatcacgtgt atag 174

<210> 351
<211> 296
<212> DNA
<213> Homo sapiens

<400> 351

tgaggatgtc accaattaac cagaaatcca gttattttcc gccctcaaaa tgacagccat 60

ggccggccgg gtgcttttgg gggctcgtcg gggggacagc tccactttga ctggcacagt 120

ctttgcatgg agacttgagg agggagggct tgaggttggt gaggttaggt gcgtgtttcc 180

tgtgcaagtc aggacatcag tttgattaaa ggtggtgcca atttatttac atttaaactt 240

gtcagggtat aaaatgacat cccattaatt atattgttaa tcaatcacgt gtatag 296

<210> 352
<211> 227

<212> DNA
<213> Homo sapiens

<400> 352

```
gaagatgaag ccccatgctc agtcccctcc catcccccac gcagctccac cccagtccca        60
agccaccagc tgtttgctcc tggtgggagg tggcctcctc agcccctcct ttctgacctt       120
taacctcact ctcaccttgc accgtgcacc aacccttcac ccctcctgga aagcaggcct       180
gatggcttcc cactggcctc caccacctga ccagagtgtt ctcttca                     227
```

<210> 353
<211> 38
<212> DNA
<213> Homo sapiens

<400> 353
agtctgccta tgatctttga atgagctttt taaggaag 38

<210> 354
<211> 174
<212> DNA
<213> Homo sapiens

<400> 354

```
tgggcgtccg ggcccccaat attcacgcac tcgcaccacg cactcatatt ccctcacccc        60
accatcacgg ccccaaagaa ggtcttccct ctcgcgaagt ccaccatatc ggggtgactg       120
atgttggacg tacaccctct cgcccctccg gagctgcacc aggccgccga accc             174
```

<210> 355
<211> 292
<212> DNA
<213> Homo sapiens

<400> 355

```
acagtgttgt atgaggtttg aggattttga tccaagctgg tcccactcag tccatagcag        60
agaatgaaag ggcccagaga gggtggtgac ctctgcctga agtcacacag tgagtcgagg       120
acagggaggt gaccccaggt ttctatgtgt agggcgggag gatgttttgg gacacagttc       180
aattctcatt tgtcacacac tttggctatt agagatcaac cccttcgctc ctgtgtcttg       240
caatggcagc cttggcaaac gctaaatgaa aatcgtgaca acacttgtgt ta               292
```

<210> 356
<211> 151
<212> DNA
<213> Homo sapiens

<400> 356

tagggattaa cttcctgtat gctgcaactc atgaacttgg ccattctttg ggtatgggac 60

attcctctga tcctaatgca gtgatgtatc caacctatgg aaatggagat ccccaaaatt 120

ttaaactttc ccaggatgat attaaaggca t 151

<210> 357
<211> 82
<212> DNA
<213> Homo sapiens

<400> 357

gtgctcagta gtcagactgg atagtccgtt tttgcttatc cgttagccgt ggggatttag 60

caggaagctg tgagagcagt tt 82

<210> 358
<211> 116
<212> DNA
<213> Homo sapiens

<400> 358

tgctgtgaaa gaggctggct acacaatcga atggtttgag gtgatctcgc aaagttattc 60

ttccaccatg gccaacaacg aaggactttt ctccctggtg gcgaggaagc tgagca 116

<210> 359
<211> 228
<212> DNA
<213> Homo sapiens

<400> 359

atgtaggaga acgtgcccta ttcacacttt gggaagacgc taatttgtga catttttttt 60

tcaagcctgc catcaaggac atttttttaag acccaactgg catgagttgg ggtaatttcc 120

tattattttc attttggaca acttttttaa cttatattct ttatagagga ttccccaaaa 180

tgtgctcctc atttttggcc tctcatgttc caaacctcat tgaataaa 228

<210> 360
<211> 37
<212> DNA
<213> Homo sapiens

<400> 360
cagggactgg ctatcccaag acctggcaga tgtggct 37

<210> 361
<211> 119
<212> DNA
<213> Homo sapiens

<400> 361

gccagagaga ccaagtgtta tgtaagaagt agtgtcggct gtgtagaacc actgactaca 60

caggccgaag ttactgagaa cttggacaga aaaaatagcc agcaagtgtt caaactact 119

<210> 362
<211> 242
<212> DNA
<213> Homo sapiens

<400> 362

catcttctag cagatctttt tcagttaaga ttacttgttc ttccatgtat tcatatttag 60

ccagctcctt gatcagccgc agtatgtcac tgcagtcggc ggcagtggct gggcggatca 120

cgaatttagc cattttcgtc ttttgctttt cttccctttg cggaccaggc ccctgtactt 180

gaacagtagg aggaggtggt tcctcattcg tctcccggga gcgtcctctt ctcagtcagg 240

ct 242

<210> 363
<211> 180
<212> DNA
<213> Homo sapiens

<400> 363
gcttccttaa aggtcagaac atcaggccaa agtacaacgt ttaatttcag aacttgcctt 60

ccaatttacg cattttcaat ttgctctccc catttgttga gtcagaagaa gcagcattgc 120

ccagaaacag gtattacgta acatgcacat actttaaaaa gtactcatcc cttgttttct 180

<210> 364
<211> 272
<212> DNA
<213> Homo sapiens

<400> 364

agagctcttg agtgggctag tgactccccc tgcagcctgg tggagatggt gtgaggagcg 60

aagagccctc tgctctagga tttgggttga aaaacagaga gagaagtggg gagttgccac 120

aggagctaac acgctgggag gcagttgggg gcgggtgaac tttgtgtagc cgaggccgca 180

ccctccctca ttccaggctc attcattttc atgctccatt gccagactct tgctgggagc 240

ccgtccagaa tgtcctccca ataaaactcc at 272

<210> 365
<211> 151
<212> DNA
<213> Homo sapiens

<400> 365

```
acacagaaga gtgacatgtt tacaaacctc aagccagcct tgctcctggc tggggcctgt        60
tgaagatgct tgtattttac ttttccattg taattgctat cgccatcaca gctgaacttg       120
ttgagatccc cgtgttactg cctatcagca t                                      151
```

<210> 366
<211> 96
<212> DNA
<213> Homo sapiens

<400> 366

```
tcgacacagt gctttccgtg gcactgcata caatctgagg cctcctctct cagtttttat        60
atagatggcg agaacctaag tttcagttga ttttac                                  96
```

<210> 367
<211> 107
<212> DNA
<213> Homo sapiens

<400> 367

```
taaatataat atcgacacag tgctttccgt ggcactgcat acaatctgag gcctcctctc        60
tcagtttttta tatagatggc gagaacctaa gtttcagttg attttac                    107
```

<210> 368
<211> 252
<212> DNA
<213> Homo sapiens
<220>
<221> misc_feature
<222> (180)..(182)
<223> n is a, c, g, or t

<400> 368

```
gtggagcagt tggactgctc tctctgctct caggatgata ctgtgagaac aatttaaata        60
tgctaagcac atgtcaggaa acagttttgt ggtctttgga cactcgctgt agccattccg       120
ttccatttca ggtgatttta ttcatttcat ttgtagaata aaataaatcc atttcacacn       180
nncacacaca cacacacaca cacacacaca caccctctat acaccactaa agcctcccat       240
taaacccata ga                                                           252
```

<210> 369
<211> 121
<212> DNA
<213> Homo sapiens

<400> 369

ggcagcgacc cggaaacagc gtatcactga gaccgagtcg ccttatcagg agctccaggg 60

tcagaggtcg gatgtctaca gcgacctcaa cacacagagg ccgtattaca aatgagcccg 120

a 121

<210> 370
<211> 123
<212> DNA
<213> Homo sapiens

<400> 370

atggtataat ggttgactgg gtttctctgt atagtactgg catggtacgg agatgtttca 60

cgaagtttgt tcatcagact cctgtgcaac tttcccaatg tggcctaaaa atgcaacttc 120

ttt 123

<210> 371
<211> 273
<212> DNA
<213> Homo sapiens

<400> 371

tagaagatga cctcgttccg gagctgggta tttcaaattt gctttcatcc agccactgcc 60

caaagccatt ttcctgccta ctggatgctt acagtgactg tggatacggg ggttcccttt 120

ccccattcag tgacatgtcc tctttgcttg gtgtaaacca ttcttgggag gacacttttg 180

ccaatgaact ctttccccag ctgattagtg tctaaggaat gatccaatac tgttgccctt 240

ttccttgact attacactgc ctggaggata gca 273

<210> 372
<211> 231
<212> DNA
<213> Homo sapiens

<400> 372

agctttgtgc tcagatccca ggtcccaagg agtgacaggg gcttcctccc accttctgtc 60

cttgtccagt catgtaaata atgtgctttt tctctccccg agtctttttt ttttaaacct 120

accgtggttc ctcagctaac tgcattccct acccaggcag agactgtcct atgcctcgag 180

cttccaaacg agactcagac cgcgacacag ccaccgtatt tatggaatga c 231

<210> 373
<211> 182
<212> DNA
<213> Homo sapiens

<400> 373

```
tgagtaggtg agtttattgg gacttacaca caggtcaatc ctgggcggcg acaagacagc       60
tctagagatc tgagcttcct cccaatgcta aactgctttc atgctaattt tctgactgtt      120
tacttaccgg gtaagagcga tgggactgtt ttcattggtt ggttctcaca tactctctgg      180
ga                                                                     182
```

<210> 374
<211> 243
<212> DNA
<213> Homo sapiens

<400> 374

```
tcccctgctt gaacactgaa gggcaggtgg tgggccatgg ccatggtccc cagctgagga       60
gcaggtgtcc ctgagaaccc aaacttccca gagagtatgt gagaaccaac caatgaaaac      120
agtcccatcg ctcttacccg gtaagtaaac agtcagaaaa ttagcatgaa agcagtttag      180
cattgggagg aagctcagat ctctagagct gtcttgtcgc cgcccaggat tgacctgtgt      240
gta                                                                    243
```

<210> 375
<211> 281
<212> DNA
<213> Homo sapiens

<400> 375

```
ggagacagat ggagggtacc ctatttacaa ctgagtcagc caagccactg atgggaatat       60
acagatttag gtgctaaacc atttattttc cacggatgag tcacaatttg aagaatcaaa      120
cttccatcct gaaaatttat atgtttcaaa accacttgcc atcctgttag attgccagtt      180
cctgggacca ggcctcagac tgtgaagtat atatcctcca gcattcagtc caggggggagc     240
cacggaaacc atgtttttgc ttaagccatt aaagtcagag a                          281
```

<210> 376
<211> 196
<212> DNA
<213> Homo sapiens

<400> 376

```
gcctttaaga taccttgatg aagacctgga ctattgaatg gagcagaaat tcacctctct       60
cactgactat tacagttgca tttttatgga gttcttcttc tcctaggatt cctaagactg      120
ctgctgaatt tataaaaatt aagtttgtga atgtgactac ttagtggtgt atatgagact      180
ttcaagggaa ttaaat                                                      196
```

<210> 377
<211> 262
<212> DNA
<213> Homo sapiens

<400> 377

gttcacccgg tgaactattt atgagttctt ttggtgtgaa gaaagggctc atgttgcatt 60
tccagccatt gctacaaaga acctttattt gttcagtaac ggtagaaaat ccttcccgat 120
taaaaacttc agacttgctg aatatcctgc aatgtcaaga tgaccgatgt tgagttgggt 180
ggatttgcta acgagtcaga tttgaacatg aggctattgg aacccaatag gcgtcattga 240
tggcggcaag ccatagcttt ca 262

<210> 378
<211> 155
<212> DNA
<213> Homo sapiens

<400> 378

gttgttgatc atggatcata ctccccttgt ttctttgggt gagaagggat cgcagtttgg 60
aaactccggc ggctgcgtgc ggggtttcag tcccagctgt aggcttgtaa atacccgccc 120
cgccaaaccg catagagaac gtggcagcaa gctga 155

<210> 379
<211> 217
<212> DNA
<213> Homo sapiens

<400> 379

atgctgtggt tggatcaagg actcattcct gccttggaga aaatacttca accagagcag 60
ggagcctggg ggtgtcgggg caggaggctg gggatggggg tgggatatga gggtggcatg 120
cagctgaggg cagggccagg gctggtgtcc ctaaggttgt acagactctt gtgaatattt 180
gtattttcca gatggaataa aaaggcccgt gtaatta 217

<210> 380
<211> 182
<212> DNA
<213> Homo sapiens
<400> 380

atgctgagtg acactcttgt gtatatttcc aaatttttgt acagtcgctg cacatatttg 60
aaatcatata ttaagacttt ccaaagatga ggtccctggt ttttcatggc aacttgatca 120
gtaaggattt cacctctgtt tgtaactaaa accatttact atatgttaga catgacattc 180
tt 182

<210> 381
<211> 259
<212> DNA
<213> Homo sapiens

<400> 381

```
ctacaacagg caggtactgc tgccaggggg ctttgaacta gtgcctgcta cccaggacac      60
ccgggccatg cccctggctg ggcagcctgg cacaagtgaa gaagaaggca gtgggaaaac     120
tgggtttatt tcaaggcagc agcctgagcc caggagcaga ggacccagtt gttataaggc     180
gctgggagag gatgggcagc tcccactgcc ccagagcgga gctcgaagca cccaggttgc     240
ccacggaaaa tccaataaa                                                  259
```

<210> 382
<211> 158
<212> DNA
<213> Homo sapiens

<400> 382

```
tttggtgcag tttccagggt gcagtacagc agggcctgaa tactggccct ggactccctt      60
ttccagaaca ccaggtgtgg ccacctgggg ctcaggtaca cagtggggtc tctcggaagc     120
caccgtgtgg ttctttcaca ggcacgttta ttttgctg                             158
```

<210> 383
<211> 267
<212> DNA
<213> Homo sapiens

<400> 383

```
gcatttcaaa gtgacttcta tgaagctttt tttttaatgt gaaattttca gaatgttgtt      60
tttttcatgt agatactcca ggaagagtta agcactgctt tcagttttaa tatccacctt     120
gaggggtcgc tgcttgaggg ctcttatccc aggggacttt ttaattcgga tgttacttaa     180
tgtggcttct ctaatgtagt ttctttgatt accgactaca caattatgta ccatcacagt     240
attagtggaa aagtaccatg tgattta                                         267
```

<210> 384
<211> 94
<212> DNA
<213> Homo sapiens

<400> 384

```
ggggagccag gcttccctca cgcagcctgt ggtggatgtg ggaaggagat caacttctcc      60
tcactctggg acagacgatg tatggaaact aaaa                                  94
```

<210> 385
<211> 214
<212> DNA
<213> Homo sapiens

<400> 385

gacttattcc cgctgactga gtttttgagg ggctaccagg aaagcgcctc caaccctagc 60

aaaagtgcaa gatggggagt gagaggctgg gaatggaggg gcagagccag gaagatcccc 120

cagaaaagaa agctacagaa gaaactgggg ctcctccagg gtggcagcaa caataaatag 180

acacgcacgg cagccacagc ttgggtgtgt gttc 214

<210> 386
<211> 270
<212> DNA
<213> Homo sapiens

<400> 386

ccagggctga gagacacgtg aaggaagatg atgggaggaa aagcccagga gaagtcccac 60

cagggaccag cccagcctgc atacttgcca cttggccacc aggactcctt gttctgctct 120

ggcaagagac tactctgcct gaacactgct tctcctggac cctggaagca gggactggtt 180

gagggagtgg ggaggtggta agaacacctg acaacttctg aatattggac attttaaaca 240

cttacaaata aatccaagac tgtcatattt 270

<210> 387
<211> 118
<212> DNA
<213> Homo sapiens

<400> 387

tttgaagatt agaaatttag ccgtaggtaa agaatacaaa ggaaaaataa ttttaaaatc 60

atcaaccaga tcaacaaaat atatgttaat gccgagactt tgaattagag tgcgaatt 118

<210> 388
<211> 278
<212> DNA
<213> Homo sapiens

<400> 388

ggaagccatt atcctaaatg aactcactca gaaacagaaa accaaatacc acatgttctc 60

acttataagt agaagctaaa cattgagtac acatggatac aaagaaggga accgcagaca 120

ctggggccta cctgaggtcg gagcatggaa ggagggtgag gatcaaaaaa ctacctatct 180

ggtactatgc tttttatctg gatgatgaaa taatctgtac aacaaaccct ggtgacatgc 240

aatttaccta tatagcaagc ctacacatgt gcccctga 278

<210> 389
<211> 173
<212> DNA
<213> Homo sapiens

<400> 389

tattttccct ctttcgaaca aagacattgg tttgcccaag gactacaaat aaaccaacgg 60

gaaaaaagaa aggttccagt tttgtctgaa aattctgatt aagcctctgg gccctacagc 120

ctggagaacc tggagaatcc tacacccaca gaacccggct ttgtccccaa aga 173

<210> 390
<211> 261
<212> DNA
<213> Homo sapiens

<400> 390

tcactcccac ctgttactgc tgggagtcaa gtcagctagg aaggaagcag gacatttttt 60

caaacagcaa gtggggccca tggaactgaa tctttactcc ttggtgcacc gcttttgtcg 120

tgcgttgcct tgctccgttt ttcccaaaaa gcactggctt catcaaggcc accgacgatt 180

tcctgagtgc actgggaaat ttgggtatag gtcaggcttg gcagccttga tcccaggaga 240

gtactaatgg taacaagtca a 261

<210> 391
<211> 74
<212> DNA
<213> Homo sapiens

<400> 391

gtgaaaggga agtagaaccg aaacaagatt agtcctgagt taacaatggc tgcaagctgg 60

atacatggaa ttca 74

<210> 392
<211> 248
<212> DNA
<213> Homo sapiens

<400> 392

aaaaactcaa cctatctggt gttttatttt aatggataaa aatgtaattt ttttaaggta 60

gcaacttatt tccaaattaa tatagatgaa aaatagatac caattagact aaattgaaag 120

ctttttgttc tatatttgca tagcctttga aatatttctt agtgcctagg aggtctgggg 180

attcctcttt cgtggtggtc actaacctta cttgatgcag ataaaatcac ttgtcaatgc 240

aaaatgtg 248

<210> 393
<211> 286
<212> DNA
<213> Homo sapiens

<400> 393

gctgtgttgg ccctcacttg ggattctcag cagttacatg aaagttgtgc tgataatctc 60

ttctcttgta ccaattttag tcaggcagaa aatggtaaac atgagggtgc tcttgtgact 120

taatttttgt tcaagggact aaattgctta tgtttattcc ctgtcagcgg agtggagaat 180

gtcattcatc aataaaccaa agccaatagc tggagaattg agatctggtt gaaagtggtt 240

tatggtttac atgctgtact atcctgagga attgcgagat attgct 286

<210> 394
<211> 246
<212> DNA
<213> Homo sapiens

<400> 394

tgtacagatt caagcaatgg atgcaaggaa catgctgtat gtaatagaag aaagaagtcc 60

acgttttcgg cagaagtagt gagtcagtgt ggaagagagg tgagggtgtg ctttactttt 120

tgataaagag aaagatgttt actcataaac ccttcaaaag gtattaacaa atgtttacca 180

aacctattgc tttattttaa aaacataatt tgtgttttct atttgtaaga tctgacattt 240

cgaggc 246

<210> 395
<211> 110
<212> DNA
<213> Homo sapiens

<400> 395

tttaacttga gggtgtagag gtcctccacg cttgtttgcc tgaaagtaat ataatgatgc 60

tgtctgaaca ggttttactg cttgctttcc aagtaaaggt taattatgat 110

<210> 396
<211> 251

<212> DNA
<213> Homo sapiens

<400> 396

agagagtgga catttgtcgg gaaactccta acatatgccc ccattctgga gagaacacag 60

agtacgacac aatccctcac actaatagaa caatcctaaa ggaagatcca gcaaatacgg 120

tttactccac tgtggaaata ccgaaaaaga tggaaaatcc ccactcactg ctcacgatgc 180

cagacacacc aaggctattt gcctatgaga atgttatcta gacagcagtg cactgcccct 240

aagtctctgc t 251

<210> 397
<211> 266
<212> DNA
<213> Homo sapiens
<400> 397

gcttggggaa caatgatggt gcacaaaggc ttagatttgc cttgtctcaa aataaggaat 60

tttgtagtgg ttttcaaaaa taattcaaca aagaaacaat acaaaaagtg ggtagaatta 120

cctatcacat ttcccaatct tgactattca gaatgctgtt tatttagtga tgaggattag 180

cacttgattg aagattcttt taaaatacta tcagttaaac atttaatatg attatgatta 240

atgtattcat tatgctacag aactga 266

<210> 398
<211> 165
<212> DNA
<213> Homo sapiens

<400> 398

tttcctgccc caaggcagat ccacatcacc gaagctccct agaggggcaa aagatggagt 60

gagccacagg aagtttgggg cgtggtgagt tggaatgata cgtccatttc tctatgaaat 120

atttgctact agactgttca tttctctctg acatgtttgt tgaat 165

<210> 399
<211> 275
<212> DNA
<213> Homo sapiens

<400> 399

acttacatac tagcttccaa ggacaggtgg aggtagggcc agcctggcgg gagtggagaa 60

gcccagtctg tcctatgtaa gggacaaagc caggtttaat ggtactgggt agggggcact 120

gccaagacaa taagctaggc tactgggtcc agctactact ttggtgggat tcaggtgagt 180

ctccatgcac ttcacatgtt acccagtgtt cttgttactt ccaaggagaa ccaagaatgg 240

ctctgtcaca ctcgaagcca ggtttgatca ataaa 275

<210> 400
<211> 115
<212> DNA
<213> Homo sapiens

<400> 400

aggagctgag gtgctacccg gagccccatt cacccccacc tgcccacttg ggaatctgag 60

gcagaggagg gtgaggcctg tgtgccaacc ttgttcacat accaccttcg tcccc 115

<210> 401
<211> 114
<212> DNA
<213> Homo sapiens

<400> 401

ctgcacagct cagcacaaca ttccaagctc aaaatagaag ccttctcagt gagctccagc 60

acgcccagag gactgttaat aacgatgatc catgtgtttt actctaaagt gcta 114

<210> 402
<211> 105
<212> DNA
<213> Homo sapiens

<400> 402

atttccatca catatgtgcc aagacttgtg ttctgtatcc aggagtgtgt tagatactaa 60

catagtgttt catttacatg tgtgtgaaac ctgggtgaag agcca 105

<210> 403
<211> 173
<212> DNA
<213> Homo sapiens

<400> 403

gtaccatctt acatgcttaa ataactccac atttatttgt gtttattact ctgtgttata 60

aatatacatt tgttggtctc tctcttggat tattttgttt ctttgtcctg taactaccac 120

tgaaagggtg caatacagct ttcttgaaat gtgtattgaa cggatgaatg tat 173

<210> 404
<211> 262
<212> DNA
<213> Homo sapiens

<400> 404

tggtgaccag ttctcggttt catagttttt actatcagtt tgcctttggg attctttgaa 60

agctcttgag gctttttccg cagcttctag gagatgtgtt aggtcattaa cagtaatgct 120

cctacagttt ttgttcccat ccaaccacca tttgatttca cttttgtaga cttgacctag 180

tgtatctgaa atataggaat ttttaggtgc tttcattttg gcctgacgtg cccagtccag 240

agctgtgtta aagtccttct ct 262

<210> 405
<211> 226
<212> DNA
<213> Homo sapiens

<400> 405

gtcattcaca actgatttca agagtcacct tcaccaggaa gtcttccttg accaccatca 60

ttcctgcctg attagagggc ttcctcatgg taatatgtgt tctcaagttt tcagtgtcaa 120

ggaatgccat cccagaagct cattttcaga tgcacaacag ccagaacagt ctcaagcagc 180

attctagagc ttggaattta agaactacgc attgcctata aagtga 226

<210> 406
<211> 286
<212> DNA
<213> Homo sapiens
<400> 406

actttgccgg cgagcacacc gcctacccgc acggctgggt ggagacggcg gtcaagttgg 60

cgctgcgcgc cgccatcaag atcaacagcc ggaaggggcc tgcatcggac acggccagcc 120

ccgaggggca cgcatttgac atggaggggc aggggcatgt gcatggggtg gccagcagcc 180

cctcgcatga cctggcaaag gaagaaggca gccaccctcc agtccaaggc cagttatctt 240

tccaaaacac gacccacacg aggacctcgc attaaagtat tttcgg 286

<210> 407
<211> 266
<212> DNA
<213> Homo sapiens

<220>
<221> misc_feature
<222> (214)..(214)
<223> n is a, c, g, or t

<400> 407

ccctgcgcct atcaggtcgt gagtccaggg gtctacaagt cccgggcccc ccagttcacg 60

attctggcgc ggacttcgct cccccaagac aacactcgga agccagggcc cgcggcctac 120

aacgtggatc agcaccggaa gccccgcggc tggagtttcg ggatccggca ctcggactac 180

ctggccccgc tggtgaccga cgcggacaac tganccgcca ggcgggagcg gccccacacg 240

tgtttgctta aagtctgcga gtccgc 266

<210> 408
<211> 123
<212> DNA
<213> Homo sapiens

<220>
<221> misc_feature
<222> (52)..(52)
<223> n is a, c, g, or t

<400> 408

gcggactcgc agactttaag caaacacgtg tggggccgct cccgcctggg tngcagttgt 60

ccgcgtcggt caccagcggg gccaggtagt ccgagtgccg gatcccgaaa ctccagccgc 120

ggg 123

<210> 409
<211> 109
<212> DNA
<213> Homo sapiens

<400> 409

aagagaagac tcacagtatc aggtctactg aaggagatac ggtgattcct gttcttggct 60

ttgtagattc atctggtata aacagcactc ctgagttatg accttttga 109

<210> 410
<211> 173
<212> DNA
<213> Homo sapiens

<400> 410

gctgagtatg ttaagctctt tatgactgtt tttgtagtgg tatagagtac tgcagaatac 60

agtaagctgc tttattgtag catttcttga tgttgcttag tcacttattt cataaacaac 120

ttaatgtttt gaataatttc ttactaaaca ttttgttatt gggcaagtga ttg 173

<210> 411
<211> 197
<212> DNA

<213> Homo sapiens

<400> 411

```
agtgctaagg agtatagcag atgacttata tgtgtgttgg ctgggagaat atcatcttaa    60
agtgagagtg atgttgtgga gacagttgaa atgtcagtgc tagagcctct gtggtgtgaa   120
tgggcacgtt aggttgttgc attagaaagt gactgtttct gacagaaatt tgtagctttg   180
tgcaaactca cccacca                                                  197
```

<210> 412
<211> 241
<212> DNA
<213> Homo sapiens

<400> 412

```
ttgttcccga ctagctgcct tgcacattat tttcattttc ctggaatttg atacagagag    60
caatttatag ccaattgata gcttatgctg tttcaatgta aattcgtggt aaataactta   120
ggaactgcct tttctttttc tttgaaaacc tacttataac tgttgctaat aagaatgtgt   180
attgttcagg acaacttgtc tccatacagt tgggttgtaa ccctcatgct tggcccaaat   240
a                                                                   241
```

<210> 413
<211> 272
<212> DNA
<213> Homo sapiens

<400> 413

```
caacctctta caacccaggc attcctttct atcgataatt actctttcaa ccaattgcca    60
atcagaaaat tgttatatct acctataatc tagaagcccc cacatcaagt tgttttgcct   120
ttctggacag gaccaatgta tatcttaaat gtatttgatt gatctctcat gtctccctaa   180
aatgtataaa accacgctgt tccccgacca cctggagcac atgttctcag ggtctcctga   240
gggctgtgtc acaggccatg ttcacttaca tt                                 272
```

<210> 414
<211> 203
<212> DNA
<213> Homo sapiens

<400> 414

```
ttttcccaac tctatagcta gatttaaaag tcccagtaaa attttgtaaa caaaatcata        60
taagaaaagg caaggctggc tcttccctat ggtcctttag tggagctata tttgcataga       120
tcctagacaa atgatgcaaa acaaattccc tccaatttcc actagcaatc tccctaattc       180
gctcaaccct tacataagca tca                                               203
```

<210> 415
<211> 146
<212> DNA
<213> Homo sapiens

<400> 415

```
atgttcatag gttctcaacc ctcaccccca ccacgggaga ctagagctgc aggatcccag        60
gggaggggtc tctcctccca ccccaaggca tcaagccctt ctccctgcac tcaataaacc       120
ctcaataaat attctcattg tcaagg                                            146
```

<210> 416
<211> 149
<212> DNA
<213> Homo sapiens

<400> 416

```
aatgttcata ggttctaaac cctcaccccc cccacgggag actagagctg caggatccca        60
ggggaggggt ctctcctccc accccaaggc atcaagccct tctccctgca ctcaataaac       120
cctcaataaa tattctcatt gtcaatcag                                         149
```

<210> 417
<211> 64
<212> DNA
<213> Homo sapiens

<400> 417

```
atgttcatag gttctcaacc ctcacccccc accacgggag actagagctc aggatcccag        60
ggga                                                                     64
```

<210> 418
<211> 152
<212> DNA
<213> Homo sapiens

<400> 418

```
aatgttcata ggttctaaac cctcaccccc cccacgggag actagagctg caggatccca        60
ggggaggggt ctctcctccc accccaaggc atcaagccct tctccctgca ctcaataaac       120
```

cctcaataaa tattctcatt gtcaatcagc aa 152

<210> 419
<211> 151
<212> DNA
<213> Homo sapiens

<400> 419

atgttcatag gttctcaacc ctcaccccc ccacgggaga ctagagctgc aggatcccag 60

gggaggggtc tctcctccca ccccaaggca tcaagccctt ctccctgcac tcaataaacc 120

ctcaataaat attctcattg tcaatcagca a 151

<210> 420
<211> 254
<212> DNA
<213> Homo sapiens

<400> 420

gcagctattt gagcctgacg cctgagcagt ggaagtccca cagaagctac agctgccagg 60

tcacgcatga agggagcacc gtggagaaga cagtggcccc tacagaatgt tcataggttc 120

taaaccctca cccccccac gggagactag agctgcagga tcccagggga ggggtctctc 180

ctcccacccc aaggcatcaa gcccttctcc ctgcactcaa taaaccctca ataaatattc 240

tcattgtcaa tcag 254

<210> 421
<211> 66
<212> DNA
<213> Homo sapiens

<400> 421

ggagaaccac cccaccccca atgtccacca tgaccctctt cccaacgctg aacctgtgct 60

ccctcc 66

<210> 422
<211> 147
<212> DNA
<213> Homo sapiens

<400> 422

atgttcatag gttctcaacc ctcaccccc accacgggag actagagctg caggatccca 60

ggggaggggt ctctcctccc accccaaggc atcaagccct tctccctgca ctcaataaac 120

cctcaatata tattctcatt gtcaatc 147

<210> 423
<211> 212

<212> DNA
<213> Homo sapiens

<400> 423

```
gatgcatgag gctctgcaca accactacac gcagaagagc ctctccctgt ctccgggtaa        60
atgagtgcga cggccggcaa gccccgctc cccaggctct cggggtcgcg cgaggatgct        120
tggcacgtac cccgtgtaca tacttcccgg gcgcccagca tggaaataaa gcacccagcg        180
ctgccctggg cccctgcgca actttcttgt ac                                      212
```

<210> 424
<211> 236
<212> DNA
<213> Homo sapiens

<400> 424

```
actctcaggc tgcgtccagc gacagtgccc agggctctga tgtgtctctc acagcttgaa        60
aagcctgaga cagctgtctt gtgagggact gagatgcagg atttcttcac gcctcccctt        120
tgtgacttca agagcctctg gcatctcttt ctgcaaaggc acctgaatgt gtctgcgtcc        180
ctgttagcat aatgtgagga ggtggagaga cagcccaccc ttgtgtccac tgtgac            236
```

<210> 425
<211> 92
<212> DNA
<213> Homo sapiens

<400> 425

```
gctgagcaaa gcagactacg agaaacacaa agtttacgcc tgcgaagtca cccatcaggg        60
cctgagctcg cccgtcacaa agagcttcaa ca                                      92
```

<210> 426
<211> 174
<212> DNA
<213> Homo sapiens

<220>
<221> misc_feature
<222> (27)..(27)
<223> n is a, c, g, or t

<220>
<221> misc_feature
<222> (78)..(78)
<223> n is a, c, g, or t

<220>
<221> misc_feature
<222> (96)..(96)

<223> n is a, c, g, or t

<220>
<221> misc_feature
<222> (123)..(123)
<223> n is a, c, g, or t

<400> 426

acgagccctc tcacagtgga atggagngca cggtctgaat ctgcacagag caagatgctg 60

agtggagtcg ggggcttngt gctgggcctg ctcttncttg ggccgggct gttcatctac 120

ttnaggaatc agaaaggaca ctctggactt cagccaacag gattcctgaa ctga 174

<210> 427
<211> 150
<212> DNA
<213> Homo sapiens

<400> 427

aatgttcata ggttctcaac cctcaccccc caccacggga gactagagct gcaggatccc 60

aggggagggg tctctcctcc caccccaagg catcaagccc ttctccctgc actcaataaa 120

ccctcaataa atattctcat tgtcaatcag 150

<210> 428
<211> 150
<212> DNA
<213> Homo sapiens

<400> 428

aatgttcata ggttctcatc cctcaccccc caccacggga gactagagct gcaggatccc 60

aggggagggg tctctcctcc caccccaagg catcaagccc ttctccctgc actcaataaa 120

ccctcaataa atattctcat tgtcaatcaa 150

<210> 429
<211> 150
<212> DNA
<213> Homo sapiens

<400> 429

aatgttcata ggttctcaac cctcaccccc caccacggga gactagagct gcaggatccc 60

aggggagggg tctctcctcc caccccaagg catcaagccc ttctccctgc actcaataaa 120

ccctcaataa atattctcat tgtcaatcaa 150

<210> 430
<211> 95

<212> DNA
<213> Homo sapiens

<400> 430

ccagacctac acctgcaacg tagatcacaa gcccagcaac accaaagtgg acaagacagt 60

tgagcgcaaa tgttgtgtcg agtgcccacc gtgcc 95

<210> 431
<211> 41
<212> DNA
<213> Homo sapiens

<400> 431
tacaagacca cgcctcccgt gctggactcc gacggctcct t 41

<210> 432
<211> 181
<212> DNA
<213> Homo sapiens

<220>
<221> misc_feature
<222> (43)..(44)
<223> n is a, c, g, or t

<220>
<221> misc_feature
<222> (78)..(79)
<223> n is a, c, g, or t

<220>
<221> misc_feature
<222> (99)..(99)
<223> n is a, c, g, or t

<220>
<221> misc_feature
<222> (110)..(110)
<223> n is a, c, g, or t

<400> 432

gccagagaag cgattagaaa cccctgaggg ccgattactg acnncataaa tcatgagttt 60

gggggctttg cctgggtnnt gttggtacca ggagacatng ttataaccan caacgtcact 120

gctggttcca gtgcaggaga tggtgatcga ctgtccagga gacccagaca cggaggcagg 180

c 181

<210> 433
<211> 131
<212> DNA
<213> Homo sapiens

<400> 433

ctacttcccc gaaccggtga cggtgtcgtg gaactcaggc gccctgacca gcggcgtgca 60

caccttcccg gctgtcctac agtcctcagg actctactcc ctcagcagcg tggtgaccgt 120

gccctccagc a 131

<210> 434
<211> 298
<212> DNA
<213> Homo sapiens

<400> 434

agcagactac gagaaacaca aagtttacgc ctgcgaagtc acccatcagg gcctgagctc 60

gcccgtcaca aagagcttca acaggggaga gtgttagagg gagaagtgcc cccacctgct 120

cctcagttcc agcctgaccc cctcccatcc tttggcctct gaccctttt ccacagggga 180

cctacccta ttgcggtcct ccagctcatc tttcacctca cccccctcct cctccttggc 240

tttaattatg ctaatgttgg aggagaatga ataaataaag tgaatctttg cacctgtg 298

<210> 435
<211> 186
<212> DNA
<213> Homo sapiens

<400> 435

gctgagcaaa gcagactacg agaaacacaa agtctacgcc tgcgaagtca cccatcaggg 60

cctgagctcg cccgtcacaa agagcttcaa caggggagag tgttagaggg agaagtgccc 120

ccacctgctc ctcagttcca gcctgacccc ctcccatcct ttggcctctg accctttttc 180

cacagg 186

<210> 436
<211> 172
<212> DNA
<213> Homo sapiens

<400> 436

agccaatgga aaatctgggt tcaaccagcc cctgccattt cttaagactt tttgctgcac 60

tcacaggatc ctgagctgca cttacctgtg agagtcttca aacttttaaa ccttgccagt 120

caggactttt gctattgcaa atagaaaacc caactcaacc tgcttaagca ga 172

<210> 437
<211> 223
<212> DNA
<213> Homo sapiens

<400> 437

tggtcatgag aagaaggtaa tttccagcct tcaagaagac agacatttag aagaagagct 60

gaaatgtcag gaacaaaaag aagaacagct gcaggaaggg gtgcaccgga aggagcccca 120

gggggccacg tagcagcggc tcagtgggtg gccatcgatt tggaccgtcc cctgcccact 180

tgctccccgt gagcactgcg tacaaacatc caaaagttca aca 223

<210> 438
<211> 283
<212> DNA
<213> Homo sapiens

<400> 438

gtgagaggta acactctaaa tagcccattt catgctcaag acatccaagt caaagaaacc 60

caatagcaca ggtgagtccc ctctgttccc ccccaacacc ccactcacat cagggcccct 120

gccctggagt gtcaccttta ttagctgtga gagacacccc agagccctgg gcactgtcag 180

tgattggggt agaacaaaaa caggacctgg tcagagccca cagatgtggc tagaggaact 240

gtggggtggt gagctccctc ataggctcct gaccacaata tcc 283

<210> 439
<211> 174
<212> DNA
<213> Homo sapiens

<400> 439

gatcagcaaa caggaatacg atgaagccgg gccttccatt gtccaccgca aatgcttcta 60

aaacactttc ctgctcctct ctgtctctag cacacaactg tgaatgtcct gtggaattat 120

gccttcagtt cttttccaaa tcattcctag ccaaagctct gactcgttac ctat 174

<210> 440
<211> 72
<212> DNA
<213> Homo sapiens

<400> 440

acatgagtat ggaatggtgt tttattatga ctttagtttg cattttcctc aattctcgtt 60

aaatccttca tt 72

<210> 441
<211> 181
<212> DNA
<213> Homo sapiens

<400> 441

tggtgacaga gtttgggacc gaggtggagc ccgagtttgg gaccaaggtg gagcccgagt 60

ttgagaccca gttggagcct gagtttgaga cccagctgga acccgagttt gaggaagagg 120

aggaggagga gaaagaggag gagatagcca ctggccaggc attccccttc acaacagtag 180

a 181

<210> 442
<211> 279
<212> DNA
<213> Homo sapiens

<400> 442

gtaggtttgg gagtataacg gtcacccagg gagggggtga agacggagaa gacttacata 60

gcacggtcag gttagggctg gacagatgag gaagagctag caaagggggc ttgaggagca 120

gtggccacta agacaggagt gtgacatttt agaagccaaa agaagaccat gtaattcaag 180

ggagaggtat gatttgctgg gtcagatcta aaaataaatc acacgttttt ttaaactgta 240

gtaattaacc actgaaaact tatgagtgat ccaatatta 279

<210> 443
<211> 275
<212> DNA
<213> Homo sapiens
<400> 443

gtaggtttgg gagtataacg gtcacccagg gagggggtga agacggagaa gacttacata 60

gcacggtcag gttagggctg gacagatgag gaagagctag caaagggggc ttgaggagca 120

gtggccacta agacaggagt gtgacatttt agaagccaaa agaagaccat gtaattcaag 180

ggagaggtat gatttgctgg gtcagatcta aaaataaatc acacgttttt ttaaactgta 240

gtaattaacc actgaaaact tatgagtgat ccaat 275

<210> 444
<211> 197
<212> DNA
<213> Homo sapiens

<400> 444

atgtcaagaa aatgacggtc acagaccagg tgaactgccc caagctctcg taaccaggtt 60

ctacagggag gctgcaccca ctccatgtta cttctgcttc gctttcccct accccacccc 120

ccccccataa agacaaacca atcaaccacg acaaaggaag ttgacctgaa catgtaacca 180

tgccctaccc tgttacc 197

<210> 445
<211> 76

<212> DNA
<213> Homo sapiens

<400> 445

ggaggatgcg ctgtggggtt gtttttgcca taagcgaact ttgtgcctgt cctagaagtg 60

aaaattgttc agtcca 76

<210> 446
<211> 173
<212> DNA
<213> Homo sapiens

<400> 446

gaaggagaac gaatccagtg gaaagaaagc tggtgaggga aaagatgccc tgcagaaaag 60

tcccctttcc cccactgtct ggacactggt gaatgacatt agaagagacc caccccattc 120

aagtcccctc actggctcct tttctcccca ctacaccact tccaaaatct gaa 173

<210> 447
<211> 169
<212> DNA
<213> Homo sapiens

<400> 447

gagaagtcac tcacactggc cacaaggacg ctggctactg tctattaaaa ttttgatgtt 60

tctgtgaaat tctcagagtg tttaattgta ctcaatggta tcattacaat tttctgtaag 120

agaaaatatt acttatttat cctagtattc ctaacctgtc agaataata 169

<210> 448
<211> 38
<212> DNA
<213> Homo sapiens

<400> 448
tttcccccac tgtctggaca ctggtgaatg acattaga 38

<210> 449
<211> 275
<212> DNA
<213> Homo sapiens

<400> 449

```
acacaataca atcaggagtt ttcaaatttt tgattcagta tttgaatttc ttcttcataa        60
atgtagttgg aatttatcct agtatttttc tttacctgaa ggagggccat ttatttttaa       120
tttcactaca tttttctttg catgattatt aaaataaaaa ctgcctctgt tgtgtttctc       180
actggaggct ggaatgaatg atcactagaa cacaaaagag tgaatgatga cacttgaagt       240
caaagcagtt gtactgatca ccagaaccaa taaag                                  275
```

<210> 450
<211> 273
<212> DNA
<213> Homo sapiens

<400> 450

```
gactgagtga gtaactgcaa gctacagggg gcagcttacg tatggcacac agggtggggc        60
tagcttggtt tcataggctc tctgtggatt ggatgattta aataattttg gtgggccctg       120
gggtttaggg actgtcccta gttgtttggt gctaggtccc agggcagatt agggcagatg       180
tgagtgtgag agcatgataa ggaaagtctt caaggtgtgg aattactcaa ctgctggaga       240
aagggaattt atcagccttt agccagggcc tca                                    273
```

<210> 451
<211> 93
<212> DNA
<213> Homo sapiens

<400> 451

```
tggagaagca ctggtgtctg cagcacccct cagttcctgt gcctcagccc acaggccact        60
gtgataatgg tctgtttagc acttctgtat tta                                     93
```

<210> 452
<211> 111
<212> DNA
<213> Homo sapiens

<400> 452

```
ttaagacatt ggagtgattt ctggaaatgt tttctttaag aaggctcacg tgatgtttgt        60
gtttacttgt ggttgcccta tcctatgctg cataaatcct tgaaaggaaa g                111
```

<210> 453
<211> 137
<212> DNA
<213> Homo sapiens

<400> 453

ttaagacatt ggagtgattt ctggaaatgt tttctttaag aaggctcacg tgatgtttgt 60

gtttacttgt ggttgcccta tcctatgctg cataaatcct tgaaaggaaa ggttttagtt 120

agttgctttc tttcttc 137

<210> 454
<211> 148
<212> DNA
<213> Homo sapiens

<220>
<221> misc_feature
<222> (89)..(89)
<223> n is a, c, g, or t

<220>
<221> misc_feature
<222> (91)..(93)
<223> n is a, c, g, or t

<400> 454

gtcattgata cctgtagtaa gttgacaatg tggtgaaatt tcaaaattat atgtaacttc 60

tactagtttt actttctccc ccaagtctnt nnnaactcat gatttttaca cacacaatcc 120

agaacttatt atatagcctc taagtctt 148

<210> 455
<211> 257
<212> DNA
<213> Homo sapiens

<400> 455

ttgttactgc tgattcttgt aaatcttttt gcttctactt tcatcttaaa ctaatacgtg 60

ccagatataa ctgtcttgtt tcagtgagag acgccctatt tctatgtcat ttttaatgta 120

tctatttgta caattttaaa gttcttattt tagtatacgt ataaatatca gtattctgac 180

atgtaagaaa atgttacggc atcacactta tattttatga acattgtact gttgctttaa 240

tatgagcttc aatataa 257

<210> 456
<211> 119
<212> DNA
<213> Homo sapiens

<400> 456

actttaataa aggttatcca taccaataaa aagtgtacaa cacagcattt tctgttaaat 60

tattattggt tttcagttgt aatttggtat tttttctggc atgcgtttat taatttatt 119

<210> 457
<211> 198
<212> DNA
<213> Homo sapiens

<400> 457

gattttcttt tgacacagct ccaaggccac cagctatgca aggccacaag ttatgcacta 60

tatgattaac tgcttttgtt ttacttttgt aagtccactt ataaaaaccc tgctctgtct 120

ttgtttaatg ctcagctttt tggatttgaa tccactcagc cggtgcacac cttaaaataa 180

acatcctcct gtactctc 198

<210> 458
<211> 38
<212> DNA
<213> Homo sapiens

<400> 458
tttggatttg aatccactca gccggtgcac accttaaa 38

<210> 459
<211> 53
<212> DNA
<213> Homo sapiens

<400> 459
gaccaggaat tcggcttcga cgttggccct gtctgcttcc tgtaaactcc ctc 53

<210> 460
<211> 37
<212> DNA
<213> Homo sapiens

<400> 460
tgggctggag ccgcacacgc tctcctccca tgttaaa 37

<210> 461
<211> 199
<212> DNA
<213> Homo sapiens

<220>
<221> misc_feature
<222> (113)..(113)
<223> n is a, c, g, or t

<220>
<221> misc_feature
<222> (115)..(115)
<223> n is a, c, g, or t

<220>
<221> misc_feature
<222> (122)..(122)
<223> n is a, c, g, or t

<400> 461

```
aagggagccg ggaggatggg ctgcagctgt ggaggagggt ttcagaggag agaggtcgga      60
gagcagaggc ctgagaagcc agaggcaggt ggagagaggg tggaaagtga gcntnagcgg     120
gncttgggct ggagccgcac acgctctcct cccatgttaa atagcacctt tagaaaaatt     180
cacaagtccc catccacat                                                  199
```

<210> 462
<211> 211
<212> DNA
<213> Homo sapiens

<400> 462

```
gaaaaaactt tctctttgcc atttcttctt cttctttttt aactgaaagc tgaatccttc      60
catttcttct gcacatctac ttgcttaaat tgtgggcaaa agagaaaaag aaggattgat     120
cagagcattg tgcaatacag tttcattaac tccttccccc gctcccccaa aaatttgaat     180
tttttttttca acactcttac acctgttatg g                                   211
```

<210> 463
<211> 211
<212> DNA
<213> Homo sapiens

<400> 463

```
gaaaaaactt tctctttgcc atttcttctt cttctttttt aactgaaagc tgaatccttc      60
catttcttct gcacatctac ttgcttaaat tgtgggcaaa agagaaaaag aaggattgat     120
cagagcattg tgcaatacag tttcattaac tccttccctc gctcccccaa aaatttgaat     180
tttttttttca acactcttac acctgttatg g                                   211
```

<210> 464
<211> 286
<212> DNA
<213> Homo sapiens

<400> 464

```
taaagacgca tgttatggtg ctaatgtact ttcactttta aactctagat cagaattgtt      60
gacttgcatt cagaacataa atgcacaaaa tctgtacatg tctcccatca gaaagattca     120
ttggcatgcc acaggggatt ctcctccttc atcctgtaaa ggtcaacaat aaaaaccaaa     180
ttatggggct gcttttgtca cactagcata gagaatgtgt tgaaatttaa ctttgtaagc     240
ttgtatgtgg ttgttgatct tttttttcct tacagacacc cataat                    286
```

<210> 465

<211> 275
<212> DNA
<213> Homo sapiens

<400> 465

```
gttatggtgc taatgtactt tcacttttaa actctagatc agaattgttg acttgcattc      60
agaacataaa tgcacaaaat ctgtacatgt ctcccatcag aaagattcat tggcatgcca     120
caggggattt tcctccttca tcctgtaaag gtcaacaata aaaaccaaat tatggggctg     180
cttttgtcac actagcatag agaatgtgtt gaaatttaac tttgtaagct tgtatgtggt     240
tgttgatctt ttttttcctt acagacaccc ataat                                275
```

<210> 466
<211> 261
<212> DNA
<213> Homo sapiens

<400> 466

```
agccgctgcc aagtctgtat gagaagaaca taatgaagcc tgactcagct aatgtcacaa      60
catggtgcta cttcttcttc tttttgttaa cagcaacgaa ccctagaaat atatcctgtg     120
tacctcactg tccaatatga aaaccgtaaa gtgccttata ggaatttgcg taactaacac     180
accctgcttc attgacctct acttgctgaa ggagaaaaag acagcgataa gctttcaata     240
gtggcatacc aaatggcact t                                               261
```

<210> 467
<211> 238
<212> DNA
<213> Homo sapiens

<400> 467

```
acccatcctg ttcgtgaata ggtctcaggg gttgggggag ggactgccag atttggacac      60
tatatttttt tttaaattca acttgaagat gtgtatttcc cctgaccttc aaaaaatgtt     120
ccaaggtaag cctcgtaaag gtcatcccac catcaccaaa gcctccgttt ttaacaacct     180
ccaacacgat ccatttagag gccaaatgtc attctgcagg tgccttcccg atggatta       238
```

<210> 468
<211> 251
<212> DNA
<213> Homo sapiens

<400> 468

atataattgg acaaacgctg gcaaaaagaa aaaaatggta agcaaaaaac ccaagataaa 60

gtttcgagga catcaggcct tttgaaatac aatgtcaaat gacacattgt acggtttcaa 120

aaaatccgct agacatgtca taagttttaa ctgtaatgcc caggaaagga tatcttaaaa 180

tattctaaac ttgtgtaaca aaggaataat taactgtaat agtttttcaa taaatcgagt 240

tgggtgtttc c 251

<210> 469
<211> 270
<212> DNA
<213> Homo sapiens

<400> 469

gaaagagcat cgttccaatg cttgttcact gttcctctgt catactgtat ctggaatgct 60

ttgtaatact tgcatgcttc ttagaccaga acatgtaggt ccccttgtgt ctcaatactt 120

ttttttttctt aattgcattt gttggctcta ttttaatttt tttcttttaa aataaacagc 180

tgggaccatc ccaaaagaca agccatgcat acaactttgg tcatgtatct ctgcaaagca 240

tcaaattaaa tgcacgcttt tgtcatgtca 270

<210> 470
<211> 245
<212> DNA
<213> Homo sapiens

<400> 470

attgtgctat aatccctatt tagttcaaaa ttaaccagaa tttttccatg tgaaatggac 60

caaactcata ttattgttat gtaaatacag agttttaatg cagtatgaca tcccacaggg 120

gaaaagaatg tctgtagtgg gtgactgtta tcaaatattt tatagaatac aatgaacggt 180

gaacagactg gtaacttgtt tgagttccca tgacagattt gagacttgtc aatagcaaat 240

cattt 245

<210> 471
<211> 221
<212> DNA
<213> Homo sapiens

<400> 471

taatgtcatc ctgtactcgg cacaaatcaa aggccaatac aagtctgaaa agcagaaata 60

aatatttttc caggtttttg ctcgggcaca tactaactgc tttgggcatt ttaatctggt 120

ctccaaacac caaagaccca tttcgagcct gctattagcc tgctgctgac tctatcactt 180

ggagcaataa tgtggggtta tggtggtgga atcttgtata t 221

<210> 472
<211> 129
<212> DNA
<213> Homo sapiens

<400> 472

aatgcatatg gaggtaggct gaaaagaatg taatttttat tttctgaaat acagatttga 60

gctatcagac caacaaacct tccccctgaa aagtgagcag caacgtaaaa acgtatgtga 120

agcctctct 129

<210> 473
<211> 94
<212> DNA
<213> Homo sapiens

<400> 473

gcctcccatt caagtgaagt tataatttac actgagggtt tcaaaattcg actagaagtg 60

gagatatatt atttatttat gcactgtact gtat 94

<210> 474
<211> 202
<212> DNA
<213> Homo sapiens

<400> 474

taatggttaa cgaaccgggt cgacatcaca aaggagggtg gagactcttt ttactaactt 60

gaatgagaca aaagcagtgg tgtcagttta taatcctgat gcatttcagt aataatgtag 120

aaaaacatta ttttaaaaaa gttccaacac acagccatga ggagcctcag ttttgaaaga 180

ggtgcataat aaaactacta ac 202

<210> 475
<211> 243
<212> DNA
<213> Homo sapiens

<400> 475

ggatttttc ccctgtagta gtgaggtaac atgcttgaat gtcactgtga tatttatttc 60

ctctttgttc agttgttttt gaattcctgt taagtacatg ttttaatact ttgagcgatt 120

taagatactt ttctttttgc ccatcatttt ccccaaggaa tgtaattcac ataaatccaa 180

agctcatttt tttttttatt gtacacaagt agtataatgt ttgcttttcc caataaacct 240

caa 243

<210> 476

<211> 137
<212> DNA
<213> Homo sapiens

<400> 476

ggatttttc ccctgtagta gtgaggtaac atgcttgaat gtcactgtga tatttatttc 60

ctctttgttc agttgttttt gaattcctgt taagtacatg ttttaatact ttgagcgatt 120

taagatactt ttctttt 137

<210> 477
<211> 112
<212> DNA
<213> Homo sapiens

<400> 477

atttagtat ggtgtctgtt tatgtaactc tgacttgctg gaaaagttga aactccaaat 60

aatctgaaac tagaaaagaa atagcacata attactacct tccccttggc gg 112

<210> 478
<211> 207
<212> DNA
<213> Homo sapiens

<400> 478

aagtgttcta caaaagaatc cctgtggtta gcttactctt aggttagatc ttctaataag 60

gctgaaattc aaaatcaaaa ccttagtgtg tccgagtcca gcctgggttc cagcattctg 120

ttcaggccac ttctgaacgg ccgaaggtgc cccattccag acctgcccat ttgatggaca 180

gagcagacag cccggaacag attcaag 207

<210> 479
<211> 237
<212> DNA
<213> Homo sapiens

<400> 479

acaaaagctc ccctgatcca actagcacac tgtcaaatac agtgtcatat gagaggtcca 60

cagacggtag tttccaagac cgtttcaggg aattcgagga ttccacctta aaacctaaca 120

gaaaaaaacc cactgaaaat attatcatag acctggacaa agaggacaag gatttaatat 180

tgacaattac agagagtacc atccttgaaa ttctacctga gctgacatcg gataaaa 237

<210> 480
<211> 113
<212> DNA
<213> Homo sapiens

<400> 480

caaagtgcta ataattaact caaccaggtc tactttttaa tggctttcat aacactaact 60

cataaggtta ccgatcaatg catttcatac ggatatagac ctagggctct gga 113

<210> 481
<211> 146
<212> DNA
<213> Homo sapiens

<400> 481

gctttgatat ttcaatgtta gcctcaattt ctgaacacca taggtagaat gtaaagcttg 60

tctgatcgtt caaagcatga aatggatact tatatggaaa ttctgctcag atagaatgac 120

agtccgtcaa aacagattgt ttgcaa 146

<210> 482
<211> 179
<212> DNA
<213> Homo sapiens
<400> 482

tgtggtagcc tcacttttaa tgaacaaatg gcctttatta aaaactgagt gactctatat 60

agctgatcag ttttttcacc tggaagcatt tgtttttact ttgatatgac tgtttttcgg 120

acagtttatt tgttgagagt gtgaccaaaa gttacatgtt tgcacctttt tagttgaaa 179

<210> 483
<211> 115
<212> DNA
<213> Homo sapiens

<400> 483

tgaacaaatg gcctttatta aaaactgagt gactctatat agctgatcag ttttttcacc 60

tggaagcatt tgtttttact ttgatatgac tgtttttcgg acagtttatt tgttg 115

<210> 484
<211> 221
<212> DNA
<213> Homo sapiens

<400> 484

ttgctggggc ttgtcctaga ggctccagct ttggcacagt ggttcctggc tgctgccatg 60

tttcagatga ggagggagag aaggaggccg ccagactcga gaggtgggag gaactccttg 120

cacacaccct gagcttttgc cacttttatc atttttgagc aactcccttt cagctaaaag 180

gccacccctt tatcgcattg ctgtccttgg gtagaatata a 221

<210> 485
<211> 73
<212> DNA
<213> Homo sapiens

<400> 485

tctgtaattc attgagcagt tagctcattt gagataaagt caaatgccaa acactagctc 60

tgtattaatc ccc 73

<210> 486
<211> 55
<212> DNA
<213> Homo sapiens

<400> 486
atactggaaa cctaactgca atgtggatgt tttacccaca tgacttatta tgcat 55

<210> 487
<211> 164
<212> DNA
<213> Homo sapiens

<400> 487

gtgtggatgc taaggtgttt gttttgtttt gtatttttat gtagcgcgtg ggtattgtgc 60

ctagaaatga agtcattatt agggatttaa atatgcaact catggagtgg atgagaccag 120

ctagaaagat aatagagtgt gaagaggaga tcggaaattc aata 164

<210> 488
<211> 233
<212> DNA
<213> Homo sapiens

<220>
<221> misc_feature
<222> (35)..(35)
<223> n is a, c, g, or t

<220>
<221> misc_feature
<222> (53)..(53)
<223> n is a, c, g, or t

<220>
<221> misc_feature
<222> (60)..(60)
<223> n is a, c, g, or t

<400> 488

tggagtaaca cccagatctc tgcagcagtt aagcntgggg gcctagaact agnctagagn 60

tagaagaagg gacaaatgca atccgacctt tggatctaca cattcctctt gcttcaatgg 120

gtgtcattta agaattagag gaaaatatta ggagatggag aactagagtt gaggaaacca 180

aaagaagagg agtcacagaa aaccagctct ctctgtgcaa ggcatcttga aag 233

<210> 489
<211> 195
<212> DNA
<213> Homo sapiens

<400> 489

tatgagttat tcaaggagga gactttttaa agacagcaac gcaattcttg taacttgtgt 60

aaatagcccc atctttcaga gtgataccat ttctacattt gataatgcct gtattcctgt 120

aggatgtata tagtttaggg gatttttttt ttgtttggtt ttgtttttta gaagtcaata 180

tgtctggttt tattt 195

<210> 490
<211> 268
<212> DNA
<213> Homo sapiens

<400> 490

atgagtcaaa atccgctctc catgcttact cttgacaccc cattgaagcc actcattgtg 60

tgtgcgtctg ggtgtgaagt ccagctccgt gtggtcctgt gcttgtactg ccctgctttg 120

cagttccttt gcacttactc atcgagtgct gttttgaaat gctgacatta tataaacgta 180

aaagaaaatg taaaaaaaaa aaacccacac acaaacaaac ccatacgatc tgtatttgta 240

tatacacgtg tccgtacaag tataacta 268

<210> 491
<211> 289
<212> DNA
<213> Homo sapiens

<400> 491

tcccaagtag tgctgactga ctctcctggt gacaggggtt tgtgtccgag cccctgcggt 60

caaggagtgt ggagcaaaac gtgggtacta gggtgggagg cggggaaagg ccacagcaca 120

ctggcgctcc agcaaagcca aatcatgtct cctctggcca ctgcggtcct ctccttggta 180

catgtcatcc cccagaggag tatccaaagc tattccacta tgcactcatc aaccctggct 240

tgtcagcctt ggggaaggtc actttattca taaaaatgcc tctttgagt 289

<210> 492
<211> 151
<212> DNA
<213> Homo sapiens

<400> 492

ttggaatgtt gtagttacct actgagtagg cggcgatttt tgtatgttat gaacatgcag 60

ttcattattt tgtggttcta ttttactttg tacttgtgtt tgcttaaaca aagtgactgt 120

ttggcttata aacacattga atgcgcttta t 151

<210> 493
<211> 178
<212> DNA
<213> Homo sapiens

<400> 493

ctttggatag aggtgaagaa cttggacatg gctgtttcag gcagctgaag tcaaagggaa 60

tagtaattgg ggaaggggaa gtgggcagaa aggattgttg gccaatatac cttccactcc 120

agtagagagg gaggacttgg ctctgagaac ctccatctga cctaagagga accctcct 178

<210> 494
<211> 100
<212> DNA
<213> Homo sapiens

<400> 494

tgcccccctt aaggctagag gtgagcatgt ccctcacaat tgcacatgtc aagccatcag 60

caaggcgcat cacacaaaag gcaccaagac gtgaaacttt 100

<210> 495
<211> 156
<212> DNA
<213> Homo sapiens

<400> 495

ctgagtaaat gggactgctg tcgttggatg gcactgcgca gctcaggggt gggctctggg 60

aggcggaggc ggaggaggcc gctggagatg gtgctgagga cgaggaggcc ggtgggttgg 120

tcatgctcac taggccactg accaagctga agaggg 156

<210> 496
<211> 233
<212> DNA
<213> Homo sapiens

<400> 496

gaaacacaag agacttaaag gacaggagga ggagatggcc ataggagagg agggttcctc 60

ttaggtcaga tggaggttct cagagccaag tcctccctct ctactggagt ggaaggtcta 120

ttggccaaca atcctttctg cccacttccc cttccccaat tactattccc tttgacttca 180

gctgcctgaa acagccatgt ccaagttctt cacctctatc caaagaactt gat 233

<210> 497
<211> 108
<212> DNA
<213> Homo sapiens

<400> 497

acatgcagta ctgtataccc cccatccctc cctcggtcca ctgaacttca gagcagttcc 60

cattcctgcc ccgcccatct ttttgtgtct cgctgtgata gatcaata 108

<210> 498
<211> 222
<212> DNA
<213> Homo sapiens

<400> 498

atgattatag aaggctgtct tagtgcaaaa aacatactta catttcagac atatccaaag 60

ggaatactca cattttgtta agaagttgaa ctatgactgg agtaaaccat gtattccctt 120

atcttttact ttttttctgt gacatttatg tctcatgtaa tttgcattac tctggtggat 180

tgttctagta ctgtattggg cttcttcgtt aatagattat tt 222

<210> 499
<211> 269
<212> DNA
<213> Homo sapiens

<400> 499

tgctggcact gatattatcc atcatctctt tttggacact tctgtaaatg tgattggatt 60

gtttgaaaga agatttaaag tttcaaagtt ttttgttctg tttttgcttt gcatttggag 120

aaaatattga aagcagggta tgttgtttca ttcaccttga aaaaaccatg agtaaatggg 180

gatatagaat ctctgaatag ctcgctaaaa gattcaagca agggacatga attttgttcc 240

atctatcaat aatatccaga agaacaact 269

<210> 500
<211> 174
<212> DNA
<213> Homo sapiens

<400> 500

gtttgaggta tttaactgac acctaagtgg atctgttgag gaaacagttg gatatacaaa 60

tttagtgttt aaggcagact tccaggcttg aaggaaaaat ttggaagtca tcacgacata 120

tatgtggtat ttaaaattgt gaggttcaag gaccaagccc cataccattt agag 174

<210> 501
<211> 232
<212> DNA
<213> Homo sapiens

<400> 501

tttgttagta catttcagtg tagtcattca tttctagctg tacataggat gaaggagaga 60

tcagatacat gaacatgttt tacatgggtt gctgtattta gaattataaa catttttcat 120

tattggaaag tgtaacgggg accttttgca tacctgttta gaaccaaaac caccatgaca 180

cagtttttat agtgtctgta tatttgtgat gcaatggtct tgtaaaggtt tt 232

<210> 502
<211> 36
<212> DNA
<213> Homo sapiens

<400> 502
aaaaaagttc aactagtatg aaagggttat aaagta 36

<210> 503
<211> 96
<212> DNA
<213> Homo sapiens

<400> 503

catatatttt ttgctacttt tgctgtttta ttttttaaa ttatgttcta aacctatttt 60

cagtttaggt ccctcaataa aaattgctgc tgcttc 96

<210> 504
<211> 122
<212> DNA
<213> Homo sapiens

<400> 504

aggtggtgtt ggcagaggct atcgggctgg acaaggacaa acccaaccgt gtgaccaaag 60

tggctgtgaa gatgttgaag tcggacgcaa cagagaaaga cttgtcagac ctgatctcag 120

aa 122

<210> 505
<211> 286
<212> DNA
<213> Homo sapiens

<400> 505

aactgggggc tctgtggggg ctctgtatat agctatgaag aaaacacaaa gtgtataaat 60
ctgagtatat atttacatgt ctttttaaaa gggtcgttac cagagattta cccatcgggt 120
aagatgctcc tggtggctgg gaggcatcag ttgctatata ttaaaaacaa aaaagaaaaa 180
aaaggaaaat gtttttaaaa aggtcatata ttttttgcta cttttgctgt tttatttttt 240
taaattatgt tctaaaccta ttttcagttt aggtccctca ataaaa 286

<210> 506
<211> 268
<212> DNA
<213> Homo sapiens

<400> 506

gtaaagcttt ggcacataca gtataaaaaa taatcaccca ccataattat accaaattcc 60
tcttatcaac tgcatactaa gtgttttcaa tacaattttt tccgtataaa aatactggga 120
aaaattgata aataacaggt aagagaaaga tatttctagg caattactag gatcatttgg 180
aaaaagtgag tactgtggat atttaaaata tcacagtaac aagatcatgc ttgttcctac 240
agtattgcgg gccagacact taagtgaa 268

<210> 507
<211> 145
<212> DNA
<213> Homo sapiens

<400> 507

gataccgact gaccgtgggc cttacccgaa gaggacagcc catgcagtac aatgtgggtc 60
cctctgtctt caagtaccca ctgaggaatc tgcagcctgc atctgagtac accgtattcc 120
tcgtggccat aaagggcaac caaga 145

<210> 508
<211> 217
<212> DNA
<213> Homo sapiens

<400> 508

gttcccagtg acacttcaga gagctggtag ttagtagcat gttgagccag gcctgggtct 60
gtgtctcttt tctctttctc cttagtcttc tcatagcatt aactaatcta ttgggttcat 120
tattggaatt aacctggtgc tggatatttt caaattgtat ctagtgcagc tgattttaac 180
aataactact gtgttcctgg caatagtgtg ttctgat 217

<210> 509

<211> 133
<212> DNA
<213> Homo sapiens

<400> 509

gtatggatgg gtatgttgag actcaattac ttttttatta gcttccccgt ttggaagatc 60

ccaaacacca aagatggaag gtgaaaataa agactgcgtg accgggaaga aagtttgaat 120

tactaatagt ggg 133

<210> 510
<211> 183
<212> DNA
<213> Homo sapiens

<400> 510

ggattataat atcctcactg gccacaatct gtaaaagtcg atactggcac tttttttgcc 60

ccctcaaagg aaatatgcta atagacagcc cctttgcaaa tataattcct ccttcccaac 120

ccttcaaatt gctaaggccc cactggtcag caccttccct ttcgagtcca ggactactgt 180

tct 183

<210> 511
<211> 227
<212> DNA
<213> Homo sapiens

<400> 511

tatttgaact atatgttgaa gacatctacc agtttctcca aatgcctttt ttaaaactca 60

tcacagaaga ttggtgaaaa tgctgagtat gacacttttc ttcttgcatg catgtcagct 120

acataaacag ttttgtacaa tgaaaattac taatttgttt gacattccat gttaaactac 180

ggtcatgttc agcttcattg catgtaatgt agacctagtc catcaga 227

<210> 512
<211> 199
<212> DNA
<213> Homo sapiens

<400> 512

gggagtcaat gaatagtacc taaaatggaa accaaacaaa acaacttcag gaagtaacaa 60

gggcttgctt agagacatga cggtaaaccc tgaaccatca gctaaaagag gtagatagca 120

gtggttgccc ctggggagag gtaaatgtga tggagaggga acaactgtgt acaaacatgt 180

gactttacgt tttgatcaa 199

<210> 513

<211> 172
<212> DNA
<213> Homo sapiens
<400> 513

```
aactgtgacc gtttctgtgt gaagattttt agctgtattt gtggtctctg tatttatatt        60
tatgtttagc accgtcagtg ttcctatcca atttcaaaaa aggaaaaaaa agagggaaaa       120
ttacaaaaag agagaaaaaa agtgaatgac gtttgtttag ccagtaggag aa               172
```

<210> 514
<211> 121
<212> DNA
<213> Homo sapiens

<400> 514

```
ttatatttat gtttagcacc gtcagtgttc ctatccaatt tcaaaaaagg aaaaaaaaga        60
gggaaaatta caaaaagaga gaaaaaaagt gaatgacgtt tgtttagcca gtaggagaaa       120
a                                                                       121
```

<210> 515
<211> 265
<212> DNA
<213> Homo sapiens

<400> 515

```
tgttttagaa ccagccgtat tttacatgaa gctgtataat taattgtcat tatttttgtt        60
agcaaagatt aaatgtgtca ttggaagcca tcccttttttt tacatttcat acaacagaaa      120
ccagaaaagc aatactgttt ccattttaag gatatgatta atattattaa tataataatg       180
atgatgatga tgatgaaaac taaggatttt tcaagagatc tttctttcca aaacattttt       240
ggacagtacc tgattgtatt ttttt                                             265
```

<210> 516
<211> 169
<212> DNA
<213> Homo sapiens

<400> 516

```
tgttaaactt tggaacacct accaaaaaat aagtttgata acatttaaaa gatgggcgtt        60
tcccccaatg aaatacacaa gtaaacattc caacattgtc tttaggagtg atttgcacct       120
tgcaaaaatg gtcctggagt tggtagattg ctgttgatct tttatcaat                   169
```

<210> 517
<211> 272
<212> DNA
<213> Homo sapiens

<400> 517

```
tttcactctt gctgtctgct cctctcacat catccttgcc tctgtctgtt taatcctcct      60
gtccttcatt ttcctttttt gcctctgcat tcagcatttc tacttccaat ctccctcctc     120
tgctctttct tatttcctct gatctgcaga cttgcttctg tcccctcctt ctgttcccct     180
cctggatgtg tctttggcca acctttcctt ctctgagact tcgtgttctt gttggtagat     240
gggggctgat acttctgggt cttgggcatg tc                                   272
```

<210> 518
<211> 262
<212> DNA
<213> Homo sapiens

<400> 518

```
tttcactctt gctgtctgct cctctcacat catccttgcc tctgtctgtt taatcctcct      60
gtccttcatt ttcctttttt gcctctgcat tcagcatttc tacttccaat ctccctcctc     120
tgctctttct tatttcctct gatctgcaga cttgcttctg tcccctcctt ctgttcccct     180
cctggatgtg tctttggcca acctttcctt ctctgagact tcgtgttctt gttggtagat     240
gggggctgat acttctgggt ct                                              262
```

<210> 519
<211> 201
<212> DNA
<213> Homo sapiens

<400> 519

```
ccggacagtg gccttctcca ctcccctctg acttctccaa gggggctcag tggccagtgc      60
cccccaggag gctccaccct caactcaacc caagcaacag ggacagatga aaaacaaaat     120
ccaatcaggg cgataaatgg cggggggcag gacgtggtgg tctccaggct ggcttcgtgc     180
gttcttgctt ttgtcactgc c                                               201
```

<210> 520
<211> 237
<212> DNA
<213> Homo sapiens

<400> 520

agaagaccca cgtgctaggg gatgaggggc ttcctgggtc ctgttcccta ccccatttgt 60

ggtcacagcc atgaagtcac cgggatgaac ctatccttcc agtggctcgc tccctgtagc 120

tctgcctccc tctccatatc tccttcccct acacctccct ccccacacct ccctactccc 180

ctgggcatct tctggcttga ctggatggaa ggagacttag gaacctacca gttggcc 237

<210> 521
<211> 263
<212> DNA
<213> Homo sapiens

<400> 521

aatcaaccaa tgcaaccagt ttgtgagaaa aaaaaaaaaa agccgaaaaa aaaaaaaaaa 60

aacacctgaa tgcggaagag ctcggctccc gtttagcatt ttgtacttaa ggaaataaaa 120

aaccaacaaa ggatttcaca tttttttaaa aagtgaagat tgctgtatac tatttattca 180

acttataatt tatgttactc cttgatcttt gtcttttgtc atgacaaagc atttatttaa 240

taaagttatg cattcagcaa ctt 263

<210> 522
<211> 300
<212> DNA
<213> Homo sapiens

<400> 522

gatgataatc tttactggtg aaaaggatgg aaaaataaat caacaaatgc aaccagtttg 60

tgagaaaaaa aaaaaaaagc cgaaaaaaaa aaaaaaaaca cctgaatgcg gaagagctcg 120

gctcccgttt agcattttgt acttaaggaa ataaaaaacc aacaaaggat ctcacatttt 180

cttaaaaagt gaagattgct gtatactatt tattcaactt ataatttatg ttactccttg 240

atctttgtct tttgtcatga caaagcattt atttaataaa gttatgcatt cagttcccaa 300

<210> 523
<211> 33
<212> DNA
<213> Homo sapiens

<400> 523
aaacgatgat aatctttact ggtgaaaagg atg 33

<210> 524
<211> 180
<212> DNA
<213> Homo sapiens

<400> 524

acagtgattc cccacgtgtg ttcatctgca cccaccgagc caggcagagg ccagccctcc 60

gtggtgcaca cagcacgcgc ctcagtccat cccattttag tctttaaacc ctcaggaagt 120

cacagtctcc ggacaccaca ccacatgagc ccaacaggtc cacgatggat ccaccagtcc 180

<210> 525
<211> 137
<212> DNA
<213> Homo sapiens

<400> 525

gtgggcagca cttagattcg gagccatgga tagtccggag tccaaggtct ctgggtgagc 60

agacagtcgg ccaaaggcca gcctggagtc aaagagacca gacccctgct tagattgcca 120

tactcgcacc attccaa 137

<210> 526
<211> 259
<212> DNA
<213> Homo sapiens
<400> 526

gctggtgtgt ggatcggatg ggcaagtccc tgccagggtc tccagatggc aatggaagct 60

cctcctgccc cactgggagt agcggctaaa gctgggggat agagggggctg cagggccact 120

ggaaggaaca tggagctgtc atcactcaac aaaaaaccga ggccctcaat ccaccttcag 180

gccccgcccc atgggcccct caccgctggt tggaaagagt gttggtgttg gctggggtgt 240

caataaagct gtgcttggg 259

<210> 527
<211> 161
<212> DNA
<213> Homo sapiens

<400> 527

aatgaaattg ttaattggcc aggcacagtg ggaagcacct gtagtcccag ctactcagga 60

ggctaaagtg agagggtggc tctagcacag tcatcaaggc tgcaggaggc tatgatggag 120

ccactgcact ccagcctaga tgacaaggtg agacgctgtc t 161

<210> 528
<211> 249
<212> DNA
<213> Homo sapiens

<400> 528

cggttgttaa aactggttta gcacaattta tattttccct ctcttgcctt ttttatttgc 60

aataaaaggt attgagccat tttttaaatg acatttttga taaattatgt ttgtactagt 120

tgatgaagga gtttttttta acctgtttat ataattttgc agcagaagcc aaattttttg 180

tatattaaag caccaaattc atgtacagca tgcatcacgg atcaatagac tgtacttatt 240

ttccaataa 249

<210> 529
<211> 221
<212> DNA
<213> Homo sapiens

<400> 529

cctgtaagac aataggccat gttaattaaa ctgaagaagg atatatttgg ctgggtgttt 60

tcaaatgtca gcttaaaatt ggtaattgaa tggaagcaaa attataagaa gaggaaatta 120

aagtcttcca ttgcatgtat tgtaaacaga aggagatggg tgattccttc aattcaaaag 180

ctctctttgg aatgaacaat gtgggcgttt gtaaattctg g 221

<210> 530
<211> 138
<212> DNA
<213> Homo sapiens
<400> 530

ttcccaagcc catgagtcct tgaaaatatt ttttatatat acagtaactt tatgtgtaaa 60

tacataagcg gcgtaagttt aaaggatgtt ggtgttccac gtgttttatt cctgtatgtt 120

gtccaattgt tgacagtt 138

<210> 531
<211> 246
<212> DNA
<213> Homo sapiens

<400> 531

tccaacaaca gtgctgactg tccggataag agctggcaag tgcccttagg atgccgcatg 60

ggaaaaatcg gttatcataa tttcaaagta taaatatatt tattatgtag cgctgcggtt 120

aagaaggaag agtgaggggt ctgtccatgg ggtggcagtg atttcccacc cgcctttctt 180

gaatggcttc gtgttattca gccgtgccct ggcaggaatg gaactccatc agggaacagg 240

gcagct 246

<210> 532
<211> 165
<212> DNA
<213> Homo sapiens

<400> 532

```
ctcagggagc gaacgtggat gaaaaccaca gggattccgg acgccagacc ccattttata      60
cttcactttt ctctacagtg ttgttttgtt gttgttggtt tttattttt atactttggc      120
cataccacag agctagattg cccaggtctg ggctgaataa aacaa                    165
```

<210> 533
<211> 103
<212> DNA
<213> Homo sapiens

<400> 533

```
aaaccttgtt ttattcagcc cagacctggg agatctagct ctgtggtatg gccaaagtat      60
aaaaaataaa aaccaacaac aacaaaacaa cactgtagag aaa                      103
```

<210> 534
<211> 243
<212> DNA
<213> Homo sapiens

<400> 534

```
gggatctcct tttgtgaaaa ccagtttgat gtgctaaaag taaaaagtct attttccagt      60
gtggtcttgt tcagaagcag ccagatttcc aatgttgttt ttcccctcca ctcagaaacc      120
cctgcccttt cccttcagaa aacgatggca ggcattcctt tgagtttaca agcagagact      180
cactccaacc caaactagct gggagttcag aaccatggtg gaataaagaa atgtgcatct      240
ggt                                                                  243
```

<210> 535
<211> 191
<212> DNA
<213> Homo sapiens

<400> 535

```
taggtggtag atattgaggc caagaatatt gcaaaataca tgaagcttca tgcacttaaa      60
gaagtatttt tagaataaga atttgcatac ttacctagtg aaacttttct agaattattt      120
ttcactctaa gtcatgtatg tttctctttg attatttgca tgttatgttt aataagctac      180
tagcaaaata a                                                         191
```

<210> 536
<211> 261
<212> DNA
<213> Homo sapiens

<400> 536

```
gaatggttac tgtttatact gtggtatgtt tttgattaca gcagataatg ctttcttttc        60
cagtcgtctt tgagaataaa ggaaaaaaaa tcttcagatg caatggtttt gtgtagcatc       120
ttgtctatca tgttttgtaa atactggaga agctttgacc aatttgactt agagatggaa       180
tgtaactttg cttacaaaaa ttgctattaa actcctgctt aaggtgttct aattttctgt       240
gagcacacta aaagcgaaaa a                                                 261
```

<210> 537
<211> 217
<212> DNA
<213> Homo sapiens

<400> 537

```
ttctttgtca atctatggac atgcccatat atgaaggaga tgggtgggtc aaaaagggat        60
atcaaatgaa gtgatagggg tcacaatggg gaaattgaag tggtgcataa cattgccaaa       120
atagtgtgcc actagaaatg gtgtaaaggc tgtttttttt ttttttttta aagaaaagtt       180
attaccatgt attttgtgag gcaggtttac aacacta                                217
```

<210> 538
<211> 286
<212> DNA
<213> Homo sapiens

<400> 538

```
acttcaggga agtttcataa aaggaagaat tttaactcag cgtagaaaga tgggtaaatt        60
ttcctcatgt gaaggtgtac tgcctggggt gctgcaggct ggagatgagt attaaaatag       120
gaggagagtg agtgaaagca caggaggagg aagggtcagg caagtttggt gaacacagca       180
actggctata gaacaggagc agtgggagaa agtccagaaa ggtctgctga gcttcagttg       240
tacaaagctg tggagtttgg cctttggtca tggctagatt aggtct                      286
```

<210> 539
<211> 191
<212> DNA
<213> Homo sapiens

<400> 539

aatctctttt tttctggagg ctggcacctg attttgtatc cccctgtagc agcattactg 60

aaatacatag gcttatatac aatgcttctt tcctgtatat tctcttgtct ggctgcaccc 120

ctttttcccg cccccagatt gataagtaat gaaagtgcac tgcagtgagg gtcaaaggag 180

agtcaacata t 191

<210> 540
<211> 267
<212> DNA
<213> Homo sapiens

<400> 540

ttgctttgta tgcactttgt ttttttcttt gggtcttgtt ttttttttcc acttagaaat 60

tgcatttcct gacagaagga ctcaggttgt ctgaagtcac tgcacagtgc atctcagccc 120

acatagtgat ggttcccctg ttcactctac ttagcatgtc cctaccgagt ctcttctcca 180

ctggatggag gaaaaccaag ccgtggcttc ccgctcagcc ctccctgccc ctcccttcaa 240

ccattcccca tgggaaatgt caacaag 267

<210> 541
<211> 211
<212> DNA
<213> Homo sapiens

<400> 541

gtcaggtctt ggtaggtgcc tgcatctgtc tgccttctgg ctgacaatcc tggaaatctg 60

ttctccagaa tccaggccaa aaagttcaca gtcaaatggg gaggggtatt cttcatgcag 120

gagaccccag gccctggagg ctgcaacata cctcaatcct gtcccaggcc ggatcctcct 180

gaagcccttt tcgcagcact gctatcctcc a 211

<210> 542
<211> 132
<212> DNA
<213> Homo sapiens

<400> 542

aaaagttcac agtcaaatgg ggaggggtat tttcatgca ggagacccca ggccctggag 60

gctgcaacat acctcaatcc tgtcccaggc cggatcctcc tgaagccctt ttcgcagcac 120

tgctatcctc ca 132

<210> 543
<211> 157
<212> DNA
<213> Homo sapiens

<400> 543

```
tatgaattcc attcaaatcg ttccttttttg ttaacaaggg gcatggggag gggtgggggt      60
gggggggcag aggcgtctga ccccaggaac ctgcagggcg gggctgggtc ggtgcccttt     120
aaggacaatt ttgaccttgt tcaacctttc cacaaag                              157
```

<210> 544
<211> 258
<212> DNA
<213> Homo sapiens

<220>
<221> misc_feature
<222> (61)..(61)
<223> n is a, c, g, or t

<400> 544

```
atggagtata agctgtttgt agtcaggcct ggagtaatga gggtacctaa atactgaagg      60
natttttatg cagattgact gaaacctgaa tcaaattgga aggagagggc tgaattttga     120
tagactggaa gtattagaga attttctata ctttgactca aggaatggtc aacttttagg     180
aaaagcaact atattatgtc tgttaagatc atagaatctt aacctgaaag ggaccttgga     240
gactatttag tacaactc                                                   258
```

<210> 545
<211> 65
<212> DNA
<213> Homo sapiens

<400> 545

```
ttaacagtac atttgtgtgg ctctcaaaca tccctttgga agggattgtg tgtactatgt      60
aatat                                                                  65
```

<210> 546
<211> 140
<212> DNA
<213> Homo sapiens

<220>
<221> misc_feature
<222> (71)..(71)
<223> n is a, c, g, or t

<220>
<221> misc_feature
<222> (82)..(82)
<223> n is a, c, g, or t

<220>
<221> misc_feature

<222> (94)..(94)
<223> n is a, c, g, or t

<400> 546

cacagccaaa acctctacat ccctacattc acacacttcc tccacacacc atcctgaagt 60

caccccaact nctaccacca anatcaccac caancccacc agtataggaa gcagcacacc 120

catggcccac actacctcag 140

<210> 547
<211> 287
<212> DNA
<213> Homo sapiens

<400> 547

agtgagactg actgcaagcc ccaccctcct tgagactgga gctggcgtct gcatacgaga 60

gacttggttg aacttggttg gtccttgtct gcaccctcga caagaccaca ctttgggact 120

tgggagctgg ggctgaagtt gctctgtacc catgaactcc cagtttgcga attatagaga 180

caatctattt tgttacttgc acttgttatt cgaaccactg agagcgagat gggaagcata 240

gatatctata tttttatttt tactatgagg gccttgtaat aaatttc 287

<210> 548
<211> 213
<212> DNA
<213> Homo sapiens

<400> 548

tgtgttgggg tagactgctc ctgcagagtt tggaagaagt caccagcaaa gccggcctaa 60

ccaagaaaag tcaaggccct tcatgacctt gctgggcaca gaaaacaccc tcgtggagta 120

cactaatttg aactggactg gtctcagtgt gagcacttgg cacactttac taaacacata 180

tacaacccca ccgtgagtca actttaaagt aaa 213

<210> 549
<211> 86
<212> DNA
<213> Homo sapiens

<400> 549

actgaggggg tcctggtgtg catttgcacc ctaaagctgc ttacggtgaa aaggcaaata 60

ggtatagcta ttttgcaggc accttt 86

<210> 550
<211> 62
<212> DNA

<213> Homo sapiens

<400> 550

tatgtaatta taagatgaag cgtagtgaat tgtacagctg ttgtaataat gacctatttc 60

ta 62

<210> 551
<211> 124
<212> DNA
<213> Homo sapiens

<400> 551

ggctcaggtc ctccctacaa gacctaccac tcacccatgc ctatgccact ccatctggac 60

atttaatgaa actgagagac agaggcttgt ttgctttgcc ctcttttcct ggtcaccccc 120

actc 124

<210> 552
<211> 220
<212> DNA
<213> Homo sapiens

<400> 552

ctgtatcact gccttcgttt atattttttt aactgtgata atccccacag gcacattaac 60

tgttgcactt ttgaatgtcc aaaatttata ttttagaaat aataaaaaga aagatactta 120

catgttccca aaacaatggt gtggtgaatg tgtgagaaaa actaacttga tagggtctac 180

caatacaaaa tgtattacga atgcccctgt tcatgttttt 220

<210> 553
<211> 255
<212> DNA
<213> Homo sapiens

<400> 553

attattttga tagcagatgt gctatttatt tatttaatat gtataaggag cctaaacaat 60

agaaagctgt agagattggg tttcattgtt aattggtttg ggagcctcct atgtgtgact 120

tatgacttct ctgtgttctg tgtatttgtt tgaattaatg acctgggata taaagctatg 180

ctagctttca aacaggagat gcctttcaga aatttgtata ttttgcagtt gccagaccaa 240

taaaatacct ggttg 255

<210> 554
<211> 179
<212> DNA
<213> Homo sapiens

<400> 554

```
agaggttata ggtcactcct ggggcctctt gggtccccca cgtgacagtg cctgggaatg        60
tattattctg cagcatgacc tgtgaccagc actgtctcag tttcactttc acatagatgt       120
ccctttcttg gccagttatc ccttcctttt agcctagttc atccaatcct cactgggtg        179
```

<210> 555
<211> 290
<212> DNA
<213> Homo sapiens

<400> 555

```
gaccaaatgg attagactgt aaactgcaaa gtgctgtccg cacatgaggt catctgatta        60
ctgtcctcag atctcttttg tagaggattt caatgtattt ctttatcatt tgagtgtgtg       120
tgtgatggac gaatatgtgt gtgagtttga gaagcatatc gttcgtgtcc agttactttg       180
caaatttgtg gacatttgtg attggacaga ggggtttgtg ctgtggccta acacttgcca       240
agtgaggtgt aggttatgcc tatatgcaaa ttaaacttca cctttcttga                  290
```

<210> 556
<211> 168
<212> DNA
<213> Homo sapiens

<400> 556

```
aggagggaaa ataatagccc agtgagagct gaatgaaaag ggactgaatt taaatatttg        60
taagaacttt gtgatgatga gtaattgtca gacgtgggat agataactga gaggctcaga       120
atctttacca aggatatttt ttaggataag gtagctgcct gttcatga                    168
```

<210> 557
<211> 151
<212> DNA
<213> Homo sapiens

<400> 557

```
tgtaatgcta aaactgaaat ggtccgtgtt tgcattgtta aaaatgatgt gtgaaataga        60
atgagtgcta tggtgttgaa aactgcagtg tccgttatga gtgccaaaaa tttgtcttga       120
aggcagctac actttgaagt ggtctttgaa t                                      151
```

<210> 558
<211> 217
<212> DNA
<213> Homo sapiens

<400> 558

gaccccagtg gaaaacaaag ccaaacaaaa ctgaaccaca aaaaaaaggc tggtgttcac 60

caaaaccaaa cttgttcatt tagataattt gaaaaagttc catagaaaag gcgtgcagta 120

ctaagggaac aatccatgtg attaatgttt tcattatgtt catgtaagaa gccccttatt 180

tttagccata attttgcata ctgaaaatcc aataatc 217

<210> 559
<211> 117
<212> DNA
<213> Homo sapiens
<400> 559

ttaaacagtc tagagtgaag ggaatgtttt aaaatccaga ggcgatcaag tgaagccaac 60

ctttggaggc ccgtagaagt catttggagg aatttggact tcgtgcagta ggaaaga 117

<210> 560
<211> 127
<212> DNA
<213> Homo sapiens

<400> 560

aagagttaaa cagtctagag tgaagggaat gttttaaaat ccagaggcga tcaagtgaag 60

ccaacctttg gaggcccgta gaagtcattt ggaggaattt ggacttcgtg cagtaggaaa 120

gagggaa 127

<210> 561
<211> 155
<212> DNA
<213> Homo sapiens

<400> 561

aaacattacc gggcatggta gcttgcacct gcagtcccag ctacttggga gactgaggtg 60

agaggatcac ctgagtgtag gaggtgaaag cctcaccgaa ctatgactga accactgcac 120

tccagcgtgg gcacttggca ccagagcaag attct 155

<210> 562
<211> 217
<212> DNA
<213> Homo sapiens

<400> 562

gtgtgttaaa atgagggtct cactgcttta ggattgaagt ggctggaaag agtgatgcct 60

ggggaaggag atggagttat gagggtactg tggctggtac tttctgtact aaacatttcc 120

tttttctatt ttaccactaa ttttgtttta aactgtgagc cgtccaagtc agaagaagac 180

agcaaaaaaa gcaacttttc caacatacaa tttactt 217

<210> 563
<211> 172
<212> DNA
<213> Homo sapiens

<400> 563

tgcagtgcta gtcccggcat cctgatggct ccgacaggcc tgctccagag cacggctgac 60

catttttgct ccgggatctc agctcccgtt ccccaagcac actcctagct gctccagtct 120

cagcctgggc agcttccccc tgccttttgc acgtttgcat ccccagcatt tc 172

<210> 564
<211> 258
<212> DNA
<213> Homo sapiens

<400> 564

atgaaaggtg gaagttctac ctagatttga atgagtgttt ttttaaggga atgagaatgt 60

catggtgcta aacctgacaa ataagagatc attgaaatgc tgaaaatttt aacagttttt 120

ttaaaagtat tgagggggca aaaattacca attatggtat acaaaaataa gcctataaat 180

gtgtttcaca ttgctaactt gagtttcagt tgattcagtt tgtaataact agtaatgagc 240

ttctgtttac aataaaaa 258

<210> 565
<211> 143
<212> DNA
<213> Homo sapiens

<400> 565

cttgttcttt tgaagcttgt gctgaggttt tagcttttct atgttttata tgccgctgct 60

ttgaaagaga acctagattc tatagttgta ttattgttgt ttcatacttt aaatttatat 120

ggctgtggaa aaacgaatta aaa 143

<210> 566
<211> 227
<212> DNA
<213> Homo sapiens

<400> 566

agcccaactt cttacccgaa agcatcactg ccttggcccc tccctcccgg ctgcccccat 60

cacctctact gtctcctccc tgggctaagc aggggagaag cgggctgggg gtagcctgga 120

tgtgggccaa gtccactgtc ctccttggcg gcaaaagccc attgaagaag aaccagccca 180

gcctgccccc tatcttgtcc tggaatattt ttggggttgg aactcaa 227

<210> 567
<211> 32
<212> DNA
<213> Homo sapiens

<400> 567
ttataatgag tgcgatatat gttgtcgagg ct 32

<210> 568
<211> 252
<212> DNA
<213> Homo sapiens

<400> 568

ggttctgtaa ggtctttatt tcccataagt aaatattgcc atgggagggg ggtggaggtg 60

gcaaggaagg ggtgaagtgc tagtatgcaa gtgggcagca attatttttg tgttaatcag 120

cagtacaatt tgatcgttgg catggttaaa aaatggaata taagattagc tgttttgtat 180

tttgatgacc aattacgctg tattttaaca cgatgtatgt ctgtttttgt ggtgctctag 240

tggtaaataa at 252

<210> 569
<211> 139
<212> DNA
<213> Homo sapiens

<400> 569

cccctccaag accctgtgtt catttggtgt tcctggaagc aggtgctaca acatgtgagg 60

cattcgggga agctgcacat gtgccacaca gtgacttggc cccagacgca tagactgagg 120

tataaagaca agtatgaat 139

<210> 570
<211> 245
<212> DNA
<213> Homo sapiens

<400> 570

gaaatacgaa tgtagagatc cctaatcatc aaattgttga ttgaaagact gatcataaac 60

caatgctggt attgcacctt ctggaactat gggcttgaga aaaccccag gatcacttct 120

ccttggcttc cttcttttct gtgcttgcat cagtgtggac tcctagaacg tgcgacctgc 180

ctcaagaaaa tgcagttttc aaaaacagac tcagcattca gcctccaatg aataagacat 240

cttcc 245

<210> 571
<211> 97
<212> DNA
<213> Homo sapiens

<400> 571

ttctccccaa ccacttagta gcaacgctac cccagggggt aatgactgca cactgggctt 60

cttttcagaa tgaccctaac gagacacatt tgcccaa 97

<210> 572
<211> 260
<212> DNA
<213> Homo sapiens

<400> 572

gagtaggttc ggtctgaaag gtgtggcctt tatatttgat ccacacacgt tggtctttta 60

accgtgctga gcagaaaaca aaacaggtta agaagagccg ggtggcagct gacagaggaa 120

gccgctcaaa taccttcaca ataaatagtg gcaatatata tatagtttaa gaaggctctc 180

catttggcat cgtttaattt atatgttatg ttctaagcac agctctcttc tcctattttc 240

atcctgcaag caactcaaaa 260

<210> 573
<211> 162
<212> DNA
<213> Homo sapiens

<400> 573

tgtttttgac atcagctgta atcattcctg tgctgtgttt tttattaccc ttggtaggta 60

ttagacttgc ccttttttaa aaaaaggttt ttgcatcgtg gaagcatttg acccagagtg 120

gaacgcgtgg cctatgcagg tggattcctt caggtctttc ct 162

<210> 574
<211> 257
<212> DNA
<213> Homo sapiens

<400> 574

gaaagcctga gtttgcaacc agttgtaggg tttttgttgt gttttttttt ttttttgaa 60

ataaaactat aatataaatt ctcctattaa ataaaattat tttaagtttt agtgtcaaaa 120

gtgagatgct gagagtaggt gataatgtat attttacaga gtgggggttg gcaggatggt 180

gacattgaac atgattgctc tctgtctctt ttttcagctt atgggtattt atcttctatt 240

agtatttgta tcttcag 257

<210> 575
<211> 193
<212> DNA
<213> Homo sapiens

<220>
<221> misc_feature
<222> (120)..(125)
<223> n is a, c, g, or t

<220>
<221> misc_feature
<222> (127)..(127)
<223> n is a, c, g, or t

<400> 575

gaagaaagct aactcagtac actaagagtg atttacatgc ctgcaaataa tttgtgtctg 60

gggtcttgac cctccccaaa tgccttgtta tttatatctc tgcttttaga taacagatgn 120

nnnnntntct atgggcttgt accggcagag gcaacagcag gtccttaaga ctccccaggt 180

gccatgatga aaa 193

<210> 576
<211> 243
<212> DNA
<213> Homo sapiens

<400> 576
gagtaaaaag aaccctttgc tgagtaacca agcctttaat tttgtgtttt tatgaaagga 60

attaaaatac ccacgataaa tatttaccac aacctgtgtc agataaatgg gaaattaaac 120

acagattgta caatgtgagc ttgggagtta atggcccaga ttttactgtt aggcagtaag 180

agttggagta ggtagtcttg ttatcatgag aagaaccttg aacagataca actaatttac 240

ata 243

<210> 577
<211> 74
<212> DNA
<213> Homo sapiens

<400> 577

cgatatgact tccatgtaaa cgttcatcca ctctgcctgc ttacaccctg ccctcatgct 60

aatgtaataa actc 74

<210> 578
<211> 241
<212> DNA
<213> Homo sapiens

<400> 578

ttcatcatat ccaagttcac tctgtcttcc tgagcagtgg aagatcatat tgctgtaact 60

tcttttaagt agttgatgtg gaaaacattt taaagtgaat ttgtcaaaat gctggttttg 120

tgttttatcc aacttttgtg catatatata aagtatgtca tggcatggtt tgcttaggag 180

ttcagagttc cttcatcatc gaaatagtga ttaagtgatc ccagaacaag gaatactaga 240

g 241

<210> 579
<211> 241
<212> DNA
<213> Homo sapiens

<220>
<221> misc_feature
<222> (93)..(93)
<223> n is a, c, g, or t

<220>
<221> misc_feature
<222> (97)..(97)
<223> n is a, c, g, or t

<400> 579

taagaattta tgaaactggg caagttattt cctgggactc aatggtaaag acacagcagt 60

aatccaaatt ttccatcttt gaaattttcc atncttncag tcaatattag taatacctgg 120

gtcaaagggg agagttaggc ataccaatta atgatcatca gaaatgacat agtcctacaa 180

aagcaaagaa aatttagaga cactttctta aaaatacgac tcttggtact gttgaagaaa 240

a 241

<210> 580
<211> 57
<212> DNA
<213> Homo sapiens

<400> 580
tattgaggac ccatggtaaa atgcaaatag atccggtgtc taaatgcatt catattt 57

<210> 581

<211> 162
<212> DNA
<213> Homo sapiens

<400> 581

```
gcattctcaa gaggtcgtgc caatcagcca ctgaaaggaa aggcatcact atggactttc      60
tctattttaa aatggtaaca atcagaggaa ctataagaac acctttagaa ataaaaatac     120
tgggatcaaa ctggcctgca aaaccatagt cagttaattc tt                        162
```

<210> 582
<211> 269
<212> DNA
<213> Homo sapiens

<400> 582

```
atttgcattt taccatgggt cctcaataaa taaatagaat gttgtttttt gtattttaag      60
tttttttttg tttttcccct cagaggaagg atgaaaaaaa gaattaactg actatggttt     120
tgcaggccag tttgatccca gtatttttat ttctaaaggg gttcttatag ttcctctgat     180
tgttaccatt ttaaaataga gaaagtccat agtgatgcct ttcctttcag tggctgattg     240
gcacgacctc ttgagaatgc atgcatgaa                                       269
```

<210> 583
<211> 163
<212> DNA
<213> Homo sapiens

<400> 583

```
agaattaact gactatggtt ttgcaggcca gtttgatccc agtattttta tttctaaagg      60
ggttcttata gttcctctga ttgttaccat tttaaaatag agaaagtcca tagtgatgcc     120
tttcctttca gtggctgatt ggcacgacct cttgagaatg cat                       163
```

<210> 584
<211> 56
<212> DNA
<213> Homo sapiens

<400> 584

```
cacaatctga gcacgctacc aaatctcaaa atatcctaag actaacaaag gcagct          56
```

<210> 585
<211> 263
<212> DNA
<213> Homo sapiens

<400> 585

gagatcactt ggtaactggt ttcatgtgta tccaaaaatc agcatttgga tttaagcttt 60

ctgaatttgg tagtttaaga aacagattta gtttttcagt ggttttaact catgtgaaat 120

aatgattttc caccagcttt gatgcaaaga gatataattt taatgaacga tttatccagc 180

agtttgttcc aggggttgcc tctccttatt tacggggatt actttgtaca tgcagataag 240

ttttcgcaaa cctatttcca ttt 263

<210> 586
<211> 223
<212> DNA
<213> Homo sapiens

<400> 586

ccatgtcagc ctggatagag gtatatgacg tgtgccaaga atttatccca gactcccctg 60

tgtgacagct tcataataaa gttacttaac tgtgcctctt cctccttcct ctccccacac 120

aggatggatg ggcatctttc tccttgacca ccctactctc ccttcctccc ctgatcacct 180

cccctccctg ctctcccctg gtgatggact tctaacatga gat 223

<210> 587
<211> 210
<212> DNA
<213> Homo sapiens

<400> 587

tttcaagatc ttttgctcac aattcactgc aactgagggg atgtgaatat cattatgcaa 60

taaattaaga gccacagttg gctgaggtga tatgaaagcc aacctgccta agggggggtat 120

gaaagatgtg tatctttcca aacttttaaa acaacgtaag tctgagataa gaacatattt 180

gatggcactg tttggaaaga ggtgtcctta 210

<210> 588
<211> 176
<212> DNA
<213> Homo sapiens

<400> 588

tcctgcaggc atccgtgggg gaaaaaaaat ctctcagaac cctcaactat tctgttccac 60

acccaatgct gctccaccct cccccagaca cagcccaagt ccctccgcgg ctggagcgaa 120

gccttctgca gcaggaactc tggacccctg ggcctcatca cagcaatatt taacaa 176

<210> 589
<211> 242
<212> DNA
<213> Homo sapiens

<400> 589

aggaagtacc cgctccataa gacccttaca tttggacagt caaggtgcac aattgtatgt 60
gaccacaacc atgcaccttg gacataaatg tgtgtaactg cacatggccc atcccatctg 120
aataaggtcc tactctcaga cccctttgc agtacagtag gtgtgctgat aaccaaggcc 180
cctcttcctg gcctgttaac gtatgtgatt atatttgtct gggttccagt gtataagaca 240
tg 242

<210> 590
<211> 237
<212> DNA
<213> Homo sapiens

<400> 590

aataaatgcc tcaggcgtgc tttttgattc atttgataaa caaagcatct tttatgtgga 60
atataccatt ttgggtcctg aggataagag agatgagggc attagatcac tgacagctga 120
agatagaaga acatctttgg tttgattgtt taaataatat ttcaatgcct attctttgca 180
aggtactatg tttcgtaaat taaataggtc tggcccagaa gacccactca attgcct 237

<210> 591
<211> 243
<212> DNA
<213> Homo sapiens

<400> 591

ggcagtagat agtcaaagtc aaatcatttc taatgtttta aaaatgtgct ggtcattttc 60
tttgaaattg acttaactat tttcctttga agagtctgta gcacagaaac agtaaaaaat 120
ttaacttcat gacctaatgt aaaaaagagt gtttgaaggt ttacacaggt ccaggccttg 180
ctttgttacc attctgatgt tggactaatt gactaatcac ctacttatca gacaggaaac 240
ttg 243

<210> 592
<211> 225
<212> DNA
<213> Homo sapiens

<400> 592

cctccaccaa tcatactttg acatttatct atttccttct ccacttatgg atgtaattgg 60
cttgctatag aaactacagt tcagatgctt tgaatgtatg aactacaatg aacaataaag 120
tcctcttctt ttgaagcata ttttggcttc agctttaaga taatcttatg acaagaaggg 180
tcacactgat tcacttaata aattccattc ttacctaaca caagg 225

<210> 593
<211> 220
<212> DNA
<213> Homo sapiens

<400> 593

ctaattgatt agaactgagt cttttatatc aagctaatat ctagctttta tatcaagcta 60

atatcttgac ttctcagcat catagaaggg ggtactgatt tcctaaagtc tttcttgaat 120

ttctattatg caaaattgcc ctgaggccgg gtgtggtggc tcacacctgt aatcccagca 180

ctttgggagg ctgaggtggg aagatccctt actgccagga 220

<210> 594
<211> 131
<212> DNA
<213> Homo sapiens

<400> 594

atacctgtta aatagatcaa ttttgattgc ctactatgtg aactcactgt taaaggcact 60

gaaaatttat catatttcat ttagccacag ccaaaaataa cgcaatacct atgttagcat 120

tttgtgaact c 131

<210> 595
<211> 189
<212> DNA
<213> Homo sapiens

<400> 595

ttaccctcca aaagcaagta gccaaagccg ttgccaaacc ccacccataa atcaatgggc 60

cctttattta tgacgacttt atttattcta atatgatttt atagtattta tatatattgg 120

gtcgtctgct tcccttgtat ttttcttcct ttttttgtaa tattgaaaac gacgatataa 180

ttattataa 189

<210> 596
<211> 178
<212> DNA
<213> Homo sapiens

<400> 596

tactgatctt tatattacag attttttttt cttttaggat tagttcagct tgcccccccct 60

ttccatttcc accatttata gtgagcctct ccataattag tgccaaccat tagtttcgtt 120

catattttta caccaggagt caacaaactg tggccattgg ccaaatatgg cctcccaa 178

<210> 597
<211> 47

<212> DNA
<213> Homo sapiens

<400> 597
aggataaact tgtgtggtgt agagaagtta aaatcctcac gttgtac 47

<210> 598
<211> 213
<212> DNA
<213> Homo sapiens

<400> 598

tacaaagaat atttgggccc agtgctacag aaaaacatga actacatctt atcgtcacaa 60

aatagccatt ataaaatgaa ttttgcagcc tctgtttttt tgaactttga aataaaatgt 120

tcagacaaat attcaacttt ttaaaaacct ccattcattg atagcctgag aaatgtacaa 180

tgaacatgtt taggcagact gctagtattt tgc 213

<210> 599
<211> 80
<212> DNA
<213> Homo sapiens

<400> 599

agagccagga cagtgaggtc aactttgaca attccatcca cccagaagtc ttggagctgc 60

tgcttgacta tgcgtactcc 80

<210> 600
<211> 223
<212> DNA
<213> Homo sapiens

<400> 600

tcatgttaaa gagccgtgtc tcccgccagc actcctcacc ccggtatgaa tgtgtttcct 60

ccacattgta tatccttcca ccctctggct gcctagatca gtaaataaaa ttgatgtaat 120

ataatttata agtaacactg ttgaaaccct gatcccagtg gaggctgtaa cccacctgcc 180

cccgcaccac ccccctgacc cctgttaccg catttgtgtg tat 223

<210> 601
<211> 175
<212> DNA
<213> Homo sapiens

<400> 601

tatttcttgc tattgtgata tgacaagaga cttaacttat cttgctctgt tttcccctgt 60

acacgctgta tataggggtc aatgtgatgc tgctggagac gagaataaac tggactagaa 120

tagtgcattg tatttagtct gtattgatca tggatgccct ccttaatagc catat 175

<210> 602
<211> 163
<212> DNA
<213> Homo sapiens

<400> 602

ctgcaaaagc cgagatgggt tccatgcagt tctccagtgg gacatcagtg cttatccgaa 60

tgtcatcaat ggcaatctct ccggaacgtc ctttccctat cactccctcg aacacaatct 120

ggtactccat gtcgtagctg ggcaggatga tccgcccgtg ctt 163

<210> 603
<211> 238
<212> DNA
<213> Homo sapiens

<400> 603

actggtatat tattgcttca tgttttgtac catcataaga ttttgtgcag attttttta 60

cagaaattat tattttttat gacaatatga cacttgtaaa ttgttgtttc aaaatgaaca 120

gcgaagcctt aactttaaat gacatttgta ttttcagaca ctgagtagca taaaaaccac 180

atagaactga actgtaactt aaattccaaa ctatgactac tacattccaa agaaacag 238

<210> 604
<211> 134
<212> DNA
<213> Homo sapiens

<400> 604

cagaggagct ttattagagg gacagggtga aacatattta caccggccga gcagggacct 60

taagaagcag gcgtgggagc agggtcccag ctcagacgag ttccaccttg gcattggggt 120

acaccgccac cacg 134

<210> 605
<211> 275
<212> DNA
<213> Homo sapiens

<220>
<221> misc_feature
<222> (195)..(195)
<223> n is a, c, g, or t

<400> 605

ccacagtgtt cccactaatg ctatttttta attttttaat ttagtttgtc ataatttggt 60

ttcatcaact cctttgtttt ttccttcttt tttttttttt gagatgaggt ctcactatct 120

tgcccagact ggtttcgaat tgccctccag caattctccc acctcagcct tcagagtagc 180

tggcattgtg ggtangcacc actgtgccca gctcctgttt tataataaat aagccagagc 240

tctatctcca aatggtgcaa atcatcaatg ctatt 275

<210> 606
<211> 157
<212> DNA
<213> Homo sapiens

<400> 606

aggtggtttt gataacacac ttataaggct ttctgtaaaa ggtactatag aagggcgaag 60

aatcgttcaa ctgtcaatca gcctcttgat tctttgtaaa ttgccagggt gggtgggtac 120

atatctcttc ttgattctgc atttcatact taactat 157

<210> 607
<211> 282
<212> DNA
<213> Homo sapiens

<400> 607

tcacctcctg ctggctaccg gggcaggcat gcacccggtg ccagccccgc tctgggcacc 60

acctgccttc cagcccctcc aggacccggt ccccctgctg cccctcactt caggaggggc 120

ctggagcagg gtgaggctgg actttggggg gctgtgaggg aaatatactg gggtccccag 180

attttgcttt aagggggcca gaccctttgc caggctggat tgtacgggcc ccaccttcgc 240

tgtgttcttg ctgcaaagtc tggtcaataa atcactgcac tg 282

<210> 608
<211> 147
<212> DNA
<213> Homo sapiens

<400> 608

tatatacatg gtgctcaata gcaacatctt agcagatgaa gcagtttatg attccactcc 60

ctcctgtatg acaggtagcc actatactga atcaaggtgc tgaactcaaa tcacaaaatt 120

ctggcttacc gatacaacaa ccaatac 147

<210> 609

<211> 216
<212> DNA
<213> Homo sapiens

<400> 609

```
gcaatatatt tgtgattccc catgtaattc ttcaatgtta aacagtgcag tcctctttcg        60
aaagctaaga tgaccatgcg ccctttcctc tgtacatata cccttaagaa cgccccctcc       120
acacactgcc ccccagtata tgccgcattg tactgctgtg ttatatgcta tgtacatgtc       180
agaaaccatt agcattgcat gcaggtttca tattct                                 216
```

<210> 610
<211> 262
<212> DNA
<213> Homo sapiens

<400> 610

```
gaggtgtgat tttaaggcat tgttatattt ttttttattt gtgagtgttt taaaattttg        60
tatttctttt aaacttttta ttttagaaaa atttccaaca tatatagaag tagactattg       120
taaggaaccc ttatgtaccc tccaccagct tcaacaacta tcaacaaaag tttgatcttg       180
ttttaaccac attcctttcc aatttttgtg tttacccccca gattattttg aagcaaattc      240
ctgacctcat aacattttca aa                                                262
```

<210> 611
<211> 280
<212> DNA
<213> Homo sapiens

<400> 611

```
acccacactt aaactaaagg ctaagaatat aggcttgatg ggaaattgaa ggtaggctga        60
gtattgggaa tccaaattga attttgattc tccttggcag tgaactactt tgaagaagtg       120
gtcaatgggt tgttgctgcc atgagcatgt acaacctttg gagctagaag ctcctcagga       180
aagccagttc tccaagtttt taacctgtgg cactgaaagg aatgttgagt tacctcttca       240
tgttttagac agcaaaccct atccattaaa gtacttgtta                             280
```

<210> 612
<211> 258
<212> DNA
<213> Homo sapiens

<400> 612

ggatgaagtg gcacacactc taactgaaag cagagtatta aagaacacta gacatccctt 60
tttaacatcc ttgaaatatt ccttccagac aaaagaccgt ttgtgttttg tgatggaata 120
tgttaatggg ggcgagctgt ttttccattt gtcgagagag cgggtgttct ctgaggaccg 180
cacacgtttc tatggtgcag aaattgtctc tgccttggac tatctacatt ccggaaagat 240
tgtgtaccgt gatctcaa 258

<210> 613
<211> 140
<212> DNA
<213> Homo sapiens

<400> 613

ttattattat gaaccttcag cctactttct tgagtgccgt aaaagtgctt gtaaattttt 60
ttttttttta agaagaaaga aaaaaatggt gtttgacgtt gatggaaatt caaaaatata 120
tatggaactg aaacattaac 140

<210> 614
<211> 156
<212> DNA
<213> Homo sapiens

<400> 614

aatatacctg atgcgctgta gaatgaaaat gtaaaagata acctgtatgt gttccgagct 60
ttaatttttt gtttacaaat tgaacagtgt tacatgggct gtccagtcct gattatagag 120
aggaagaaat ggtaacagta tggcagataa gaatta 156

<210> 615
<211> 298
<212> DNA
<213> Homo sapiens

<400> 615

agaggcccat gccaacagtc taatctaaga gattagtctt tcaaactcac catccagttg 60
cctgttacag aataactctt cttaactaaa aacctagtca aacaaggaag ctgtaggtga 120
ggagatctgt ataatattct aatttaagta agtttgagtt tagtcactgc aaatttgact 180
gtgactttaa tctaaattac tatgtaaaca aaaagtagat agtttcactt tttaaaaaat 240
ccattactgt tttgcatttc aaaagttgga ttaaagggtt gtaactgact acagcatg 298

<210> 616
<211> 267
<212> DNA
<213> Homo sapiens

<400> 616

aggcattata tatactacac agagtacaat taaaccataa ttgggaatta tattttgttt 60
ttttcttccc aggcaataca cctctgaaca tgtgtgtgat aaatgggttt gctaatgtgc 120
tgttttaaag tataaagcat aatatgtttt ggttaacaca atgtactttt tgaactataa 180
atctttattt taatatggaa atgtttggaa caggagatgc aagccactaa cagagaactt 240
taataattct accctgtatt ttataaa 267

<210> 617
<211> 187
<212> DNA
<213> Homo sapiens

<400> 617

ttcagtttgt cctcataggg aatcaagtat tttagctagg tgatgtcttg caagtacgtt 60
ccactttgtt acaatctact atctgtatat actatttgta tcttaattct tttatgagat 120
gttctgtaac atttttctca ctttgacaaa tgtttttaga ctgtacagtc aagatctggc 180
gcttggg 187

<210> 618
<211> 212
<212> DNA
<213> Homo sapiens

<400> 618

cctgagtcct gggatcagac accccttcac gtgtatcccc acacaaatgc aagctcacca 60
aggtcccctt tcagtcccct tccctacacc ctgaccggcc actgccgcac acccacccag 120
agcacgccac ccgccatggg agtgtgctca ggagtcgcgg gcagcgtgga catctgtccc 180
agagggggca gaatctccaa tagaggactg ag 212

<210> 619
<211> 183
<212> DNA
<213> Homo sapiens

<400> 619

tcacctgagg cgttcaaaag atataaccaa ataaacaagt catccacaat caaaatacaa 60
cattcaatac ttccaggtgt gtcagacttg ggatgggacg ctgatataat agggtagaaa 120
gaagtaacac gaagaagtgg tggaaatgta aaatccaagt catatggcag tgatcaatta 180
tta 183

<210> 620
<211> 48
<212> DNA
<213> Homo sapiens

<400> 620
taggttgtca acaggtacta tttgtcacat aactaacttt cgaggcac 48

<210> 621
<211> 246
<212> DNA
<213> Homo sapiens

<400> 621

```
cagggttcct ttgcctgcta acaagcccac gtggaccagt ttgaatgtct ttcctttaca       60
cctatgtttt taagtagtca aacttcaaga aacaatttaa acaagttttt gttgcatatg      120
tgtttgtgaa cttgtatttg tatttagtag gcttctatat tgcatttaac ttgtttttgt      180
aactcctgat ttttcctttt cggatactat tgatgaataa agaaattaaa gtgatagttt      240
tattgg                                                                 246
```

<210> 622
<211> 158
<212> DNA
<213> Homo sapiens

<400> 622

```
ttttcgtagt ccaaaggctt tattgttctg ctgaaatgct tacaaatact gaaaaccccc       60
agcctgggcc caggcaacca agggctcaat gctgggaagg agagcagggg aggtgggctt      120
agtgttaagg cgtgaagggc gaggccagac agctggag                              158
```

<210> 623
<211> 57
<212> DNA
<213> Homo sapiens

<400> 623

```
atgaggcaga atctggttgg gtatgtttct tatatatgtt tgaagcagat ggctgac          57
```

<210> 624
<211> 229
<212> DNA
<213> Homo sapiens

<400> 624

gatggctggg agataagtcc ttgaatggag gaaccaagag gtgagttaga ggcataaact 60

aaggaatttc agttagtagg gtttaggagt gacagtctag aatgagtgga gactaggaga 120

ttcatcttga tgcaagcata cttagatcca tgttactcag gatagcatag gtgagagagg 180

agctggtaga attttaatgt catacctggg tagtacaact ggttattat 229

<210> 625
<211> 242
<212> DNA
<213> Homo sapiens

<400> 625

actcaaggct gtgaacaaac atacgctgct ttattctttc caatttttct cttgttttct 60

agaactctta tccatatgtt tttaaaataa gtacctaaaa gtggtttgat agtgtcctaa 120

acgacttttt taacttccta aatggaaaga gcataacaat gtagttgatt ggtaagattt 180

acagggattt ggtttctgag tttgaggcac attcccagtg aataagctga gtcccatacc 240

ac 242

<210> 626
<211> 156
<212> DNA
<213> Homo sapiens

<400> 626

aactgatggg aaaggaccaa ttatttatag tttcccaaca aaagttttaa gattttttac 60

ctttgcatca gtgcattttt atttatatca aaaggtgcta aaatgattca atttgcattt 120

tttgatcctg tagtgcctct atagaagtac ccacag 156

<210> 627
<211> 121
<212> DNA
<213> Homo sapiens

<400> 627

atataccata ggctaaaact aaggctttca ctctagaatg caaagctgtt ttgcagctgt 60

tttcccttaa agatgtcctg ttgctttagt gatatttaga cccctctcag ttaagaaatg 120

c 121

<210> 628
<211> 234
<212> DNA
<213> Homo sapiens

<400> 628

atggtaatgt aggatccttt cacagagtgc caggctaaag agctgaactt tgtggtggaa 60

gagacagacc ccctatgtgc tctgtagaca cctgtgatga agtagaactc atgaggatat 120

gaagagaaac atttgtaatt tgagtgatta aactaggaac gaaagaggag gggagaaata 180

ggaagagaga atcaccggcc ctgttgactg atttgagctg ggaatgaaga agaa 234

<210> 629
<211> 116
<212> DNA
<213> Homo sapiens

<400> 629

gtgagtaaat aatgttctag tgcaacagga caaactactc tctccacagg aaacccaacc 60

acaacaggat caatagaaag aaaagagaaa acgttagccc ccaactacaa ataaat 116

<210> 630
<211> 49
<212> DNA
<213> Homo sapiens

<400> 630
tgattactag tgtaaactgg ttattgagat agattatgac attggtgga 49

<210> 631
<211> 87
<212> DNA
<213> Homo sapiens

<400> 631

aaatgacttt caactaacct tgtgaatctt ttgcagtgta ctgtgtgcaa taccaagggc 60

atagctccct gtaatttggg aaataca 87

<210> 632
<211> 239
<212> DNA
<213> Homo sapiens

<400> 632

aagaacaact cctcaccagt tcatcctgag gctgggagga ccgggatgct ggattctgtt 60

ttccgaagtc actgcagcgg atgatggaac tgaatcgata cggtgttttc tgtccctcct 120

actttccttc acaccagaca gcccctcatg tctccaggac aggacaggac tacagacaac 180

tctttcttta aataaattaa gtctttacaa taaaaacaca actgcaaagt accttcata 239

<210> 633
<211> 115
<212> DNA

<213> Homo sapiens

<400> 633

gcatgctcag tcttttcctc ttatctacaa tacaaagggt ttgtctgaaa agtctggttt 60

tttttctttt tacaaatgta ccttagctgc atcaacagga gtaagatgta gaaaa 115

<210> 634
<211> 72
<212> DNA
<213> Homo sapiens

<400> 634

gggagattgg tggcccttgc caggaagtgt cttaacactt tgtggatact gctgcctgtt 60

gtctttaaaa gc 72

<210> 635
<211> 113
<212> DNA
<213> Homo sapiens

<400> 635

gaaaactgtt agatgcacac tgttgatttt catggtggat tcaagaactc cctagtgagg 60

agctgaactt gctcaatcta aggctgattg tcgtgttcct ctttaaattg ttt 113

<210> 636
<211> 41
<212> DNA
<213> Homo sapiens

<400> 636
tcctcatggt ggcagcgctc atagcgaaag cctactgtaa t 41

<210> 637
<211> 275
<212> DNA
<213> Homo sapiens

<400> 637

agcattaata atttccatgc atgtgtcttt ttccagtagg tatggttgaa tttatgtaaa 60

tttattgcta atcccatccc ttacgattta gagtataagc tgcgcaaggg cagaagtttt 120

tatttggttt gttcatggat gtattttaag agctgagaac agggcctgga cacaataagc 180

attcaataaa tatttactga atgaatgaac tcctacctat attcctattt ataatttggc 240

tccactttat cctactttag ctcccattca attca 275

<210> 638

<211> 157
<212> DNA
<213> Homo sapiens

<400> 638

tgtgttttca gagctaggta cagaggaatg tttgctacct ttagcggtga aaaaagaaag 60

agagtcaaga attttgttgg attgtgtttg tgtgtgcata tatttgatat catcattata 120

tttgtaatct ttggacttgt aatcatagcc tgtttat 157

<210> 639
<211> 249
<212> DNA
<213> Homo sapiens

<400> 639

tctccagagg gcacttcggc tgcctttgct tcctttcatt cgaggcccgg ctcttgctga 60

cagaataggt tccgttttgg gcggtggttc tcgagcctgc cattcaaaac caaagcaaat 120

tggagcattt ctcacaacat ggtattgaag ttcctttttg ttctcaaaag ttgtgaccgt 180

gttaaattgt actcccttag tcctgtaagg tatgttaagt gaatcgcagt tacgctgtac 240

ttttattaa 249

<210> 640
<211> 87
<212> DNA
<213> Homo sapiens

<400> 640

gccctgcgcc ggcaaccacc tagtggccca gggaaggccg ataatttaaa cagtctccca 60

ccacctaccc caagagatac tggttgt 87

<210> 641
<211> 128
<212> DNA
<213> Homo sapiens

<400> 641

caggctgggc cgtgagcagg tgggccgttg agttacctct gtgctggatc ccgtgccccc 60

acttgcctac cctctgtcct gccttgttat tgtaagtgcc ttcaatactt tgcattttgg 120

gataataa 128

<210> 642
<211> 274

<212> DNA
<213> Homo sapiens

<400> 642

```
ggttgagttt gtccattgct agggagagac ttccagtaat aaaatttact attctagatg      60
cttctactgt tatgttttat ctgcccattt atctttctta gttaccagga gaaatgtgtg     120
acacctatat tataatgaaa acaatcttat tacttatagt ttatctatat taaacaaatt     180
taattgcatt ttaaagcatt ctttgatatt gttgcttttg caataaatat ggataatctt     240
ggttataagg gagttaaaac aatgctgtaa taaa                                274
```

<210> 643
<211> 247
<212> DNA
<213> Homo sapiens

<400> 643

```
ggcaggcttc tctgtagaac cccaggggct tcggcccaga ccacagcgtc ttgccctgag      60
cctagagcag ggagtcccga acttctgcat tcacagacca cctccacaat tgttataacc     120
aaaggcctcc tgttctgtta tttcacttaa atcaacatgc tattttgttt tcactcactt     180
ctgactttag cctcgtgctg agccgtgtat ccatgcagtc atgttcacgt gctagttacg     240
tttttct                                                              247
```

<210> 644
<211> 263
<212> DNA
<213> Homo sapiens

<400> 644

```
ttgttggtag agacggtgat tcactatgtt ggccaggcta gtcacgaact cctgacctcg      60
tgatccgccc acctcggcct cccaaagtgc tgggattaca ggtgtgagcc accgtgcccg     120
gcctcttttt atttattcct aaaatattac cttgaggcca aattctgcgc ttaaggagaa     180
tgtgcaccaa gtgctggggt gggggctggt tataaacgag gccacaaatc atgcttgtta     240
ataaattgtg tggttcaaat ctg                                            263
```

<210> 645
<211> 108
<212> DNA
<213> Homo sapiens

<400> 645

gaggaggtgg gcctcgttag actgctcaga ttactggaac ctcgttatca catcccattc 60

tacaagtttt tcactgaatg tttcctgaca tctataaatg agggtgcc 108

<210> 646
<211> 97
<212> DNA
<213> Homo sapiens

<400> 646

ggccaaaaag tctacattgc gtgtgtggat ggatgaatga gcagtgggag tgcagcgcca 60

ggtgacaaga tgttgtgagg ggttttgagt catccag 97

<210> 647
<211> 220
<212> DNA
<213> Homo sapiens

<400> 647

tgcccccagt atgtttagga cgcgagcccc agaaggattt gggagtaaac ttaacattca 60

ctgtgttttt gctttgcatc cgccatttgt gtgtgttttt ggactgtggg ctgtgtgtac 120

cttggttggt gactcagtga gaagaagcag gaatgccaaa gatactatga atgttttgag 180

ttttgttgct gttgttgttg agaggttgtt tcactggtat 220

<210> 648
<211> 201
<212> DNA
<213> Homo sapiens

<400> 648

aactctgtaa ggaggaccat gtcagactat tgtaagctaa gcattaggac tgatacaaat 60

aatatatgct cctggcatag aaaaataaac cacagagaac gagttcaaag aatagcaaag 120

aaagaaagag gacccagtgg gcgaaagatg agagtgtact tttaccaaaa gttatctaag 180

cctgagcact tgaagtctgc a 201

<210> 649
<211> 205
<212> DNA
<213> Homo sapiens

<400> 649

aatcccaatt ttcaggagtg gtggtgtcaa taaacgctct gtggccagtg taaaagaaaa 60

tccctcgcag ttgtggacat ttctgttcct gtccagatac catttttcct agtatttctt 120

tgttatgtcc cagaactgat gttttttttt taaggtactg aaaagaaatg aagttgatgt 180

atgtcccaag ttttgatgaa actgt 205

<210> 650
<211> 235
<212> DNA
<213> Homo sapiens

<400> 650

gcagcttcct gataaagcgt gctgtgctgt gcagtaggaa cacatcctat ttattgtgat 60

gttgtggttt tattatttta aactttgttc catacacttg tataaataca tggatatttt 120

tatgtacaga agtatgtttt ttaaccagtt cacttattgt actttggcaa tttaaaagaa 180

aatcagtaaa atattttgct tgtaaaatgc ttaatatcgt gcctaggtta tgtgg 235

<210> 651
<211> 163
<212> DNA
<213> Homo sapiens

<400> 651

ctttcattgg aagttcatca ctgttaggcg ttatcttgag tattataaca aagcaaatcc 60

acaagtattc aatataagat taggaaaaaa attcctgcga tactttgttg tcaaacactt 120

gccactgata gacgttattt tagcttttaa ggcctgtcac att 163

<210> 652
<211> 115
<212> DNA
<213> Homo sapiens

<400> 652

taatattttt tgctgcctag gcaaatggct tttgtgaaaa cacttgtatg aaaagcaata 60

caccatttgt ttttacttac caatcactat cattaggttt tgatgcaaat gggaa 115

<210> 653
<211> 210
<212> DNA
<213> Homo sapiens

<400> 653

gttttgatgc tgtgtgcttt tggctgggcc tcgggctcca ggccctggga ccccttgcca 60

gggagacccc cgaacctttg tgccaggaca cctcctggtc ccctgcacct ttcctgttcg 120

gtttagaccc ccaaactgga gggggcatgg agaaccgtag agcgcaggaa cgggtgggta 180

attttagaga caaaagccaa ttaaagtcca 210

<210> 654
<211> 99
<212> DNA
<213> Homo sapiens

<400> 654

aattcatatc ccctgttcgt ctcatgcgcg tcctccgtcc ccaatctaaa aagcaattga 60

aaaggtctat gcaataaagg cagtcgcttc attcctctc 99

<210> 655
<211> 89
<212> DNA
<213> Homo sapiens

<400> 655

tgtgcccagc ctaagccatt tcttaaaata aaaatgctaa aggactagta agtaaaaata 60

aaacttccta tgggatttcc cagtggaaa 89

<210> 656
<211> 36
<212> DNA
<213> Homo sapiens

<400> 656
ataatgaaag ttagtaacgt ccattattta ataaag 36

<210> 657
<211> 100
<212> DNA
<213> Homo sapiens

<400> 657

ttttctaacc ttgattgacc atgggcaaat gaaactgcag aaagtgaaac tgcggatagg 60

ggggatgact gtattcaata gattccgaca ttatgtctgc 100

<210> 658
<211> 259
<212> DNA
<213> Homo sapiens

<220>

<221> misc_feature
<222> (139)..(139)
<223> n is a, c, g, or t

<400> 658

```
caaaaatgag tcacaactct tgattccatc cacctccaaa atccaggtat tttccttaat        60
tctcattctt tcacaactcc acatatatcc atatcctcat tatctcagga caacgtgacc       120
atttcttgcc acttcccanc acttccatgc ctaccaaaga agcctatctt ctctcaccag       180
gaccactgaa aaagtcttgc aactgatttc ccttgtcctc ttcttgcctc tctacagtca       240
attctctata caacagtca                                                    259
```

<210> 659
<211> 34
<212> DNA
<213> Homo sapiens

<400> 659
tttatgaaca gcagactcta tgtaaaggca tttt 34

<210> 660
<211> 262
<212> DNA
<213> Homo sapiens

<400> 660

```
ccaggttagg aaaggattca gcactacagc atacccctct acaacataca gccctgtcac        60
attgagatca taatccctcc tgtcccactc ctctctacca accccaccct actagctagg       120
tcttcagtgt tttacattga atattggtac attttaatta ttttttctca taaatgggtt       180
atttatagaa attttgttaa ctcttgagcc atatgcatgt gtagatactg gcagggctat       240
gtttgtttat gatgctctgc aa                                                262
```

<210> 661
<211> 174
<212> DNA
<213> Homo sapiens

<400> 661

```
ccatcagttt ttccaatgtg aatggactgg ttcatatcac accatattta gagatacaag        60
gtgattataa ctaacgtgtc tacaagacat actgggtcaa acaatgtgat caatccaaag       120
ggtatctttt taaaaagaat ttaagtactc agctgcaaag ataagttcac taat             174
```

<210> 662
<211> 161
<212> DNA
<213> Homo sapiens

<400> 662

```
attagtgaac ttatctttgc agctgagtac ttaaattctt tttaaaaaga taccctttgg      60
attgatcaca ttgtttgacc cagtatgttt tgtagacacg ttagttataa tcaccttgta     120
tctctaaata tggtgtgata tgaaccagtc cattcacatt g                         161
```

<210> 663
<211> 248
<212> DNA
<213> Homo sapiens

<400> 663

```
atgccctgag gccagttggc gaggggtggc tcctgagggt ttttataccc tttgtttgct      60
aatgtttaat tttgcatcat aatttctaca ttgtccctga gtgtcagaac tataatttat     120
tccatttctc tctgtgtctg tgccaagaaa cgcaggctct gggcctgccc cttgcccagg     180
aggccttgcc agcctgtgtg cttgtgggaa caccttgtac ctgagcttac aggtaccaat     240
aaagaggc                                                              248
```

<210> 664
<211> 188
<212> DNA
<213> Homo sapiens

<400> 664

```
tctgtgtgtg tcctagcatg gagagttcct tccttttccc ttcagttagg ttgacccttt      60
ccatttgttt agtatccgtg cacatgtcgt actagacccc aatcaagttg cttatttaaa     120
attctttcag ctgtttccct attatttcct tactttgctg aacatgtccg ctgttttacc     180
tcactgct                                                              188
```

<210> 665
<211> 289
<212> DNA
<213> Homo sapiens

<400> 665

```
tatacagaat agtcactggt cttgggctaa atggtgactt caagtgtagt ggctgcatag      60
tcaaaaatga attagatgag tacaaaagtg acgaaatgaa agaatgtcaa gaatggacca     120
caaagacagt gttttatgac ctaaagatta agatttatcc atttgtgtac aattgtggac     180
tatataaaat aaaacaagac tttgacctca gtggataaga agtatttgga tgtactaatc     240
aatattttg gtctgggtca gtggtgggtt catctgtgtt tgttgtatt                 289
```

<210> 666
<211> 230
<212> DNA
<213> Homo sapiens

<400> 666

```
aagccgaggg gcccgagact ctgcagcggg gccagaaaga gaagagtggg ggaggaggcc       60
gggagtggtg catggaccag ggggtagagg gaggtgggtg tggacctggg gtcgggcgcc      120
agtcagcttg cagcctatga aggacggaag ggagggctac agagataggg gaagagtggg      180
gctgaggata gccagagcgg cttggcacac agttttaggg taaaagcatc                 230
```

<210> 667
<211> 238
<212> DNA
<213> Homo sapiens

<400> 667

```
agttggtgcc ctgattccat ttttctcaag ttgtagaaga aacaaactaa tttacggtag       60
gagaaattca aattcagatt ctccccatcc ccaccagtta cctttggttg gtggagaggg      120
ggagaattgg caggaaaggg gcacaaagaa actttttggg gtgatggaaa tattttgtac      180
cttgctttag atgttggtca gatggaatat acgtttgtgg aaacctgcca aactgtac        238
```

<210> 668
<211> 188
<212> DNA
<213> Homo sapiens

<400> 668

```
tattcctggt agaggaattt ctgtatttga aaattctcca gaaggaataa tataaactgt       60
ggactttggg tgataatgat atgtaggttc gtcagttgtt aacaaatgta tccctctgtt      120
gggggctatt gataatgggg aaggctgtgc atgtgtggga gtaggaggtg tatgggacat      180
ctctgtac                                                               188
```

<210> 669
<211> 189
<212> DNA
<213> Homo sapiens

<400> 669

```
tcaagttaat ggcagctaaa acgctcccag tccatttatt ggccacatga ggtggtcgtc       60
aagaaacaag ttagaaggtt atgacaggaa gtagtataat aaatgcccgg cagtacgagg      120
ggttcaacag aagtgaacaa ggcacaagaa agaggtctgt gttcaggaaa caggccagtc      180
```

cccacatgg 189

<210> 670
<211> 298
<212> DNA
<213> Homo sapiens

<400> 670

agcaggagta agtttctcat cccatgggcg accagggcca tctcctccca ccagtggccc 60

ccactcacag ggagctggca atgccctacc tgcctgttct ccagatggag aaacaggctc 120

tgagatttca caggtcttgc ccaaagtcat tgattttgat gattaaaaag aataaacaca 180

gtgtttcctg agtagcagtg attgttatgc cttgctattt taataaagat tctattttcg 240

tataacattg tcaagtggaa acatgctgaa atctattaaa ccatctttgt ttgtggaa 298

<210> 671
<211> 269
<212> DNA
<213> Homo sapiens

<400> 671

cttaggatat gaaatcttca gaggatggta gaagagaaag gtaaatgcca taagaaagta 60

gagtacttgt ggtcattgaa accatggaat ttactcaggg atagtgtata tagtgagaaa 120

tcaggtaact aagatttgag cctaaacgta atctgtaaaa ggggagctca aaggaaaagg 180

gaatgggagg accggatttt gtaaggatag tagggcaaat gttaagagag agaatgagag 240

agttgagtat ggcaaagagt gactcaatt 269

<210> 672
<211> 277
<212> DNA
<213> Homo sapiens

<400> 672

tggaggattc aagatgagca ggtatgtttt ctgctttcaa taactcattt tctgctgcag 60

agatagcctt gtaagcaagc aaaggaaaac tttgacattt ctctgcaaag ataatgcatt 120

acatataagg gtgtgtctgg gagggtacca ggtgcctgtc agcaaaagtt gcaaaaacag 180

cttgataagg gtattaagtg ggcctgttgg gaaaggcagg agtgtcaaat gtcggacaga 240

actccagaca gagaaatcca gatatccagt aggttag 277

<210> 673
<211> 246
<212> DNA
<213> Homo sapiens

<400> 673

tgaactaaaa ctatgagcct tattcaatat ctataattct atgatttttt taaattatgg 60

gaaattaatg aaagatgttt acatgaataa tgtttgccct tactgtgtta tgaatgagtt 120

ttttgtagtg tgtctgggtg catgatgcaa gagagtagga aaaatgtttt tgaaacaaaa 180

cttgacaaat atttgtaatg aaagtaaatt taaagattgc tataattgcg ctatagaaac 240

aatgca 246

<210> 674
<211> 236
<212> DNA
<213> Homo sapiens

<400> 674

attggagtgg atgggttctg ccttaaattg ggaggactcc aagccgggaa ggaaaattcc 60

cttttccaac ctgtatcaat ttttacaact tttttcctga aagcagttta gtccatactt 120

tgcactgaca tactttttcc ttctgtgcta aggtaaggta tccaccctcg atgcaatcca 180

ccttgtgttt tcttagggtg gaatgtgatg ttcagcagca aacttgcaac agactg 236

<210> 675
<211> 177
<212> DNA
<213> Homo sapiens

<400> 675

gataacagtc ttgcatgttt atcatgttac aatttaatat tccatcctgc ccaacccttc 60

ctttcccatc ctcaaaaaag ggccatttta tgatgcattg cacaccctct ggggaaattg 120

atctttaaat tttgagacag tataaggaaa atctggttgg tgtcttacaa gtgagct 177

<210> 676
<211> 213
<212> DNA
<213> Homo sapiens

<400> 676

tattttccgg ttgaccccag aattcattag atttttttaa aaaacaattt caaaatagtt 60

gctgttttaa attagttgca tccagttcat atcaatgttt gcatgctttt tagtctttgt 120

tatttattga aaacctttgg tacctaaact taagtttgat tgtttcagtg tgtacttggt 180

aaatatgtca gtggcctttt aactaaacat caa 213

<210> 677
<211> 235

<212> DNA
<213> Homo sapiens

<400> 677

ttctacacta gtgccatggg aaccaggtct gaaaaagtag agagaagtga aagtagagtc 60

tgggaagtag ctgcctataa ctgagactag acggaaaagg aatactcgtg tattttaaga 120

tatgaatgtg actcaagact cgaggccgat acgaggctgt gattctgcct ttggatggat 180

gttgctgtac acagatgcta cagacttgta ctaacacacc gtaatttggc atttg 235

<210> 678
<211> 52
<212> DNA
<213> Homo sapiens

<400> 678
agtgcaacgt attcaagtcc tcaatatcct gatcataata ccatgctata gg 52

<210> 679
<211> 218
<212> DNA
<213> Homo sapiens

<400> 679

gtacatggtc tggagtaacc tttatatgaa gtggctgccc aggtgtgtgg cttaggacta 60

gatctccagg ttgcacaaag ttggcattgg gtttagtttg catttttcca ttctgaagat 120

ggccctcctt ggatttcatc caggaaatcc atagctttct gttaacagga catggagtag 180

actggctgca tttgaaggac agcacagatc cctcatca 218

<210> 680
<211> 131
<212> DNA
<213> Homo sapiens

<400> 680

gtaatttgtg attgtcgagg aagaggtgtg gctgttggtg tgatagtaat actgctggtg 60

actttattgg ttgttttgtt tagtgccccg ttaattaagc cttgagttcg gttatcctgc 120

agtggtgctg a 131

<210> 681
<211> 120
<212> DNA
<213> Homo sapiens

<400> 681

tacaatgatg gttgagtgaa aatacagaag gggggtttga gtattcagat ttcataaaac 60

acttccttgg aatatagctg cattaacttg gaaagaagcc tgttgggcca gaagacagaa 120

<210> 682
<211> 179
<212> DNA
<213> Homo sapiens

<400> 682

tcatggaatg ctacatgctt tctgtttttt tcattttgga tttctccaaa actaactgaa 60

tttaagcttc aggtcccttt gtatgcagta gaaaggaatt attaaaaaca ccaccaaaga 120

aaataaatat atcctacttg aaatttactt tatggactta cccactgcta gaataaatg 179

<210> 683
<211> 294
<212> DNA
<213> Homo sapiens

<400> 683

tgggaagcgg cgagatcctc gggctggggg tgcccacgtt tgctacctcc ccctgtgaaa 60

tcgctggtgc tcacaattgt ctttcacagt gtatgtgatt tttttaagga aaaaaaaaaa 120

atccctattt aagattttga aggtgctacc attattttgc cacagacttt gaagaaactt 180

ttggatgtgg ggcatcatcc gcatctttct ctctcctcca aatgacaaag tttggggaat 240

ttttgaattt tcctagcatc gcccttgtgc tcatcaggta atctgctaag gagg 294

<210> 684
<211> 277
<212> DNA
<213> Homo sapiens

<400> 684

taccccettt tatatctaat gtagaaaaag cgaaattgaa tctggaaagc aaactgttgt 60

atatagttgc ggtaacaatc atgaagagag agccgggctg tcccctcagt aattcatttt 120

aaataacaaa ttatttaaaa ataaaattca tgccagagcc agctgaagag gccttccttc 180

atcaccactg aggccacccc caatctgggc cctctgtcca tctggcatgt ctcctcccag 240

caagattcat ctgttcaatg ccatttgcgt ttcaata 277

<210> 685
<211> 235
<212> DNA
<213> Homo sapiens

<220>
<221> misc_feature

<222> (157)..(157)
<223> n is a, c, g, or t

<400> 685

aggatttccc aaaccagccg aggccccagc acccgccgtc tccccagaag ccccctcctc 60

cttcccccat gggtcatatg ttgaaagtct attttaaaaa ctatgttcct tgccgtagat 120

tgcagagcta atttatcacg tttctctcct gtgaganccc cccttttata tgatatatcc 180

agaggaagtt ttgtaatata aaacaggacg cccacactga tggttttgca ctggt 235

<210> 686
<211> 193
<212> DNA
<213> Homo sapiens

<400> 686

agagggaact gtgcagtccc caggccgccc cggctccggg ctagaggcaa taaataaacc 60

cgatcctgcc gggcacagcc gcgcccgcgc ctccggcgcc gtccccgggc tgacgggga 120

gggagcggag aagcgagcgc agattctgcg tataaatcag ctctggagca gacacagccc 180

ggctgtgaaa agc 193

<210> 687
<211> 178
<212> DNA
<213> Homo sapiens

<220>
<221> misc_feature
<222> (84)..(84)
<223> n is a, c, g, or t

<220>
<221> misc_feature
<222> (89)..(89)
<223> n is a, c, g, or t

<220>
<221> misc_feature
<222> (123)..(123)
<223> n is a, c, g, or t

<400> 687

gagacaagga cagacaaatt ttctggctgt ccccatttct cctgggggag gggtttgggg 60

ctggtttgac tttaattggt gggngggtng tttctgccgc tctgtttgct gcagtccccg 120

tgncctgctt ggggactgag aaatttgagc caggtatcca gagccacagc ccatcttg 178

<210> 688

<211> 252
<212> DNA
<213> Homo sapiens

<400> 688

```
agttcggcaa aggtgtccta gaaatggcca gagtttttga cagacagagt gtagtctcct     60
tattagaaga aaggaaaaaa aagcagaggc caaagaagtt ttgtgtttgc tgatgagagc    120
cccactcatt tgcgaaacgc acgtaaaaca aagtgaaccg tgactgttaa actagggatg    180
ggaaattttg catcttgggg ggctgtacat ttatttattt agttgaagat tcactgatcc    240
cactttgaaa ta                                                         252
```

<210> 689
<211> 174
<212> DNA
<213> Homo sapiens

<400> 689

```
caccccagga ctttatcaac ttgttcaagt tctgaatccc agcacatgac aacacttcag     60
aagggtcccc ctgctgactg gagagctggg aatatggcat ttggacactt catttgtaaa    120
tagtgtacat tttaaacatt ggctcgaaac ttcagagata agtcatggag agga          174
```

<210> 690
<211> 293
<212> DNA
<213> Homo sapiens

<400> 690

```
ttgcttttca ttcagatgta tacataaact tatttaaaat gtcatttaag tgaaccattc     60
caaggcataa taaaacccga ggtagcaaat gaaaattaaa gcatttattt tggtagttct    120
tcaataatga tgcgagaaac tgaattccat ccagtagaag catctccttt tgggtaatct    180
gaacaagtgc caacccagat agcaacatcc actaatccag caccaattcc ttcacaaagt    240
ccttccacag aagaagtgcg atgaatatta attgttgaat tcatttcagg gct           293
```

<210> 691
<211> 168
<212> DNA
<213> Homo sapiens

<400> 691

```
catttcatgt ttggcgggca tgtgagtgca caagatggaa agagcgattg gagcatcctg        60
gtataattac ccccattgtg cttttaatgg aaatttcaaa ggacgggagt attttgttgg       120
ttggtgtcca ggtttgtggc actgttccaa gaggccttac acacacac                    168
```

<210> 692
<211> 243
<212> DNA
<213> Homo sapiens

<400> 692

```
acaaaagagc cagagttctg gacccatgtt tggagcattt gtagccttat ttttttgcgt        60
gtgaatcttt taccctgaaa aaaagccata atgaattaag ccagactgac cacttgcttg       120
gagtgtgtgc ttgaaaaaac cagagcaata ctgttgggta ttgtatcagg cttcagtaca       180
aactggtaac accaatgtgg atcctgacag ctttcagttt tagcaaaaat acacgtgaaa       240
tct                                                                     243
```

<210> 693
<211> 207
<212> DNA
<213> Homo sapiens

<400> 693

```
gagtatgagt atgcctgagt gtgaatgtgt atgagtgtga atgagtgtgt gcacatgagt        60
gcacgggtgt cagcatgtgt atataagtgt gggcatgtgt atgtgattgt gtgagtgtgg       120
gcaggtgagt gtgttgggga tgtgggttag ggtggggagt ggtgctttct ctagtgtgtc       180
ctccggaaca tcttgcctac ctagcaa                                           207
```

<210> 694
<211> 105
<212> DNA
<213> Homo sapiens

<400> 694

```
gtatttattg agtcacggat tattgtgcat caagcaattg ttaatatgac ctggtcctat        60
ggggtagaac ttaggaaaaa taaagttggt tcttattcaa tattt                       105
```

<210> 695
<211> 231
<212> DNA
<213> Homo sapiens

<220>
<221> misc_feature

<222> (92)..(92)
<223> n is a, c, g, or t

<220>
<221> misc_feature
<222> (105)..(105)
<223> n is a, c, g, or t

<220>
<221> misc_feature
<222> (160)..(160)
<223> n is a, c, g, or t

<220>
<221> misc_feature
<222> (169)..(170)
<223> n is a, c, g, or t

<220>
<221> misc_feature
<222> (172)..(176)
<223> n is a, c, g, or t

<220>
<221> misc_feature
<222> (178)..(180)
<223> n is a, c, g, or t

<220>
<221> misc_feature
<222> (182)..(182)
<223> n is a, c, g, or t

<220>
<221> misc_feature
<222> (186)..(187)
<223> n is a, c, g, or t

<400> 695

gaatggcaaa acacaaatgg caacataaat gtccatttga cttacctaac ttcacaactt 60

tcaagttgag gatgtcattt attcttgaat tntgtttttt tactnagatg ctttcaatta 120

atagccctat atttttgtgc aggcgaactg tataacaggn ataaaaaann annnnnannn 180

antgannagg aggagaaatt ctcacagaac accatatgag ctttagacca a 231

<210> 696
<211> 208
<212> DNA
<213> Homo sapiens

<400> 696

ctctgagttt tatatgctgg aatccaatgc agagttggtt tgggactgtg atcaagacac 60

cttttattaa taaagaagag acacaggtgt agatatgtat atacaaaaag atgtacggtc 120

tggccaaacc accttcccag cctttatgca aaaaaagggg agaatcaaag ctttcatttc 180

agaaatgttg cgtggaaaag tatctgta 208

<210> 697
<211> 35
<212> DNA
<213> Homo sapiens

<400> 697
ttggatagca gctatcttgt tggatgtgag gtgga 35

<210> 698
<211> 237
<212> DNA
<213> Homo sapiens

<400> 698

cagacacatg agcaggactt tggggagtgt gttttatatc tgtcagatgc ctagaacagc 60

acctgaaata tgggactcaa tcattttagt ccccttcttt ctataagtgt gtgtgtgcgg 120

atatgtgtgc tagatgttct tgctgtgtta ggaggtgata aacatttgtc catgttatat 180

aggtggaaag ggtcagacta ctaaattgtg aagacatcat ctgtctgcat ttattga 237

<210> 699
<211> 151
<212> DNA
<213> Homo sapiens

<400> 699

gcagaaatat gtaaccttag actcagccag tttcctctgc agctgctaaa actacatgtg 60

gccagctcca ttcttccaca ctgcgtacta catttcctgc ctttttcttt cagtgttttt 120

ctaagactaa ataaatagca aactttcacc t 151

<210> 700
<211> 91
<212> DNA
<213> Homo sapiens
<400> 700

tgtacagggt cattttgaca gtaactggta tattcctttg cattttatgt tgcattgcca 60

atttttagtg tatccagttt gaaagtataa t 91

<210> 701
<211> 269
<212> DNA

<213> Homo sapiens

<400> 701

```
agacctagaa ggttgtagat gggaaatcag gaatgatttg aactgataaa gatttcagac      60
tcataagaac acattttata aatgttaaac acaaaaacta catgactgaa gatagaagag     120
aatgcgatgg attttattac acatggtgga agagagaaga ggcgtgtagg tttgcaaaca     180
aagttaagaa ataggaaact gaatttttca ttgtacagaa aatgtatctc ttggggaggg     240
cctgtgtacc cccattctct gattataaa                                       269
```

<210> 702
<211> 223
<212> DNA
<213> Homo sapiens

<400> 702

```
tacattgtaa gagcaacatc ctacgttaat aaatgttcta gcccggtgct tcgcgaacat      60
ttatgtgcat acaaatcacc tgggatcttg ttagaaggca gtaggtctgg ggtggggcct     120
gagattctgc atttctaacc aggtcctggg agatgctgat gctatcgagc cacaaccaca     180
ctttgagtag caagcctctg gcctatcctt attgtttgtt ata                       223
```

<210> 703
<211> 258
<212> DNA
<213> Homo sapiens

<400> 703

```
cattctttcc tccacaggat tgctttgtcc atctcctgct ttcatttcaa gtgcataaac      60
aaaacctcaa agggcctggg aaggtgaggc aggccagagt ctgtgttctg tgttgagtgt     120
caagctattt gttaagaagg tttgcaacag gcctttggtg tgggctttgc cagagactgt     180
tttgaacact ttgcttgaga tccgtgccct gtaaaatgga tatgatgttt tactgatgtc     240
tgtaatacat ttgtaaac                                                   258
```

<210> 704
<211> 85
<212> DNA
<213> Homo sapiens

<400> 704

```
ttaagcgcat tagaaaacaa ttgtttgtaa tggaatcaaa gtgtttccct ggacagtttg      60
atgtgcttat ggttgagatt tataa                                            85
```

<210> 705
<211> 33
<212> DNA
<213> Homo sapiens

<400> 705
gtgagcccat atcgtactgc tgcagtccag cct 33

<210> 706
<211> 135
<212> DNA
<213> Homo sapiens

<400> 706

gatatcagat cagttgagac taacagttga aagcagtaaa catattacgg acatattatt 60

gataaaagac atttatgaag aggataaact gtgaaggtgt acagacacta aaccatagtt 120

gctaaacaca tacaa 135

<210> 707
<211> 208
<212> DNA
<213> Homo sapiens

<400> 707

gaggccccag aggacttatc tcagctgtac atgctctgcc tgtggagaca tggcttttct 60

ttgtgctgtg gcagactggg gctttggaag tggtgtatgt ttaacttacc tgagagtgag 120

agatgtgtag gaagaatagc tggaagaaag tgaaagatga gtgccagtac ttttggcctg 180

ttatccagta gagagaaagt gacagtga 208

<210> 708
<211> 103
<212> DNA
<213> Homo sapiens

<400> 708

attaaagtac tcaagttagt tgttttgcag agatgttgcc ttcagatgtt aatcaggtct 60

ctcaagtttc atggagtcta tgctgatcct ttaattgaca aat 103

<210> 709
<211> 223
<212> DNA
<213> Homo sapiens

<400> 709

gcatttgaat tgactaggct tttcctatat aaaaaactca aaacttgtta actctgtact 60

ttaataaaat ttaaaattaa aactgtgttg tttttttctc ttctgctaga tacatatata 120

attaaagtac tcaagttagt tgttttgcag agatgttgcc ttcagatgtt aatcaggtct 180

ctcaagtttc atggagtcta tgctgatcct ttaattgaca aat 223

<210> 710
<211> 281
<212> DNA
<213> Homo sapiens

<400> 710

gggcccctgg tatttatagg gtccaagagg aggcacctgc ttttcaactg caccctcagt 60

gctgcctctt cacggcccct aaacgtttcc ctttgaggtt gtgatgctgg gaatcacaga 120

cttcactctc tgcctgcacc cttccccgag gtctcatctt ttctgggtcc cacatctttg 180

taataatgtg aaaaagcaca atttgtctga tcacccccca ggtggttccc caccttatta 240

tcactacctg atccgagtta ctgcaataag tacggtgtcg c 281

<210> 711
<211> 130
<212> DNA
<213> Homo sapiens

<400> 711

gggacaaggt cccttggtgc tgatggcctg aaggggcctg agctgtgggc agatgcagtt 60

ttctgtgggc ttggggaacc tctcacgttg ctgtgtcctg gtgagcagcc cgaccaataa 120

acctgctttt 130

<210> 712
<211> 55
<212> DNA
<213> Homo sapiens

<400> 712
cagatcgaca cgcagctagc ctcctgcatt gtatggttat aaatagcacc ctagt 55

<210> 713
<211> 168
<212> DNA
<213> Homo sapiens

<400> 713

gtttgagcta tcgaagagta agtaaccaga aatttgagaa cagcctgggc agcatagtga 60

gaccccatct ttacaaaaac ttcacagatt agccaggtat ggtggcattt tcctgtagtc 120

ccagctactc agaatgctga ggcaggagag tggcttgtga ccaagagt 168

<210> 714
<211> 255
<212> DNA
<213> Homo sapiens

<400> 714

tggagatagc tcttaaagat ataaatgttt atggctgaaa tgttatggca tcttggattt 60

gctttaaaat aacccagctt gctgcaggag gtgggtattg tgtgtgtggg aaggtgggga 120

ggctgcggga ggaagagatg acccaagatt aggcagatgt tgttaactgt ggaagcaggg 180

tggtgagtgg gggctcatga cattatgctc tctactttgt gtacgtgtga acatttccgt 240

aataaaagat gcctt 255

<210> 715
<211> 83
<212> DNA
<213> Homo sapiens

<400> 715

taaccttagg aaaccagaat agcgtttggc agacacgacg ttttcagttt acctttgaca 60

cctgccccac tccattttgc ttt 83

<210> 716
<211> 52
<212> DNA
<213> Homo sapiens

<400> 716
gaggccggtg ccagtgcagg tccttggtgt gctgtgtgcc ggtcccctgg gc 52

<210> 717
<211> 238
<212> DNA
<213> Homo sapiens

<400> 717

agaattccag ataaacacac agcctttccc ataccttttt ttttcttact ataaaatatt 60

ataagatcca ttgatgtcca aataatacca ccgagcatct cttcacctct cctcctcttg 120

gtccacttgc taatgcccag ttttcttctc catttccact ttttcttagg ctccctattt 180

actattcatt ttgacttcct tctgttttat ttttttccct ttagcattgc atgtgaat 238

<210> 718
<211> 220
<212> DNA
<213> Homo sapiens

<400> 718

tctggtagat aaagccctag accttgtcca cactctcacc cccatcccca actttccctg 60

gaccggtgcc gcccccactt gatgcttgct caaggccggg gactggagcg ggctacttgt 120

atatttcgtt gtcagtctgc agaatgtgtt tgatttttat ttttccctcc ttctctgaca 180

tgtgtcaagg aataaagact ggatacaggt ccattacgtc 220

<210> 719
<211> 234
<212> DNA
<213> Homo sapiens

<400> 719

ctgtggcatg ctcagaggtt cctgctggat tccagctgga gcggtgtgat acccttcttt 60

ttcagctgtt cgtgccttcc tttcttgtat ccaccaaagt ggagacaaat acatgatttc 120

aaagatacac agtacctact taattccagc tgatgggaga ccaaagaatt tgcaagtgga 180

tggtttggta tcactgtaaa taaaaagagg gcctgggaat tcttgcgatt ccat 234

<210> 720
<211> 225
<212> DNA
<213> Homo sapiens

<400> 720

gagtaccgcg cagacattaa aagtcatgta aaagaacatt tgactgaaag aaaaatgctc 60

cttgaatatt aaaaggttgt aaaaatagtg catgttatgt gatttcaatt ttgtttttta 120

aaatatgggt gtatgcttgt atacgtagag cagataaaaa agacggaagg catactaaaa 180

aatgttgagt ggttatcttt gtatggtgga acaaagtcac tgtaa 225

<210> 721
<211> 173
<212> DNA
<213> Homo sapiens

<400> 721

aaatgctcct tgaatattaa aaggttgtaa aaatagtgca tgttatgtga tttcaatttt 60

gtttttttaaa atatgggtgt atgcttgtat acgtagagca gataaaaaag acggaaggca 120

tactaaaaaa tgttgagtgg ttatctttgt atggtggaac aaagtcactg taa 173

<210> 722
<211> 266
<212> DNA
<213> Homo sapiens

<400> 722

```
aaatgtttga cattcattat cagaatgagg gaaaatttaa acaattccgt tttttctttt        60
gctagtaggc atattatgct aataaaatta ctaaattaaa agtgtgtcaa ggatttgaca       120
aactgccatt tttctccaga agtcaagccc ctaagtgatt gtctagaggc aagaattttt       180
tgatatgttg tctcaacaat gcttctcact tcgtcttcag gtgccccaac ccgcaagtac       240
acatactatg tactcacttg aaaatg                                            266
```

<210> 723
<211> 151
<212> DNA
<213> Homo sapiens

<400> 723

```
gtgtgtcaag gatttgacaa actgccattt ttctccagaa gtcaagcccc taagtgattg        60
tctagaggca agaatttttt gatatgttgt ctcaacaatg cttctcactt cgtcttcagg       120
tgccccaacc cgcaagtaca catactatgt a                                      151
```

<210> 724
<211> 166
<212> DNA
<213> Homo sapiens

<220>
<221> misc_feature
<222> (101)..(101)
<223> n is a, c, g, or t

<400> 724

```
agagaggttt gtcccatata tcttgttcca gcagccatat atcttgtggt ctacagcctg        60
aagcatgatt tcccttgaag tcttggggtt gtttaaagga nagtcccttc aatataaaac       120
ctctgaaata ttagtgagaa tggctcacta atgtgaacaa tgttta                      166
```

<210> 725
<211> 141
<212> DNA
<213> Homo sapiens

<400> 725

atttttgcct ttccgtttct gtctatgatg taggcttctg aggagaacca agaagcttgg 60

ctttagtggt agaatgacag aacttaggga tcccttgcag gctagaacaa agttctgacc 120

cttagaccaa atctttatgt t 141

<210> 726
<211> 299
<212> DNA
<213> Homo sapiens

<220>
<221> misc_feature
<222> (127)..(127)
<223> n is a, c, g, or t

<220>
<221> misc_feature
<222> (194)..(194)
<223> n is a, c, g, or t

<400> 726

atttgcaaca gcctagtgga tttggtaggg tcctgaatca tctctataag gcaaacaagg 60

aaattgtaac acaaagaaat aaacatattg aatataattg ctattctgta agacatacag 120

tctgtgnaag atgtatctta tttacagaga catttttgaa aattaaaata ttaaatactt 180

tttgttatat aganacaatg atctggaagt ataaaaagaa aaatattatc ttgttgatgt 240

aaatatgata tccttatata tattagaatc caataagata tcatgggcgc aatattagc 299

<210> 727
<211> 139
<212> DNA
<213> Homo sapiens

<400> 727

aaaaagagag aatatgtcct gtgttagctt gcttaggaaa taaagagaga gagagagagg 60

gagggaggga aagagagaaa gagagagaga gaaagagagt gagagaaaga gagagcaaga 120

gagcaagagt aagaaagaa 139

<210> 728
<211> 172
<212> DNA
<213> Homo sapiens

<400> 728

aacacccata atcaatcaca gagataacca ctgttcataa ttccttccag tcttcttact 60

tggcacatat acatttgtct ttctttatat atgacatatg gatattttac aaagttagga 120

tcctactcta tgcactgctt ggtgatcgga tctattcaat gtacaaaata tt 172

<210> 729
<211> 205
<212> DNA
<213> Homo sapiens

<400> 729

cctttctaag gatgagggaa tccacaacag actttctcta gaaaacacta atgatggaca 60

actttttggt gtcatcaatg agttggctac taccttgatg taaaaatttg taaggaaaat 120

tttcaccatt tcgagtgtca agtgtatttt taactgtctg gtttgtactt ttatgacttt 180

tgtactacca aagcggagtt aaaaa 205

<210> 730
<211> 98
<212> DNA
<213> Homo sapiens

<400> 730

tggatcgatg atgagtcccc ccaaaaaaac attccttgga aaagctgaac aaaatgagtg 60

aaaactcata ccgtcgttct cagcggaact gaggtcca 98

<210> 731
<211> 97
<212> DNA
<213> Homo sapiens

<400> 731

tggatcgatg atgagtcctc caaaaaaaca ttccttggaa aagctgaaca aaatgagtga 60

gaactcatac cgtcgttctc atcggaactg aggtcca 97

<210> 732
<211> 94
<212> DNA
<213> Homo sapiens

<400> 732

tggatcgatg atgacctcaa tacatgcatt ccttggaaag ctgaacaaaa tgagtgaaaa 60

ctctataccg tcgtcctcgt caaactgagg tcca 94

<210> 733
<211> 194
<212> DNA
<213> Homo sapiens

<400> 733

```
tcaaactcag gtatgtgaca ctctacagtt caatgctagc acacctgtgt gaggcttaac      60
aacatgagga actgatagcc agtgatacac aaatccagca cttcctctcc atttactctg     120
tcaggctgta tatggggagc aacacatatg gctttgtggc agccagaaag tgaaggtctt     180
tttaggaggt gaca                                                       194
```

<210> 734
<211> 290
<212> DNA
<213> Homo sapiens

<400> 734

```
ttgacagtct gccttactat gaaattctag gtacaaaaat tttccttaag ctctgaagat      60
gttgatccat tgacttctgg cattcagtgt tgctgatgac aaatctgtta gcagtctatt     120
tctcatccat ttttgtgttg agctaatcgt gatgacctca tttgttttct ccctggccac     180
tttaatatct tccttctatc cttaatattc caaaatttta caatattgtg tctagatgta     240
gattgtattg ggccctctca atctggagac ttagcttgct ctgttcttca                290
```

<210> 735
<211> 258
<212> DNA
<213> Homo sapiens

<400> 735

```
gatcgtctgg taactttcta actttaaata atatgtttga gcaataattt cttgacttac      60
tgactttaca acatctttaa taattcccca ttacaaaaga taaggattta acttacacta     120
tcgccacttt cctttgtcca tctctctcca aatgtctgat agttacatca ctttttaata     180
catctattgg tttgatttta tagctttgaa caatacacta atcctctagt tcttgttcca     240
ttaactgaag atcttttc                                                   258
```

<210> 736
<211> 269
<212> DNA
<213> Homo sapiens

<400> 736

gcctggcctg attcagggcc ttgtggcccc cagcttctgt ttcaagctgg gcagacccca 60

ggatcccttc cctccctaag gactcagctg aggggcccct ctgccccctt ctacctccac 120

ctcagcaccc tcccccagct tgatgtttgg gtctccccag caccctcctc cctggccggt 180

gcaaagtaca gggaggtaaa gcaggaccct tgcagacatg ttgcccagca cacagtaggc 240

cctcaataaa agccatttgc actttaaat 269

<210> 737
<211> 264
<212> DNA
<213> Homo sapiens

<400> 737

tttcttcttt atgtccaatt ttgtgggtgg gaggaggatt tagtctcttc ctgatttcga 60

agagctcatt tactatttcg ggaaataaga tttggattgt caaccattat agctattttt 120

tacacacttt tcaactttgt ttttgttata agaatgtgta tgattgttac atgtccaagt 180

ataaccatgt tcgcttttat ggcttttgag tttcatgtca tttttggaaa gatatatata 240

ttgagtccat aaaacccttc acct 264

<210> 738
<211> 46
<212> DNA
<213> Homo sapiens

<400> 738
gacaggggtt ctaattactg tctgtgagag ttactacttt gtaact 46

<210> 739
<211> 129
<212> DNA
<213> Homo sapiens

<400> 739

cgtccccaac atgcatctca ctctgggtgt cttggtcttt tattttttgt aagtgtcatt 60

tgtataactc taaacgccca tgatagtagc ttcaaactgg aaatagcgaa ataaaataac 120

tcagtctgc 129

<210> 740
<211> 237
<212> DNA
<213> Homo sapiens

<400> 740

ttattttatt tcgctatttc cagtttgaag ctactatcat gggcgtttag agttatacaa 60

atgacactta caaaaaataa aagaccaaga cacccagagt gagatgcatg ttggggacgg 120

gggaggctgg cagcaggggg gccccggcgg ctcaccccag ggctcccgga gggggctgtt 180

tccatccacc acccaaaaaa acaccacaag ggtcagtcct agcccacccg acagctt 237

<210> 741
<211> 92
<212> DNA
<213> Homo sapiens

<400> 741

cctgcctggg aaagcgagtc cacagttcac taacaaacac aataccatcc acaaacaagt 60

agccacaaag accacagtta gcaaacacac ac 92

<210> 742
<211> 278
<212> DNA
<213> Homo sapiens

<220>
<221> misc_feature
<222> (214)..(214)
<223> n is a, c, g, or t

<400> 742

gctggtctcg ggcaacaagc agcctcctag gagcagaagg tgatggaggg ccacgggggc 60

agggaggagc agatggccat gtggctcagc ccctgcctgg gaaagcgagt ccacagttca 120

ctaacaaaca caataccatc cacaaacaag tagccacaaa gaccacagtt agcaaacaca 180

cacagtcaca cacacacaca cacacacaca catnacggga ggtgggcagg accgcagtct 240

gcagtgggga ggcaagtgtt agttgcatca tcaggtgg 278

<210> 743
<211> 32
<212> DNA
<213> Homo sapiens

<400> 743
atggacaatt ctgtacccca ataatcagaa ca 32

<210> 744
<211> 164
<212> DNA
<213> Homo sapiens

<400> 744

caggggggcc tgagagtaac agtgaggatt atccaggtgc cctgagtgta acagtgagga 60

ttatccaggg gggccctgag tgtaacagcg aggattatcc agggggccct gagtgcaaca 120

gtgaggatta tccagggggg cctgagtgta acagtgagga ttat 164

<210> 745
<211> 286
<212> DNA
<213> Homo sapiens

<400> 745

gtcttcacca actaagaaag ctctgttatt ctggcctggg tgcttgctcc agactcagga 60

acatctggtc aacacaagca tcactgggct ggggaattgt gtgtgtgctg catcatctcc 120

gactctcttg tagttccttc cttccctccc tcactcttac atgcagacac agacagacac 180

agtctggttg ggacatgcag tggcagctcc tggtgtataa catctttcac acaccttgag 240

tctatctgct tgctgccttt gactgatcct gaaatggttg gccttt 286

<210> 746
<211> 211
<212> DNA
<213> Homo sapiens

<400> 746

gtttgtgtgg gaactttgca agtcagtttc cctgtatgaa gtgatggaga gagtgattaa 60

gatactgagc tttctctgtg ttcttgccgt taaccattgc cggtttgtgg gagattaaga 120

agtcgatgcg ttttatggag aattaattta ttttgatata gacagatgga cgggtcatga 180

aaatttgttg acatacttta ctaaactgct a 211

<210> 747
<211> 113
<212> DNA
<213> Homo sapiens

<400> 747

tgtcactggt gaaagacgac ttggtgccca atttttaata aacacaatgc tattagcgtc 60

actccaattt agtgtctgat tgttaaatgt taatgtactg cactctacag ttt 113

<210> 748
<211> 251
<212> DNA
<213> Homo sapiens

<400> 748

gaaccgcaga ttatggttaa tccaattctg tgcacctgag gtccataaat aaaagaataa 60

gtattgaaat gaaagaatga cagaaagaat gaatggacac atgaacgact gaattagaaa 120

tggaaatgcc tggcacagcc aggaaggagc tgcccatggg attgtcattc atttcactct 180

gggcacctga ggtccataag cgtgaaaaga ggcaggaaga gaagtgtcag ggagtcaaag 240

atagagctaa g 251

<210> 749
<211> 135
<212> DNA
<213> Homo sapiens

<400> 749

gggcccagct cagaaccggg cagacacccc cttcaaatgt cttcgcacgt aggttttgca 60

cagtgtttat ttgctggtgt ctcagggatt tgacagtttc cttaatattc ccacacatgg 120

ccgagaaaaa taaat 135

<210> 750
<211> 187
<212> DNA
<213> Homo sapiens

<400> 750

ttctttatag tgttatgctt attttcaatt tttttttcc tgattctgtc tggtacttag 60

aattgtagtg tcttcatcat caattaaaga aaactgtcta aatgaattca tggatgtaaa 120

tattagtggt ccttaatgtc tttgattgct ggacatgaaa caaactgcca attaaatttt 180

gcggaga 187

<210> 751
<211> 66
<212> DNA
<213> Homo sapiens

<400> 751

gtcaccccag agtcattgtc acctcagagt cattgtcact ccagagtcat tgtcacctca 60

gagtca 66

<210> 752
<211> 258
<212> DNA
<213> Homo sapiens

<400> 752

ctcaccagga ctcgtctctc cattcccgtc agagtttgct ttgatttccc tttcctttcc 60

ttctcgggga ccagttctta cttcctttta tttttagctt tgcactccat gtggtttcag 120

ggttcagttt gatccatcaa aaggttcttt ttttataatc ccttttgaaa atgataatca 180

aaggaagaga tgtggtgttt ggtcatgtgg aaaactcaat gtataattta gacgtctgtc 240

aaaaatccga caaataaa 258

<210> 753
<211> 119
<212> DNA
<213> Homo sapiens

<220>
<221> misc_feature
<222> (91)..(91)
<223> n is a, c, g, or t

<400> 753

aaaatagaga gacatacctg gctccaaaac aaggctgtat cttctgccac tgtaataaaa 60

tagatgcaat tgaggttcat aaataaaaga ntaaatactt aaacgtgaaa ggtgactaa 119

<210> 754
<211> 218
<212> DNA
<213> Homo sapiens

<400> 754

gtttcaattg attcacaact ttaaaaaata tccacagagg cgtaagagga gatattgtat 60

tgcacccacg aaccagtttt atgctgttta agaaagggac attcaagaaa caaaaaggga 120

gctttgggaa atttgaacaa taaataatag tagaataaaa aattcagaga aagtttggat 180

gataaatttg agccaatctc ccagtaagca gagcaaaa 218

<210> 755
<211> 209
<212> DNA
<213> Homo sapiens

<400> 755

cagagtcatt cattgtcacc ccagagtcat tgtcacccga gtcattgtca gctcagagtc 60

attgtcaccc cagagtcatt gtcaccccag agtcattgtc accccattgt caccccagag 120

tcattgtcac ctcagagtca ttgtcactcc agagtcattg tcacctcaga gtcaatgtca 180

ccccagagtc atcgtcaccc cagagacat 209

<210> 756

<211> 123
<212> DNA
<213> Homo sapiens

<400> 756

```
catttaaata agaggagatc cacaaagagc aacagagata agagaagaaa ggaaataatc      60

aataaaataa ttcaagaaaa tccaaggaca tgagttttca gattgtaaga gactacttga     120

gtg                                                                   123
```

<210> 757
<211> 278
<212> DNA
<213> Homo sapiens

<400> 757

```
aaatggcttc cgatttccag cttgggcctg gggattggag atgtccccac tgagagtagg      60

gcacaagtga ggaaatggtt tggagaggaa gatgataagt tacatcatgg atgtgctgag     120

tctgagttgc ctatgggact tggaatgggg ggtggcaaaa ggtgtgtgat cttgagcaag     180

atattcaact cttctgggcc ttggtcttct catttgtaaa acggtgataa gaatattact     240

tcccatttgt gttgctgtga atattaaatg cgctacca                             278
```

<210> 758
<211> 123
<212> DNA
<213> Homo sapiens

<400> 758

```
catttaaata agaggagatc cacaaagagc aacagagata agagaagaaa ggaaataatc      60

aataaaataa ttcaagaaaa tccaaggaca tgagttttca gattgtaaga gactacttga     120

gtg                                                                   123
```

<210> 759
<211> 278
<212> DNA
<213> Homo sapiens

<400> 759

ctatcaggta ctgtgctcat ctgaacaaca aacctcaaca acacgcaatt tatccatgta 60

attaacctgc acatgtaccc ccgaaaccta aaataaaagt tcaaaaaaaa cctgtggtat 120

ttaaataggt attgtgtcta aaaatgcatg ctatctaaaa atgtagtttt attgcactgt 180

ataagaatac gagaggttta aaatagacac tctaaaagtt ataagcccta atttacatat 240

attctctagc ctttctccac cttctatcta ccaaaaaa 278

<210> 760
<211> 119
<212> DNA
<213> Homo sapiens

<400> 760

attttctatc tgtggttgga ttcagaccac atgaacactg agggctgact gtagttttga 60

atgtctgtta ctgaggaggc accagcataa agtattttat cacttcagac gctgacaat 119

<210> 761
<211> 103
<212> DNA
<213> Homo sapiens

<400> 761

gtttgtcttg cgcagtgctt actccagctg tggcatgcag gtgtcagcaa gtatgatcag 60

caatgaggcg gtggtcaata tcctgtcgag ctcatcacca cag 103

<210> 762
<211> 196
<212> DNA
<213> Homo sapiens
<400> 762

tttatttata ccaagcctcc tcctgaaatt tctcttcctt tctttctacc tacccaagac 60

ataccacgtg cttcaataac cagtcccttc ctcctacaaa cactacaacc tggaaagcac 120

tcttgctttt ctgaagtcct ctatacttag tgtaactctt ctgtgatgaa gattaaagtg 180

tattatggca actctc 196

<210> 763
<211> 115
<212> DNA
<213> Homo sapiens

<400> 763

ttcattgagg ttcgtgtgtg ctgtgttcgc gtgtgtgtgc tgtgtgtgca tgtgtgtgct 60

gtgtcttagt gtgtgtgctg tgtgctagtg tgtgtgctgt ggggtaccga gctcg 115

<210> 764
<211> 47
<212> DNA
<213> Homo sapiens

<400> 764
gggatgcata aagtatgagt gccttttagg atgggaattg agatgta 47

<210> 765
<211> 66
<212> DNA
<213> Homo sapiens

<400> 765

gtttttatta aaacacaaac gcacacacac acaaaattag ccaggcatgg tggtccatcc 60

ctgtaa 66

<210> 766
<211> 143
<212> DNA
<213> Homo sapiens

<400> 766

gtgtgagcca ctgctggcta atttttgtat tttcagtaga gacggggctt caccatattg 60

gccaggctgg tcttgaactc cttccctcgc tcccccaaaa atttgaattt ttttttcaac 120

acttttacac ctgttatgga aaa 143

<210> 767
<211> 104
<212> DNA
<213> Homo sapiens

<400> 767

tgctgcggtc gcctctctca ggctcccccg ctcccccggg cctccgcttc tcagccgggt 60

gctgtgcgtt tgagtgtgtg ctgctgctcg ctgtgtgtgc tgtg 104

<210> 768
<211> 242
<212> DNA
<213> Homo sapiens

<400> 768

aaagagtcaa ctttccggcg gcgggggaga aatgataaaa gagagagagg agggcagatc 60

accacctaga agcacatcct ttgttcgcag agggggggaga aaaagtcgga gagaaagaat 120

gaaagagggg aaaaaaggca gttcggcacc cggagaaagg aggcaattcg gggagaggag 180

gaggagaaga agaaaacaca cacgcgcgca cgcacacaca caccgcggag agaaaagaac 240

ag 242

<210> 769
<211> 274
<212> DNA
<213> Homo sapiens

<400> 769

tgctcagtgt catgctgtgt gtgcatgtgt gtgctgtgtg ttttgtgtgt gtgctgtgtt 60

catgtgtgct gtgcatgcgt gtgtgctgtg tgtgcctgtg tgtgcggtgt gctgtgtcct 120

tgtgtgtgct gtgtgtgcgt gtgctgtgtg catgtgtgtg ctgtgttatg tcgtgtgtgc 180

agtgtgtgct gtgtccgatg tgtgctgtgt acacatgaga gagcagagtg tacatgtgtg 240

tgctgagtct atcagaagat gtgtgtagct gcgg 274

<210> 770
<211> 267
<212> DNA
<213> Homo sapiens

<400> 770

atgactgtgc tagagccacc ctctcacttt agcctcctga gtagctggga ctacaggtgc 60

ttcccactgt gcctggccaa ttaacaattt catttttatt tttagtagag atgagatctc 120

actatgttgc ccaggctggt cctgaactcc tgagctcaag agatcctccc accttggcct 180

cccaaagtac tgggattaca aacaagagcc actgtgcctg accaggctct aagattgcta 240

atctggctat agaaggacta atgttag 267

<210> 771
<211> 98
<212> DNA
<213> Homo sapiens

<400> 771

aaagagtcaa ctttccggcg gcgggggaga aatgataaaa gagagagagg agggcagatc 60

accacctaga agcacatcct ttgttcgcag agggggga 98

<210> 772
<211> 127

<212> DNA
<213> Homo sapiens

<400> 772

tttttagtt gccaacagtt gtatttttgc tgattattta tgaccttaaa taatatattt 60

tttttttaa gaagacattt tgttacataa ggaaaacttt tttattcaat ggaataaatt 120

atggcat 127

<210> 773
<211> 114
<212> DNA
<213> Homo sapiens

<400> 773

tttttagtt gccaacagtt tatgtttgct gattatttat gacctgaaat tatatatttt 60

tttttaag aagacatttt gttacataag gatgactttt ttatacaaag gaaa 114

<210> 774
<211> 168
<212> DNA
<213> Homo sapiens

<400> 774

aagaaaagga gaagcactac aagcttttga aaaatggaaa gagaaaaaga tggaatatct 60

taaagagaaa aatagaaagg agagagaata tgaaagagca aagaaacaga aagaggagga 120

aactgttgcc gagaaaaaga aagataattt aactgctgtt gagaaatg 168

<210> 775
<211> 133
<212> DNA
<213> Homo sapiens

<400> 775

aatgcagaat attcctctga acacttccct catacatcat tcaatattat aaattaaaca 60

caaagagcct ctccacttag atttttatca tgcatcctac attgtaatgt ctttactctt 120

ccatagaaaa ggt 133

<210> 776
<211> 116
<212> DNA
<213> Homo sapiens

<400> 776

ctgggccttg gtccccagaa gatggcggct agggcctcgc cgccaggaca gagaagggac 60

ggggtggctg ggcagtcagg gaaggagggt cgcccggatc cgacattttg gagaga 116

<210> 777
<211> 96
<212> DNA
<213> Homo sapiens

<400> 777

ggatcgatga tgagtccccc ataaaaacat tccttggaaa agctgaacaa aatgagtgag 60

aactcatacc gtcgttctca tcagaactga ggtcca 96

<210> 778
<211> 96
<212> DNA
<213> Homo sapiens

<400> 778

ggatcgatga tgagtccccc ataaaaacat tccttggaaa agctgaacaa aatgagtgag 60

aactcatacc gtcgttctca tcggaactga ggtcca 96

<210> 779
<211> 64
<212> DNA
<213> Homo sapiens

<400> 779

ccatgaagga gcaagttttg tatttgtgac ctcagctttg ggaataaagg atcttttgaa 60

ggcc 64

<210> 780
<211> 101
<212> DNA
<213> Homo sapiens

<400> 780

tgagtgtgct gtgctcgcgt gtgtgctgtg ttcatgcgtg tgctgtgtgt tgtgtgtgtg 60

tagctgcggg gatgcataaa gtatgagtgc tttttaggat g 101

<210> 781
<211> 106
<212> DNA
<213> Homo sapiens

<400> 781

gtgcatgtgt gtgctgtgtc tttgtgtgtg tgctgtgtgc tagtgtgtgt gctgtgtgtg 60

catgtgtgtg cgtgtgctgt gcgtttgtgt gctgtgtgct cgtgtg 106

<210> 782
<211> 115
<212> DNA
<213> Homo sapiens

<400> 782

gatgggaatt gagatgtaag atttgggggt gagggtcgtg ccaattacat ttcatttgca 60

tggattttgg ttttcatgct ctgtcctccc ctcctttggt cttactgggt ccctc 115

<210> 783
<211> 217
<212> DNA
<213> Homo sapiens

<400> 783

agcgaaggga aggttgcggg agaaagagcg ggggccgggg ccagacgcca agaggggcgc 60

ggggagcaca gagaagcgga gggaagggcg ccacgtcgag gggccggggg aggcggtgac 120

tgggggggcgg agtggaggct gcacccggac cgcgggcgcc cagctcggtt tgggccgacg 180

gagccctctg ccgtcgcgag cccgggcctc gggaggg 217

<210> 784
<211> 158
<212> DNA
<213> Homo sapiens

<400> 784

gttcaaatga tctacactta cattttgcaa atcttttttt ttaaattttt taaattttat 60

attttttttc cagccaactc aaggccaaaa aaaatttctt aatatagtta ttatgcgagg 120

ggaggggaag caaaggagca caggtagtcc acagaata 158

<210> 785
<211> 74
<212> DNA
<213> Homo sapiens

<400> 785

ggtgcaatta ttatactgcg aaaatgaaaa tattgcatac taaacagtac ctagggtatg 60

atctcaatgt aaaa 74

<210> 786
<211> 150
<212> DNA
<213> Homo sapiens

<400> 786

```
agggacccag taagaccaaa ggaggggagg acagagcatg aaaaccaaaa tccatgcaaa      60
tgaaatgtaa ttggcacgac cctcaccccc aaatcttaca tctcaattcc catcctaaaa     120
agcactcata ctttatgcat ccccgcagct                                      150
```

<210> 787
<211> 86
<212> DNA
<213> Homo sapiens

<400> 787

```
atcatttcat agtcatttat gtttcatcgg tcctcatgtg tactagtgcg ttattttact      60
tatactcccg gatatcatat tattta                                           86
```

<210> 788
<211> 183
<212> DNA
<213> Homo sapiens

<400> 788

```
acaaaactct ccaataatga ctctgctcag ctcaggggca gcaggagtgg agtgtcgggg      60
gcccttggtg ctgagtgaag ataaattaaa aatcccaaca agccagtgac attatgtaca     120
ggaggaaagg ggtggggctt ccaggacaga ggccgagggt ggcagggcag gacttggagt     180
ggc                                                                   183
```

<210> 789
<211> 349
<212> PRT
<213> Homo sapiens

<400> 789

```
Met Glu Thr Pro Pro Val Asn Thr Ile Gly Glu Lys Asp Thr Ser Gln
1               5                   10                  15

Pro Gln Gln Glu Trp Glu Lys Asn Leu Arg Glu Asn Leu Asp Ser Val
            20                  25                  30

Ile Gln Ile Arg Gln Gln Pro Arg Asp Pro Pro Thr Glu Thr Leu Glu
        35                  40                  45

Leu Glu Val Ser Pro Asp Pro Ala Ser Gln Ile Leu Glu His Thr Gln
    50                  55                  60

Gly Ala Glu Lys Leu Val Ala Glu Leu Glu Gly Asp Ser His Lys Ser
65                  70                  75                  80

His Gly Ser Thr Ser Gln Met Pro Glu Ala Leu Gln Ala Ser Asp Leu
                85                  90                  95

Trp Tyr Cys Pro Asp Gly Ser Phe Val Lys Lys Ile Val Ile Arg Gly
            100                 105                 110

His Gly Leu Asp Lys Pro Lys Leu Gly Ser Cys Cys Arg Val Leu Ala
        115                 120                 125

Leu Gly Phe Pro Phe Gly Ser Gly Pro Pro Glu Gly Trp Thr Glu Leu
    130                 135                 140

Thr Met Gly Val Gly Pro Trp Arg Glu Glu Thr Trp Gly Glu Leu Ile
145                 150                 155                 160
```

Glu Lys Cys Leu Glu Ser Met Cys Gln Gly Glu Glu Ala Glu Leu Gln
165 170 175

Leu Pro Gly His Ser Gly Pro Pro Val Arg Leu Thr Leu Ala Ser Phe
180 185 190

Thr Gln Gly Arg Asp Ser Trp Glu Leu Glu Thr Ser Glu Lys Glu Ala
195 200 205

Leu Ala Arg Glu Glu Arg Ala Arg Gly Thr Glu Leu Phe Arg Ala Gly
210 215 220

Asn Pro Glu Gly Ala Ala Arg Cys Tyr Gly Arg Ala Leu Arg Leu Leu
225 230 235 240

Leu Thr Leu Pro Pro Pro Gly Pro Pro Glu Arg Thr Val Leu His Ala
245 250 255

Asn Leu Ala Ala Cys Gln Leu Leu Leu Gly Gln Pro Gln Leu Ala Ala
260 265 270

Gln Ser Cys Asp Arg Val Leu Glu Arg Glu Pro Gly His Leu Lys Ala
275 280 285

Leu Tyr Arg Arg Gly Val Ala Gln Ala Ala Leu Gly Asn Leu Glu Lys
290 295 300

Ala Thr Ala Asp Leu Lys Lys Val Leu Ala Ile Asp Pro Lys Asn Arg
305 310 315 320

Ala Ala Gln Glu Glu Leu Gly Lys Val Val Ile Gln Gly Lys Asn Gln
325 330 335

Asp Ala Gly Leu Ala Gln Gly Leu Arg Lys Met Phe Gly
340 345

<210> 790
<211> 349
<212> PRT
<213> Homo sapiens

<400> 790

Met Glu Thr Pro Pro Val Asn Thr Ile Gly Glu Lys Asp Thr Ser Gln
1 5 10 15

Pro Gln Gln Glu Trp Glu Lys Asn Leu Arg Glu Asn Leu Asp Ser Val
20 25 30

```
Ile Gln Ile Arg Gln Gln Pro Arg Asp Pro Pro Thr Glu Thr Leu Glu
        35                  40                  45

Leu Glu Val Ser Pro Asp Pro Ala Ser Gln Ile Leu Glu His Thr Gln
    50                  55                  60

Gly Ala Glu Lys Leu Val Ala Glu Leu Glu Gly Asp Ser His Lys Ser
65                  70                  75                  80

His Gly Ser Thr Ser Gln Met Pro Glu Ala Leu Gln Ala Ser Asp Leu
                85                  90                  95

Trp Tyr Cys Pro Asp Gly Ser Phe Val Lys Lys Ile Val Ile Arg Gly
            100                 105                 110

His Gly Leu Asp Lys Pro Lys Leu Gly Ser Cys Cys Arg Val Leu Ala
        115                 120                 125

Leu Gly Phe Pro Phe Gly Ser Gly Pro Pro Glu Gly Trp Thr Glu Leu
    130                 135                 140

Thr Met Gly Val Gly Pro Trp Arg Glu Glu Thr Trp Gly Glu Leu Ile
145                 150                 155                 160

Glu Lys Cys Leu Glu Ser Met Cys Gln Gly Glu Glu Ala Glu Leu Gln
                165                 170                 175

Leu Pro Gly His Thr Gly Pro Pro Val Gly Leu Thr Leu Ala Ser Phe
            180                 185                 190

Thr Gln Gly Arg Asp Ser Trp Glu Leu Glu Thr Ser Glu Lys Glu Ala
        195                 200                 205

Leu Ala Arg Glu Glu Arg Ala Arg Gly Thr Glu Leu Phe Arg Ala Gly
    210                 215                 220

Asn Pro Glu Gly Ala Ala Arg Cys Tyr Gly Arg Ala Leu Arg Leu Leu
225                 230                 235                 240

Leu Thr Leu Pro Pro Pro Gly Pro Pro Glu Arg Thr Val Leu His Ala
                245                 250                 255

Asn Leu Ala Ala Cys Gln Leu Leu Leu Gly Gln Pro Gln Leu Ala Ala
            260                 265                 270

Gln Ser Cys Asp Arg Val Leu Glu Arg Glu Pro Gly His Leu Lys Ala
        275                 280                 285
```

Leu Tyr Arg Arg Gly Val Ala Gln Ala Ala Leu Gly Asn Leu Glu Lys
290 295 300

Ala Thr Ala Asp Leu Lys Lys Val Leu Ala Ile Asp Pro Lys Asn Arg
305 310 315 320

Ala Ala Gln Glu Glu Leu Gly Lys Val Val Ile Gln Gly Lys Asn Gln
325 330 335

Asp Ala Gly Leu Ala Gln Gly Leu Arg Lys Met Phe Gly
340 345

<210> 791
<211> 23
<212> PRT
<213> Homo sapiens

<400> 791

Ile Arg Gln Gln Pro Arg Asp Pro Pro Thr Glu Thr Leu Glu Leu Glu
1 5 10 15

Val Ser Pro Asp Pro Ala Ser
20

<210> 792
<211> 24
<212> PRT
<213> Homo sapiens

<400> 792

Gln Ile Arg Gln Gln Pro Arg Asp Pro Pro Thr Glu Thr Leu Glu Leu
1 5 10 15

Glu Val Ser Pro Asp Pro Ala Ser
20

<210> 793
<211> 199
<212> PRT
<213> Homo sapiens

<400> 793

Met Glu Thr Pro Pro Val Asn Thr Ile Gly Glu Lys Asp Thr Ser Gln
1 5 10 15

Pro Gln Gln Glu Trp Glu Lys Asn Leu Arg Glu Asn Leu Asp Ser Val
20 25 30

```
Ile Gln Ile Arg Gln Gln Pro Arg Asp Pro Pro Thr Glu Thr Leu Glu
        35                  40                  45

Leu Glu Val Ser Pro Asp Pro Ala Ser Gln Ile Leu Glu His Thr Gln
    50                  55                  60

Gly Ala Glu Lys Leu Val Ala Glu Leu Glu Gly Asp Ser His Lys Ser
65                  70                  75                  80

His Gly Ser Thr Ser Gln Met Pro Glu Ala Leu Gln Ala Ser Asp Leu
                85                  90                  95

Trp Tyr Cys Pro Asp Gly Ser Phe Val Lys Lys Ile Val Ile Arg Gly
            100                 105                 110

His Gly Leu Asp Lys Pro Lys Leu Gly Ser Cys Cys Arg Val Leu Ala
        115                 120                 125

Leu Gly Phe Pro Phe Gly Ser Gly Pro Pro Glu Gly Trp Thr Glu Leu
    130                 135                 140

Thr Met Gly Val Gly Pro Trp Arg Glu Glu Thr Trp Gly Glu Leu Ile
145                 150                 155                 160

Glu Lys Cys Leu Glu Ser Met Cys Gln Gly Glu Glu Ala Glu Leu Gln
                165                 170                 175

Leu Pro Gly His Thr Gly Pro Pro Val Gly Leu Thr Leu Ala Ser Phe
            180                 185                 190

Thr Gln Gly Arg Asp Ser Trp
        195
```

<210> 794
<211> 150
<212> PRT
<213> Homo sapiens

<400> 794

```
Met Glu Thr Pro Pro Val Asn Thr Ile Gly Glu Lys Asp Thr Ser Gln
1               5                   10                  15

Pro Gln Gln Glu Trp Glu Lys Asn Leu Arg Glu Asn Leu Asp Ser Val
            20                  25                  30

Ile Gln Ile Arg Gln Gln Pro Arg Asp Pro Pro Thr Glu Thr Leu Glu
        35                  40                  45
```

Leu Glu Val Ser Pro Asp Pro Ala Ser Gln Ile Leu Glu His Thr Gln
50 55 60

Gly Ala Glu Lys Leu Val Ala Glu Leu Glu Gly Asp Ser His Lys Ser
65 70 75 80

His Gly Ser Thr Ser Gln Met Pro Glu Ala Leu Gln Ala Ser Asp Leu
85 90 95

Trp Tyr Cys Pro Asp Gly Ser Phe Val Lys Lys Ile Val Ile Arg Gly
100 105 110

His Gly Leu Asp Lys Pro Lys Leu Gly Ser Cys Cys Arg Val Leu Ala
115 120 125

Leu Gly Phe Pro Phe Gly Ser Gly Pro Pro Glu Gly Trp Thr Glu Leu
130 135 140

Thr Met Gly Val Gly Pro
145 150

<210> 795
<211> 85
<212> PRT
<213> Homo sapiens

<400> 795

Met Glu Thr Pro Pro Val Asn Thr Ile Gly Glu Lys Asp Thr Ser Gln
1 5 10 15

Pro Gln Gln Glu Trp Glu Lys Asn Leu Arg Glu Asn Leu Asp Ser Val
20 25 30

Ile Gln Ile Arg Gln Gln Pro Arg Asp Pro Pro Thr Glu Thr Leu Glu
35 40 45

Leu Glu Val Ser Pro Asp Pro Ala Ser Gln Ile Leu Glu His Thr Gln
50 55 60

Gly Ala Glu Lys Leu Val Ala Glu Leu Glu Gly Asp Ser His Lys Ser
65 70 75 80

His Gly Ser Thr Ser
85

<210> 796
<211> 57
<212> PRT

<213> Homo sapiens

<400> 796

```
Met Glu Thr Pro Pro Val Asn Thr Ile Gly Glu Lys Asp Thr Ser Gln
1               5                   10                  15

Pro Gln Gln Glu Trp Glu Lys Asn Leu Arg Glu Asn Leu Asp Ser Val
            20                  25                  30

Ile Gln Ile Arg Gln Gln Pro Arg Asp Pro Pro Thr Glu Thr Leu Glu
        35                  40                  45

Leu Glu Val Ser Pro Asp Pro Ala Ser
    50                  55
```

<210> 797
<211> 47
<212> **PRT**
<213> Homo sapiens

<400> 797

```
Met Glu Thr Pro Pro Val Asn Thr Ile Gly Glu Lys Asp Thr Ser Gln
1               5                   10                  15

Pro Gln Gln Glu Trp Glu Lys Asn Leu Arg Glu Asn Leu Asp Ser Val
            20                  25                  30

Ile Gln Ile Arg Gln Gln Pro Arg Asp Pro Pro Thr Glu Thr Leu
        35                  40                  45
```

<210> 798
<211> 18
<212> PRT
<213> Homo sapiens

<400> 798

```
Gln Gln Pro Arg Asp Pro Pro Thr Glu Thr Leu Glu Leu Glu Val Ser
1               5                   10                  15

Pro Asp
```

<210> 799
<211> 21
<212> PRT
<213> Homo sapiens

<400> 799

```
Gln Ile Arg Gln Gln Pro Arg Asp Pro Pro Thr Glu Thr Leu Glu Leu
1               5                   10                  15
```

Glu Val Ser Pro Asp
20

<210> 800
<211> 18
<212> PRT
<213> Homo sapiens

<400> 800

Gln Ile Arg Gln Gln Pro Arg Asp Pro Pro Thr Glu Thr Leu Glu Leu
1 5 10 15

Glu Val

<210> 801
<211> 15
<212> PRT
<213> Homo sapiens

<400> 801

Gln Ile Arg Gln Gln Pro Arg Asp Pro Pro Thr Glu Thr Leu Glu
1 5 10 15

<210> 802
<211> 12
<212> PRT
<213> Homo sapiens

<400> 802

Gln Ile Arg Gln Gln Pro Arg Asp Pro Pro Thr Glu
1 5 10

<210> 803
<211> 21
<212> PRT
<213> Homo sapiens

<400> 803

Gln Gln Pro Arg Asp Pro Pro Thr Glu Thr Leu Glu Leu Glu Val Ser
1 5 10 15

Pro Asp Pro Ala Ser
20

<210> 804
<211> 18
<212> PRT
<213> Homo sapiens

<400> 804

Arg Asp Pro Pro Thr Glu Thr Leu Glu Leu Glu Val Ser Pro Asp Pro
1 5 10 15

Ala Ser

<210> 805
<211> 15
<212> PRT
<213> Homo sapiens

<400> 805

Pro Thr Glu Thr Leu Glu Leu Glu Val Ser Pro Asp Pro Ala Ser
1 5 10 15

<210> 806
<211> 12
<212> PRT
<213> Homo sapiens

<400> 806

Thr Leu Glu Leu Glu Val Ser Pro Asp Pro Ala Ser
1 5 10

<210> 807
<211> 19
<212> PRT
<213> Homo sapiens

<400> 807

Arg Gln Gln Pro Arg Asp Pro Pro Thr Glu Thr Leu Glu Leu Glu Val
1 5 10 15

Ser Pro Asp

<210> 808
<211> 18
<212> PRT
<213> Homo sapiens

<400> 808

Arg Gln Gln Pro Arg Asp Pro Pro Thr Glu Thr Leu Glu Leu Glu Val
1 5 10 15

Ser Pro

<210> 809
<211> 17
<212> PRT
<213> Homo sapiens

<400> 809

```
Arg Gln Gln Pro Arg Asp Pro Pro Thr Glu Thr Leu Glu Leu Glu Val
1               5                   10                  15

Ser
```

<210> 810
<211> 16
<212> PRT
<213> Homo sapiens

<400> 810

```
Pro Arg Asp Pro Pro Thr Glu Thr Leu Glu Leu Glu Val Ser Pro Asp
1               5                   10                  15
```

<210> 811
<211> 15
<212> PRT
<213> Homo sapiens

<400> 811

```
Arg Asp Pro Pro Thr Glu Thr Leu Glu Leu Glu Val Ser Pro Asp
1               5                   10                  15
```